(19) Europäisches Patentamt
European Patent Office
Office européen des brevets

(11) **EP 4 596 536 A1**

(12) **EUROPEAN PATENT APPLICATION**
published in accordance with Art. 153(4) EPC

(43) Date of publication:
**06.08.2025 Bulletin 2025/32**

(21) Application number: **23871065.1**

(22) Date of filing: **28.09.2023**

(51) International Patent Classification (IPC):
***C07D 223/16*** *(2006.01)* ***A61K 47/68*** *(2017.01)*
***C07K 16/28*** *(2006.01)* ***A61P 35/00*** *(2006.01)*

(52) Cooperative Patent Classification (CPC):
**A61K 31/55; A61K 47/68; A61P 35/00; C07D 223/16; C07D 401/12; C07D 471/04; C07F 9/6558; C07K 5/06017; C07K 5/0804; C07K 16/28; C07K 16/32**

(86) International application number:
**PCT/CN2023/122797**

(87) International publication number:
**WO 2024/067841 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.09.2022 CN 202211211450**
**18.11.2022 CN 202211449284**
**20.09.2023 CN 202311221551**

(71) Applicant: **Shanghai de Novo Pharmatech Co., Ltd.**
**Shanghai 201203 (CN)**

(72) Inventors:
- **ZHANG, Wei**
  **Shanghai 201203 (CN)**
- **QIN, Ping**
  **Shanghai 201203 (CN)**
- **YANG, Wei**
  **Shanghai 201203 (CN)**
- **WANG, Longsheng**
  **Shanghai 201203 (CN)**
- **GAO, Daxin**
  **Shanghai 201203 (CN)**

(74) Representative: **Murgitroyd & Company**
**165-169 Scotland Street**
**Glasgow G5 8PL (GB)**

# (54) BENZAZEPINE DERIVATIVE, CONJUGATE CONTAINING SAME, AND USE THEREOF

(57) Provided are a benzazepine derivative, a conjugate containing same, and use thereof. Provided is an antibody-immunostimulatory conjugate represented by formula (I) or a pharmaceutically acceptable salt thereof. The benzazepine derivative has a good regulation effect on TLR8, and can effectively treat, relieve and/or prevent various related diseases, such as cancer, caused by immunosuppression.

$$Ab\text{—}(L\text{—}D)_t$$

(I)

EP 4 596 536 A1

## Description

The present application claims priority to the Chinese Patent Application No. 2022112114506 filed on September 30, 2022, Chinese Patent Application No. 2022114492843 filed on November 18, 2022, and Chinese Patent Application No. 2023112215516 filed on September 20, 2023. The above-mentioned Chinese patent applications are incorporated herein by reference in their entirety.

## TECHNICAL FIELD

The present disclosure relates to a benzazepine derivative, a conjugate containing the same and use thereof.

## BACKGROUND

The Toll-like receptor (TLR) family is a family of important proteins for recognizing pathogen-related molecular patterns, which can sense and initiate innate immune responses and promote the development of adaptive immune responses. TLRs are mainly expressed in immune cells, such as myeloid dendritic cells (mDCs), plasmacytoid dendritic cells (pDCs), monocytes and B cells (Kawai and Akira, 2010), as well as in the lungs. In humans, more than 10 TLRs are considered to have significant functions. TLR1/2/4/5 and 6 are located in the cell membrane, and their main function is to recognize extracellular macromolecular ligands from bacteria and fungi. In contrast, TLR3/7/8/9 is located in intracellular endosomal membranes, and its main function is to recognize exogenous nucleic acids from pathogen cells. Although most TLRs function through specific signaling pathways (mainly through MyD88-dependent pathways), different TLRs can coordinate different downstream molecules. The addition of specific TLRs leads to the activation of different cell populations (Schreibelt, et al., 2010) and the generation of different patterns of cytokines and other inflammatory mediators (Ghosh, et al., 2006), thereby eliciting different immune responses. For example, upon binding to a ligand, TLR8 dimerizes and undergoes conformational changes, leading to the recruitment of the adaptor protein MyD88. MyD88 recruits interleukin 1 receptor-related kinases, leading to the activation of downstream signaling pathways, including mitogen-related protein kinases and transcription factor NF-κB.

TLRs located in endosomes, mainly TLR7/8/9, have been considered highly attractive new targets for anticancer immunotherapy (Kanzler, et al., 2007; Kreig 2008; Smits, et al., 2008; Hennessy, et al., 2010; Kaczanowska, et al., 2013; Beesu, et al., 2016). For example, TLR7 activates pDCs in response to viral infections, induces high levels of interferonα, and induces adaptive T cell responses against endogenous viral antigens (Liu, et al., 2009). Compared with TLR7/9, TLR8 is more widely expressed in different subtypes of immune cells. Regulatory T cells (Tregs) possess potent capabilities in immune response suppression, and are a major obstacle to effective cancer immunotherapy. The TLR8 signaling pathway has been shown to be a necessary and sufficient condition for reversing the suppressive function of Treg cells and leading to potent tumor suppression. TLR8 selective agonists effectively activate a variety of immune cells, including mDCs and monocytes (Gorden, et al., 2005), and can promote the generation of adaptive immune responses against cancer cells (Krug, et al., 2003; Schnurr, et al., 2005). Activated mDCs phagocytose apoptotic and necrotic tumor cells and then cross-present tumor-associated antigens to CD8+ CTLs more efficiently than pDCs (Berard, et al., 2000; Dalgaard, et al., 2005). In addition, the activation of mDCs leads to the release of TNFα and interleukin-12 (IL-12), which can stimulate the activation of T cells and NK cells. The activation of NK cells is the main mechanism of antibody-dependent cell-mediated cytotoxicity (ADCC). Therefore, enhanced killing of tumor cells through ADCC may present an important therapeutic opportunity for TLR8 selective inhibitors (Lu, et al., 2011). Some monoclonal antibody therapies are widely used in the treatment of cancer patients, such as Rituximab and Trastuzumab, which can exert therapeutic effects through ADCC (Ferris, et al., 2010). In fact, adding TLR8 agonists to mAb therapy can enhance ADCC and increase the efficacy of mAb therapy (Ferris, et al., 2015). In addition, recent studies have shown that TLR8 agonists can directly exert anti-tumor effects, independent of their immunomodulatory functions (Ignatz-Hoover, et al., 2015). Therefore, TLR8 agonists can not only act as monotherapy, but also improve the efficacy of various chemotherapies and targeted anticancer drugs by enhancing host immune responses.

Among the members of the TLR family that recognize the nucleic acids of pathogenic microorganisms, TLR7 and TLR8 have high homology and can recognize some artificially synthesized small molecules with antiviral effects, such as small molecular compounds Imidazoquinolines (ligands of TLR7 and TLR8). In models of genital herpes in guinea pigs infected with HSV, Imidazoquinolins were studied and found to have a relatively small effect on virus replication in vitro, but a strong effect in vivo, suggesting that such compounds promote the production of pro-inflammatory factors and regulatory cytokines by immune cells, resulting in antiviral responses (Int Immunopharmacol 2002; 2: 443-451). More importantly, TLR7 and TLR8 can recognize viral ssRNA. Studies have demonstrated that ssRNA viruses are natural ligands of TLR7 and TLR8, such as human immunodeficiency virus type I (HIV), influenza virus, Sendai virus, dengue virus, Newcastle disease virus (NDV), vesicular stomatitis virus (VSV), hepatitis B virus (HBV), and hepatitis C virus (HCV), etc. TLR8 can recognize antiviral compounds, ssRNA viruses, artificially synthesized oligonucleotides, etc., induce Th1 and inhibit Th2

cytokine secretion and Treg proliferation through the MyD88-dependent signaling pathway, mediate antiviral immunity, and exert anti-infection and anti-allergic effects.

Therefore, TLR8 is currently a highly attractive therapeutic target. Despite extensive research on TLRs, there is still a huge opportunity to further broaden their applications and advantages. The compounds and applications described in the present disclosure will contribute to the development of TLR8 agonists and meet unmet clinical needs.

## SUMMARY

The technical problem to be solved by the present disclosure is the relatively limited structural diversity of existing TLR8 agonists. Therefore, the present disclosure provides a benzazepine derivative, a conjugate containing the same and use thereof. The benzazepine derivative exhibits good modulatory effects on TLR8, and can effectively treat, alleviate and/or prevent various related diseases caused by immunosuppression, such as cancer.

The present disclosure provides an antibody-immunostimulatory conjugate of formula I or a pharmaceutically acceptable salt thereof,

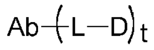

(I)

where Ab is an antibody;

t is any numerical value from 1 to 8;

D is a benzazepine group as shown below:

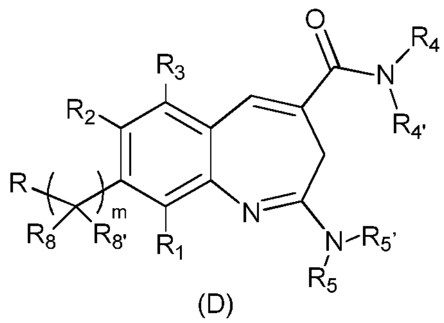

(D)

;

m is 0 or 1;

R is $-C(O)-NR_9-L_1-R_7$, $-C(S)-NR_9-L_1-R_7$ or $-NR_9-C(O)-L_1-R_7$;

$R_1$, $R_2$ and $R_3$ are each independently hydrogen, deuterium, halogen, hydroxyl, amino, cyano, alkyl, haloalkyl, $-L_2-OR_a$ or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl or heteroarylalkyl; and the $R_4$ or $R_{4'}$ is unsubstituted or optionally substituted at any position with one or more substituents selected from halogen, cyano, $-L_2-OR_a$, $-L_2-OR_g$, $-L_2-OC(O)R_a$, $-L_2-OC(O)OR_a$, $-L_2-OC(O)NR_aR_b$, $-L_2-NR_aR_b$, $-L_2-NR_aC(O)OR_b$, $-L_2-NR_aC(O)NR_aR_b$, $-L_2-NR_bC(NR_b)NR_aR_b$ and $-L_2-C(O)OR_b$; or $R_4$ and $R_{4'}$ together with the N atom to which they are attached form 3-8 membered heterocycloalkyl; and the 3-8 membered heterocycloalkyl is unsubstituted or further substituted at any position with 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$ and $-L_2-C(O)OR_b$;

$R_5$ is hydrogen, $-OR_a$, $-C(O)R_a$, $-C(O)OR_a$ or $-C(O)NR_aR_b$;

$R_{5'}$ is $-R_c$;

$R_7$ is phenyl, 5-6 membered heteroaryl, 3-8 membered heterocycloalkyl or an 8-12 membered fused ring group; and the $R_7$ is unsubstituted or optionally substituted at any position with one or more substituents selected from $-L_3-W$, $-R_c$, halogen, cyano, nitro, alkyl, alkenyl, alkynyl, alkoxy, haloalkyl, haloalkoxy and alkylamino;

$R_8$ and $R_{8'}$ are each independently hydrogen, halogen or alkyl, and the $R_8$ or $R_{8'}$ is unsubstituted or optionally substituted at any position with one or more substituents selected from $-L_3-W$, halogen, cyano, nitro, alkyl, alkenyl, alkynyl, alkoxy, haloalkyl, haloalkoxy and alkylamino; $R_8$ and $R_{8'}$ are each independently a substituent, or $R_8$ and $R_{8'}$ together with the carbon atom to which they are attached form oxo, thio, $C_{1-6}$ alkylene, $C_{3-10}$ cycloalkyl or 3-10 membered heterocycloalkyl; and the $C_{1-6}$ alkylene, $C_{3-10}$ cycloalkyl or 3-10 membered heterocycloalkyl is unsubstituted or optionally substituted at any position with one or more substituents selected from deuterium, halogen,

hydroxyl, amino, cyano, oxo, alkyl, haloalkyl, - $L_2$-$OR_a$ and -$L_2$-$NR_aR_b$;
$R_9$ is hydrogen, alkyl, haloalkyl, -$L_2$-$OR_a$ or -$L_2$-$NR_aR_b$;
W is $Cy^1$, -$SR_d$, -$OR_d$, -OC(O)$R_e$, -OC(O)$NR_eR_{e'}$, -C(O)$OR_e$, -C(O)$R_e$, -C(O)$NR_eR_{e'}$,-C(O)$NR_eS(O)_2R_e$, -$NR_dR_e$, -$NR_dC(O)R_e$, -N($R_d$)C(O)$OR_e$, -N($R_d$)C(O)$NR_eR_{e'}$, -$NR_dS(O)_2R_e$, - $NR_dS(O)_2NR_eR_{e'}$, -$S(O)_{1-2}R_e$, -$S(O)_2NR_eR_{e'}$, -S(O)(=$NR_d$)$R_e$, -$S(O)_2N(R_e)C(O)R_{e'}$, -P(O)$(OR_e)_2$, - P(O)($OR_e$)$R_{e'}$, -OP(O)$(OR_e)_2$ or -B$(OR_e)_2$;
$Cy^1$ is cycloalkyl, heterocycloalkyl, aryl or heteroaryl; and the $Cy^1$ is unsubstituted or optionally substituted at any position with one or more substituents selected from halogen, alkyl, haloalkyl, haloalkoxy, alkenyl, alkynyl, cyano, -$R_c$, -$L_4$-$SR_d$, -$L_4$-OC(O)$R_e$, -$L_4$-C(O)$OR_e$, -$L_4$-C(O)$R_e$, -$L_4$-C(O)$NR_eR_{e'}$, -$L_4$-$NR_dC(O)R_e$, -$L_4$-$NR_dS(O)_2R_e$, -$L_4$-$S(O)_{1-2}R_e$, -$L_4$-$S(O)_2NR_eR_{e'}$, -$L_4$-$OR_d$ and -$L_4$-$NR_eR_{e'}$;
each $R_a$, $R_b$, $R_d$, $R_e$ and $R_{e'}$ is independently -$R_c$, amino, $C_{1-6}$ alkyl, $C_{2-6}$ alkenyl, $C_{2-6}$ alkynyl, $C_{3-10}$ cycloalkyl, 3-10 membered heterocycloalkyl, $C_{6-10}$ aryl, 5-10 membered heteroaryl, $C_{3-10}$ cycloalkyl $C_{1-6}$ alkyl, 3-10 membered heterocycloalkyl $C_{1-6}$ alkyl, phenyl $C_{1-6}$ alkyl or 5-10 membered heteroaryl $C_{1-6}$ alkyl; and the $R_a$, $R_b$, $R_d$, $R_e$ or $R_{e'}$ is unsubstituted or optionally substituted at any position with 1 to 3 substituents selected from -$L_4$-$OR_f$, -OC(O)-$L_4$-$R_f$, -$L_4$-$NR_fR_{f'}$, halogen, cyano, nitro, $C_{1-6}$ alkyl, halo $C_{1-6}$ alkyl and halo $C_{1-6}$ alkoxy;
$L_1$, $L_2$, $L_3$ and $L_4$ are each independently a linkage bond, $C_{1-6}$ alkylene, $C_{2-6}$ alkenylene, $C_{2-6}$ alkynylene or a polyethylene glycol fragment including 1 to 20 -$OCH_2CH_2$- units; and the $L_1$, $L_2$, $L_3$ or $L_4$ is unsubstituted or optionally substituted at any position with one or more substituents selected from oxo, hydroxyl, amino, halogen, cyano, alkyl, haloalkyl, alkoxy and haloalkoxy;
each $R_f$ and $R_{f'}$ is independently -$R_c$, -$NHR_c$ or $C_{1-6}$ alkyl;
$R_g$ is a prodrug group including phosphoryl; and
each $R_c$ is independently hydrogen or a linkage bond attached to L; and at least one $R_c$ in the D is a linkage bond attached to L;
and the benzazepine group of formula D satisfies 1 or 2 of conditions of:

(1) m is 1; and
(2) at least one of $R_4$ and $R_{4'}$ is substituted at any position with one or more substituents selected from halogen, cyano, -$L_2$-$OR_a$, -$L_2$-$OR_g$, -$L_2$-OC(O)$R_a$, -$L_2$-OC(O)$OR_a$, -$L_2$-OC(O)$NR_aR_b$, -$L_2$-$NR_aR_b$, -$L_2$-$NR_aC(O)OR_b$, -$L_2$-C(O)$OR_b$, -$L_2$-$NR_aC(O)NR_aR_b$ and -$L_2$-$NR_bC(NR_b)NR_aR_b$;

L is -M-$(T)_w$-PEG-$(T)_o$-*, -M-PEG-*, -M-$(T)_w$-*, -M-$(T)_w$-$(A)_v$-$(T)_o$-PEG-*, -M-$(T)_w$-$(A)_v$-$(T)_o$-*, - M-$(T)_w$-$L_5$-*, -M-$(T)_w$-PEG-$L_5$-*, -M-$(T)_w$-PEG-$(A)_v$-$L_5$-*, -M-$(T)_w$-PEG-$(A)_v$-$(T)_o$-*, -M-PEG-$(T)_o$-PEG-* or -M-$(T)_w$-$(A)_v$-$L_5$-*; v is any integer from 1 to 5; w is any integer from 1 to 10; o is any integer from 0 to 10; and * is a linking site of the L that is attached to the D;
each T is independently -$(CH_2)_x$-C(O)-, -$NR_h$-, -O-, -S-, -$(CH_2)_x$-arylene-$(CH_2)_y$-, -$(CH_2)_x$-heteroarylene-$(CH_2)_y$-, -$(CH_2)_x$-cycloalkylene-$(CH_2)_y$-, -$(CH_2)_x$-heterocycloalkylene-$(CH_2)_y$-, -$NR_h$-$(CH_2)_x$-C(O)-, -O-$(CH_2)_x$-C(O)-, -S-$(CH_2)_x$-C(O)-, -$(CH_2)_x$CH($NHR_h$)-C(O)-, -$(CH_2)_x$-S-S-alkylene-, alkylene or alkenylene; and x and y are each independently any integer from 0 to 10;
$R_h$ is hydrogen, alkyl, -$PO(OH)_2$, -$PO(OCH_3)_2$, -C(O)-$(CH_2CH_2O)_n$-$CH_3$ or -$(CH_2CH_2O)_n$-$CH_3$;
PEG is -$(CH_2CH_2O)_n$-$(CH_2)_u$- or -$(CH_2CH_2O)_n$-$(CH_2)_u$-C(O)-; n is any integer from 1 to 50; and u is any integer from 0 to 5;
each A is independently an amino acid residue or an amino acid residue modified by -C(O)-$(CH_2CH_2O)_n$-$CH_3$ or -$(CH_2CH_2O)_n$-$CH_3$;
$L_5$ is a self-immolative group; and
M is a linkage bond or a linker head attached to an antibody.

In some embodiments, in the antibody-immunostimulatory conjugate of formula I or the pharmaceutically acceptable salt thereof, some groups are defined as below, and the remaining groups are defined as any other solution (hereinafter referred to as "in some embodiments"):

$$Ab\text{—}(L\text{—}D)_t$$

(I)

where Ab is an antibody;
t is any numerical value from 1 to 8;
L is a linker attached to the Ab and the D; and
the D is a group formed by loss of one hydrogen atom in a compound of formula D';

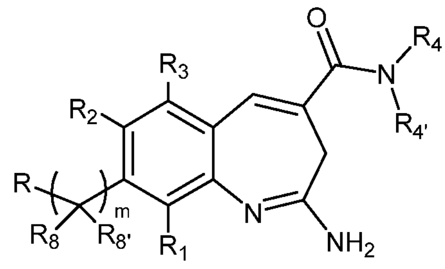

D'

where R is $-L'-L_1-R_7$; and L' is -C(=O)-, $-C(=O)-NR_9-$ or $-C(=S)-NR_9-$;

$R_9$ is independently hydrogen, $C_{1-6}$ alkyl or halo $C_{1-6}$ alkyl;

$L_1$ is independently a linkage bond, $C_{1-6}$ alkylene or $C_{2-6}$ alkenylene;

$R_7$ is independently phenyl, 5-6 membered heteroaryl, 3-8 membered heterocycloalkyl, an 8-12 membered fused ring group, phenyl substituted with 1, 2 or 3 $R_{7-1}$, 5-6 membered heteroaryl substituted with 1, 2 or 3 $R_{7-2}$, 3-8 membered heterocycloalkyl substituted with 1, 2 or 3 $R_{7-3}$ or an 8-12 membered fused ring group substituted with 1, 2 or 3 $R_{7-4}$;

in the $R_7$, in "5-6 membered heteroaryl" in the 5-6 membered heteroaryl and the 5-6 membered heteroaryl substituted with 1, 2 or 3 $R_{7-2}$, the heteroatom is independently selected from one or more of N, O and S, and the number of the heteroatom is independently 1, 2 or 3; and in "3-8 membered heterocycloalkyl" in the 3-8 membered heterocycloalkyl and the 3-8 membered heterocycloalkyl substituted with 1, 2 or 3 $R_{7-3}$, the heteroatom is independently selected from one or more of N, O and S, and the number of the heteroatom is independently 1, 2 or 3;

in the $R_7$, an 8-12 membered fused ring group in the 8-12 membered fused ring group and the 8-12 membered fused ring group substituted with 1, 2 or 3 $R_{7-4}$ is independently a fused ring of ring A and ring B, the ring A is a 5-6 membered heteroaryl ring or a phenyl ring, and the ring B is a 5-6 membered heteroalkenyl ring; in the 5-6 membered heteroaryl ring, the heteroatom is selected from one or more of N, O and S, and the number of the heteroatom is 1, 2 or 3; and in the 5-6 membered heteroalkenyl ring, the heteroatom is selected from one or more of N, O and S, and the number of the heteroatom is 1, 2 or 3;

$R_{7-1}$, $R_{7-2}$, $R_{7-3}$ and $R_{7-4}$ are independently amino, $-L_3-NH_2$, $-L_3-OH$, $-C(=O)-L_3-OH$ or $-C(=O)-L_3-NH_2$, and $L_3$ is independently $C_{1-6}$ alkylene or $-(CH_2CH_2O)_{m'}-C_{1-6}$ alkylene-; and m' is 1, 2, 3, 4, 5, 6, 7, 8, 9 or 10;

m is 0 or 1;

$R_8$ and $R_{8'}$ are independently hydrogen or $C_{1-6}$ alkyl, or $R_8$ and $R_{8'}$ together with the carbon atom to which they are attached form carbonyl, $C_{3-10}$ cycloalkylene or 3-10 membered heterocycloalkylene; and in the 3-10 membered heterocycloalkylene, the heteroatom is selected from one or more of N, O and S, and the number of the heteroatom is 1, 2 or 3;

$R_1$, $R_2$ and $R_3$ are independently hydrogen, deuterium or halogen;

$R_4$ and $R_{4'}$ are independently $C_{1-6}$ alkyl, $C_{1-6}$ alkoxy or $C_{1-6}$ alkyl substituted with 1, 2 or 3 $R_{4-1}$; and the $R_{4-1}$ is independently hydroxyl, amino, $-OC(=O)NR_aR_b$ or

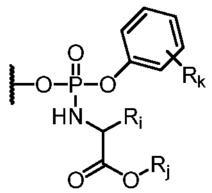

;

$R_a$ and $R_b$ are independently hydrogen, $C_{1-6}$ alkyl or $C_{3-10}$ cycloalkyl;

$R_i$ is hydrogen, $C_{1-6}$ alkyl or benzyl; $R_k$ is hydrogen, halogen or $C_{1-6}$ alkyl; and $R_j$ is $C_{1-6}$ alkyl, $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; and

in the $R_j$, in "3-10 membered heterocycloalkyl" in the 3-10 membered heterocycloalkyl and the 3-10 membered heterocycloalkyl-$C_{1-6}$ alkyl, the heteroatom is independently selected from one or more of N, O and S, and the number of the heteroatom is independently 1, 2 or 3; and in "5-10 membered heteroaryl" in the 5-10 membered heteroaryl and the 5-10 membered heteroaryl-$C_{1-6}$ alkyl, the heteroatom is independently selected from one or more of N, O and S, and the number of the heteroatom is independently 1, 2 or 3;

and the compound of formula D' satisfies 1 or 2 of conditions of:

(1) m is 1; and

(2) at least one of $R_4$ and $R_{4'}$ is $C_{1-6}$ alkoxy or $C_{1-6}$ alkyl substituted with 1, 2 or 3 $R_{4-1}$;

and the antibody-immunostimulatory conjugate of formula I is not an antibody-immunostimulatory conjugate shown in Table 1, where mAb is Trastuzumab:

Table 1

| Structure | t |
|---|---|
| 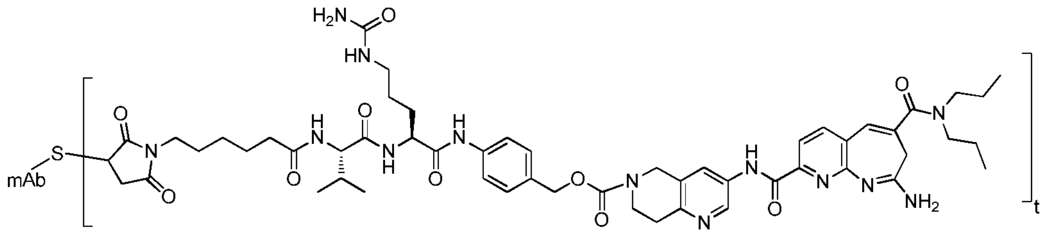 | 4.27 |
| 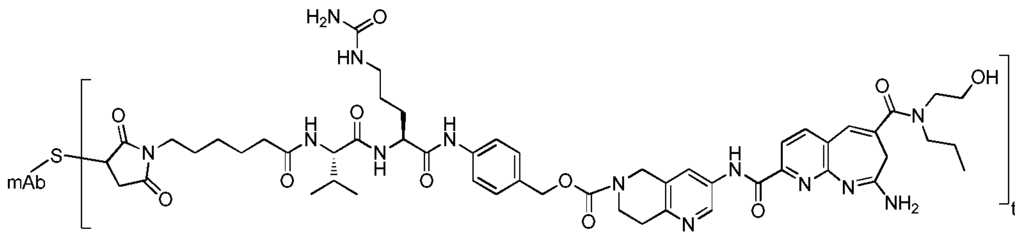 | 4.39 |
| 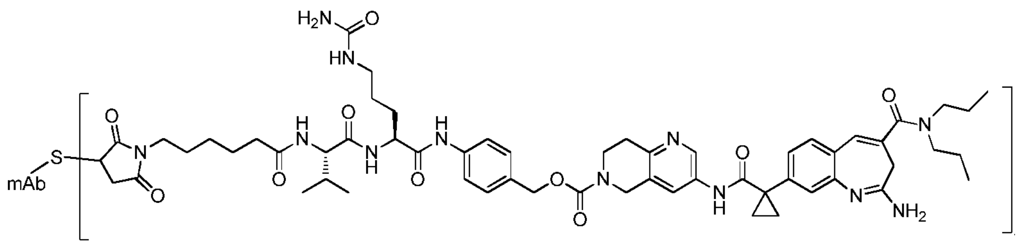 | 4.14 |
| 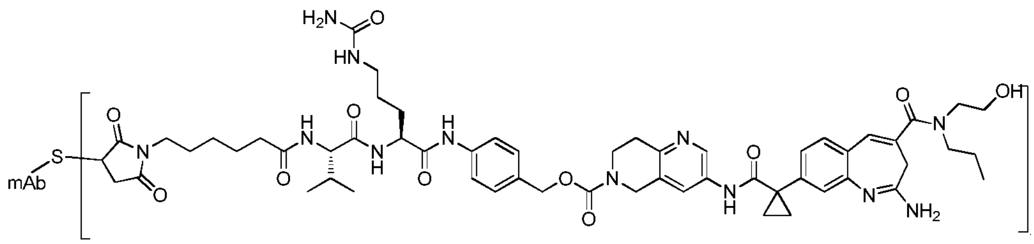 | 4.57 |
| 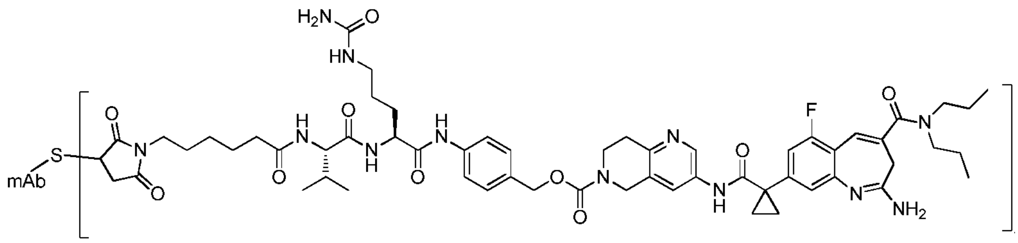 | 4.14 |
| 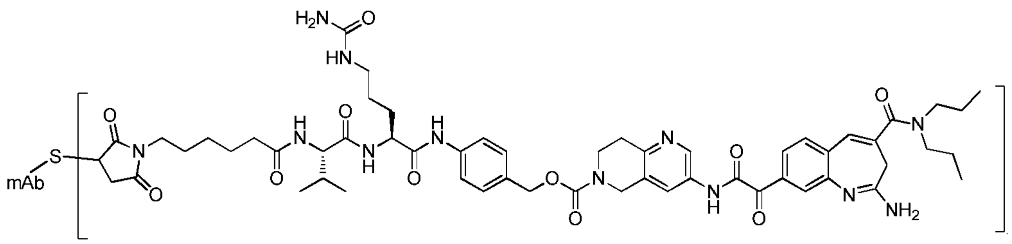 | 4.23 |

(continued)

| Structure | t |
|---|---|
| 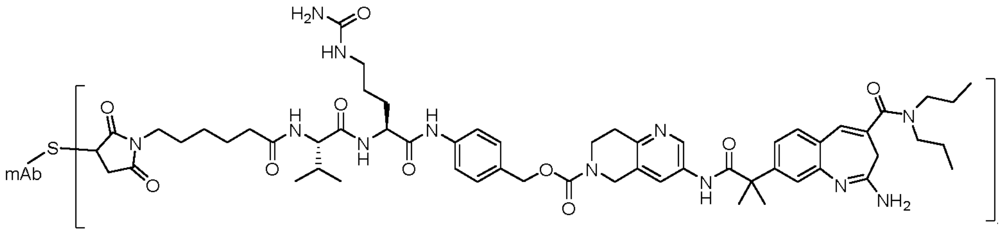 | 4.29 |
| 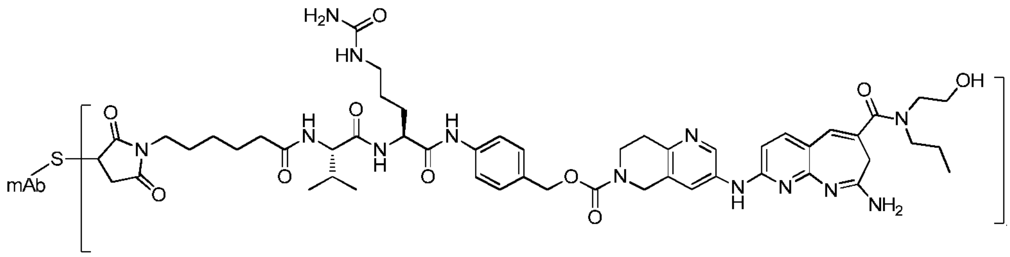 | 4.04 |
| 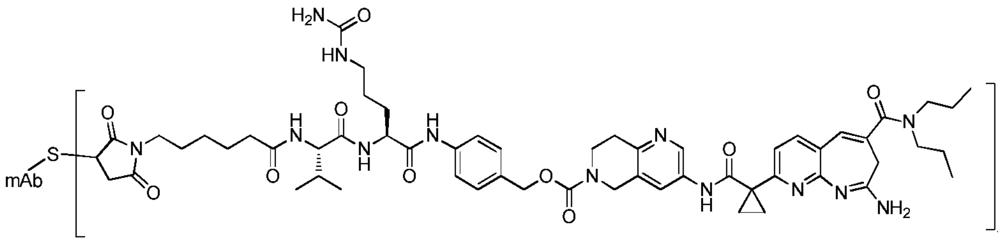 | 4.16 |
| 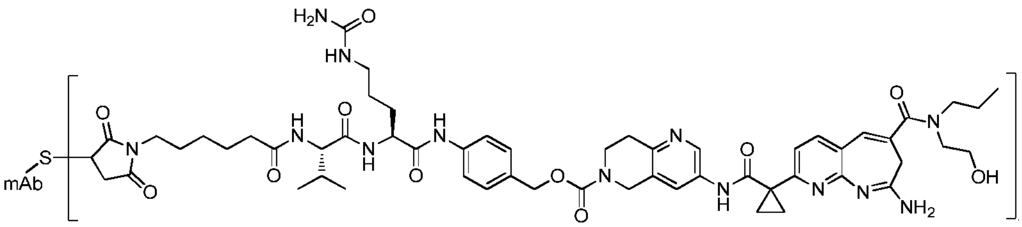 | 4.28 |
| 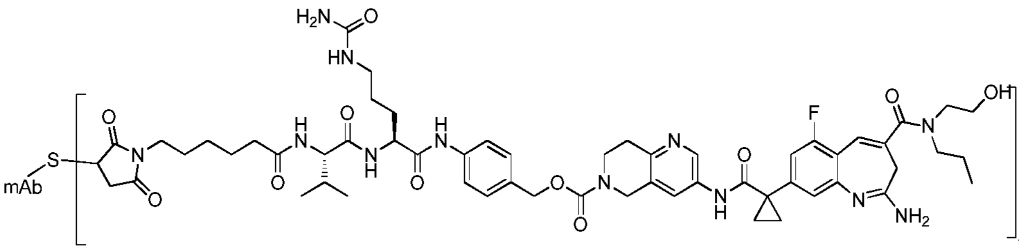 | 3.93 |
| 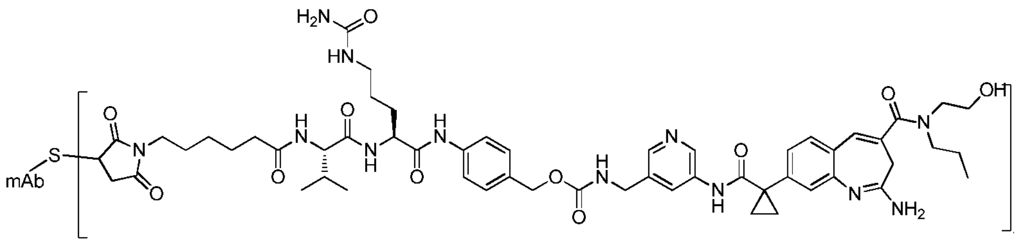 | 4.72 |

(continued)

| Structure | t |
|---|---|
| 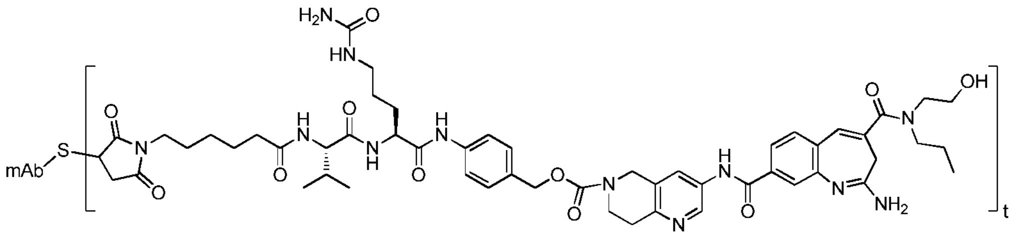 | 4.61 |
| 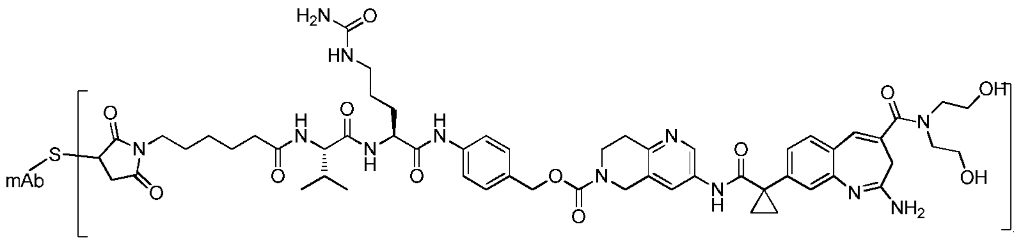 | 4.45 |
| 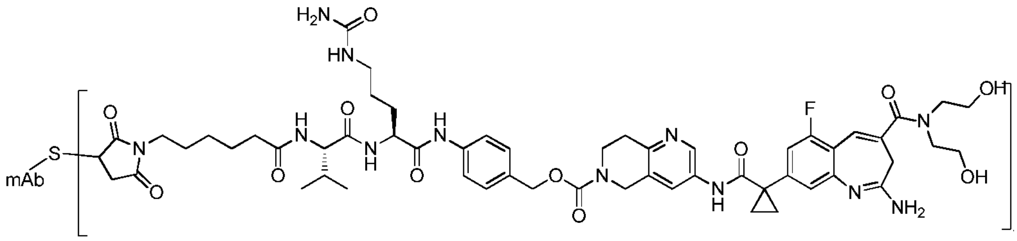 | 4.39 |
| 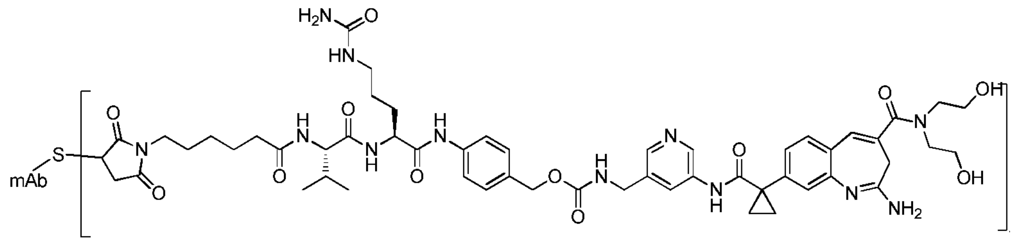 | 4.35 |
| 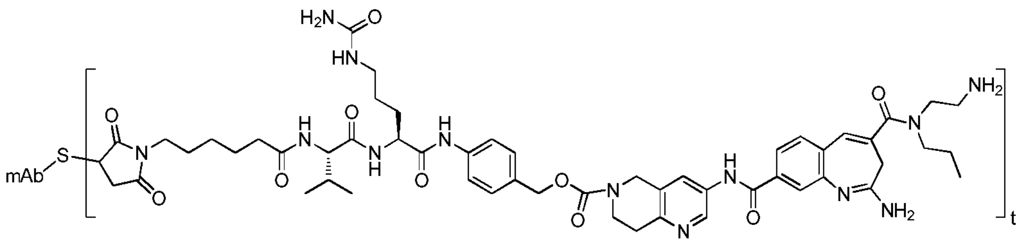 | 3.21 |
| 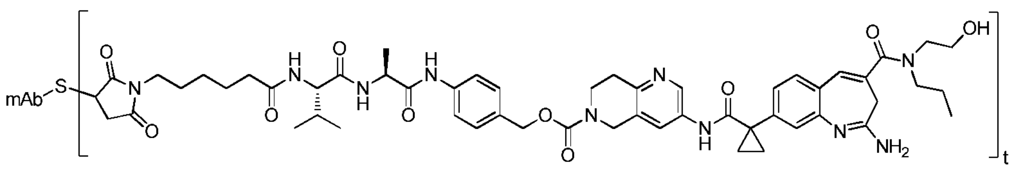 | 4.03 |
| 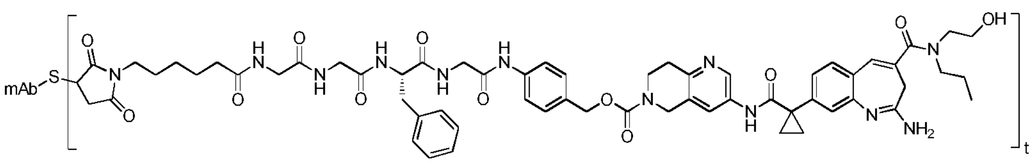 | 3.86 |

(continued)

| Structure | t |
|---|---|
| 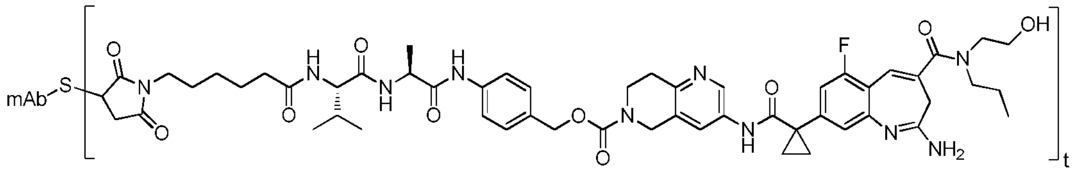 | 3.98 |
| 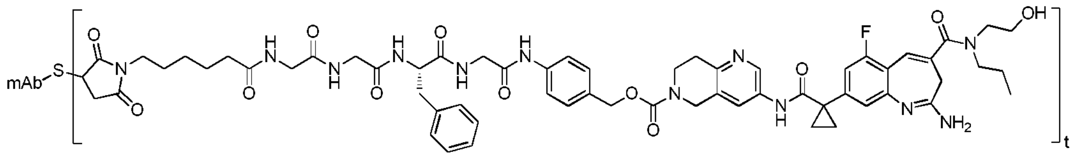 | 4.03 |
| 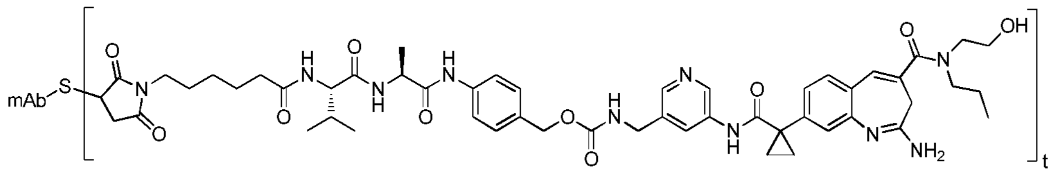 | 4.12 |
| 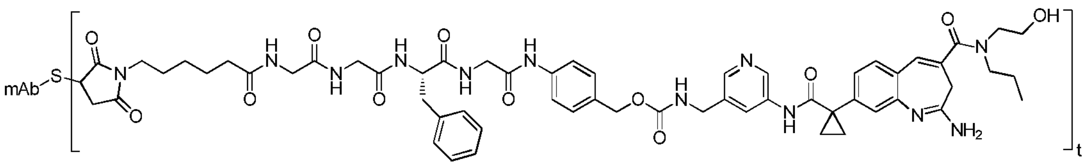 | 4.06 |

In some embodiments, the antibody-immunostimulatory conjugate of formula I is not an antibody-immunostimulatory conjugate shown in Table 2, where mAb is a monoclonal antibody, and t is any numerical value from 1 to 8:

Table 2

| |
|---|
| 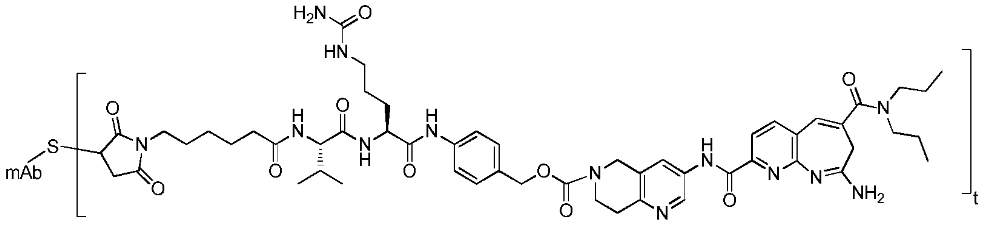 |
| 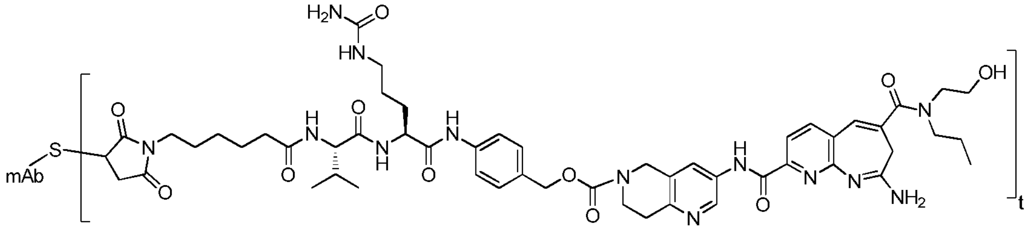 |
| 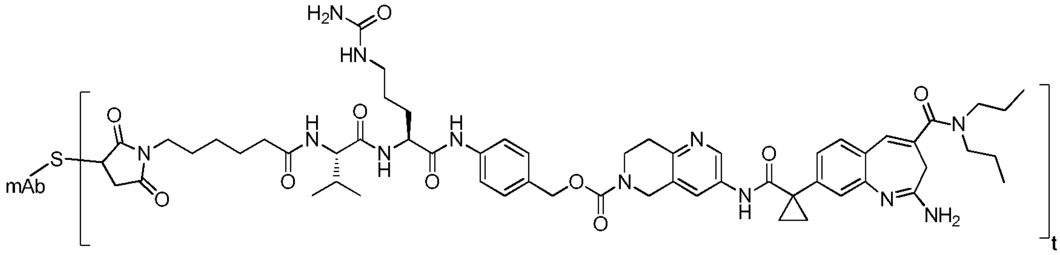 |

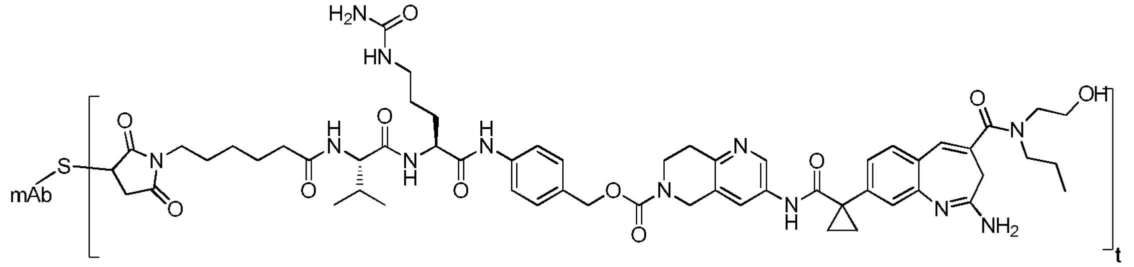

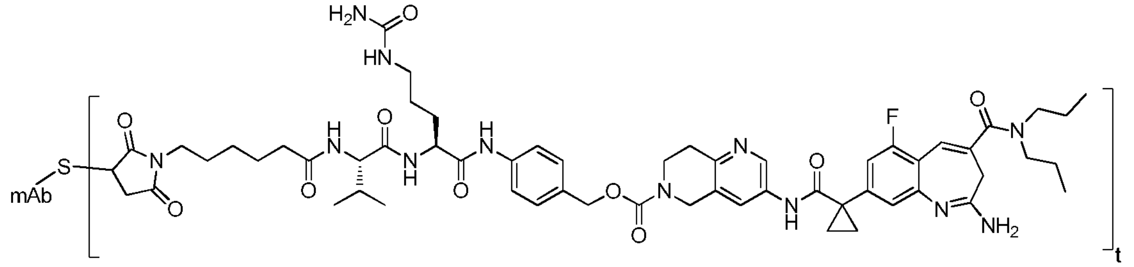

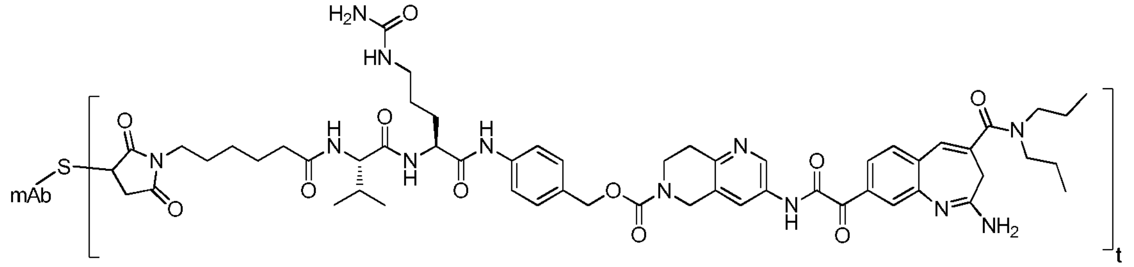

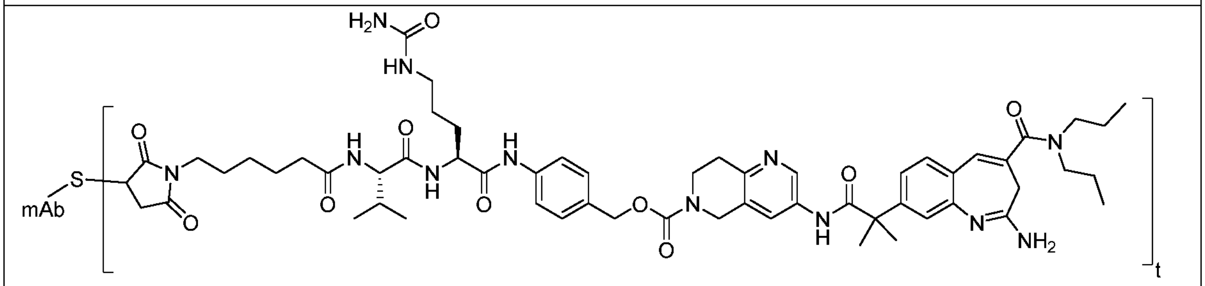

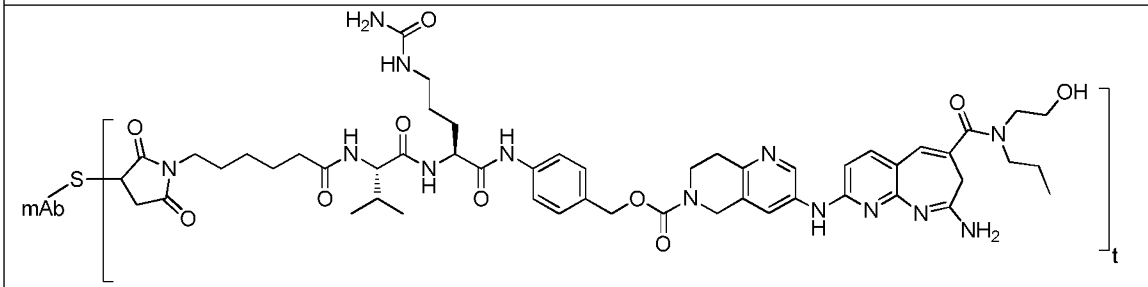

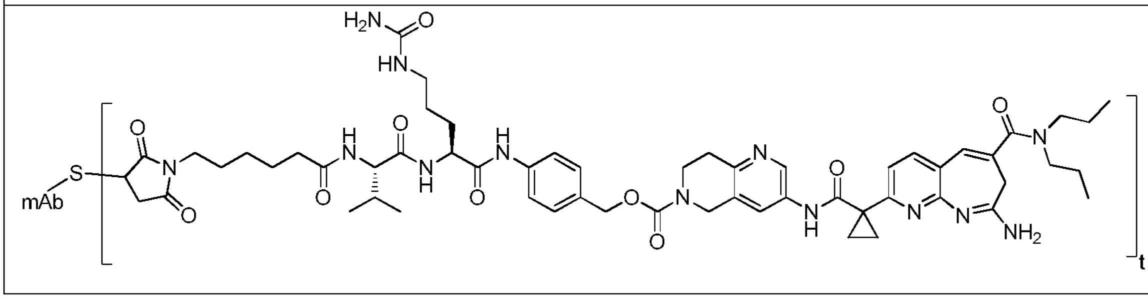

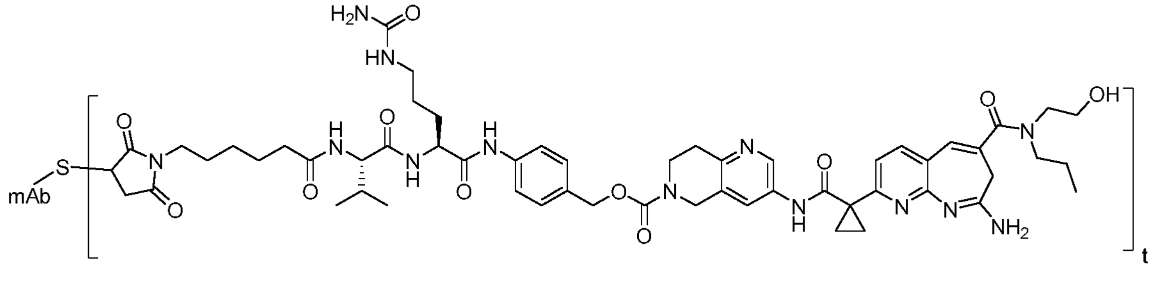

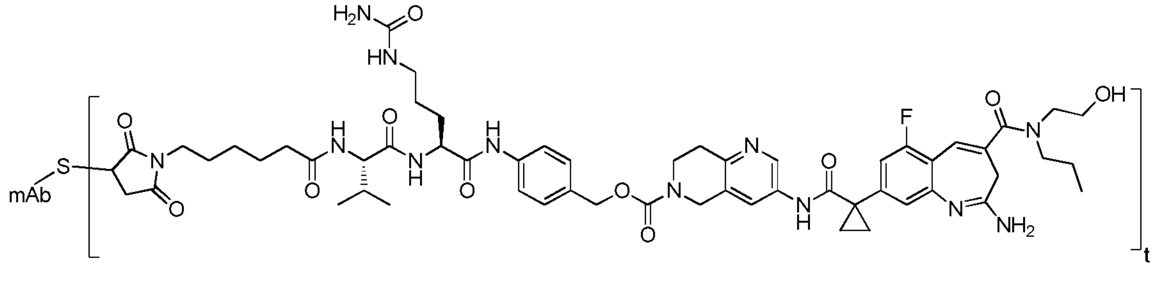

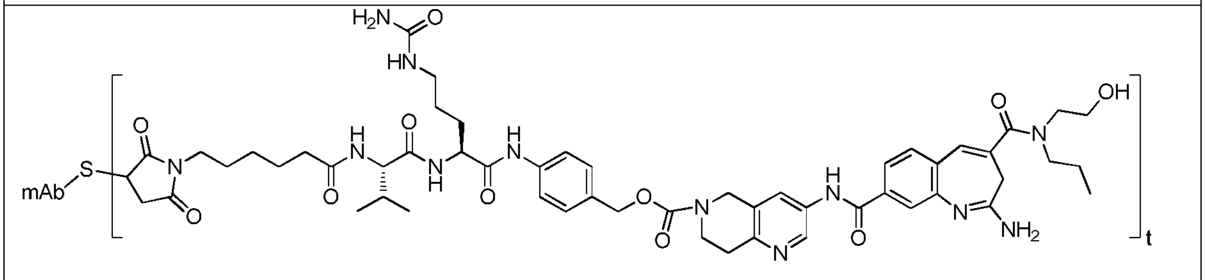

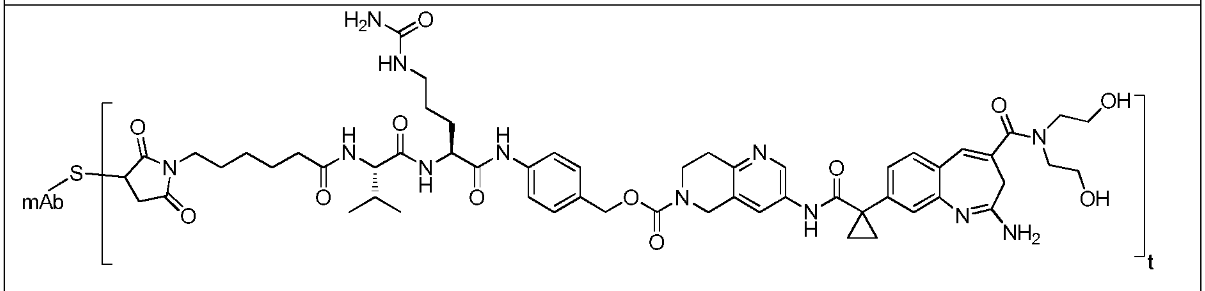

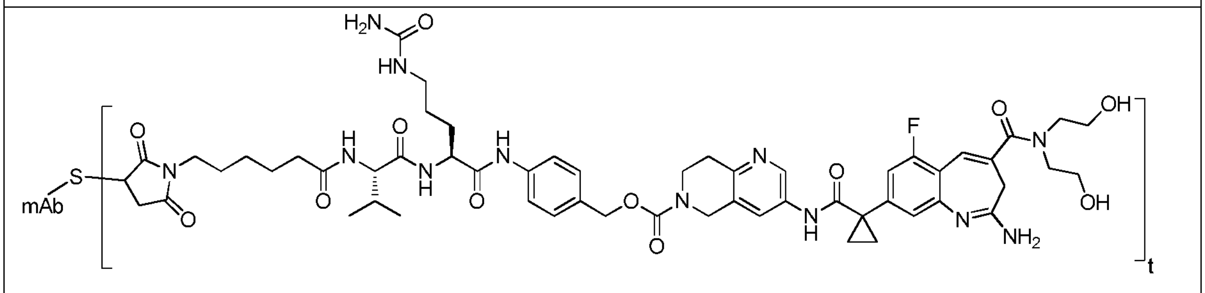

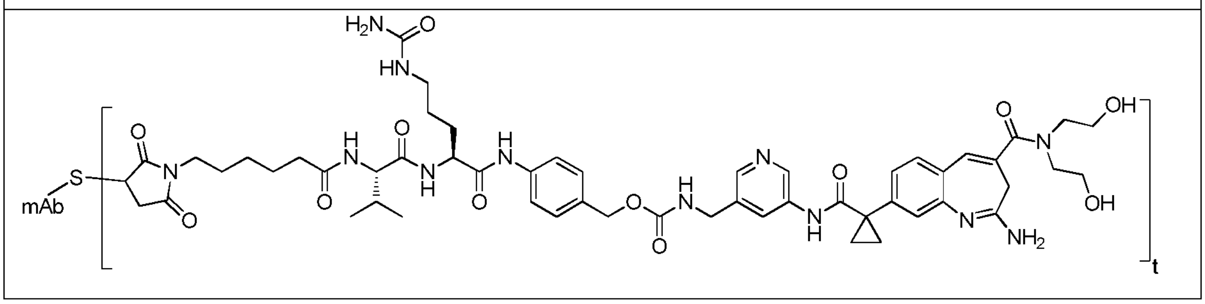

| |
|---|
| 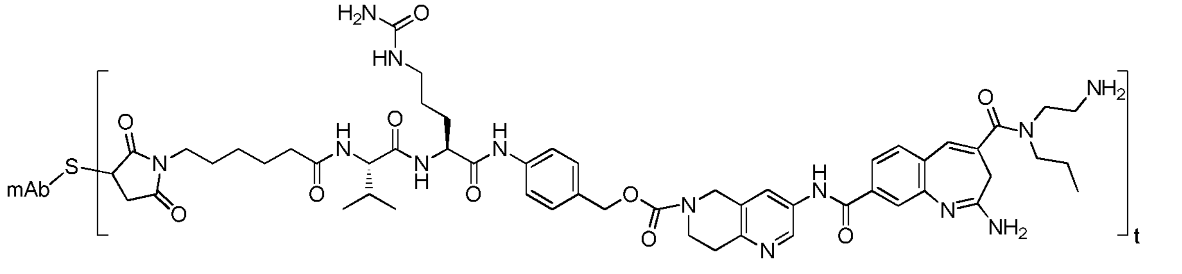 |
| 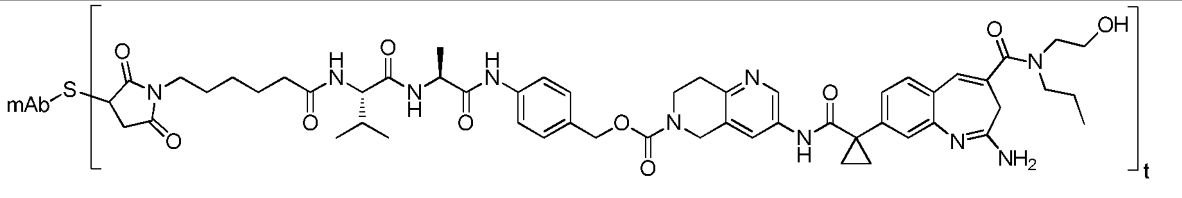 |
| 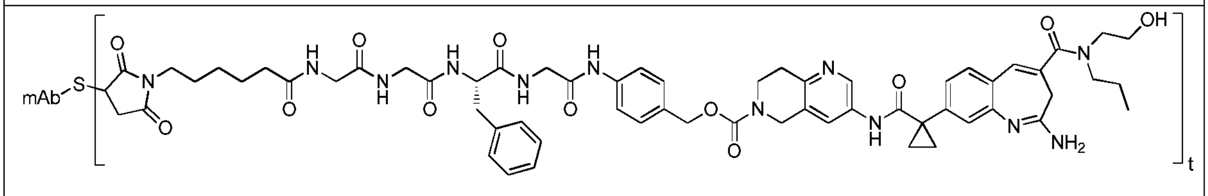 |
| 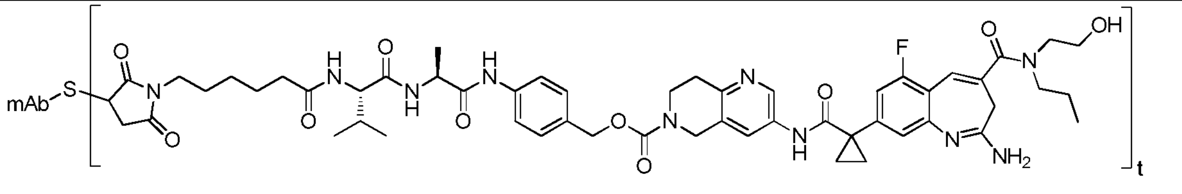 |
| 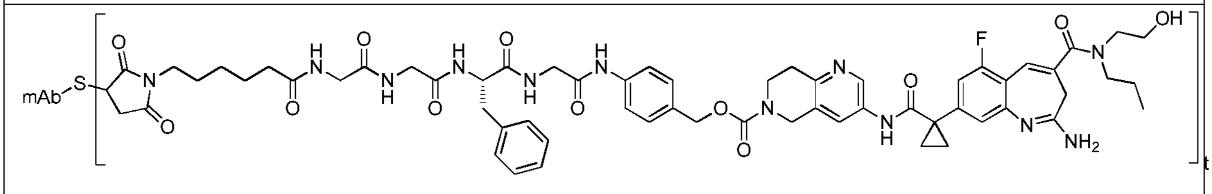 |
| 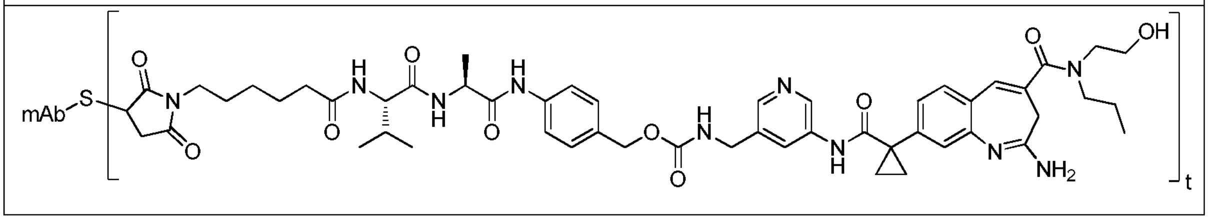 |

In some embodiments, the compound of formula D' is not a compound shown in Table 3:

**[0062]** Table 3

| | |
|---|---|
| 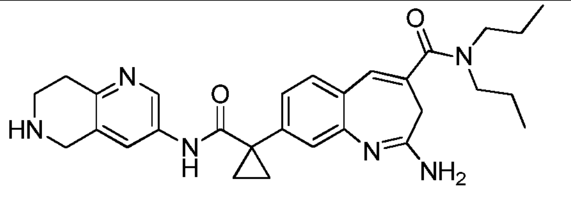 | 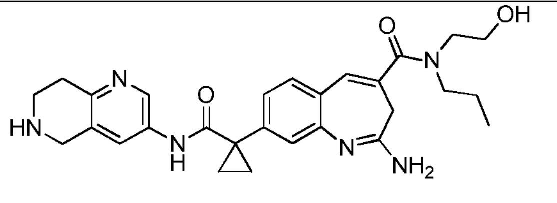 |
| 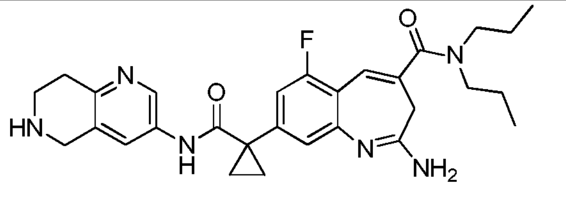 | 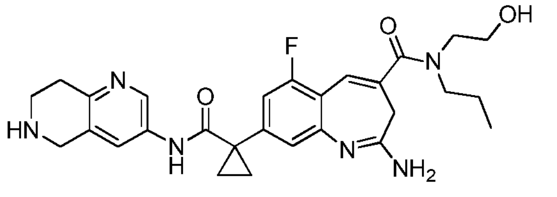 |

| | |
|---|---|
| 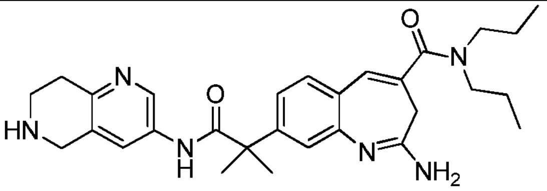 | 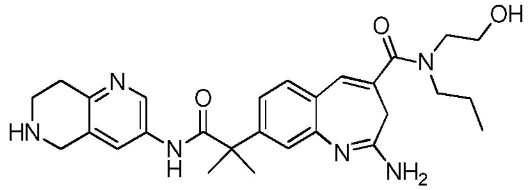 |
| 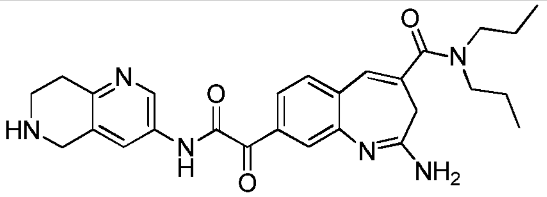 | 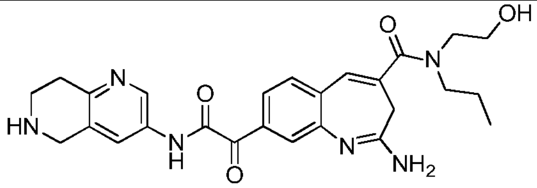 |
| 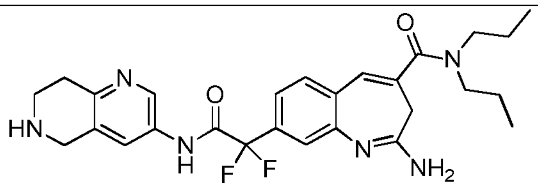 | 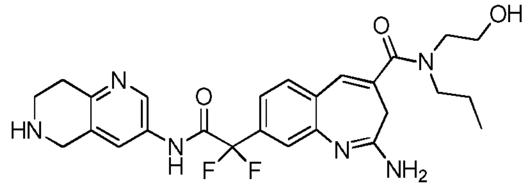 |
| 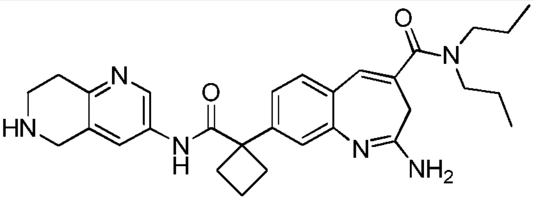 | 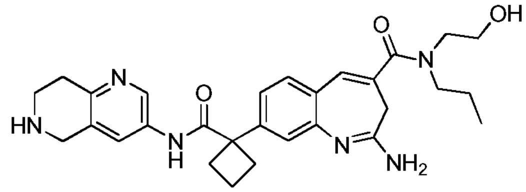 |
| 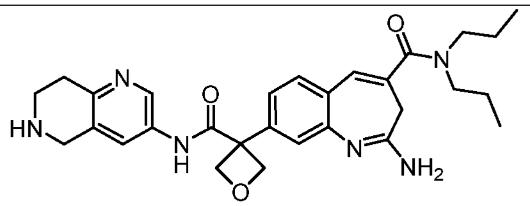 | 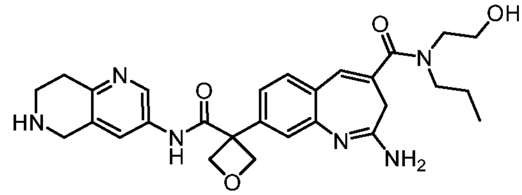 |
| 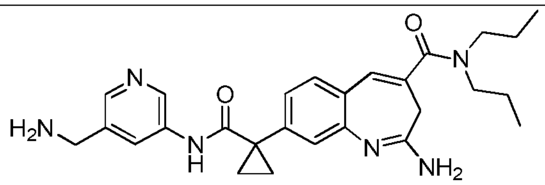 | 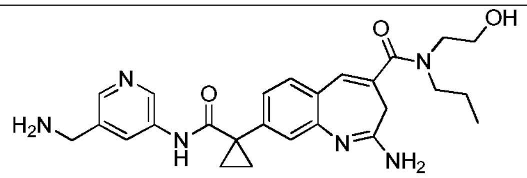 |
| 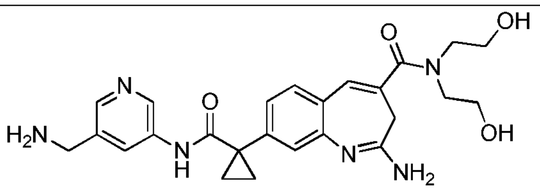 | 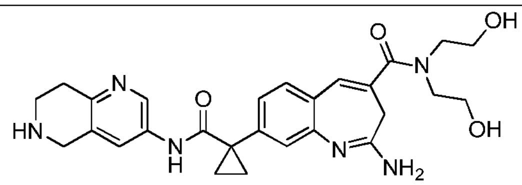 |
| 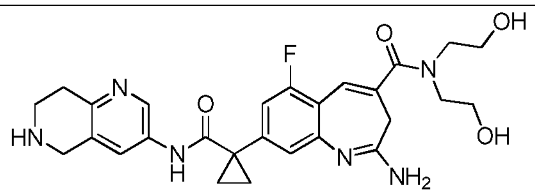 | 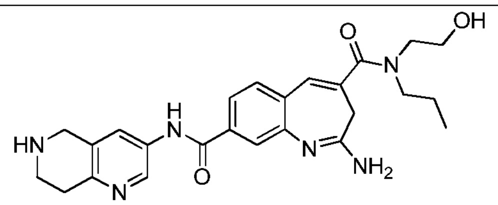 |

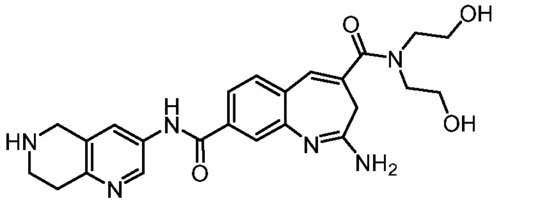

In some embodiments, the compound of formula D' satisfies 1, 2 or 3 of conditions of:

(1) $L_1$ is independently $C_{1-6}$ alkylene or $C_{2-6}$ alkenylene;
(2) $R_7$ is independently phenyl substituted with 1 amino, 5-6 membered heteroaryl substituted with one amino, 3-8 membered heterocycloalkyl substituted with one amino or an 8-12 membered fused ring group substituted with one amino; and
(3) at least one of $R_4$ and $R_{4'}$ is $C_{1-6}$ alkoxy or $C_{1-6}$ alkyl substituted with 1, 2 or 3 $R_{4-1}$; and the $R_{4-1}$ is independently $-OC(=O)NR_aR_b$ or

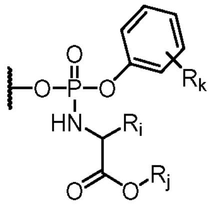
.

In some embodiments, the linker is not of any one of structures shown in Table 4:

**[0065]** Table 4

| | |
|---|---|
| 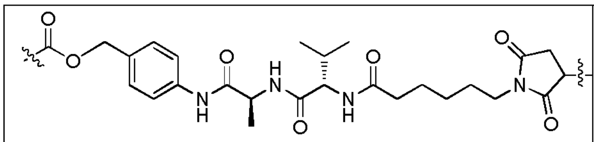 | 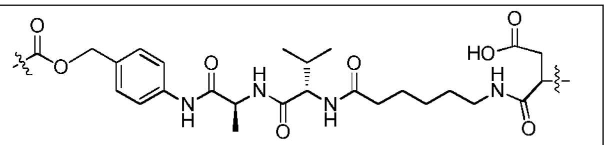 |
| 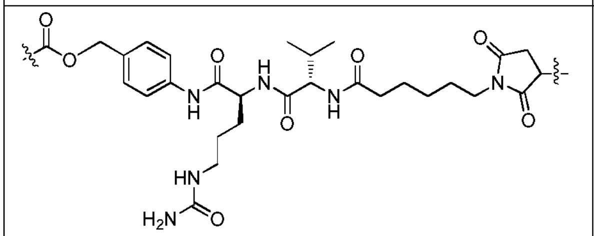 | 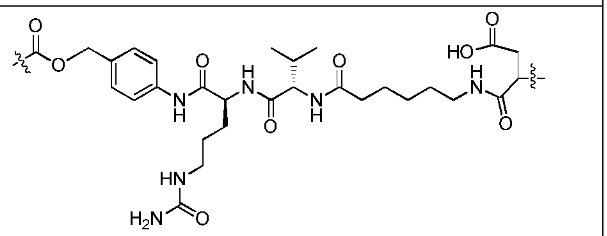 |
| 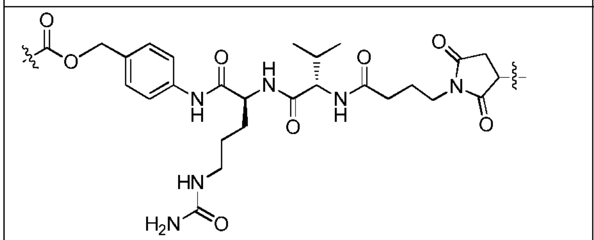 | 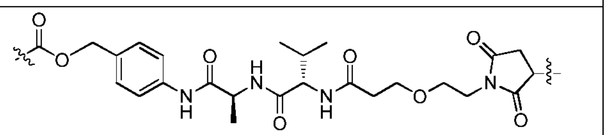 |
| 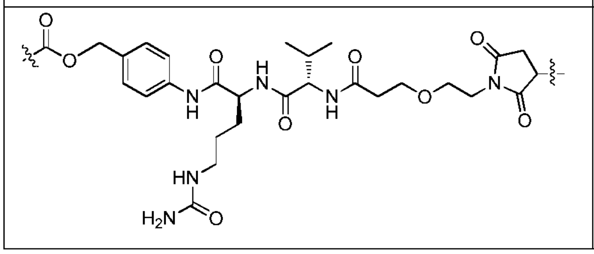 | 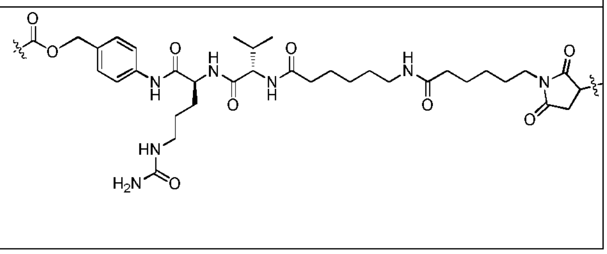 |

| | |
|---|---|
| 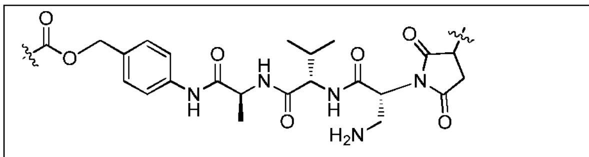 | 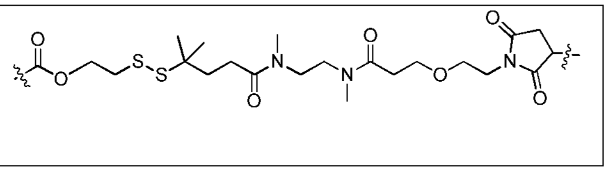 |
| 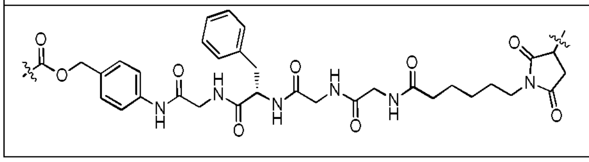 | |

In some embodiments, the L is $-M-(T)_w-PEG-(T)_o-^*$, -M-PEG-*, $-M-(T)_w-(A)_v-(T)_o-PEG-^*$, $-M-(T)_w-(A)_v-(T)_o-^*$, $-M-(T)_w-L_5-^*$, $-M-(T)_w-PEG-L_5-^*$, $-M-(T)_w-PEG-(A)_v-L_5-^*$, $-M-(T)_w-PEG-(A)_v-(T)_o-^*$ or - $M-PEG-(T)_o-PEG-^*$; and * is a linking site of the L that is attached to the D;

M is a linkage bond or a linker head attached to Ab;
w is independently 1, 2, 3, 4, 5, 6, 7, 8, 9 or 10;
o is independently 0, 1, 2, 3, 4, 5, 6, 7, 8, 9 or 10;
T is independently $-(CH_2)_x-C(O)-$, $-NR_h-$, -O-, -S-, $-(CH_2)_x-C_{6-14}$ arylene-$(CH_2)_y$-, $-(CH_2)_x$-5-6 membered heteroarylene-$(CH_2)_y$-, $-(CH_2)_x-C_{3-6}$ cycloalkylene-$(CH_2)_y$-, $-(CH_2)_x$-3-6 membered heterocycloalkylene-$(CH_2)_y$-, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x-S-S-C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the $-(CH_2)_x$-5-6 membered heteroarylene-$(CH_2)_y$-, the heteroatom is selected from one or more of N, O and S, and the number of the heteroatom is 1, 2 or 3; and in "3-6 membered heterocycloalkylene" in the $-(CH_2)_x$-3-6 membered heterocycloalkylene-$(CH_2)_y$-, the heteroatom is selected from one or more of N, O and S, and the number of the heteroatom is 1, 2 or 3;
$R_h$ is independently hydrogen, $C_{1-6}$ alkyl, $-PO(OH)_2$, $-PO(OCH_3)_2$, $-C(O)-(CH_2CH_2O)_n-CH_3$ or - $(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;
PEG is $-(CH_2CH_2O)_n-(CH_2)_u-$ or $-(CH_2CH_2O)_n-(CH_2)_u-C(O)-$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;
v is independently 1, 2, 3, 4 or 5;
A is independently an amino acid residue or an amino acid residue substituted with 1 $R_A$; the $R_A$ is independently $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and
$L_5$ is a self-immolative group.

In some embodiments, the antibody can include one or more antigen-binding domains that can bind to an antigen.

In some embodiments, the antibody may include only one or two antigen-binding domains that can bind to an antigen.

In some embodiments, the antibody may include one antigen-binding domain that can bind to an antigen.

In some embodiments, the antibody may include only one antigen-binding domain that can bind to an antigen.

In some embodiments, the antibody may include one Fc-region.

In some embodiments, the antibody may include only one Fc-region.

In some embodiments, the antibody may include only one antigen-binding domain that can bind to an antigen and one Fc-region.

In some embodiments, the antibody may be a monoclonal antibody.

In some embodiments, the antibody may be an anti-HER2 antibody.

In some embodiments, the anti-HER2 monoclonal antibody includes, but is not limited to: Trastuzumab, Trastuzumab biosimilar, Pertuzumab, Pertuzumab biosimilar, Margetuximab, HT-19, etc.

In some embodiments, the antibody may be an anti-5T4 antibody.

In some embodiments, the anti-5T4 monoclonal antibody includes, but is not limited to: huA1.

In some embodiments, the antibody may be an anti-Claudin18.2 antibody.

In some embodiments, the antibody may be an anti-EGFR antibody.

In some embodiments, the antibody may be an anti-CEACAM5 antibody.

In some embodiments, the antibody may be an anti-Nectin-4 antibody.

In some embodiments, the antibody may be an anti-Trop-2 antibody.

In some embodiments, the antibody is Trastuzumab, Pertuzumab, Margetuximab or HT-19.

In some embodiments, the antibody is Cetuximab, huA1, Sacituzumab, Zolbetuximab, Labetuzumab or Enfortumab.

In the antibody-immunostimulatory conjugate of formula I or a pharmaceutically acceptable salt thereof, t is an integer or a non-integer, and when it is a non-integer, it means that the antibody-immunostimulatory conjugate of formula I is a mixture of antibody-immunostimulatory conjugates with different conjugation ratios; and when it is an integer, it may mean that the antibody-immunostimulatory conjugate of formula I is a single antibody-immunostimulatory conjugate with a fixed conjugation ratio, or it may mean that the antibody-immunostimulatory conjugate of formula I is a mixture of antibody-immunostimulatory conjugates with different conjugation ratios.

In some embodiments, t may be any numerical value from 2 to 5.

In some embodiments, t may be any numerical value from 3 to 5.

In the antibody-immunostimulatory conjugate of formula I or the pharmaceutically acceptable salt thereof, a left end or a right end of L' is attached to $L_1$.

In some embodiments, L' is -C(=O)-, -C(=O)-$NR_9$- or -C(=S)-$NR_9$-, and a right end thereof is attached to $L_1$.

In some embodiments, R is -C(O)-$NR_9$-$L_1$-$R_7$.

In some embodiments, $R_9$ is hydrogen, $C_{1-6}$ alkyl, halo $C_{1-6}$ alkyl, -$L_2$-$OR_a$ or -$L_2$-$NR_aR_b$.

In some embodiments, $R_9$ is hydrogen.

In some embodiments, in the $L_1$, the $C_{1-6}$ alkylene is methylene or ethylene.

In some embodiments, $L_1$ is a linkage bond or $C_{1-6}$ alkylene; and the $L_1$ is unsubstituted or optionally substituted at any position with 1 to 3 substituents selected from oxo, halogen, hydroxyl, amino, cyano, $C_{1-6}$ alkyl, halogenated $C_{1-6}$ alkyl, $C_{1-6}$ alkoxy or halogenated $C_{1-6}$ alkoxy.

In some embodiments, $L_1$ is a linkage bond or -$CH_2$-.

In some embodiments, $R_7$ is phenyl, 5-6 membered heteroaryl, 3-8 membered heterocycloalkyl or an 8-12 membered fused ring group; and the $R_7$ is unsubstituted or optionally substituted at any position with one or more substituents selected from -$L_3$-W, -$R_c$, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, $R_7$ is an 8-12 membered fused ring group; and the $R_7$ is unsubstituted or optionally substituted at any position with one or more substituents selected from -$L_3$-W, -$R_c$, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, in the $R_7$, the 8-12 membered fused ring group may be a fused ring of ring A and ring B, the ring A is 5-6 membered heteroaryl or phenyl, and the ring B is a 5-6 membered heteroalkenyl ring; in the 5-6-membered heteroaryl, the heteroatom is selected from one or more of N, O and S, and the number of the heteroatom is 1, 2 or 3; and in the 5-6-membered heteroalkenyl ring, the heteroatom is selected from one or more of N, O and S, and the number of the heteroatom is 1, 2 or 3.

In some embodiments, in the $R_7$, the 8-12 membered fused ring group may be a fused ring of ring A and ring B, the ring A is 5-6 membered heteroaryl or phenyl, and the ring B is a 5-6 membered heteroalkenyl ring attached to the $L_1$ through the ring A; in the 5-6 membered heteroaryl, the heteroatom is N, and the number of the heteroatom is 1 or 2; and in the 5-6 membered heteroalkenyl ring, the heteroatom is N, and the number of the heteroatom is 1 or 2.

In some embodiments, in the $R_7$, in the 5-6 membered heteroaryl, the heteroatom may be selected from one or more of N, O and S, and the number of the heteroatom may be 1, 2 or 3.

In some embodiments, in the $R_7$, in the 5-6 membered heteroaryl, the heteroatom may be N, and the number of the heteroatom may be 1 or 2.

In some embodiments, in the $R_7$, the 5-6 membered heteroaryl may be pyridinyl, pyrazinyl or pyridazinyl, and may be pyridin-3-yl, pyrazin-2-yl or pyridazin-3-yl.

In some embodiments, $R_7$ is

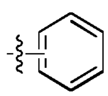, 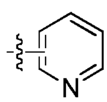, 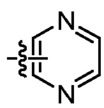, 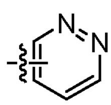, 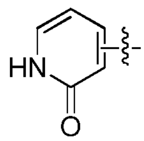,

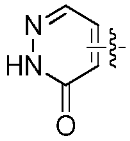, 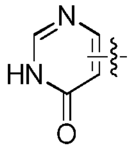, 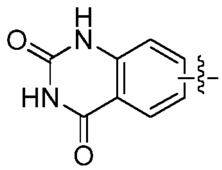, 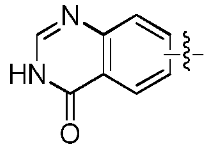, 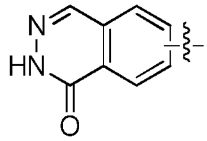, 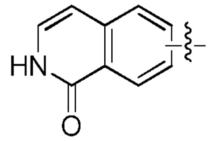,

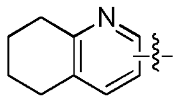, 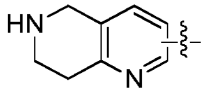, 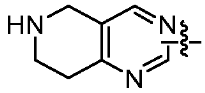, 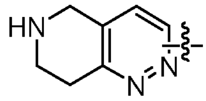, 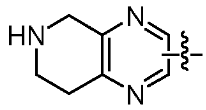,

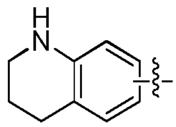, 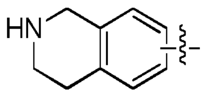, 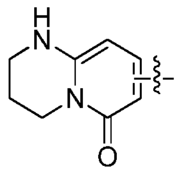, 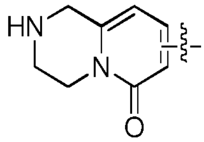 or 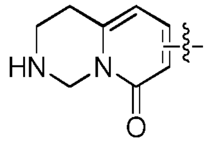;

and the $R_7$ is unsubstituted or optionally substituted at any position with 1 to 3 substituents selected from $-L_3$-W, $-R_c$, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, in the D, $R_7$ is

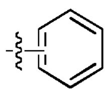, 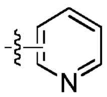, 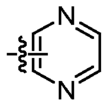, 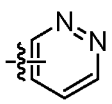, 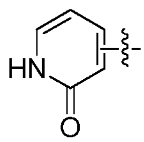,

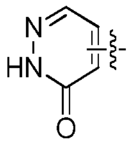, 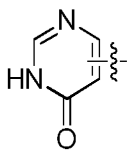, 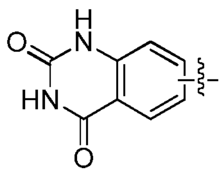, 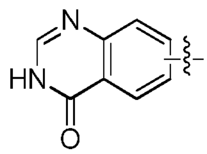, 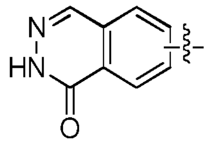, 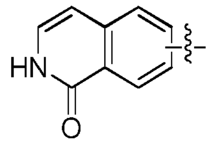,

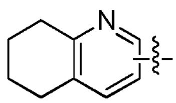, 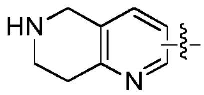, 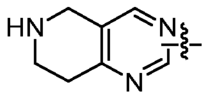, 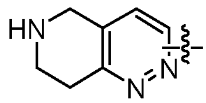, 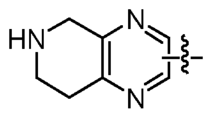,

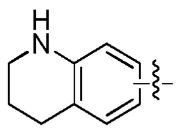, 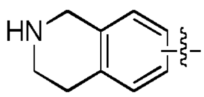, 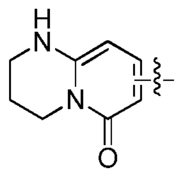, 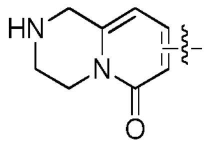 or 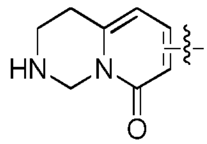;

and the $R_7$ is unsubstituted or optionally substituted at any position with 1 to 3 substituents selected from $-L_3$-W, $-R_c$, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, $R_7$ is

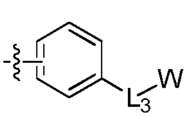, 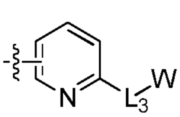, 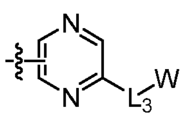,

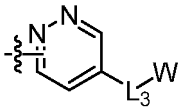, 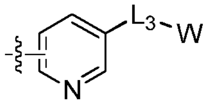, 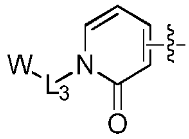, 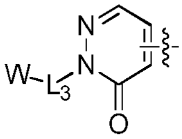, 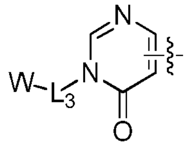,

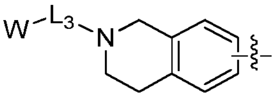, 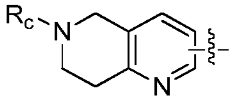, 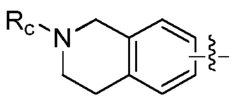, 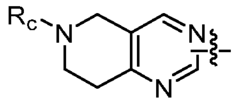,

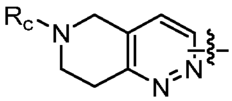, 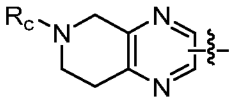, 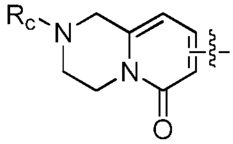, 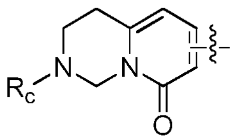,

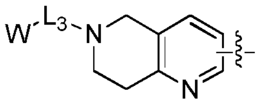 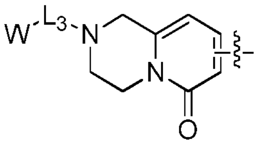 or 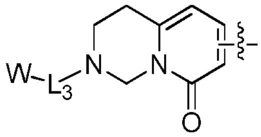.

In some embodiments, R is -C(O)-$NR_9$-$L_1$-$R_7$ or -C(S)-$NR_9$-$L_1$-$R_7$; $R_9$ is hydrogen, $L_1$ is a linkage bond, and $R_7$ is an unsubstituted 8-12 membered fused ring group, an 8-12 membered fused ring group substituted with one -$L_3$-W, or 5-6 membered heteroaryl substituted with one -$L_3$-W; $L_3$ is a linkage bond or $C_{1-6}$ alkylene, W is -$NR_dR_e$; and $R_d$ is H, and $R_e$ is -$R_c$.

In some embodiments, $R_7$ is

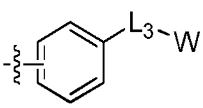, 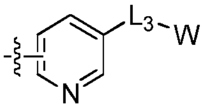, 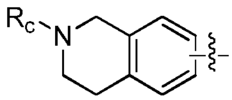

or

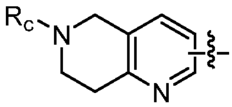.

In some embodiments, $R_7$ is

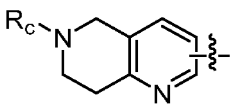

and may also be

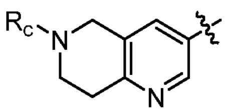.

In some embodiments, $R_7$ is

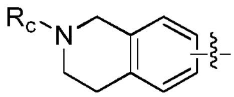

and may also be

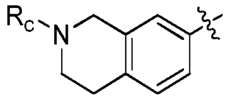

.

In some embodiments, in the $R_7$, the 5-6 membered heteroaryl substituted with one $-L_3$-W may be

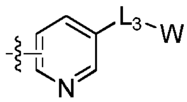

,

may be

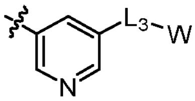

,

and may be

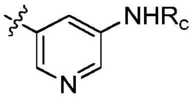

or

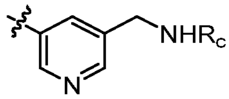

.

In some embodiments, in the $R_7$, the 5-6 membered heteroaryl substituted with one $-L_3$-W may be

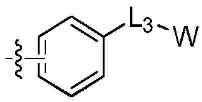

,

may be

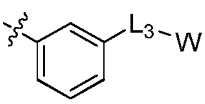

or

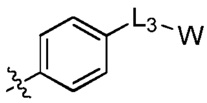

,

and may be

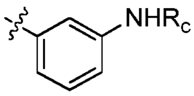

,

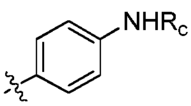

,

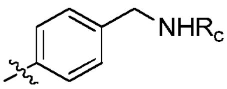

or

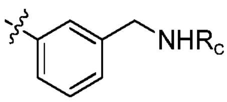

.

In some embodiments, the $R_7$ is

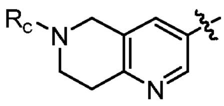,
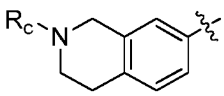,
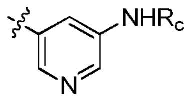,

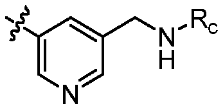,
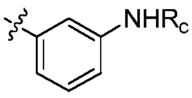,
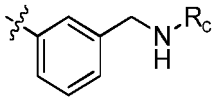
or
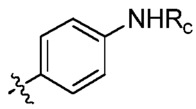,

and $R_c$ is a linkage bond attached to L.

In some embodiments, in the $R_7$, in "5-6 membered heteroaryl" in the 5-6 membered heteroaryl and the 5-6 membered heteroaryl substituted with 1, 2 or 3 $R_{7-2}$, the heteroatom is independently N.

In some embodiments, in the $R_7$, in "5-6 membered heteroaryl" in the 5-6 membered heteroaryl and the 5-6 membered heteroaryl substituted with 1, 2 or 3 $R_{7-2}$, the number of the heteroatom is independently 1 or 2.

In some embodiments, in the $R_7$, in "5-6 membered heteroaryl" of the 5-6 membered heteroaryl and the 5-6 membered heteroaryl substituted with 1, 2 or 3 $R_{7-2}$, the 5-6 membered heteroaryl may be pyridinyl, pyrimidinyl, pyridazinyl or pyrazinyl.

In some embodiments, in the ring A, the heteroatom in the 5-6 membered heteroaryl ring is N.

In some embodiments, in the ring A, the number of the heteroatom in the 5-6 membered heteroaryl ring is 1 or 2.

In some embodiments, in the ring A, the 5-6 membered heteroaryl ring may be a pyridine ring, a pyrimidine ring, a pyridazine ring or a pyrazine ring.

In some embodiments, in the ring B, the heteroatom in the 5-6 membered heteroalkenyl ring is N.

In some embodiments, in the ring B, the number of the heteroatom in the 5-6 membered heteroalkenyl ring is 1 or 2.

In some embodiments, the $R_7$ is

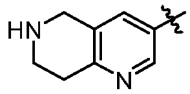,
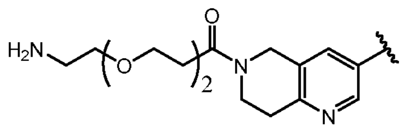,

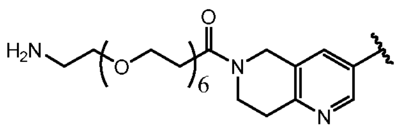,
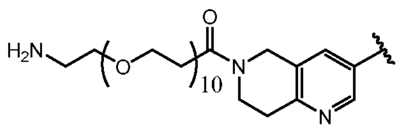,
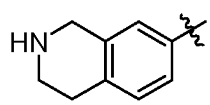,

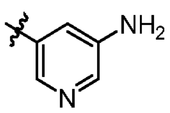,
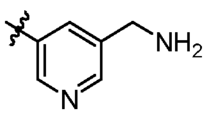,
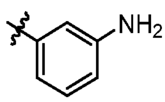,
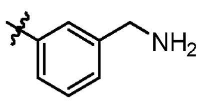
or
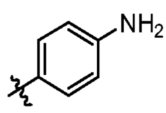.

In some embodiments, one hydrogen atom lost by the compound of formula D' is located on a secondary amine or a primary amine of $R_7$.

In some embodiments, the $R_7$ is

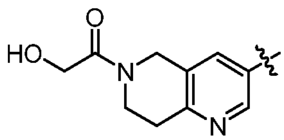
or
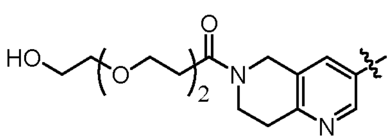.

In some embodiments, one hydrogen atom lost by the compound of formula D' is located on hydroxyl of $R_7$.

In some embodiments, m is 1.

In some embodiments, $R_8$ and $R_{8'}$ are each independently hydrogen, halogen or $C_{1-6}$ alkyl; and the $C_{1-6}$ alkyl is unsubstituted or optionally substituted at any position with 1 to 3 substituents selected from - $L_3$-W, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, $R_8$ and $R_{8'}$ are each independently $C_{1-6}$ alkyl, and the $C_{1-6}$ alkyl is preferably methyl.

In some embodiments, $R_8$ and $R_{8'}$ together with the carbon atom to which they are attached form oxo, thio, $C_{3-6}$ cycloalkyl or 3-6 membered heterocycloalkyl; and the $C_{3-6}$ cycloalkyl or 3-6 membered heterocycloalkyl is unsubstituted or optionally substituted at any position with 1 to 3 substituents selected from deuterium, halogen, hydroxyl, amino, cyano, oxo, $C_{1-6}$ alkyl, halo $C_{1-6}$ alkyl, $-L_2-OR_a$ and $-L_2-NR_aR_b$.

In some embodiments, $R_8$ and $R_{8'}$together with the carbon atom to which they are attached form oxo, thio, cyclopropyl, cyclobutyl, azetidinyl or oxetanyl; and the cyclopropyl, cyclobutyl, azetidinyl or oxetanyl is unsubstituted or optionally substituted at any position with 1 to 3 substituents selected from deuterium, halogen, hydroxyl, amino, cyano, oxo, $C_{1-6}$ alkyl, halo $C_{1-6}$ alkyl, $-L_2-OR_a$ and $-L_2-NR_aR_b$.

In some embodiments, $R_8$ and $R_{8'}$ are each independently unsubstituted $C_{1-6}$ alkyl; or $R_8$ and $R_{8'}$ together with a carbon atom to which they are jointly attached form oxo or unsubstituted $C_{3-6}$ cycloalkyl.

In some embodiments, $R_8$ and $R_{8'}$ together with a carbon atom to which they are jointly attached form oxo, cyclopropyl or cyclobutyl.

In some embodiments, in the $R_8$ and $R_{8'}$, the $C_{1-6}$ alkyl may be methyl, ethyl, n-propyl or isopropyl.

In some embodiments, $R_8$ and $R_{8'}$ together with the carbon atom to which they are attached form $C_{3-10}$ cycloalkylene, and the $C_{3-10}$ cycloalkylene is cyclopropyl or cyclobutyl.

In some embodiments, $R_8$ and $R_{8'}$ together with the carbon atom to which they are attached form 3-10 membered heterocycloalkylene, and a heteroatom in the 3-10 membered heterocycloalkylene is N or O.

In some embodiments, $R_8$ and $R_{8'}$ together with the carbon atom to which they are attached form 3-10 membered heterocycloalkylene, and the number of the heteroatom in the 3-10 membered heterocycloalkylene is 1 or 2.

In some embodiments, $R_8$ and $R_{8'}$ together with the carbon atom to which they are attached form 3-10 membered heterocycloalkylene, and the 3-10 membered heterocycloalkylene may be oxacyclopropylene, azacyclopropylene or oxacyclobutylene or azacyclobutylene.

In some embodiments, $R_1$, $R_2$ and $R_3$ are each independently hydrogen, deuterium, halogen, hydroxyl, amino, cyano, $C_{1-6}$ alkyl, halo $C_{1-6}$ alkyl, $-L_2-OR_a$ or $-L_2-NR_aR_b$.

In some embodiments, $R_1$, $R_2$ and $R_3$ are each independently preferably H, F, Cl, Br, $-CH_3$, $-OCH_3$, $-CF_3$, $-CH_2F$, $-CHF_2$, $-OCF_3$, -CN or $-(CH_2)_{0-5}-NH_2$.

In some embodiments, $R_1$ is H; $R_2$ is H; and $R_3$ is H or F.

In some embodiments, in $R_1$, $R_2$ and $R_3$, the halogen is F, Cl or Br.

In some embodiments, $R_4$ and $R_{4'}$ are each independently hydrogen, $C_{1-6}$ alkyl, $C_{2-6}$ alkenyl, $C_{2-6}$ alkynyl, $C_{3-8}$ cycloalkyl, 3-8 membered heterocycloalkyl, $C_{6-10}$ aryl, 5-10 membered heteroaryl, $C_{3-8}$ cycloalkyl $C_{1-6}$ alkyl, 3-8 membered heterocycloalkyl $C_{1-6}$ alkyl, $C_{6-10}$ aryl $C_{1-6}$ alkyl or 5-10 membered heteroaryl $C_{1-6}$ alkyl; and the $R_4$ or $R_{4'}$ is unsubstituted or optionally substituted at any position with 1 to 3 substituents selected from halogen, cyano, $-L_2-OR_a$, $-L_2-OR_g$, $-L_2-OC(O)R_a$, $-L_2-OC(O)OR_a$, $-L_2-OC(O)NR_aR_b$, $-L_2-NR_aR_b$, $-L_2-NR_aC(O)OR_b$, $-L_2-NR_aC(O)NR_aR_b$, $-L_2-NR_bC(NR_b)NR_aR_b$ or $-L_2-C(O)OR_b$; or $R_4$ and $R_{4'}$ together with the N atom to which they are attached form 3-8 membered heterocycloalkyl; and the 3-8 membered heterocycloalkyl is unsubstituted or optionally substituted at any position with 1-3 substituents selected from halogen, cyano, $-L_2-OR_a$, $-L_2-NR_aR_b$, $-L_2-NR_aC(O)OR_b$, $-L_2-NR_aC(O)NR_aR_b$, $- L_2-NR_bC(NR_b)NR_aR_b$ or $-L_2-C(O)OR_b$.

In some embodiments, $R_4$ or $R_{4'}$ are each independently hydrogen or $C_{1-6}$ alkyl; and the $R_4$ or $R_{4'}$ is unsubstituted or optionally substituted at any position with 1 to 3 substituents selected from $-OR_a$, $-OR_g$, $-OC(O)R_a$, $-OC(O)OR_a$, $-OC(O)NR_aR_b$, $-NR_aR_b$, $-NR_aC(O)OR_b$, $-NR_aC(O)NR_aR_b$, $-NR_bC(NR_b)NR_aR_b$ and $-C(O)OR_b$.

In some embodiments, $R_4$ is hydrogen or $C_{1-6}$ alkyl; the $R_4$ is unsubstituted or optionally substituted at any position with one substituent from $-OR_a$, $-OR_g$, $-OC(O)R_a$, $-OC(O)OR_a$, $-OC(O)NR_aR_b$, $-NR_aR_b$, $- NR_aC(O)OR_b$, $-NR_aC(O)NR_aR_b$, $-NR_bC(NR_b)NR_aR_b$ and $-C(O)OR_b$; $R_{4'}$ is $C_{1-6}$ alkyl; and the $R_{4'}$ is unsubstituted or optionally substituted at any position with 1 to 3 substituents selected from halogen, $-OR_a$ and $-NR_aR_b$.

In some embodiments, $R_4$ and $R_{4'}$ are each independently hydrogen or $C_{1-6}$ alkyl; and the $R_4$ or $R_{4'}$ is unsubstituted or optionally substituted at any position with one substituent selected from halogen,$-OR_a$ and $-NR_aR_b$.

In some embodiments, $R_4$ and $R_{4'}$ are each independently $C_{1-6}$ alkyl; and at least one of the $R_4$ or $R_{4'}$ is substituted at any position with one substituent selected from hydroxyl and amino.

In some embodiments, $R_4$ and $R_{4'}$ are each independently $C_{1-6}$ alkyl; and at least one of the $R_4$ or $R_{4'}$ is substituted at any position with one hydroxyl.

In some embodiments, $R_4$ is $-OCH_2CH_3$, $-CH_2CH_2NH_2$, $-CH_2CH_2OH$ or $-CH_2CH_2CH_3$; and $R_{4'}$ is $-CH_2CH_2OH$ or $-CH_2CH_2CH_3$.

In some embodiments, $R_4$ is $-CH_2CH_2OH$ or $-CH_2CH_2CH_3$; and $R_{4'}$ is $-CH_2CH_2OH$ or $- CH_2CH_2CH_3$.

In some embodiments, $R_5$ is H.

In some embodiments, in the $R_g$, the phosphoryl-containing prodrug group is

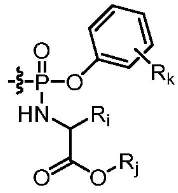

;

$R_i$ is hydrogen, $C_{1-6}$ alkyl (e.g. methyl, ethyl, isobutyl) or benzyl; $R_j$ is $C_{1-6}$ alkyl (e.g., methyl, ethyl, isopropyl, tert-butyl, isobutyl), $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; and $R_k$ is hydrogen, halogen or $C_{1-6}$ alkyl.

In some embodiments, in the $R_g$, the phosphoryl-containing prodrug group is

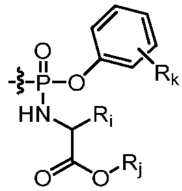

;

$R_i$ is methyl; $R_j$ is isopropyl or benzyl; and $R_k$ is hydrogen.

In some embodiments, $R_g$ is

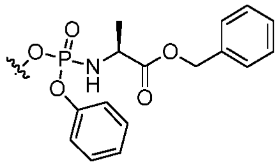

or

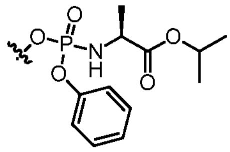

.

In some embodiments, in the $R_4$ and $R_{4'}$, the $C_{1-6}$ alkyl may be methyl, ethyl, n-propyl or isopropyl.

In some embodiments, in the $R_4$ and $R_{4'}$, the $C_{1-6}$ alkoxy may be methoxy, ethoxy, n-propoxy or isopropoxy.

In some embodiments, in the $R_4$ and $R_{4'}$, $C_{1-6}$ alkyl in the $C_{1-6}$ alkyl substituted with 1, 2 or 3 $R_{4-1}$ may be methyl, ethyl, n-propyl or isopropyl.

In some embodiments, in the $R_a$ and $R_b$, the $C_{1-6}$ alkyl may be methyl, ethyl, n-propyl or isopropyl.

In some embodiments, in the $R_a$ and $R_b$, the $C_{3-10}$ cycloalkyl may be cyclopropyl, cyclobutyl or cyclopentyl.

In some embodiments, in the $R_i$, the $C_{1-6}$ alkyl may be methyl, ethyl, n-propyl or isopropyl.

In some embodiments, in the $R_k$, the $C_{1-6}$ alkyl may be methyl, ethyl, n-propyl or isopropyl.

In some embodiments, in the $R_j$, the $C_{1-6}$ alkyl may be methyl, ethyl, n-propyl or isopropyl.

In some embodiments, in the $R_j$, in the $C_{6-10}$ aryl-$C_{1-6}$ alkyl, the $C_{6-10}$ aryl may be phenyl.

In some embodiments, in the $R_j$, in the $C_{6-10}$ aryl-$C_{1-6}$ alkyl, the $C_{1-6}$ alkyl may be methyl, ethyl, n-propyl or isopropyl.

In some embodiments, $R_4$ and $R_{4'}$ are independently $-OCH_2CH_3$, $-CH_2CH_2NH_2$, $-CH_2CH_2OH$ or - $CH_2CH_2CH_3$.

In some embodiments, at least one of $R_4$ and $R_{4'}$ is $-OCH_2CH_3$, $-CH_2CH_2NH_2$ or $-CH_2CH_2OH$.

In some embodiments, $R_5$ is H.

In some embodiments, $R_{5'}$ is H.

In some embodiments, $Cy^1$ is $C_{3-8}$ cycloalkyl, 3-8 membered heterocycloalkyl, $C_{6-10}$ aryl or 5-10 membered heteroaryl; and the $Cy^1$ is unsubstituted or optionally substituted at any position with 1 to 3 substituents selected from halogen, cyano, $C_{1-6}$ alkyl, halo $C_{1-6}$ alkyl, halo $C_{1-6}$ alkoxy, $C_{2-6}$ alkenyl, $C_{2-6}$ alkynyl, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$ or $-L_4-NR_eR_{e'}$.

In some embodiments, $L_2$ is a linkage bond or $C_{1-6}$ alkylene; and the $L_2$ is unsubstituted or optionally substituted at any position with 1 to 3 substituents selected from oxo, halogen, hydroxyl, amino, cyano, $C_{1-6}$ alkyl, halogenated $C_{1-6}$ alkyl, $C_{1-6}$ alkoxy or halogenated $C_{1-6}$ alkoxy.

In some embodiments, in the $L_2$, $C_{1-6}$ alkylene may be $C_{1-3}$ alkylene.

In some embodiments, $L_2$ is a linkage bond, $-CH_2-$, $-CH_2CH_2-$, $-CH_2CH_2CH_2-$ or - $CH_2CH_2CH_2CH_2-$.

In some embodiments, $L_2$ is a linkage bond or $-CH_2CH_2-$.

In some embodiments, $L_3$ is a linkage bond or $C_{1-6}$ alkylene; and the $L_3$ is unsubstituted or optionally substituted at any position with 1 to 3 substituents selected from oxo, halogen, hydroxyl, amino, cyano, $C_{1-6}$ alkyl, halogenated $C_{1-6}$ alkyl, $C_{1-6}$ alkoxy or halogenated $C_{1-6}$ alkoxy.

In some embodiments, in the $L_3$, $C_{1-6}$ alkylene may be $C_{1-3}$ alkylene.

In some embodiments, $L_3$ is a linkage bond, $-CH_2-$, $-CH_2CH_2-$, $-CH_2CH_2CH_2-$, $-CH_2CH_2C(CH_3)_2-$, $-CH_2CH_2CH_2CH_2-$ or $-CH_2CH(CH_3)CH_2-$.

In some embodiments, $L_3$ is a linkage bond or $-CH_2-$.

In some embodiments, $L_4$ is a linkage bond or $C_{1-6}$ alkylene; and the $L_4$ is unsubstituted or optionally substituted at any position with 1 to 3 substituents selected from oxo, halogen, hydroxyl, amino, cyano, $C_{1-6}$ alkyl, halogenated $C_{1-6}$ alkyl, $C_{1-6}$ alkoxy or halogenated $C_{1-6}$ alkoxy.

In some embodiments, each $R_c$ is independently hydrogen or a linkage bond attached to L; and only one $R_c$ in D is a linkage bond attached to L.

In some embodiments, D is:

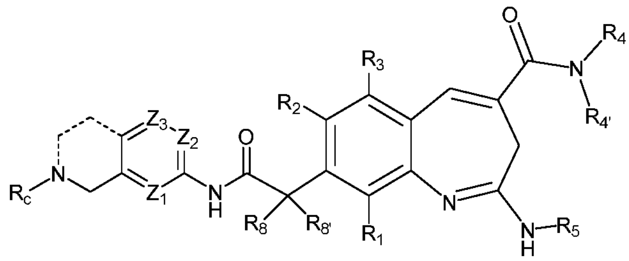

(D-1)

or

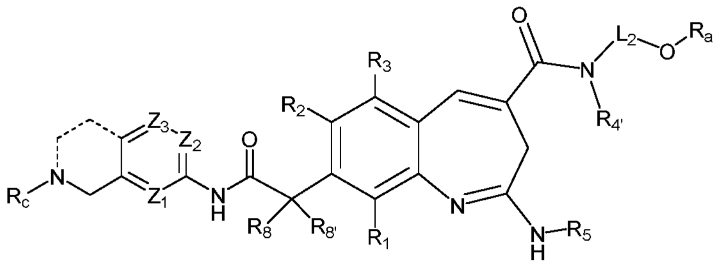

(D-2)

,

where

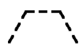

is absent, $-CH_2-$ or $-CH_2-CH_2-$;

$Z_1$, $Z_2$ and $Z_3$ are each independently N or CH;

$R_c$ is a linkage bond attached to L; and

definitions of $R_1$, $R_2$, $R_3$, $R_4$, $R_{4'}$, $R_5$, $R_8$, $R_{8'}$ and $R_a$ are as previously described.

In some embodiments, $Z_1$ is CH, $Z_2$ is CH, $Z_3$ is N or CH.

In some embodiments, formula D' is any one of formulas D'-1 to D'-6:

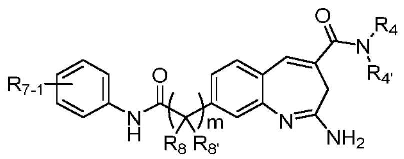

D'-1

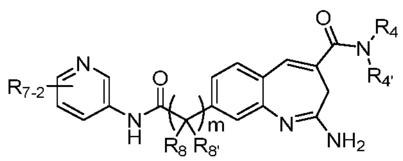

D'-2

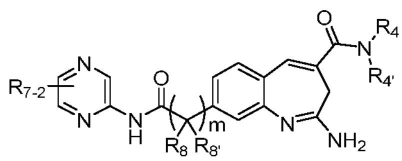

D'-3

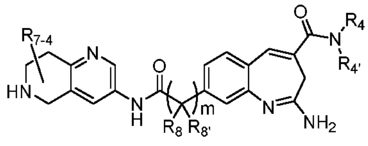

D'-4

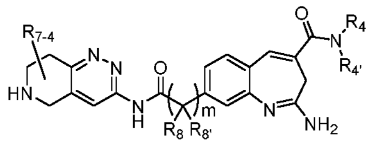

D'-5

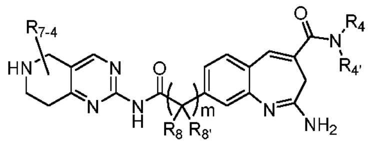

D'-6

.

In some embodiments, D is of a structure shown in Table 5:

Table 5

| | |
|---|---|
| 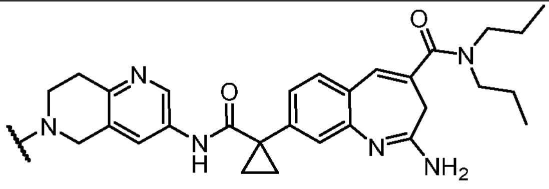 | 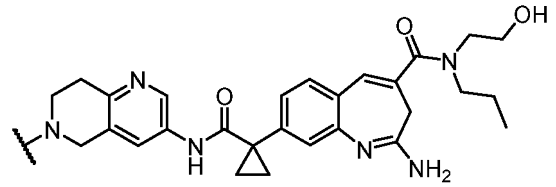 |
| 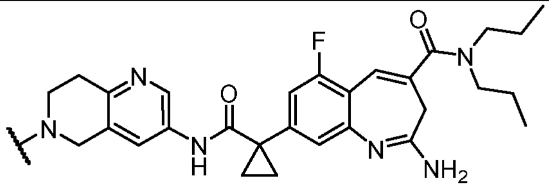 | 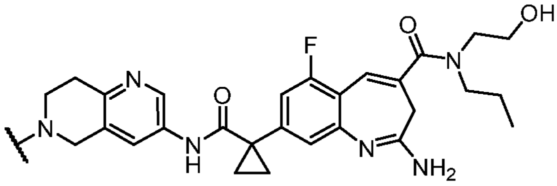 |
| 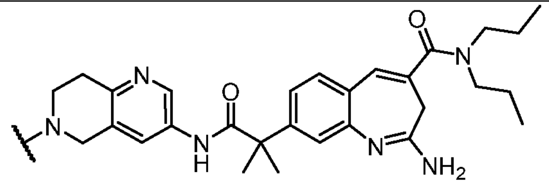 | 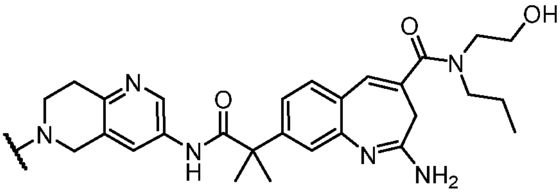 |
| 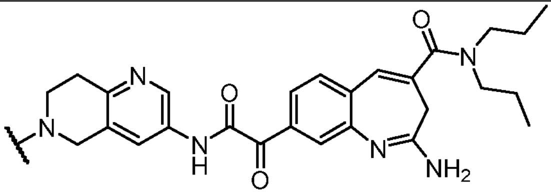 | 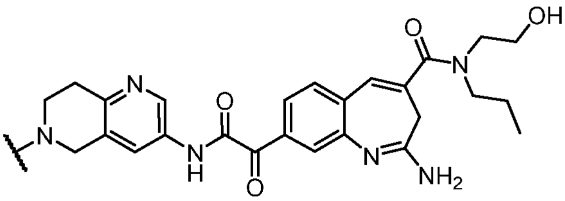 |
| 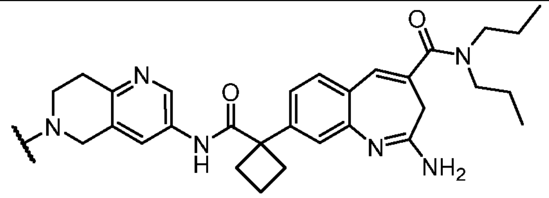 | 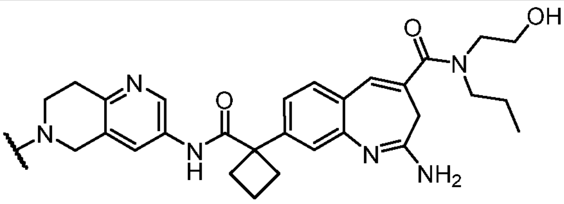 |
| 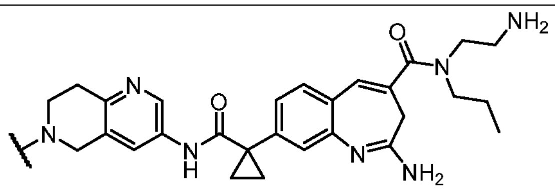 | 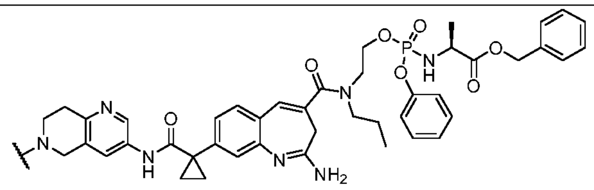 |
| 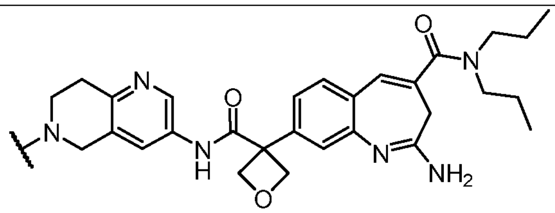 | 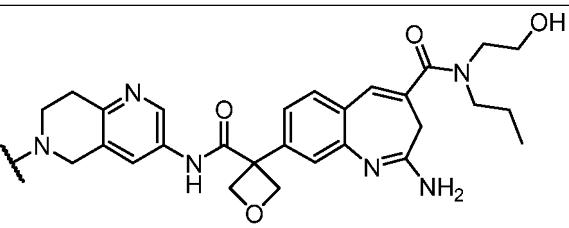 |
| 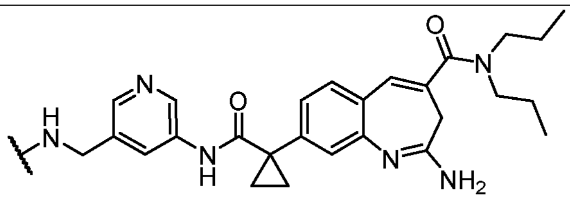 | 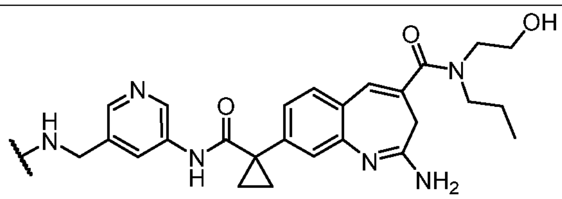 |

| | |
|---|---|
| 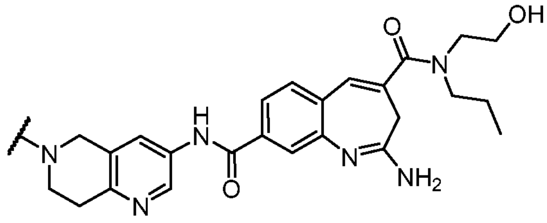 | 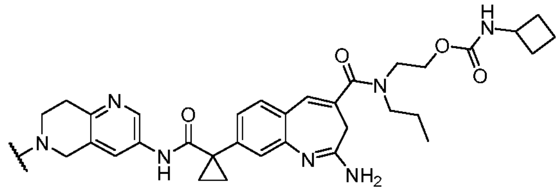 |
| 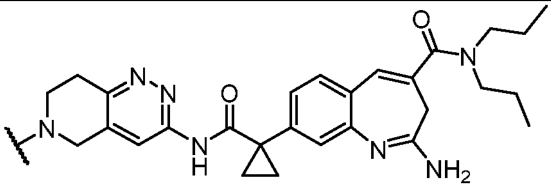 | 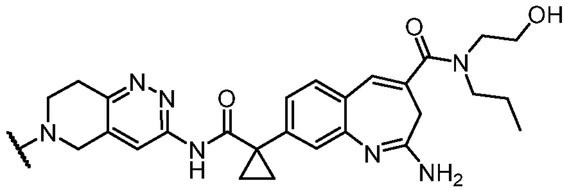 |
| 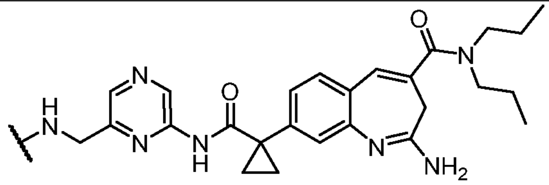 | 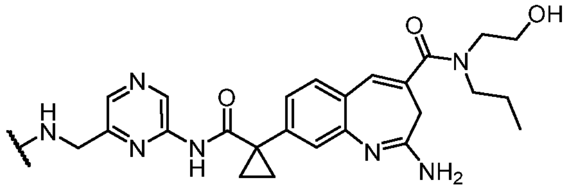 |
| 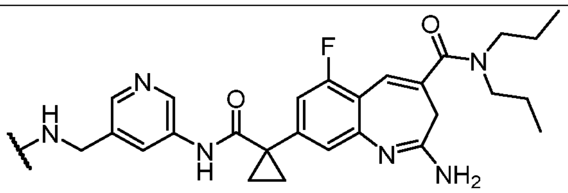 | 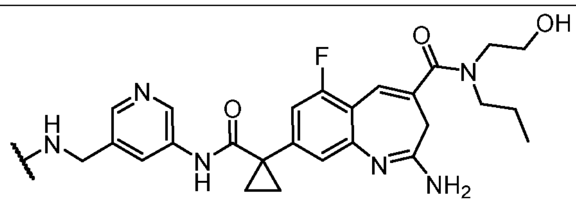 |
| 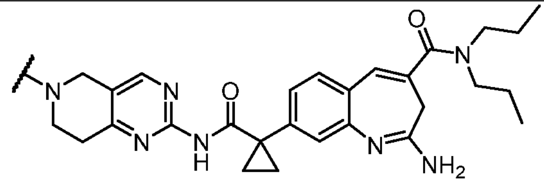 | 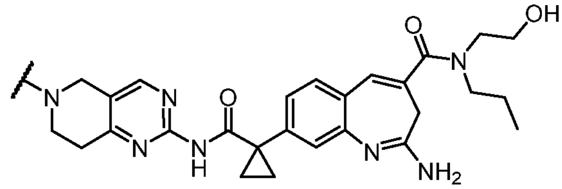 |
| 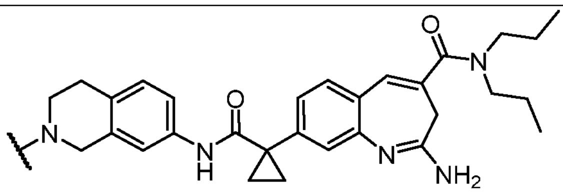 | 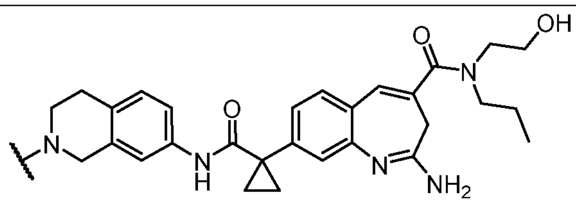 |
| 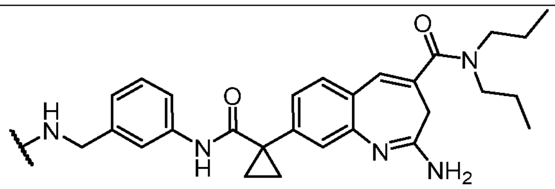 | 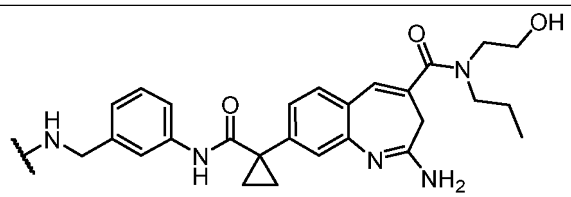 |
| 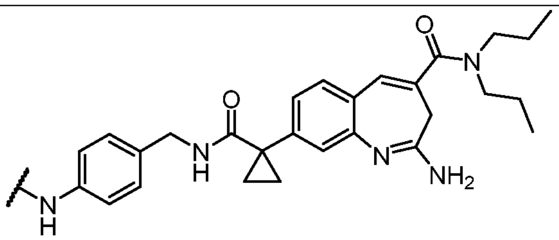 | 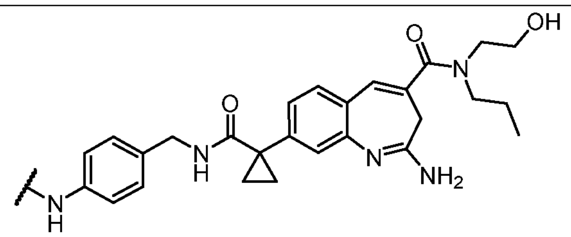 |

| | |
|---|---|
| 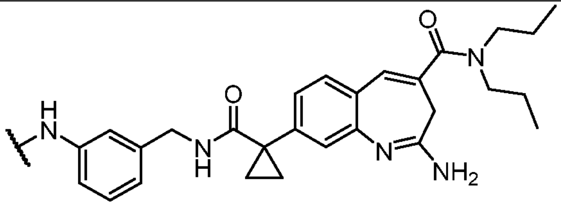 | 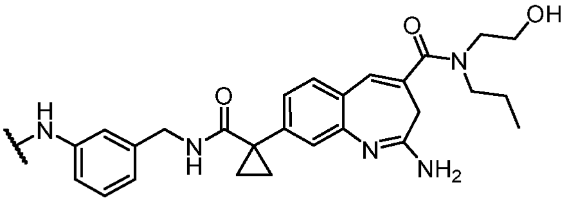 |
| 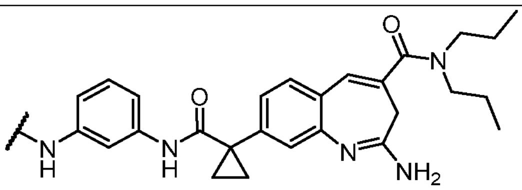 | 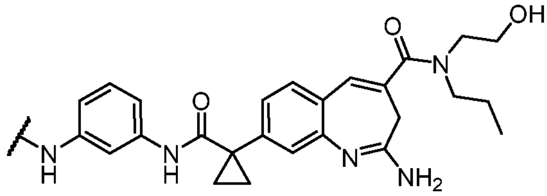 |
| 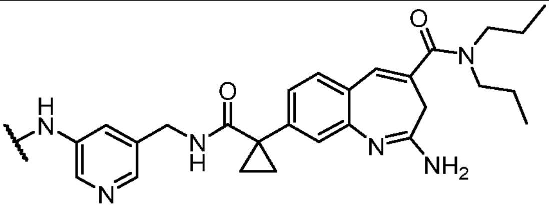 | 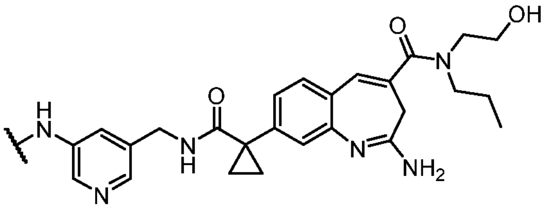 |
| 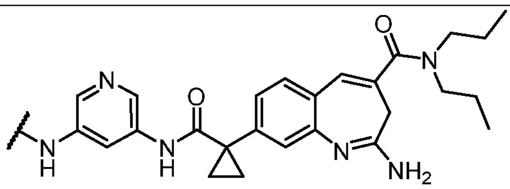 | 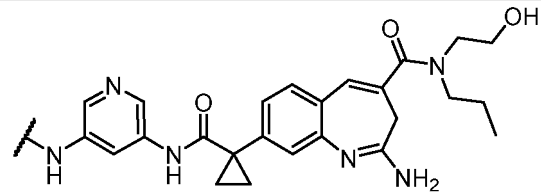 |
| 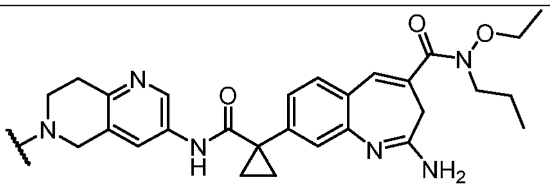 | 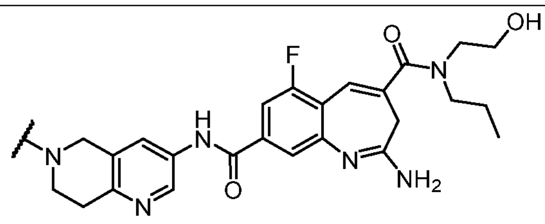 |
| 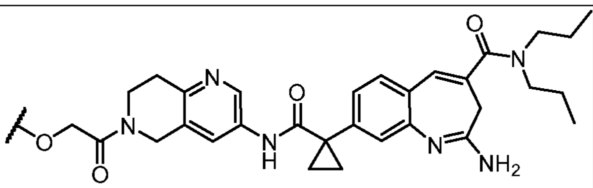 | 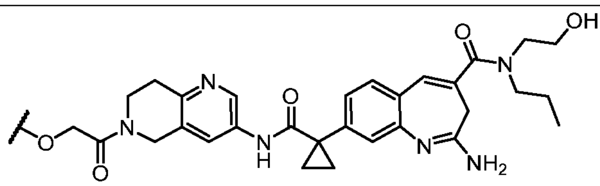 |
| 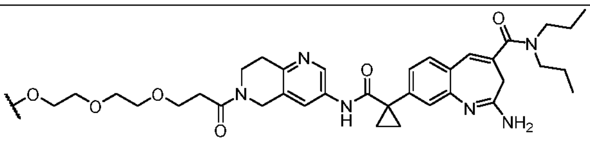 | 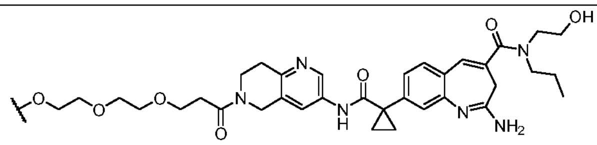 |
| 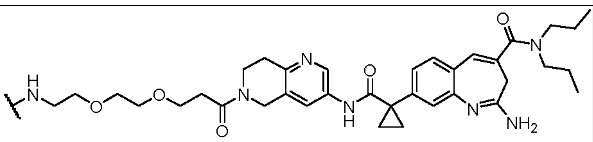 | 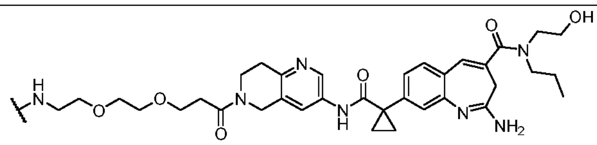 |
| 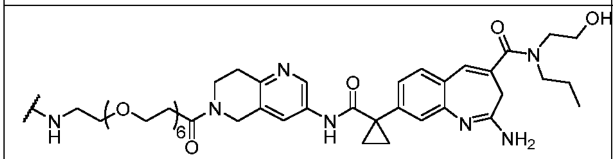 | 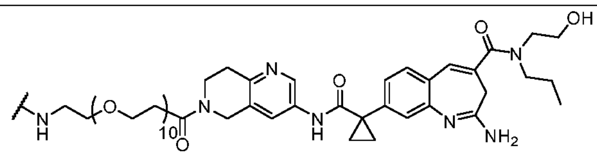 |

.

In some embodiments, the L is -M-$(T)_w$-PEG-$(T)_o$-*, -M-PEG-*, -M-$(T)_w$-*, -M-$(T)_w$-$(A)_v$-$(T)_o$-PEG-*, -M-$(T)_w$-$(A)_v$-$(T)_o$-*, -M-$(T)_w$-$L_5$-*, -M-$(T)_w$-PEG-$L_5$-*, -M-$(T)_w$-PEG-$(A)_v$-$L_5$-*, -M-$(T)_w$-PEG-$(A)_v$-$(T)_o$-*, -M-PEG-$(T)_o$-PEG-* or -M-$(T)_w$-$(A)_v$-$L_5$-*; and * is a linking site of the L that is attached to the D;

M is a linkage bond or a linker head attached to Ab;
w is independently 1, 2, 3, 4, 5, 6, 7, 8, 9 or 10;
o is independently 0, 1, 2, 3, 4, 5, 6, 7, 8, 9 or 10;
T is independently -$(CH_2)_x$-C(O)-, -$NR_h$-, -O-, -S-, -$(CH_2)_x$-$C_{6-14}$ arylene-$(CH_2)_y$-, -$(CH_2)_x$-5-6 membered heteroarylene-$(CH_2)_y$-, -$(CH_2)_x$-$C_{3-6}$ cycloalkylene-$(CH_2)_y$-, -$(CH_2)_x$-3-6 membered heterocycloalkylene-$(CH_2)_y$-, -$NR_h$-$(CH_2)_x$-C(O)-, -O-$(CH_2)_x$-C(O)-, -S-$(CH_2)_x$-C(O)-, -$(CH_2)_x$CH($NHR_h$)-C(O)-, -$(CH_2)_x$-S-S-$C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the -$(CH_2)_x$-5-6 membered heteroarylene-$(CH_2)_y$-, the heteroatom is selected from one or more of N, O and S, and the number of the heteroatom is 1, 2 or 3; and in "3-6 membered heterocycloalkylene" in the -$(CH_2)_x$-3-6 membered heterocycloalkylene-$(CH_2)_y$-, the heteroatom is selected from one or more of N, O and S, and the number of the heteroatom is 1, 2 or 3;
$R_h$ is independently hydrogen, $C_{1-6}$ alkyl, -$PO(OH)_2$, -$PO(OCH_3)_2$, -C(O)-$(CH_2CH_2O)_n$-$CH_3$ or - $(CH_2CH_2O)_n$-$CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;
PEG is -$(CH_2CH_2O)_n$-$(CH_2)_u$- or -$(CH_2CH_2O)_n$-$(CH_2)_u$-C(O)-; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;
v is independently 1, 2, 3, 4 or 5;
A is independently an amino acid residue or an amino acid residue substituted with 1 $R_A$; the $R_A$ is independently -C(O)-$(CH_2CH_2O)_n$-$CH_3$ or -$(CH_2CH_2O)_n$-$CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and
$L_5$ is a self-immolative group.

In some embodiments, in the M, the linker head is a group formed by click reaction ligation, click-like reaction ligation, thiol ligation, amine ligation, oxime ligation or hydrazone ligation.

In some embodiments, in the M, the linker head is a group formed by thiol ligation on the side chain of a cysteine residue or amine ligation on the side chain of a lysine residue of the antibody.

In some embodiments, in the M, the linker head is a group formed by thiol ligation, preferably

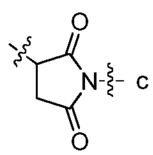, 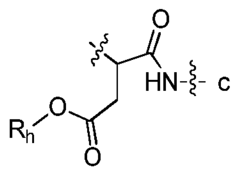, 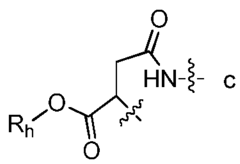, 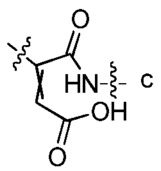, 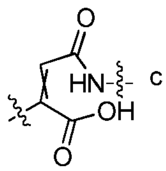, 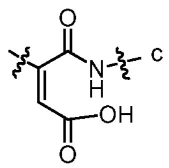, 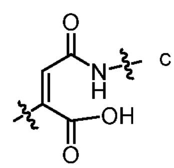, 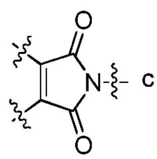, 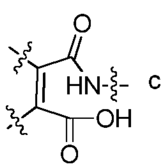, 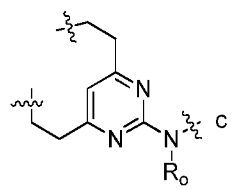, 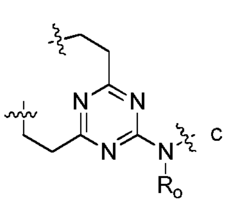, 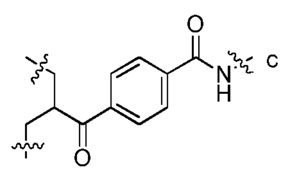, 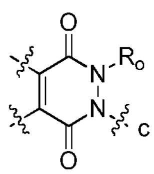, 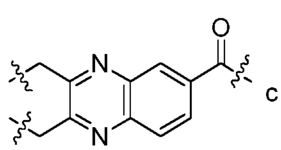

or

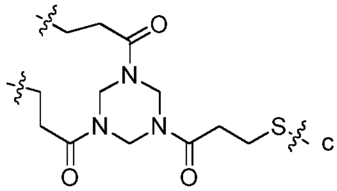;

$R_o$ is hydrogen or $C_{1-4}$ alkyl; and the linker head is attached to the rest of L through a c-terminus.

In some embodiments, $R_o$ is hydrogen or methyl.

In some embodiments, in the M, the linker head is a group formed by thiol ligation, preferably

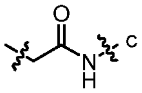, 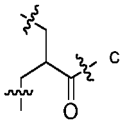, 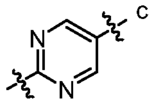, 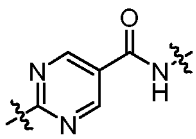, 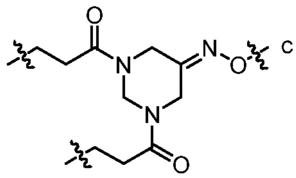,

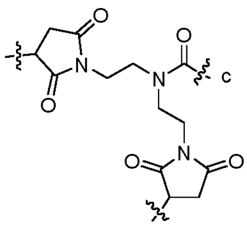, 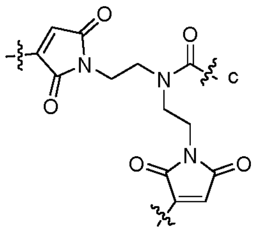, 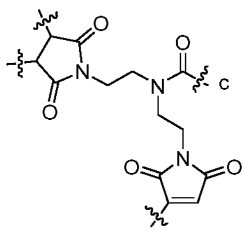 or 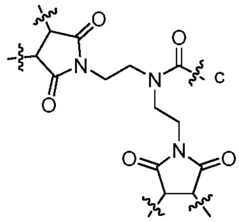;

and the linker head is attached to the rest of L through a c-terminus.

In some embodiments, in the M, the linker head is a group formed by oxime ligation, preferably

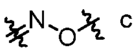;

and the linker head is attached to the rest of L through a c-terminus.

In some embodiments, in the M, the linker head is a group formed by hydrazone ligation, preferably

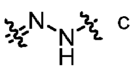,

and the linker head is attached to the rest of L through a c-terminus.

In some embodiments, in the M, the linker head is a group formed by amine ligation, and the group formed by amine ligation is preferably a group formed by amine ligation of a lysine side chain, and more preferably

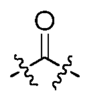.

In some embodiments, the linker head is a group formed by click reaction ligation or click-like reaction ligation, preferably

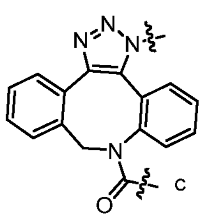, 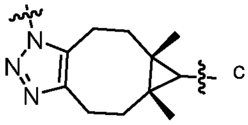, 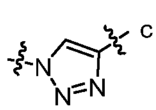 or 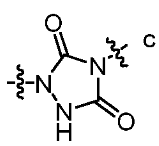;

and the linker head is attached to the rest of L through a c-terminus.

In some embodiments, in the M, the linker head is

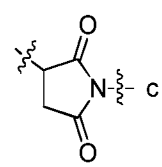 , 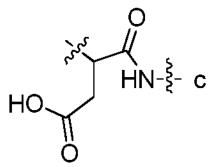 ,

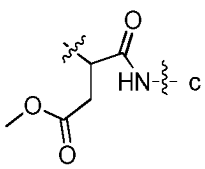 , 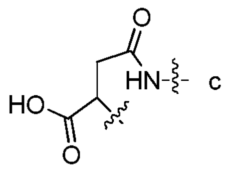 , 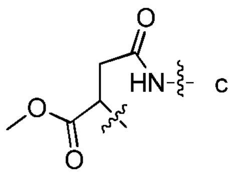 , 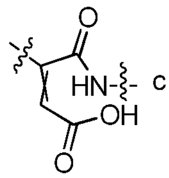 , 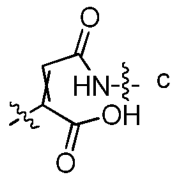 ,

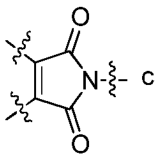 , 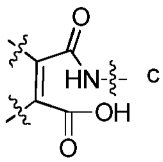 , 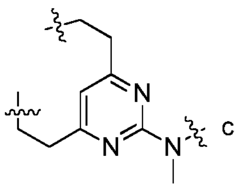 , 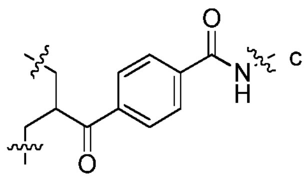 , 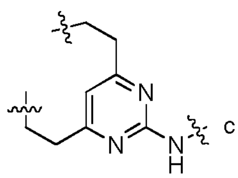 ,

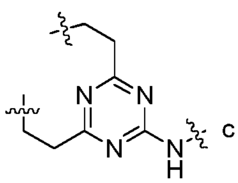 , 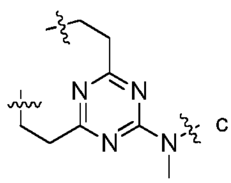 , 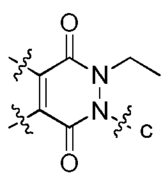 , 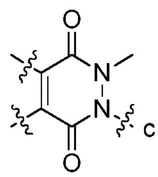 , 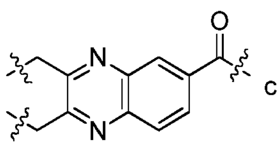 ,

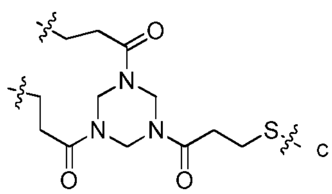 , 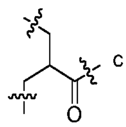 , 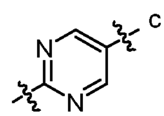 , 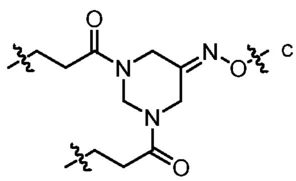 , 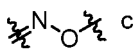 ,

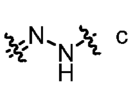 , 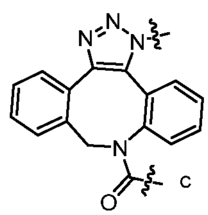 , 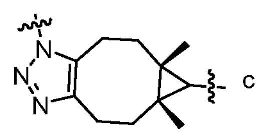 , 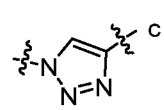 , 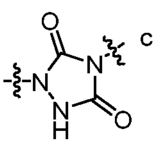 or 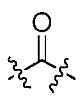 ;

and the linker head is attached to the rest of L through a c-terminus.

In some embodiments, each A is independently

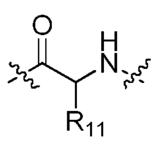 ;

each $R_{11}$ is independently an amino acid side chain or an amino acid side chain modified by $-C(O)(CH_2CH_2O)_nCH_3$ or $-(CH_2CH_2O)_nCH_3$; or $R_{11}$ and an adjacent nitrogen atom form a five-membered heterocycloalkyl.

In some embodiments, in the A, the amino acid is a natural amino acid or a non-natural amino acid.

In some embodiments, the

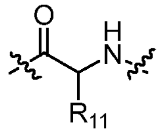

may be

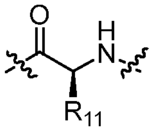 or 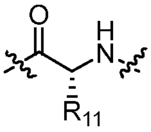,

and may also be

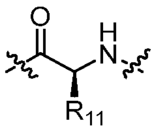.

In some embodiments, in the A, each $R_{11}$ is independently hydrogen, methyl, ethyl, n-propyl, isopropyl, isobutyl, $-SO_3H$,

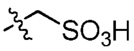, 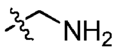, 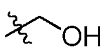, 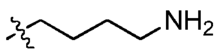, 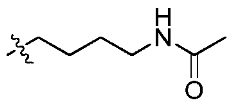,

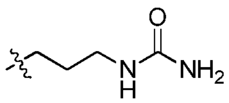, 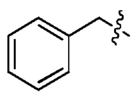, 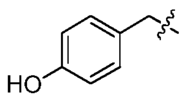, 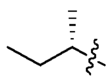, 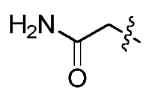, 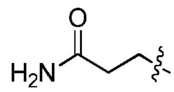,

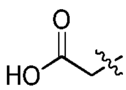, 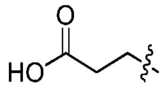 or 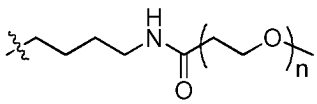;

or $R_{11}$ and an adjacent nitrogen atom form a five-membered heterocycloalkyl.

In some embodiments, $-(A)_v-$ is

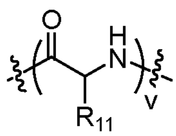;

each $R_{11}$ is independently hydrogen, methyl, ethyl, n-propyl, isopropyl, isobutyl, $-SO_3H$,

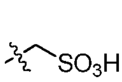, 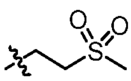, 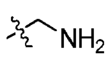, 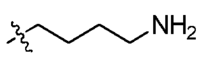,

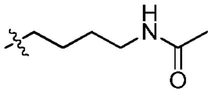, 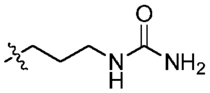, 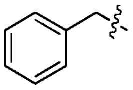, 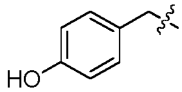, 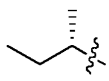, 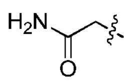,

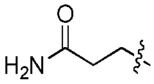, 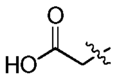, 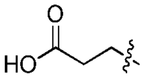 or 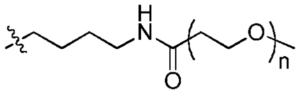;

or $R_{11}$ and an adjacent nitrogen atom form a five-membered heterocycloalkyl; and v is any integer from 1 to 4, and v is preferably 1 or 2.

In some embodiments, v is 3 or 4.

In some embodiments, A is attached to $-(T)_o-$ or $-L_5-$ through a carbonyl terminus.

In some embodiments, $L_5$ is

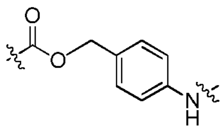, 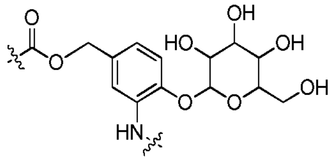,

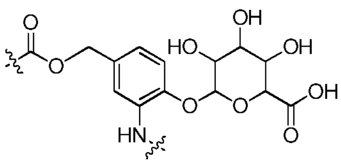, 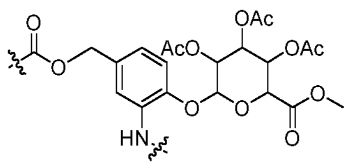, 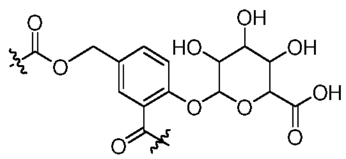,

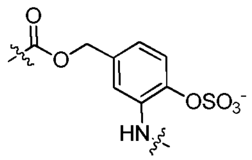 or 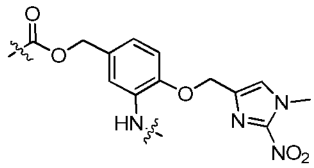.

In some embodiments, $L_5$ is

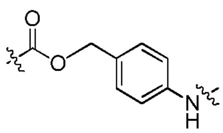, 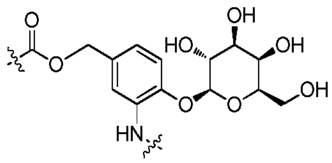,

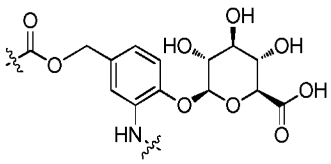, 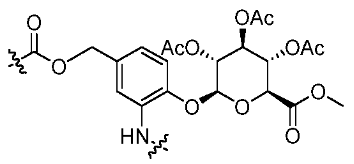, 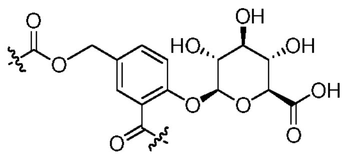,

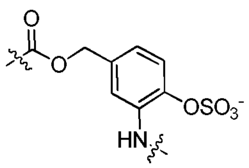 or 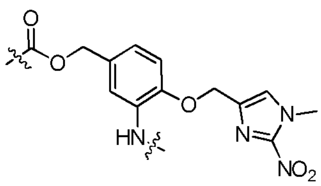.

In some embodiments, $L_5$ is

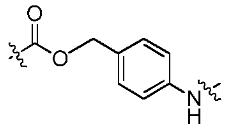,
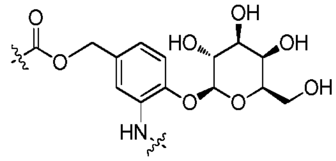,
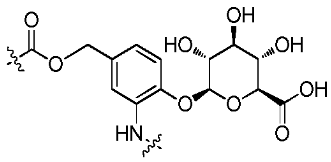,
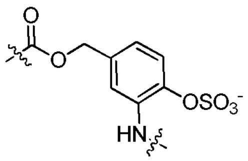
or
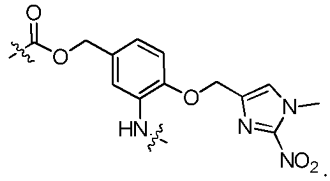.

In some embodiments, $-L_5-$ is attached to D through a carbonyl terminus.

In some embodiments, each T is independently $-(CH_2)_x-C(O)-$, $-NR_h-$, -O-, -S-, $-(CH_2)_x-C_{6-10}$ arylene-$(CH_2)_y-$, $-(CH_2)_x$-5-10 heteroarylene-$(CH_2)_y-$, $-(CH_2)_x-C_{3-10}$ cycloalkylene-$(CH_2)_y-$, $-(CH_2)_x$-3-10 membered heterocycloalkylene-$(CH_2)_y-$, $-NR_h-(CH_2)_x-C(O)-$, $-O-(CH_2)_x-C(O)-$, $-S-(CH_2)_x-C(O)-$, - $(CH_2)_xCH(NHR_h)-C(O)-$, $C_{1-20}$ alkylene or $C_{2-20}$ alkenylene, and x and y are each independently any integer from 0 to 10.

In some embodiments, each T is independently $-(CH_2)_x-C(O)-$, $-NR_h-$, -O-, -S-, $-(CH_2)_x$-phenylene-$(CH_2)_y-$, $-(CH_2)_x$-5-6 heteroarylene-$(CH_2)_y-$, $-(CH_2)_x-C_{3-8}$ cycloalkylene-$(CH_2)_y-$, $-(CH_2)_x$-3-8 membered heterocycloalkylene-$(CH_2)_y-$, $-NR_h-(CH_2)_x-C(O)-$, $-O-(CH_2)_x-C(O)-$, $-S-(CH_2)_x-C(O)-$, - $(CH_2)_xCH(NHR_h)-C(O)-$ or $C_{1-6}$ alkylene; and x and y are each independently any integer from 0 to 10;

In some embodiments, $R_h$ is hydrogen or $C_{1-6}$ alkyl; and $R_h$ is preferably hydrogen or $C_{1-3}$ alkyl.

In some embodiments, $-(T)_w-$ is

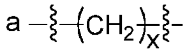,
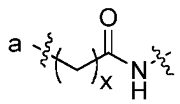,
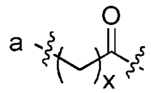,
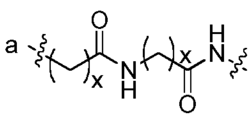,
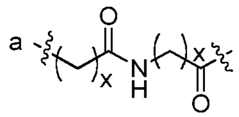,
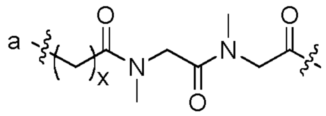,
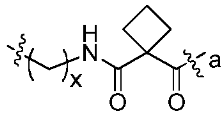,
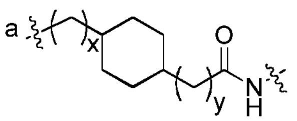,
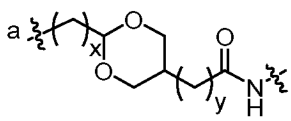,
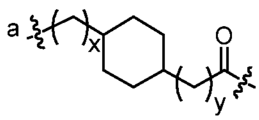,
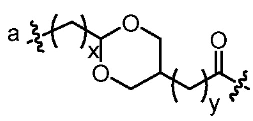,
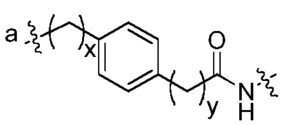,
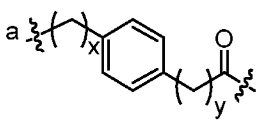,
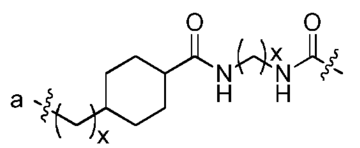,
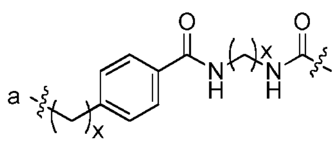,
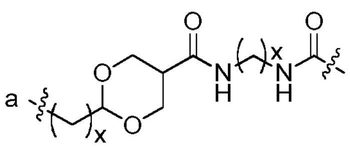,
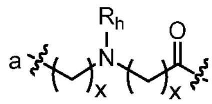

or

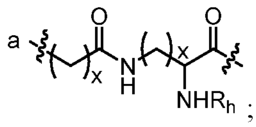 ;

and -$(T)_w$- is attached to the -M- through an a-terminus. In some embodiments, each x is independently an integer from 0 to 10, and the x is more preferably an integer from 0 to 6.

In some embodiments, each y is independently an integer from 0 to 10, and the y is more preferably an integer from 0 to 6.

In some embodiments, -$(T)_o$- is

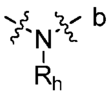 , 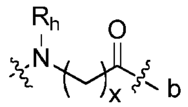 , 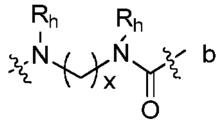 ,

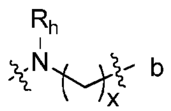 or 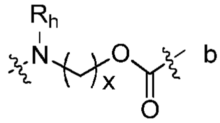 ;

and -$(T)_o$- is attached to the -PEG- or the D via a b-terminus.

In some embodiments, each x is independently an integer from 0 to 10, and more preferably an integer from 0 to 6.

In some embodiments, PEG is -$(CH_2CH_2O)_n$-$(CH_2)_u$- or -$(CH_2CH_2O)_n$-$(CH_2)_u$-C(O)-, n is any integer from 1 to 30, and u is any integer from 1 to 5;

In some embodiments, when L is -M-$(T)_w$-$(A)_v$-$L_5$-*, a combination of -M-, -$(T)_w$-, -$(A)_v$- and -$L_5$-does not simultaneously satisfy conditions of: -M- is succinimidyl (

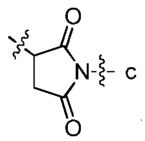

); -$(T)_w$- is hexanoyl; -$(A)_v$-is -Val-Cit-, -Val-Ala- or -Gly-Gly-Phe-Gly-, and -$L_5$- is PABC (

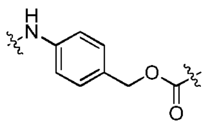

).

In some embodiments, L is -M-$(T)_w$-$(A)_v$-$L_5$-*; and the L is preferably -M-$CH_2$-($C_{5-6}$ cycloalkylene)-C(=O)-$(A)_v$-$L_5$-, -M-$CH_2$-(5-6 membered heterocyclylene)-C(=O)-$(A)_v$-$L_5$-, -M-(phenylene)-$CH_2$-C(=O)-$(A)_v$-$L_5$- or -M-$(CH_2)_x$-C(=O)NH-$CH_2$-CH($NHR_h$)-C(O)-$(A)_v$-$L_5$-.

In some embodiments, L is -M-$(T)_w$-PEG-$(A)_v$-$L_5$-*; and the L is preferably -M-$(CH_2)_x$-C(=O)NH-PEG-$(A)_v$-$L_5$-, -M-(phenylene)-$CH_2$-C(=O)NH-PEG-$(A)_v$-$L_5$-, -M-$CH_2$-($C_{5-6}$ cycloalkylene)-C(=O)NH-PEG-$(A)_v$-$L_5$- or -M-$CH_2$-(5-6 membered heterocyclylene)-C(=O)NH-PEG-$(A)_v$-$L_5$-.

In some embodiments, L is -M-$(T)_w$-$(A)_v$-$(T)_o$-*; and the L is preferably -M-$(CH_2)_x$-C(=O)NH-$(A)_v$-NH-$CH_2$-.

In some embodiments, L is any one of combinations of:

a) -M-PEG-;
b) -M-$(CH_2)_x$-;
c) -M-$(CH_2)_x$-C(O)-;
d) -M-$(CH_2)_x$-($C_{5-6}$ cycloalkylene)-$(CH_2)_y$-C(O)NH-PEG-;
e) -M-$(CH_2)_x$-(5-6 membered heterocyclylene)-$(CH_2)_y$-C(O)NH-PEG-;
f) -M-$(CH_2)_x$-(phenylene)-$(CH_2)_y$-C(O)NH-PEG-;

g) -M-$(CH_2)_x$-C(O)NH-PEG-;
h) -M-$(CH_2)_x$-C(O)NH-$(CH_2)_x$-C(O)NH-PEG-;
i) -M-$(CH_2)_x$-C(O)NH-$(CH_2)_x$-C(O)NH-PEG-NH-$(CH_2)_x$-C(O)-;
j) -M-$(CH_2)_x$-($C_{5-6}$ cycloalkylene)-$(CH_2)_y$-C(O)NH-$(CH_2)_x$-NHC(O)-;
k) -M-$(CH_2)_x$-(5-6 membered heterocyclylene)-$(CH_2)_y$-C(O)NH-$(CH_2)_x$-NHC(O)-;
l) -M-$(CH_2)_x$-(phenylene)-$(CH_2)_y$-C(O)NH-$(CH_2)_x$-NHC(O)-;
m) -M-$(CH_2)_x$-C(O)NH-$(CH_2)_x$-C(O)-$L_5$-;
n) -M-$(CH_2)_x$-C(O)NH-PEG-$L_5$-;
o) -M-$(CH_2)_x$-(phenylene)-$(CH_2)_y$-C(O)-$(A)_v$-;
p) -M-$(CH_2)_x$-C(O)NH-($C_{3-6}$ cycloalkylene)-C(O)-$(A)_v$-;
q) -M-$(CH_2)_x$-C(O)NH-PEG-$(A)_v$-;
r) -M-$(CH_2)_x$-C(O)NH-PEG-$(A)_v$-$L_5$-;
s) -M-$(CH_2)_x$-($C_{5-6}$ cycloalkylene)-C(O)-;
t) -M-$(CH_2)_x$-(5-6 membered heterocyclylene)-C(O)-;
u) -M-(phenylene)-$(CH_2)_y$-C(O)-;
v) -M-$(CH_2)_x$-C(O)-$(A)_v$-NH-PEG-;
w) -M-$(CH_2)_x$-C(O)-$(A)_v$-NH-$(CH_2)_x$-C(O)-;
x) -M-$(CH_2)_x$-C(O)-$(A)_v$-;
y) -M-$(CH_2)_x$-(5-6 membered heterocyclylene)-C(O)-$(A)_v$-
z) -M-$(CH_2)_x$-C(O)NH-$CH_2$-CH($NHR_h$)-C(O)-$(A)_v$-;
aa) -M-(phenylene)-(5-6 membered heterocyclylene)-C(O)-;
ab) -M-(phenylene)-$(CH_2)_y$-C(O)NH-$(CH_2)$x-PEG-$(A)_v$-;
ac) -M-(phenylene)-C(O)NH-$(CH_2)$x-$(A)_v$-$L_5$- or
ad) -M-$(CH_2)_x$-PEG-$(A)_v$-.

In some embodiments, L is

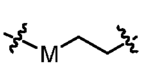, 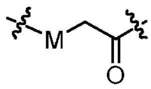, 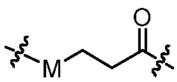, 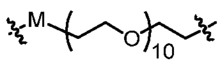,

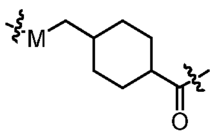, 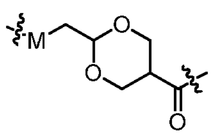, 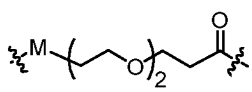, 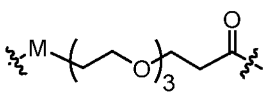,

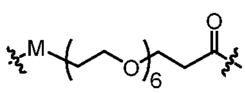, 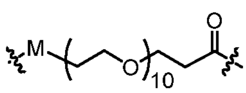, 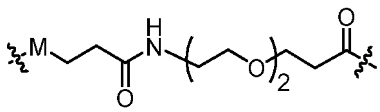,

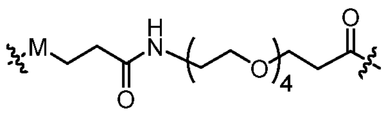, 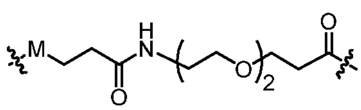,

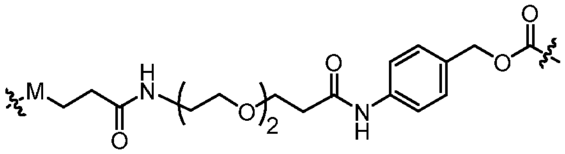, 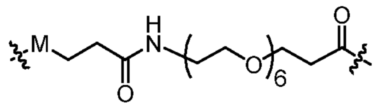,

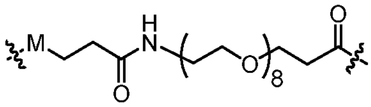, 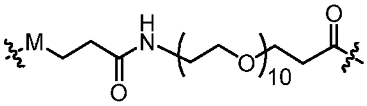, 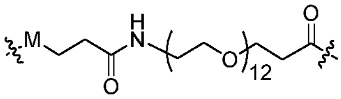,

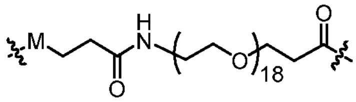 , 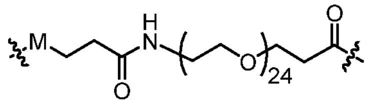 , 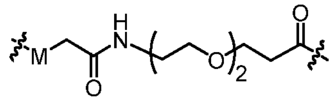 ,

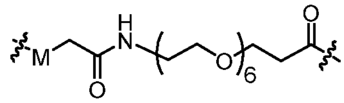 , 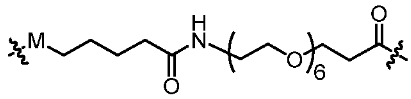 ,

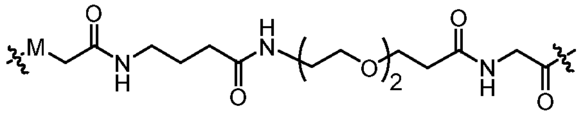 , 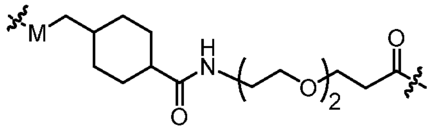 ,

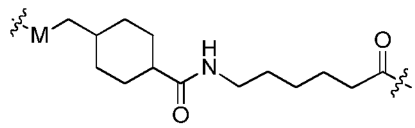 , 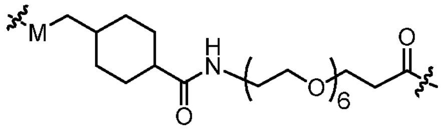 ,

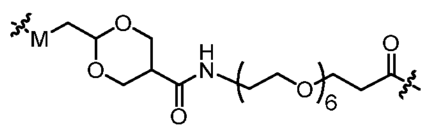 , 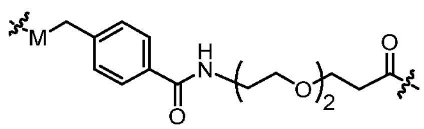 ,

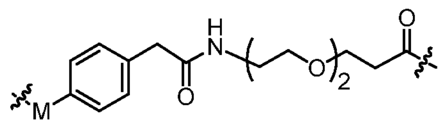 , 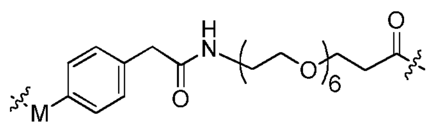 ,

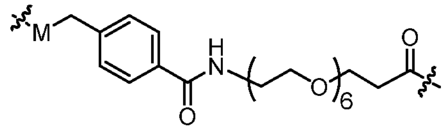 , 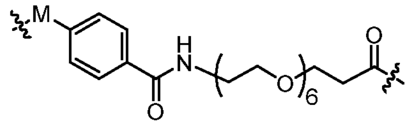 ,

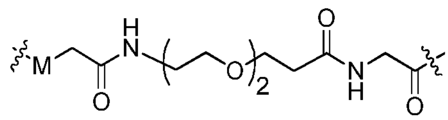 , 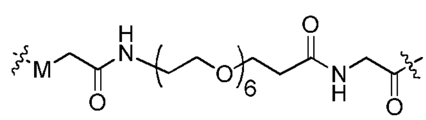 ,

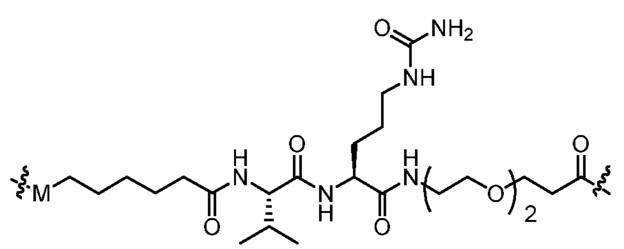 , 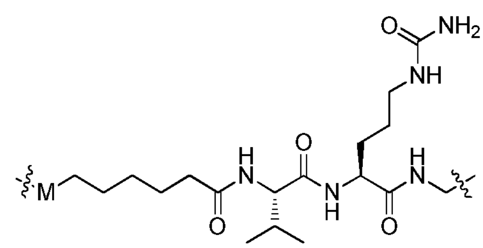 ,

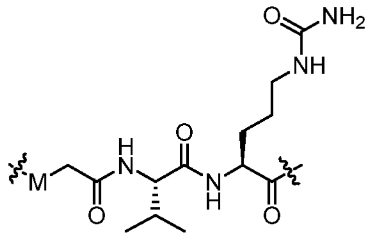
,
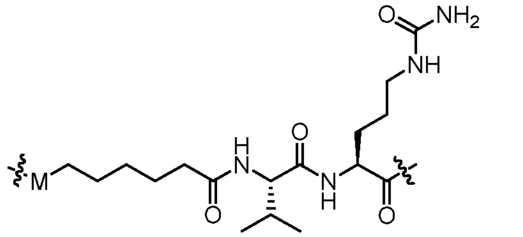
,
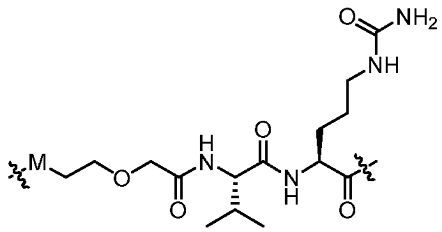
,
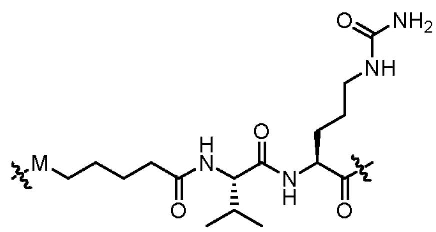
,
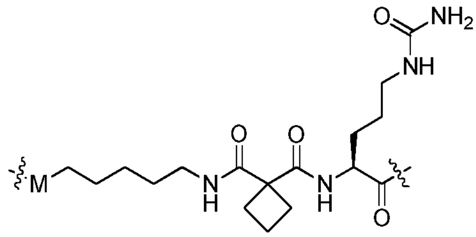
,
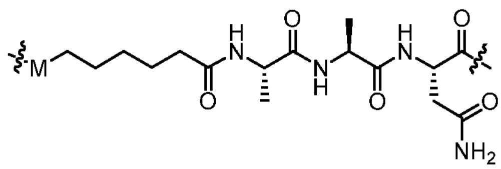
,
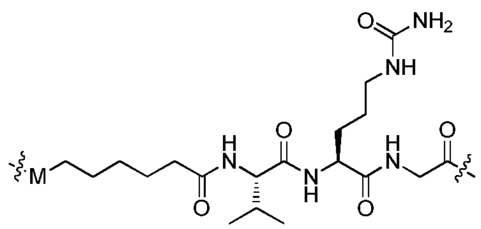
,
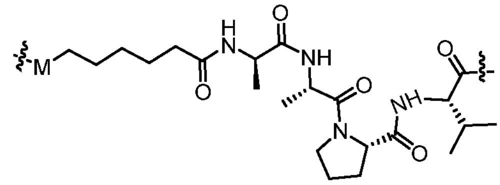
,
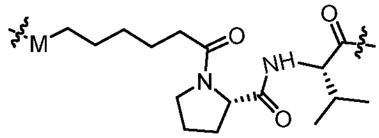
,
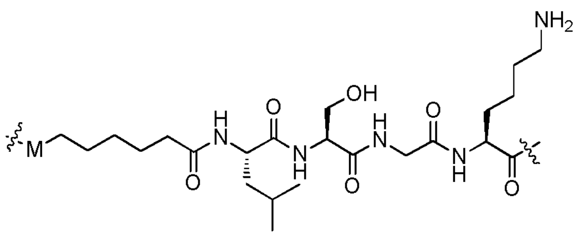
,
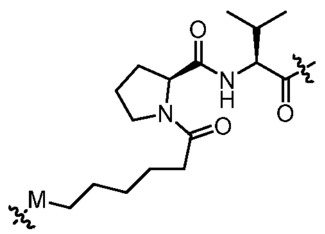
,
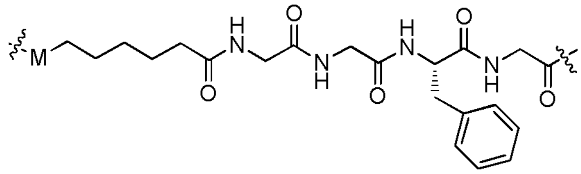
,

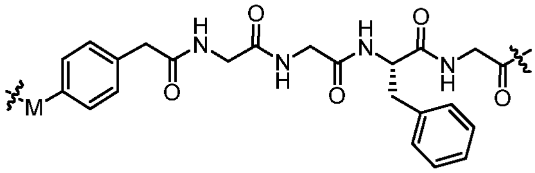
,
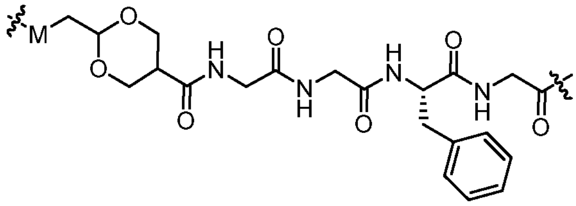
,

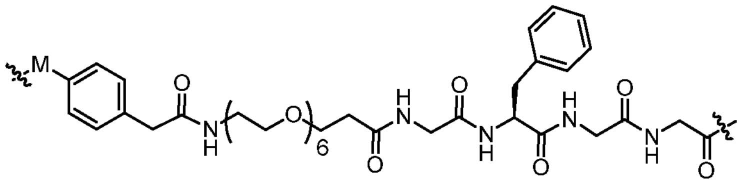
,

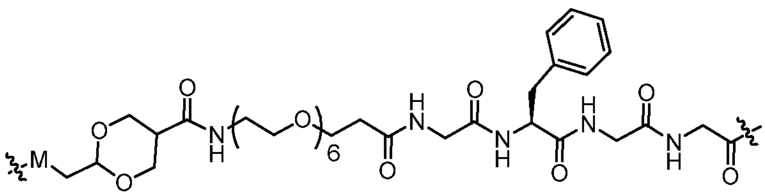
,
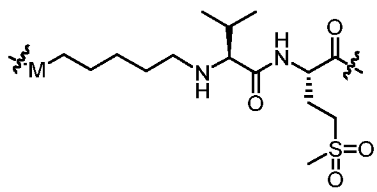
,

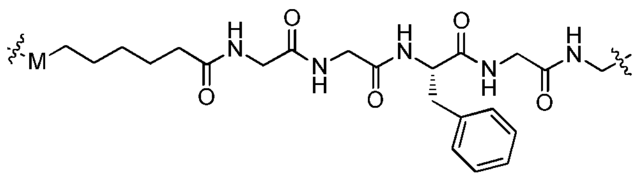
,
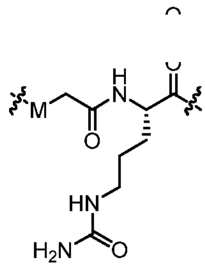
,

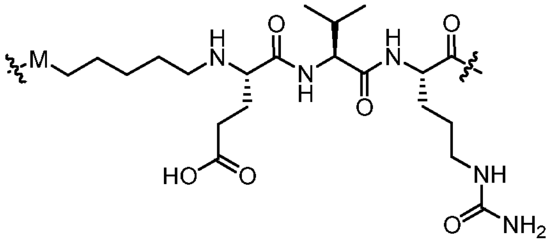
,
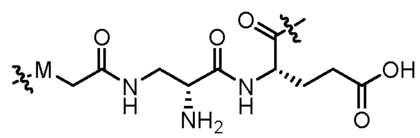
,

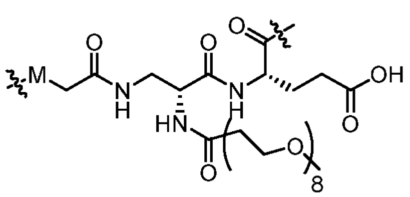
,
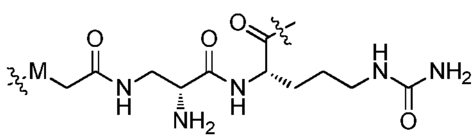
,

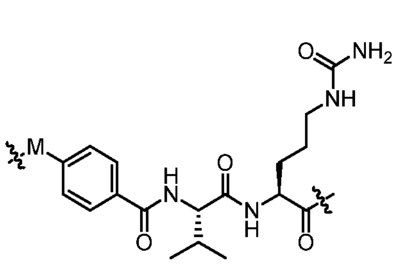
,
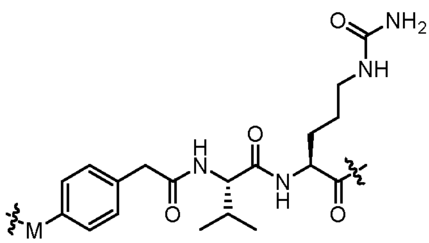
,

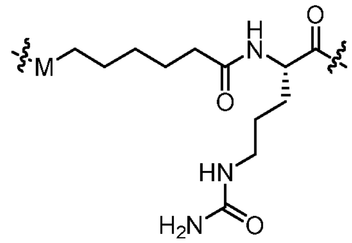

,

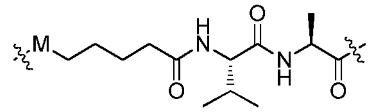

,

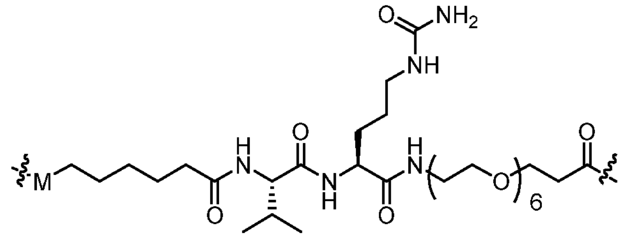

,

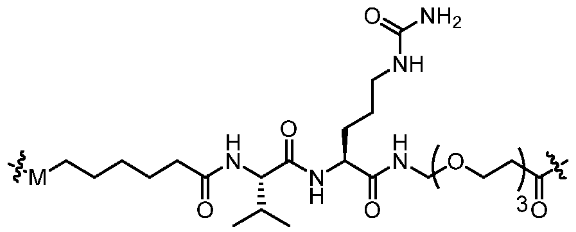

,

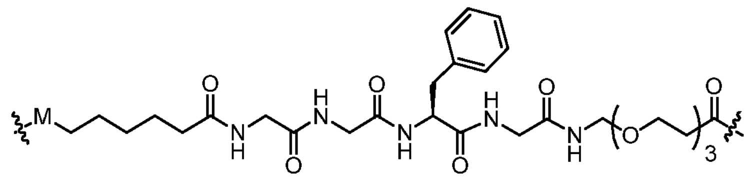

,

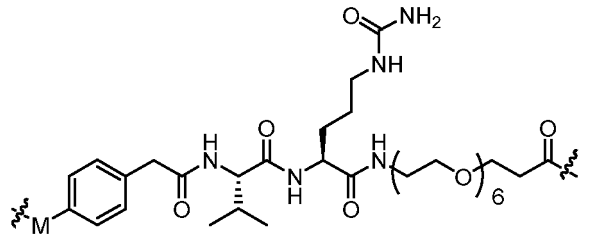

,

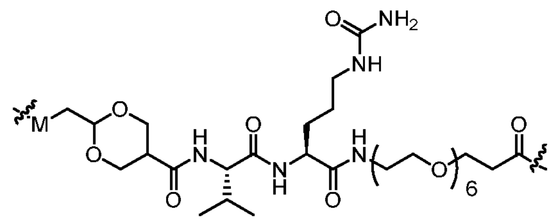

,

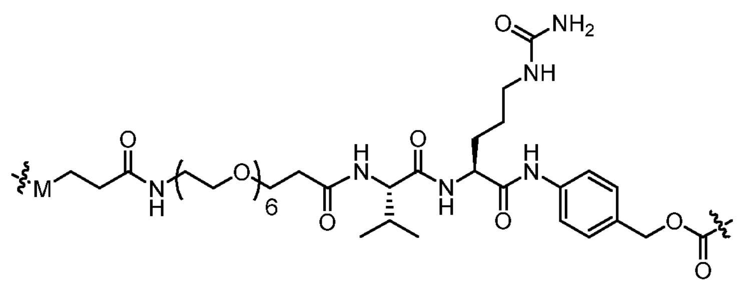

,

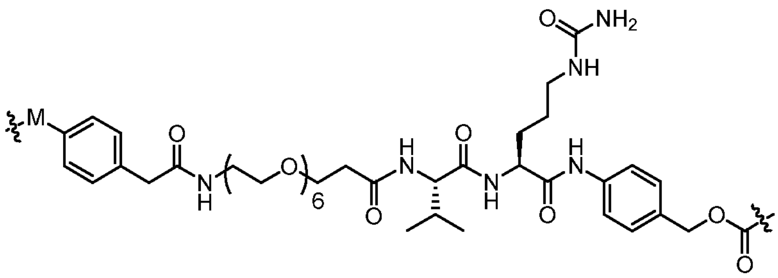

,

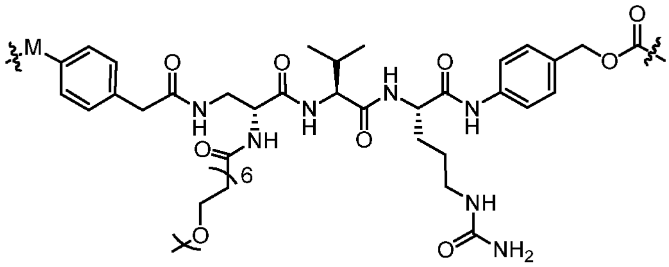

,

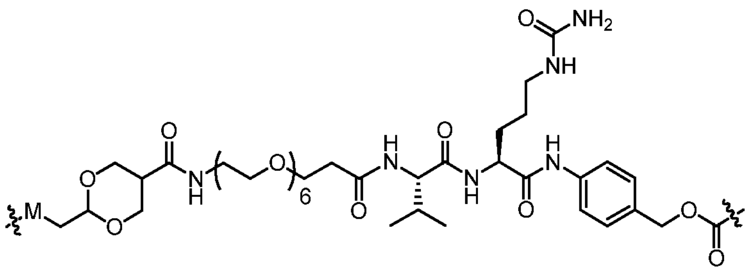

,

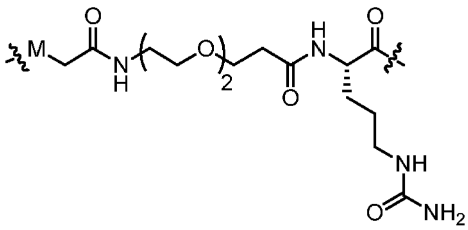

,

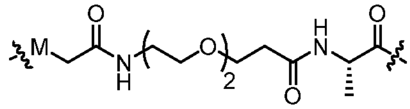

,

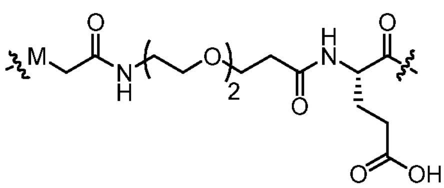

,

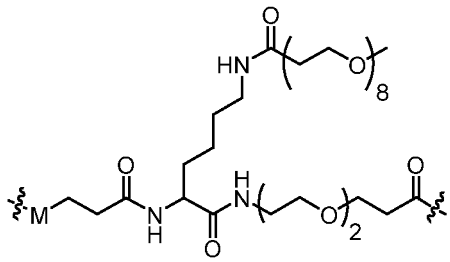

,

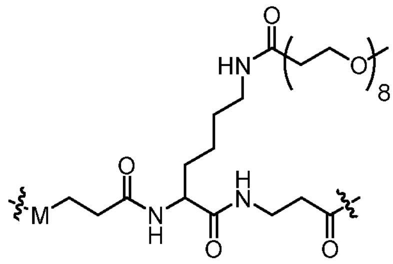
,
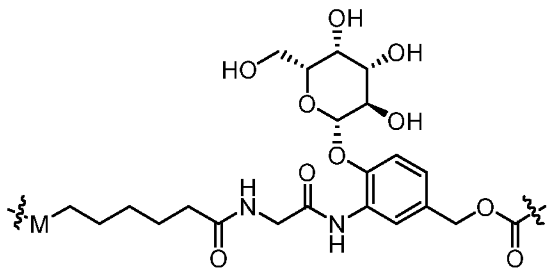
,
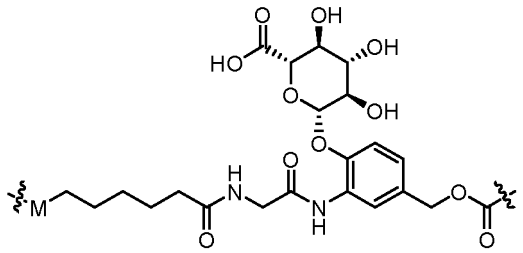
,
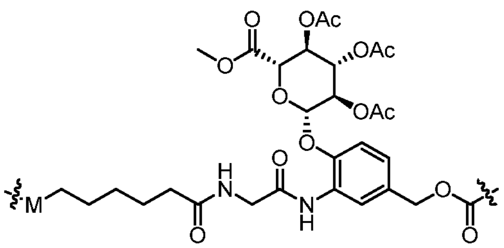
,
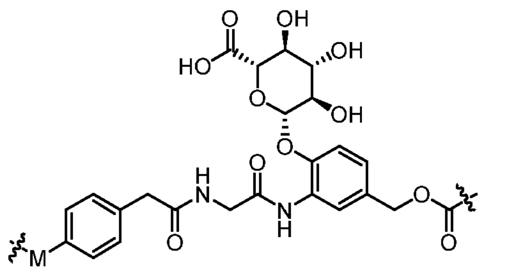
,
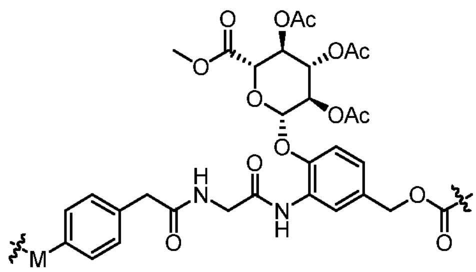
,
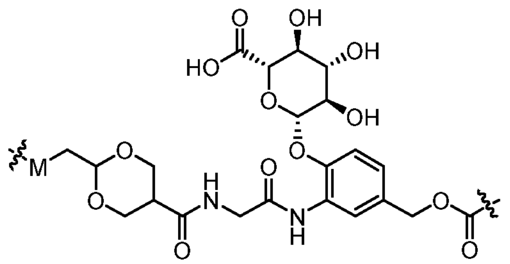
,
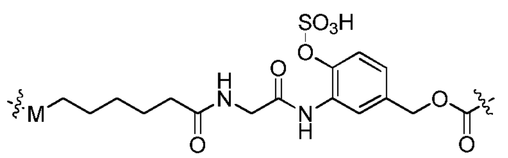
,
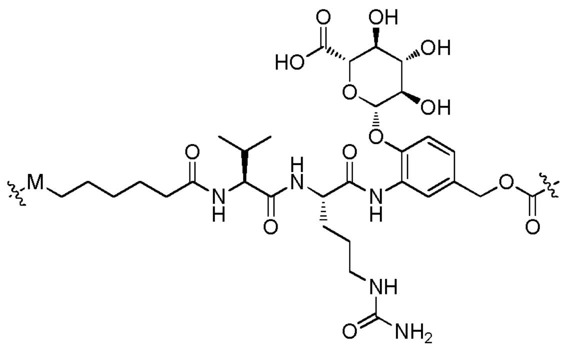
,
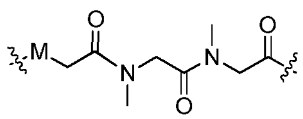
,

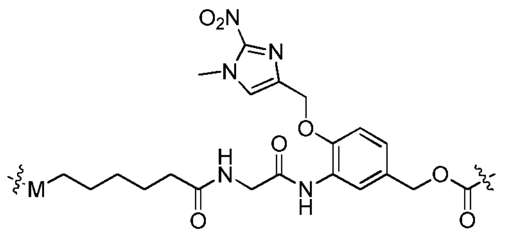,

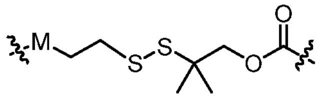,

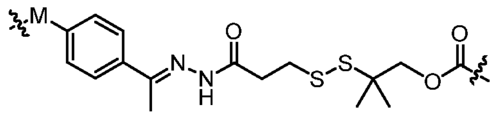,

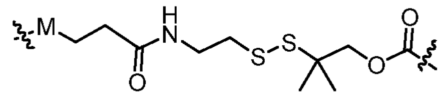,

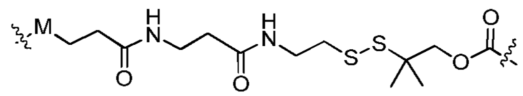,

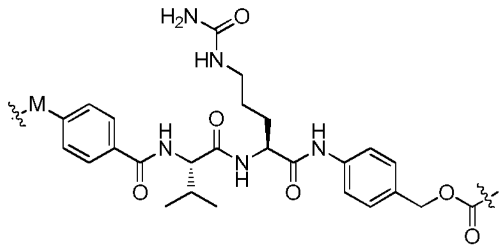,

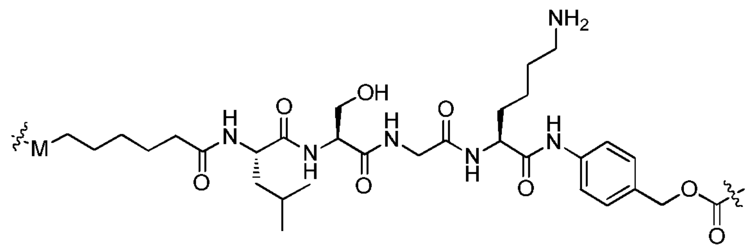,

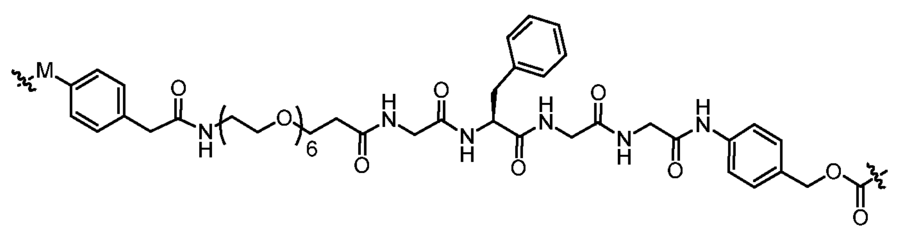,

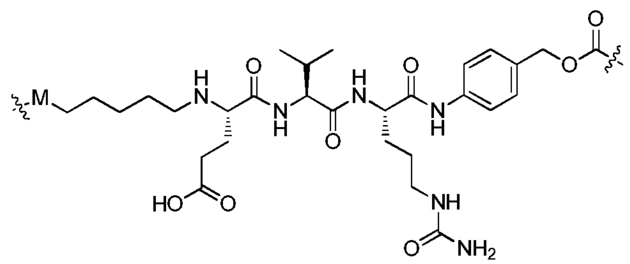,

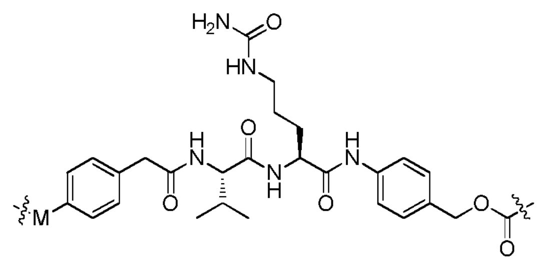,

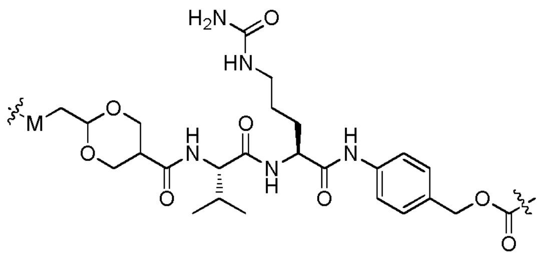
,
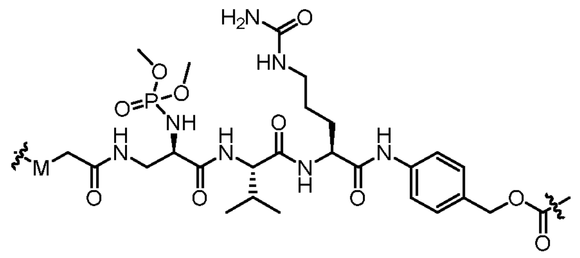
,

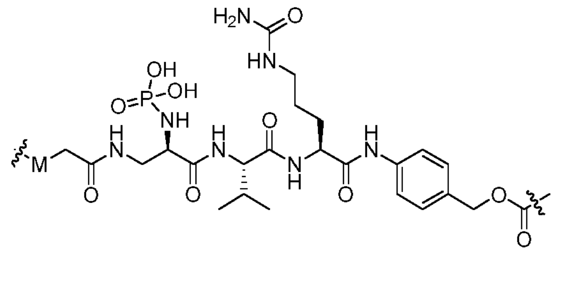
,

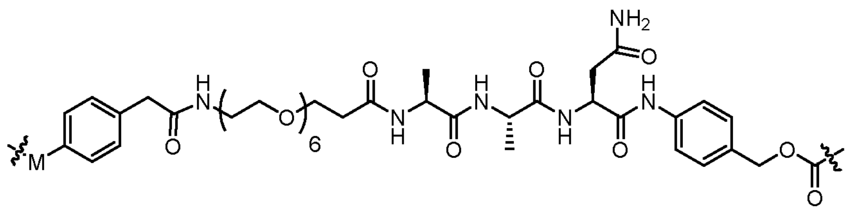
,

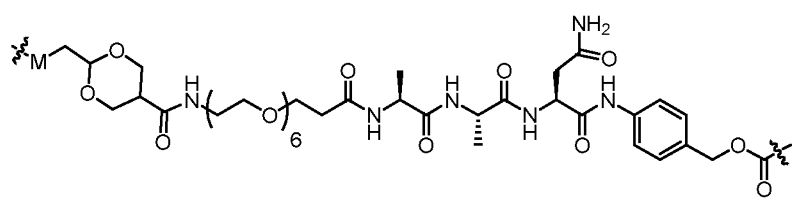
,

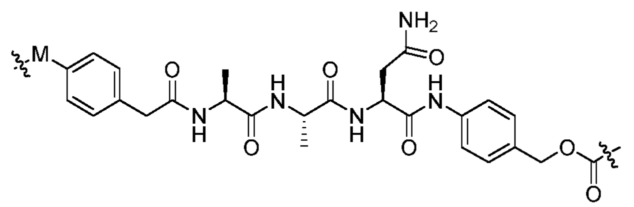
,

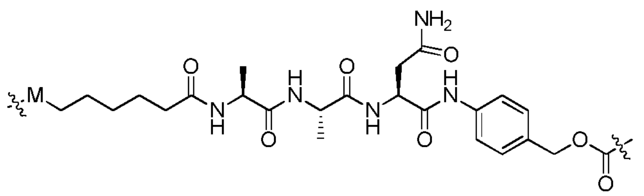
,

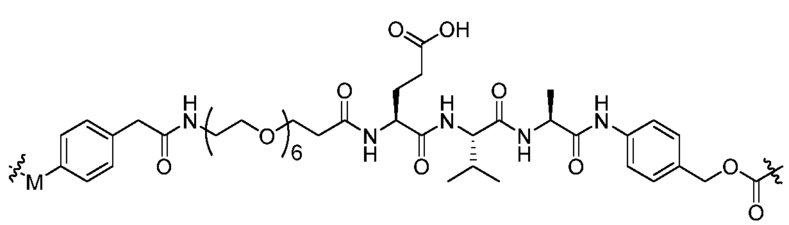

or

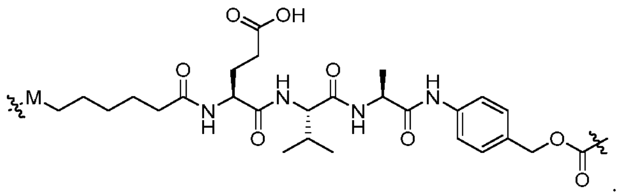

.

In some embodiments, L is

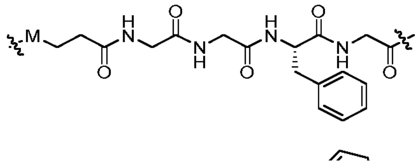

,

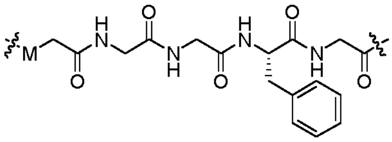

,

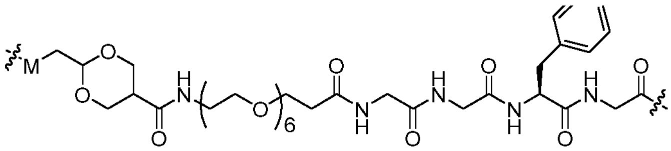

,

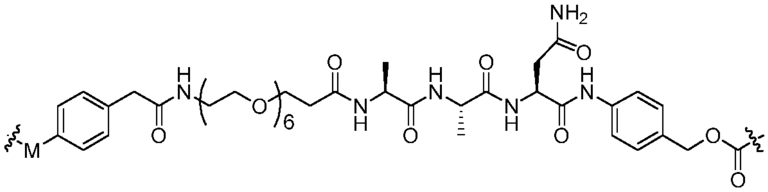

,

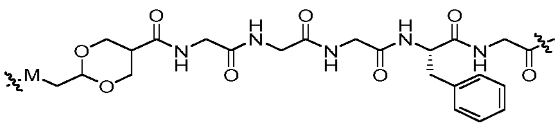

,

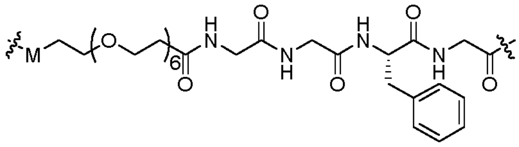

,

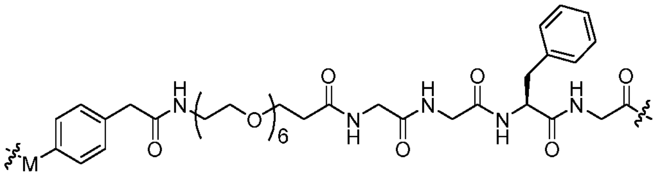

,

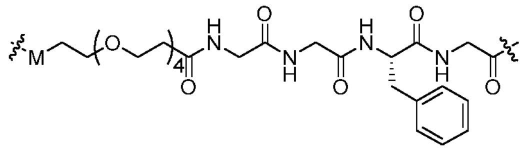

,

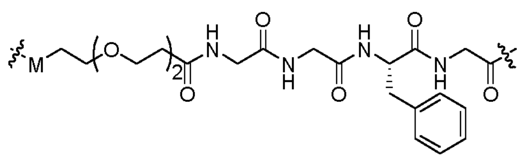

,

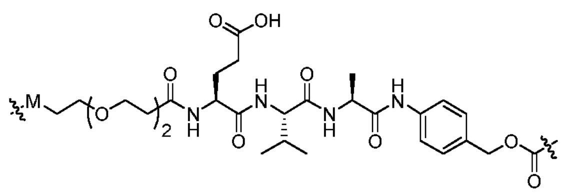

,

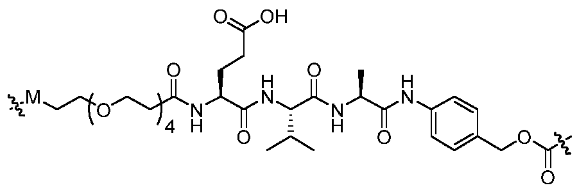, 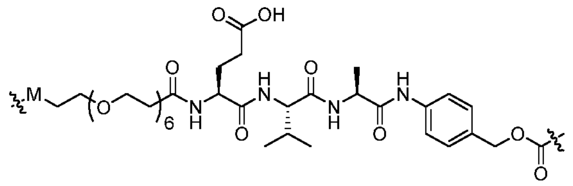,

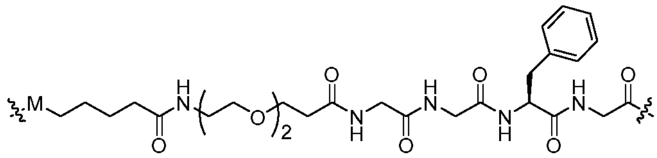, 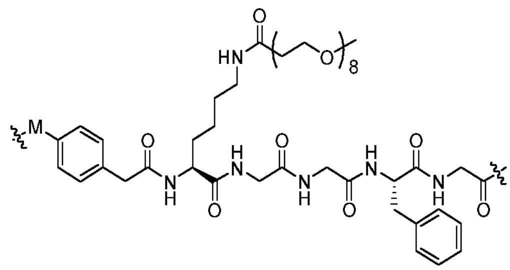,

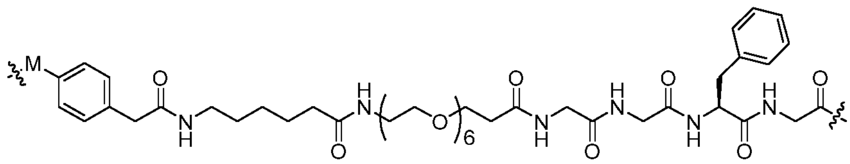, 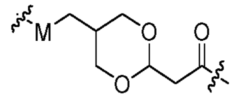,

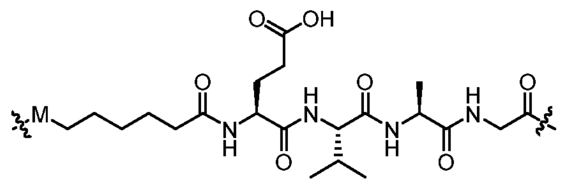, 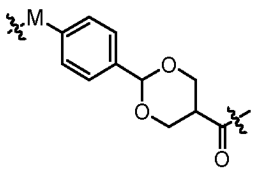, 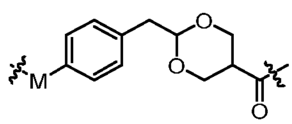,

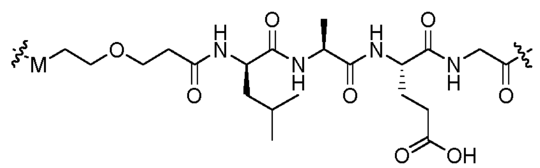, 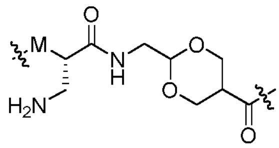,

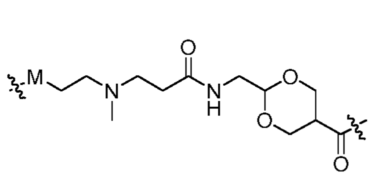, 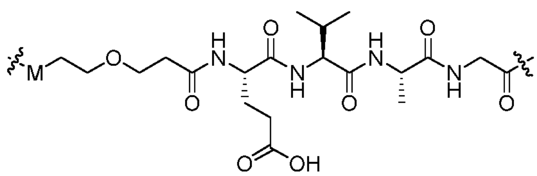, 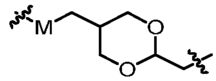,

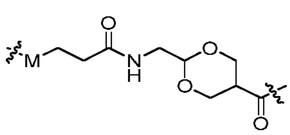 or 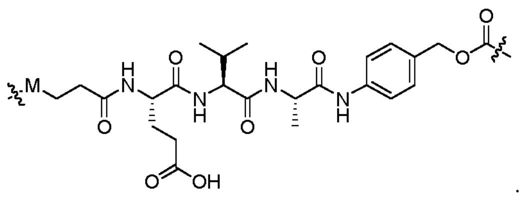.

In some embodiments, for the antibody-immunostimulatory conjugate of formula I or the pharmaceutically acceptable salt thereof,

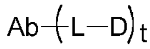

(I)

Ab is Pertuzumab, Cetuximab, huA1, hRS7, Zolbetuximab, Labetuzumab or Enfortumab;
t is any numerical value from 1 to 8;
L is a linker (e.g. $-M-(CH_2)_x-C(O)-(A)_v-L_5-$, $-M-(CH_2)_x-C(O)NH-PEG-$, $-M-(CH_2)_x-C(O)-(A)_v-$ or -M-(phenylene)-(5-6 membered heterocyclylene)-C(O)-) linking the Ab and the D;
the D is a group formed by loss of one hydrogen atom in a compound of formula D';

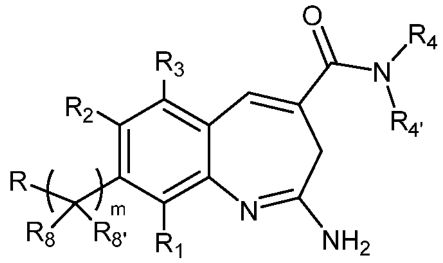

D'

where R is $-L'-L_1-R_7$; and L' is $-C(=O)-NR_9-$, $R_9$ is hydrogen, and $L_1$ is independently a linkage bond;
$R_7$ is independently an 8-12 membered fused ring group or an 8-12 membered fused ring group substituted with 1, 2 or 3 $R_{7-4}$; and
$R_{7-4}$ is independently $-C(=O)-L_3-NH_2$, $L_3$ is independently $C_{1-6}$ alkylene or $-(CH_2CH_2O)_{m'}-C_{1-6}$ alkylene-; and m' is 1, 2, 3, 4, 5, 6, 7, 8, 9 or 10.

In some embodiments, for the antibody-immunostimulatory conjugate of formula I or the pharmaceutically acceptable salt thereof, the antibody-immunostimulatory conjugate is selected from Tables 10, 11, 12, 13 or 14.

The present disclosure provides a compound of formula II or a pharmaceutically acceptable salt thereof,

D-LX (II)

where D is a benzazepine group as shown below:

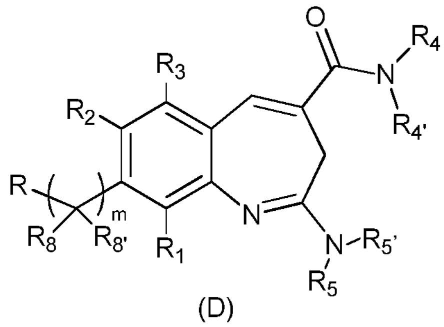

(D) ;

m is 0 or 1;
R is $-C(O)-NR_9-L_1-R_7$, $-C(S)-NR_9-L_1-R_7$ or $-NR_9-C(O)-L_1-R_7$;
$R_1$, $R_2$ and $R_3$ are each independently hydrogen, deuterium, halogen, hydroxyl, amino, cyano, alkyl, haloalkyl, $-L_2-OR_a$ or $-L_2-NR_aR_b$;
$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl or heteroarylalkyl; and the $R_4$ or $R_{4'}$ is unsubstituted or optionally substituted at any position with one or more substituents selected from halogen, cyano, $-L_2-OR_a$, $-L_2-OR_g$, $-L_2-OC(O)R_a$, $-L_2-OC(O)OR_a$, $-L_2-OC(O)NR_aR_b$, $-L_2-NR_aR_b$, $-L_2-NR_aC(O)OR_b$, $-L_2-NR_aC(O)NR_aR_b$, $-L_2-NR_bC(NR_b)NR_aR_b$ and $-L_2-C(O)OR_b$; or $R_4$ and $R_{4'}$ together with the N atom to which they are attached form 3-8 membered heterocycloalkyl; and the 3-8 membered heterocycloalkyl is unsubstituted or further substituted at any position with 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)$

$NR_aR_b$, $-L_2-NR_bC(NR_b)NR_aR_b$ and $-L_2-C(O)OR_b$;
$R_5$ is hydrogen, $-OR_a$, $-C(O)R_a$, $-C(O)OR_a$ or $-C(O)NR_aR_b$;
$R_{5'}$ is $-R_c$;
$R_7$ is phenyl, 5-6 membered heteroaryl, 3-8 membered heterocycloalkyl or an 8-12 membered fused ring group; and the $R_7$ is unsubstituted or optionally substituted at any position with one or more substituents selected from $-L_3-W$, $-R_c$, halogen, cyano, nitro, alkyl, alkenyl, alkynyl, alkoxy, haloalkyl, haloalkoxy and alkylamino;
$R_8$ and $R_{8'}$ are each independently hydrogen, halogen or alkyl, and the $R_8$ or $R_{8'}$ is unsubstituted or optionally substituted at any position with one or more substituents selected from $-L_3-W$, halogen, cyano, nitro, alkyl, alkenyl, alkynyl, alkoxy, haloalkyl, haloalkoxy and alkylamino; $R_8$ and $R_{8'}$ are each independently a substituent, or $R_8$ and $R_{8'}$ together with the carbon atom to which they are attached form oxo, thio, $C_{1-6}$ alkylene, $C_{3-10}$ cycloalkyl or 3-10 membered heterocycloalkyl; and the $C_{1-6}$ alkylene, $C_{3-10}$ cycloalkyl or 3-10 membered heterocycloalkyl is unsubstituted or optionally substituted at any position with one or more substituents selected from deuterium, halogen, hydroxyl, amino, cyano, oxo, alkyl, haloalkyl, $- L_2-OR_a$ and $-L_2-NR_aR_b$;
$R_9$ is hydrogen, alkyl, haloalkyl, $-L_2-OR_a$ or $-L_2-NR_aR_b$;
W is $Cy^1$, $-SR_d$, $-OR_d$, $-OC(O)R_e$, $-OC(O)NR_eR_{e'}$, $-C(O)OR_e$, $-C(O)R_e$, $-C(0)NR_eR_{e'}$, $- C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $- NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $- P(O)(OR_e)R_{e'}$, $-OP(O)(OR_e)_2$ or $-B(OR_e)_2$;
$Cy^1$ is cycloalkyl, heterocycloalkyl, aryl or heteroaryl; and the $Cy^1$ is unsubstituted or optionally substituted at any position with one or more substituents selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$ and $-L_4-NR_eR_{e'}$;
each $R_a$, $R_b$, $R_d$, $R_e$ and $R_{e'}$ is independently $-R_c$, amino, $C_{1-6}$ alkyl, $C_{2-6}$ alkenyl, $C_{2-6}$ alkynyl, $C_{3-10}$ cycloalkyl, 3-10 membered heterocycloalkyl, $C_{6-10}$ aryl, 5-10 membered heteroaryl, $C_{3-10}$ cycloalkyl $C_{1-6}$ alkyl, 3-10 membered heterocycloalkyl $C_{1-6}$ alkyl, phenyl $C_{1-6}$ alkyl or 5-10 membered heteroaryl $C_{1-6}$ alkyl; and the $R_a$, $R_b$, $R_d$, $R_e$ or $R_{e'}$ is unsubstituted or optionally substituted at any position with 1 to 3 substituents selected from $-L_4-OR_f$, $-OC(O)-L_4-R_f$, $-L_4-NR_fR_{f'}$, halogen, cyano, nitro, $C_{1-6}$ alkyl, halo $C_{1-6}$ alkyl and halo $C_{1-6}$ alkoxy;
$L_1$, $L_2$, $L_3$ and $L_4$ are each independently a linkage bond, $C_{1-6}$ alkylene, $C_{2-6}$ alkenylene, or $C_{2-6}$ alkynylene; the $L_1$, $L_2$, $L_3$ or $L_4$ is unsubstituted or optionally substituted at any position with one or more substituents selected from oxo, hydroxyl, amino, halogen, cyano, alkyl, haloalkyl, alkoxy and haloalkoxy;
each $R_f$ and $R_{f'}$ is independently $-R_c$, $-NHR_c$ or $C_{1-6}$ alkyl;
$R_g$ is a prodrug group including phosphoryl; and
each $R_c$ is independently hydrogen or a linkage bond attached to L; and at least one $R_c$ in the D is a linkage bond attached to LX;
and the benzazepine group of formula D satisfies 1 or 2 of conditions of:

(1) m is 1; and
(2) at least one of $R_4$ and $R_{4'}$ is substituted at any position with one or more substituents selected from halogen, cyano, $-L_2-OR_a$, $-L_2-OR_g$, $-L_2-OC(O)R_a$, $-L_2-OC(O)OR_a$, $-L_2-OC(O)NR_aR_b$, $-L_2-NR_aR_b$, $-L_2-NR_aC(O)OR_b$, $-L_2-C(O)OR_b$ $-L_2-NR_aC(O)NR_aR_b$ and $-L_2-NR_bC(NR_b)NR_aR_b$;

LX is $M'-(T)_w-PEG-(T)_o-*$, $M'-PEG-*$, $M'-(T)_w-*$, $M'-(T)_w-(A)_v-(T)_o-PEG-*$, $M'-(T)_w-(A)_v-(T)_o-*$, $M'-(T)_w-L_5-*$, $M'-(T)_w-PEG-L_5-*$, $M'-(T)_w-PEG-(A)_v-L_5-*$, $M'-(T)_w-PEG-(A)_v-(T)_o-*$, $M'-PEG-(T)_o-PEG-*$ or $M'-(T)_w-(A)_v-L_5-*$; v is any integer from 1 to 5; w is any integer from 1 to 10; o is any integer from 0 to 10; and * is a linking site of the LX that is attached to the D;
each T is independently $-(CH_2)_x-C(O)-$, $-NR_h-$, -O-, -S-, $-(CH_2)_x$-arylene-$(CH_2)_y-$, $-(CH_2)_x$-heteroarylene-$(CH_2)_y-$, $-(CH_2)_x$-cycloalkylene-$(CH_2)_y-$, $-(CH_2)_x$-heterocycloalkylene-$(CH_2)_y-$, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x$-S-S-alkylene-, alkylene or alkenylene; and x and y are each independently any integer from 0 to 10;
$R_h$ is hydrogen, alkyl, $-PO(OH)_2$, $-PO(OCH_3)_2$, $-C(O)-(CH_2CH_2O)_n-CH_3$ or $-(CH_2CH_2O)_n-CH_3$;
PEG is $-(CH_2CH_2O)_n-(CH_2)_u-$ or $-(CH_2CH_2O)_n-(CH_2)_u-C(O)-$, n is any integer from 1 to 50, and u is any integer from 0 to 5;
each A is independently an amino acid residue or an amino acid residue modified by $-C(O)-(CH_2CH_2O)_n-CH_3$ or $-(CH_2CH_2O)_n-CH_3$;
$L_5$ is a self-immolative group; and
M' is a linker head precursor or a linker head attached to 1 to 2 amino acids.

In some embodiments, in the compound of formula II or the pharmaceutically acceptable salt thereof, some groups are defined as below, and the remaining groups are defined as any other solution (hereinafter referred to as "in

some embodiments"):

D-LX (II)

where LX is a linker precursor; and
the D is a group formed by loss of one hydrogen atom in a compound of formula D';

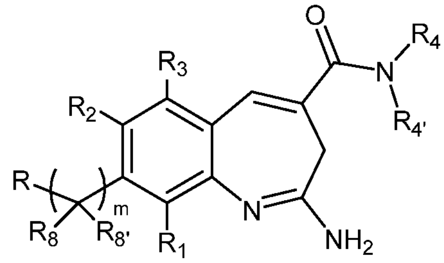

D'

where R is -L'-$L_1$-$R_7$; and L' is -C(=O)-, -C(=O)-$NR_9$- or -C(=S)-$NR_9$-;
$R_9$ is independently hydrogen, $C_{1-6}$ alkyl or halo $C_{1-6}$ alkyl;
$L_1$ is independently a linkage bond, $C_{1-6}$ alkylene or $C_{2-6}$ alkenylene;
$R_7$ is independently phenyl, 5-6 membered heteroaryl, 3-8 membered heterocycloalkyl, an 8-12 membered fused ring group, phenyl substituted with 1, 2 or 3 $R_{7-1}$, 5-6 membered heteroaryl substituted with 1, 2 or 3 $R_{7-2}$, 3-8 membered heterocycloalkyl substituted with 1, 2 or 3 $R_{7-3}$ or an 8-12 membered fused ring group substituted with 1, 2 or 3 $R_{7-4}$;
in the $R_7$, in "5-6 membered heteroaryl" in the 5-6 membered heteroaryl and the 5-6 membered heteroaryl substituted with 1, 2 or 3 $R_{7-2}$, the heteroatom is independently selected from one or more of N, O and S, and the number of the heteroatom is independently 1, 2 or 3; and in "3-8 membered heterocycloalkyl" in the 3-8 membered heterocycloalkyl and the 3-8 membered heterocycloalkyl substituted with 1, 2 or 3 $R_{7-3}$, the heteroatom is independently selected from one or more of N, O and S, and the number of the heteroatom is independently 1, 2 or 3;
in the $R_7$, an 8-12 membered fused ring group in the 8-12 membered fused ring group and the 8-12 membered fused ring group substituted with 1, 2 or 3 $R_{7-4}$ is independently a fused ring of ring A and ring B, the ring A is a 5-6 membered heteroaryl ring or a phenyl ring, and the ring B is a 5-6 membered heteroalkenyl ring; in the 5-6 membered heteroaryl ring, the heteroatom is selected from one or more of N, O and S, and the number of the heteroatom is 1, 2 or 3; and in the 5-6 membered heteroalkenyl ring, the heteroatom is selected from one or more of N, O and S, and the number of the heteroatom is 1, 2 or 3;
$R_{7-1}$, $R_{7-2}$, $R_{7-3}$ and $R_{7-4}$ are independently amino, -$L_3$-$NH_2$, -$L_3$-OH, -C(=O)-$L_3$-OH or -C(=O)-$L_3$-$NH_2$, and $L_3$ is independently $C_{1-6}$ alkylene or -$(CH_2CH_2O)_{m'}$-$C_{1-6}$ alkylene-; and m' is 1, 2, 3, 4, 5, 6, 7, 8, 9 or 10;
m is 0 or 1;
$R_8$ and $R_{8'}$ are independently hydrogen or $C_{1-6}$ alkyl, or $R_8$ and $R_{8'}$ together with the carbon atom to which they are attached form carbonyl, $C_{3-10}$ cycloalkylene or 3-10 membered heterocycloalkylene; and in the 3-10 membered heterocycloalkylene, the heteroatom is selected from one or more of N, O and S, and the number of the heteroatom is 1, 2 or 3;
$R_1$, $R_2$ and $R_3$ are independently hydrogen, deuterium or halogen;
$R_4$ and $R_{4'}$ are independently $C_{1-6}$ alkyl, $C_{1-6}$ alkoxy or $C_{1-6}$ alkyl substituted with 1, 2 or 3 $R_{4-1}$; and the $R_{4-1}$ is independently hydroxyl, amino, -OC(=O)$NR_aR_b$ or

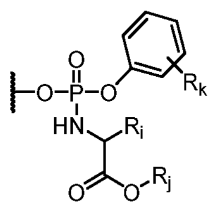
;

$R_a$ and $R_b$ are independently hydrogen, $C_{1-6}$ alkyl or $C_{3-10}$ cycloalkyl;
$R_i$ is hydrogen, $C_{1-6}$ alkyl or benzyl; $R_k$ is hydrogen, halogen or $C_{1-6}$ alkyl; and $R_j$ is $C_{1-6}$ alkyl, $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; and
in the $R_j$, in "3-10 membered heterocycloalkyl" in the 3-10 membered heterocycloalkyl and the 3-10 membered

heterocycloalkyl-$C_{1-6}$ alkyl, the heteroatom is independently selected from one or more of N, O and S, and the number of the heteroatom is independently 1, 2 or 3; and in "5-10 membered heteroaryl" in the 5-10 membered heteroaryl and the 5-10 membered heteroaryl-$C_{1-6}$ alkyl, the heteroatom is independently selected from one or more of N, O and S, and the number of the heteroatom is independently 1, 2 or 3;
and the compound of formula D' satisfies 1 or 2 of conditions of:

(1) m is 1; and
(2) at least one of $R_4$ and $R_{4'}$ is $C_{1-6}$ alkoxy or $C_{1-6}$ alkyl substituted with 1, 2 or 3 $R_{4-1}$;

and the compound of formula II is not of a structure shown in Table 6:

Table 6

| |
|---|
| 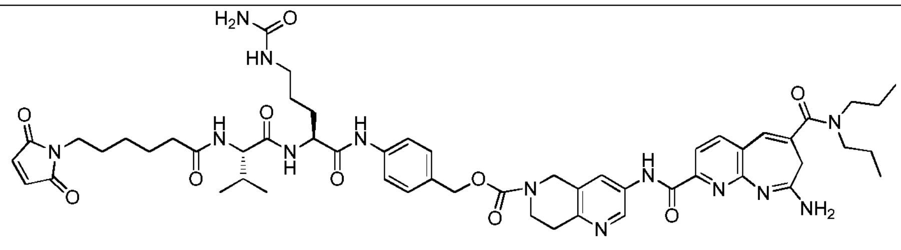 |
| 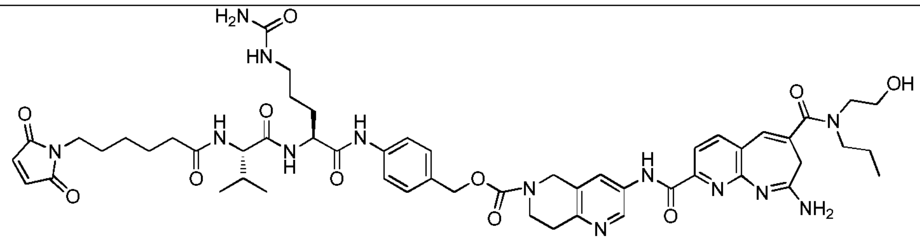 |
| 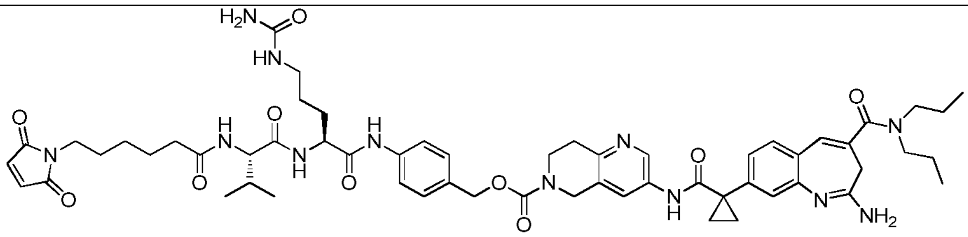 |

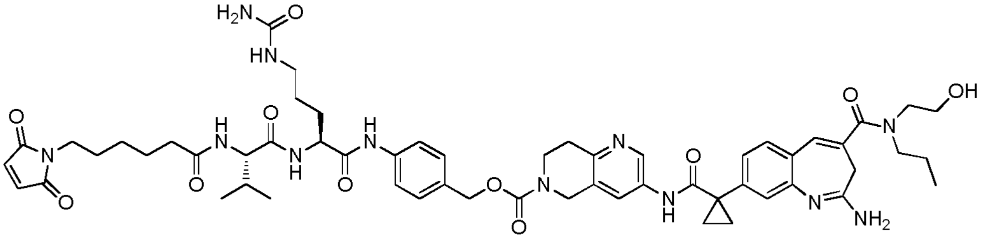

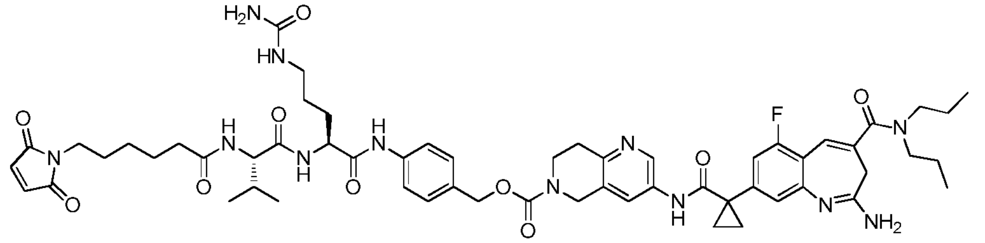

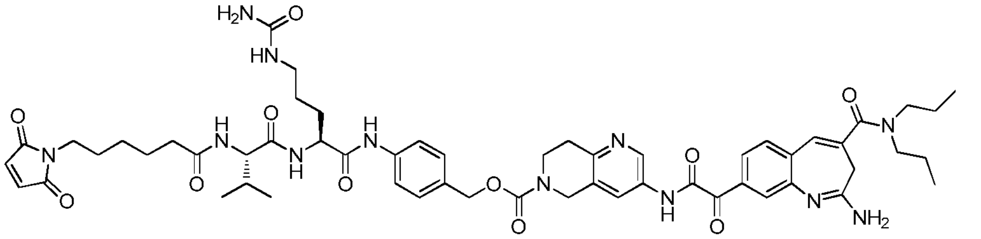

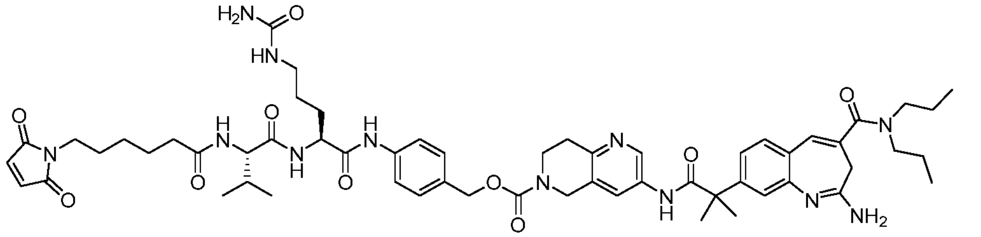

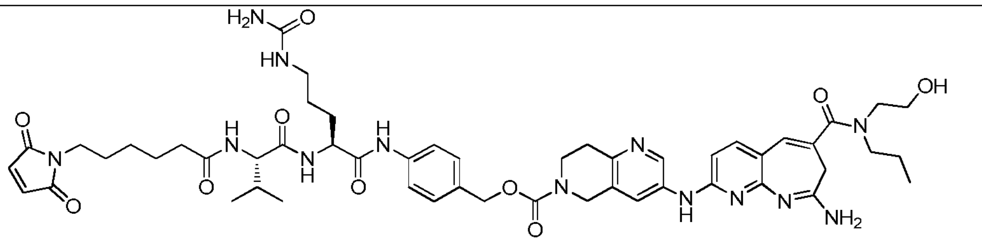

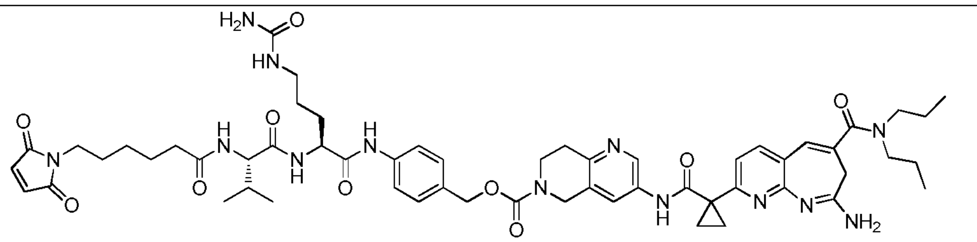

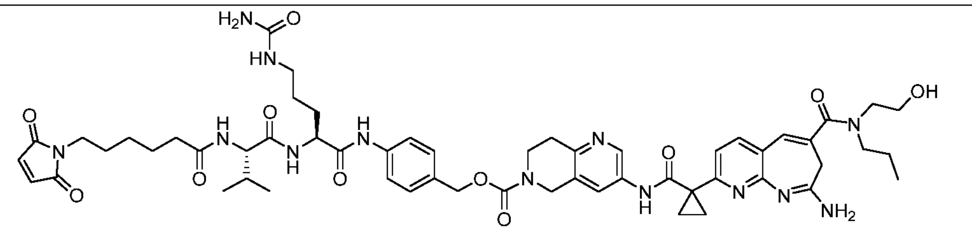

| |
|---|
| 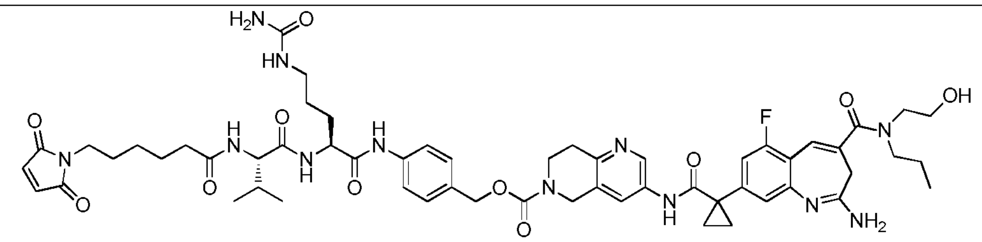 |
| 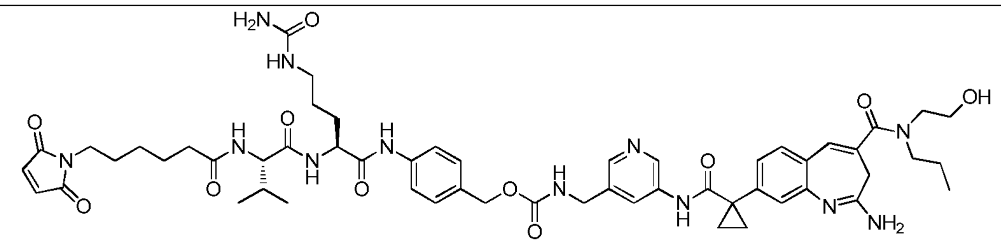 |
| 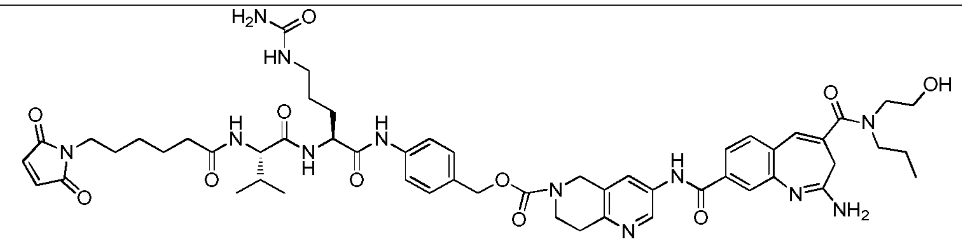 |
| 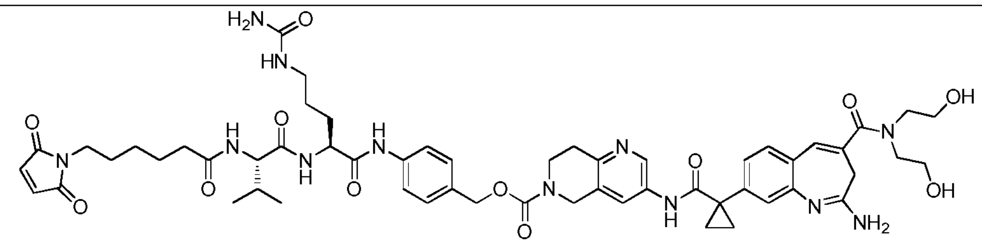 |
| 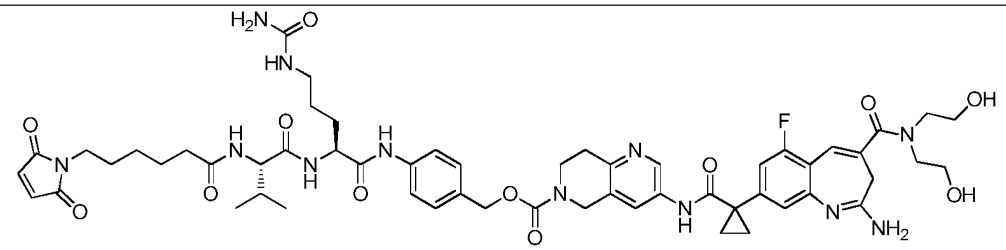 |
| 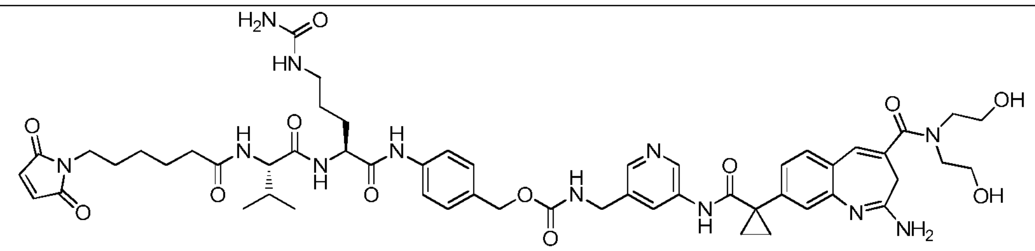 |
| 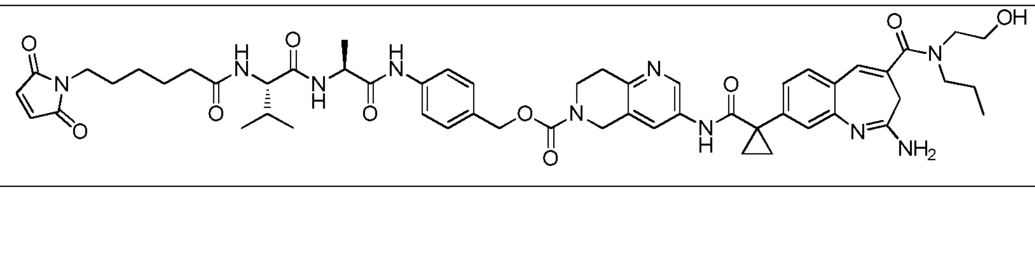 |

| |
|---|
| 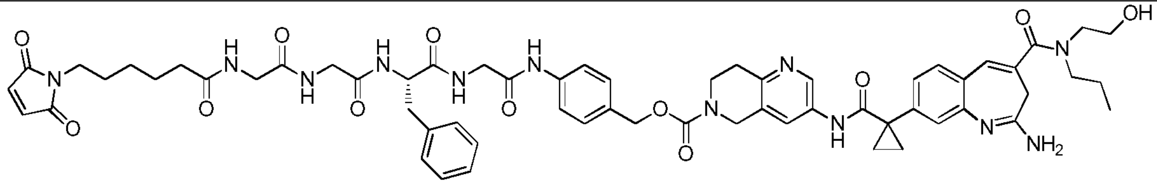 |
| 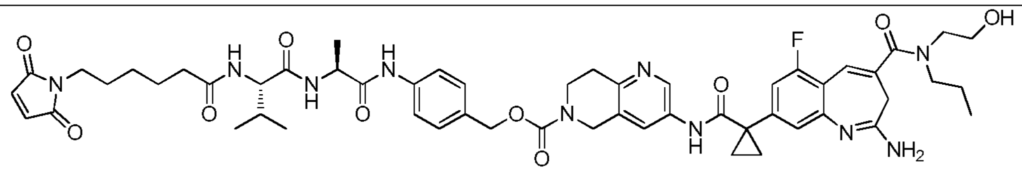 |
| 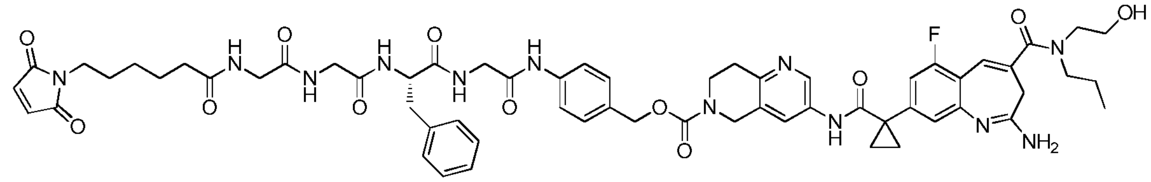 |
| 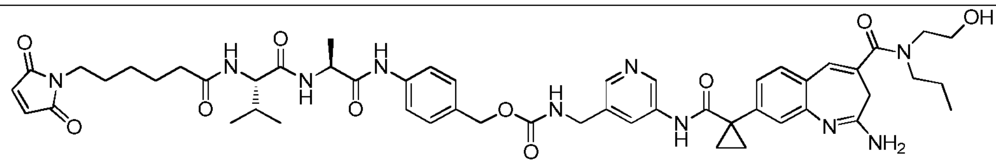 |
| 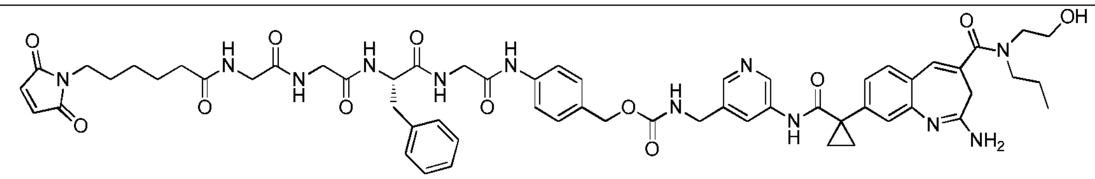 |
| 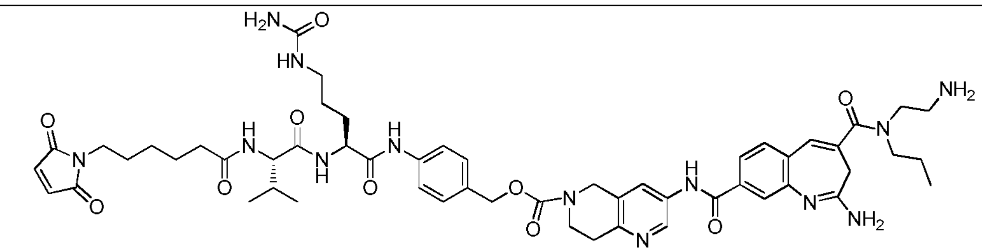 |
| 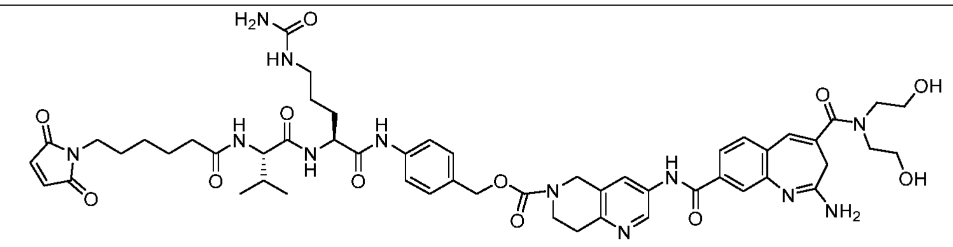 |

.

In some embodiments, the compound of formula D' is not a compound shown in Table 3.

In some embodiments, the compound of formula D' satisfies 1, 2 or 3 of conditions of:

(1) $L_1$ is independently $C_{1-6}$ alkylene or $C_{2-6}$ alkenylene;

(2) $R_7$ is independently phenyl substituted with one amino, 5-6 membered heteroaryl substituted with one amino, 3-8 membered heterocycloalkyl substituted with one amino or an 8-12 membered fused ring group substituted with one amino; and

(3) at least one of $R_4$ and $R_{4'}$ is $C_{1-6}$ alkoxy or $C_{1-6}$ alkyl substituted with 1, 2 or 3 $R_{4-1}$; and the $R_{4-1}$ is independently $-OC(=O)NR_aR_b$ or

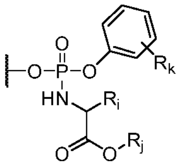.

In some embodiments, the LX is not of a structure of:

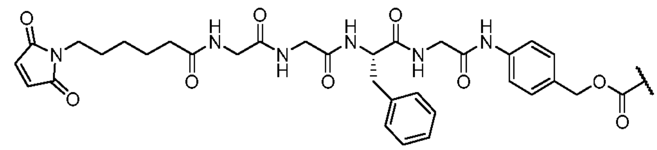,

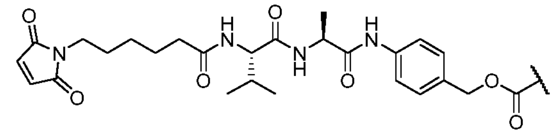 or 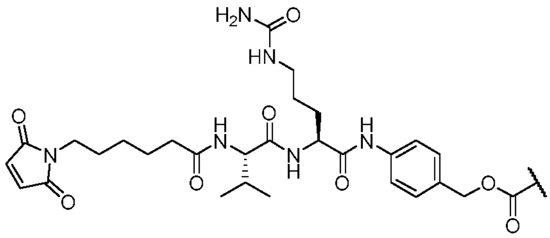.

In some embodiments, the LX is M'-$(T)_w$-PEG-$(T)_o$-*, M'-PEG-*, M'-$(T)_w$-$(A)_v$-$(T)_o$-PEG-*, M'-$(T)_w$-$(A)_v$-$(T)_o$-*, M'-$(T)_w$-$L_5$-*, M'-$(T)_w$-PEG-$L_5$-*, M'-$(T)_w$-PEG-$(A)_v$-$L_5$-*, M'-$(T)_w$-PEG-$(A)_v$-$(T)_o$-* or M'-PEG-$(T)_o$-PEG-*; and * is a linkage site of the LX and the D;

M' is hydrogen, a linker head precursor or a linker head attached to 1 to 2 amino acids;
w is independently 1, 2, 3, 4, 5, 6, 7, 8, 9 or 10;
o is independently 0, 1, 2, 3, 4, 5, 6, 7, 8, 9 or 10;
T is independently $-(CH_2)_x-C(O)-$, $-NR_h-$, -O-, -S-, $-(CH_2)_x-C_{6-14}$ arylene-$(CH_2)_y$-, $-(CH_2)_x$-5-6 membered heteroarylene-$(CH_2)_y$-, $-(CH_2)_x-C_{3-6}$ cycloalkylene-$(CH_2)_y$-, $-(CH_2)_x$-3-6 membered heterocycloalkylene-$(CH_2)_y$-, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x-S-S-C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the $-(CH_2)_x$-5-6 membered heteroarylene-$(CH_2)_y$-, the heteroatom is selected from one or more of N, O and S, and the number of the heteroatom is 1, 2 or 3; and in "3-6 membered heterocycloalkylene" in the $-(CH_2)_x$-3-6 membered heterocycloalkylene-$(CH_2)_y$-, the heteroatom is selected from one or more of N, O and S, and the number of the heteroatom is 1, 2 or 3;
$R_h$ is independently hydrogen, $C_{1-6}$ alkyl, $-PO(OH)_2$, $-PO(OCH_3)_2$, $-C(O)-(CH_2CH_2O)_n-CH_3$ or - $(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;
PEG is $-(CH_2CH_2O)_n-(CH_2)_u-$ or $-(CH_2CH_2O)_n-(CH_2)_u-C(O)-$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;
v is independently 1, 2, 3, 4 or 5;
A is independently an amino acid residue or an amino acid residue substituted with 1 $R_A$; the $R_A$ is independently $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and
$L_5$ is a self-immolative group.

In the compound of formula II or the pharmaceutically acceptable salt thereof, a left end or a right end of L' is attached to $L_1$.
In some embodiments, L' is -C(=O)-, $-C(=O)-NR_9-$ or $-C(=S)-NR_9-$, and a right end thereof is attached to $L_1$.
In some embodiments, in the $L_1$, the $C_{1-6}$ alkylene is methylene or ethylene.
In some embodiments, in the $R_7$, in "5-6 membered heteroaryl" in the 5-6 membered heteroaryl and the 5-6 membered heteroaryl substituted with 1, 2 or 3 $R_{7-2}$, the heteroatom is independently N.

In some embodiments, in the $R_7$, in "5-6 membered heteroaryl" in the 5-6 membered heteroaryl and the 5-6 membered heteroaryl substituted with 1, 2 or 3 $R_{7-2}$, the number of the heteroatom is independently 1 or 2.

In some embodiments, in the $R_7$, in "5-6 membered heteroaryl" of the 5-6 membered heteroaryl and the 5-6 membered heteroaryl substituted with 1, 2 or 3 $R_{7-2}$, the 5-6 membered heteroaryl may be pyridinyl, pyrimidinyl, pyridazinyl or pyrazinyl.

In some embodiments, in the ring A, the heteroatom in the 5-6 membered heteroaryl ring is N.

In some embodiments, in the ring A, the number of the heteroatom in the 5-6 membered heteroaryl ring is 1 or 2.

In some embodiments, in the ring A, the 5-6 membered heteroaryl ring may be a pyridine ring, a pyrimidine ring, a pyridazine ring or a pyrazine ring.

In some embodiments, in the ring B, the heteroatom in the 5-6 membered heteroalkenyl ring is N.

In some embodiments, in the ring B, the number of the heteroatom in the 5-6 membered heteroalkenyl ring is 1 or 2.

In some embodiments, the $R_7$ is

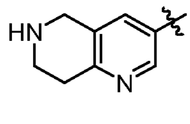,
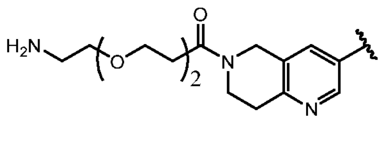,
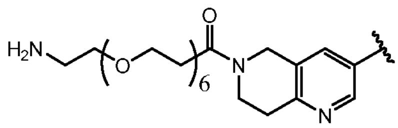,
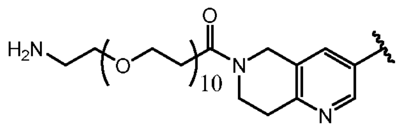,
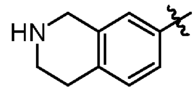,
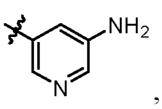,
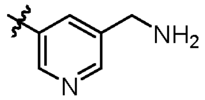,
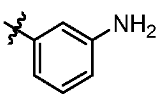,
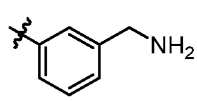 or
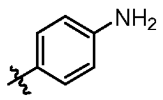.

In some embodiments, one hydrogen atom lost by the compound of formula D' is located on a secondary amine or a primary amine of $R_7$.

In some embodiments, the $R_7$ is

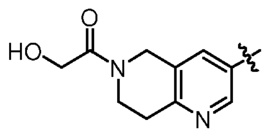 or
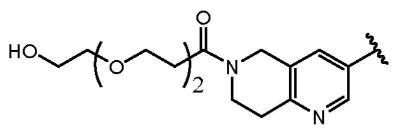.

In some embodiments, one hydrogen atom lost by the compound of formula D' is located on hydroxyl of $R_7$.

In some embodiments, in the $R_8$ and $R_{8'}$, the $C_{1-6}$ alkyl may be methyl, ethyl, n-propyl or isopropyl.

In some embodiments, $R_8$ and $R_{8'}$ together with the carbon atom to which they are attached form $C_{3-10}$ cycloalkylene, and the $C_{3-10}$ cycloalkylene is cyclopropyl or cyclobutyl.

In some embodiments, $R_8$ and $R_{8'}$ together with the carbon atom to which they are attached form 3-10 membered heterocycloalkylene, and a heteroatom in the 3-10 membered heterocycloalkylene is N or O.

In some embodiments, $R_8$ and $R_{8'}$ together with the carbon atom to which they are attached form 3-10 membered heterocycloalkylene, and the number of the heteroatom in the 3-10 membered heterocycloalkylene is 1 or 2.

In some embodiments, $R_8$ and $R_{8'}$ together with the carbon atom to which they are attached form 3-10 membered heterocycloalkylene, and the 3-10 membered heterocycloalkylene may be oxacyclopropylene, azacyclopropylene or oxacyclobutylene or azacyclobutylene.

In some embodiments, in $R_1$, $R_2$ and $R_3$, the halogen is F, Cl or Br.

In some embodiments, in the $R_4$ and $R_{4'}$, the $C_{1-6}$ alkyl may be methyl, ethyl, n-propyl or isopropyl.

In some embodiments, in the $R_4$ and $R_{4'}$, the $C_{1-6}$ alkoxy may be methoxy, ethoxy, n-propoxy or isopropoxy.

In some embodiments, in the $R_4$ and $R_{4'}$, $C_{1-6}$ alkyl in the $C_{1-6}$ alkyl substituted with 1, 2 or 3 $R_{4-1}$ may be methyl, ethyl, n-propyl or isopropyl.

In some embodiments, in the $R_a$ and $R_b$, the $C_{1-6}$ alkyl may be methyl, ethyl, n-propyl or isopropyl.

In some embodiments, in the $R_a$ and $R_b$, the $C_{3-10}$ cycloalkyl may be cyclopropyl, cyclobutyl or cyclopentyl.

In some embodiments, in the $R_i$, the $C_{1-6}$ alkyl may be methyl, ethyl, n-propyl or isopropyl.

In some embodiments, in the $R_k$, the $C_{1-6}$ alkyl may be methyl, ethyl, n-propyl or isopropyl.

In some embodiments, in the $R_j$, the $C_{1-6}$ alkyl may be methyl, ethyl, n-propyl or isopropyl.

In some embodiments, in the $R_j$, in the $C_{6-10}$ aryl-$C_{1-6}$ alkyl, the $C_{6-10}$ aryl may be phenyl.

In some embodiments, in the $R_j$, in the $C_{6-10}$ aryl-$C_{1-6}$ alkyl, the $C_{1-6}$ alkyl may be methyl, ethyl, n-propyl or isopropyl.

In some embodiments, $R_4$ and $R_{4'}$ are independently $-OCH_2CH_3$, $-CH_2CH_2NH_2$, $-CH_2CH_2OH$ or - $CH_2CH_2CH_3$.

In some embodiments, at least one of $R_4$ and $R_{4'}$ is $-OCH_2CH_3$, $-CH_2CH_2NH_2$ or $-CH_2CH_2OH$.

In some embodiments, m is 1.

In some embodiments, R is $-C(O)-NR_9-L_1-R_7$.

In some embodiments, $R_1$, $R_2$ and $R_3$ are each independently hydrogen, deuterium, halogen, hydroxyl, amino, cyano, $C_{1-6}$ alkyl, halo $C_{1-6}$ alkyl, $-L_2-OR_a$ or $-L_2-NR_aR_b$.

In some embodiments, $R_1$, $R_2$ and $R_3$ are each independently preferably H, F, Cl, Br, $-CH_3$, $-OCH_3$, $-CF_3$, $-CH_2F$, $-CHF_2$, $-OCF_3$, -CN or $-(CH_2)_{0-5}-NH_2$.

In some embodiments, $R_1$ is H; $R_2$ is H; and $R_3$ is H or F.

In some embodiments, in the $R_g$, the phosphoryl-containing prodrug group is

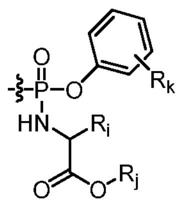 ;

$R_i$ is hydrogen, $C_{1-6}$ alkyl (e.g., methyl, ethyl, isobutyl), $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; $R_j$ is $C_{1-6}$ alkyl (e.g. methyl, ethyl, isopropyl, tert-butyl, isobutyl) or benzyl; and $R_k$ is hydrogen, halogen or $C_{1-6}$ alkyl.

In some embodiments, in the $R_g$, the phosphoryl-containing prodrug group is

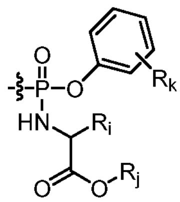 ;

$R_i$ is methyl; $R_j$ is isopropyl or benzyl; and $R_k$ is hydrogen.

In some embodiments, $R_g$ is

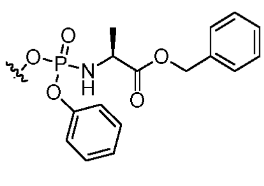 or 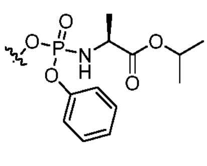 .

In some embodiments, $R_4$ and $R_{4'}$ are each independently hydrogen, $C_{1-6}$ alkyl, $C_{2-6}$ alkenyl, $C_{2-6}$ alkynyl, $C_{3-8}$ cycloalkyl, 3-8 membered heterocycloalkyl, $C_{6-10}$ aryl, 5-10 membered heteroaryl, $C_{3-8}$ cycloalkyl $C_{1-6}$ alkyl, 3-8 membered heterocycloalkyl $C_{1-6}$ alkyl, $C_{6-10}$ aryl $C_{1-6}$ alkyl or 5-10 membered heteroaryl $C_{1-6}$ alkyl; and the $R_4$ or $R_{4'}$ is unsubstituted or optionally substituted at any position with 1 to 3 substituents selected from halogen, cyano, $-L_2-OR_a$, $-L_2-OR_g$, $-L_2-OC(O)R_a$, $-L_2-OC(O)OR_a$, $-L_2-OC(O)NR_aR_b$, $-L_2-NR_aR_b$, $-L_2-NR_aC(O)OR_b$, $-L_2-NR_aC(O)NR_aR_b$, $-L_2-NR_bC(NR_b)NR_aR_b$ or $-L_2-C(O)OR_b$; or $R_4$ and $R_{4'}$ together with the N atom to which they are attached form 3-8 membered heterocycloalkyl; and the 3-8 membered heterocycloalkyl is unsubstituted or optionally substituted at any position with 1-3 substituents selected from halogen, cyano, $-L_2-OR_a$, $-L_2-NR_aR_b$, $-L_2-NR_aC(O)OR_b$, $-L_2-NR_aC(O)NR_aR_b$, - $L_2-NR_bC(NR_b)NR_aR_b$ or $-L_2-C(O)OR_b$.

In some embodiments, $R_4$ or $R_4$ are each independently $C_{1-6}$ alkyl; and the $R_4$ or $R_{4'}$ is unsubstituted or optionally substituted at any position with 1 to 3 substituents selected from $-OR_a$, $-OR_g$, - $OC(O)R_a$, $-OC(O)OR_a$, $-OC(O)NR_aR_b$,

$-NR_aR_b$, $-NR_aC(O)OR_b$, $-NR_aC(O)NR_aR_b$, $-NR_bC(NR_b)NR_aR_b$ and - $C(O)OR_b$.

In some embodiments, $R_4$ is $C_{1-6}$ alkyl; the $R_4$ is unsubstituted or optionally substituted at any position with one substituent from $-OR_a$, $-OR_g$, $-OC(O)R_a$, $-OC(O)OR_a$, $-OC(O)NR_aR_b$, $-NR_aR_b$, - $NR_aC(O)OR_b$, $-NR_aC(O)NR_aR_b$, $-NR_bC(NR_b)NR_aR_b$ and $-C(O)OR_b$; $R_{4'}$ is $C_{1-6}$ alkyl; and the $R_{4'}$ is unsubstituted or optionally substituted at any position with 1 to 3 substituents selected from halogen, $-OR_a$ and $-NR_aR_b$.

In some embodiments, $R_4$ and $R_4$ are each independently $C_{1-6}$ alkyl; and the $R_4$ or $R_{4'}$ is unsubstituted or optionally substituted at any position with one substituent selected from halogen,$-OR_a$ and $-NR_aR_b$.

In some embodiments, $R_4$ and $R_{4'}$ are each independently $C_{1-6}$ alkyl; and at least one of the $R_4$ or $R_{4'}$ is substituted at any position with one substituent selected from hydroxyl and amino.

In some embodiments, $R_4$ and $R_{4'}$ are each independently $C_{1-6}$ alkyl; and at least one of the $R_4$ or $R_{4'}$ is substituted at any position with one hydroxyl.

In some embodiments, $R_4$ is $-OCH_2CH_3$, $-CH_2CH_2NH_2$, $-CH_2CH_2OH$ or $-CH_2CH_2CH_3$; and $R_{4'}$ is $-CH_2CH_2OH$ or $-CH_2CH_2CH_3$.

In some embodiments, $R_4$ is $-CH_2CH_2OH$ or $-CH_2CH_2CH_3$; and $R_{4'}$ is $-CH_2CH_2OH$ or - $CH_2CH_2CH_3$.

In some embodiments, $R_5$ is H.

In some embodiments, $R_{5'}$ is H.

In some embodiments, $R_8$ and $R_{8'}$ are each independently hydrogen, halogen or $C_{1-6}$ alkyl; and the $C_{1-6}$ alkyl is unsubstituted or optionally substituted at any position with 1 to 3 substituents selected from - $L_3$-W, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, $R_8$ and $R_{8'}$ are each independently $C_{1-6}$ alkyl, and the $C_{1-6}$ alkyl is preferably methyl.

In some embodiments, $R_8$ and $R_{8'}$ together with the carbon atom to which they are attached form oxo, thio, $C_{3-6}$ cycloalkyl or 3-6 membered heterocycloalkyl; and the $C_{3-6}$ cycloalkyl or 3-6 membered heterocycloalkyl is unsubstituted or optionally substituted at any position with 1 to 3 substituents selected from deuterium, halogen, hydroxyl, amino, cyano, oxo, $C_{1-6}$ alkyl, halo $C_{1-6}$ alkyl, $-L_2-OR_a$ and $-L_2-NR_aR_b$.

In some embodiments, $R_8$ and $R_{8'}$ together with the carbon atom to which they are attached form oxo, thio, cyclopropyl, cyclobutyl, azetidinyl or oxetanyl; and the cyclopropyl, cyclobutyl, azetidinyl or oxetanyl is unsubstituted or optionally substituted at any position with 1 to 3 substituents selected from deuterium, halogen, hydroxyl, amino, cyano, oxo, $C_{1-6}$ alkyl, halo $C_{1-6}$ alkyl, $-L_2-OR_a$ and $-L_2-NR_aR_b$.

In some embodiments, $R_8$ and $R_{8'}$ are each independently unsubstituted $C_{1-6}$ alkyl; or $R_8$ and $R_{8'}$ together with the carbon atom to which they are attached form oxo or unsubstituted $C_{3-10}$ cycloalkyl.

In some embodiments, $R_8$ and $R_{8'}$ together with the carbon atom to which they are attached form oxo, cyclopropyl or cyclobutyl.

In some embodiments, $R_9$ is hydrogen, $C_{1-6}$ alkyl, halo $C_{1-6}$ alkyl, $-L_2-OR_a$ or $-L_2-NR_aR_b$.

In some embodiments, $R_9$ is hydrogen.

In some embodiments, $Cy^1$ is $C_{3-8}$ cycloalkyl, 3-8 membered heterocycloalkyl, $C_{6-10}$ aryl or 5-10 membered heteroaryl; and the $Cy^1$ is unsubstituted or optionally substituted at any position with 1 to 3 substituents selected from halogen, cyano, $C_{1-6}$ alkyl, halo $C_{1-6}$ alkyl, halo $C_{1-6}$ alkoxy, $C_{2-6}$ alkenyl, $C_{2-6}$ alkynyl, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$ or $-L_4-NR_eR_{e'}$;

In some embodiments, $L_1$ is a linkage bond or $C_{1-6}$ alkylene; and the $L_1$ is unsubstituted or optionally substituted at any position with 1 to 3 substituents selected from oxo, halogen, hydroxyl, amino, cyano, $C_{1-6}$ alkyl, halogenated $C_{1-6}$ alkyl, $C_{1-6}$ alkoxy or halogenated $C_{1-6}$ alkoxy.

In some embodiments, $L_1$ is a linkage bond or $-CH_2-$.

In some embodiments, $L_2$ is a linkage bond or $C_{1-6}$ alkylene; and the $L_2$ is unsubstituted or optionally substituted at any position with 1 to 3 substituents selected from oxo, halogen, hydroxyl, amino, cyano, $C_{1-6}$ alkyl, halogenated $C_{1-6}$ alkyl, $C_{1-6}$ alkoxy or halogenated $C_{1-6}$ alkoxy.

In some embodiments, in the $L_2$, $C_{1-6}$ alkylene may be $C_{1-3}$ alkylene.

In some embodiments, $L_2$ is a linkage bond, $-CH_2-$, $-CH_2CH_2-$, $-CH_2CH_2CH_2-$ or - $CH_2CH_2CH_2CH_2-$.

In some embodiments, $L_2$ is a linkage bond or $-CH_2CH_2-$.

In some embodiments, $L_3$ is a linkage bond or $C_{1-6}$ alkylene; and the $L_3$ is unsubstituted or optionally substituted at any position with 1 to 3 substituents selected from oxo, halogen, hydroxyl, amino, cyano, $C_{1-6}$ alkyl, halogenated $C_{1-6}$ alkyl, $C_{1-6}$ alkoxy or halogenated $C_{1-6}$ alkoxy.

In some embodiments, in the $L_3$, $C_{1-6}$ alkylene may be $C_{1-3}$ alkylene.

In some embodiments, $L_3$ is a linkage bond, $-CH_2-$, $-CH_2CH_2-$, $-CH_2CH_2CH_2-$, $-CH_2CH_2C(CH_3)_2-$, $-CH_2CH_2CH_2CH_2-$ or $-CH_2CH(CH_3)CH_2-$.

In some embodiments, $L_3$ is a linkage bond or $-CH_2-$.

In some embodiments, $L_4$ is a linkage bond or $C_{1-6}$ alkylene; and the $L_4$ is unsubstituted or optionally substituted at any position with 1 to 3 substituents selected from oxo, halogen, hydroxyl, amino, cyano, $C_{1-6}$ alkyl, halogenated $C_{1-6}$

alkyl, $C_{1-6}$ alkoxy or halogenated $C_{1-6}$ alkoxy.

In some embodiments, each $R_c$ is independently hydrogen or a linkage bond attached to LX; and only one $R_c$ in D is a linkage bond attached to LX;

In some embodiments, $R_7$ is phenyl, 5-6 membered heteroaryl, 3-8 membered heterocycloalkyl or an 8-12 membered fused ring group; and the $R_7$ is unsubstituted or optionally substituted at any position with one or more substituents selected from -$L_3$-W, -$R_c$, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, $R_7$ is an 8-12 membered fused ring group; and the $R_7$ is unsubstituted or optionally substituted at any position with one or more substituents selected from -$L_3$-W, -$R_c$, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, in the $R_7$, the 8-12 membered fused ring group may be a fused ring of ring A and ring B, the ring A is 5-6 membered heteroaryl or phenyl, and the ring B is a 5-6 membered heteroalkenyl ring; in the 5-6-membered heteroaryl, the heteroatom is selected from one or more of N, O and S, and the number of the heteroatom is 1, 2 or 3; and in the 5-6-membered heteroalkenyl ring, the heteroatom is selected from one or more of N, O and S, and the number of the heteroatom is 1, 2 or 3.

In some embodiments, in the $R_7$, the 8-12 membered fused ring group may be a fused ring of ring A and ring B, the ring A is 5-6 membered heteroaryl or phenyl, and the ring B is a 5-6 membered heteroalkenyl ring attached to the $L_1$ through the ring A; in the 5-6 membered heteroaryl, the heteroatom is N, and the number of the heteroatom is 1 or 2; and in the 5-6 membered heteroalkene ring, the heteroatom is N, and the number of the heteroatom is 1 or 2.

In some embodiments, in the $R_7$, in the 5-6 membered heteroaryl, the heteroatom may be selected from one or more of N, O and S, and the number of the heteroatom may be 1, 2 or 3.

In some embodiments, in the $R_7$, in the 5-6 membered heteroaryl, the heteroatom may be N, and the number of the heteroatom may be 1 or 2.

In some embodiments, in the $R_7$, the 5-6 membered heteroaryl may be pyridinyl, pyrazinyl or pyridazinyl, and may be pyridin-3-yl, pyrazin-2-yl or pyridazin-3-yl.

In some embodiments, in the D, $R_7$ is

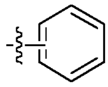 , 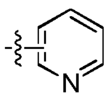 , 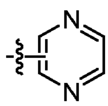 , 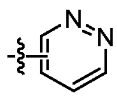 , 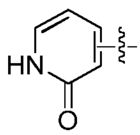 ,

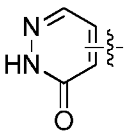 , 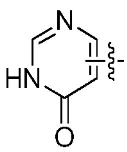 , 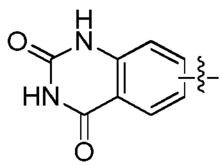 , 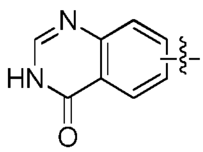 , 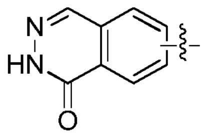 , 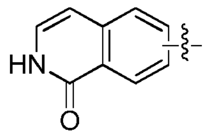 ,

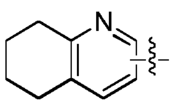 , 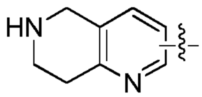 , 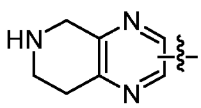 , 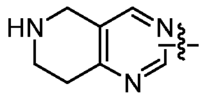 , 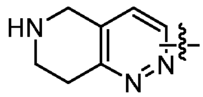 ,

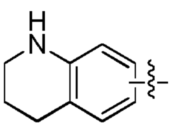 , 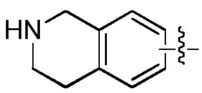 , 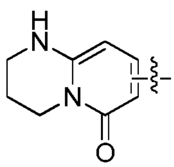 , 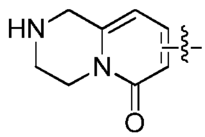 or 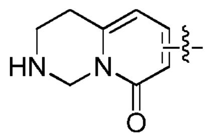 ;

and the $R_7$ is unsubstituted or optionally substituted at any position with 1 to 3 substituents selected from -$L_3$-W, -$R_c$, halogen, cyano, nitro, $C_{1-6}$ alkyl, $C_{2-6}$ alkenyl, $C_{2-6}$ alkynyl, $C_{1-6}$ alkoxy, halo $C_{1-6}$ alkyl, halo $C_{1-6}$ alkoxy and $C_{1-6}$ alkylamino.

In some embodiments, $R_7$ is

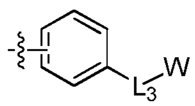, 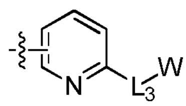, 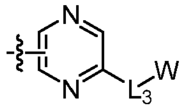,

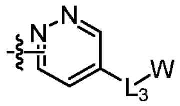, 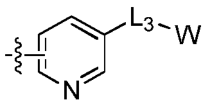, 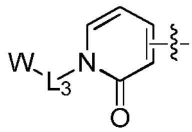, 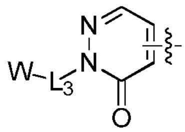, 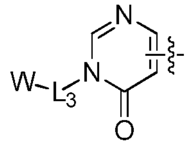,

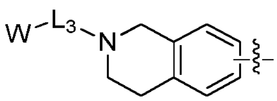, 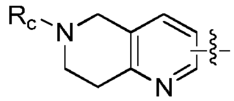, 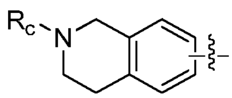, 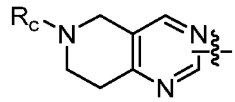,

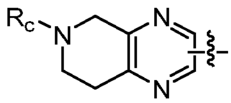, 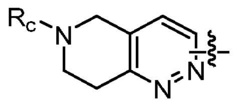, 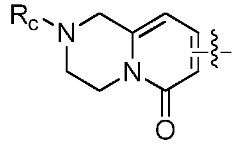, 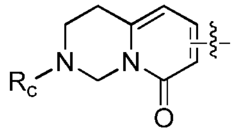,

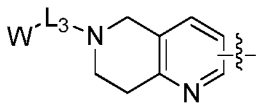 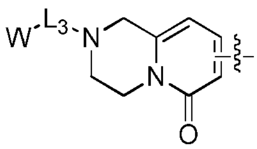 or 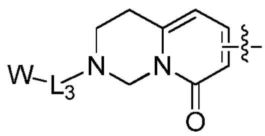.

In some embodiments, R is -C(O)-$NR_9$-$L_1$-$R_7$ or -C(S)-$NR_9$-$L_1$-$R_7$; $R_9$ is hydrogen, $L_1$ is a linkage bond, and $R_7$ is an unsubstituted 8-12 membered fused ring group, an 8-12 membered fused ring group substituted with one -$L_3$-W, or 5-6 membered heteroaryl substituted with one -$L_3$-W; $L_3$ is a linkage bond or $C_{1-6}$ alkylene, W is -$NR_dR_e$; and $R_d$ is H, and $R_e$ is -$R_c$.

In some embodiments, $R_7$ is

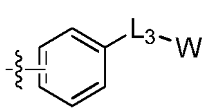, 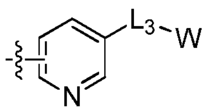, 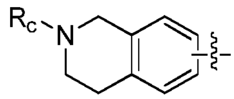

or

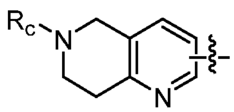.

In some embodiments, $R_7$ is

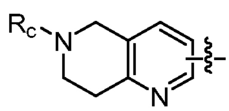

and may also be

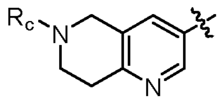
.

In some embodiments, $R_7$ is

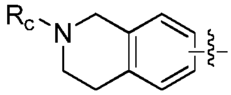

and may also be

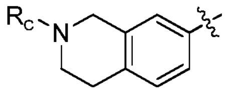
.

In some embodiments, in the $R_7$, the 5-6 membered heteroaryl substituted with one $-L_3$-W may be

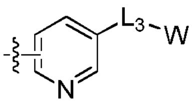
,

may be

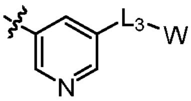
,

and may be

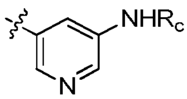

or

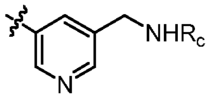
.

In some embodiments, in the $R_7$, the 5-6 membered heteroaryl substituted with one $-L_3$-W may be

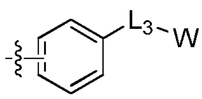
,

may be

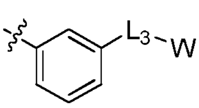
or
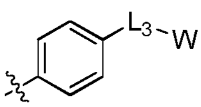
,

and may be

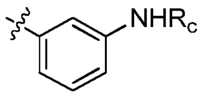

,

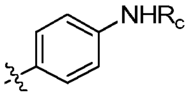 , 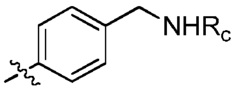 or 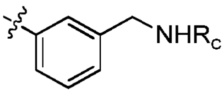 .

In some embodiments, the $R_7$ is

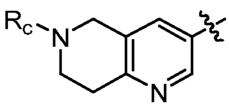 , 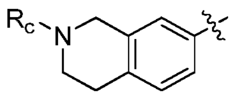 , 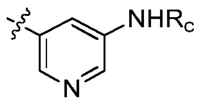 ,

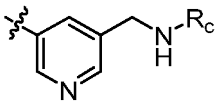 , 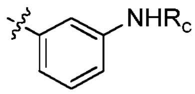 , 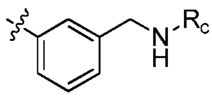 or 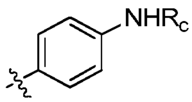 ,

and $R_c$ is a linkage bond attached to L.

In some embodiments, D is:

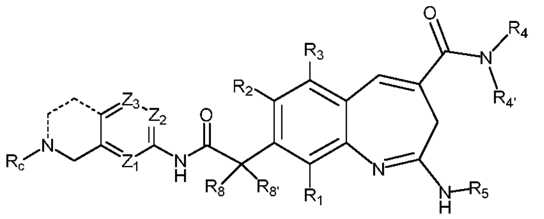

(D-1)

or

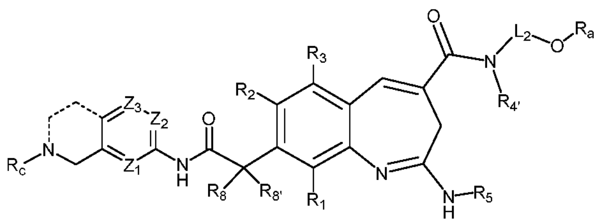

(D-2)

,

where

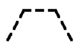

is absent, $-CH_2-$ or $-CH_2-CH_2-$;

$Z_1$, $Z_2$ and $Z_3$ are each independently N or CH;

$R_c$ is a linkage bond attached to L; and

definitions of $R_1$, $R_2$, $R_3$, $R_4$, $R_{4'}$, $R_5$, $R_8$, $R_{8'}$ and $R_a$ are as previously described.

In some embodiments, $Z_1$ is CH, $Z_2$ is CH, $Z_3$ is N or CH.

In some embodiments, $R_5$ is H.

In some embodiments, formula D' is any one of formulas D'-1 to D'-6:

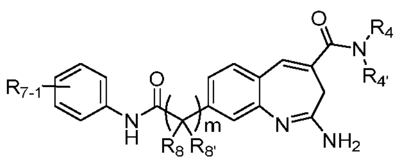

D'-1

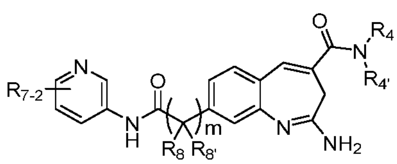

D'-2

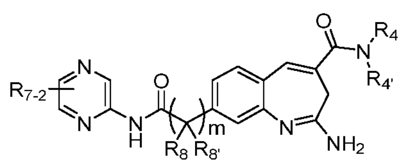

D'-3

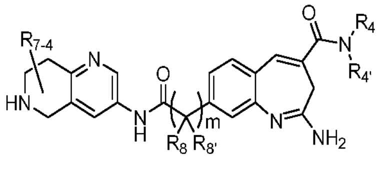

D'-4

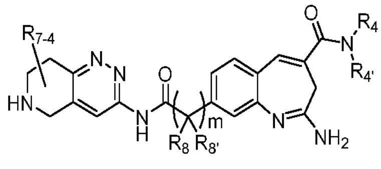

D'-5

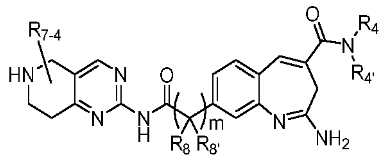

D'-6

where definitions of $R_{7-1}$, $R_{7-2}$, $R_{7-4}$, $R_4$, $R_{4'}$, $R_8$ and $R_{8'}$ are as previously described.

In some embodiments, D is of any one of structures shown in Table 5:

In some embodiments, the LX is M'-$(T)_w$-PEG-$(T)_o$-*, M'-PEG-*, M'-$(T)_w$-*, M'-$(T)_w$-$(A)_v$-$(T)_o$-PEG-*, M'-$(T)_w$-$(A)_v$-$(T)_o$-*, M'-$(T)_w$-$L_5$-*, M'-$(T)_w$-PEG-$L_5$-*, M'-$(T)_w$-PEG-$(A)_v$-$L_5$-*, M'-$(T)_w$-PEG-$(A)_v$-$(T)_o$-*, M'-PEG-$(T)_o$-PEG-* or M'-$(T)_w$-$(A)_v$-$L_5$-*; and * is a linkage site of the LX and the D;

M' is hydrogen, a linker head precursor or a linker head attached to 1 to 2 amino acids;

w is independently 1, 2, 3, 4, 5, 6, 7, 8, 9 or 10;

o is independently 0, 1, 2, 3, 4, 5, 6, 7, 8, 9 or 10;

T is independently $-(CH_2)_x-C(O)-$, $-NR_h-$, -O-, -S-, $-(CH_2)_x-C_{6-14}$ arylene-$(CH_2)_y$-, $-(CH_2)_x$-5-6 membered heteroarylene-$(CH_2)_y$-, $-(CH_2)_x-C_{3-6}$ cycloalkylene-$(CH_2)_y$-, $-(CH_2)_x$-3-6 membered heterocycloalkylene-$(CH_2)_y$-, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x-S-S-C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the $-(CH_2)_x$-5-6 membered heteroarylene-$(CH_2)_y$-, the heteroatom is selected from one or more of N, O and S, and the number of the heteroatom is 1, 2 or 3; and in "3-6 membered heterocycloalkylene" in the $-(CH_2)_x$-3-6 membered heterocycloalkylene-$(CH_2)_y$-, the heteroatom is selected from one or more of N, O and S, and the number of the heteroatom is 1, 2 or 3;

$R_h$ is independently hydrogen, $C_{1-6}$ alkyl, $-PO(OH)_2$, $-PO(OCH_3)_2$, $-C(O)-(CH_2CH_2O)_n-CH_3$ or - $(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;

PEG is $-(CH_2CH_2O)_n-(CH_2)_u-$ or $-(CH_2CH_2O)_n-(CH_2)_u-C(O)-$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;

v is independently 1, 2, 3, 4 or 5;

A is independently an amino acid residue or an amino acid residue substituted with 1 $R_A$; the $R_A$ is independently $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and

$L_5$ is a self-immolative group.

In the LX, w in M'-$(T)_w$-PEG-$(T)_o$-*, M'-PEG-*, M'-$(T)_w$-*, M'-$(T)_w$-$(A)_v$-$(T)_o$-PEG-*, M'-$(T)_w$-$(A)_v$-$(T)_o$-*, M'-$(T)_w$-$L_5$-*, M'-$(T)_w$-PEG-$L_5$-*, M'-$(T)_w$-PEG-$(A)_v$-$L_5$-*, M'-$(T)_w$-PEG-$(A)_v$-$(T)_o$-*, M'-PEG-$(T)_o$-PEG-* and M'-$(T)_w$-$(A)_v$-$L_5$-* may also be independently replaced with 0, 1, 2, 3, 4, 5, 6, 7, 8, 9 or 10;

when w is 0, LX may also be M'-$(A)_v$-$(T)_o$-PEG-*, M'-$(A)_v$-$(T)_o$-* or M'-$(A)_v$-$L_5$-*; * is a linkage site of the LX and the D; and M' is hydrogen.

In some embodiments, M' is a linker head precursor for reacting with an amino acid residue side chain of an antibody, preferably a linker head precursor for reacting with amino or thiol in an amino acid residue side chain of an antibody; and more preferably a linker head precursor for reacting with thiol in an amino acid residue side chain of an antibody.

In some embodiments, M' is ethynyl, vinyl, hydroxylamino,

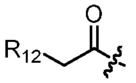, 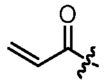,

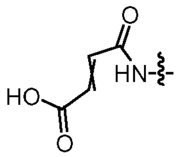, 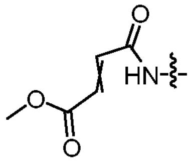, 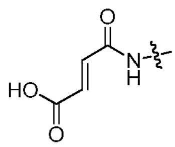 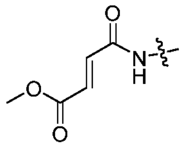 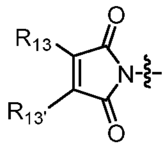, 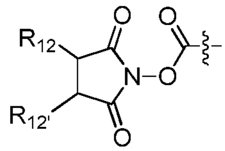,

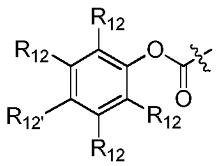, 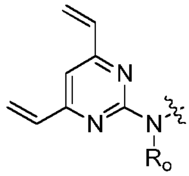, 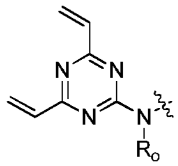, 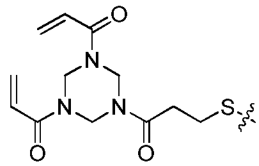, 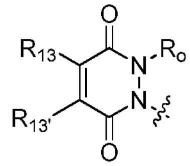,

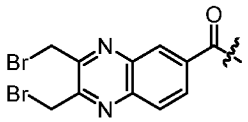, 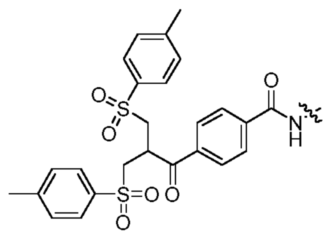, 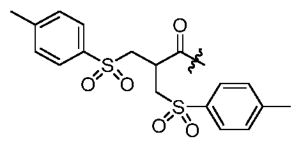, 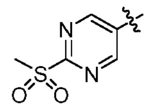,

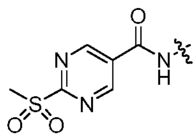, 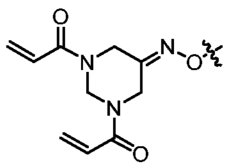, 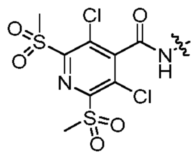, 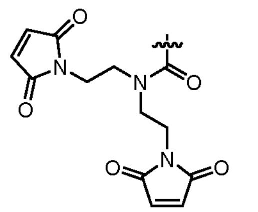,

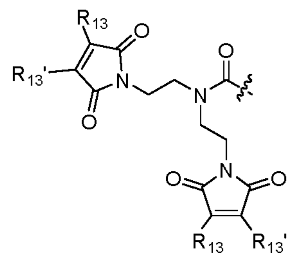, 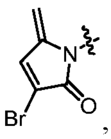, 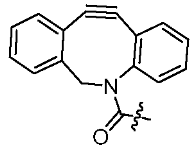, 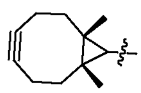 or 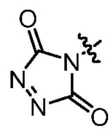;

$R_o$ is hydrogen or $C_{1-4}$ alkyl;

each $R_{12}$ and $R_{12'}$ is independently halogen (e.g. bromo or iodo), nitro or $-SO_3^-$;

$R_{13}$ and $R_{13'}$ are each independently hydrogen, halogen (e.g. bromo), phenylthio or pyridylthio; and $R_{13}$ and $R_{13'}$ are not both hydrogen at the same time.

In some embodiments, M' is

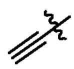, 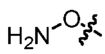, 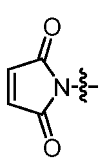, 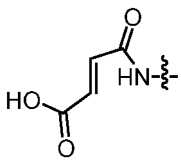, 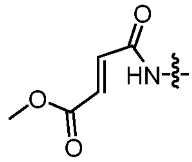,

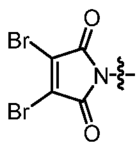, 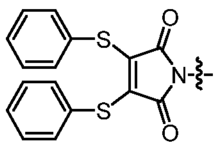, 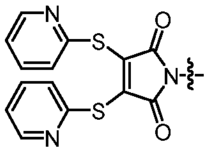, 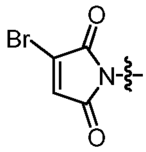, 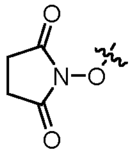, 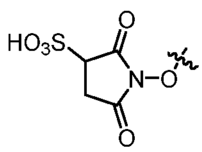,

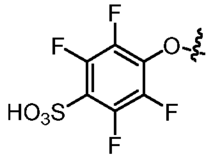, 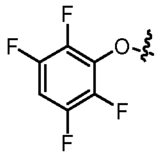, 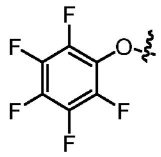, 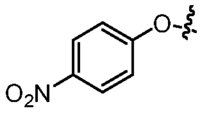, 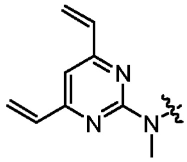, 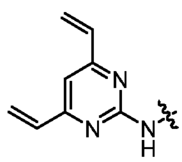,

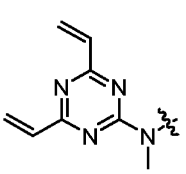, 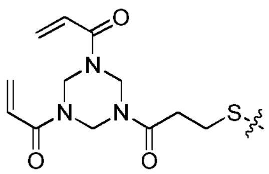, 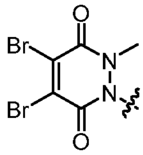, 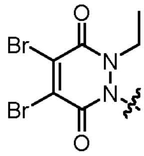, 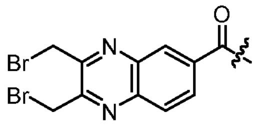,

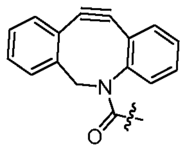, 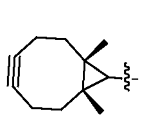 or 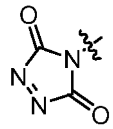.

In some embodiments, M' is

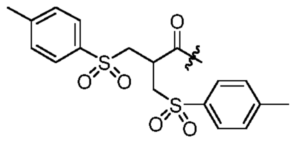, 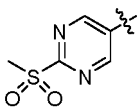, 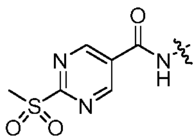,

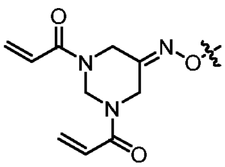, 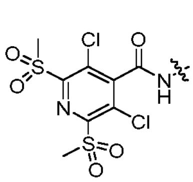, 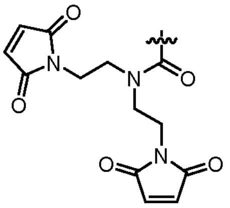 or 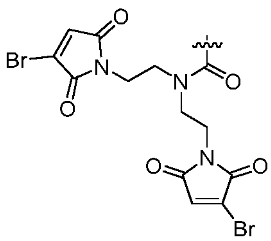.

In some embodiments, M' is a linker head attached to 1 to 2 amino acids, and the amino acids are covalently attached to the linker head through an amino acid side chain.

In some embodiments, LX is M'-$(T)_w$-PEG-$(T)_o$-*, M'-PEG-*, M'-$(T)_w$*-, M'-$(T)_w$-$(A)_v$-*, M'-$(T)_w$-PEG-$(A)_v$-* or M'-PEG-$(T)_o$-PEG-*, M' -(T) w-PEG-(A) v-*, or M'-PEG-(T) o-PEG-*; and M' is

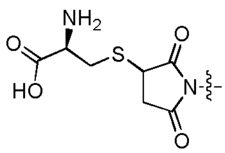, 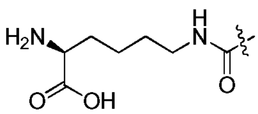 or 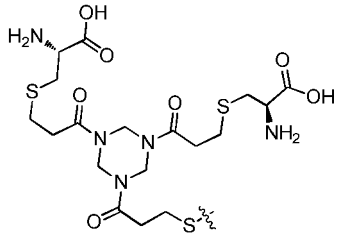 or 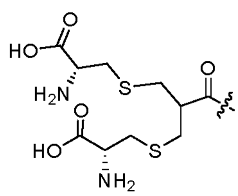.

In some embodiments, LX is M'-$(T)_w$-$(A)_v$-$L_5$-*; and M' is

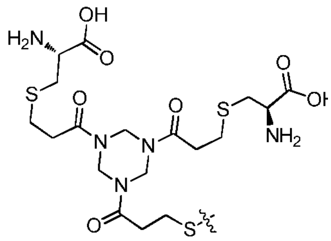

or

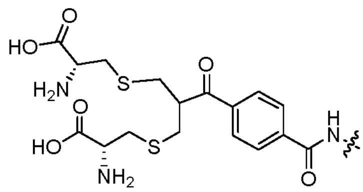

.

In some embodiments, LX is M'-$(T)_w$-PEG-$(T)_o$-*, M'-PEG-*, M'-$(T)_w$-*, M'-$(T)_w$-$(A)_v$-$(T)_o$-PEG-*, M'-$(T)_w$-$(A)_v$-$(T)_o$-*, M'-$(T)_w$-$L_5$-*, M'-$(T)_w$-PEG-$L_5$-*, M'-$(T)_w$-PEG-$(A)_v$-$L_5$-*, M'-$(T)_w$-PEG-$(A)_v$-$(T)_o$-*, M'-PEG-$(T)_o$-PEG-*, M'-$(T)_w$-$(A)_v$-$L_5$-*, M'-$(A)_v$-$(T)_o$-PEG-*, M'-$(A)_v$-$(T)_o$-* or M'-$(A)_v$-$L_5$-*; * is a linkage site of the LX and the D; and M' is hydrogen.

In some embodiments, each A is independently

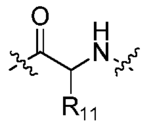

;

each $R_{11}$ is independently an amino acid side chain or an amino acid side chain modified by $-C(O)(CH_2CH_2O)_nCH_3$ or $-(CH_2CH_2O)_nCH_3$; or $R_{11}$ and an adjacent nitrogen atom form a five-membered heterocycloalkyl. In some embodiments, in the A, the amino acid is a natural amino acid or a non-natural amino acid.

In some embodiments, the

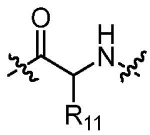

may be

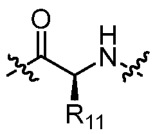

or

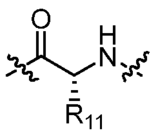

,

and may also be

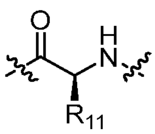

.

In some embodiments, in the A, each $R_{11}$ is independently hydrogen, methyl, ethyl, n-propyl, isopropyl, isobutyl,

$-SO_3H$,

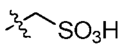, 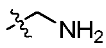, 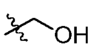, 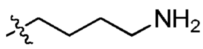, 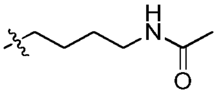,

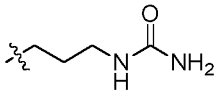, 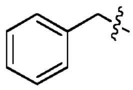, 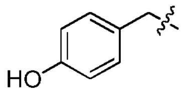, 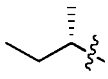, 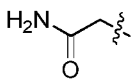, 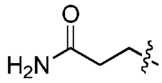, 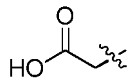,

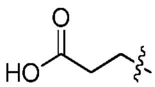 or 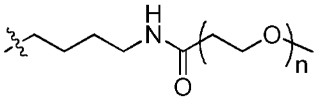;

or $R_{11}$ and an adjacent nitrogen atom form a five-membered heterocycloalkyl.

In some embodiments, $-(A)_v-$ is

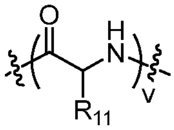;

each $R_{11}$ is independently hydrogen, methyl, ethyl, n-propyl, isopropyl, isobutyl, $-SO_3H$,

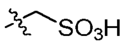, 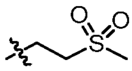, 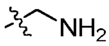, 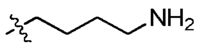,

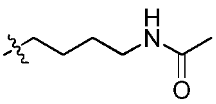, 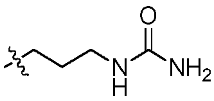, 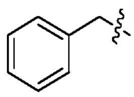, 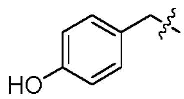, 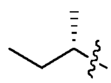, 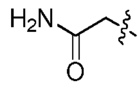,

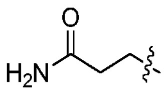, 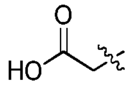, 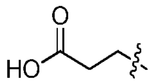 or 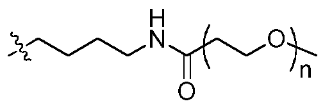;

or $R_{11}$ and an adjacent nitrogen atom form a five-membered heterocycloalkyl; and v is any integer from 1 to 4, and v is preferably 1 or 2.

In some embodiments, A is attached to $-(T)_o-$ or $-L_5-$ through a carbonyl terminus.

In some embodiments, $L_5$ is

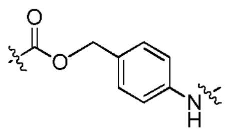, 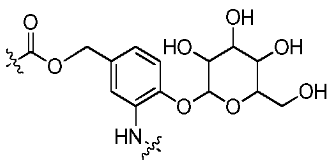,

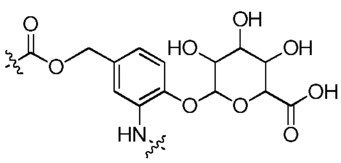, 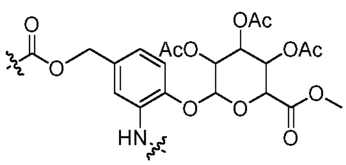, 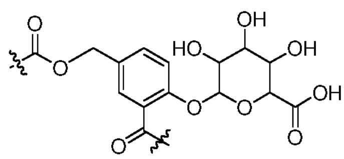,

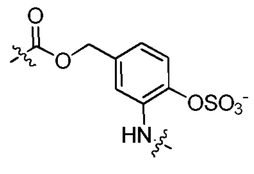

or

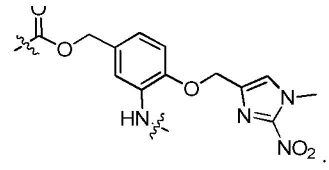

In some embodiments, $L_5$ is

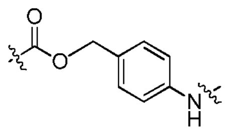

,

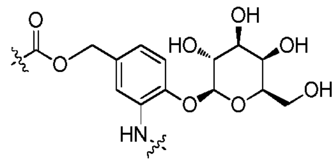

,

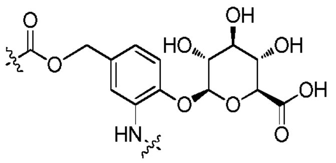

,

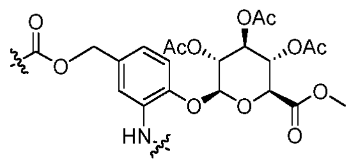

,

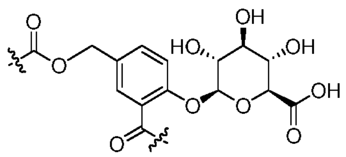

,

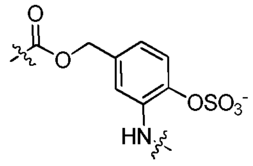

or

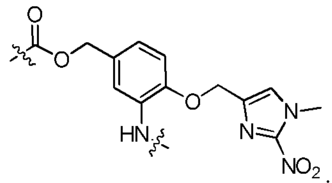

In some embodiments, $L_5$ is

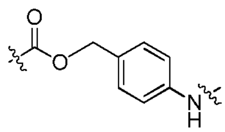

,

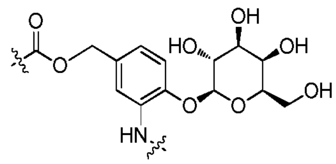

,

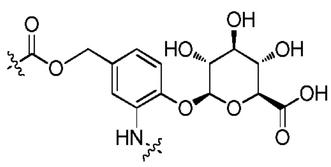

,

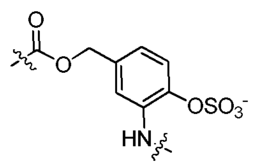

or

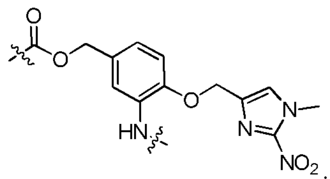

In some embodiments, $-L_5-$ is attached to D through a carbonyl terminus.

In some embodiments, each T is independently $-(CH_2)_x-C(O)-$, $-NR_h-$, -O-, -S-, $-(CH_2)_x-C_{6-10}$ arylene-$(CH_2)_y-$, $-(CH_2)_x-$5-10 heteroarylene-$(CH_2)_y-$, $-(CH_2)_x-C_{3-10}$ cycloalkylene-$(CH_2)_y-$, $-(CH_2)_x-$3-10 membered heterocycloalkylene-$(CH_2)_y-$, $-NR_h-(CH_2)_x-C(O)-$, $-O-(CH_2)_x-C(O)-$, $-S-(CH_2)_x-C(O)-$, - $(CH_2)_xCH(NHR_h)-C(O)-$, $C_{1-20}$ alkylene or $C_{2-20}$ alkenylene, and x and y are each independently any integer from 0 to 10.

In some embodiments, each T is independently $-(CH_2)_x-C(O)-$, $-NR_h-$, -O-, -S-, $-(CH_2)_x$-phenylene-$(CH_2)_y-$, $-(CH_2)_x-$5-6 heteroarylene-$(CH_2)_y-$, $-(CH_2)_x-C_{3-8}$ cycloalkylene-$(CH_2)_y-$, $-(CH_2)_x-$3-8 membered heterocycloalkylene-$(CH_2)_y-$, $-NR_h-(CH_2)_x-C(O)-$, $-O-(CH_2)_x-C(O)-$, $-S-(CH_2)_x-C(O)-$, - $(CH_2)_xCH(NHR_h)-C(O)-$ or $C_{1-6}$ alkylene; and x and y are each independently any integer from 0 to 10.

In some embodiments, $R_h$ is hydrogen or $C_{1-6}$ alkyl; and $R_h$ is preferably hydrogen or $C_{1-3}$ alkyl.

In some embodiments, $-(T)_w-$ is

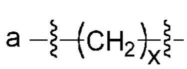, 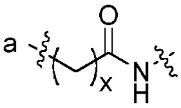, 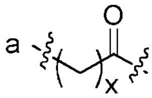,

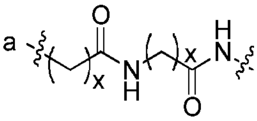, 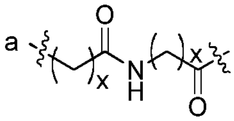, 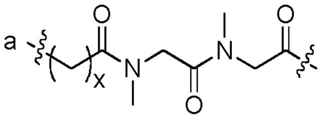, 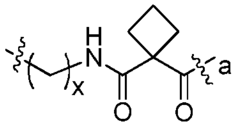,

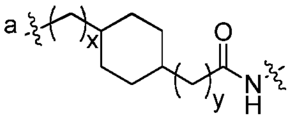, 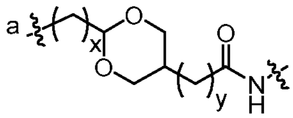, 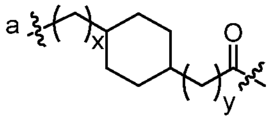, 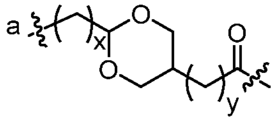,

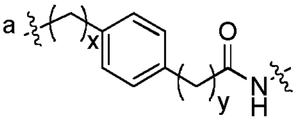, 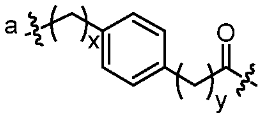, 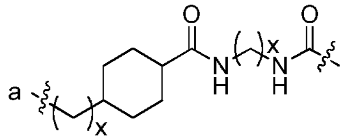,

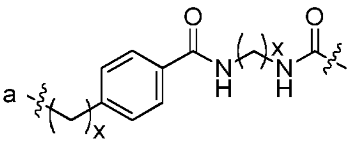, 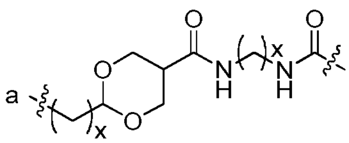, 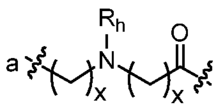,

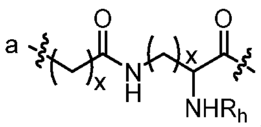;

and $-(T)_w-$ is attached to the M' through an a-terminus. In some embodiments, each x is independently an integer from 0 to 10, and the x is more preferably an integer from 0 to 6.

In some embodiments, each y is independently an integer from 0 to 10, and the y is more preferably an integer from 0 to 6.

In some embodiments, $-(T)_o-$ is

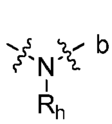, 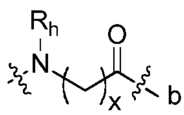, 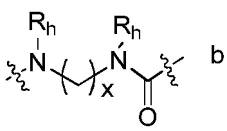,

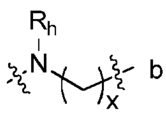 or 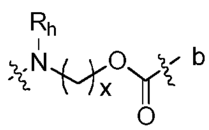;

and $-(T)_o-$ is attached to the -PEG- or the D via a b-terminus.

In some embodiments, each x is independently an integer from 0 to 10, and more preferably an integer from 0 to 6.

In some embodiments, PEG is $-(CH_2CH_2O)_n-(CH_2)_u-$ or $-(CH_2CH_2O)_n-(CH_2)_u-C(O)-$, n is any integer from 1 to 30, and u is any integer from 1 to 5.

In some embodiments, when LX is $M'-(T)_w-(A)_v-L_5-*$, a combination of M', $-(T)_w-$, $-(A)_v-$ and $-L_5-$ does not simultaneously satisfy conditions of: M' is maleimido (

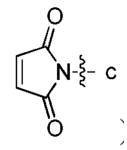

); $-(T)_w-$ is hexanoyl; $-(A)_v-$ is -Val-Cit-, -Val-Ala or -Gly-Gly-Phe-Gly-; and $-L_5-$ is PABC (

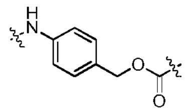

).

In some embodiments, LX is $M'-(T)_w-(A)_v-L_5-*$; and the LX is preferably $M'-CH_2-(C_{5-6}$ cycloalkylene)-$C(=O)-(A)_v-L_5-$, $M'-(CH_2)_x-C(=O)NH-CH_2-CH(NHR_h)-C(=O)-(A)_v-L_5-$, M'-(phenylene)-$CH_2-C(=O)-(A)_v-L_5-$ or $M'-CH_2-$(5-6 membered heterocyclylene)-$C(=O)-(A)_v-L_5-$.

In some embodiments, LX is $M'-(T)_w-PEG-(A)_v-L_5-*$; and the LX is preferably $M'-(CH_2)_x-C(=O)NH-PEG-(A)_v-L_5-$, M'-(phenylene)-$CH_2-C(=O)NH-PEG-(A)_v-L_5-$, $M'-CH_2-(C_{5-6}$ cycloalkylene)-$C(=O)NH-PEG-(A)_v-L_5-$ or $M'-CH_2-$(5-6 membered heterocyclylene)-$C(=O)NH-PEG-(A)_v-L_5-$.

In some embodiments, LX is $M'-(T)_w-(A)_v-(T)_o-*$; and the LX is preferably $M'-(CH_2)_x-C(=O)NH-(A)_v-NH-CH_2-$.

In some embodiments, LX is any one of combinations of:

A) M'-PEG-;
B) $M'-(CH_2)_x-$;
C) $M'-(CH_2)_x-C(O)-$;
D) $M'-(CH_2)_x-(C_{5-6}$ cycloalkylene)-$(CH_2)_y-C(O)NH-PEG-$;
E) $M'-(CH_2)_x-$(5-6 membered heterocyclylene)-$(CH_2)_y-C(O)NH-PEG-$;
F) $M'-(CH_2)_x-$(phenylene)-$(CH_2)_y-C(O)NH-PEG-$;
G) $M'-(CH_2)_x-C(O)NH-PEG-$;
H) $M'-(CH_2)_x-C(O)NH-(CH_2)_x-C(O)NH-PEG-$;
I) $M'-(CH_2)_x-C(O)NH-(CH_2)_x-C(O)NH-PEG-NH-(CH_2)_x-C(O)-$;
J) $M'-(CH_2)_x-(C_{5-6}$ cycloalkylene)-$(CH_2)_y-C(O)NH-(CH_2)_x-NHC(O)-$;
K) $M'-(CH_2)_x-$(5-6 membered heterocyclylene)-$(CH_2)_y-C(O)NH-(CH_2)_x-NHC(O)-$;
L) $M'-(CH_2)_x-$(phenylene)-$(CH_2)_y-C(O)NH-(CH_2)_x-NHC(O)-$;
M) $M'-(CH_2)_x-C(O)NH-(CH_2)_x-C(O)-L_5-$;
N) $M'-(CH_2)_x-C(O)NH-PEG-L_5-$;
O) $M'-(CH_2)_x-$(phenylene)-$(CH_2)_y-C(O)-(A),-$;
P) $M'-(CH_2)_x-C(O)NH-(C_{3-6}$ cycloalkylene)-$C(O)-(A)_v-$;
Q) $M'-(CH_2)_x-C(O)NH-PEG-(A)_v-$;
R) $M'-(CH_2)_x-C(O)NH-PEG-(A)_v-L_5-$;
S) $M'-(CH_2)_x-(C_{5-6}$ cycloalkylene)-$C(O)-$;
T) $M'-(CH_2)_x-$(5-6 membered heterocyclylene)-$C(O)-$;
U) M'-(phenylene)-$(CH_2)_y-C(O)-$;
V) $M'-(CH_2)_x-C(O)-(A)_v-NH-PEG-$;
W) $M'-(CH_2)_x-C(O)-(A)_v-NH-(CH_2)_x-C(O)-$;
X) $M'-(CH_2)_x-C(O)-(A)_v-$;
Y) $M'-(CH_2)_x-$(5-6 membered heterocyclylene)-$C(O)-(A)_v-$;
Z) $M'-(CH_2)_x-C(O)NH-CH_2-CH(NHR_h)-C(O)-(A)_v-$ or
AA) M'-(phenylene)-(5-6 membered heterocyclylene)-C(O)-;
AB) M'-(phenylene)-$(CH_2)_y-C(O)NH-(CH_2)x-PEG-(A)_v-$;
AC) M'-(phenylene)-$C(O)NH-(CH_2)x-(A)_v-L_5-$; or
AD) $M'-(CH_2)_x-PEG-(A)_v-$.

In some embodiments, LX is

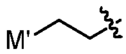, 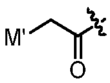, 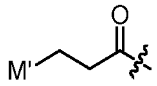, 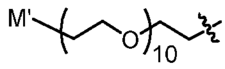,

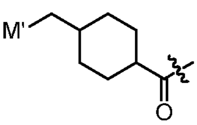, 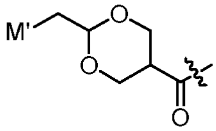, 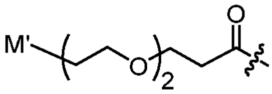, 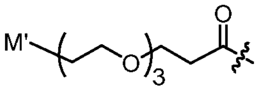,

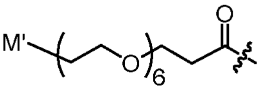, 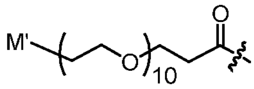, 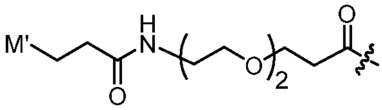,

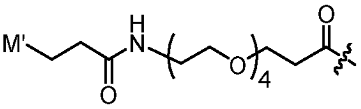, 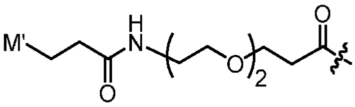,

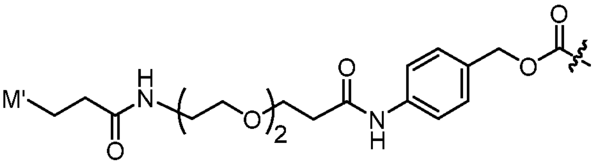, 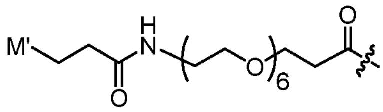,

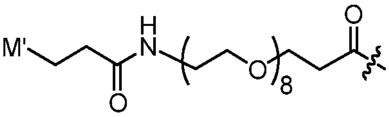, 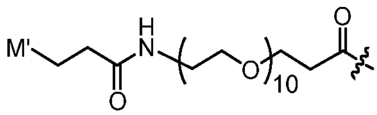, 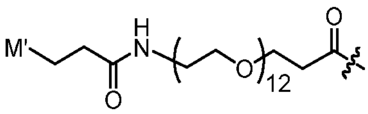,

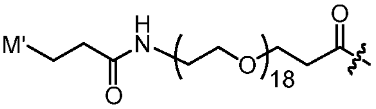, 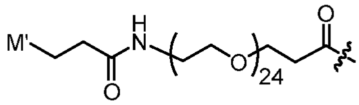, 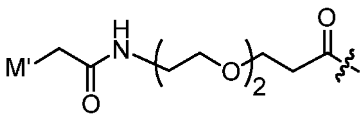,

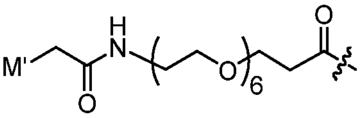, 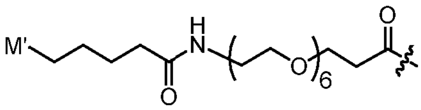,

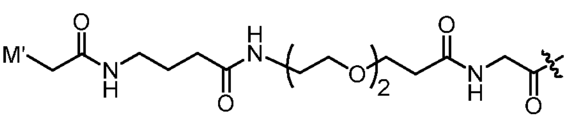, 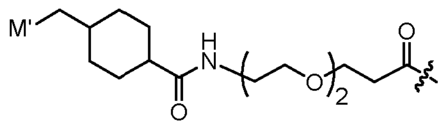,

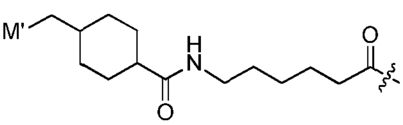, 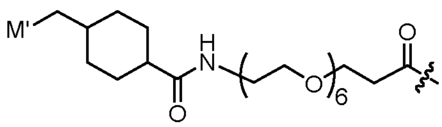,

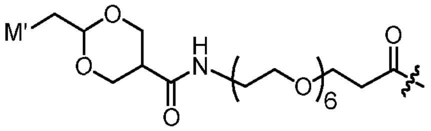

,

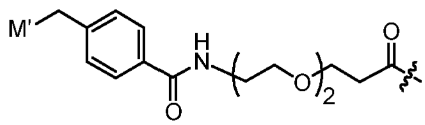

,

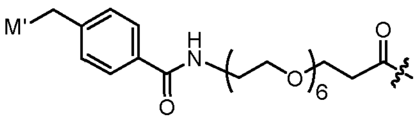

,

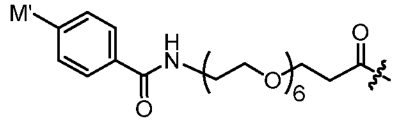

,

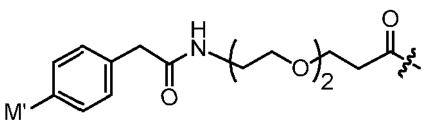

,

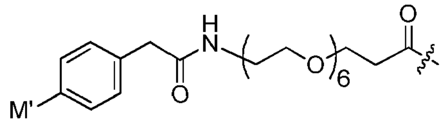

,

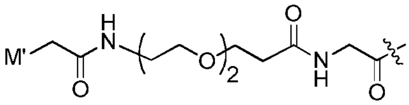

,

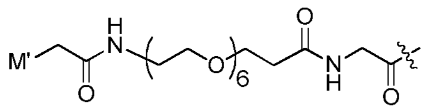

,

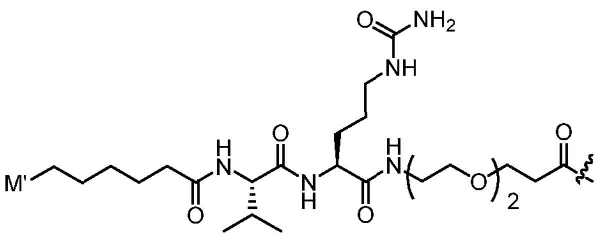

,

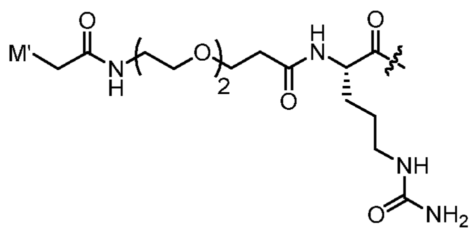

,

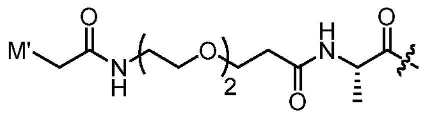

,

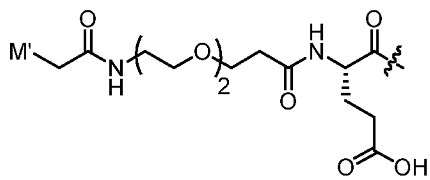

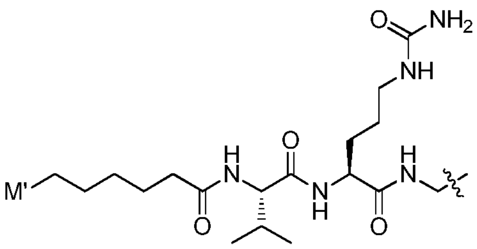

,

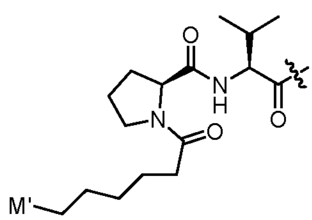

,

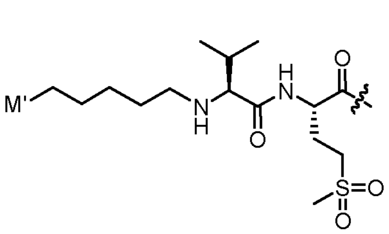

,

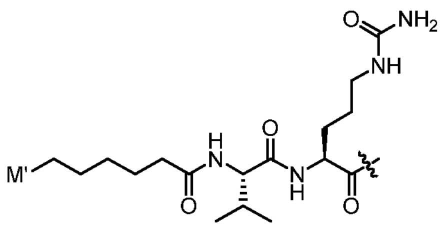

,

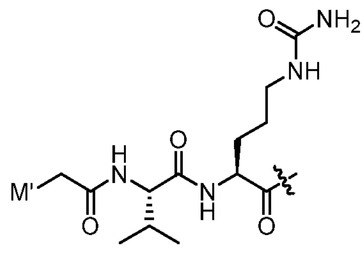
,
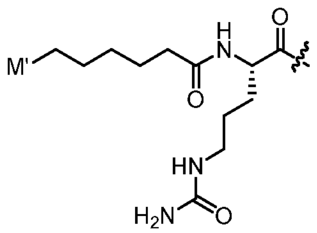
,
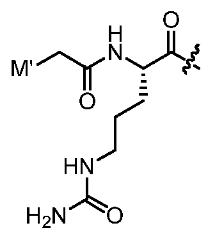
,

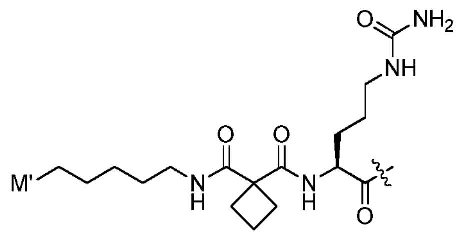
,
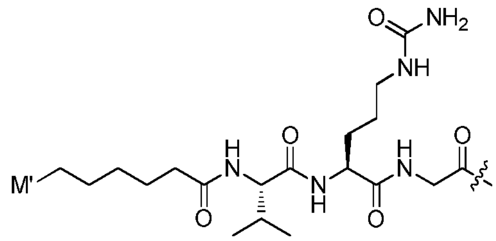

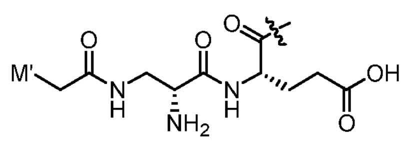
,
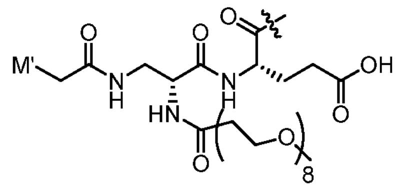
,

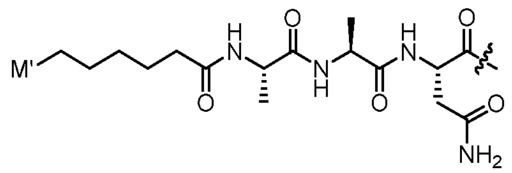
,
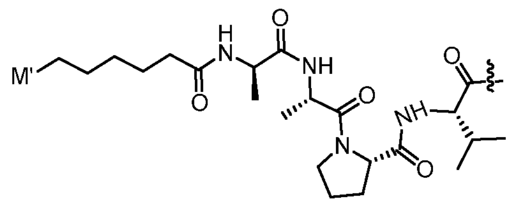
,

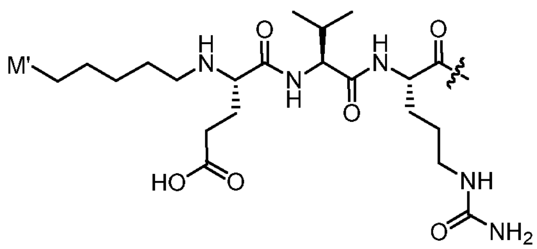
,
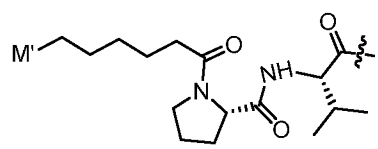
,

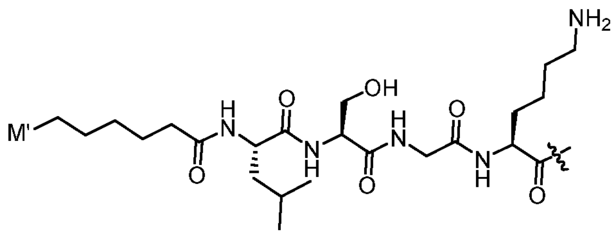
,
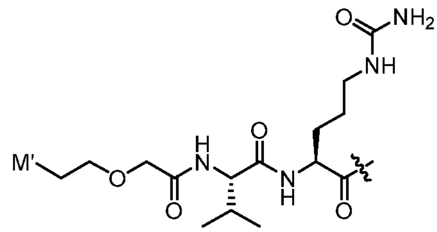
,

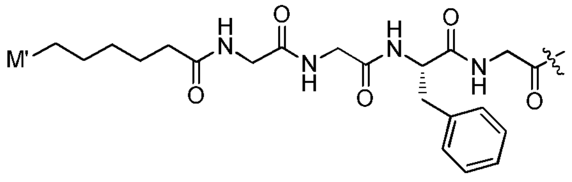

,

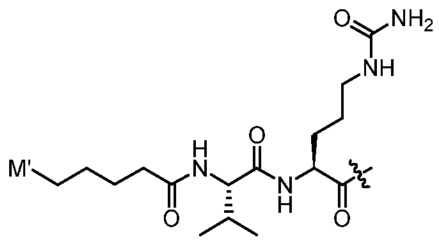

,

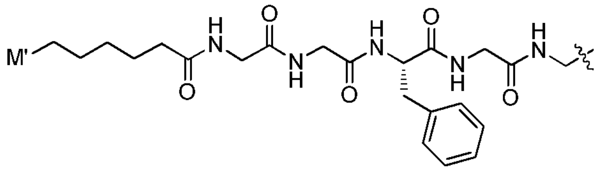

,

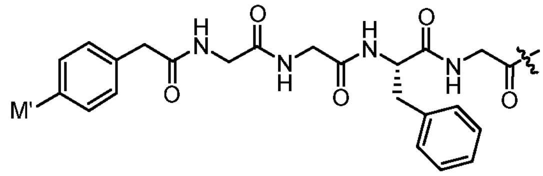

,

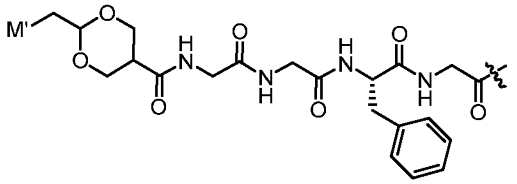

,

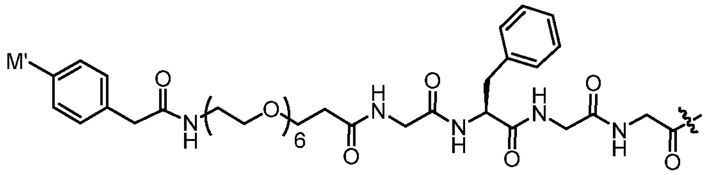

,

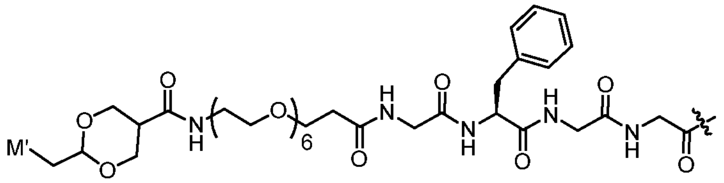

,

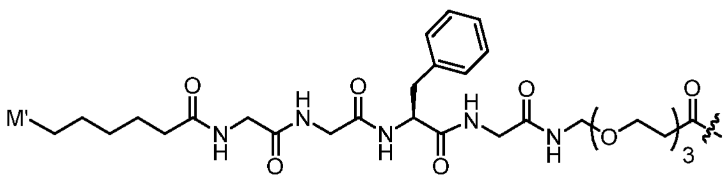

,

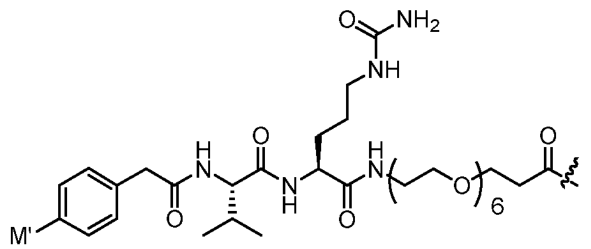

,

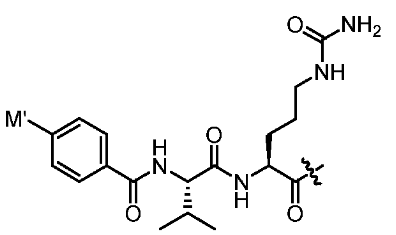

,

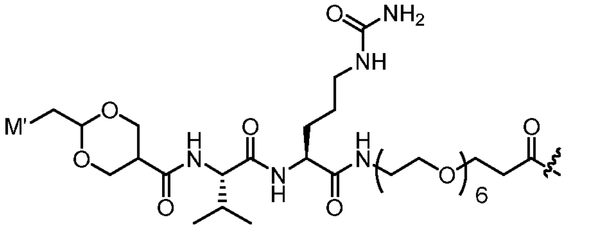

,

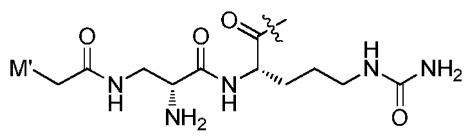

,

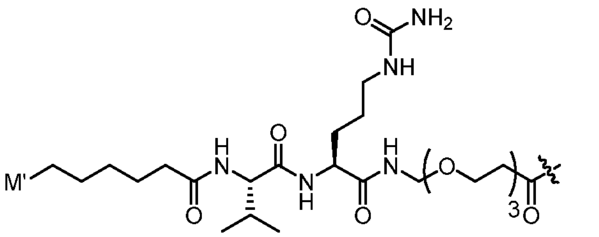

,

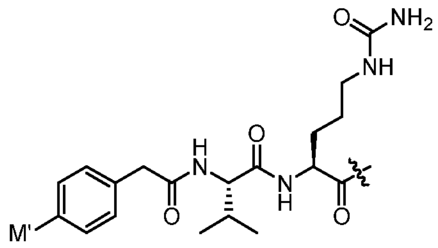

,

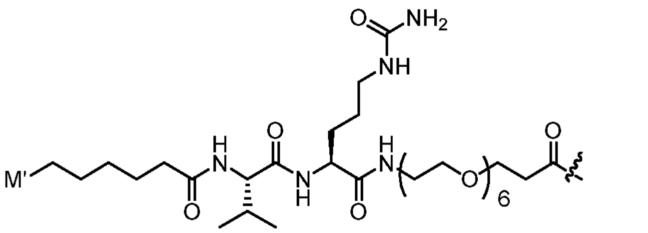

,

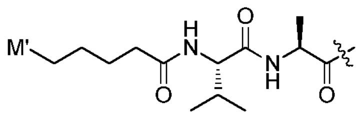

,

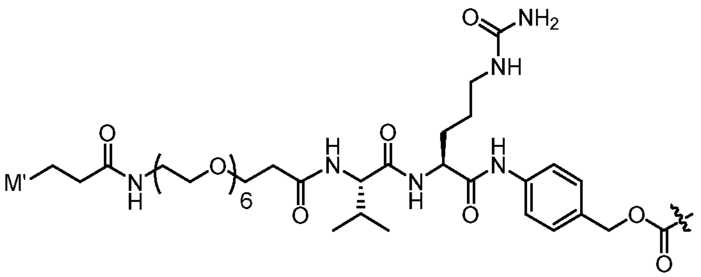

,

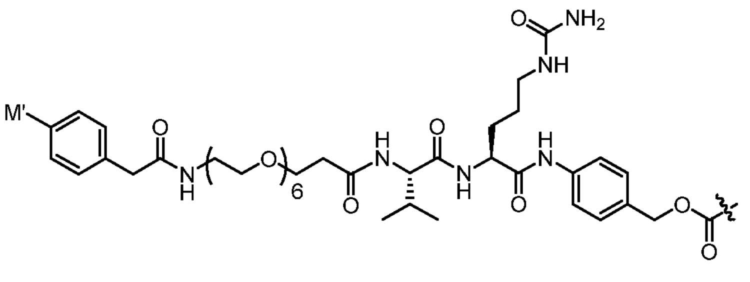

,

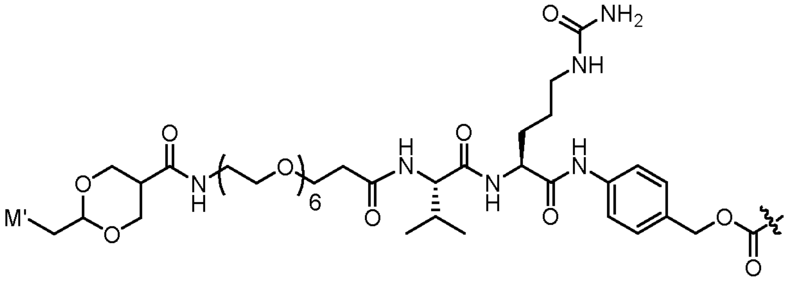

,

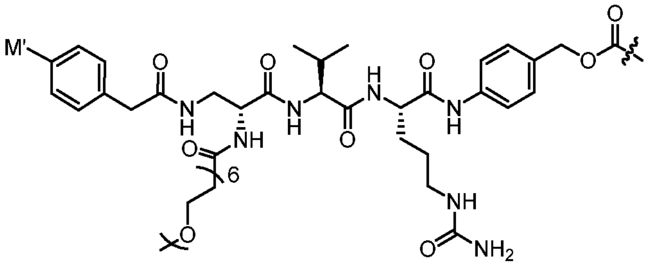
,
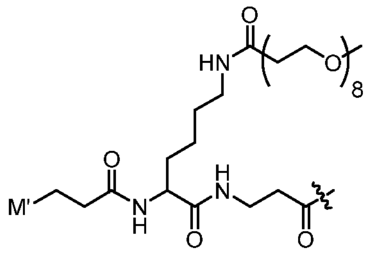
,

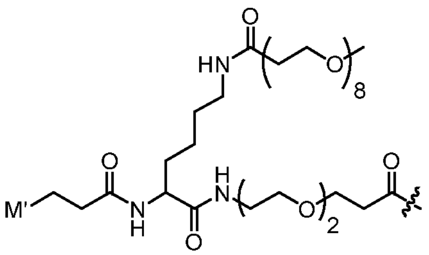
,
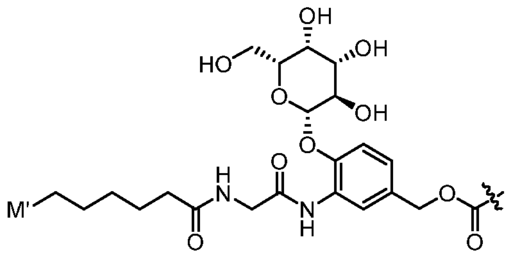
,

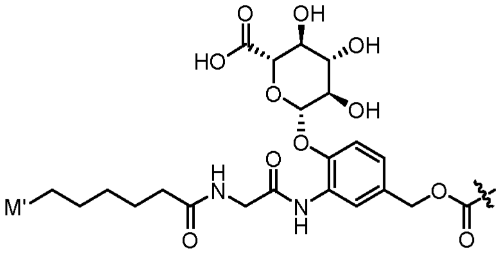
,
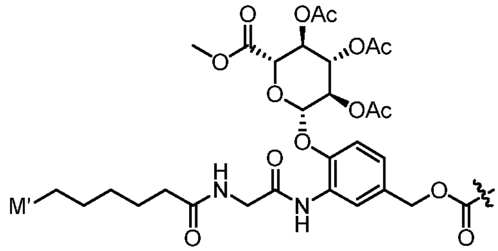
,

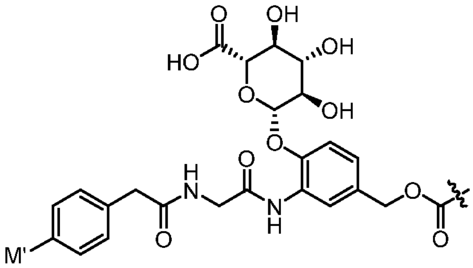
,
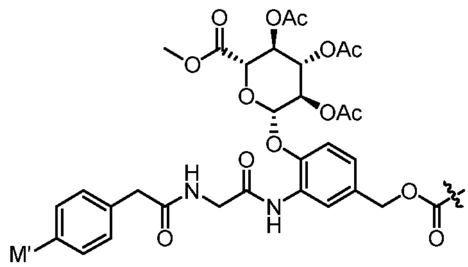
,

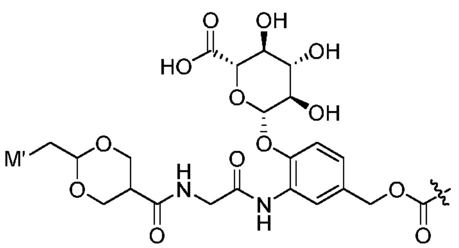
,
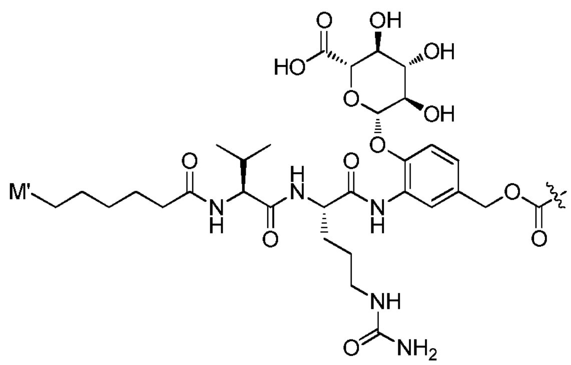
,

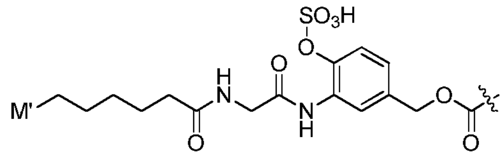
,
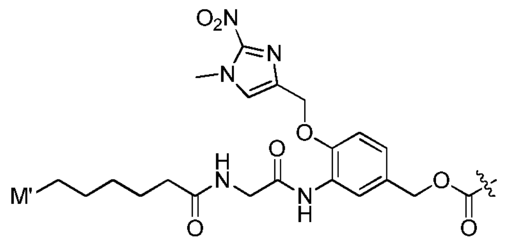
,

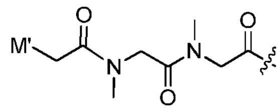
,
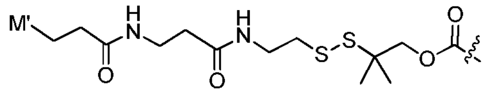
,

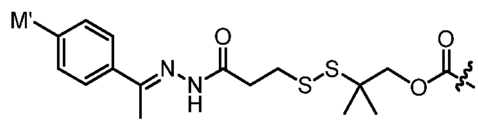
,
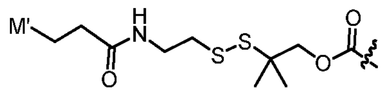
,
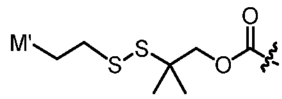
,

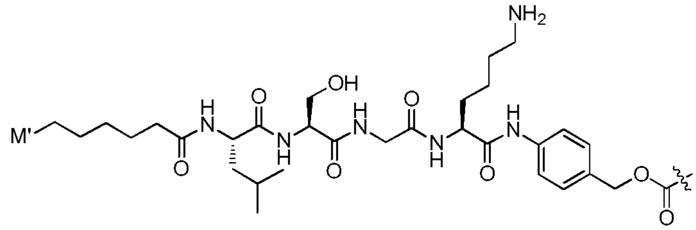
,

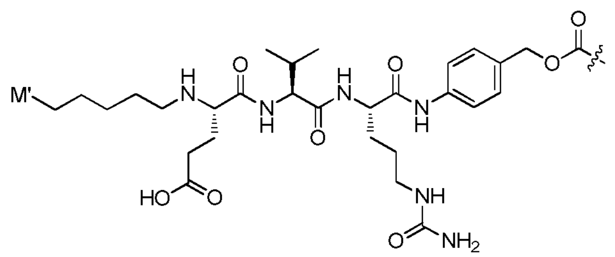
,
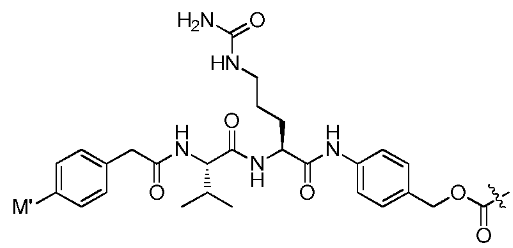
,

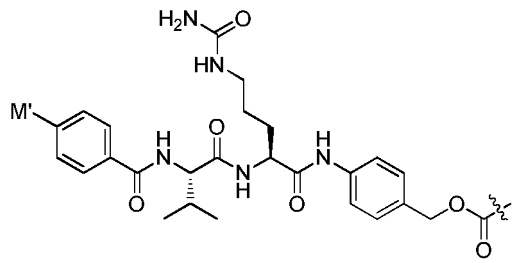
,
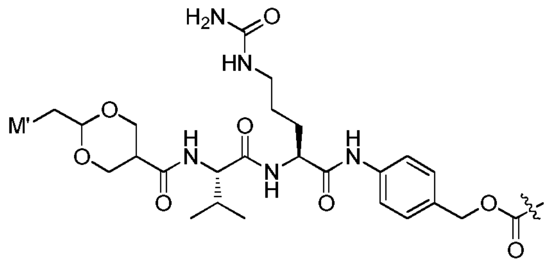
,

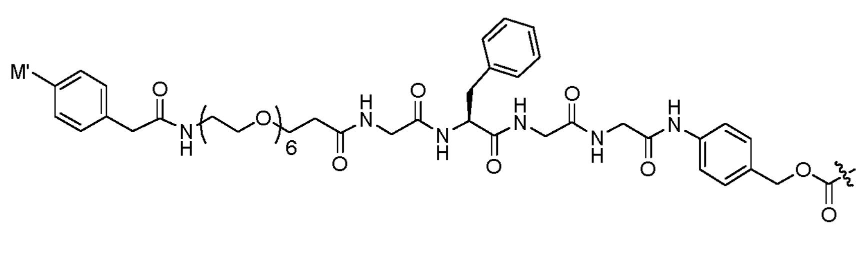
,

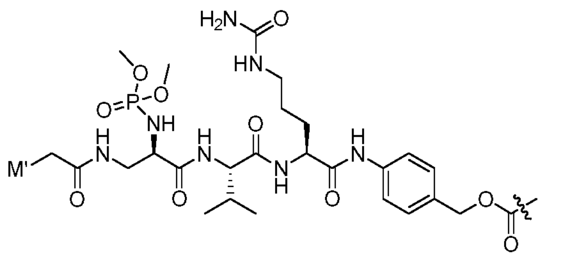,

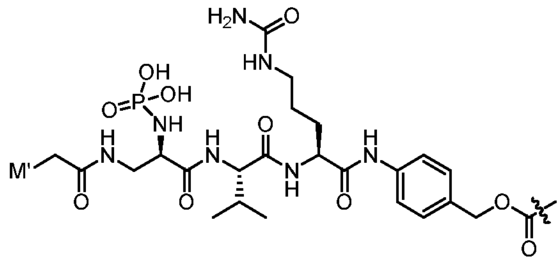,

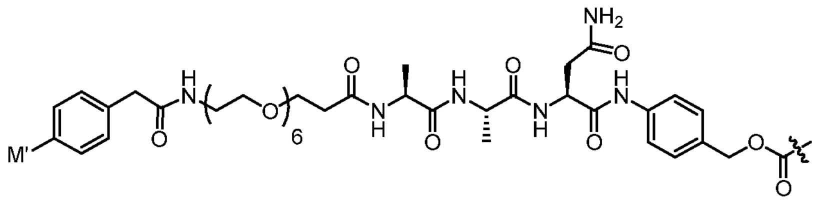,

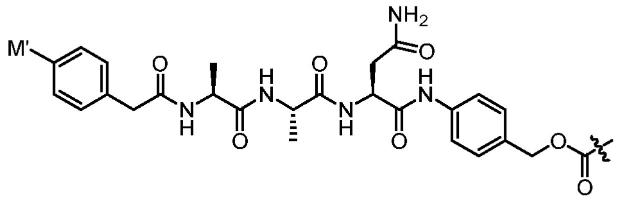,

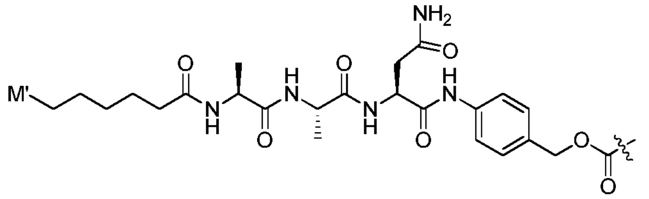,

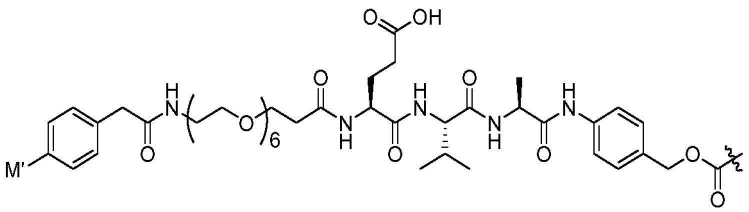

or

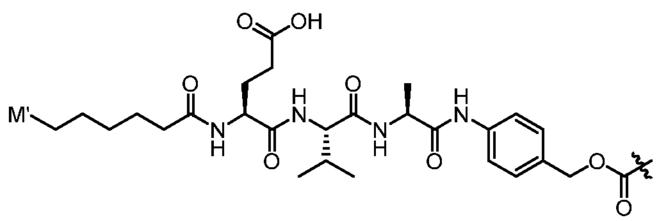.

In some embodiments, LX is

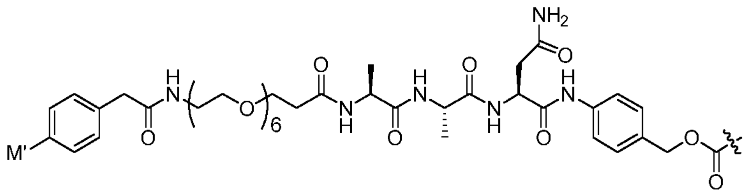,

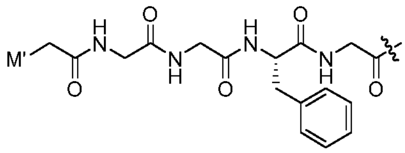,

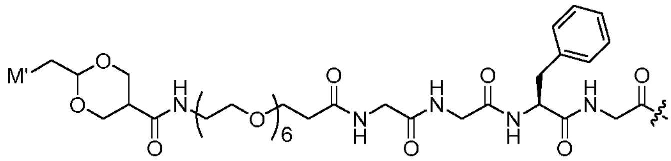,

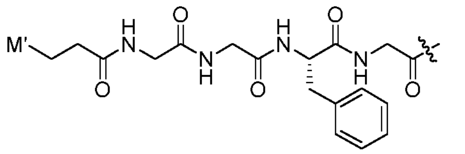,

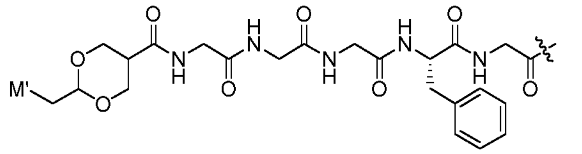,

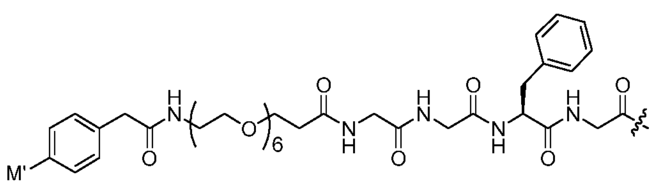,

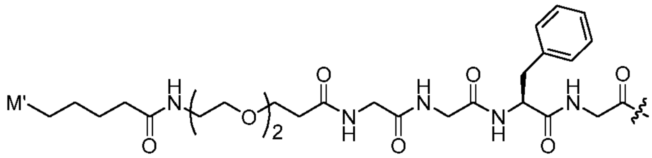,

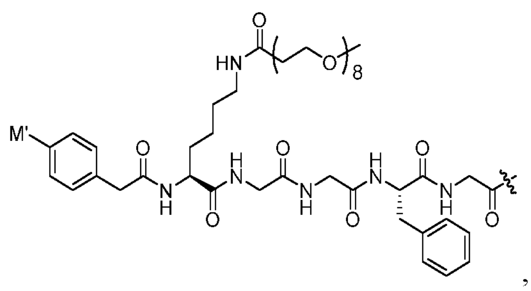,

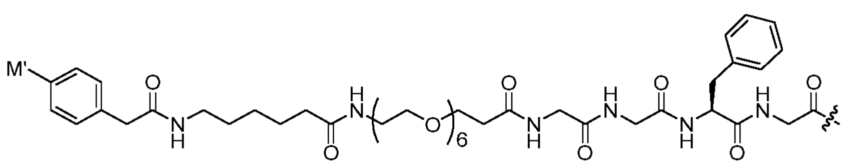,

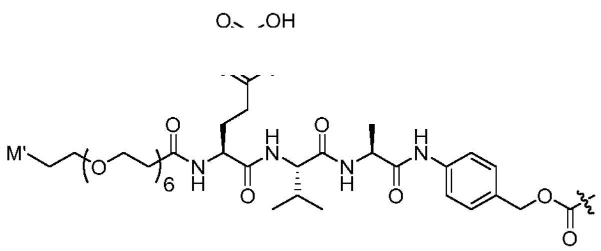,

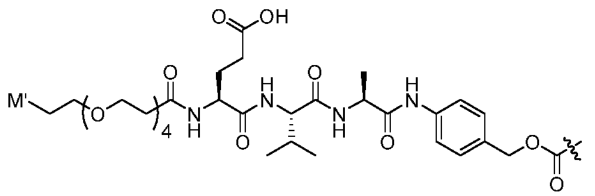

,

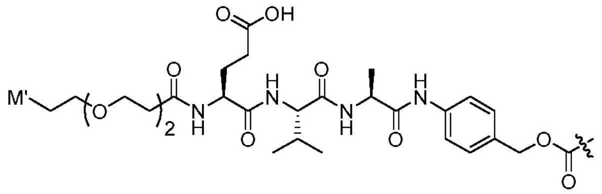

,

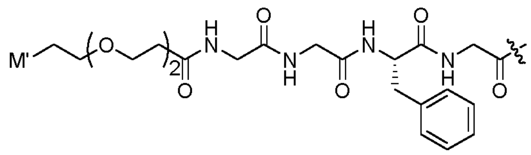

,

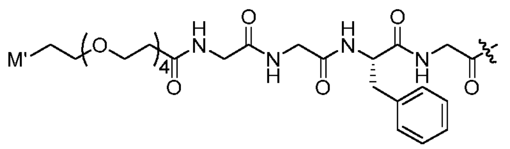

,

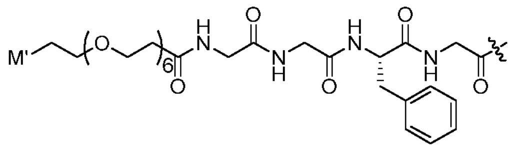

,

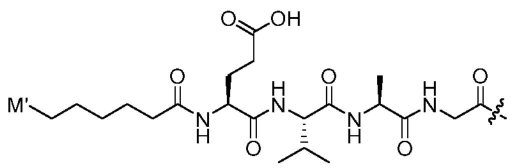

,

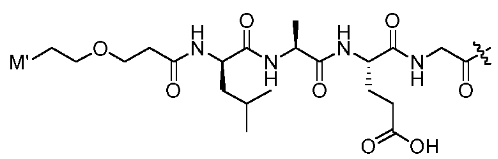

,

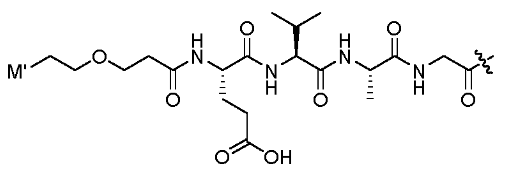

,

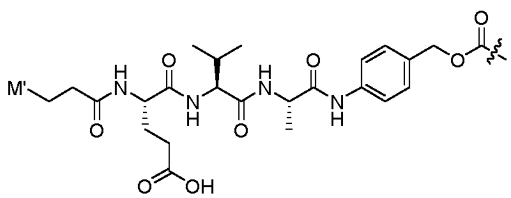

,

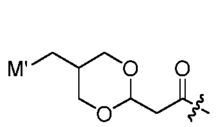

,

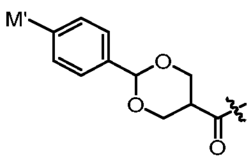

,

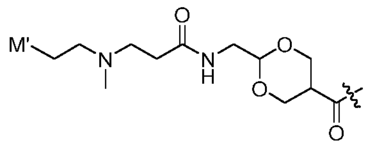

,

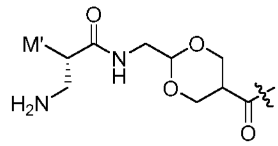

,

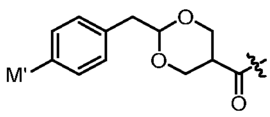

or

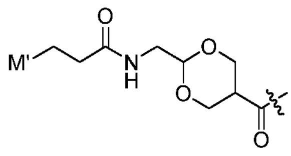

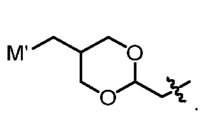

.

In some embodiments, the compound of formula II is any one of compounds of:

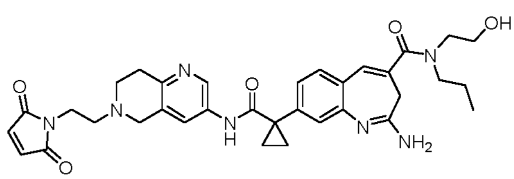

,

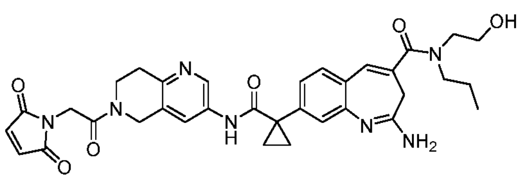

,

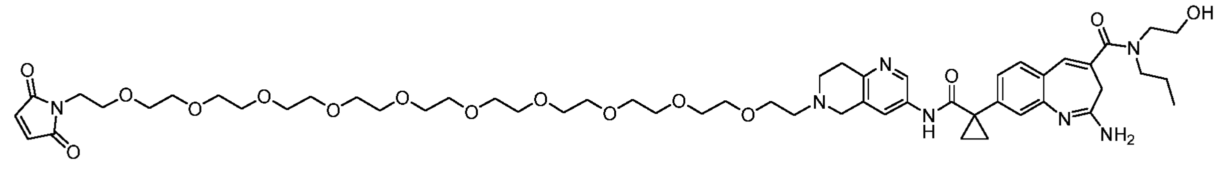

,

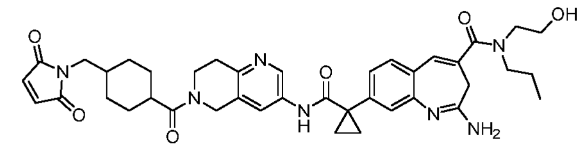

,

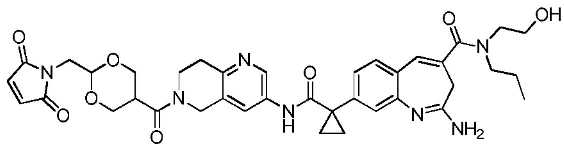

,

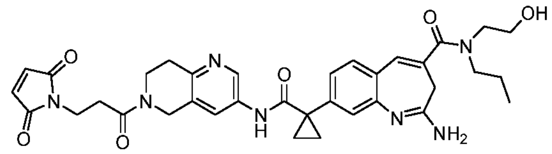

,

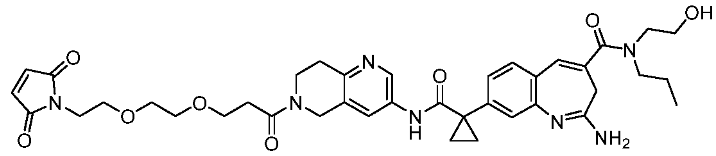

,

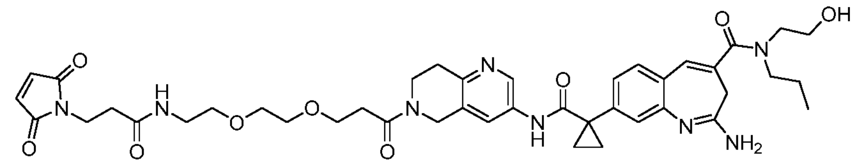

,

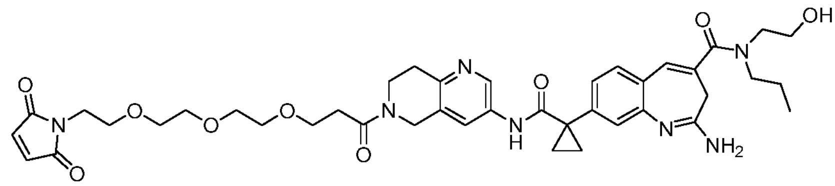

,

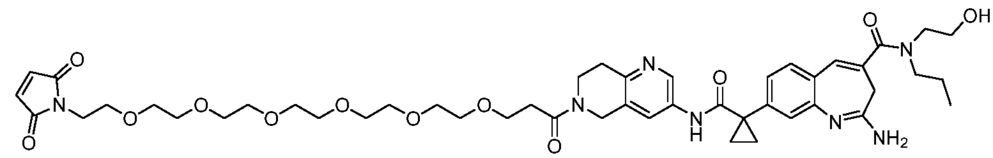

,

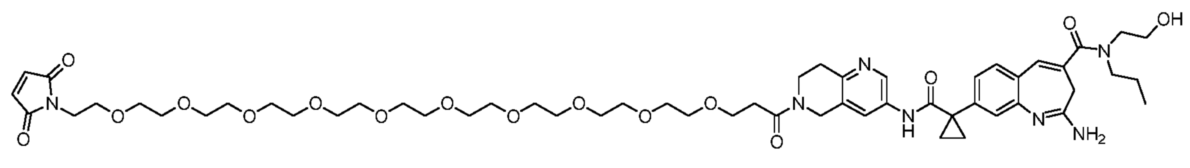

,

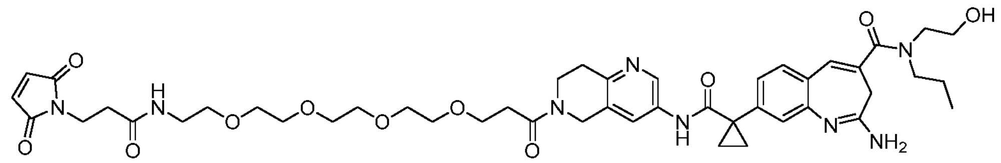

,

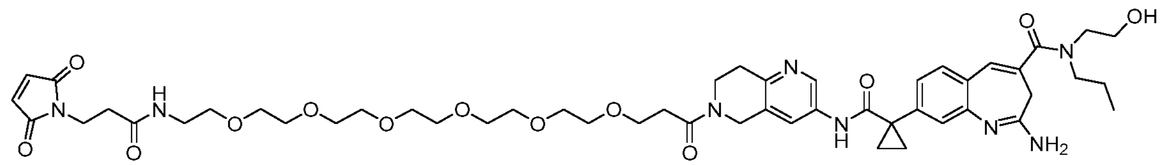

,

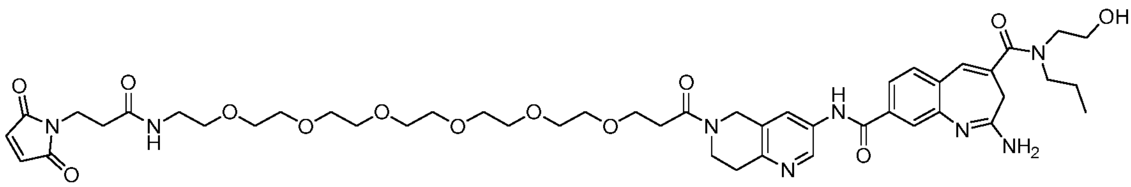

,

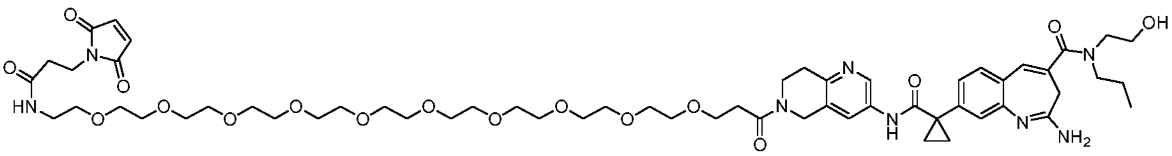

,

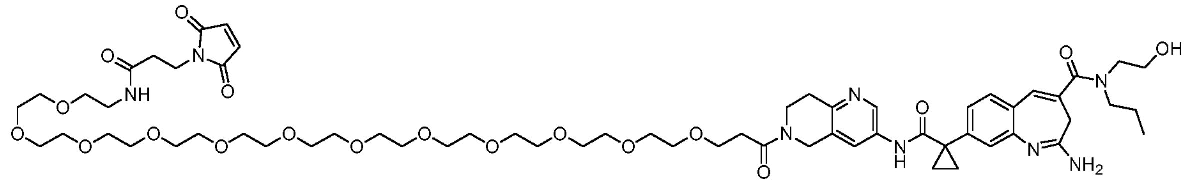

,

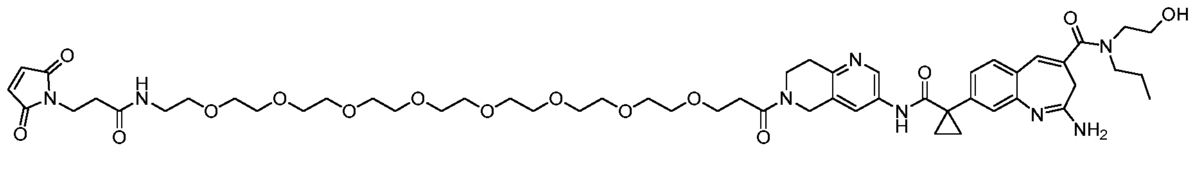

,

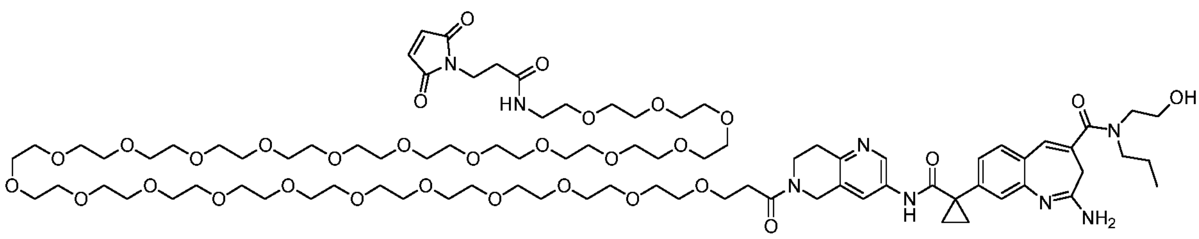

,

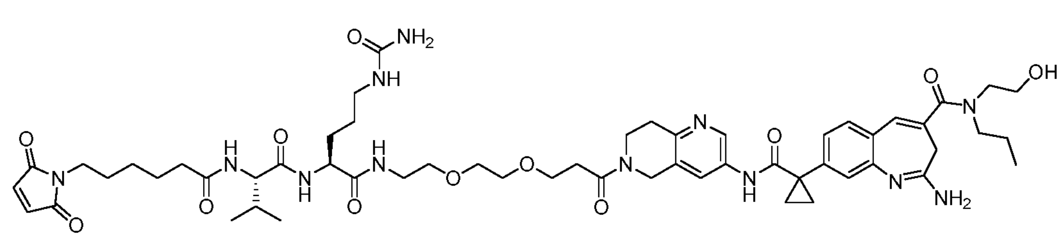

,

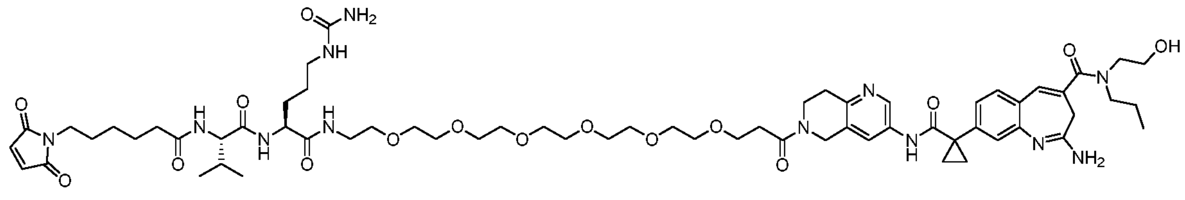

,

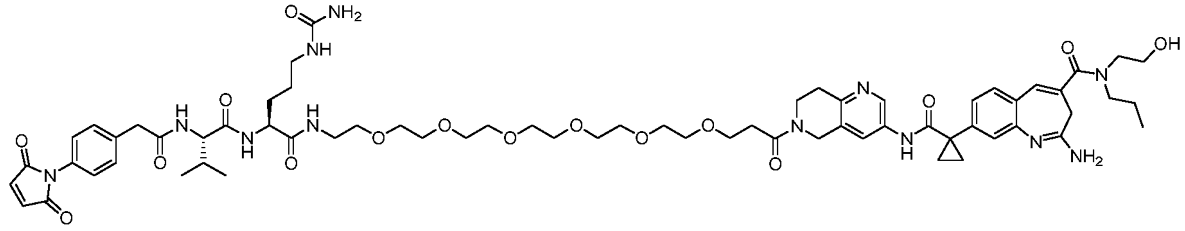

,

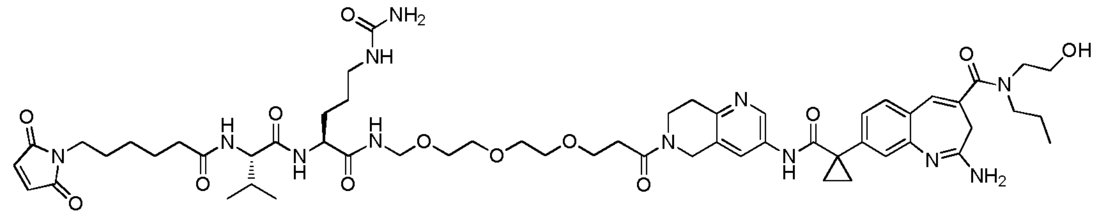

,

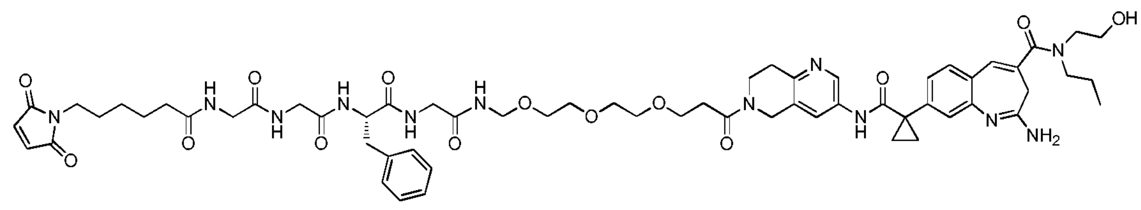

,

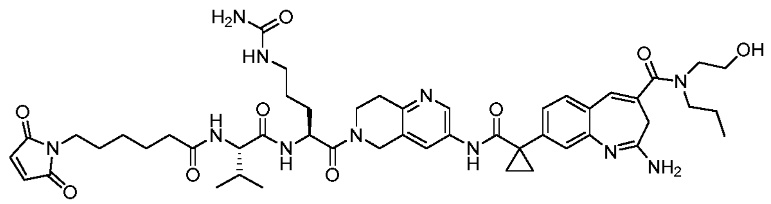

,

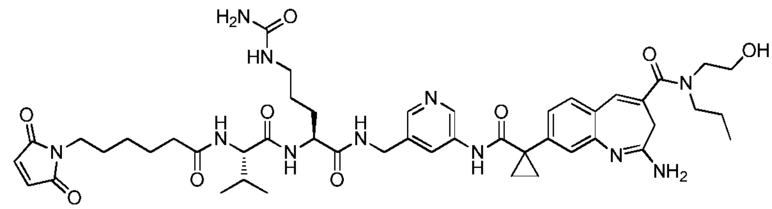

,

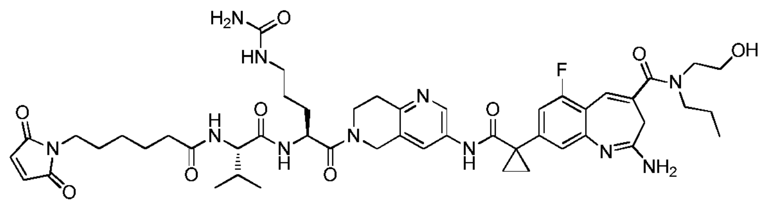

,

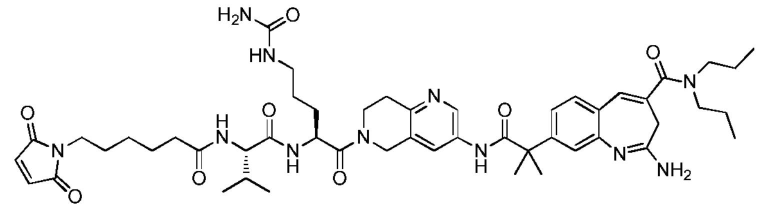

,

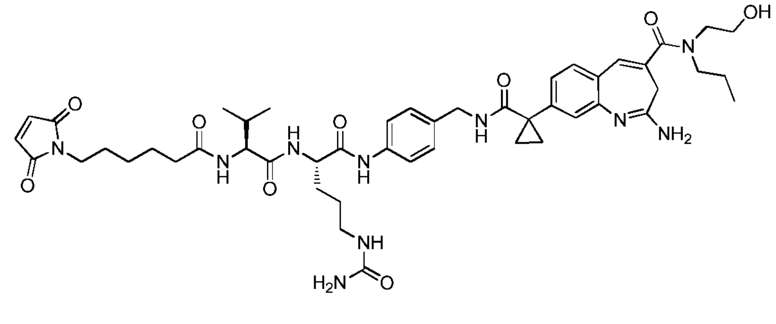

,

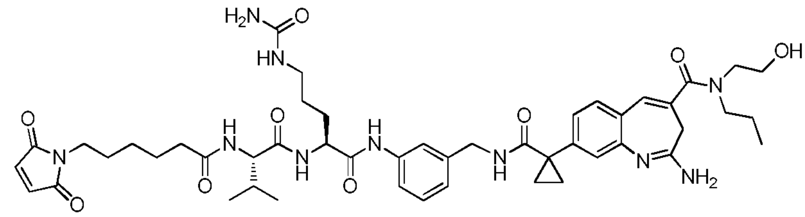

,

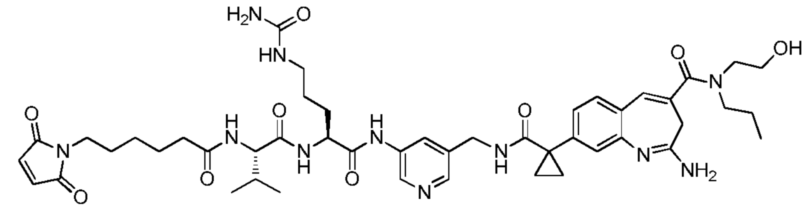

,

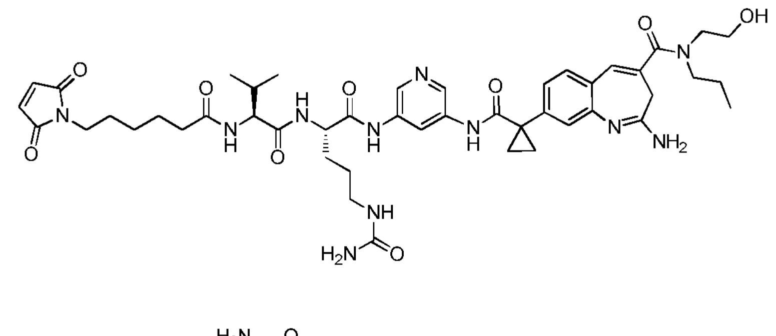

,

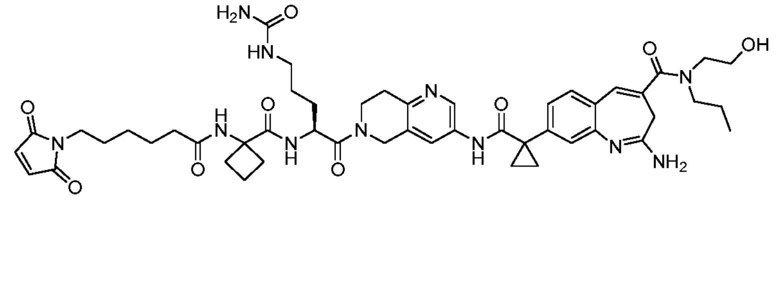

,

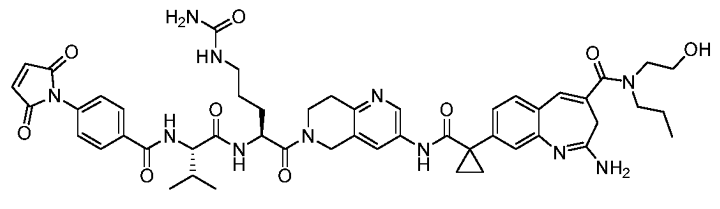

,

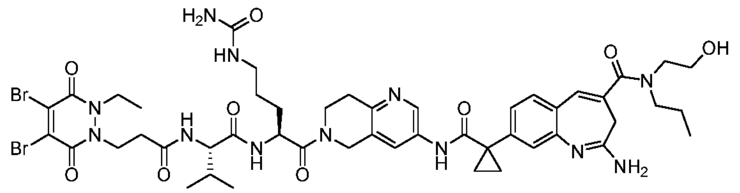

,

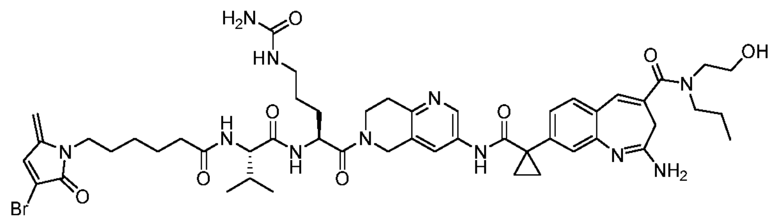

,

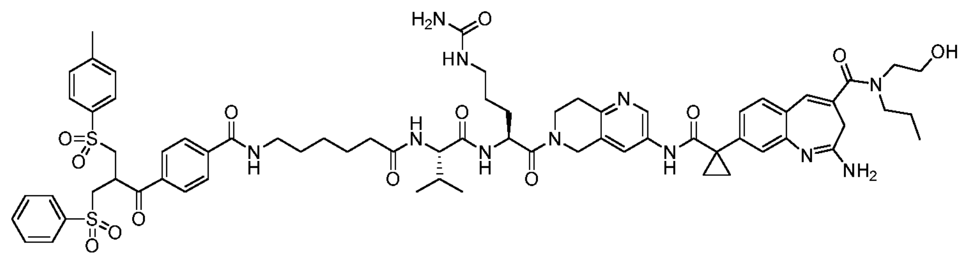

,

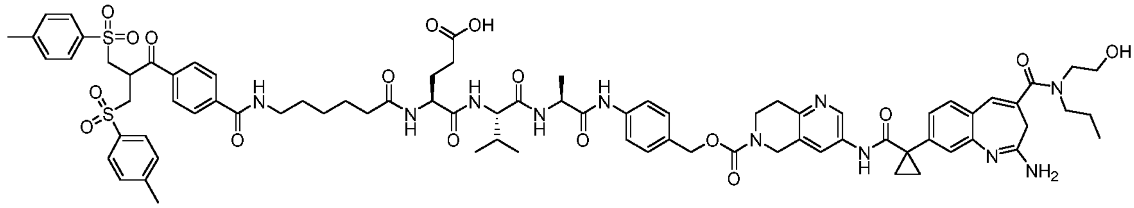

,

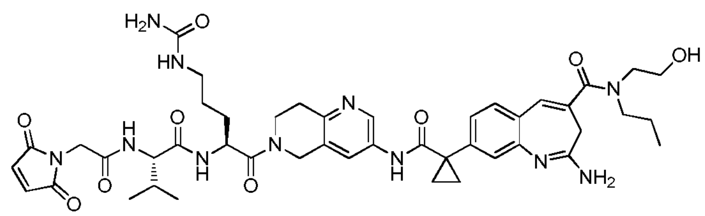

,

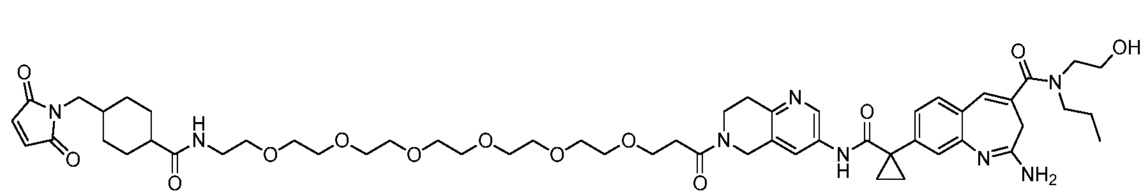

,

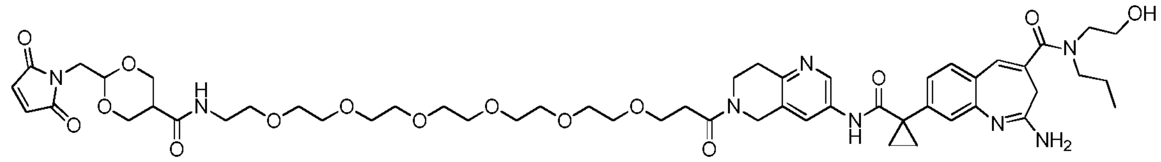

,

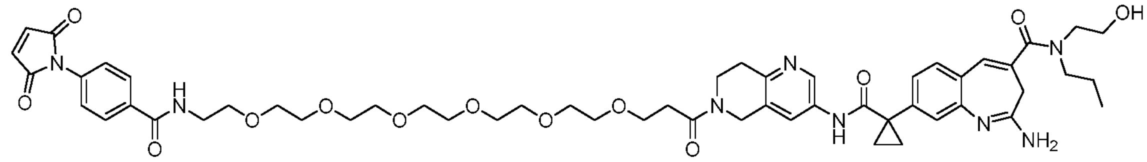

,

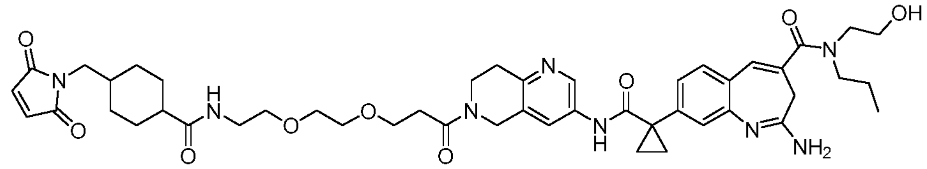

,

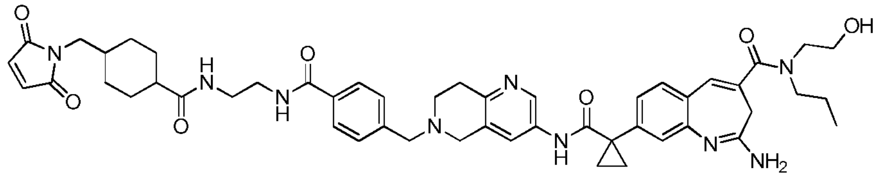

,

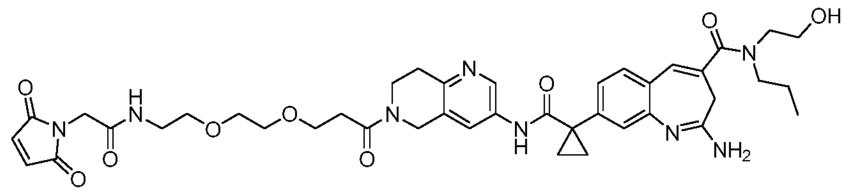

,

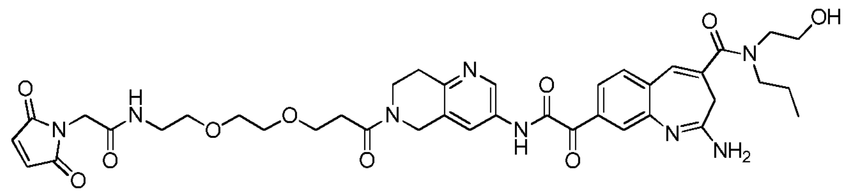

,

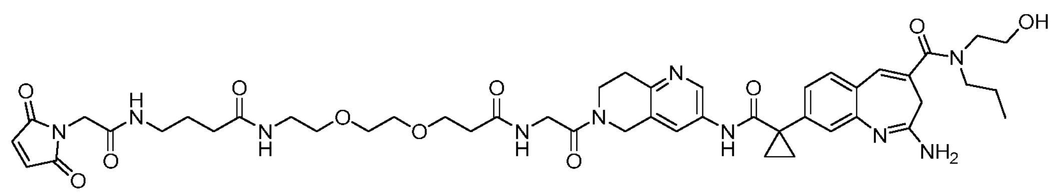

,

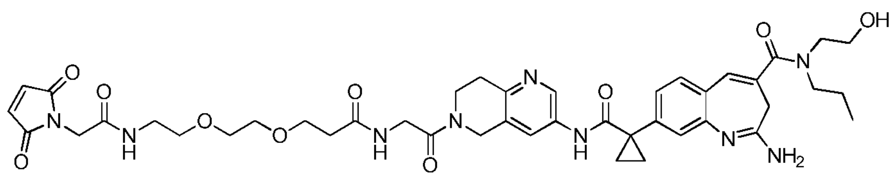

,

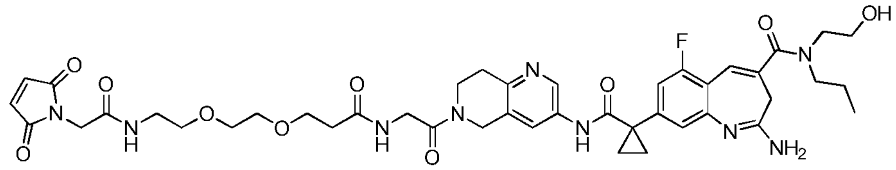

,

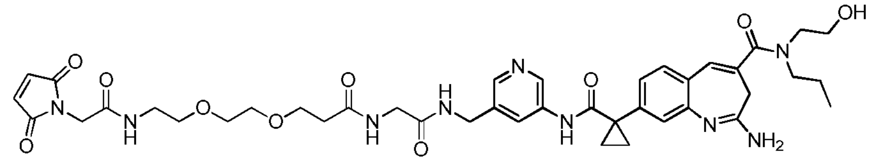

,

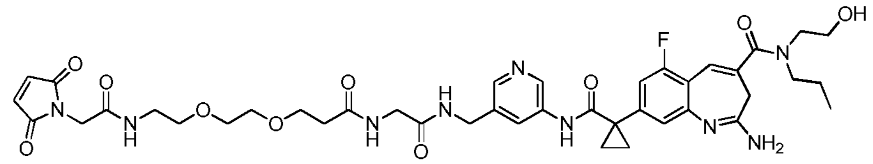

,

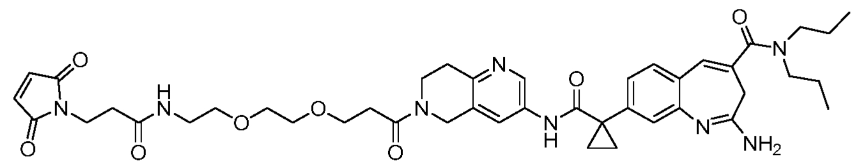

,

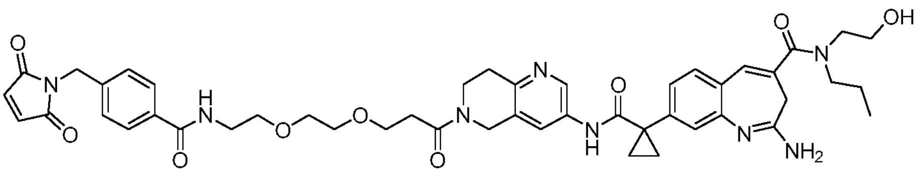

,

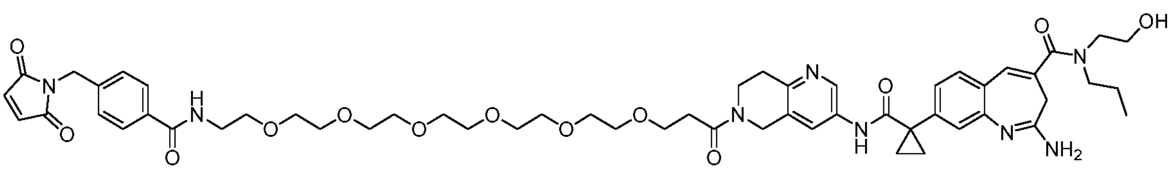

,

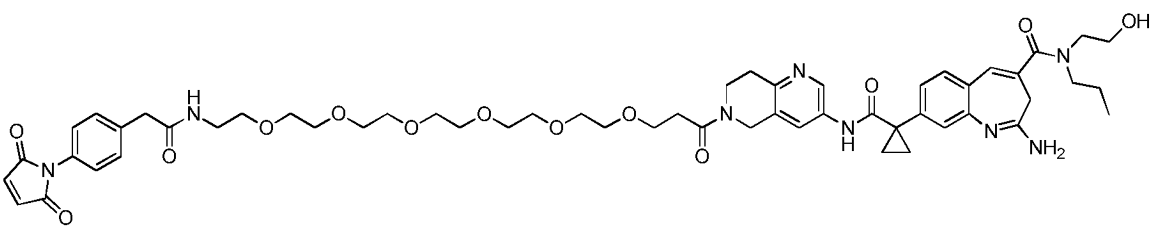

,

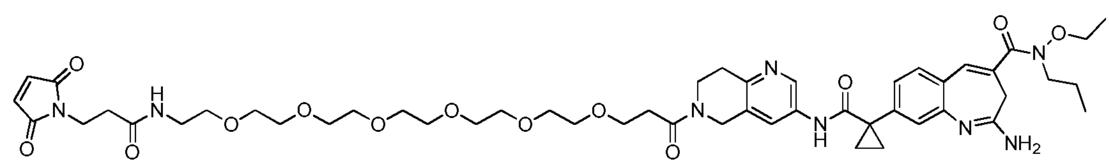

,

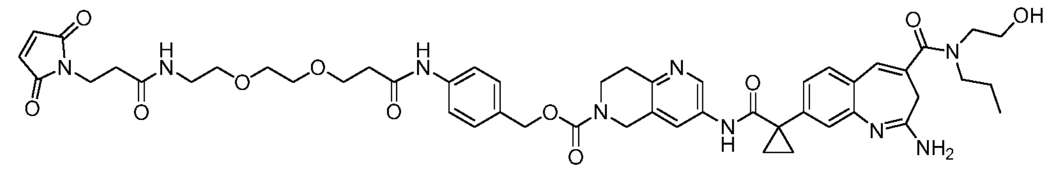

,

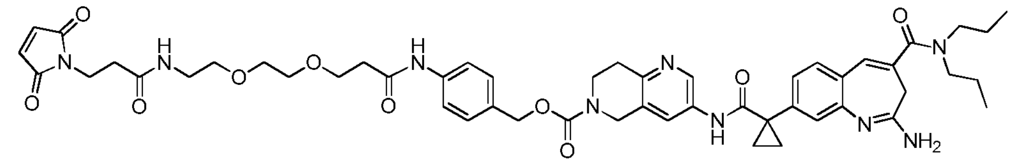

,

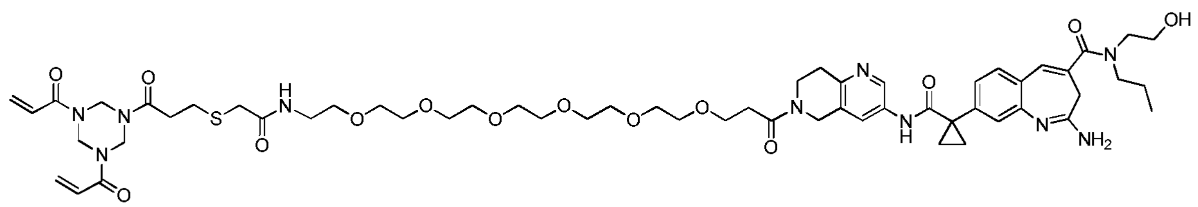

,

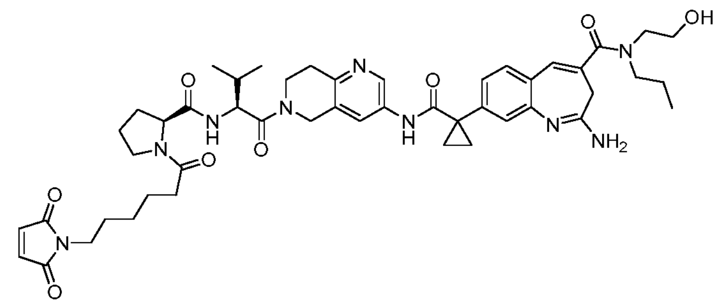

,

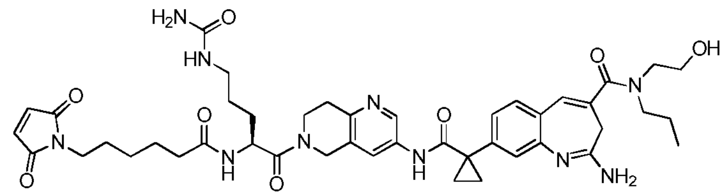

,

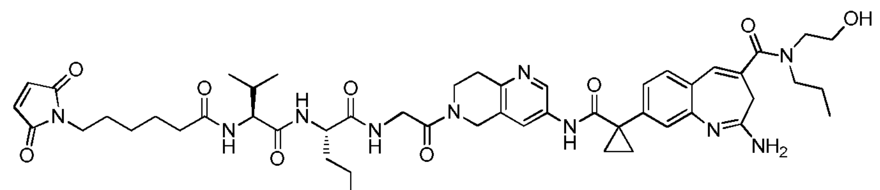

,

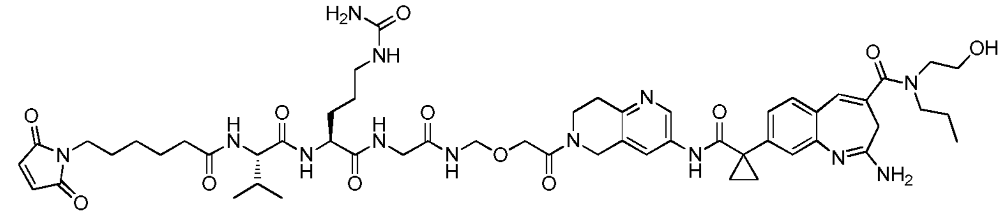

,

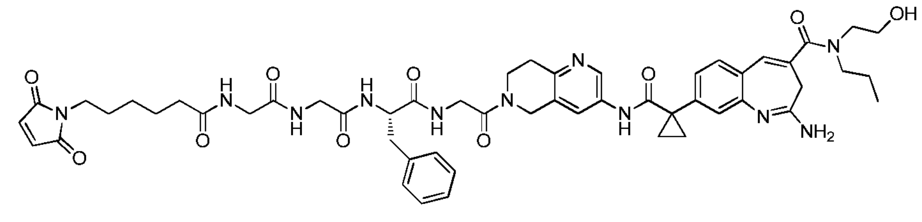

,

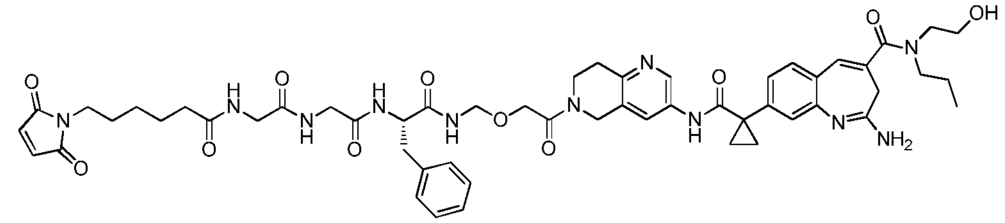

,

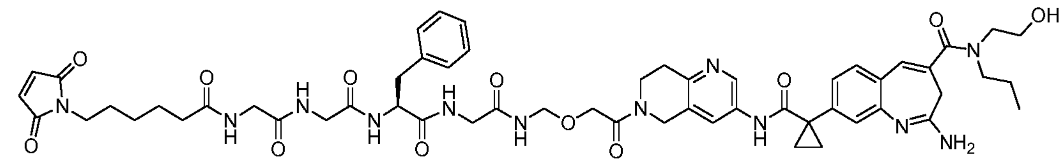

,

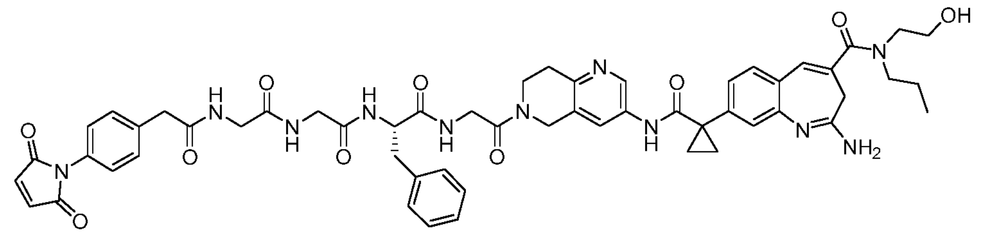

,

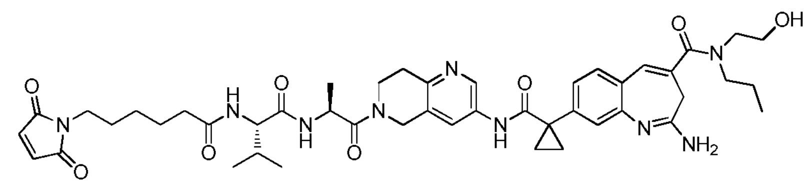

,

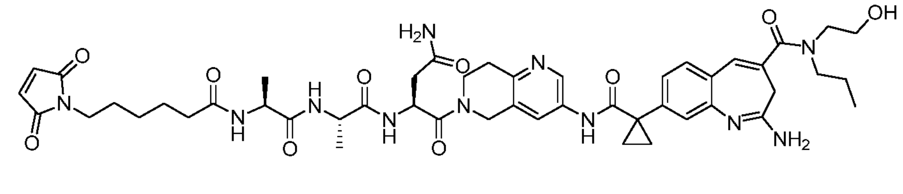

,

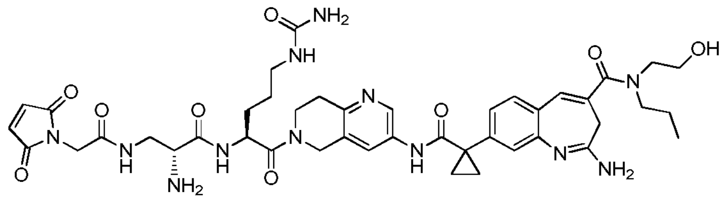

,

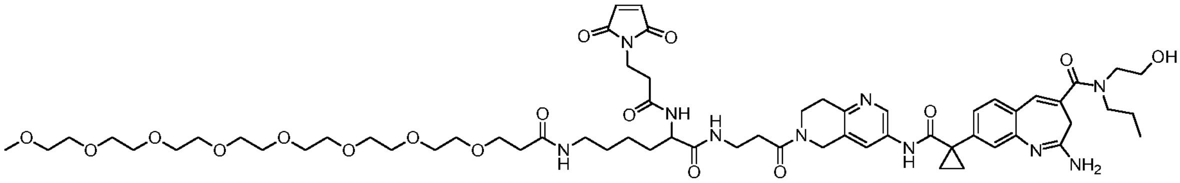

,

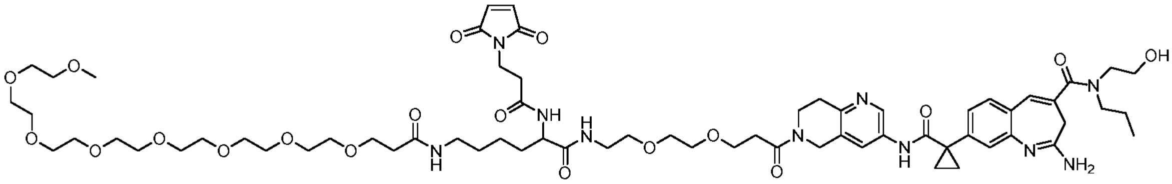

,

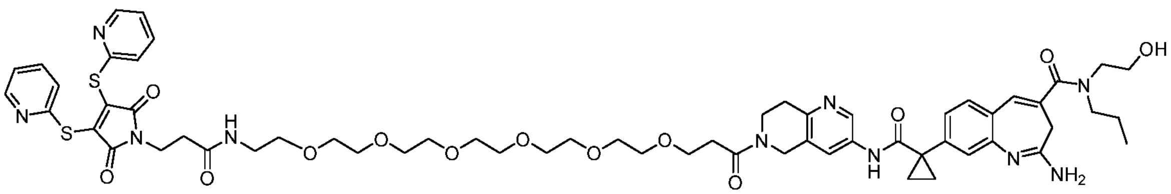

,

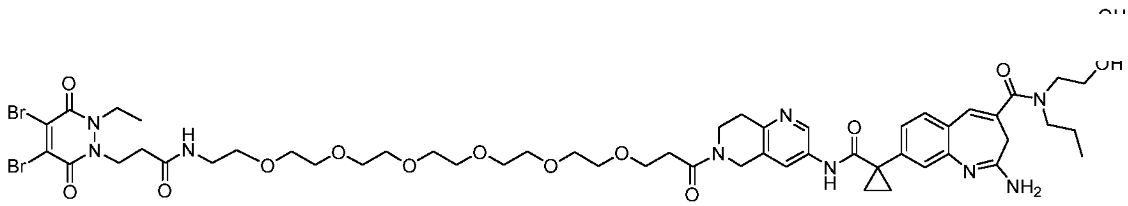

,

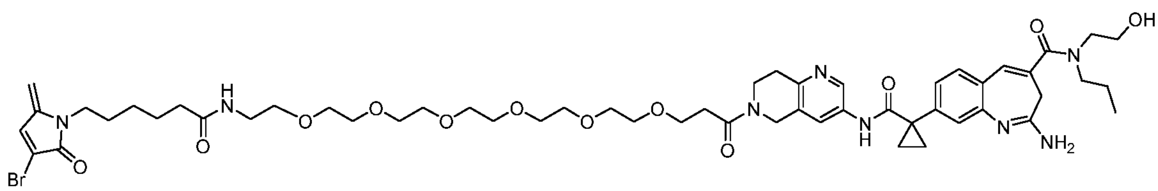

,

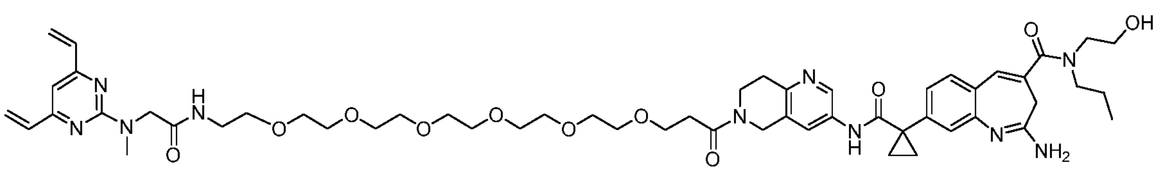

,

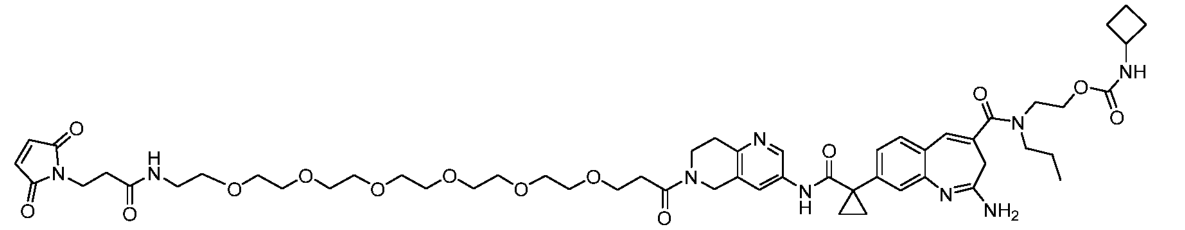

,

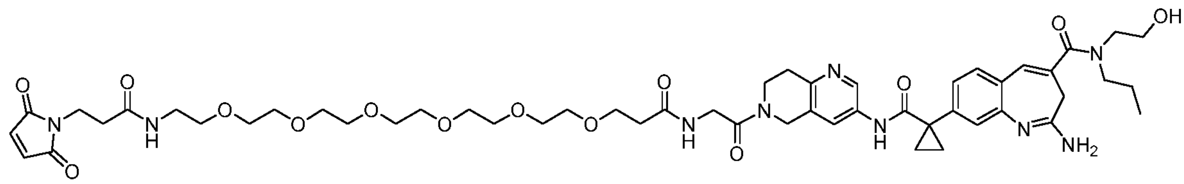

,

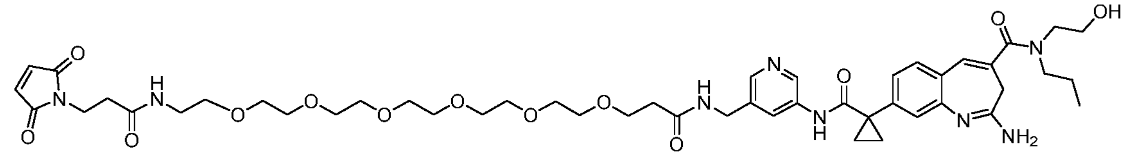

,

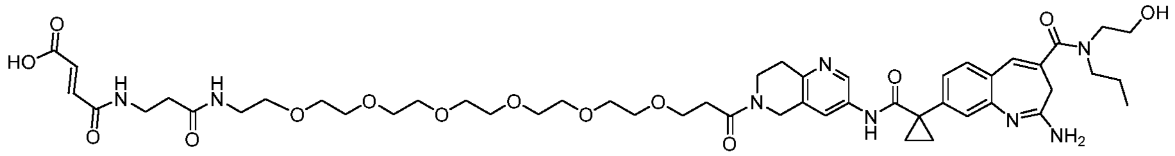

,

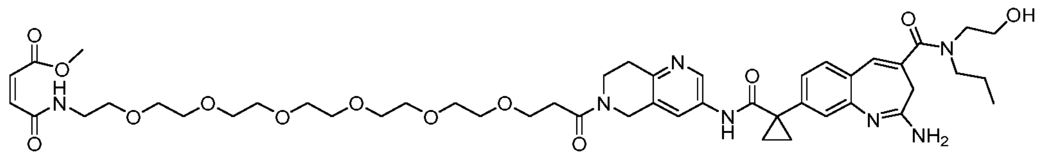

,

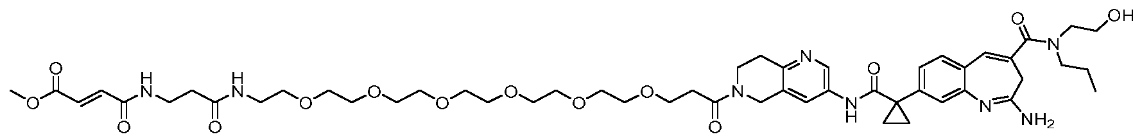

,

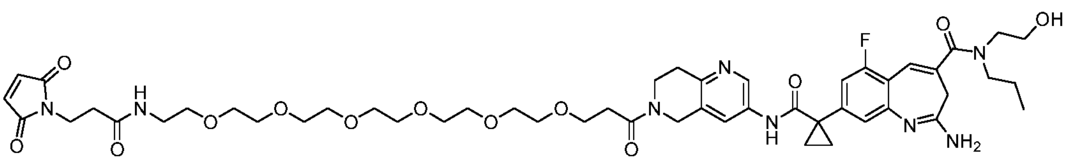

,

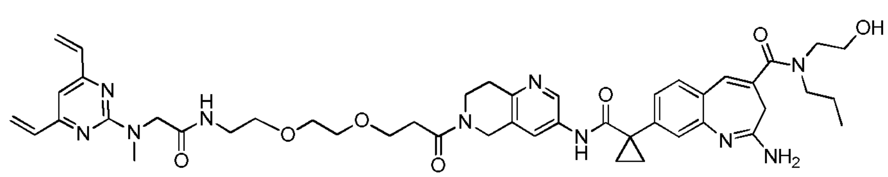

,

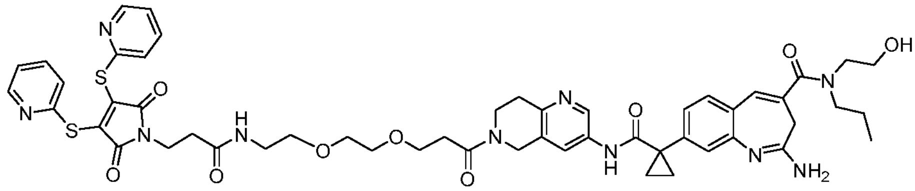

,

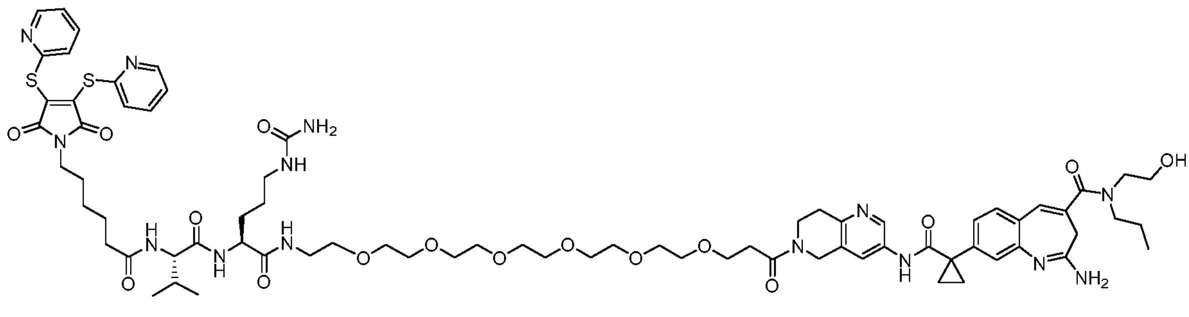

,

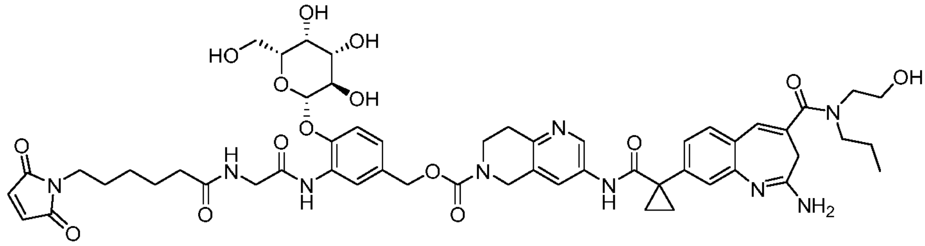

,

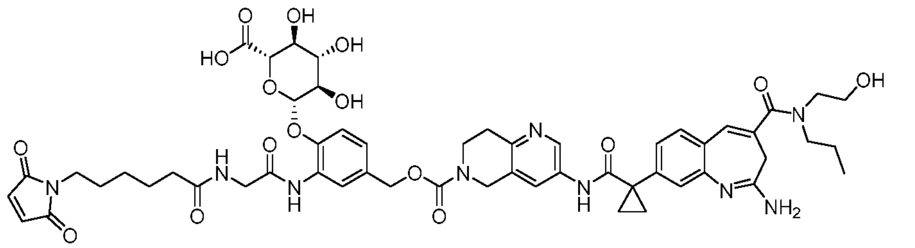

,

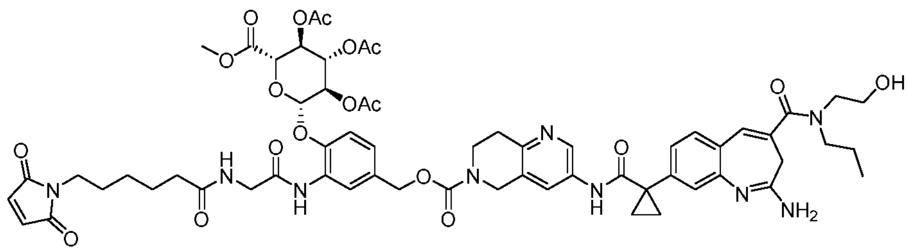

,

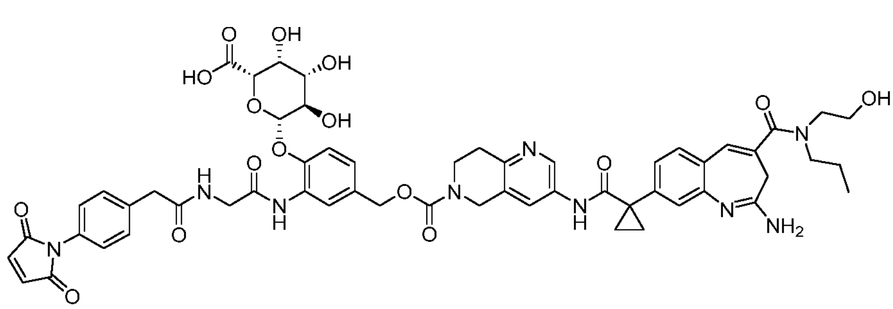

,

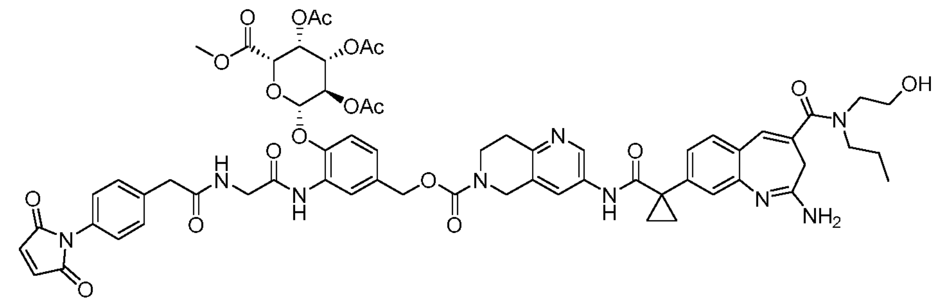

,

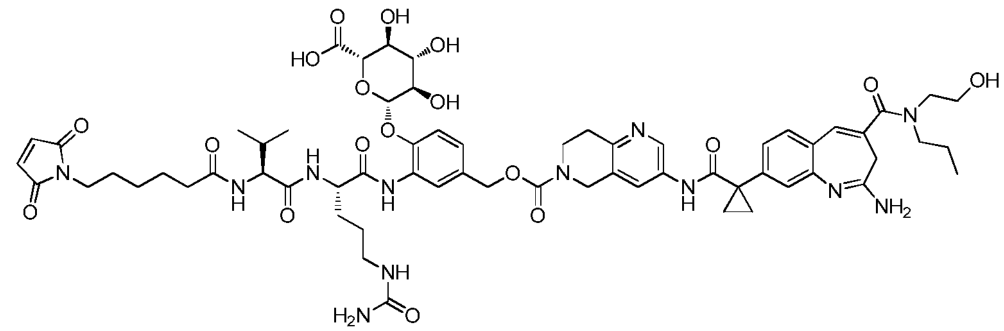

,

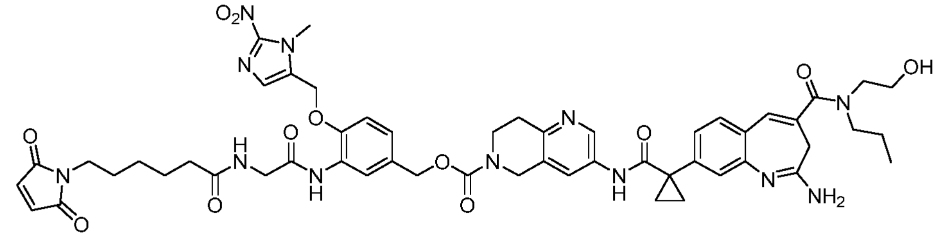

,

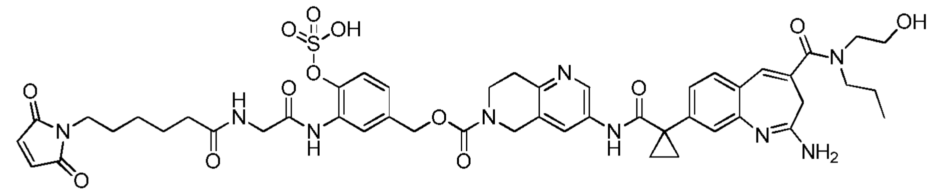

,

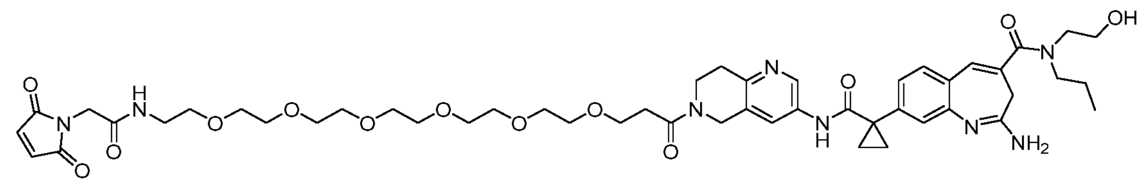

,

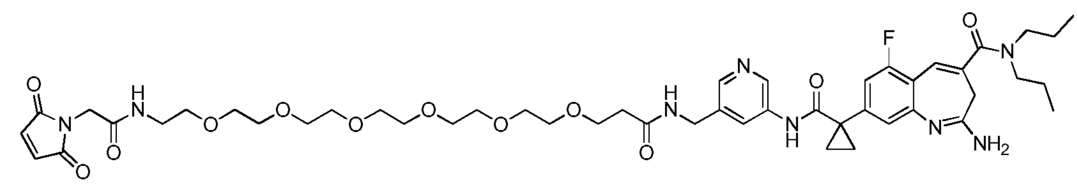

,

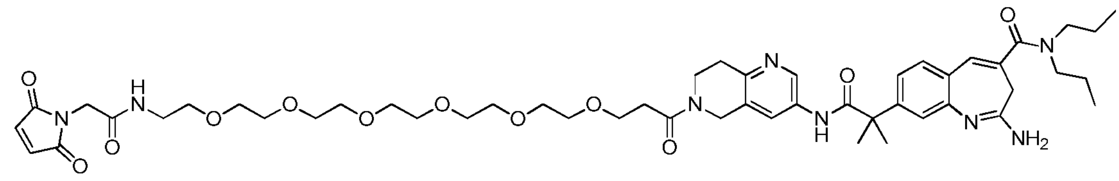

,

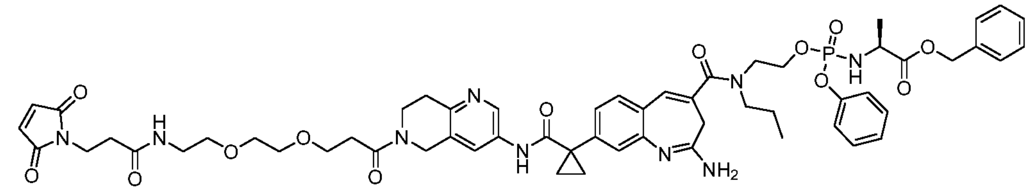

,

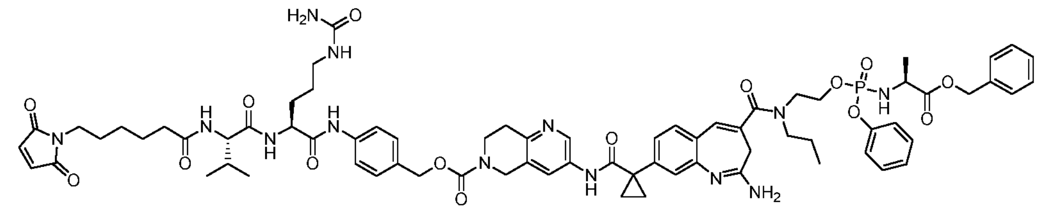

,

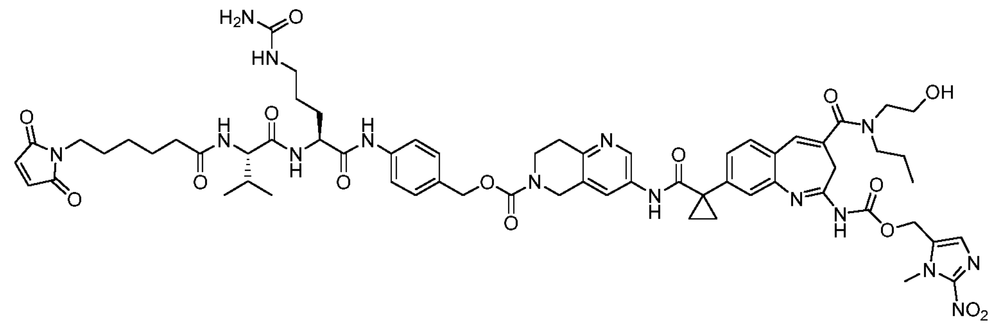

,

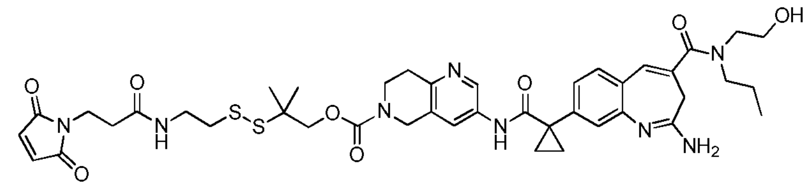

,

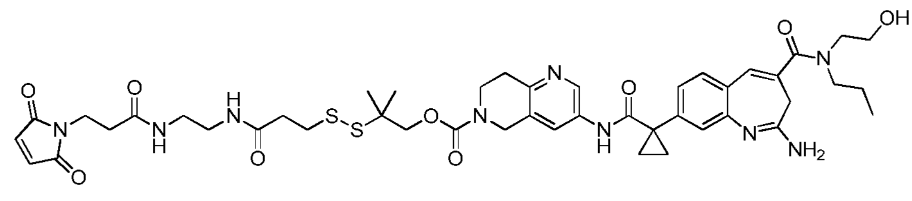

,

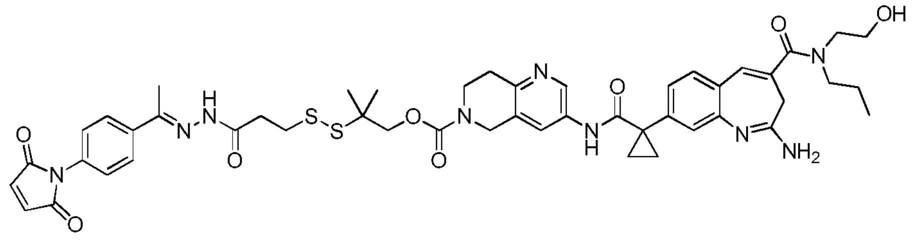

,

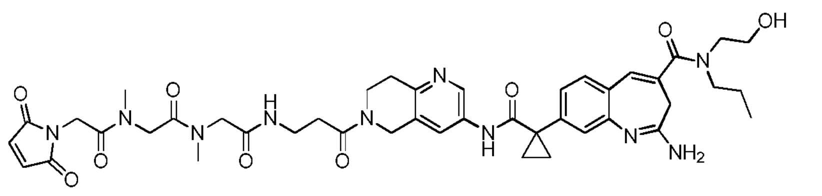

,

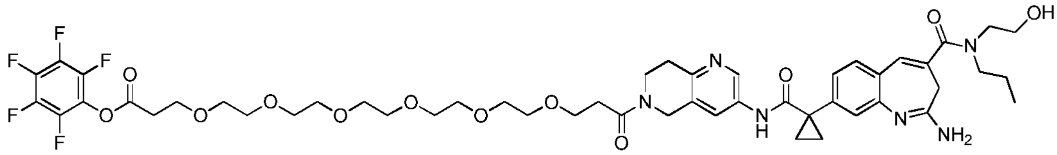

,

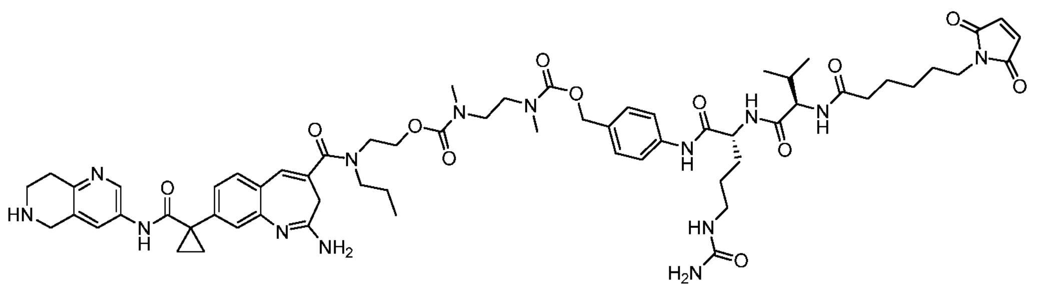

,

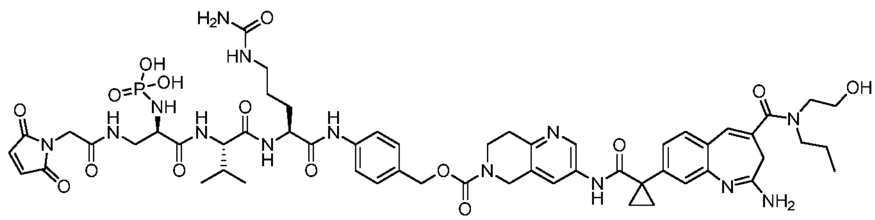

,

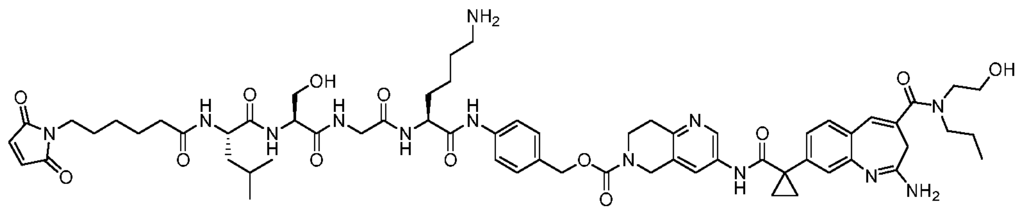

,

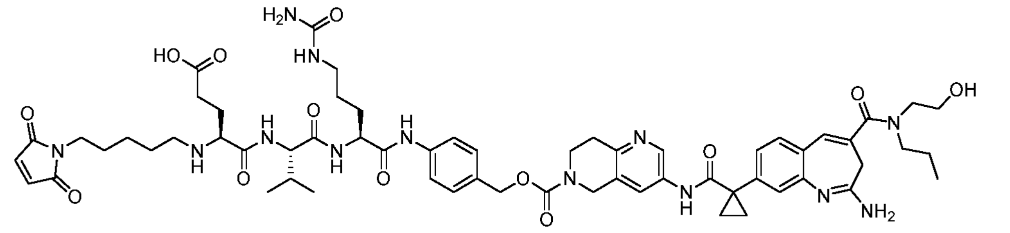

,

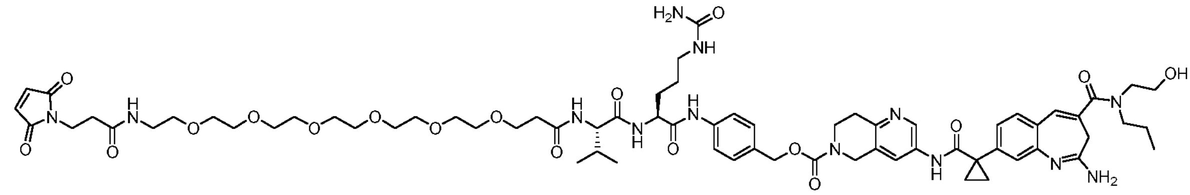

,

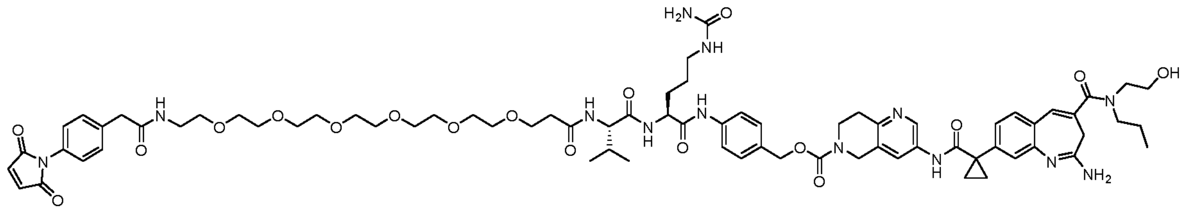

,

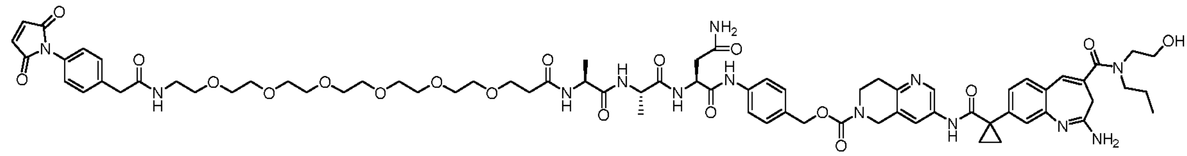

,

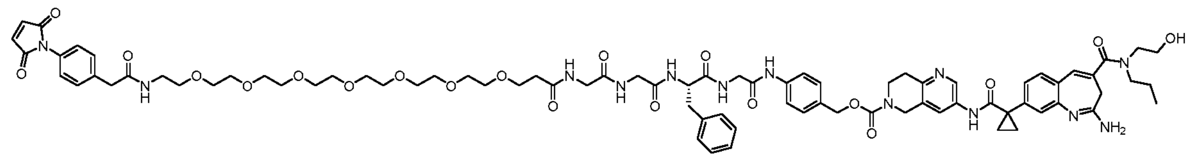

,

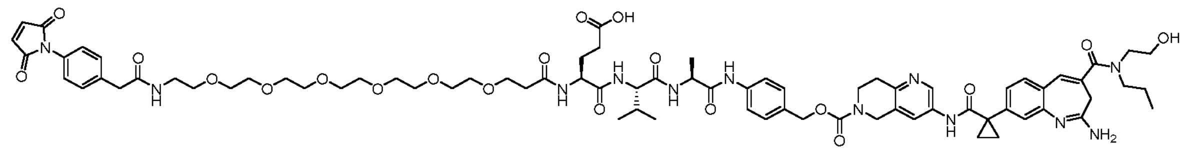

,

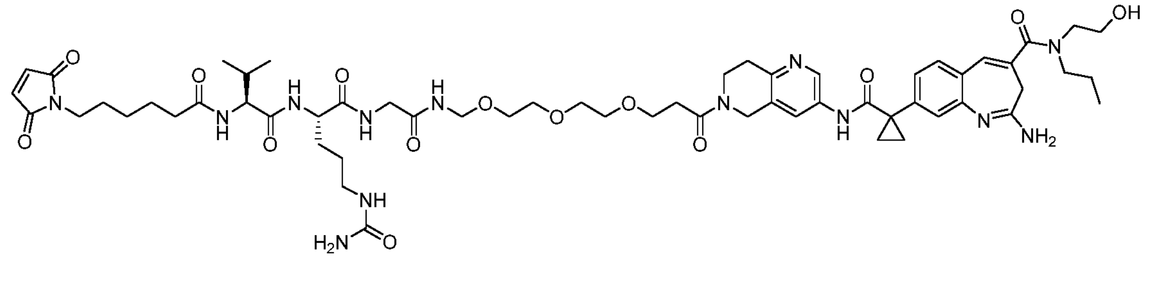

,

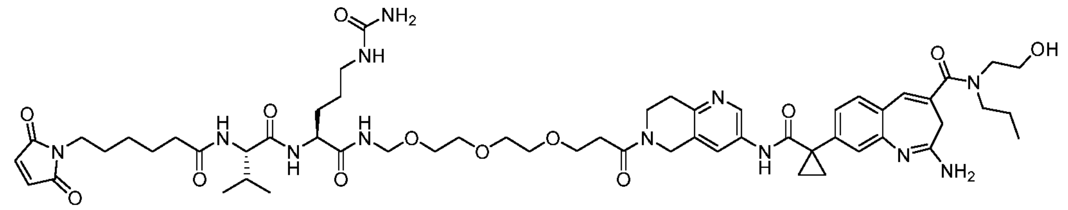

,

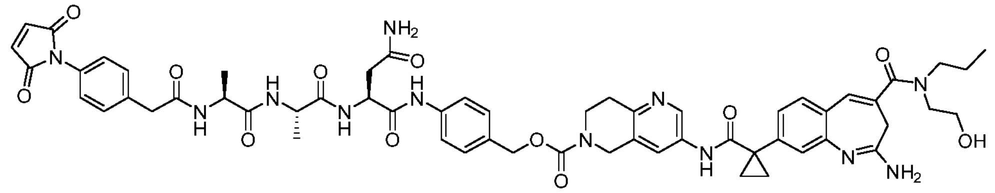

,

or a pharmaceutically acceptable salt thereof.

In some embodiments, the compound of formula II is any one of compounds of:

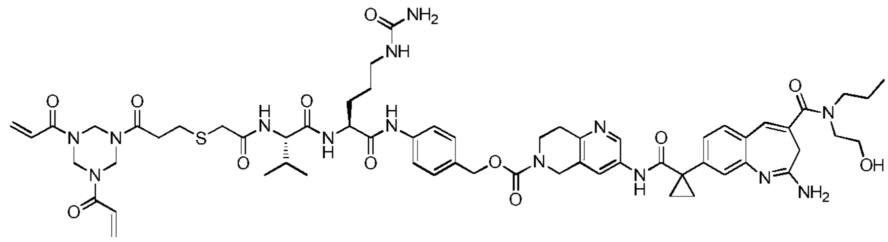

,

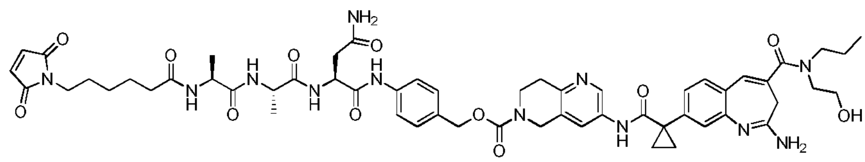

,

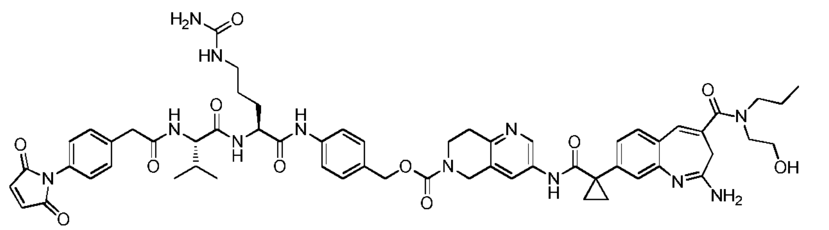

,

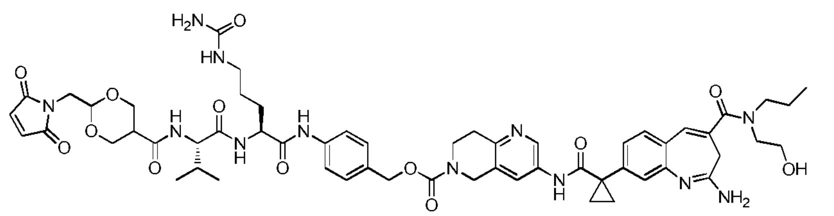

,

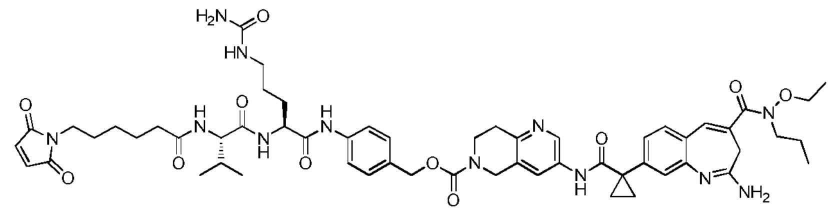

,

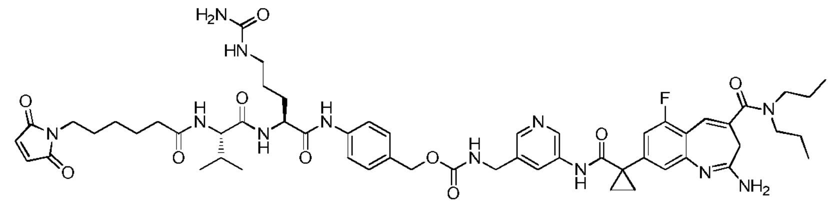

,

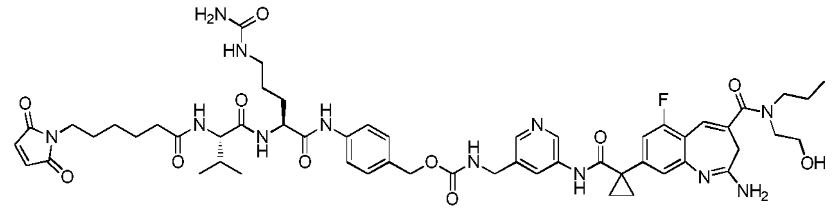

,

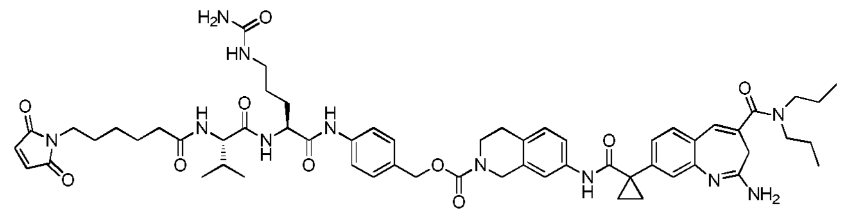

,

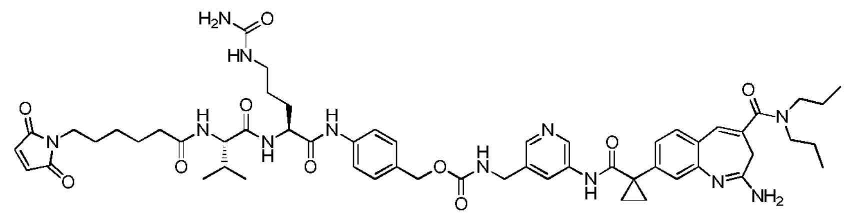

,

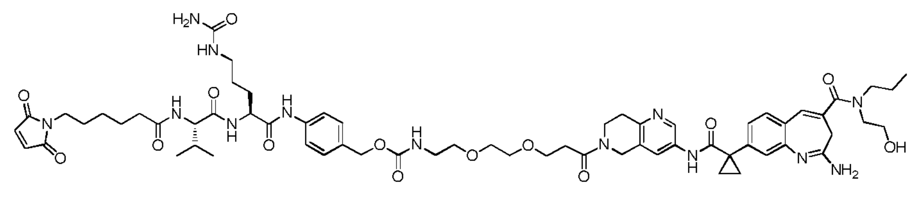

,

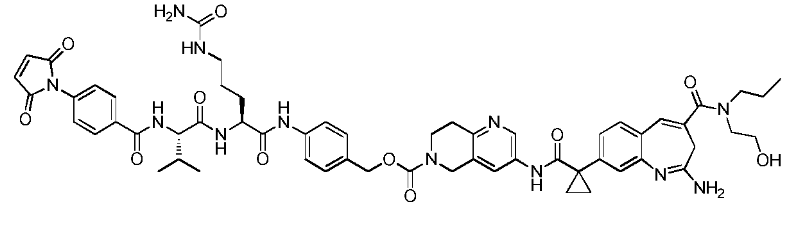

,

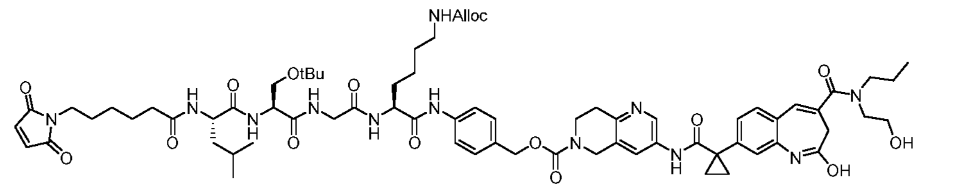

,

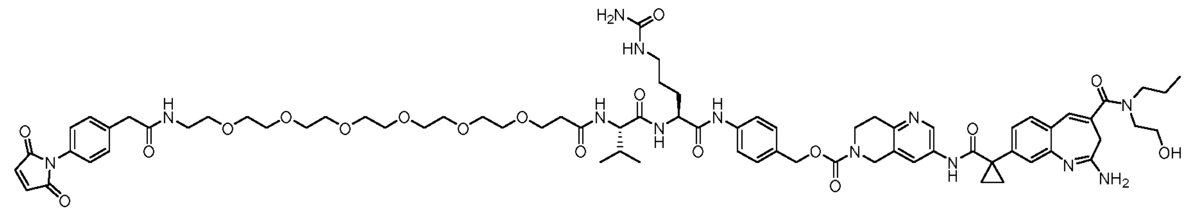

,

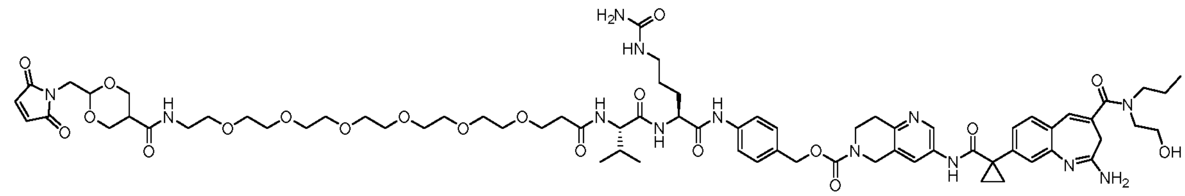

,

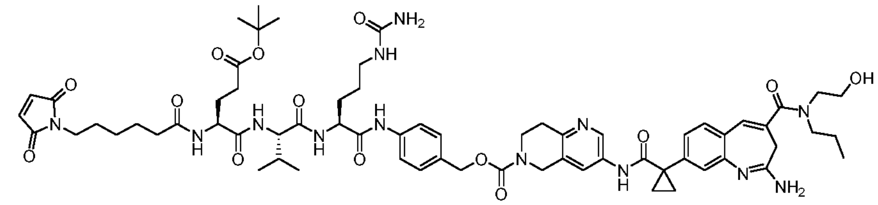

,

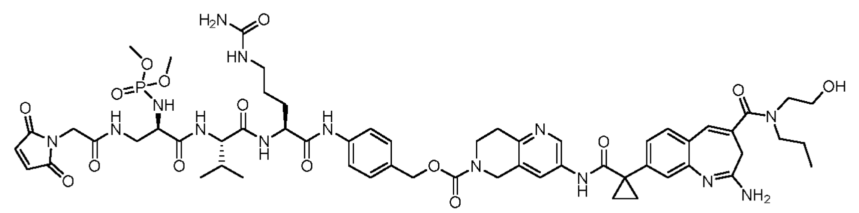

,

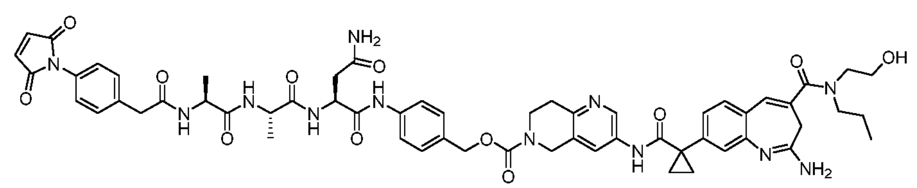

,

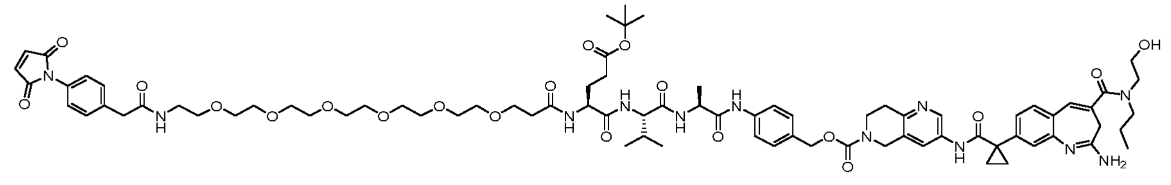

,

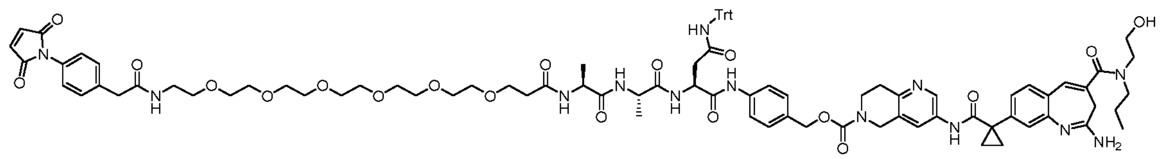

,

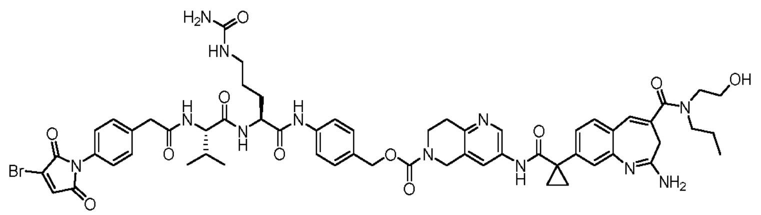

,

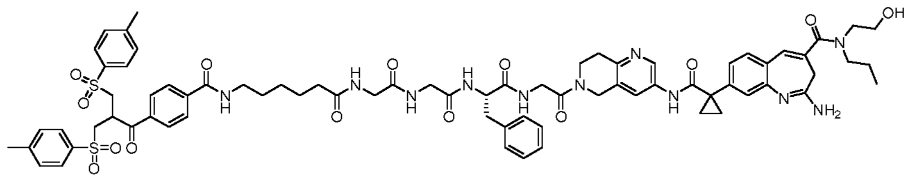

,

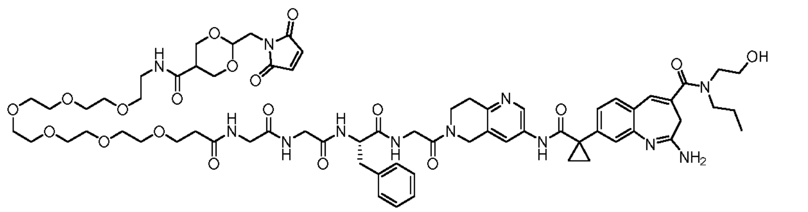

,

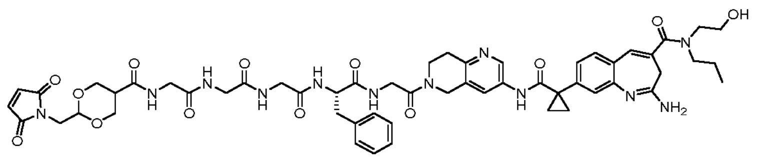

,

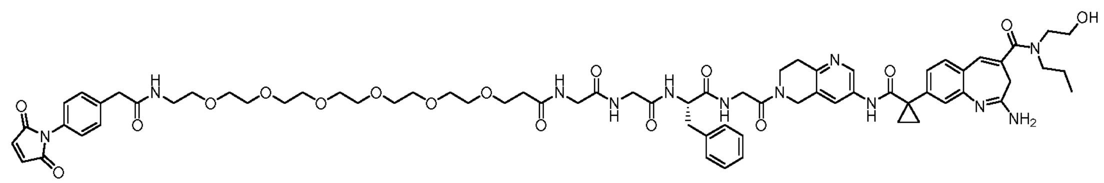

,

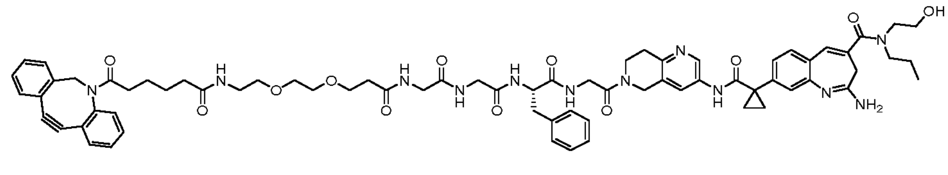

,

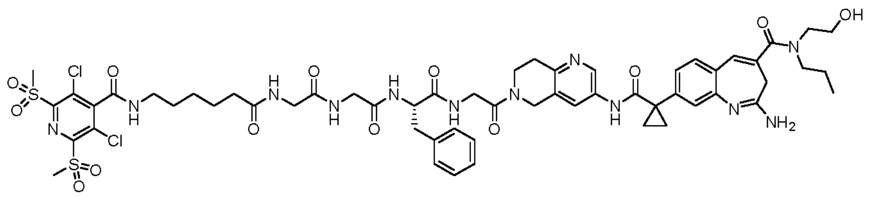

,

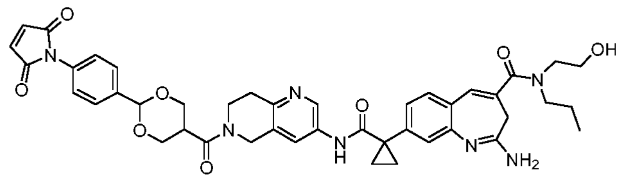

,

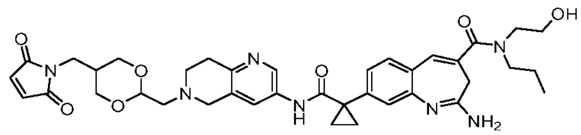

,

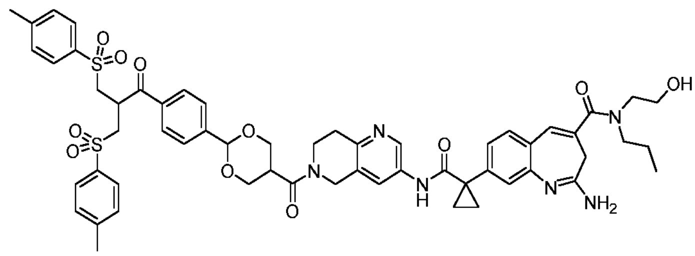

,

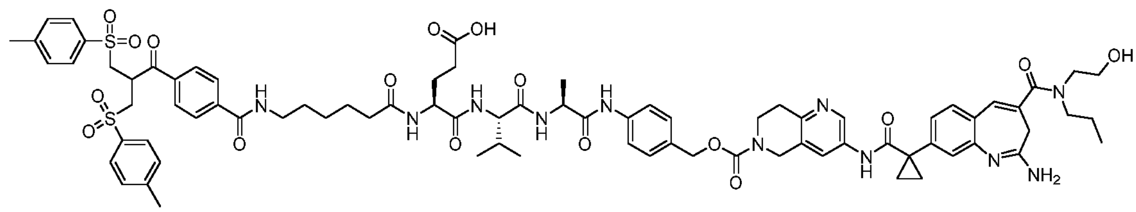

,

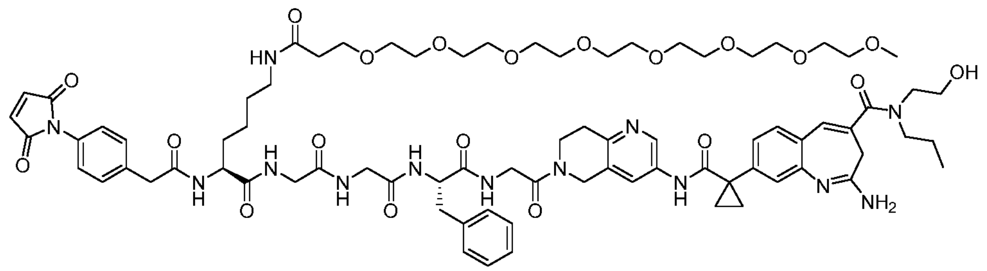

,

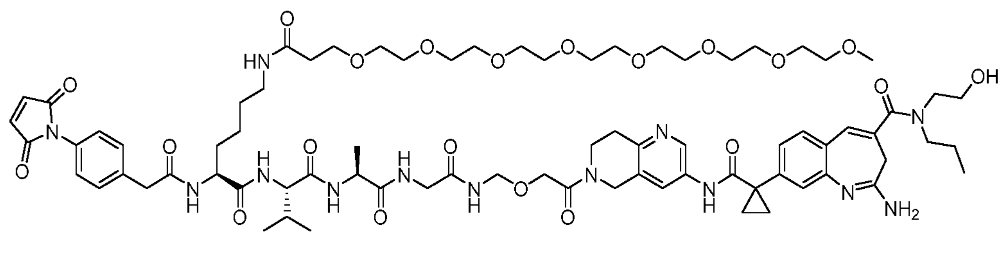

,

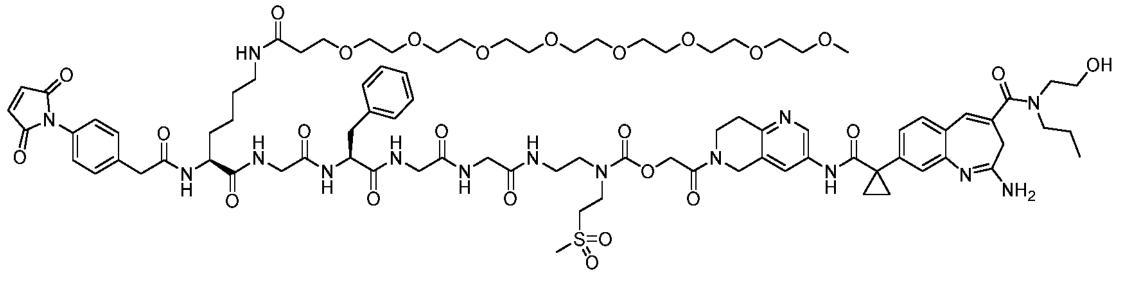

,

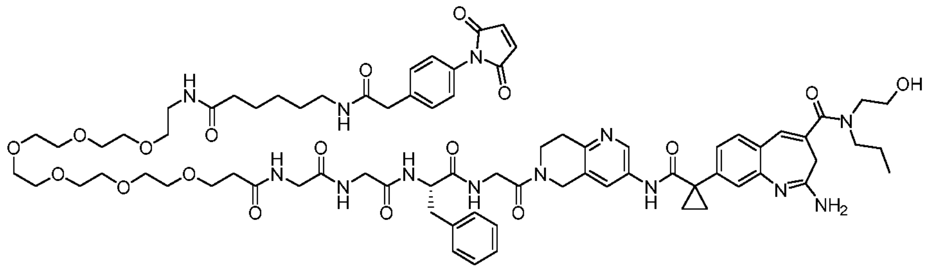

,

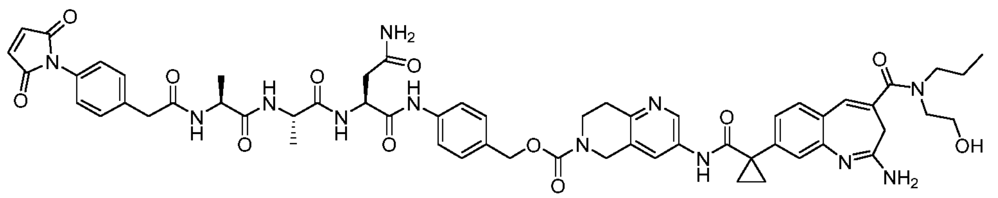

,

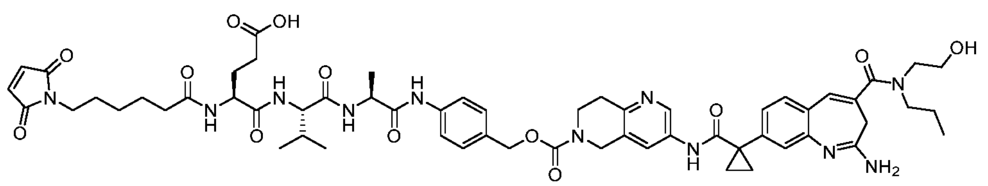

,

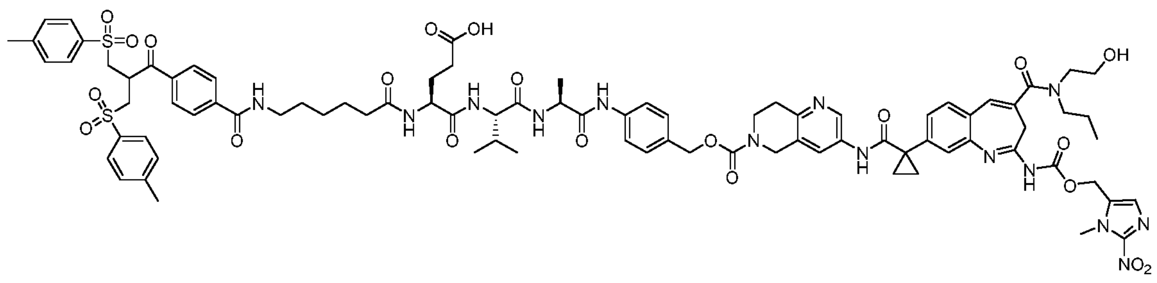

,

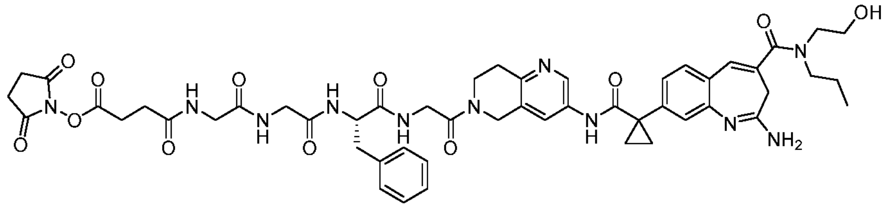

,

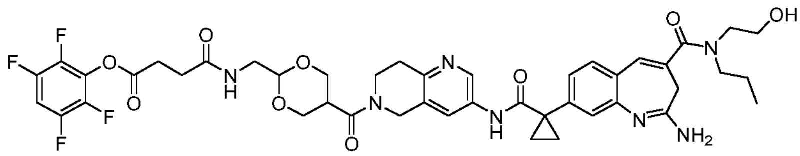

,

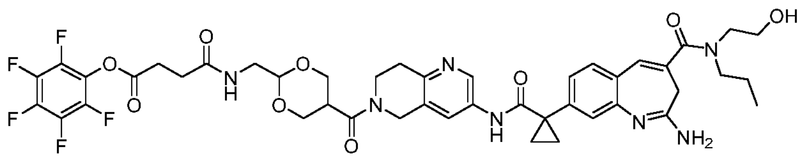

,

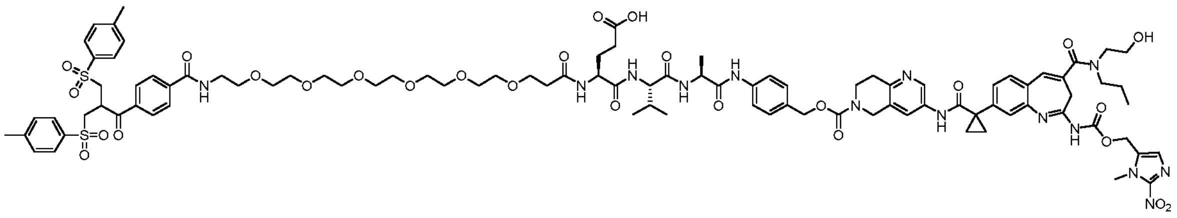

,

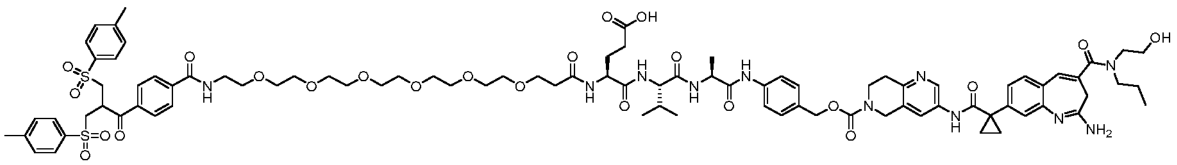

,

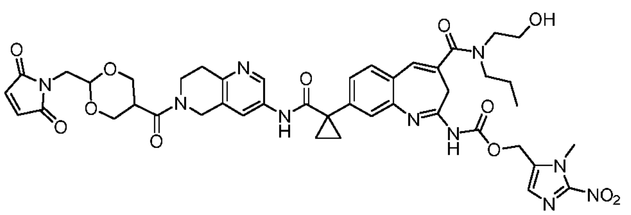

,

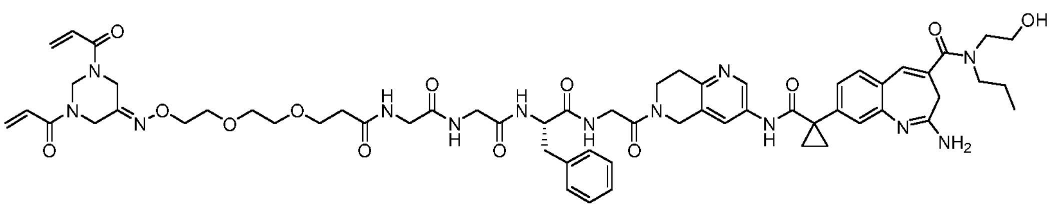

,

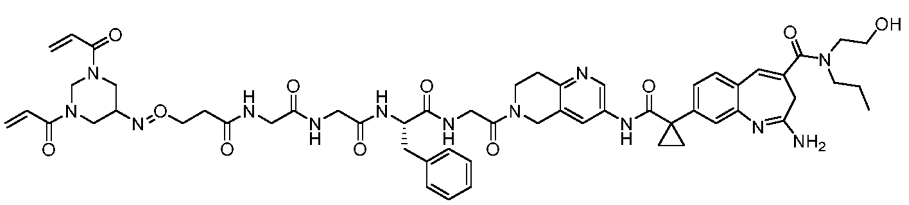

,

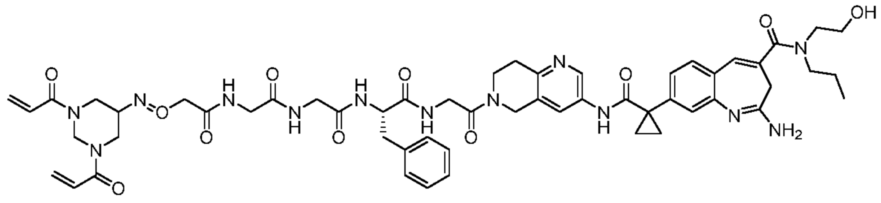

,

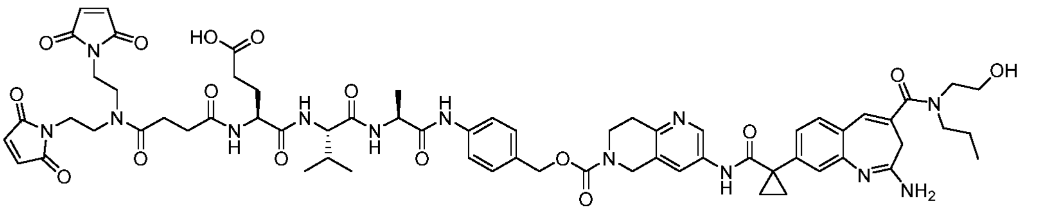

,

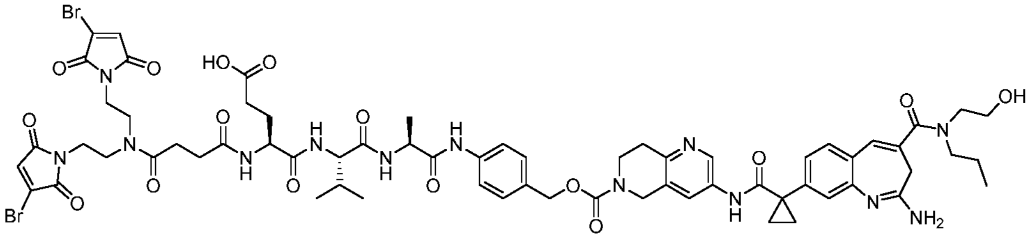

,

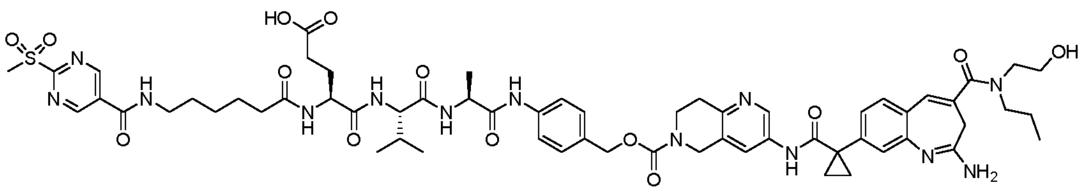

,

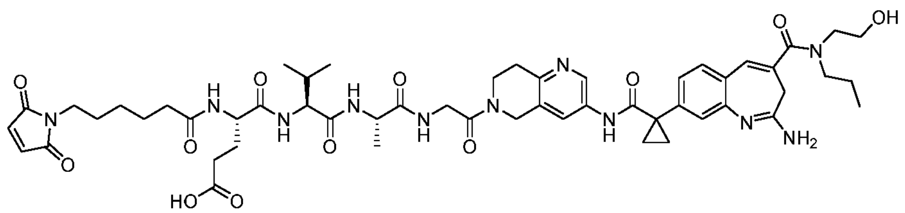

,

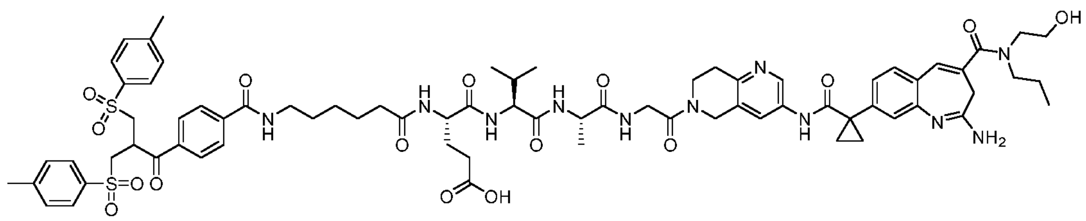

,

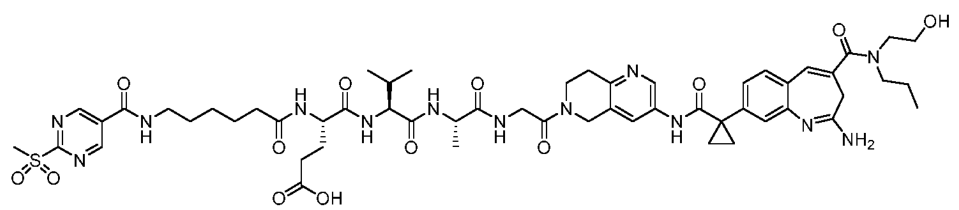

,

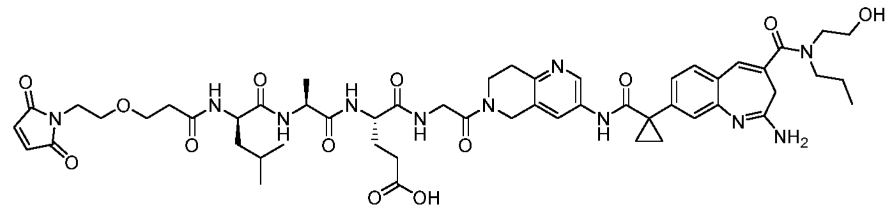

,

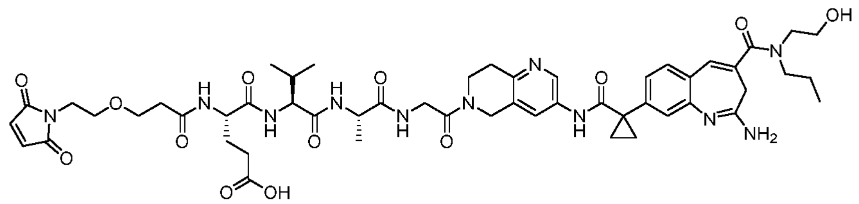

,

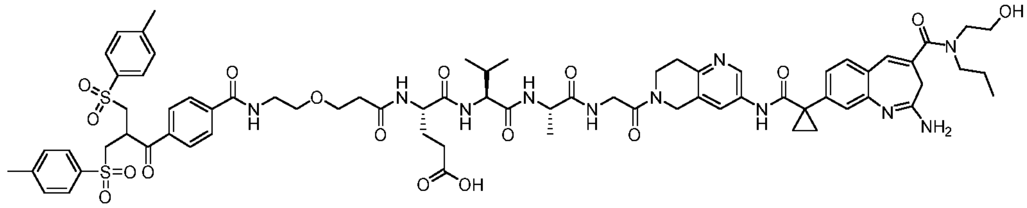

,

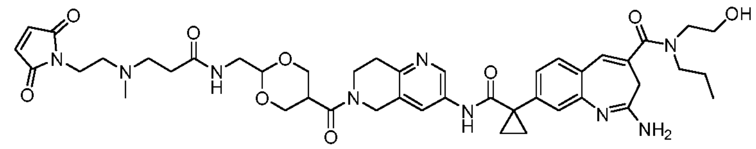

,

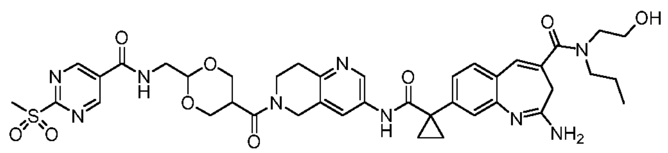

,

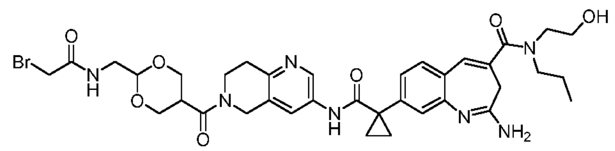

,

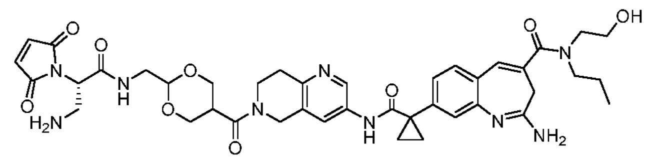

,

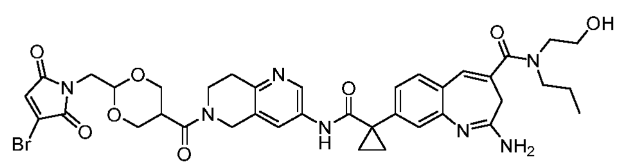

, '

or a pharmaceutically acceptable salt thereof.

In some embodiments, the compound of formula II is as follows: compound A-1, A-2, A-3, A-4, A-5, A-6, A-7, A-8,

A-9, A-10, A-11, A-12, A-13, A-14, A-15, A-16, A-17, A-18, A-19, A-20, A-21, A-22, A-23, A-24, A-25, A-26 or A-27.

In some embodiments, the compound of formula II is as follows: compound B-1, B-2, B-3, B-4, B-5, B-6, B-7, B-8, B-9, B-10 or B-11.

The present disclosure also provides a compound of formula D', a pharmaceutically acceptable salt thereof, a solvate thereof or a solvate of a pharmaceutically acceptable salt thereof;

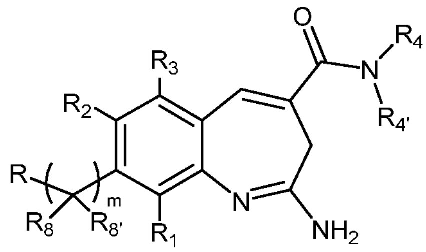

D'

where R is -L' -$L_1$-$R_7$; and L' is -C(=O)-, -C(=O)-$NR_9$- or -C(=S)-$NR_9$-;

$R_9$ is independently hydrogen, $C_{1-6}$ alkyl or halo $C_{1-6}$ alkyl;

$L_1$ is independently a linkage bond, $C_{1-6}$ alkylene or $C_{2-6}$ alkenylene;

$R_7$ is independently phenyl, 5-6 membered heteroaryl, 3-8 membered heterocycloalkyl, an 8-12 membered fused ring group, phenyl substituted with 1, 2 or 3 $R_{7-1}$, 5-6 membered heteroaryl substituted with 1, 2 or 3 $R_{7-2}$, 3-8 membered heterocycloalkyl substituted with 1, 2 or 3 $R_{7-3}$ or an 8-12 membered fused ring group substituted with 1, 2 or 3 $R_{7-4}$;

in the $R_7$, in "5-6 membered heteroaryl" in the 5-6 membered heteroaryl and the 5-6 membered heteroaryl substituted with 1, 2 or 3 $R_{7-2}$, the heteroatom is independently selected from one or more of N, O and S, and the number of the heteroatom is independently 1, 2 or 3; and in "3-8 membered heterocycloalkyl" in the 3-8 membered heterocycloalkyl and the 3-8 membered heterocycloalkyl substituted with 1, 2 or 3 $R_{7-3}$, the heteroatom is independently selected from one or more of N, O and S, and the number of the heteroatom is independently 1, 2 or 3;

in the $R_7$, an 8-12 membered fused ring group in the 8-12 membered fused ring group and the 8-12 membered fused ring group substituted with 1, 2 or 3 $R_{7-4}$ is independently a fused ring of ring A and ring B, the ring A is a 5-6 membered heteroaryl ring or a phenyl ring, and the ring B is a 5-6 membered heteroalkenyl ring; in the 5-6 membered heteroaromatic ring, the heteroatom is selected from one or more of N, O and S, and the number of the heteroatom is 1, 2 or 3; and in the 5-6 membered heteroalkene ring, the heteroatom is selected from one or more of N, O and S, and the number of the heteroatom is 1, 2 or 3;

$R_{7-1}$, $R_{7-2}$, $R_{7-3}$ and $R_{7-4}$ are independently amino, -$L_3$-$NH_2$, -$L_3$-OH, -C(=O)-$L_3$-OH or -C(=O)-$L_3$-$NH_2$, and $L_3$ is independently $C_{1-6}$ alkylene or -$(CH_2CH_2O)_{m'}$-$C_{1-6}$ alkylene-; and m' is 1, 2, 3, 4, 5, 6, 7, 8, 9 or 10;

m is 0 or 1;

$R_8$ and $R_{8'}$ are independently hydrogen or $C_{1-6}$ alkyl, or $R_8$ and $R_{8'}$ together with the carbon atom to which they are attached form carbonyl, $C_{3-10}$ cycloalkylene or 3-10 membered heterocycloalkylene; and in the 3-10 membered heterocycloalkylene, the heteroatom is selected from one or more of N, O and S, and the number of the heteroatom is 1, 2 or 3;

$R_1$, $R_2$ and $R_3$ are independently hydrogen, deuterium or halogen;

$R_4$ and $R_{4'}$ are independently $C_{1-6}$ alkyl, $C_{1-6}$ alkoxy or $C_{1-6}$ alkyl substituted with 1, 2 or 3 $R_{4-1}$; and the $R_{4-1}$ is independently hydroxyl, amino, -OC(=O)$NR_aR_b$ or

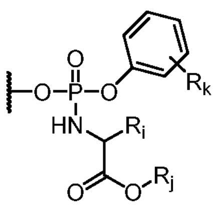

;

$R_a$ and $R_b$ are independently hydrogen, $C_{1-6}$ alkyl or $C_{3-10}$ cycloalkyl;

$R_i$ is hydrogen, $C_{1-6}$ alkyl or benzyl; $R_k$ is hydrogen, halogen or $C_{1-6}$ alkyl; and $R_j$ is $C_{1-6}$ alkyl, $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; and

in the $R_j$, in "3-10 membered heterocycloalkyl" in the 3-10 membered heterocycloalkyl and the 3-10 membered heterocycloalkyl-$C_{1-6}$ alkyl, the heteroatom is independently selected from one or more of N, O and S, and the number

of the heteroatom is independently 1, 2 or 3; and in "5-10 membered heteroaryl" in the 5-10 membered heteroaryl and the 5-10 membered heteroaryl-$C_{1-6}$ alkyl, the heteroatom is independently selected from one or more of N, O and S, and the number of the heteroatom is independently 1, 2 or 3;
and the compound of formula D' satisfies 1 or 2 of conditions of:

(1) m is 1; and
(2) at least one of $R_4$ and $R_{4'}$ is $C_{1-6}$ alkoxy or $C_{1-6}$ alkyl substituted with 1, 2 or 3 $R_{4-1}$;

and the compound of formula D' is not a compound shown in Table 3.

In some embodiments, in the compound of formula D', the pharmaceutically acceptable salt thereof, the solvate thereof or the solvate of a pharmaceutically acceptable salt thereof, some groups are defined as below, and the remaining groups are defined as any other solution (hereinafter referred to as "in some embodiments"):
the compound of formula D' satisfies 1, 2 or 3 of conditions of:

(1) $L_1$ is independently $C_{1-6}$ alkylene or $C_{2-6}$ alkenylene;
(2) $R_7$ is independently phenyl substituted with 1 amino, 5-6 membered heteroaryl substituted with one amino, 3-8 membered heterocycloalkyl substituted with one amino or an 8-12 membered fused ring group substituted with one amino; and
(3) at least one of $R_4$ and $R_{4'}$ is $C_{1-6}$ alkoxy or $C_{1-6}$ alkyl substituted with 1, 2 or 3 $R_{4-1}$; and the $R_{4-1}$ is independently -OC(=O)$NR_aR_b$ or

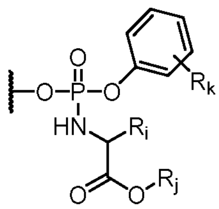
.

In the compound of D' or the pharmaceutically acceptable salt thereof, a left end or a right end of L' is attached to $L_1$.
In some embodiments, L' is -C(=O)-, -C(=O)-$NR_9$- or -C(=S)-$NR_9$-, and a right end thereof is attached to $L_1$.
In some embodiments, R is -C(O)-$NR_9$-$L_1$-$R_7$.
In some embodiments, $R_9$ is hydrogen.
In some embodiments, in the $L_1$, the $C_{1-6}$ alkylene is methylene or ethylene.
In some embodiments, $L_1$ is a linkage bond or -$CH_2$-.
In some embodiments, in the $R_7$, the 8-12 membered fused ring group may be a fused ring of ring A and ring B, the ring A is 5-6 membered heteroaryl or phenyl, and the ring B is a 5-6 membered heteroalkenyl ring; in the 5-6-membered heteroaryl, the heteroatom is selected from one or more of N, O and S, and the number of the heteroatom is 1, 2 or 3; and in the 5-6-membered heteroalkenyl ring, the heteroatom is selected from one or more of N, O and S, and the number of the heteroatom is 1, 2 or 3.
In some embodiments, in the $R_7$, the 8-12 membered fused ring group may be a fused ring of ring A and ring B, the ring A is 5-6 membered heteroaryl or phenyl, and the ring B is a 5-6 membered heteroalkenyl ring attached to the $L_1$ through the ring A; in the 5-6 membered heteroaryl, the heteroatom is N, and the number of the heteroatom is 1 or 2; and in the 5-6 membered heteroalkenyl ring, the heteroatom is N, and the number of the heteroatom is 1 or 2.
In some embodiments, in the $R_7$, in the 5-6 membered heteroaryl, the heteroatom may be selected from one or more of N, O and S, and the number of the heteroatom may be 1, 2 or 3.
In some embodiments, in the $R_7$, in the 5-6 membered heteroaryl, the heteroatom may be N, and the number of the heteroatom may be 1 or 2.
In some embodiments, in the $R_7$, the 5-6 membered heteroaryl may be pyridinyl, pyrazinyl or pyridazinyl, and may be pyridin-3-yl, pyrazin-2-yl or pyridazin-3-yl.
In some embodiments, in the $R_7$, in "5-6 membered heteroaryl" in the 5-6 membered heteroaryl and the 5-6 membered heteroaryl substituted with 1, 2 or 3 $R_{7-2}$, the heteroatom is independently N.
In some embodiments, in the $R_7$, in "5-6 membered heteroaryl" in the 5-6 membered heteroaryl and the 5-6 membered heteroaryl substituted with 1, 2 or 3 $R_{7-2}$, the number of the heteroatom is independently 1 or 2.
In some embodiments, in the $R_7$, in "5-6 membered heteroaryl" of the 5-6 membered heteroaryl and the 5-6 membered heteroaryl substituted with 1, 2 or 3 $R_{7-2}$, the 5-6 membered heteroaryl may be pyridinyl, pyrimidinyl, pyridazinyl

or pyrazinyl.

In some embodiments, in the ring A, the heteroatom in the 5-6 membered heteroaromatic ring is N.

In some embodiments, in the ring A, the number of the heteroatom in the 5-6 membered heteroaromatic ring is 1 or 2.

In some embodiments, in the ring A, the 5-6 membered heteroaromatic ring may be a pyridine ring, a pyrimidine ring, a pyridazine ring or a pyrazine ring.

In some embodiments, in the ring B, the heteroatom in the 5-6 membered heteroalkene ring is N.

In some embodiments, in the ring B, the number of the heteroatom in the 5-6 membered heteroalkene ring is 1 or 2.

In some embodiments, the $R_7$ is

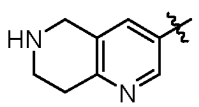,
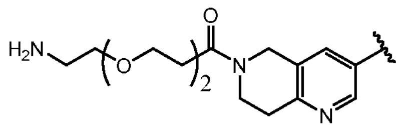,
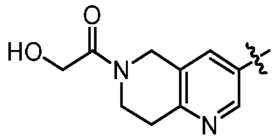,
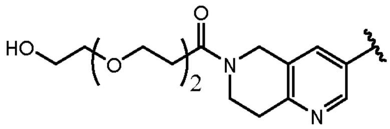,
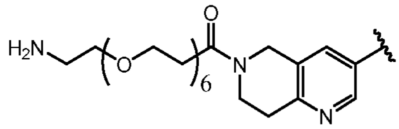,
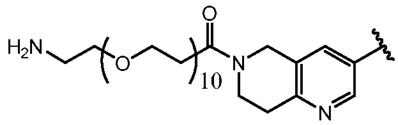,
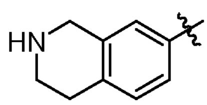,
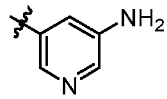,
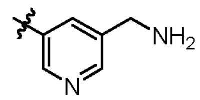,
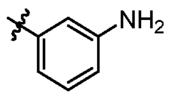,
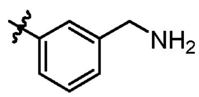
or
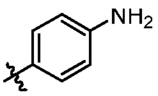.

In some embodiments, the $R_7$ is

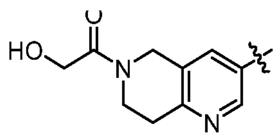
or
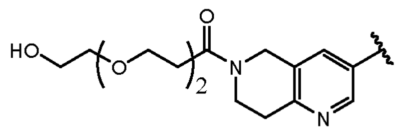.

In some embodiments, m is 1.

In some embodiments, $R_8$ and $R_{8'}$ are each independently $C_{1-6}$ alkyl, and the $C_{1-6}$ alkyl is preferably methyl.

In some embodiments, $R_8$ and $R_{8'}$ are each independently unsubstituted $C_{1-6}$ alkyl; or $R_8$ and $R_{8'}$ together with the carbon atom to which they are attached form oxo or unsubstituted $C_{3-6}$ cycloalkyl.

In some embodiments, $R_8$ and $R_{8'}$ together with the carbon atom to which they are attached form oxo, cyclopropyl or cyclobutyl.

In some embodiments, in the $R_8$ and $R_{8'}$, the $C_{1-6}$ alkyl may be methyl, ethyl, n-propyl or isopropyl.

In some embodiments, $R_8$ and $R_{8'}$ together with the carbon atom to which they are attached form $C_{3-10}$ cycloalkylene, and the $C_{3-10}$ cycloalkylene is cyclopropyl or cyclobutyl.

In some embodiments, $R_8$ and $R_{8'}$ together with the carbon atom to which they are attached form 3-10 membered heterocycloalkylene, and a heteroatom in the 3-10 membered heterocycloalkylene is N or O.

In some embodiments, $R_8$ and $R_{8'}$ together with the carbon atom to which they are attached form 3-10 membered heterocycloalkylene, and the number of the heteroatom in the 3-10 membered heterocycloalkylene is 1 or 2.

In some embodiments, $R_8$ and $R_{8'}$ together with the carbon atom to which they are attached form 3-10 membered heterocycloalkylene, and the 3-10 membered heterocycloalkylene may be oxacyclopropylene, azacyclopropylene or oxacyclobutylene or azacyclobutylene.

In some embodiments, $R_1$ is H; $R_2$ is H; and $R_3$ is H or F.

In some embodiments, in $R_1$, $R_2$ and $R_3$, the halogen is F, Cl or Br.

In some embodiments, $R_4$ and $R_{4'}$ are each independently $C_{1-6}$ alkyl; and at least one of the $R_4$ or $R_{4'}$ is substituted at any position with one substituent selected from hydroxyl and amino.

In some embodiments, $R_4$ and $R_{4'}$ are each independently $C_{1-6}$ alkyl; and at least one of the $R_4$ or $R_{4'}$ is substituted at any position with one hydroxyl.

In some embodiments, $R_4$ is $-OCH_2CH_3$, $-CH_2CH_2NH_2$, $-CH_2CH_2OH$ or $-CH_2CH_2CH_3$; and $R_{4'}$ is $-CH_2CH_2OH$ or $-CH_2CH_2CH_3$.

In some embodiments, in the $R_4$ and $R_{4'}$, the $C_{1-6}$ alkyl may be methyl, ethyl, n-propyl or isopropyl.

In some embodiments, in the $R_4$ and $R_{4'}$, the $C_{1-6}$ alkoxy may be methoxy, ethoxy, n-propoxy or isopropoxy.

In some embodiments, in the $R_4$ and $R_{4'}$, $C_{1-6}$ alkyl in the $C_{1-6}$ alkyl substituted with 1, 2 or 3 $R_{4-1}$ may be methyl, ethyl, n-propyl or isopropyl.

In some embodiments, in the $R_i$, the $C_{1-6}$ alkyl may be methyl, ethyl, n-propyl or isopropyl.

In some embodiments, in the $R_k$, the $C_{1-6}$ alkyl may be methyl, ethyl, n-propyl or isopropyl.

In some embodiments, in the $R_j$, the $C_{1-6}$ alkyl may be methyl, ethyl, n-propyl or isopropyl.

In some embodiments, in the $R_j$, in the $C_{6-10}$ aryl-$C_{1-6}$ alkyl, the $C_{6-10}$ aryl may be phenyl.

In some embodiments, in the $R_j$, in the $C_{6-10}$ aryl-$C_{1-6}$ alkyl, the $C_{1-6}$ alkyl may be methyl, ethyl, n-propyl or isopropyl.

In some embodiments, in

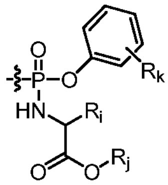

,

$R_i$ is methyl; $R_j$ is isopropyl or benzyl; and $R_k$ is hydrogen.

In some embodiments,

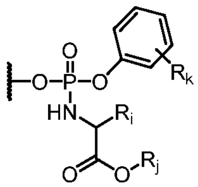

is

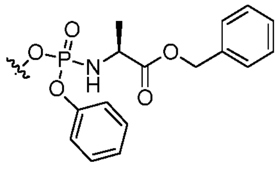

or

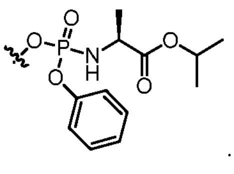

.

In some embodiments, in the $R_a$ and $R_b$, the $C_{1-6}$ alkyl may be methyl, ethyl, n-propyl or isopropyl.

In some embodiments, in the $R_a$ and $R_b$, the $C_{3-10}$ cycloalkyl may be cyclopropyl, cyclobutyl or cyclopentyl.

In some embodiments, $R_4$ and $R_{4'}$ are independently $-OCH_2CH_3$, $-CH_2CH_2NH_2$, $-CH_2CH_2OH$ or $-CH_2CH_2CH_3$.

In some embodiments, at least one of $R_4$ and $R_{4'}$ is $-OCH_2CH_3$, $-CH_2CH_2NH_2$ or $-CH_2CH_2OH$.

In some embodiments, in the $L_3$, $C_{1-6}$ alkylene may be $C_{1-3}$ alkylene.

In some embodiments, M' is 1, 2, 3, 4, 5, 6, 7, 8, 9 or 10.

In some embodiments, the compound of formula D' is any one of compounds of:

compounds 12, 13, 14, 15, 16, 17, 18, 19, 20, 21, 22, 23, 24, 25-1, 25-2, 26, 27, 28, 29, 30, 31 and 32.

The present disclosure provides a pharmaceutical composition including a substance K and a pharmaceutically acceptable excipient,

where the substance K is a substance K-1, a substance K-2 or a substance K-3;

the substance K-1 is the antibody-immunostimulatory conjugate of formula I or the pharmaceutically acceptable salt thereof described above;
the substance K-2 is the compound of formula II or the pharmaceutically acceptable salt thereof described above;
the substance K-3 is the compound of formula D', the pharmaceutically acceptable salt thereof, the solvate thereof or the solvate of a pharmaceutically acceptable salt thereof described above.

In some embodiments, the compound of formula D' is compound 12, 13, 14, 15, 16, 17, 18, 19, 20, 21, 22, 23, 24, 25-1, 25-2, 26, 27, 28, 29, 30, 31 or 32.
In some embodiments, a usage amount of the substance K may be a therapeutically effective amount.
In some embodiments, the antibody-immunostimulatory conjugate of formula I or the pharmaceutically acceptable salt thereof, the compound of formula II or the pharmaceutically acceptable salt thereof, or the compound of formula D', the pharmaceutically acceptable salt thereof, the solvate thereof or the solvate of a pharmaceutically acceptable salt thereof may be in a therapeutically effective amount.
In some embodiments, the antibody-immunostimulatory conjugate of formula I or the pharmaceutically acceptable salt thereof, the compound of formula II or the pharmaceutically acceptable salt thereof, or the compound 12, 13, 14, 15, 16, 17, 18, 19, 20, 21, 22, 23, 24, 25-1, 25-2, 26, 27, 28, 29, 30, 31 or 32 or the pharmaceutically acceptable salt thereof may be in a therapeutically effective amount.
In the pharmaceutical composition, the pharmaceutically acceptable excipient may include a pharmaceutically acceptable carrier, diluent and/or excipient.
The pharmaceutical composition may be administered by a conventional route including, but not limited to, intramuscular, intraperitoneal, intravenous, subcutaneous, intradermal, topical administration (e.g. intratumoral injection), etc.
The present disclosure further provides use of a substance K or the pharmaceutical composition described above in preparation of a medicament of a regulatory T cell and other immune cells, where the substance K is a substance K-1, a substance K-2 or a substance K-3;

the substance K-1 is the antibody-immunostimulatory conjugate of formula I or the pharmaceutically acceptable salt thereof described above;
the substance K-2 is the compound of formula II or the pharmaceutically acceptable salt thereof described above;
the substance K-3 is the compound of formula D', the pharmaceutically acceptable salt thereof, the solvate thereof or the solvate of a pharmaceutically acceptable salt thereof described above.

In some embodiments, the compound of formula D' is compound 12, 13, 14, 15, 16, 17, 18, 19, 20, 21, 22, 23, 24, 25-1, 25-2, 26, 27, 28, 29, 30, 31 or 32.
In some embodiments, a usage amount of the substance K may be a therapeutically effective amount.
The present disclosure provides use of a substance K or the pharmaceutical composition described above in preparation of a medicament for treating and/or alleviating a tumor, where the substance K is a substance K-1, a substance K-2 or a substance K-3;

the substance K-1 is the antibody-immunostimulatory conjugate of formula I or the pharmaceutically acceptable salt thereof described above;
the substance K-2 is the compound of formula II or the pharmaceutically acceptable salt thereof described above;
the substance K-3 is the compound of formula D', the pharmaceutically acceptable salt thereof, the solvate thereof or the solvate of a pharmaceutically acceptable salt thereof described above.

In some embodiments, the compound of formula D' is compound 12, 13, 14, 15, 16, 17, 18, 19, 20, 21, 22, 23, 24, 25-1, 25-2, 26, 27, 28, 29, 30, 31 or 32.
In some embodiments, a usage amount of the substance K may be a therapeutically effective amount.
The present disclosure provides use of a substance K or the pharmaceutical composition described above in preparation of a medicament for treating, alleviating and/or preventing a related disease mediated by TLR8, where the substance K is a substance K-1, a substance K-2 or a substance K-3;

the substance K-1 is the antibody-immunostimulatory conjugate of formula I or the pharmaceutically acceptable salt thereof described above;
the substance K-2 is the compound of formula II or the pharmaceutically acceptable salt thereof described above;
the substance K-3 is the compound of formula D', the pharmaceutically acceptable salt thereof, the solvate thereof or the solvate of a pharmaceutically acceptable salt thereof described above.

In some embodiments, the compound of formula D' is compound 12, 13, 14, 15, 16, 17, 18, 19, 20, 21, 22, 23, 24, 25-1, 25-2, 26, 27, 28, 29, 30, 31 or 32.

In some embodiments, the related disease mediated by TLR8 refers to a tumor or a viral infectious disease.

In some embodiments, a usage amount of the substance K may be a therapeutically effective amount.

The tumor may be a malignant tumor, includes metastatic and non-metastatic cancer, also includes hereditary and sporadic cancer, and may also include a solid tumor and a non-solid tumor.

The present disclosure further provides a therapeutic method for treating or preventing a disease, including administering the substance K-1 described above, the substance K-2 described above or the substance K-3 described above to a subject;

The disease is a disease related to the regulatory T cell and other immune cells described above, the tumor described above, or the related disease mediated by TLR8 described above.

In the present disclosure, the "tumor" and "cancer" have the same meaning, including, but not limited to, one or more of eye cancer, bone cancer, lung cancer, gastric cancer, pancreatic cancer, breast cancer, prostate cancer, brain cancer (including malignant glioma, medulloblastoma), ovarian cancer, bladder cancer, cervical cancer, testicular cancer, kidney cancer (including adenocarcinoma and Wilm's tumor), oral cancer (including squamous cell carcinoma), tongue cancer, laryngeal cancer, nasopharyngeal cancer, head and neck cancer, colon cancer, small bowel cancer, rectal cancer, parathyroid cancer, thyroid cancer, esophageal cancer, gallbladder cancer, cholangiocarcinoma, cervical cancer, liver cancer, sarcoma, skin cancer, lymphocytic leukemia (including acute lymphoblastic leukemia, lymphoma, myeloma, chronic lymphoblastic leukemia, Hodgkin's lymphoma, non-Hodgkin's lymphoma, T-cell chronic lymphocytic leukemia, B-cell chronic lymphocytic leukemia), myeloid-related leukemia (including acute myeloid leukemia, chronic myeloid leukemia) and AID-related leukemia.

In the present disclosure, the tumor is preferably a tumor having HER2 expression (which may be a tumor having high HER2 expression or a tumor having low HER2 expression). In the present disclosure, the tumor is more preferably a tumor having high HER2 expression.

In the present disclosure, unless otherwise specified, the term "optionally substituted at any position with one or more groups" means that any one or more hydrogen atoms of the designated atom or atoms on a group are substituted with the designated group, provided that the normal valence of the designated atom is not exceeded, and the substitution at any position is a reasonable substitution common in the art.

In the present disclosure, when a bond to a substituent shows an intersection with a bond connecting two atoms in the ring, then such a substituent may be bonded to any bondable ring atom on the ring.

In the present disclosure, any combination of variables is only allowed if such combination results in a stable compound.

In the present disclosure, any variable that occurs more than once in the composition or structure of a compound is defined independently in each case. For example, when R is substituted with one or more groups, each substituent is an independent substituent and may be the same or different.

Unless otherwise indicated, the following terms appearing in the specification and claims of the present disclosure have the following meanings:

The term "antibody" refers to any form of an antibody that exhibits a desired biological activity, such as inhibition of binding of a ligand to its receptor or by inhibition of ligand-induced receptor signaling. Accordingly, an "antibody" is administered in its broadest sense and explicitly includes, but is not limited to, a monoclonal antibody (including a full-length monoclonal antibody), a polyclonal antibody and a multispecific antibody (including a bispecific antibody). Naturally occurring "antibodies" are glycoproteins including at least two heavy (H) chains and two light (L) chains interconnected by disulfide bonds. Each heavy chain consists of a heavy chain variable region and a heavy chain constant region. The heavy chain constant region consists of three domains (CH1, CH2 and CH3). Each light chain consists of a light chain variable region and a light chain constant region. The light chain constant region includes a CL domain. The variable regions of the heavy chain and the light chain contain a binding domain (antigen-binding domain) that interacts with an antigen. The antigen-binding domain may be provided by one or more variable regions on an antibody, and specifically, the antigen-binding domain includes a light chain variable domain (VL) of the antibody and a heavy chain variable domain (VH) of the antibody. The antibody may be a monoclonal antibody, a human antibody, a humanized antibody or a chimeric antibody. These antibodies can be of any class (IgG, IgE, IgM, IgD, IgA and IgY) or subtype (IgG1, IgG2, IgG3, IgG4, IgA1 and IgA2). The present disclosure includes not only intact antibodies, but also fragments of antibodies with immunological activity (including Fab, F(ab')2, scFv or Fv fragments) or fusion proteins formed by antibodies and other sequences. Accordingly, the "antibody" of the present disclosure also includes fragments, derivatives and analogs of the antibody. In some specific embodiments, the antibody binds to an optional antigen or antigen combination including, but not limited to: HER2, 5T4 (TPBG), Trop-2, FZD10, GCC, PTK7, Globo H, LIVI, HER3, CD142 (Tissue Factor), ROR1, ROR2, P-cadherin, IGF-1R, AXL, NaPi2b, EpCAM, Nectin-4 (191P4D12), PSMA, FR$\alpha$ (FolR$\alpha$), MSLN (mesothelin), c-Met, B7-H3, B7-H4, CEACAM5, EGFR, CD70, CD71, CD73, CD74, FGFR2, FGFR4, BCMA, CD25, EphA2, EphB3, c-Kit, CDH6, IL-13R$\alpha$2, CD47, SIRP$\alpha$, CD48, CD40, CD105, CD228, NCAM-1, SEZ6, DPEP3, RNF43, CS1, DLL3, DLL4, EMR2, TNFRSF21, TNFSF9,

MMP16, LRRC15, UPK1B, Claudin6, Claudin9, Claudin18.2, MFI2, PRLR, BMPR1B, VEGF, PDGF, PDGFR-α, PAI-1, LING01, KaAG1, Steap1, ASCT2, DLK1, HLL2, MUC1, CA6, ADAM9, PMEL17, DC-SiGN, CCR2, CCR7, BDNF, Notch, EGFRvIII, CD3, IGF1R, EFNA4, EBD, CDCP1, GD3, 24P4C12, STn, lewisY, NTB-A, 158P1D7, TIGIT, C4.4a, MMP-9, MG7Ag, 4-1BB (CD137), CanAg, kremen2, CD19, CD22, CD33, CD37, CD79b, CD30, CD20, CD123, PD-1 and PD-L1.

In the present disclosure, the "antibody" may further include an engineered antibody. The engineered antibody may 1) include one or more non-naturally encoded amino acids incorporated into the heavy chain, the light chain, or both the heavy chain and the light chain, including but not limited to one or more of the following non-natural amino acids: p-acetylphenylalanine, o-acylphenylalanine, m-acylphenylalanine, p-acetyl-L-phenylalanine, p-acyl-L-phenylalanine, p-propargyloxy-L-phenylalanine, 4-azido-L-phenylalanine, p-azidoethoxyphenylalanine, p-azidomethyl-phenylalanine, and the like; 2) cysteine residues are inserted into different positions of the heavy chain or the light chain of the antibody, or specific amino acid residues of the heavy chain or the light chain of the antibody are replaced with cysteine residues, thereby forming unpaired cysteine residues for conjugation.

The "antibody" or antigen-binding fragment thereof in the present disclosure may include an Fc region, and the Fc region can be further modified. In some cases, one or more mutations in the Fc region result in improvements in a drug including the Fc region with such modifications in aspects such as reduction of effector functions, alteration in the regulation of the drug's metabolic half-life, and alteration in drug stability. In some cases, the modified Fc region may include one or more mutations, and these mutations will reduce or eliminate the interaction between the antibody and the immune system. Key interactions may include the interaction between the Fc of the antibody and the Fcy receptor, as well as the interaction between the Fc of the antibody and C1q of the complement system. When IgG1 is used as the isotype of the antibody of the present disclosure, the effector functions can be adjusted by substituting a part of the amino acid residues in the constant region. Variants of IgG1 with reduced or attenuated effector functions include, but are not limited to: IgG1 LALA (IgG1-L234A, L235A), IgG1 LAGA (IgG1-L235A, G237A), IgG1 AAG (IgG1- L234A, L235A, P329G), etc. The L234A and L235A described above indicate that leucine at positions 234 and 235 determined by the EU index (Proc. Natl. Acad. Sci. U.S.A.,Vol.63, No.1 (May 15, 1969), p78-85) is substituted with alanine, G237A indicates that glycine at position 237 determined by the EU index is substituted with alanine, and P329G indicates that proline at position 329 determined by the EU index is substituted with alanine. In some cases, pharmacodynamic and pharmacokinetic properties of drugs can be altered by glycosylation modifications at the Fc-region (Journal of Pharmaceutical Sciences. 2015, 104(6), 1866-1884). For example, native antibodies produced by mammalian cells typically contain branched, biantennary-type oligosaccharides that are typically attached to Asn297 of the CH2 domain at the Fc-region via N-linking bonds. The oligosaccharides may include various carbohydrates, such as mannose, N-acetylglucosamine (GlcNAc), galactose and sialic acid, as well as fucose attached to GlcNAc in the stem of a biantennary-type oligosaccharide structure. Modification of oligosaccharides in antibodies can produce antibody variants with some improved properties. For example, afucylation can enhance antibody-dependent cellular cytotoxicity (ADCC) and antibody-dependent cellular phagocytosis (ADCP); reducing terminal sialylation or increasing terminal acetylglycosamination can enhance ADCC; and treating of CHO cells with mannosidase inhibitors can increase mannosylation and afucylation, thereby increasing the ADCC effect and slightly reducing the CDC effect. In addition, glycosylation modifications can also be made by mutations, such as the N297Q mutation, where asparagine at position 297 is mutated into glutamine.

The term "mAb", also referred to as "monoclonal antibody", refers to a polypeptide having substantially the same amino acid sequence or derived from the same genetic origin. Monoclonal antibodies are highly specific and can be directed against a single antigenic site. In addition, in contrast to conventional (polyclonal) antibody preparations, which typically include multiple different antibodies directed against multiple different determinants (epitopes), each monoclonal antibody is directed against only a single determinant on the antigen. Table 7 lists some monoclonal antibodies, corresponding antigens and indications (the indications include, but are not limited to, those shown in Table 7 below):

Table 7

| Antibody name | Antigen | Indication |
|---|---|---|
| trastuzumab, pertuzumab, margetuximab, HT-19 | Her2 | Breast cancer (with high/low expression of Her2), HR-positive breast cancer, non-small cell lung cancer, colorectal cancer, gastric cancer, biliary tract cancer, esophageal adenocarcinoma, gastroesophageal junction cancer, triple-negative breast cancer, salivary gland cancer, cervical cancer, bladder cancer, ovarian cancer, prostate cancer, glioblastoma, endometrial carcinoma, urothelial carcinoma, pancreatic carcinoma, gallbladder carcinoma, skin cancer, lung adenocarcinoma, B-cell acute lymphoblastic leukemia, brain cancer, castration-resistant prostate cancer, uterine serous carcinoma, melanoma, head and neck cancer, liver cancer, renal cell carcinoma, oral cancer, peritoneal cancer, brain metastasis of cancer |

(continued)

| Antibody name | Antigen | Indication |
|---|---|---|
| cetuximab, necitumumab, nimotuzumab, panitumumab, depatuxizumab, matuzumab, zalutumumab, imgatuzumab, pimurutamab, tomuzotuximab | EGFR | Esophageal cancer, gastric cancer, head and neck cancer, skin cancer, non-small cell lung cancer, small cell lung cancer, kidney cancer, colorectal cancer, glioma, cervical cancer, hypopharyngeal cancer, pancreatic cancer, glioblastoma, esophageal cancer, gastroesophageal junction cancer, biliary tract cancer, triple-negative breast cancer, bladder cancer, urothelial cancer, ovarian cancer, castration-resistant prostate cancer, nasopharyngeal cancer, hepatocellular carcinoma, small bowel cancer, peritoneal cancer, liver metastasis of cancer |
| sacituzumab , datopotamab | Trop-2 | Triple-negative breast cancer, HR-positive breast cancer, head and neck squamous cell carcinoma, glioblastoma, non-small cell lung cancer, small cell lung cancer, bladder cancer, pancreatic cancer, ovarian cancer, gastric cancer, endometrial cancer, colorectal cancer, liver cancer, esophageal cancer, cholangiocarcinoma, head and neck cancer, prostate cancer, kidney cancer, urethral cancer, cervical cancer, salivary gland cancer, castration-resistant prostate cancer, brain metastases of cancer |
| enfortumab | Nectin-4 | Urothelial cancer, bladder cancer, castration-resistant prostate cancer, HR-positive breast cancer, triple-negative breast cancer, non-small cell lung cancer, head and neck cancer, gastric cancer, esophageal cancer, gastroesophageal junction cancer, bladder cancer, pancreatic cancer, ovarian cancer |
| edrecolomab , adecatumumab, AM-928 | EpCAM | Colorectal cancer, breast cancer, bladder cancer, glioblastoma |
| Tisotumab, MORAb-066 | Tissue Factor | Cervical cancer, ovarian cancer, esophageal cancer, endometrial cancer, bladder cancer, prostate cancer, non-small cell lung cancer, head and neck squamous cell carcinoma, pancreatic cancer, colorectal cancer |
| amatuximab, anetumab | MSLN | Mesothelioma, ovarian cancer, fallopian tube cancer, peritoneal cancer, pancreatic cancer, lung adenocarcinoma |
| rosopatamab | PSMA | Prostate cancer |
| labetuzumab, SAR408377, RG6123 | CEACAM5 | Non-small cell lung cancer, breast cancer, pancreatic cancer, gastric cancer, gastroesophageal junction cancer, colorectal cancer, head and neck cancer, cervical cancer, endometrial cancer |
| zolbetuximab , osemitamab, ASKB589, LM-102, MIL93, NBL-015 | [0577] Claudin 18.2 | Gastric cancer, esophageal cancer, pancreatic cancer, gastroesophageal junction cancer, cholangiocarcinoma, non-small cell lung cancer, ovarian cancer |
| mirvetuximab , farletuzumab, luveltamab | FRα | Peritoneal cancer, fallopian tube cancer, ovarian cancer, endometrial cancer, non-small cell lung cancer, triple-negative breast cancer, acute myeloid leukemia, mesothelioma, HR-positive breast cancer, pancreatic cancer |
| durvalumab, sugemalimab, adebrelimab, atezolizumab, avelumab, cosibelimab | PD-L1 | Bladder cancer, mesothelioma, colorectal cancer, germinoma, triple-negative breast cancer, gastric cancer, ovarian cancer, thyroid cancer, kidney cancer, cervical cancer, endometrial cancer, gastroesophageal junction cancer, fallopian tube cancer, peritoneal cancer, sarcoma, castration-resistant prostate cancer, esophageal cancer, HR-positive breast cancer, Her2-positive breast cancer, gallbladder cancer, cholangiocarcinoma, glioma, melanoma, urothelial cancer, non-small cell lung cancer, gastric cancer, squamous cell cancer, pancreatic cancer, liver cancer, small cell lung cancer, diffuse large B-cell lymphoma, esophageal squamous cell carcinoma, biliary tract carcinoma, multiple myeloma, lymphoma, malignant rhabdoid tumor, osteosarcoma |

(continued)

| Antibody name | Antigen | Indication |
|---|---|---|
| naptumomab, huA1 | 5T4 | Renal cell carcinoma, non-small cell lung cancer, small cell lung cancer, triple-negative breast cancer, ovarian cancer, cervical cancer, pancreatic cancer, endometrial cancer, urothelial cancer, head and neck cancer, mesothelioma, melanoma, hepatocellular carcinoma, prostate cancer, colorectal cancer, gastric cancer, esophageal cancer |
| cusatuzumab | CD70 | Renal cell carcinoma, non-Hodgkin's lymphoma, nasopharyngeal carcinoma, cutaneous T-cell lymphoma, acute myeloid leukemia, myelodysplastic syndrome, chronic myelomonocytic leukemia |
| mupadolimab, oleclumab, BMS-986179 | CD73 | Renal cell carcinoma, pancreatic cancer, ovarian cancer, triple-negative breast cancer, castration-resistant prostate cancer, cervical cancer, endometrial cancer, sarcoma, head and neck squamous cell carcinoma, bladder cancer, colorectal cancer, non-small cell lung cancer, non-Hodgkin's lymphoma, peritoneal cancer, esophageal cancer, multiple myeloma |
| Onartuzumab , telisotuzumab , emibetuzumab | c-Met | Non-small cell lung cancer, gastric cancer, hepatocellular carcinoma, renal cell carcinoma, gastroesophageal junction carcinoma, glioblastoma, colorectal cancer, head and neck squamous cell carcinoma |

The terms "fragment", "derivative" and "analog" refer to a polypeptide that substantially maintains the same biological function or activity as an antibody of the present disclosure. The polypeptide fragment, derivative or analog of the present disclosure may be a) a polypeptide in which one or more conservative or non-conservative amino acid residues (preferably conservative amino acid residues) are substituted, and such substituted amino acid residues may or may not be encoded by the genetic code, or b) a polypeptide having a substituent group in one or more amino acid residues, or c) a polypeptide formed by fusion of a mature polypeptide to another compound (such as a compound that prolongs the half-life of the polypeptide, for example, polyethylene glycol), or d) a polypeptide formed by fusion of an additional amino acid sequence to this polypeptide sequence (such as a leader sequence or a secretory sequence or a sequence used for purifying this polypeptide or a proprotein sequence, or a fusion protein formed with a 6His tag).

In the present disclosure, the biosimilar of the monoclonal antibody means that the biosimilar of the monoclonal antibody is highly similar to the monoclonal antibody, and there is no clinically significant difference in safety and/or efficacy among others, although there is a slight difference in the inactive ingredients thereof clinically.

The term "HER2" (also known as ERBB2, NEU, NGL, TKR1, CD340, p185, MLN19, HER-2/neu) refers to a transmembrane tyrosine kinase receptor of the epidermal growth factor (EGF) receptor family. HER2 includes an extracellular binding domain, a transmembrane domain, and an intracellular tyrosine kinase domain. HER2 does not have its own ligand-binding domain and therefore cannot bind growth factors. However, HER2 binds tightly to other ligand-binding EGF receptor family members (e.g. HER1 or HER3) to form heterodimers that stabilize ligand-binding and enhance kinase-mediated activation of downstream signaling pathways. In humans, there are species HER2 isoforms: A, B, C, D and E. The "HER2" in the present disclosure includes all HER2 isoforms.

The term "anti-HER2 antibody" refers to an antibody that targets the HER2 protein, and the anti-HER2 antibody can be derived from any species, such as humans, rats, mice and rabbits. The anti-HER2 antibody is preferably a monoclonal anti-HER2 antibody, and the anti-HER2 antibody is more preferably a humanized anti-HER2 antibody. The anti-HER2 antibody includes, but is not limited to: Pertuzumab, Trastuzumab, Trastuzumab biosimilar (e.g., Inetetamab, Coprelotamab, SIBP-01, HL02, TX05, ALT02, EG12014), Pertuzumab biosimilar (e.g., SYSA1901, TQB2440, HLX11, HS627, KM118), Margetuximab, HT-19, Anbenitamab, Zanidatamab, etc.,

where Trastuzumab (also known as Herceptin or Herclon) is a humanized monoclonal antibody that can bind to the juxtamembrane region of the extracellular structure of the HER2 receptor (Hudis CA, N Engl J Med. 2007; 357(1):39-51). The amino acid sequences of the heavy chain and light chain variable regions of Trastuzumab are disclosed in U.S. Patent No. 5,821,337. Trastuzumab interacts with the tricycle region formed by human HER2 residues 557-561, 570-573 and 593-603 (Cho et al., Nature 421: 756-760, 2003). Trastuzumab can interfere with HER2 signaling by preventing HER2 receptor dimerization, promoting HER2 receptor endocytosis, and inhibiting extracellular domain shedding. In addition, another important mechanism of action of anti-HER2 antibodies is to mediate antibody-dependent cellular cytotoxicity (ADCC). In ADCC, anti-HER2 antibodies bind to tumor cells and then recruit immune cells, such as macrophages, through Fcγ receptor (FcγR) interactions. Trastuzumab has a conserved human IgG Fc region and is capable of recruiting immune effector cells responsible for antibody-dependent cellular cytotoxicity (Hudis CA, N Engl J Med. 2007; 357(1):39-51). Trastuzumab was approved by the U.S. FDA in September 1998 for the treatment of patients whose tumors overexpress

HER2 and receive one or more chemotherapy regimens for metastatic breast cancer;

Pertuzumab (also known as Perjeta, Omnitarg) is a humanized monoclonal antibody that binds to the extracellular domain of the HER2 receptor and inhibits dimerization of HER2 and the HER receptor together. The amino acid sequences of the heavy chain and light chain variable regions of Pertuzumab are disclosed in U.S. Patent No. 7,560,111. Pertuzumab primarily interacts with residues in the 245-333 region of human HER2, particularly residues His245, Val286, Ser288, Leu295, His296 or Lys311 (Franklin et al., Cancer Cell 5: 317-328, 2004). Studies have shown that Pertuzumab is more effective than Trastuzumab in disrupting the formation of HER1-HER2 and HER3-HER2 complexes in breast and prostate cancer cell lines (Agus et al., J Clin Oncol. 2005; 23(11):2534-43. Epub Feb 7, 2005). Pertuzumab was approved by the U.S. FDA in June 2012 for use in combination with Trastuzumab and Docetaxel to treat patients with HER2-positive metastatic breast cancer who have not received anti-HER2 therapy or chemotherapy;

Margetuximab (also known as MGAH22, Margenza) is an Fc-engineered monoclonal antibody that targets the HER2 protein and binds to the extracellular domain of HER2. Margetuximab only differs from Trastuzumab in the variable region sequence by a few amino acids, and the Fc is mutated at five sites of F243L/R292P/Y300L/L235V/P396L, which improves the affinity for CD16A and enhances ADCC activity. The modified Fc region of Margetuximab increases the binding to the activating Fc receptor FCGR3A (CD16A) and decreases the binding to the inhibitory Fc receptor FCGR2B (CD32B), thereby resulting in the stronger ADCC effect and NK cell activation (Nordstrom J. et al., Breast Cancer Research, 2011; 13: R123). Margetuximab was approved by the U.S. FDA in December 2020 for the treatment of adult patients with metastatic HER2-positive breast cancer (MBC) who have received 2 or more anti-HER2 targeted therapies, of which at least one anti-HER2 targeted therapy is for the treatment of metastatic breast cancer;

HT-19 is another anti-HER2 monoclonal antibody that binds to an epitope of human HER2 that is different from that for Trastuzumab or Pertuzumab and has been shown to be comparable to Trastuzumab in its ability to inhibit HER2 signaling, and it promotes HER2 degradation in combination with Trastuzumab and Pertuzumab (Bergstrom D. A. et al., Cancer Res. 2015; 75:LB-231).

Anbenitamab and Zanidatamab are bispecific antibodies against two different epitopes of Her2 (ECDII and ECDIV).

In the present disclosure, the anti-HER2 antibody is not limited to the antibodies described above as long as it specifically binds to HER2 (for example, an anti-HER2 antibody having internalization activity in HER2-expressing cells by binding to HER2).

The isotype of the "anti-HER2 antibody" in the present disclosure includes IgG1, IgG2, IgG3, IgG4, etc., preferably IgG1, IgG2 or IgG4.

The term "low HER2 expression" generally refers to an HER2 expression level being IHC 1 +, or IHC 2 +/FISH negative (i.e., IHC 2 + and simultaneously negative in FISH testing) in clinical testing. The terms "HER2 high expression" and "HER2 positive" are used interchangeably and generally refer to HER2 expression levels that are IHC 2 +/FISH positive (i.e., IHC 2 + and simultaneously positive in FISH testing), or IHC 3 + in clinical testing. When IHC staining intensity is reported as a range, the term "low HER2 expression" herein includes the ranges from IHC 0 to 1 + and IHC 1 + to 2 + in addition to IHC 1 +, or IHC 2 +/FISH negative. The terms "high HER2 expression" and "HER2 positive" each include the range from IHC 2 + to 3 + in addition to IHC 2 +/FISH positive or IHC 3 +. In the present disclosure, FISH negative means that the result of FISH testing shows no amplification of the HER2 gene, and FISH positive means that the result of FISH testing shows amplification of the HER2 gene.

The term "5T4", also referred to as 5T4 carcinoembryonic antigen or trophoblast cell glycoprotein TPBG, is a 72 kDa glycoprotein defined by a monoclonal antibody produced against a glycoprotein isolated from wheat germ agglutinin from the microvillous membrane of the human placental syncytiotrophoblast. It has limited expression in normal tissues, but it is overexpressed by many types of cancer cells. Anti-5T4 monoclonal antibodies include, but are not limited to, H8 and humanized monoclonal antibodies thereof disclosed in WO2006/031653A1, A1, A2, A3 and humanized monoclonal antibodies thereof (e.g. huA1) disclosed in US8044178B2, engineered A1 monoclonal antibodies disclosed in WO2013068874, naptumomab, etc.

The term "Claudin18.2", also known as CLDN18.2, is a highly selective cell lineage marker whose expression in normal tissues is strictly limited to epithelial cells differentiated from the gastric mucosa, but not in the gastric stem cell region. It is expressed in a considerable part of primary gastric cancer, and its expression level is retained in gastric metastatic cancer tissues. Besides gastric cancer, Claudin18.2 expression is also found in pancreatic cancer and lung cancer. Anti-Claudin18.2 monoclonal antibodies include, but are not limited to: Zolbetuximab, Osemitamab, ASKB589, LM-102, MIL93, NBL-015, etc.

The term "CEACAM5", carcinoembryonic antigen, abbreviated as CEAand also known as CD66e, is an acidic glycoprotein that belongs to a member of the immunoglobulin superfamily and contains 7 domains linked to the cell membrane via glycosylphosphatidylinositol anchors. It mainly exists in adult cancer tissues and fetal gastrointestinal tissues, participates in the intercellular adhesion of cancer cell invasion and metastasis, and is a relatively broad-spectrum tumor marker. Anti-CEA monoclonal antibodies include, but are not limited to: Labetuzumab, SAR408377 (the antibody portion of Tusamitamab Ravtansine), RG6123, etc.

The term "Trop-2", human trophoblast cell surface antigen 2, is a cell surface glycoprotein encoded by the TACSTD2 gene. Trop-2 is rarely or not expressed in adult normal tissues, and is only expressed at a very low level in cells in the epithelial region. However, it is overexpressed in various epithelial-derived tumors, such as gastric cancer, lung cancer, intestinal cancer, ovarian cancer, breast cancer, prostate cancer, pancreatic cancer and liver cancer. Anti-Trop-2 monoclonal antibodies include, but are not limited to, Sacituzumab (hRS7, the antibody portion of Sacituzumab Govitecan), Datopotamab (the antibody portion of Dato-Dxd), etc.

The term "nectin-4", also known as the 191P4D12 protein, is a cell adhesion molecule, a surface molecule belonging to the nectin protein family, which plays a key role in various biological processes of epithelial cells, endothelial cells, immune cells and nerve cells during development and adulthood. Nectin-4 is a tumor-associated antigen on predominantly poor-prognosis tumors. Anti-Nectin-4 monoclonal antibodies include, but are not limited to, Enfortumab (Ha22-2), etc.

The term "linker" refers to a degradable or non-degradable linking fragment used to link a small molecule drug D and an antibody. An antibody molecule may be linked to a plurality of linkers bearing a small molecule drug D. Generally, each linker may be linked to one or more small molecule drugs. In the present disclosure, preferably, each linker is linked to one small molecule drug D. Generally, each antibody may be linked to a plurality of linkers. In the present disclosure, preferably, each antibody is linked to 1 to 10 linkers; and more preferably, each antibody is linked to 1 to 8 linkers.

In the present disclosure, the non-degradable linker means that the linker has enzymatic stability and/or chemical stability in vivo and in vitro, and the release of the small molecule drug D may not depend on the differentiated properties of the levels of enzymes in plasma, tumor tissues and cells. The release of the small molecule drug D can degrade the antibody to the amino acid level after endocytosis of the antibody-immunostimulatory conjugate through antigen-mediated phagocytosis, thereby releasing a derivative of the small molecule drug D consisting of the small molecule drug D, a linker and an amino acid residue or a residue to which the small molecule drug D and the linker are covalently linked. Antibody-immunostimulatory conjugates constructed with such non-degradable linkers have better stability. Non-degradable linkers include alkylene chains and polymers thereof (e.g., alkylene amide polymers, alkylene glycol polymers or combinations including alkylene glycol, alkylene amide polymer fragments), ethylene glycol fragments and polyethylene glycol fragments, aromatic rings or non-aromatic rings, and combinations thereof.

In the present disclosure, the degradable linker can be degraded in vivo or in vitro, including a linker that can be degraded by a specific enzyme in vivo or in vitro, or including a linker that is chemically unstable by itself. The degradable linker can be degraded with the cell to release the small molecule drug D, for example, can be reduced in the cytoplasm, degraded under lysosomal acidic conditions, or degraded by specific proteases or other enzymes within the cell. The degradable linker includes one or more enzymatically degradable linkers, chemically unstable linkers or other degradable linkers, and the other parts may be linkers that are not degraded by enzymes or are chemically stable. The chemically unstable linker includes oxime, hydrazone and/or disulfide groups (e.g.,

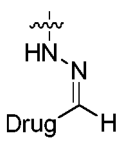, 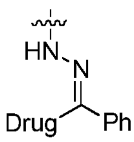, 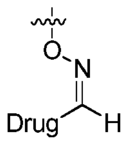, 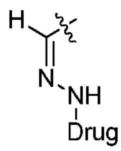,

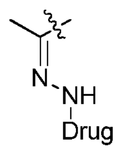 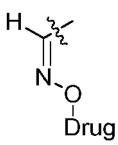, 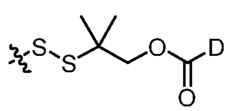

). The linker specifically degraded by the enzyme is a linker formed based on 1) amino acid residues or peptides. Peptide bonds can have good serum stability because the activity of lysosomal proteolytic enzymes in blood is much lower than that in some tumor tissues. Therefore, the linker can be selectively degraded in some tumor tissues or cells to release the small molecule drug D. The lysosomal enzymes may be selected from the group consisting of cathepsin B, cathepsin S, plasmin, elastase, β-glucuronidase or β-galactosidase, etc. The peptide-based linker ($-(A)_V-$) may be a tetrapeptide (including but not limited to: -Gly-Phe-Leu-Gly-, -Ala-Leu-Ala-Leu-, -Gly-Gly-Phe-Gly-, -Leu-Ser-Gly-lys-, -Ala-Ala-Pro-Val-, -Glu-Val-Ala-Gly-, -Glu-Val-Cit-Gly-, -Leu-Ala-Glu-Gly-), a tripeptide (including but not limited to: -Val-Leu-Lys-, -Ala-Pro-Val-, -Ala-Ala-Asn-, -Glu-Val-Cit-, -Leu-Ala-Glu-, - Glu-Val-Ala-, -Val-Cit-Gly-, -Val-Ala-Gly-, -Leu-Ala-Glu-), a dipeptide (including but not limited to: -Val-Cit-, -Cit-Val-, -Val-Ala-, -Ala-Val-, -Ala-Cit-, -Cit-Ala-, -Asn-Cit-, -Cit-Asn-, -Cit-Cit-, -Val-Glu-, -Glu-Val-, -Ser-Cit-, -Cit-Ser-, -Lys-Cit-, -Cit-Lys-, -Asp-Cit-, -Cit-Asp-, -Ala-Val-, -Val-Ala-, -Phe-Lys-, -Lys-Phe-, -Val-Lys-, -Lys-Val-, -Ala-Lys-, -Lys-Ala-, -Phe-Cit-, -Cit-Phe-, -Leu-Cit-, -Cit-Leu-, -Ile-Cit-, -Cit-Ile-, -Phe-Arg-, -Arg-Phe-, -Cit-Trp-, -Trp-Cit-, -Pro-Val-) or an amino acid monomer (including but not limited to:-Lys-,-Gly-,-Cit-). The peptide-based linker is

preferably a dipeptide linker, a tripeptide linker or a tetrapeptide linker, and more preferably a dipeptide linker. The linker specifically degraded by the enzyme is 2) a linker formed by a pyrophosphate or a phosphate. Lysosomal acid pyrophosphatases and acid phosphatases are enzymes that hydrolyze pyrophosphates and terminal monophosphates, respectively, into their parent alcohols in lysosomes. Targeting these enzymes can effectively release the small molecule drug D with an alkyl alcohol at the terminus. The other degradable linkers may include ester linkages formed by the reaction of PEG carboxylic acid or activated PEG carboxylic acid with hydroxyl on the small molecule drug D, where such ester linkages can be hydrolyzed under physiological conditions to release the small molecule drug D. Hydrolyzable linkages include, but are not limited to, carbonate bonds, imine linkages generated by the reaction of amines and aldehydes, phosphate bonds obtained by the reaction of hydroxyl and phosphate groups, acetal linkages obtained by the reaction of hydroxyl and aldehydes, and orthoester linkages obtained by the reaction of formates and hydroxyl. The cleavable linkers may also include noncleavable fragments, including, for example, alkylene chains and polymers thereof, polyethylene glycol (PEG) and related polymers thereof, aromatic rings or non-aromatic rings, and combinations thereof.

In the present disclosure, the "linker" may further include a spacer group, and the linker may be directly linked to the linker in the linker body, or may be linked to the linker in the linker body through the spacer group. The spacer group may be polyethylene glycol and related polymers thereof, an alkylene group including 1 to 10 carbon atoms, cyclohexyl, phenyl, 1,3-dioxane, amide, an ester group, oxo, substituted amino, triazolyl or

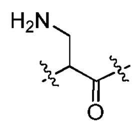,

and a combination of any one or more of the above spacer groups.

In the present disclosure, the "linker" may further include a self-immolative group that spatially separates the small molecule drug D and the enzymatic degradation site. Common self-immolative groups typically include the following basic structures:

1)

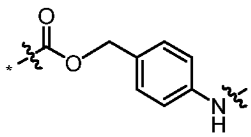,

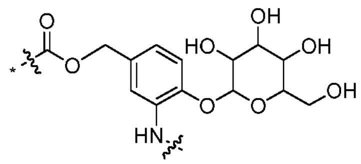

(preferably being

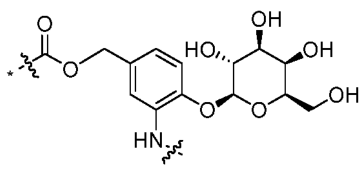

),

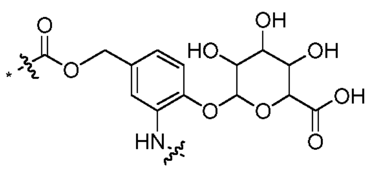

(preferably being

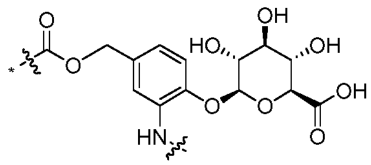

),

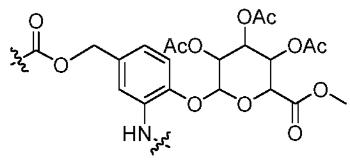

(preferably being

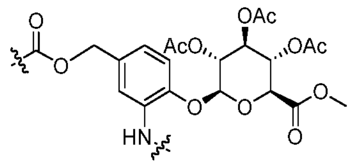

),

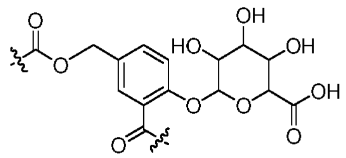

(preferably being

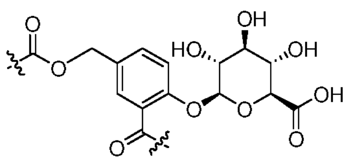

),

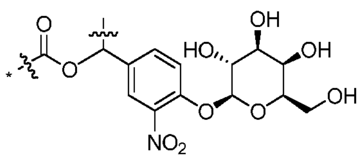 , 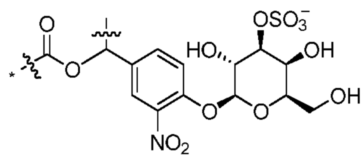

or

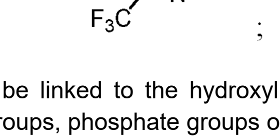 ;

The self-immolative groups can also be linked to the hydroxyl groups on the small molecule D through aminomethylene groups, amino $C_{3-5}$ alkanoyl groups, phosphate groups or pyrophosphate groups. 2)

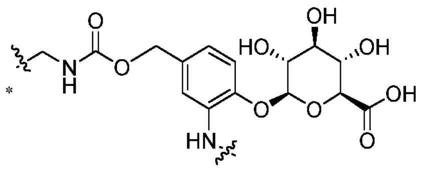,
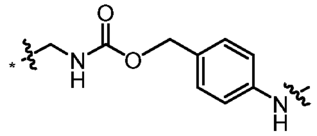,
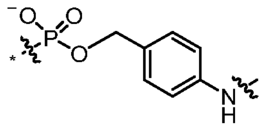,

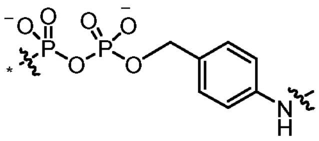 or 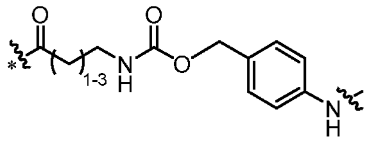;

the site marked with * in (1) is the site linked to the small molecule drug D, preferably linked to the amino group on the small molecule drug D; and the site marked with * in (2) is the site linked to the small molecule drug D, preferably linked to hydroxyl on the small molecule drug D.

In the present disclosure, the "linker" also includes a linker head, and the "linker head" is attached to the antibody, which can be formed by the reaction of the linker head precursor with groups in the antibody such as thiol (e.g. cysteine), amino (e.g. lysine), carbonyl (e.g. p-acetylphenylalanine), an aldehyde group, an azide group (e.g. p-azidomethylphenylalanine),a phenol groups (e.g. tyrosine).

In the present disclosure, the "linker head precursor" can react with the antibody and form a linker head attached to the antibody.

In the present disclosure, the linker head precursor may react with thiol (e.g. cysteine), amino (e.g. lysine), carbonyl (e.g. p-acetylphenylalanine), an aldehyde group, an azide group (e.g. p-azidomethylphenylalanine),a phenol groups (e.g. tyrosine), etc. in the antibody.

In the present disclosure, the linker head precursor may react with thiol in the antibody to form a linker head attached to the antibody, preferably conjugated with 1, 2, 3 or 4 cysteine side chain thiol in the antibody, and more preferably conjugated with 1 or 2 cysteine side chain thiol in the antibody. The linker head precursor may be: 1) a maleimide linker head precursor. For example, i) marketed ADC drugs Enhertu®, Trodelvy® are obtained by randomly conjugating maleimide (

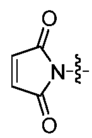

) with cysteine in an antibody, and ii) CN103933575B discloses a class of tridentate linker head precursors (

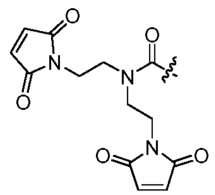,

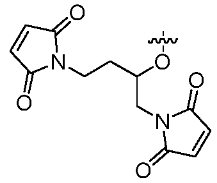, 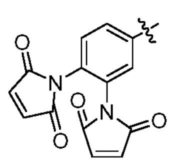, 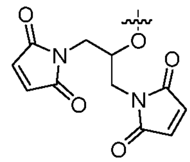

) including a maleimide linker head precursor, which can be simultaneously conjugated with 2 cysteine side chain thiol in an antibody; 2) a substituted maleimide (including 3-substituted maleimide or 3-, 4-disubstituted maleimide) linker head precursor. For example, i) CN103933575B and Mol. Pharmaceutics 2015, 12, 3986-3998 disclose a class of brominated maleimide linker head precursors (

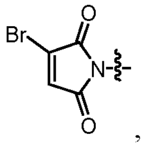
,
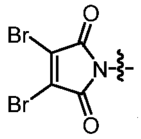

), ii) Org. Biomol. Chem., 2014, 12, 7261-7269 discloses a class of phenylthio-substituted maleimide linker head precursors (

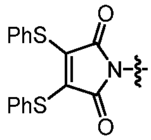

), iii) WO2014197871A2 discloses a class of 2-pyridylthio-substituted maleimide linker head precursors (

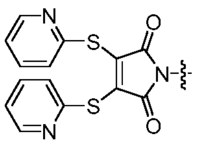

), and iv) CN111433188B discloses a class of tridentate linker head precursors (

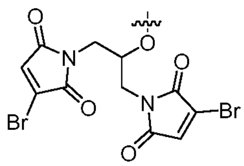
,

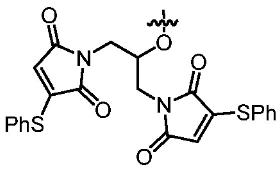
,
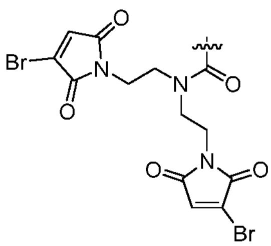
,
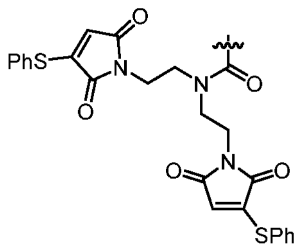

) containing substituted maleimide linker head precursors, which can be simultaneously conjugated with 2, 3 or 4 cysteine side chain thiol in an antibody; 3) α-haloamide linker head precursor. For example: i) Bioconjugate Chem. 2008, 19, 759-765 discloses an α-bromoamide linker head precursor (

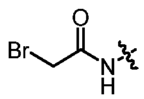

), and ii) Bioorganic Med. Chem. Lett., 2017, 27, 1154-1158 discloses an α-iodoamide linker head precursor (

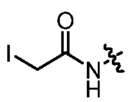

); 4) a methanesulfonyl-substituted heteroaromatic ring linker head precursor. For example: i) WO2016142049A1 and US20220024904A1 disclose a methanesulfonyl-substituted oxadiazole linker head precursor (

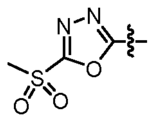, 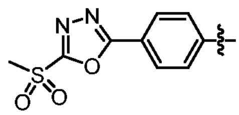, 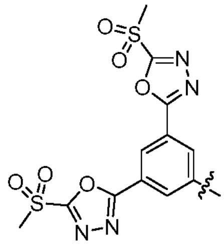

), ii) CN113698414A discloses a class of methanesulfonyl-substituted pyrimidine linker head precursors (

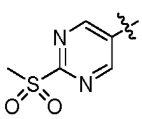, 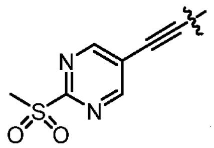

), and iii) CN112341521A discloses a class of methanesulfonyl-substituted pyrrolidinone[3,4-d]pyrimidine linker head precursors (

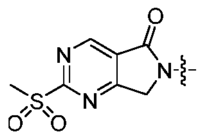

); 5) a halogenated heteroaryl or halogenated methyl heteroaryl linker head precursor. For example, i) CN104379168B discloses a series of bromo (chloro) heteroaryl or bromo (chloro) methyl heteroaryl linker head precursors (

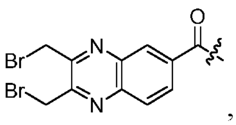, 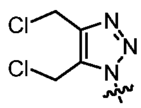, 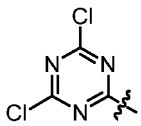

), and ii) CN114106088A discloses a class of bromomethylpyrazinyl linker head precursors (

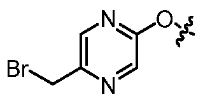,

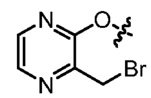, 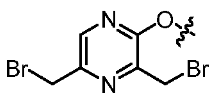, 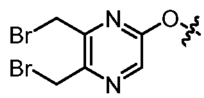, 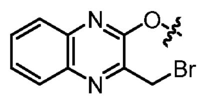

); 6) a pyridazinedione linker head precursor. For example, RSC Adv., 2017, 7, 9073-9077; Org. biomol. Chem., 2018, 16, 1359-1366; and WO2023076848A1 disclose a class of dibromopyridazinedione linker head precursors (

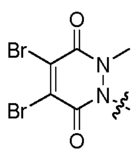,

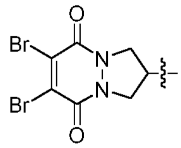

); 7) an alkenyl-substituted heteroaryl or dienyl-substituted heterocyclic linker head precursor. For example, i) US20210308275A1 discloses a class of divinylpyrimidine linker head precursors (

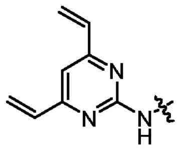
,
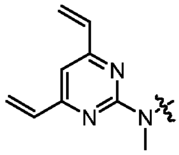

), ii) Org. Biomol. Chem., 2020, 18, 4739-4743 discloses a class of divinyltriazine linker head precursors (

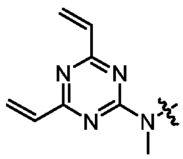

), iii) Chem. Sci., 2021, 12, 9060-9068 discloses a class of vinylpyrimidine (triazine) linker head precursors (

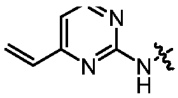
,
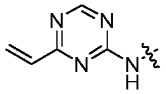

), and iv) US10772965B2 discloses a class of diacrylonyl-substituted triazine linker head precursors (

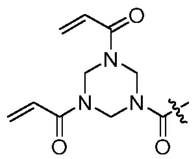

); 8) a p-toluenesulfonyl linker head precursor. For example, WO2005007197A2 and Bioconjugate Chem. 2007, 18, 61-76 disclose a class of p-toluenesulfonyl linker head precursors (

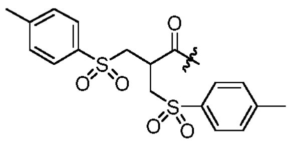
,
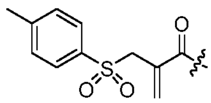

); 9) an arylpropynenitrile linker head precursors. For example: CN105474015B; Bioconjugate Chem. 2014, 25, 2, 202-206; and Med. Chem. Commun., 2018, 9, 827-830 disclose a class of arylpropynenitrile linker head precursors (

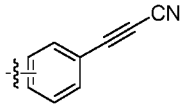
,

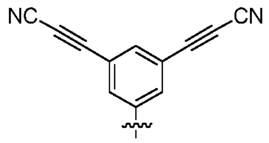

); 10) a carbonylacrylic acid linker head precursor. For example, Nat. Commun., 2016, 7, 13128 discloses a class of carbonylacrylic acid linker head precursors (

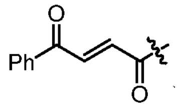

); and 11) a 5-methylenepyrrolidone linker head precursor. For example, Nat. Commun., 2020, 11(1015), 1-10 discloses a class of 5-methylene-pyrrolidone linker head precursors (

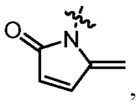, 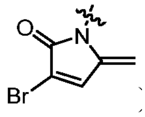

).

In the present disclosure, the linker head precursor may react with amino in the antibody to form a linker head attached to the antibody, each linker head precursor is preferably conjugated with 1 lysine side chain amino in the antibody, and the conjugating preferably forms *an amide bond by reacting lysine side chain amino with activated carboxyl in the linker head precursor.* The linker head precursor may be: 1) an NHS ester *linker head precursor. For example,* the marketed ADC drugs Mylotarg®, Kadcyla®, etc. are obtained by randomly conjugating NHS-activated esters (

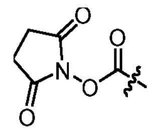

) and lysine in an antibody; and 2) a phenol ester linker head precursor. For example, RSC Advances, 2012, 2, 908-914 and US20120201809A1 disclose a series of phenol ester linker head precursors (

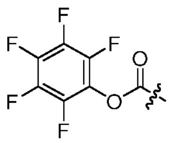, 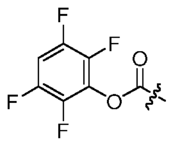, 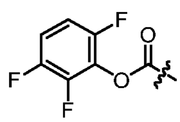,

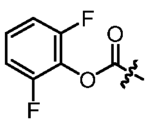, 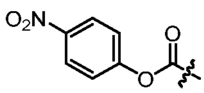

).

Such linker head precursors that can react with side chain thiol of the amino acid residue in the antibody include, but are not limited to: vinyl,

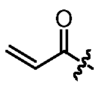, 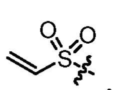, 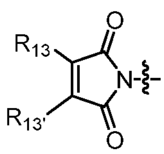, 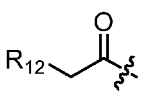,

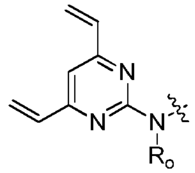, 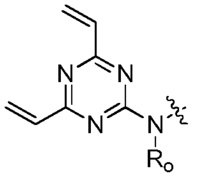, 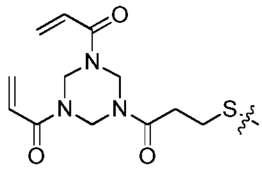, 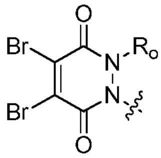, 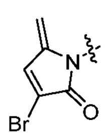,

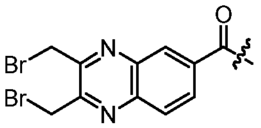, 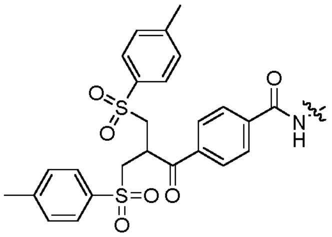, 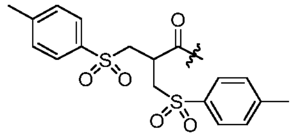, 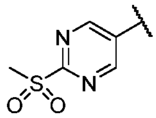,

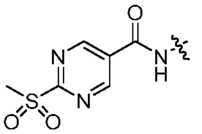, 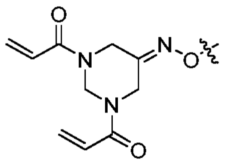, 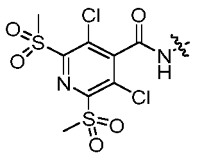, 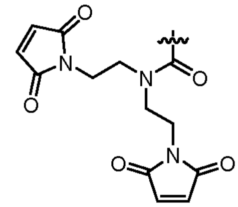

or

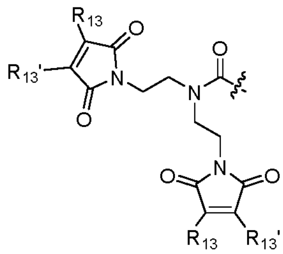;

and the definitions of $R_{12}$, $R_{13}$, $R_{13'}$ and $R_o$ are as previously described. The reaction between the linker head precursor and thiol (for example, cysteine) in the antibody is specific, for example, as shown in formulas 1 to 6, 12 to 16:

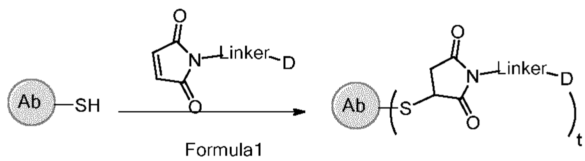

,
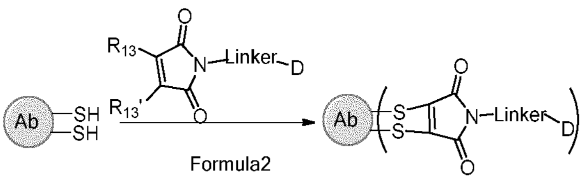

,

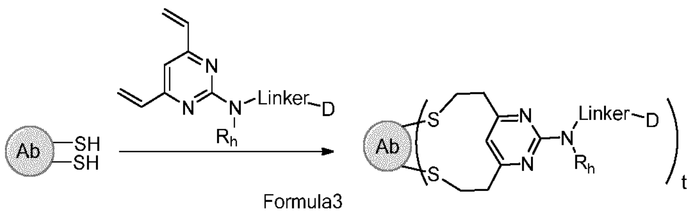

,

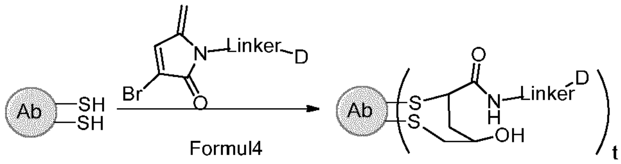
Formul4

,

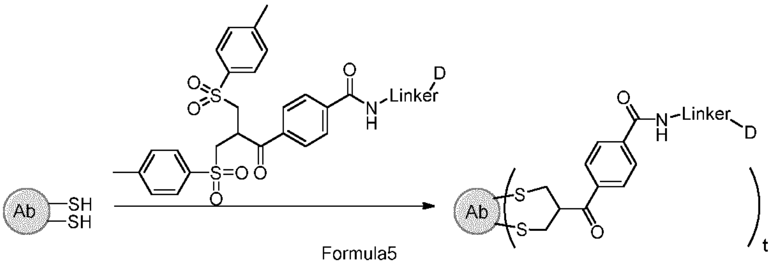
Formula5

,

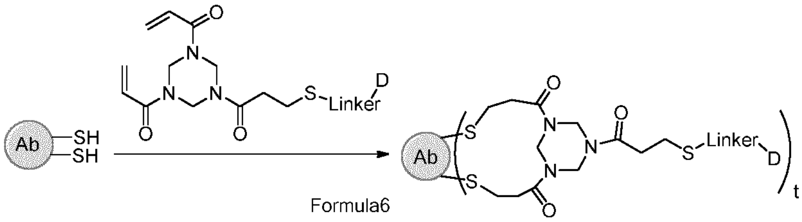
Formula6

,

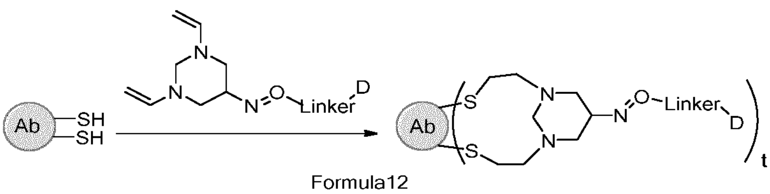
Formula12

,

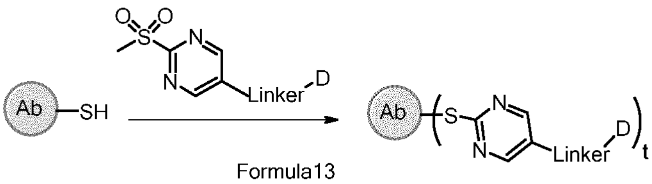
Formula13

,

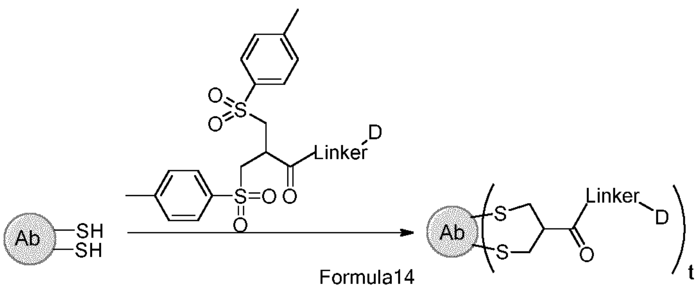
Formula14

,

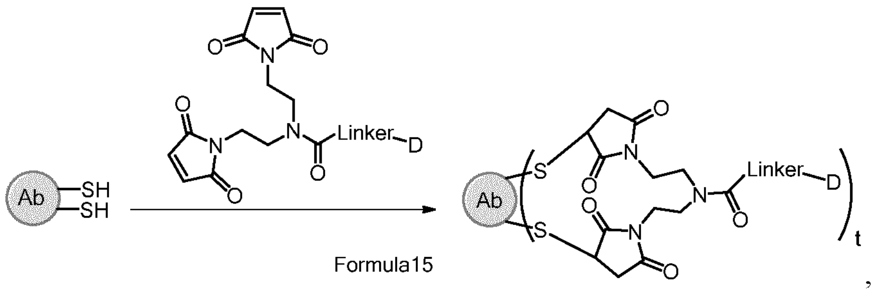

Formula15 ,

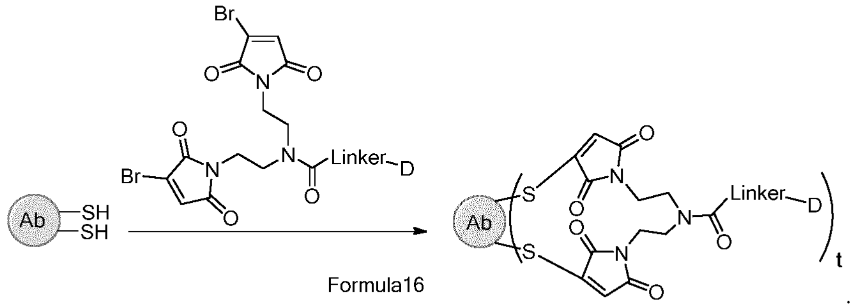

Formula16 .

When the linker head is maleimide or succinimide, it can be further hydrolyzed to obtain a hydrolysate, for example, as shown in reaction formula 1' or reaction formula 2':

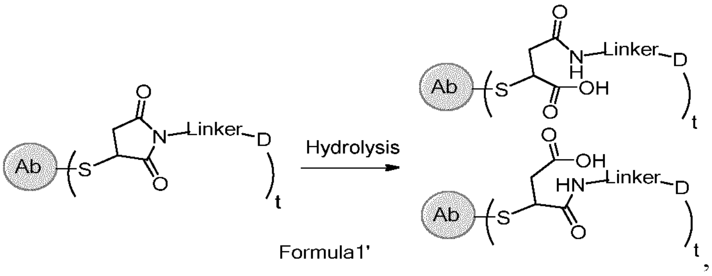


Formula1' ,

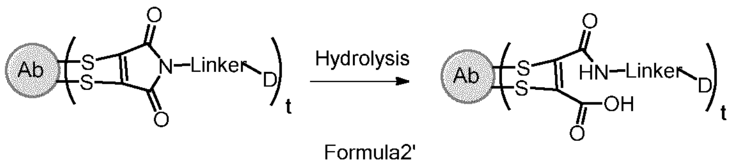


Formula2' .

Such linker head precursors that can react with side chain azide of the amino acid residue in the antibody include, but are not limited to: ethynyl,

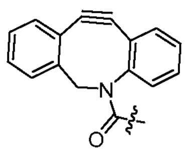 , 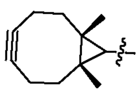 ;

and the click reaction between the linker head precursor and azide (for example, azidomethylphenylalanine) in the antibody is specific, for example, as shown in formula 7:

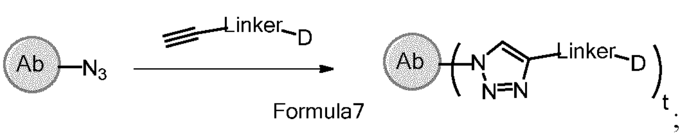

Formula7 ;

Such linker head precursors that can react with side chain carbonyl or aldehyde of the amino acid residue in the antibody include, but are not limited to, hydroxylamino or hydrazino; and the reaction between the linker head precursor and carbonyl (for example, p-acetylphenylalanine) or an aldehyde group in the antibody is specific, for example, as shown in formulas 8 to 9:

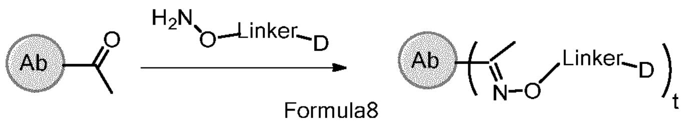

,

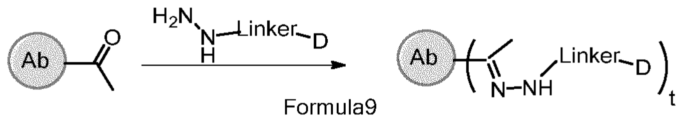

.

The linker head precursors that can react with side chain phenol of the amino acid residue in the antibody include, but are not limited to:

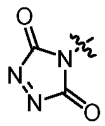
;

and the click-like reaction of the linker head precursor and phenol (e.g., tyrosine) in the antibody is specific, for example, as shown in formula 10:

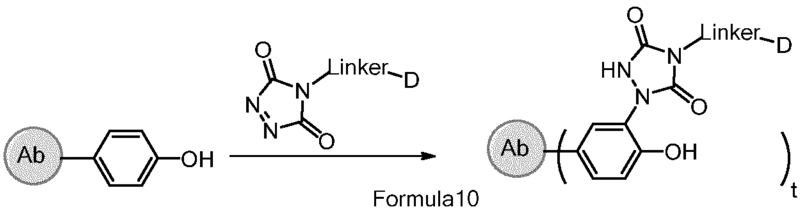

;

The linker head precursor that directly reacts the linker with side chain amino of the amino acid residue in the antibody by reaction is an active ester group, including but not limited to:

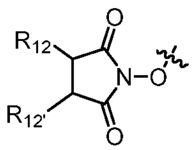
,

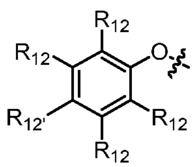
;

definitions of $R_{12}$ and $R_{12'}$ are as previously described; and the reaction of the linker head precursor with amino (e.g., lysine) in the antibody is specific, for example, as shown in formula 11:

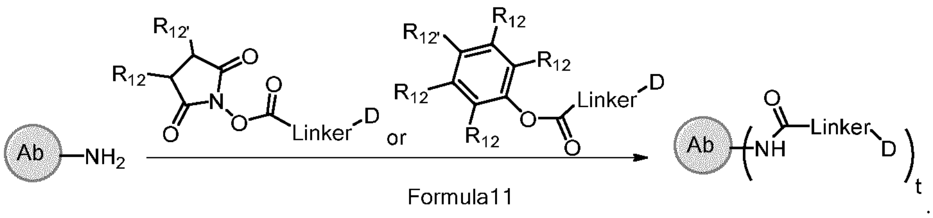

Formula11

The term "linker precursor" may be reacted directly with the antibody, or may be reacted sequentially with the remaining linker parts, the antibody, and form a degradable or non-degradable linker for linking the small molecule drug D and the antibody.

The term "and/or" includes any and all combinations of one or more related listed items.

The term "$C_{A-B}$" refers to a range including a starting point to an ending point, where A and B and each point in the range thereof is an integer indicating the number of carbon atoms. For example, $C_{1-4}$ indicates that the number of carbon atoms is 1, 2, 3 or 4; $C_{1-6}$ indicates that the number of carbon atoms is 1, 2, 3, 4, 5 or 6; $C_{3-8}$ indicates that the number of carbon atoms is 3, 4, 5, 6, 7 or 8; $C_{A-B}$ may be used in conjunction with any group including carbon atoms to define the number of carbon atoms, such as $C_{1-6}$ alkyl, $C_{3-8}$ cycloalkyl, $C_{6-10}$ aryl, $C_{1-4}$ alkoxy, $C_{3-8}$ cycloalkyl$C_{1-4}$ alkyl, etc.

The term "alkyl" refers to a saturated linear or branched hydrocarbon group including 1 to 20 carbon atoms, preferably 1 to 10 carbon atoms, more preferably 1 to 8, 1 to 6, 1 to 4 or 1 to 3 carbon atoms. Representative examples of alkyl include, but are not limited to: methyl, ethyl, n-propyl, isopropyl, n-butyl, sec-butyl, tert-butyl, isobutyl, n-pentyl, n-hexyl, n-heptyl, octyl, nonyl, decyl, 1,1-dimethylpropyl, 1,2-dimethylpropyl, 2,2-dimethylpropyl, 1-ethylpropyl, 2-methylbutyl, 3-methylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2,3-dimethylbutyl, 2-ethylbutyl, 2-methylpentyl, 3-methylpentyl, 4-methylpentyl, 4,4-dimethylpentyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, 2,2,4-trimethylpentyl, undecyl, dodecyl, and various isomers thereof, etc.

The term "alkenyl" refers to a linear or branched non-aromatic hydrocarbon group containing at least one carbon-carbon double bond. There may be 1 to 3 carbon-carbon double bonds, preferably 1 carbon-carbon double bond. The term "$C_{2-4}$ alkenyl" refers to alkenyl having from 2 to 4 carbon atoms, and the term "$C_{2-6}$ alkenyl" refers to alkenyl having from 2 to 6 carbon atoms, including vinyl, propenyl, butenyl, 2-methylbutenyl.

The term "alkynyl" refers to a linear or branched hydrocarbon group containing at least one carbon-carbon triple bond. There may be 1 to 3 carbon-carbon triple bonds, preferably 1 carbon-carbon triple bond. The term "$C_{2-6}$ alkynyl" refers to alkynyl having 2 to 6 carbon atoms, including ethynyl, propynyl, butynyl and 3-methylbutynyl.

The term "alkylene" refers to saturated linear or branched unbridged divalent alkyl including 1 to 20 carbon atoms, preferably 1 to 10 carbon atoms, and more preferably 1 to 8, 1 to 6 or 1 to 4 carbon atoms, and examples include but are not limited to $-CH_2-$, $-CH_2CH_2-$, $-CH_2CH_2CH_2-$, $-CH_2CH_2CH_2CH_2-$, - $CH_2CH(CH_3)-$, $-CH_2C(CH_3)_2CH_2-$, $-CH_2C(CH_3)_2CH_2CH_2-$, $=CH_2$, $=CHCH_3$, $=C(CH_3)_2$.

The term "alkenylene" refers to a linear or branched non-aromatic divalent hydrocarbon group including at least 1 carbon-carbon double bond, where there may be 1 to 3 carbon-carbon double bonds, preferably 1 carbon-carbon double bond. The alkenylene preferably includes 2 to 10 carbon atoms, more preferably 2 to 8, 2 to 6 or 2 to 4 carbon atoms.

The term "alkynylene" refers to a linear or branched non-aromatic divalent hydrocarbon group including at least one carbon-carbon triple bond. There may be 1 to 3 carbon-carbon triple bonds, preferably 1 carbon-carbon triple bond. The alkynylene preferably includes 2 to 10 carbon atoms, more preferably 2 to 8, 2 to 6 or 2 to 4 carbon atoms.

The term "cycloalkyl" refers to a saturated or partially unsaturated (including 1 or 2 double bonds) monocyclic or polycyclic group including 3 to 20 carbon atoms. "Monocyclic cycloalkyl" is preferably 3-10 membered monocycloalkyl, more preferably 3-8 membered monocycloalkyl, such as cyclopropyl, cyclobutyl, cyclopentyl, cyclohexyl, cycloheptyl, cyclooctyl, cyclodecyl, cyclododecyl, cyclohexenyl. "Polycyclic cycloalkyl" includes "bridged ring groups", "fused cycloalkyl" and "spirocycloalkyl", and representative examples of "bridged ring groups" include, but are not limited to, bornyl, bicyclo[2.2.1]heptenyl, bicyclo[3.1.1]heptanyl, bicyclo[2.2. l]heptanyl, bicyclo[2.2.2]octanyl, bicyclo[3.2.2]nonanyl, bicyclo[3.3.1]nonanyl, bicyclo[4.2.1]nonanyl and adamantyl, etc. "Fused cycloalkyl" includes a cycloalkyl ring fused to phenyl, cycloalkyl, or heteroaryl, including, but not limited to, benzocyclobutenyl, 2,3-dihydroindenyl, decahydronaphthyl, etc. Monocyclic cycloalkyl or polycyclic cycloalkyl may be attached to the parent molecule by any carbon atom on the ring.

The term "cycloalkylene" refers to divalent cycloalkyl. Thus, "cycloalkylene" includes the above definition of cycloalkyl. "Cycloalkylene" is preferably $C_{3-10}$ cycloalkylene, and more preferably $C_{3-8}$ cycloalkylene or $C_{3-6}$ cycloalkylene.

The term "heterocycloalkyl" refers to a saturated or partially unsaturated (containing 1 or 2 double bonds) non-aromatic cyclic group consisting of carbon atoms and heteroatoms selected from nitrogen, oxygen, sulfur, boron, etc. and/or sulfur-containing heteroatom groups, and this cyclic group may be a monocyclic or polycyclic group, where the

sulfur-containing heteroatom groups are selected from, but not limited to, S(O), $S(O)_2$ and S(O)(NH). In the present disclosure, the number of heteroatoms and/or heteroatom groups in heterocycloalkyl is preferably 1, 2, 3 or 4, and the boron, nitrogen or carbon atoms in heterocycloalkyl may optionally be oxidized. The nitrogen atom may optionally be further substituted with other groups to form a tertiary amine or a quaternary ammonium salt. "Monocyclic heterocycloalkyl" is preferably 3-10 membered monocyclic heterocycloalkyl, and more preferably 3-8 membered monocyclic heterocycloalkyl. Examples thereof include pyrrolidinyl, dihydropyrrolidinyl, dihydroimidazolyl, dihydropyrazolyl, tetrahydrofuranyl, tetrahydropyrazinyl, dihydrofuranyl, tetrahydrothiopyranyl, tetrahydropyranyl, dihydropyranyl, tetrahydropyranyl, tetrahydropyridinyl, tetrahydropyrimidinyl, piperidinyl, aziridinyl, morpholinyl, thiomorpholinyl, thiomorpholine-S-oxide-4-yl, piperidinyl, piperazinyl, 1,4-dioxanyl, homopiperazinyl, 1-imino-1-oxytetrahydro-2*H*-thiopyranyl, 1,1-dioxytetrahydrothienyl, 1-imino-1-oxotetrahydrothienyl, 1,1-dioxo-3,4-dihydro-2*H*-thiopyranyl, 1-imino-1-oxo-3,4-dihydro-2H-thiopyranyl, 1,1-dioxo-2,3-dihydrothienyl, 1-imino-1-oxo-2,3-dihydrothienyl, etc. "Polycyclic heterocycloalkyl" includes "fused heterocycloalkyl," "spiroheterocyclyl," and "bridged heterocycloalkyl". "Fused heterocycloalkyl" includes a monocyclic heterocycloalkyl ring fused to phenyl, cycloalkyl, heterocycloalkyl or heteroaryl, including but not limited to: 2,3-dihydrobenzofuranyl, 1,3-dihydroisobenzofuranyl, indolinyl, 2,3-dihydrobenzo[b]thienyl, dihydrobenzopiperanyl, 1,2,3,4-tetrahydroquinolinyl, 1,2,3,4-tetrahydroisoquinolinyl, 1,2,3,4-tetrahydronaphthyridinyl, 5,6,7,8-tetrahydronaphthyridinyl, 1-hydroxy-1,3-dihydrobenzo[*c*][1,2]oxaborolanyl, etc. Monocyclic heterocycloalkyl and polycyclic heterocycloalkyl may be attached to the parent molecule through any ring atom on the ring. The above ring atoms specifically refer to carbon atoms and/or nitrogen atoms constituting the ring skeleton.

The term "heterocycloalkylene" refers to divalent heterocycloalkyl. Thus, "heterocycloalkylene" includes the above definition of heterocycloalkyl. "Cycloalkylene" is preferably 3-10 membered heterocycloalkylene, and more preferably 3-8 membered heterocycloalkylene or 3-6 membered heterocycloalkylene.

The term "cycloalkylalkyl" means that cycloalkyl is attached to the parent nucleus structure through alkyl. Thus, "cycloalkylalkyl" includes the above definitions of alkyl and cycloalkyl.

The term "heterocycloalkylalkyl" means that heterocycloalkyl is attached to the parent nucleus structure through alkyl. Thus, "heterocycloalkylalkyl" includes the above definitions of alkyl and heterocycloalkyl.

The term "alkoxy" refers to alkyloxy having the stated number of carbon atoms linked by an oxygen bridge. Thus, "alkoxy" includes the above definition of alkyl.

The term "hydroxyalkyl" means that any one of the hydrogen atoms on alkyl is substituted by hydroxyl, including, but not limited to: $-CH_2OH$, $-CH_2CH_2OH$, $-CH_2CH_2C(CH_3)_2OH$.

The term "heterocycloalkenyl" refers to a cyclic, unsaturated monovalent hydrocarbon group having the specified number of ring atoms (e.g., 5 to 10 membered), the specified number of heteroatoms (e.g., 1, 2 or 3), and the specified heteroatom species (one or more of N, O and S), having one or more (e.g., 1, 2 or 3) carbon-carbon $sp^2$ double bonds, which is monocyclic and does not have aromaticity. Heterocycloalkenyl is attached to the rest of the molecule through a carbon atom or a heteroatom. Heterocycloalkenyl includes, but is not limited to:

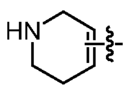,
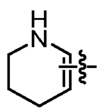,
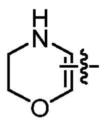,
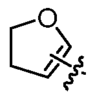,

etc.

The term "heteroalkenyl ring" satisfies any of the following conditions, and the remaining definitions are the same as the term "heterocycloalkenyl": sharing two atoms and one bond with the rest of the molecule.

The term "aryl" refers to any stable 6-20 membered monocyclic or polycyclic aromatic group, e.g., phenyl, naphthyl, tetrahydronaphthyl, 2,3-dihydroindenyl or biphenyl, etc.

The term "arylene" refers to divalent heteroaryl. Thus, "arylene" includes the above definition of aryl. "Arylene" is preferably $C_{6-10}$ arylene, and more preferably phenylene.

The term "heteroaryl" refers to an aromatic ring group formed by replacing at least one carbon atom on the ring with a heteroatom selected from nitrogen, oxygen or sulfur, which may be a 5-7 membered monocyclic structure or a 7-20 fused ring structure, and preferably 5-6 membered heteroaryl. In the present disclosure, the number of heteroatoms is preferably 1, 2 or 3, including: pyridinyl, pyridinonyl, pyrimidinyl, pyrimidine-2,4(1H,3H)-dionyl, pyrimidinonyl, piperazinyl, pyridazinonyl, furanyl, thienyl, thiazolyl, pyrrolyl, imidazolyl, pyrazolyl, oxazolyl, isoxazolyl, 1,2,5-oxadiazolyl, 1,2,4-oxadiazolyl, 1,3,4-oxadiazolyl, 1,3,4-thiadiazole, 1,2,4-triazolyl, 1,2,3-triazolyl, tetrazolyl, indazolyl, isoindazolyl, indolyl, isoindolyl, benzofuranyl, benzothienyl, benzo[d][1,3]dioxolanyl, benzothiazolyl, benzoxazolyl, quinolinyl, isoquinolinyl, isoquinolinonyl, quinazolinyl, 4-hydroxythieno[3,2-c]pyridyl, 4,5-dihydro-4-oxofuran[3,2]pyridyl, 4-hydroxy-5-azaindolyl, furo[2,3-c]pyridin-7(6H)-onyl, thieno[2,3-c]pyridin-7(6H)-onyl, etc.

The term "heteroarylene" refers to divalent heteroaryl. Thus, "heteroarylene" includes the above definition of heteroaryl. The "heteroarylene" is preferably 5-10 membered heteroarylene, and more preferably 5-6 membered

heteroarylene.

The term "fused ring group" refers to a fused ring structure formed by two, three or four cyclic structures sharing two adjacent atoms with each other, and the fused ring group may further include a spiro or bridged ring group. The fused ring group referred to in the present disclosure is a saturated, unsaturated or partially saturated fused ring structure, and preferably at least one ring is an aromatic ring. More preferably the fused ring group is a bicyclic or tricyclic acyclic group, and at least one ring is an aromatic ring. In the present disclosure, the fused ring group is an 8-20 membered fused ring group, preferably an 8-12 membered fused ring group, and more preferably an 8-10 membered fused ring group. Specific examples of the fused ring group include, but are not limited to, benzocyclobutenyl, 2,3-dihydro-1H-indenyl, 1,2,3,4-tetrahydronaphthyl, 6,7,8,9-tetrahydro-5H-benzo[7]annulenyl, 6,9-dihydro-5H-benzo[7]annulenyl, 5,6,7,8,9,10-hexahydrobenzo[8]annulenyl, 2,3-cyclopentenopyridyl, 5,6-dihydro-4H-cyclopentyl[b]thienyl, 5,6-dihydro-4H-cyclopentyl[b]furanyl, 2,3-dihydrobenzofuranyl, 1,3-dihydroisobenzofuranyl, indolinyl, 2,3-dihydrobenzo[b]thienyl, dihydrobenzopyranyl, 1,2,3,4-tetrahydroquinolinyl, 2,3-dihydro-1,4-benzodioxanyl, 3,4-dihydro-2H-1,4-benzoxazinyl, naphthyridinyl, naphthyl, benzofuryl, benzothienyl, benzopyrrolyl, benzothiazolyl, benzoxazolyl, indazolyl, benzopyridazinyl, benzimidazolyl, indolyl, quinolinyl, isoquinolinyl, purinyl, pteridinyl,

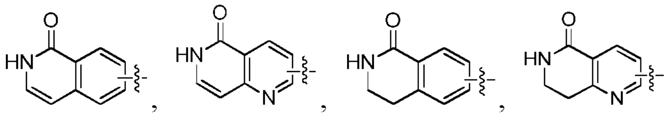 , , , ,

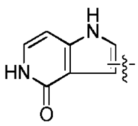 , 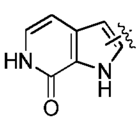 , 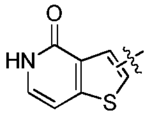 , 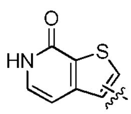 , 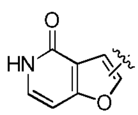 , 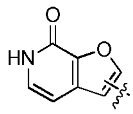 , 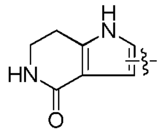 ,

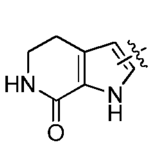 , 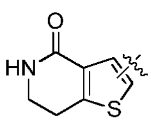 , 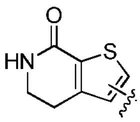 , 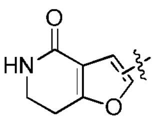 , 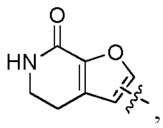 , 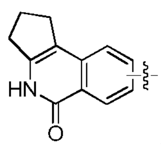 , 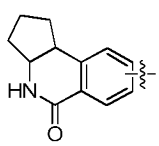 ,

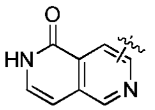 , 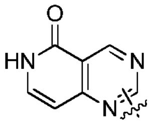 , 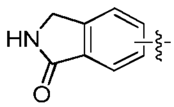 , 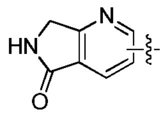 , 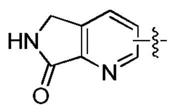 , 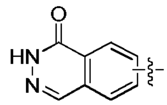 ,

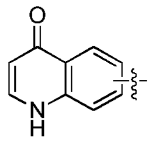 , 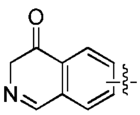 , 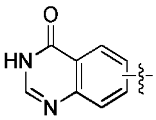 , 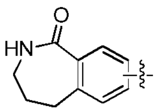 , 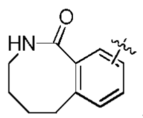 , 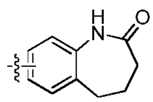 , 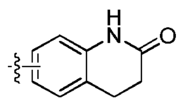 ,

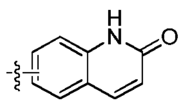 , 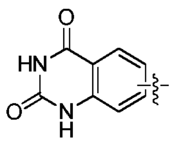 , 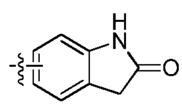 , 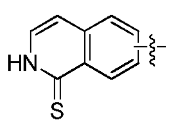 , 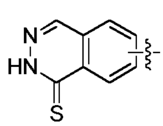 , 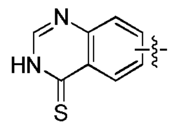 ,

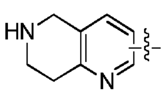 , 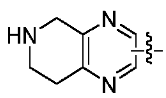 , 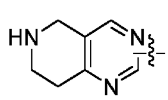 , 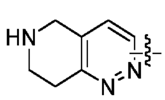 , 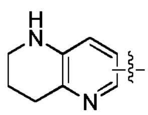 , 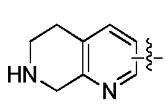 ,

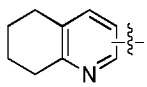, 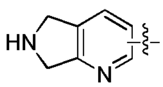, 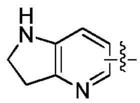, 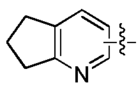, 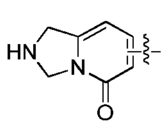, 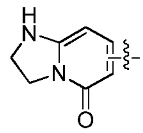, 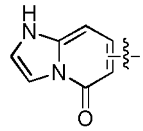,

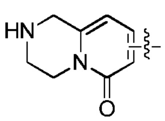, 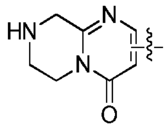, 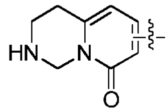, 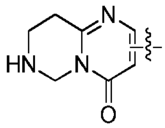, 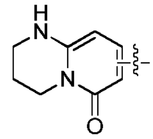, 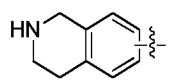,

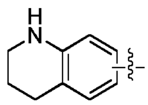, 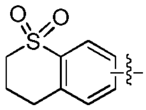, 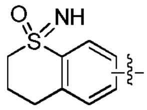, 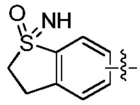, 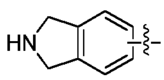, 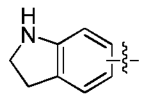, 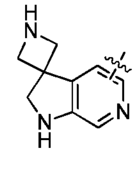,

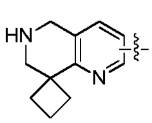, 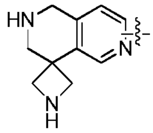, 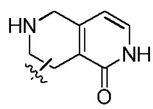, 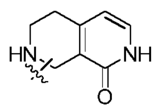 or 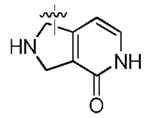;

and the fused ring group may be attached to the parent molecule through a ring carbon atom, and preferably through an aromatic ring carbon atom.

The term "arylalkyl" means that aryl is attached to the parent nucleus structure through alkyl. Thus, "arylalkyl" includes the above definitions of alkyl and aryl.

The term "heteroarylalkyl" means that heterocycloalkyl is attached to the parent nucleus structure through alkyl. Thus, "heteroarylalkyl" includes the above definitions of alkyl and heteroaryl.

The term "halogen" denotes fluorine, chlorine, bromine or iodine.

The term "haloalkyl" refers to alkyl optionally substituted with the halogen. Thus, "haloalkyl" includes the above definitions of the halogen and alkyl.

The term "haloalkoxy" refers to alkoxy optionally substituted with the halogen. Thus, "haloalkoxy" includes the above definitions of the halogen and alkoxy.

The term "amino" refers to $-NH_2$, and the term "alkylamino" means that at least one hydrogen atom on the amino group is substituted with alkyl, including, but not limited to: $-NHCH_3$, $-N(CH_3)_2$, $-NHCH_2CH_3$, $-N(CH_2CH_3)_2$. Thus, "alkylamino" includes the above definitions of alkyl and amino.

The term "nitro" refers to $-NO_2$.

The term "cyano" refers to -CN.

The term "oxo" refers to =O.

In the present disclosure, the abbreviations of amino acids are conventional abbreviations (refer to the Nomenclature of Organic Chemistry by the Chinese Chemical Society in 2017). For example: alanine (Ala), arginine (Arg), aspartic acid (Asp), asparagine (Asn), cysteine (Cys), glutamic acid (Glu), glutamine (Gln), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr), citrulline (Cit), valine (Val). In the present disclosure, the symbol of the peptide (taking -Gly-($-NH-CH_2-CO-$) as an example) indicates that when the short line of the peptide bond is to the right of Gly, it represents the removal of an OH group from the -COOH group of the amino acid, and when the short line is to the left of Gly, it represents the removal of an H atom from $-NH_2-$ of the amino acid.

The "room temperature" in the present disclosure refers to 15-30°C.

If tautomers are present among the substituents R, $R_1$, $R_2$, $R_3$, $R_4$, $R_{4'}$, $R_5$, $R_6$, $R_7$, $R_8$, $R_{8'}$, $R_9$, $R_{10}$, $R_{11}$, $R_{12}$, $R_{12'}$, $R_{13}$, $R_{13'}$, $R_a$, $R_b$, $R_d$, $R_e$, $R_{e'}$, $R_f$, $R_{f'}$ and $L_{1-5}$ in the "antibody-immunostimulatory conjugate", "compound" and "pharmaceutically acceptable salt" of the present disclosure, they may be present in the form of a single tautomer or a mixture thereof, preferably mainly in the form of a relatively stable tautomer.

The "pharmaceutically acceptable salt" in the present disclosure has been discussed in Berge, et al., "Pharmaceutically acceptable salts", J. Pharm. Sci., 66, 1-19 (1977) and is obvious to medicinal chemists, the salt is substantially non-toxic and can provide desired pharmacokinetic properties, palatability, absorption, distribution, metabolism or excretion, etc. The compound of the present disclosure may have acidic groups, basic groups or amphoteric groups,

and typical pharmaceutically acceptable salts include salts prepared by reacting the compound of the present disclosure with acids.

Without departing from common knowledge in the art, the above preferred conditions can be arbitrarily combined to obtain the preferred examples of the present disclosure.

The reagents and raw materials used in the present disclosure are commercially available.

The positive and progressive effects of the present disclosure lie in that the nitrogen-containing compound has a good regulatory effect on TLR8 and can effectively treat, alleviate and/or prevent various related diseases caused by immunosuppression, such as cancer.

## Description of the embodiments

Hereinafter, the present disclosure will be further described by way of examples, but the present disclosure is not limited to the scope of the described examples. The experimental methods for which specific conditions are not specified in the following examples are selected according to conventional methods and conditions, or according to commercial instructions.

The structures of all compounds of the present disclosure can be identified by nuclear magnetic resonance ($^1H$ NMR) and/or mass spectrometric detection (MS).

$^1H$ NMR chemical shifts ($\delta$) were recorded in PPM ($10^{-6}$). NMR was performed by a Bruker AVANCE-400 spectrometer. Suitable solvents were deuterated chloroform ($CDCl_3$), deuterated methanol ($CD_3OD$), deuterated dimethyl sulfoxide (DMSO-$d_6$), heavy water ($D_2O$), etc., with tetramethylsilane (TMS) as an internal standard.

Liquid chromatography-mass spectrometry (LCMS) was determined by an Agilent 1200 HPLC/6120 mass spectrometer using a chromatographic column: Xtimate C18, 3.0 $\times$ 50 mm, 3 $\mu$m, column temperature 40°C, or determined by a Thermo UltiMate 3000 HPLC/MSQ PLUS mass spectrometer using a chromatographic column Xbridge C18, 3.0 $\times$ 50 mm, 3.5 $\mu$m, column temperature 30°C. Agilent gradient elution condition 1: 95-5% solvent $A_1$ and 5-95% solvent $B_1$ (0-2.0 minutes), then 95% solvent $B_1$ and 5% solvent $A_1$ (held for 1.1 minutes), and the percentage is the volume percentage of a certain solvent to the total solvent volume. Solvent $A_1$: 0.01% trifluoroacetic acid (TFA) in water; solvent $B_1$: 0.01% trifluoroacetic acid solution in acetonitrile; and the percentage is the volume percentage of the solute in the solution. Thermo gradient elution condition 2: 95-5% solvent $A_2$ and 5-95% solvent $B_2$ (0-2 minutes), then 95% solvent $B_2$ and 5% solvent $A_2$ (held for 1.8 minutes), and the percentage is the volume percentage of a certain solvent to the total solvent volume. Solvent $A_2$: 10 mM ammonium bicarbonate in water; and solvent $B_2$: acetonitrile.

Preparative high performance liquid chromatography *(prep-HPLC)* Gilson GX-281 preparative liquid chromatography or Agela FLEXA-HP preparative liquid chromatography was used. The column was: Xtimate 21.2 * 250 mm, 10 $\mu$m; separation conditions 1: mobile phase A: 0.05% aqueous hydrochloric acid solution, mobile phase B: acetonitrile; separation conditions 2: mobile phase A: 10 mmol/L aqueous ammonium bicarbonate solution, mobile phase B: acetonitrile; and separation conditions 3: mobile phase A: 0.1% aqueous trifluoroacetic acid, mobile phase B: acetonitrile. Chromatographic column : Gemini 5 $\mu$m, C18, 150 * 21.2 nm; and mobile phase A: 0.1% aqueous formic acid solution, mobile phase B: acetonitrile. Detection wavelength: 214 nm & 254 nm; and flow rate: 15.0-20.0 mL/minutes.

Ultra-high performance liquid chromatography (UPLC) Waters ACQUITY Hclass was used; and the chromatographic column was: Waters ACQUITY UPLC BEH Shield RP18 2.1 mm * 100 mm, 1.7 $\mu$m, 5 $\mu$m, mobile phase A: 5 mm potassium dihydrogen phosphate buffer, pH adjusted to 2.5 with phosphoric acid, mobile phase B: acetonitrile. Gradient elution of mobile phase B From 90% to 60%, elution time 5 minutes, B from 60% to 10%, elution time 2 minutes, B held at 10% for 6 minutes, B from 10% to 90%, elution time 0.1 minutes, B held at 90% for 1.9 minutes. Detection wavelength: 214 & 262 nm; and column temperature: 40°C. Flow rate: 0.4 mL/min.

Flash column chromatography (flash system/CheetahTM) Agela Technologies MP200 was used, and the supporting normal phase separation column was Flash columm Silica-CS (25 g, 40 g, 80 g, 120 g or 330 g), Tianjin Agela, and the elution system was ethyl acetate/petroleum ether, or dichloromethane/methanol; the reverse phase separation column was a C18 reverse phase column (Spherical C18, 40-75 $\mu$m, 100 Å, SepaFlash® model: SW-040), and the elution system was 10 mM aqueous ammonium bicarbonate/acetonitrile or 0.1% aqueous trifluoroacetic acid/acetonitrile.

The abbreviations used in the embodiments of the present disclosure have the following meanings:

$(Boc)_2O$: di-tert-butyl dicarbonate; BINAP: 1,1'-binaphthyl-2,2'-bisdiphenylphosphine; DBAD: di-tert-butyl azodicarboxylate; DMF: N,N-dimethylformamide; DMSO: dimethyl sulfoxide; DIC: N,N'-diisopropylcarbodiimide; DIPEA: N,N-diisopropylethylamine; EDCI: 1-(3-dimethylaminopropyl)-3-ethylcarbodiimide hydrochloride; EEDQ: 2-ethoxy-1-ethoxycarbonyl-1,2-dihydroquinoline; HATU: 2-(7-azabenzotriazole)-N,N,N',N'-tetramethylurea hexafluorophosphate; HOBT: 1-hydroxybenzotriazole; LiHMDS: lithium bistrimethylsilylamine; NBS: N-bromosuccinimide; NHS: N-hydroxysuccinimide; $PdCl_2dppf\ CH_2Cl_2$: [1,1'-bis(diphenylphosphine)ferrocene]palladium dichloride dichloromethane complex; $Pd_2(dba)_3$: tris(dibenzylideneacetone)dipalladium; $Pd(PPh_3)_4$: tetrakis(triphenylphosphine)palladium; PyBOP: 1H-benzotriazol-1-yloxy tripyrrolidinyl hexafluorophosphate; TBSCl: tert-butyldimethylchlorosilane; Xantphos: 4,5-bisdiphenylphosphine-9,9-dimethyloxanthene; Xphos: 2-dicyclohexylphospho-2,4,6-triisopropylbiphenyl; Val-Cit-PAB-OH: (S)-2-((S)-2-amino-3-methylbutanamido)-N-(4-(hydroxymethyl)phenyl)-5-ureidopentanamide; Mc-Val-Cit-PAB-OH: 6-(2,5-dioxo-2,5-dihydro-1H-pyrrol-1-yl)-N-((S)-1-(((S)-1-((4-(hydroxymethyl)phenyl)amino)-1-oxo-5-ureidopentan-2-yl)amino)-3-methyl-1-oxobutan-2-yl)hexanamide; Fmoc-Cit-OH: (S)-2-((((9H-fluoren-9-yl)methoxy) carbonyl)amino)-5-ureidopentanoic acid; Fmoc-Val-Cit-OH: (S)-2-((S)-2-((((9H-fluoren-9-yl)methoxy)carbonyl)amino)-3-methylbutanoylamino)-5-ureidopentanoic acid; Fmoc-Val-Ala-OH: (((9H-fluoren-9-yl)methoxy)carbonyl)-L-valine-L-alanine; Fmoc-Gly-OH: (((9H-fluoren-9-yl)methoxy)carbonyl)glycine; Fmoc-Leu-OH: (((9H-fluoren-9-yl)methoxy)carbonyl)-L-leucine; Fmoc-Ala-OH: (((9H-fluoren-9-yl)methoxy) carbonyl)-L-alanine; Fmoc-Phe-OSu: 2,5-dioxopyrrolidin-1-yl(((9H-fluoren-9-yl)methoxy)carbonyl)-L-phenylalanine; Fmoc-Ser(tBu)-OH: N-(((9H-fluoren-9-yl)methoxy)carbonyl)-O-(tert-butyl)-L-serine; Fmoc-Asn (Trt)-OH: $N^2$-(((9H-fluoren-9-yl)methoxy)carbonyl)-$N^4$-trityl-L-asparagine; Boc-Val-Cti-OH: (S)-2-((S)-2-((tert-butoxycarbonyl)amino)-3-methylbutanoylamino)-5-ureidopentanoic acid; AMAS: 2,5-dioxopyrrolidin-1-yl 3-(2,5-dioxo-2,5-dihydro-1H-pyrrol-1-yl)acetate; BMPS: 2,5-dioxopyrrolidin-1-yl 3-(2,5-dioxo-2,5-dihydro-1H-pyrrol-1-yl)propionate; and EMCS: 2,5-dioxopyrrolidin-1-yl 6-(2,5-dioxo-2,5-dihydro-1H pyrrol-1-yl)hexanoate.

Synthesis of intermediates:

Synthesis of Intermediate 1.5: benzyl((2-formyl-1,3-dioxan-5-yl)methyl)carbamate

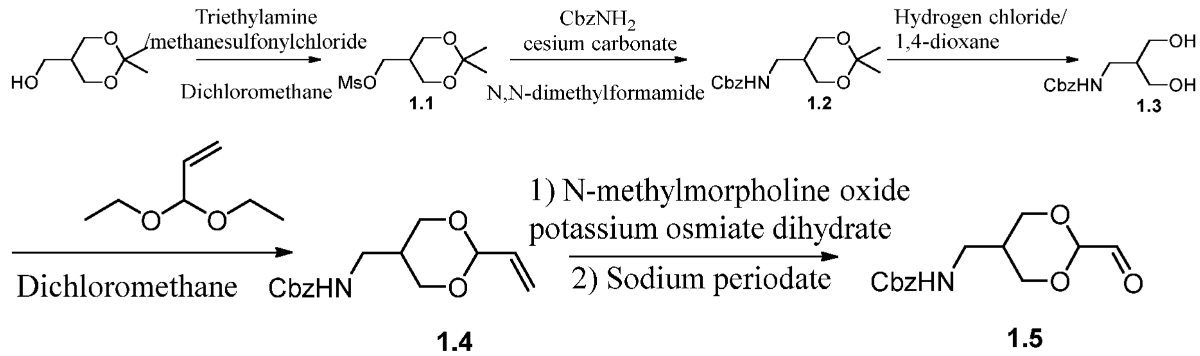


Step 1: To a solution of 5-hydroxymethyl-2,2-dimethyl-1,3-dioxane (6.78 g, 47.0 mmol) and triethylamine (7.15 g, 70.7 mmol) in dichloromethane (100 mL) was added methanesulfonyl chloride (6.51 g, 56.9 mmol) dropwise under ice bath conditions, and the reaction system was stirred at room temperature for 2 hours. The mixture was then quenched with ice water, the aqueous phase was extracted with dichloromethane, the organic phases were combined and then dried over anhydrous sodium sulfate, filtered and concentrated under reduced pressure to give compound 1.1 (10.0 g) as a yellow oil. m/z:$[M+H]^+$ 225.2.

Step 2: To a solution of compound 1.1 (1.0 g, 4.46 mmol) and benzyl carbamate (0.67 g, 4.46 mmol) in DMF (10 mL) was added cesium carbonate (2.91 g, 8.92 mmol), and the reaction system was stirred at 80°C for 16 hours. The reaction mixture was then cooled to room temperature, the reaction was quenched with ice water, the aqueous phase was extracted with ethyl acetate, the organic phases were combined and concentrated under reduced pressure, and the residue was purified by Flash column chromatography (C18, eluent 0-50% acetonitrile in a 0.05% aqueous trifluoroacetic acid solution) to give compound 1.2 (0.45 g) as a white solid. m/z:$[M+Na]^+$ 302.2.

Step 3: A solution of compound 1.2 (0.45 g, 1.61 mmol) in hydrochloric acid/1,4-dioxane (4 M, 5 mL) was stirred at room temperature for 30 minutes. The reaction mixture was then concentrated under reduced pressure and the residue was purified by Flash column chromatography (C18, eluent 0-43% acetonitrile in a 10 mM aqueous ammonium bicarbonate solution) to give compound 1.3 (0.38 g) as a white solid. m/z:$[M+H]^+$240.2.

Step 4: Concentrated hydrochloric acid (0.04 mL) was pre-added to a solution of dichloromethane (2 mL), followed by compound 1.3 (0.38 g, 1.59 mmol) and acrolein diethanol acetal (1.03 g, 7.95 mmol), the reaction system was stirred at room temperature for 2 hours and then concentrated under reduced pressure, and the residue was purified by Flash column chromatography (C18, eluent 0%-51% acetonitrile in a 0.05% aqueous trifluoroacetic acid solution) to give compound 1.4 (316 mg) as a white solid. m/z:$[M+Na]^+$300.2.

Step 5: To a mixed solution of compound 1.4 (316 mg, 1.14 mmol) in tetrahydrofuran (9 mL) and water (3 mL) were added N-methylmorpholine oxide (401 mg, 3.42 mmol) and potassium osmiate dihydrate (21 mg, 0.06 mmol) and the reaction system was stirred at 35°C for 16 hours. Sodium periodate (1.46 g, 6.84 mmol) was added to the above reaction system and the resulting mixture was stirred at 35°C for 45 minutes. The reaction was quenched by adding water, the aqueous phase was extracted with ethyl acetate, the organic phases were combined and concentrated under reduced

pressure, and the residue was purified by Flash column chromatography (C18, eluent 0-50% acetonitrile in a 10 mM aqueous ammonium bicarbonate solution) to give compound 1.5 (154 mg) as a white solid. m/z:$[M+H]^+$280.1.

Synthesis of Intermediate 1.6: benzyl(2-((2-(methylsulfonyl)ethyl)amino)ethyl)carbamate

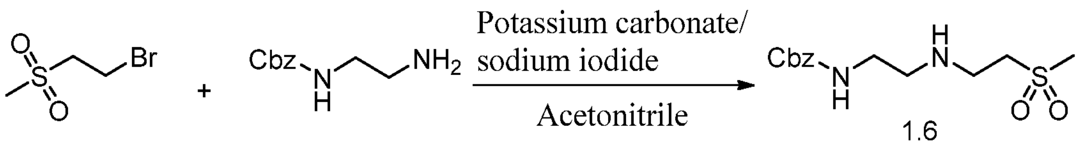


1.6

To a solution of 1-bromo-2-(methylsulfonyl)ethane (500 mg, 2.67 mmol) and benzyl(2-aminoethyl)carbamate in acetonitrile (6 mL) were added potassium carbonate (406 mg, 2.94 mmol) and sodium iodide (200 mg, 1.33 mmol) sequentially. The reaction system was stirred in a sealed tube at 90°C for 48 hours, cooled to room temperature and filtered, and the filtrate was concentrated under reduced pressure and then purified by Flash column chromatography (dichloromethane/methanol = 10/1) to give an intermediate 1.6 (600 mg) as a colorless oil. m/z: $[M+H]^+$301.2.

Synthesis of Intermediate 1.9: 7-(2-(methylsulfonyl)ethyl)-3,8-dioxo-1-phenyl-2,9-dioxa-4,7-diazaundecan-11-oic acid

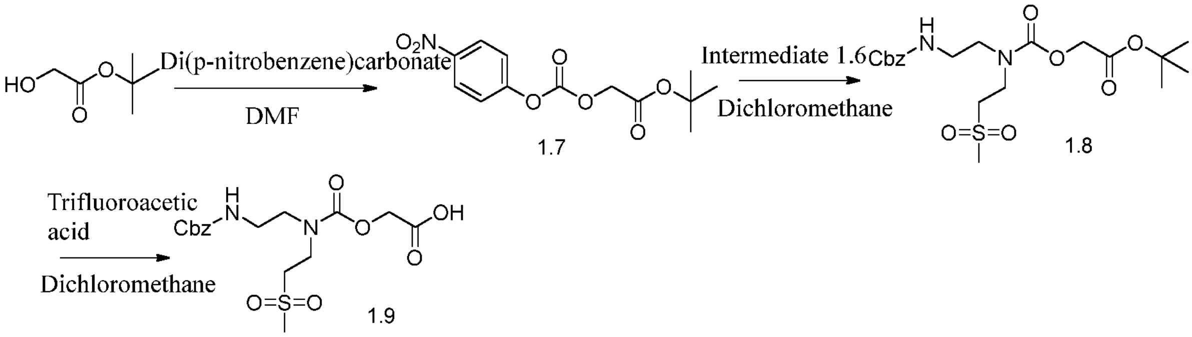


1.7 1.8 1.9

Step 1: To a solution of tert-butyl 2-hydroxyacetate (300 mg, 2.27 mmol) and di(p-nitrobenzene)carbonate (760 mg, 2.5 mmol) in DMF (5 mL) was added DIPEA (440 mg, 3.41 mmol). The reaction solution was stirred at room temperature for 4 hours and then concentrated directly under reduced pressure, and the residue was purified by Flash column chromatography (C18, eluent 10-80% acetonitrile in a 0.1% aqueous trifluoroacetic acid solution) to give intermediate 1.7 (500 mg) as a white solid. m/z:$[M+Na]^+$320.2.

Step 2: To a solution of intermediates 1.6 (300 mg, 1 mmol) and 1.7 (300 mg, 1 mmol) in dichloromethane (3 mL) was added DIPEA (258 mg, 2 mmol). The reaction solution was stirred at room temperature overnight and then concentrated directly under reduced pressure, and the residue was purified by Flash column chromatography (C18, eluent 10-70% acetonitrile in a 0.05% aqueous trifluoroacetic acid solution) to give intermediate 1.8 (400 mg) as a colorless oil. m/z:$[M+H]^+$482.2.

Step 3: To a solution of intermediate 1.8 (200 mg, 0.44 mmol) in dichloromethane (4 mL) was added trifluoroacetic acid (1 mL). The reaction solution was stirred at room temperature for 1 hour and then concentrated directly under reduced pressure, and the residue was purified by Flash column chromatography (C18, eluent 10-50% acetonitrile in a 0.1% aqueous trifluoroacetic acid solution) to give intermediate 1.9 (100 mg) as a colorless oil. m/z:$[M+H]^+$403.2.

Synthesis of **Intermediate 2.6:** 8-bromo-2-((tert-butoxycarbonyl)amino)-3H-benzo[b]azepine-4-carboxylic acid

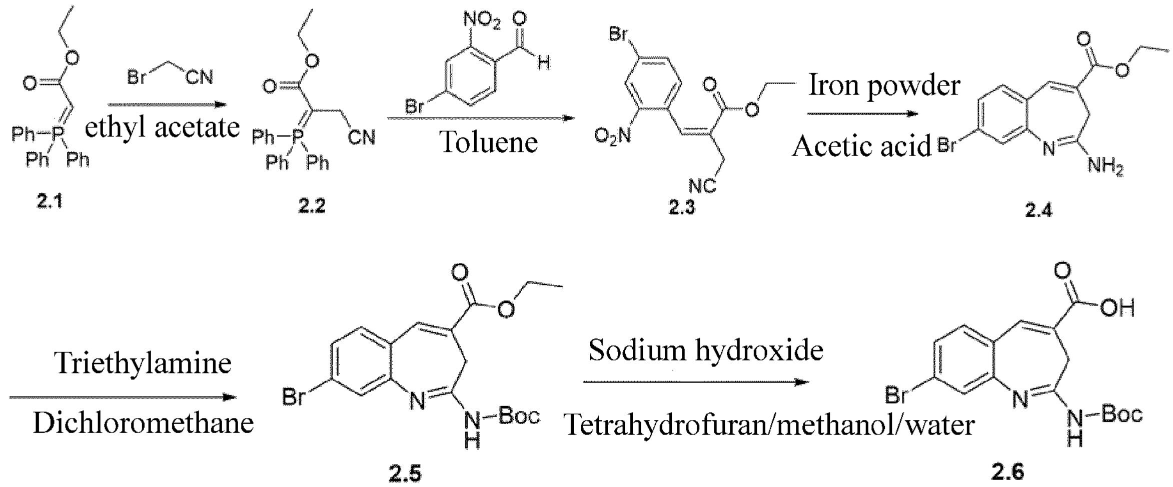


2.1 2.2 2.3 2.4 2.5 2.6

Step 1: A solution of intermediate 2.1 (20 g, 57.4 mmol) and bromoacetonitrile (6.9 g, 57.4 mmol) in ethyl acetate (200 mL) was stirred at reflux for 3 hours, the solids were removed by filtration, and the filter cake was washed with ethyl acetate. The filtrate was concentrated under reduced pressure to give intermediate 2.2 (17 g) as a light yellow oil.

Step 2: A solution of 4-bromo-2-nitrobenzaldehyde (10 g, 43.9 mmol) and intermediate 2.2 (17 g, 43.9 mmol) in toluene (170 mL) was stirred at reflux for 2 hours, the mixture was cooled to room temperature and then filtered through a short column of silica gel, eluted with 25% ethyl acetate in petroleum ether and concentrated under reduced pressure to remove most of the eluate, and the remaining solution was placed at -18°C for 16 hours. A solid precipitated, and after filtration and vacuum drying of the filter cake, intermediate 2.3 (8.2 g) was obtained as an off-white solid.

Step 3: After a solution of intermediate 2.3 (4.2 g, 12.4 mmol) in acetic acid (80 mL) was heated to 80°C, iron powder (4.1 g, 74.3 mmol) was added portionwise to the above solution within 15 minutes, the internal temperature was kept no more than 90°C, and after the addition was completed, stirring was continued for 3 hours. The reaction system was cooled to room temperature and filtered through diatomaceous earth, and the filter cake was rinsed with ethyl acetate. The filtrate was concentrated under reduced pressure, the residue was diluted with cold water and then adjusted to pH > 8 with a saturated aqueous sodium bicarbonate solution, the aqueous phase was extracted with ethyl acetate, the organic phases were combined and washed with saturated brine, the organic phases were separated and dried over anhydrous sodium sulfate and filtered, the filtrate was concentrated under reduced pressure, the residue was triturated with a 10% ethyl acetate petroleum ether solution and filtered, and after vacuum drying of the filter cake, intermediate 2.4 (3 g) was obtained as an off-white solid.

Step 4: To a solution of intermediate 2.4 (3 g, 9.7 mmol) and triethylamine (1.47 g, 14.6 mmol) in dichloromethane (50 mL) was added $(BOC)_2O$ (3.2 g, 14.6 mmol). The reaction system was stirred at room temperature for 2 days and then diluted with dichloromethane, the organic phases were washed with hydrochloric acid (3 M), a saturated aqueous sodium bicarbonate solution and saturated brine, the organic phases were separated and dried over anhydrous sodium sulfate and filtered, the filtrate was concentrated under reduced pressure, the residue was triturated with a 10% ethyl acetate petroleum ether solution and filtered, and after drying of the filter cake, intermediate 2.5 (1.6 g) was obtained as an off-white solid.

Step 5: To a solution of intermediate 2.5 (1.6 g, 3.91 mmol) in tetrahydrofuran (50 mL) was added aqueous sodium hydroxide solution (1.0 M, 5.9 mL) under ice bath conditions. The reaction system was stirred at room temperature for 16 hours and then adjusted to pH = 6 with hydrochloric acid (0.5 M), the aqueous phase was extracted with ethyl acetate, the organic phases were combined and washed with saturated brine, the organic phases were separated and dried over anhydrous sodium sulfate and filtered, and the filtrate was concentrated under reduced pressure to give intermediate 2.6 (1.1 g) as a light yellow foamy solid.

Synthesis of **Intermediate** 3.1: 8-bromo-2-((tert-butoxycarbonyl)amino)-6-fluoro-3H-benzo[b]azepine-4-carboxylic acid

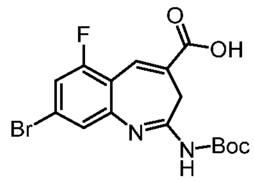

**3.1**

Using the synthesis method of intermediate 2.6, intermediate 3.1 was obtained by replacing 4-bromo-2-nitrobenzaldehyde in step 2 with 4-bromo-2-fluoro-6-nitrobenzaldehyde.

Synthesis of Intermediate 3.2: 2-amino-8-bromo-6-fluoro-3H-benzo[b]azepine-4-carboxylic acid

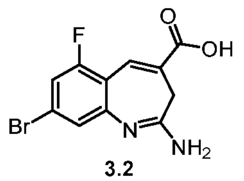

**3.2**

Using the synthesis method in step 5 of intermediate 2.6, intermediate 3.2 was obtained by reaction with ethyl 2-amino-8-bromo-6-fluoro-3H-benzo[b]azepine-4-carboxylate.

Synthesis of **Intermediate 4.1:** 2-amino-8-bromo-3H-benzo[b]azepine-4-carboxylic acid

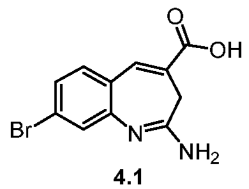

**4.1**

Using the synthesis method in step 5 of intermediate 2.6, intermediate 4.1 was obtained by reaction with intermediate 2.4.

Synthesis of **L-1:** tert-butyl(32-(3-amino-7,8-dihydro-1,6-naphthyridin-6(5H)-yl)-3,6,9,12,15,1

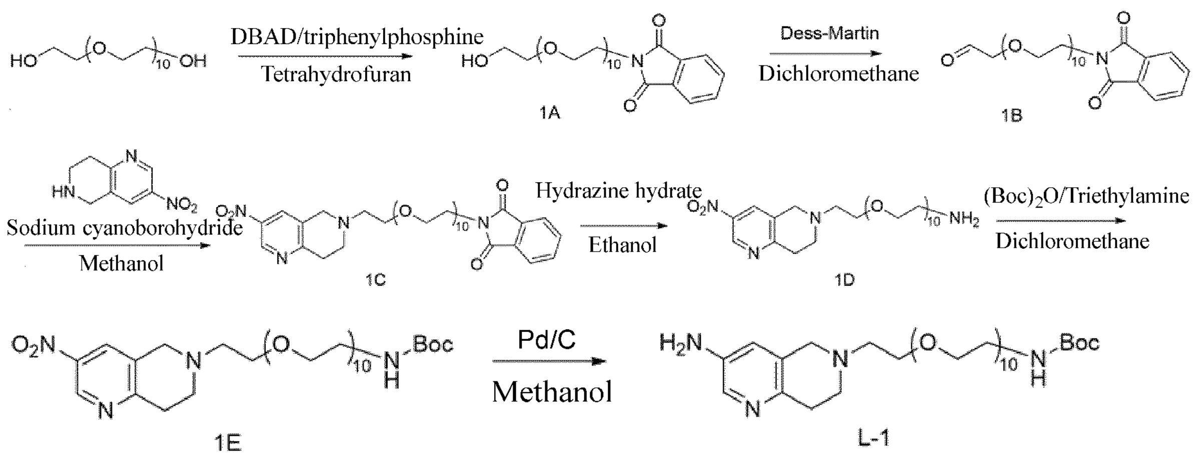

Step 1: To a solution of 3,6,9,12,15,18,21,24,27,30-decaoxadotriacontane-1,32-diol (4.8 g, 9.56 mmol) in tetrahydrofuran (50 mL) were added triphenylphosphine (3.76 g, 14.3 mmol) and DBAD (3.30 g, 14.3 mmol) sequentially, and then a solution of phthalimide in tetrahydrofuran (0.98 g, 6.69 mmol) was slowly added dropwise, the reaction solution was stirred at room temperature overnight and then concentrated under reduced pressure, and the residue was directly purified by Flash column chromatography (C18, eluent 0%-40% acetonitrile in a 10 mM aqueous ammonium bicarbonate solution) to give intermediate 1A (1.5 g) as a light yellow oil. m/z: $[M+NH_4]^+$ 649.2.

Step 2: To a solution of intermediate 1A (1.3 g, 2.06 mmol) in dichloromethane (20 mL) was added Dess-Martin oxidant (1.05 g, 2.47 mmol), the reaction solution was stirred at room temperature for 2 hours, the insoluble matter was removed by filtration, the filtrate was concentrated under reduced pressure and then dissolved in ethyl acetate, the insoluble matter was removed by filtration again, and the filtrate was concentrated under reduced pressure to give intermediate 1B (1.5 g, crude). m/z: $[M+H]^+$ 630.3.

Step 3: To a solution of 3-nitro-5,6,7,8-tetrahydro-1,6-naphthyridine (120 mg, 0.67 mmol) in methanol (5 mL) were added intermediate 1B (421 mg, 0.67 mmol) and sodium cyanoborohydride (126 mg, 2.01 mmol), the reaction system was stirred at room temperature for 3 hours, and the reaction solution was directly purified by Flash column chromatography (C18, eluent 0%-30% acetonitrile in a 10 mM aqueous ammonium bicarbonate solution) to give intermediate 1C (300 mg) as a yellow oil. m/z: $[M+H]^+$ 793.4.

Step 4: To a solution of intermediate 1C (250 mg, 0.32 mmol) in ethanol (5 mL) was added hydrazine hydrate (48 mg, 0.96 mmol), and the reaction system was stirred at 80°C for 2 hours and concentrated under reduced pressure to give intermediate 1D (210 mg, crude). m/z: $[M+H]^+$ 663.4.

Step 5: A mixture of intermediate 1D (210 mg, 0.32 mmol), triethylamine (32 mg, 0.32 mmol), $(Boc)_2O$ (70 mg, 0.32 mmol) and dichloromethane (10 mL) was stirred at room temperature for 2 hours, the reaction system was concentrated directly under reduced pressure, and the residue was purified by Flash column chromatography (C18, eluent 0-40% acetonitrile in a 10 mM aqueous ammonium bicarbonate solution) to give intermediate 1E (290 mg) as a yellow oil. m/z: $[M+H]^+$ 763.4.

Step 6: A solution of intermediate 1E (140 mg, 0.18 mmol) and palladium on carbon (19 mg, 10%wt) in methanol (5 mL) was replaced three times with hydrogen and stirred overnight at room temperature under hydrogen atmosphere, and the reaction solution was filtered and then concentrated under reduced pressure to give L-1 (110 mg) as a light yellow solid. m/z: $[M+H]^+$ 733.4.

Synthesis of **L-2:** 1-(2,5-dioxo-2,5-dihydro-1H-pyrrol-1-yl)-3,6,9,12,15,18,21,24,27,30-decaoxatritriacontan-33-oic acid

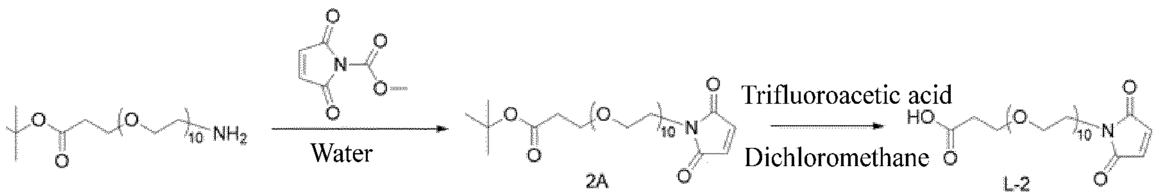


Step 1: To a solution of tert-butyl 1-amino-3,6,9,12,15,18,21,24,27,30-decaoxatritriacontan-33-oate (180 mg, 0.31 mmol) in water (5 mL) were added methyl 2,5-dioxa-2,5-dihydro-1H-pyrrole-1-carboxylate (57 mg, 0.37 mmol) and sodium bicarbonate (52 mg, 0.62 mmol) under ice bath conditions. The reaction system was stirred at room temperature for 2 hours, and the residue was purified by Flash column chromatography (C18, eluent 0%-70% acetonitrile in a 10 mM aqueous ammonium bicarbonate solution) to give intermediate 2A (170 mg) as a colorless oil. m/z: $[M+NH_4]^+$ 683.2.

Step 2: To a solution of intermediate 2A (160 mg, 0.23 mmol) in dichloromethane (3 mL) was added trifluoroacetic acid (1 mL) under ice bath conditions. The reaction solution was stirred at room temperature for 1 hour and concentrated under reduced pressure, and the residue was purified by Flash column chromatography (C18, eluent 0-50% acetonitrile in a 10 mM aqueous ammonium bicarbonate solution) to give L-2 (110 mg) as a colorless oil. m/z: $[M+NH_4]^+$ 610.4.

Synthesis of L-3: (12S,15S)-22-(2,5-dioxo-2,5-dihydro-1H-pyrrol-1-yl)-15-isopropyl-11,14,17-trioxo-12-(3-ureidopropyl)-4,7-dioxa-10,13,16-triazadocosan-1-oic acid

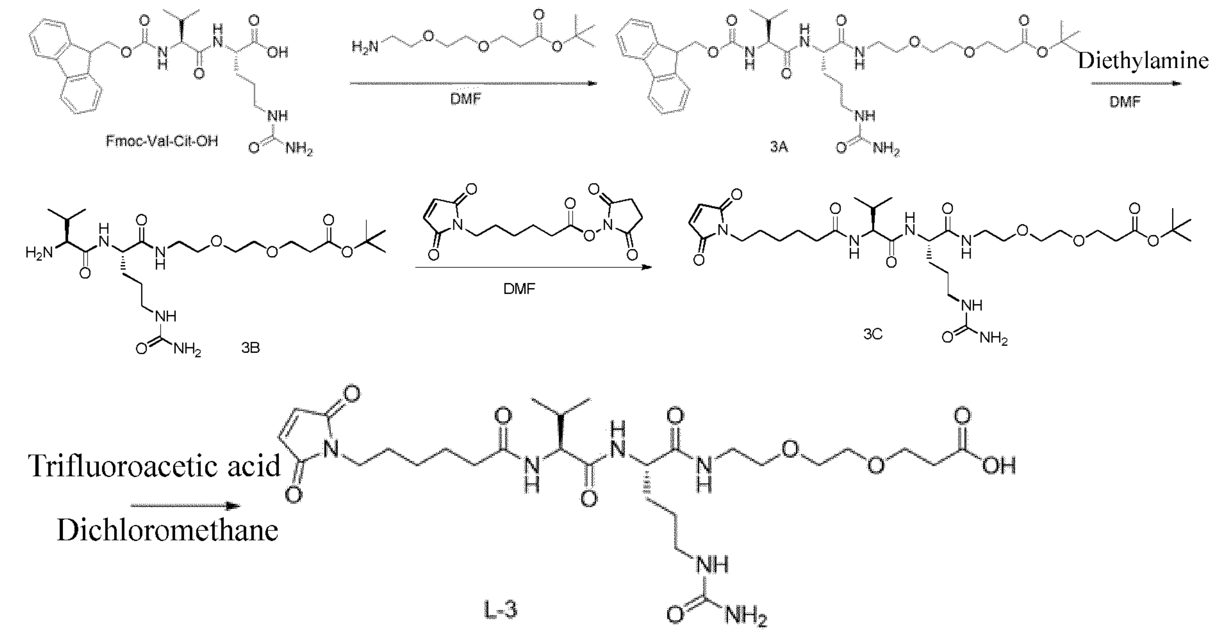

Fmoc-Val-Cit-OH
3A
3B
3C
L-3

Steps 1 & 2: To a solution of Fmoc-Val-Cit-OH (300 mg, 0.60 mmol) in DMF (5 mL) were added HATU (251 mg, 0.66 mmol), DIPEA (116 mg, 0.9 mmol) and tert-butyl 3-(2-(2-aminoethoxy)ethoxy)propionate (154 mg, 0.66 mmol) each. The reaction system was stirred at room temperature for 1 hour. Diethylamine (1 mL) was added to the above reaction system and the resulting mixture was continued to be stirred at room temperature for 1 hour. The reaction solution was directly purified by Flash column chromatography (C18, eluent 0%-35% acetonitrile in a 0.1% aqueous trifluoroacetic acid solution) to give intermediate 3B (336 mg) as a yellow oil. m/z:$[M+H]^+$ 490.3.

Step 3: To a solution of intermediate 3B (336 mg, 0.56 mmol) and EMCS (190 mg, 0.62 mmol) in DMF (4 mL) was added DIEPA (181 mg, 1.4 mmol). The reaction system was stirred at room temperature for 2 hours. The reaction solution was directly purified by Flash column chromatography (C18, eluent 0%-48% acetonitrile in a 0.1% aqueous trifluoroacetic acid solution) to give intermediate 3C (297 mg) as a white solid. m/z:$[M+H]^+$ 683.4.

Step 4: To a solution of intermediate 3C (297 mg, 0.43 mmol) in dichloromethane (3 mL) was added trifluoroacetic acid (1 mL) dropwise under ice bath conditions. The reaction system was stirred at room temperature for 1 hour. The reaction solution was concentrated under reduced pressure and then purified by Flash column chromatography (C18, eluent 0%-38% acetonitrile in a 10 mM aqueous ammonium bicarbonate solution) to give L-3 (220 mg) as a white solid. m/z:$[M+H]^+$ 627.4.

Synthesis of L-4: (24S,27S)-34-(2,5-dioxo-2,5-dihydro-1H-pyrrol-1-yl)-27-isopropyl-23,26,29-trioxo-24-(3-ureidopropyl)-4,7,10,13,16,19-hexaoxa-22,25,28-triazatetratriacontan-1-oic acid

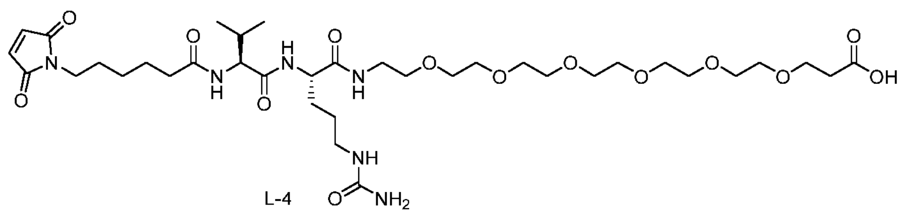
L-4

Using the synthesis method of L-3, L-4 was synthesized. m/z:$[M+H]^+$ 803.4.

Synthesis of L-5: 4-((2S,5S)-31-(2,5-dioxo-2,5-dihydro-1H-pyrrol-1-yl)-5-isopropyl-4,7,29-trioxo-2-(3-ureidopropyl)-10,13,16,19,22,25-hexaoxa-3,6,28-triazahentriacontanylamino)benzyl(4-nitrophenyl)carbonate

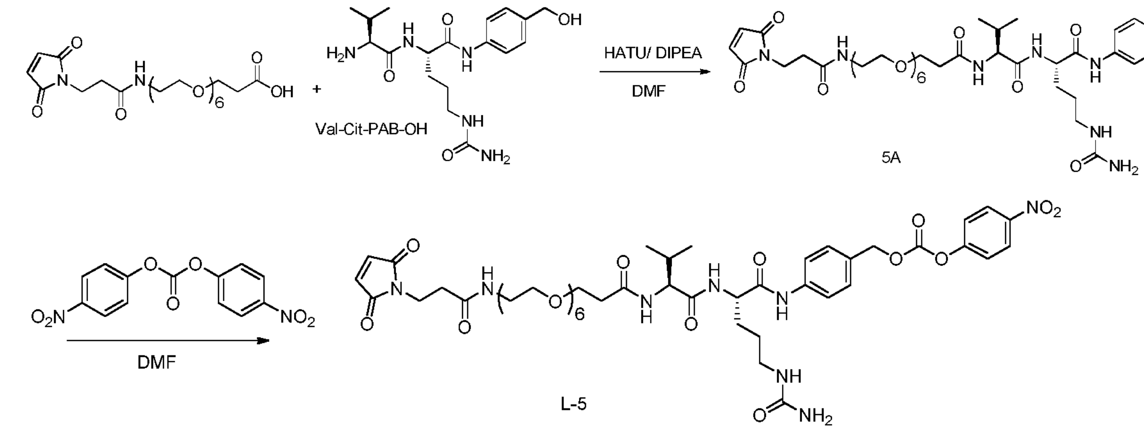


Val-Cit-PAB-OH

5A

L-5

Step 1: To a solution of 1-(2,5-dioxo-2,5-dihydro-1H-pyrrol-1-yl)-3-oxo-7,10,13,16,19,22-hexaoxa-4-azapentacosan-25-oic acid (120 mg, 0.24 mmol) and Val-Cit-PAB-OH (109 mg, 0.29 mmol) in DMF (3 mL) were added HATU (110 mg, 0.29 mmol) and DIPEA (93 mg, 0.72 mmol), respectively. The reaction system was stirred at room temperature for 2 hours. The reaction solution was directly purified by Flash column chromatography (C18, eluent 0%-60% acetonitrile in a 0.1% aqueous trifluoroacetic acid solution) to give intermediate 5A (110 mg) as a yellow solid. m/z: $[M+H]^+$ 866.4.

Step 2: To a solution of intermediate 5A (110 mg, 0.13 mmol) in DMF (3 mL) were added bis(4-nitrobenzene) carbonate (59mg, 0.2 mmol) and DIPEA (42 mg, 0.33 mmol). The reaction solution was stirred at room temperature for 2 hours and directly purified by Flash column chromatography (C18, eluent 0%-60% acetonitrile in a 10 mM aqueous ammonium bicarbonate solution) to give L-5 (60 mg) as a light yellow solid. m/z: $[1/2M+H]^+$ 516.3.

Synthesis of L-6: 1-(4-((2,5-dioxo-2,5-dihydro-1H-pyrrol-1-yl)methyl)cyclohexyl)-1-oxo-5,8,11,14,17,20-hexaoxa-2-azatricosan-23-oic acid

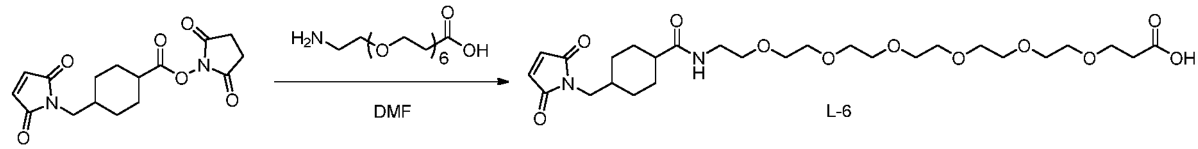


L-6

A solution of 2,5-dioxopyrrolidin-1-yl 4-((2,5-dioxo-2,5-dihydro-1H-pyrrol-1-yl)methyl)cyclohexanecarboxylate (100 mg, 0.3 mmol) and 1-amino-3,6,9,12,15,18-hexaoxaheneicosan-21-oic acid (106 mg, 0.3 mmol) in DMF (3 mL) was stirred at room temperature for 1 hour and then directly purified by Flash column chromatography (C18, eluent 0%-50% acetonitrile in a 0.1% aqueous trifluoroacetic acid solution) to give L-6 (75 mg) as a white solid. m/z: $[M+H]^+$ 573.2.

Synthesis of L-7: 3-(2-(2-(4-((2,5-dioxo-2,5-dihydro-1H-pyrrol-1-yl)methyl)cyclohexane-1-amido)ethoxy)ethoxy)propionic acid

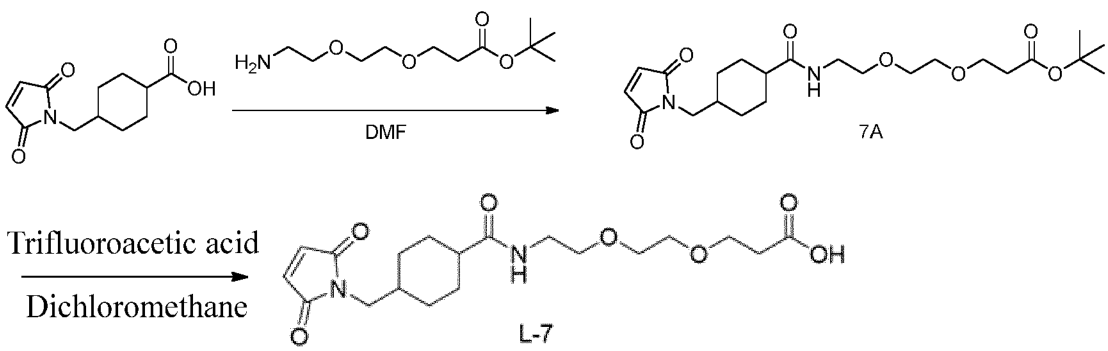


7A

L-7

Step 1: To a solution of 4-((2,5-dioxo-2,5-dihydro-1H-pyrrol-1-yl)methyl)cyclohexane-1-carboxylic acid (237 mg, 1 mmol) in DMF (3 mL) were added HATU (456 mg, 1.2 mmol), DIPEA(258 mg, 2 mmol) and tert-butyl 3-(2-(2-aminoethoxy)ethoxy)propionate (256 mg, 1.1 mmol) sequentially. The reaction solution was stirred at room temperature for 2 hours and then directly purified by Flash column chromatography (C18, eluent 0%-80% acetonitrile in a 0.1% aqueous trifluoroacetic acid solution) to give intermediate 7A (130 mg) as a white solid. m/z: $[M+H]^+$ 454.2.

Step 2: To a solution of intermediate 7A (100 mg, 0.22 mmol) in dichloromethane (2 mL) was added trifluoroacetic acid (1 mL) dropwise under ice bath conditions. The reaction system was stirred at room temperature for 1 hour. The reaction solution was concentrated under reduced pressure and then purified by Flash column chromatography (C18, eluent 0%-50% acetonitrile in a 0.1% aqueous trifluoroacetic acid solution) to give L-7 (220 mg) as a white solid. m/z:$[M+H]^+$ 397.2.

Synthesis of **L-8:** 3-(2-(2-(2-(2,5-dioxo-2,5-dihydro-1H-pyrrol-1-yl)acetylamino)ethoxy)ethoxy)propionic acid

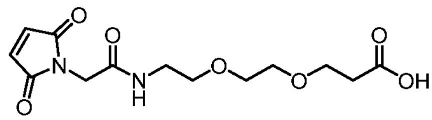

L-8

Using the synthesis method of L-7, L-8 was synthesized from 2-(2,5-dioxo-2,5-dihydro-1H-pyrrol-1-yl)acetic acid. m/z:$[M+Na]^+$ 393.2.

Synthesis of L-9: 4-(2-(2,5-dioxo-2,5-dihydro-1H-pyrrol-1-yl)acetamido)butanoic acid

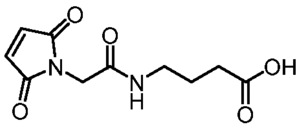

L-9

Using the synthesis method of L-7, L-9 was synthesized from 2-(2,5-dioxo-2,5-dihydro-1H-pyrrol-1-yl)acetic acid and tert-butyl 4-aminobutyrate. m/z:$[M+Na]^+$ 241.0.

Synthesis of L-10: 1-(2,5-dioxo-2,5-dihydro-1H-pyrrol-1-yl)-2,7-dioxo-11,14-dioxa-3,8-diazaheptadecan-17-oic acid

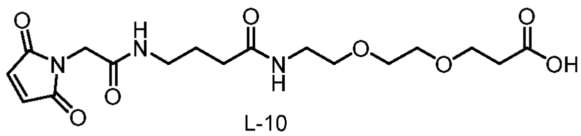

L-10

Using the synthesis method of L-7, L-10 was synthesized from L-9. m/z: $[M+H]^+$ 400.2.

Synthesis of **L-11:** (1-(2,5-dioxo-2,5-dihydro-1H-pyrrol-1-yl)-2,7-dioxo-11,14-dioxa-3,8-diazaheptadecyl-17-acyl)glycine

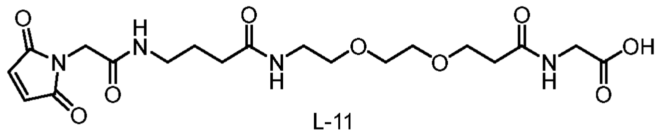

L-11

Using the synthesis method of L-7, L-11 was synthesized from L-10 and tert-butyl glycine ester. m/z:$[M+H]^+$ 457.2.

Synthesis of **L-12:** 3-(2-(2-(4-((2,5-dioxo-2,5-dihydro-1H-pyrrol-1-yl)methyl)benzoylamino)ethoxy)ethoxy)propionic acid

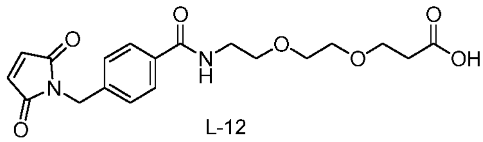

L-12

Using the synthesis method of L-7, L-12 was synthesized from 4-((2,5-dioxo-2,5-dihydro-1H-pyrrol-1-yl)methyl) benzoic acid. m/z: $[M+Na]^+$ 391.0.

Synthesis of **L-13:** 1-(4-((2,5-dioxo-2,5-dihydro-1H-pyrrol-1-yl)methyl)phenyl)-1-oxo-5,8,11,14,17,20-hexaoxa-2-aza-tricosan-23-oic acid

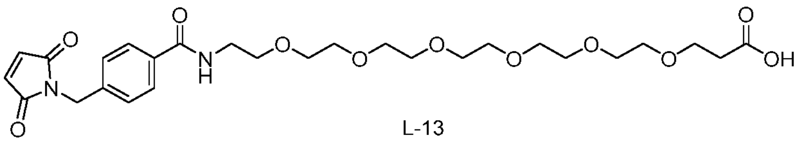

L-13

Using the synthesis method of L-7, L-13 was synthesized from 4-((2,5-dioxo-2,5-dihydro-1H-pyrrol-1-yl)methyl) benzoic acid. m/z: $[M+H]^+$ 567.2.

Synthesis of L-14: 4-(3-(2-(2-(4-((2,5-dioxo-2,5-dihydro-1H-pyrrol-1-yl)methyl)propanoylamino)ethoxy)ethoxy)propanoylamino)benzyl(4-nitrophenyl)carbonate

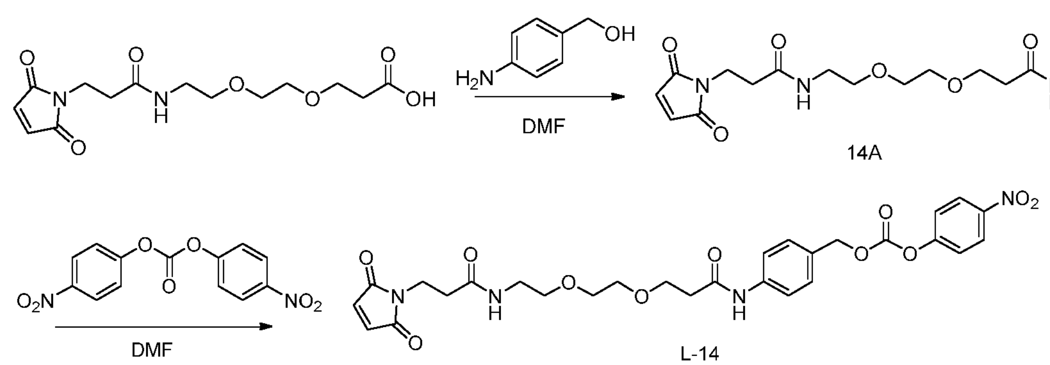


14A

L-14

Step 1: To a solution of 3-(2-(2-(3-(2,5-dioxo-2,5-dihydro-1H-pyrrol-1-yl)propanoylamino)ethoxy)ethoxy)propionic acid (100 mg, 0.3 mmol) in DMF (3 mL) were added HATU (140 mg, 0.36 mmol) and DIPEA (78 mg, 0.6 mmol) each. The reaction solution was stirred at room temperature for 5 minutes and then (4-aminophenyl)methanol (44 mg, 0.36 mmol) was added. The reaction solution was stirred for 1 hour and directly purified by Flash column chromatography (C18, eluent 0%-60% acetonitrile in a 0.1% aqueous trifluoroacetic acid solution) to give intermediate 14A (97 mg) as a yellow solid. m/z: $[M+Na]^+$ 456.2.

Step 2: Using the synthesis method in step 2 of L-5 , L-14 (92 mg) was obtained as a white solid by reaction with 14A. m/z: $[M+H]^+$ 599.2.

Synthesis of **L-15:** 32-(3-(2,5-dioxo-2,5-dihydro-1H-pyrrol-1-yl)propionylamino)-26,33-dioxo-2,5,8,11,14,17,20,23-octaoxa-27, 34-diazeptadecane-37-acid

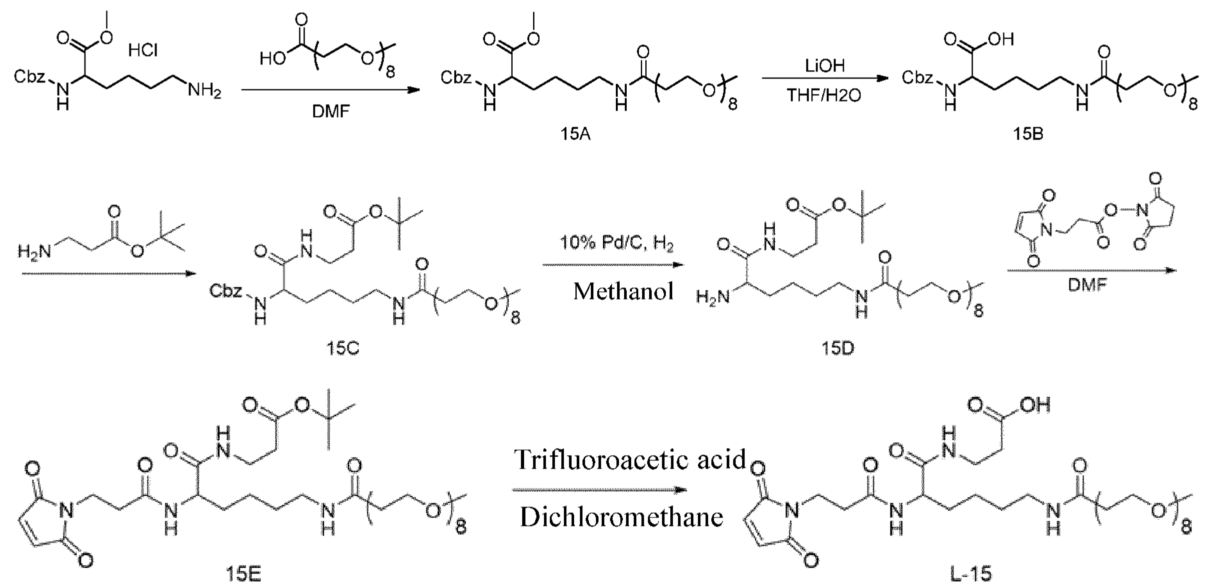


Step 1: To a solution of methyl 6-amino-2-(((benzyloxy)carbonyl)amino)hexanoate hydrochloride (900 mg, 2.18 mmol) and 2,5,8,11,14,17,20,23-octaoxaehexane-26-acid (793 mg, 2.4 mmol) in DMF (3 mL) was added DIPEA (1.1 g, 8.72 mmol). The reaction solution was stirred at room temperature for 4 hours and concentrated under reduced pressure, and the residue was purified by Flash column chromatography (C18, eluent 0%-70% acetonitrile in a 0.1% aqueous trifluoroacetic acid solution) to give intermediate 15A (1.1 g) as a colorless oil. m/z: $[M+H]^+$ 689.0.

Step 2: To a solution of intermediate 15A (1.1 g, 1.6 mmol) in tetrahydrofuran (2.5 mL) was added dropwise a solution of lithium hydroxide monohydrate (134 mg, 3.2 mmol) in water (0.5 mL). The reaction solution was stirred at room temperature for 4 hours. The mixture was diluted with water, the aqueous phase was extracted with ethyl acetate, and the organic phases were combined and concentrated under reduced pressure to give intermediate 15B (900 mg) as a colorless liquid.

Step 3: To a solution of intermediate 15B (1 g, 1.48 mmol) and tert-butyl 3-aminopropionate (210 mg, 1.48 mmol) in DMF (2.5 mL) were added HATU (619 mg, 1.63 mmol) and DIPEA (770 mg, 5.92 mmol) sequentially. The reaction solution was stirred at room temperature for 4 hours and concentrated under reduced pressure, and the residue was purified by Flash column chromatography (C18, eluent 0%-60% acetonitrile in a 0.1% aqueous trifluoroacetic acid solution) to give intermediate 15C (1 g) as a colorless liquid. m/z: $[M+H]^+$ 802.7.

Step 4: To a solution of intermediate 15C (1 g, 1.25 mmol) in methanol (3 mL) was added Pd/C (10%wt, 100 mg), and the reaction system was replaced with hydrogen and then stirred at room temperature under hydrogen atmosphere for 4 hours. The reaction solution was filtered through diatomaceous earth, and the filtrate was concentrated under reduced pressure to give intermediate 15D (850 mg) as a colorless oil. m/z: $[M+H]^+$ 668.6.

Step 5: A solution of intermediate 15D (450 mg, 0.67 mmol) and BMPS (180 mg, 0.67 mmol) in DMF (2.5 mL) was stirred at room temperature for 4 hours and concentrated directly under reduced pressure to give intermediate 15E (350 mg) as a colorless liquid. m/z: $[M+H]^+$ 818.9.

Step 6: To intermediate 15E (350 mg, 0.43 mmol) was added trifluoroacetic acid (1 mL) and dichloromethane (15 mL). The reaction solution was stirred at room temperature for 4 hours and then concentrated directly under reduced pressure. The residue was purified by Flash column chromatography (C18, eluent 0%-55% acetonitrile in a 0.1% aqueous trifluoroacetic acid solution) to give L-15 (180 mg) as a colorless viscous liquid. m/z: $[M+H]^+$ 762.9; $^1H$ NMR (400 MHz, DMSO-$d_6$): δ 12.17 (s, 1H), 8.02 (d, *J* = 8.0Hz, 1 H), 7.89 (t, *J*= 5.6Hz, 1H), 7.74 (t, *J*= 5.2Hz, 1 H), 6.97 (s, 2H), 4.10-4.04 (m, 1H), 3.58-3.52 (m, 5H), 3.49-3.48 (m, 1H), 3.46 -3.45 (m, 7H), 3.45-3.41 (m, 4H), 3.41-3.37 (m, 2H), 3.30 (s, 13H), 3.25-3.12 (m, 5H), 2.99-2.92 (m, 2H), 2.37-2.31 (m, 4H), 2.25 (t, *J* = 6.8Hz, 2H), 1.57-1.46 (m, 1H), 1.45-1.35 (m, 1H), 1.34-1.26 (m, 2H), 1.21-1.08 (m, 2H).

Synthesis of **L-16:** 32-(3-(2,5-dioxo-2,5-dihydro-1H-pyrrol-1-yl)propionylamino)-26,33-dioxo-2,5,8,11,14,17,20,23,37,40-decaoxa-27,34-diazatritetracontan-43-oic acid

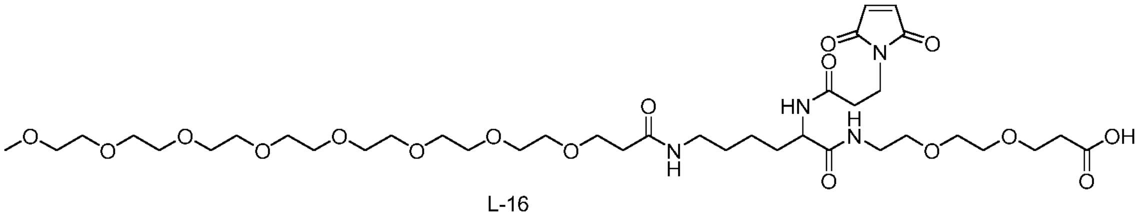

L-16

Using the synthesis method of L-15, L-16 was synthesized from tert-butyl 3-(2-(2-aminoethoxy)ethoxy)propionate and 15B. m/z: $[M+H]^+$ 850.9. $^1H$ NMR (400 MHz, DMSO-$d_6$): δ 12.12 (br. s, 1H), 8.02 (d, J= 8.4Hz, 1H), 7.87 (t, J= 5.6Hz, 1H), 7.74 (t, *J* = 5.6Hz, 1H), 6.97 (s, 2H), 4.13-4.07 (m, 1H), 3.57-3.52 (m, 7H), 3.49- 3.48 (m, 1H), 3.47-3.46 (m, 10H), 3.46-3.45 (m, 6H), 3.45-3.41 (m, 10H), 3.40-3.37 (m, 3H), 3.44 (t, *J* = 6.0Hz, 2H), 3.20 (s, 3H), 3.18- 3.11 (m, 2H), 2.99-2.92 (m, 2H), 2.41-2.34 (m, 5H), 2.25 (t, *J* = 6.4Hz, 2H), 1.57-1.48 (m, 1H), 1.45-1.36 (m, 1H), 1.34-1.26 (m, 2H), 1.22-1.07 (m, 2H).

Synthesis of L-17: 1-(2,5-dioxo-3,4-bis(pyridin-2-ylmercapto)-2,5-dihydro-1H-pyrrol-1-yl)-3-oxo-7,10,13,16,19,22-hexaoxa-4-azapentacosan-25-oic acid

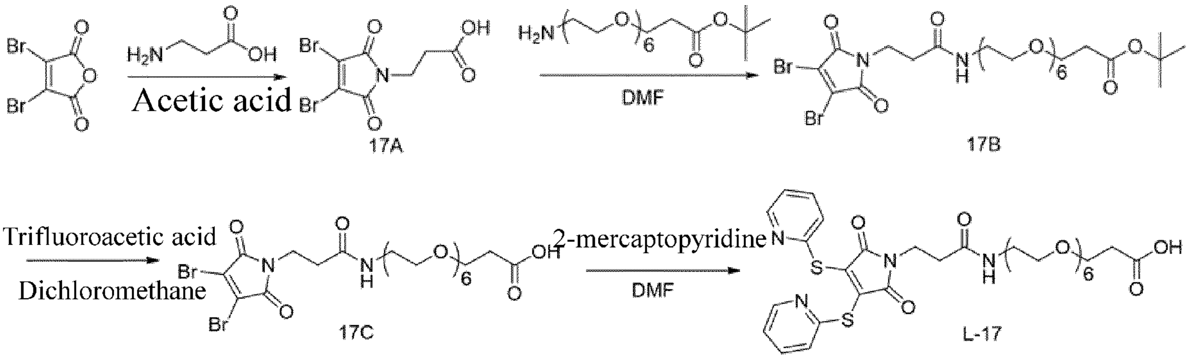


Step 1: A solution of 3,4-dibromofuran-2,5-dione (900 mg, 3.41 mmol) and 3-aminopropionic acid (320 mg, 3.41 mmol) in acetic acid (10 mL) was stirred at 100°C for 10 hours, the reaction solution was concentrated under reduced pressure, the residue was triturated with a mixed solution of petroleum ether and ethyl acetate (5/1) and filtered, and the filter cake was dried in vacuum to give intermediate 17A (1.2 g) as a light yellow solid. m/z: $[M+H]^+$ 349.8.

Step 2: To a solution of tert-butyl 1-amino-3,6,9,12,15,18-hexaoxaheneicosan-21-oate (99 mg, 0.23 mmol) in DMF (2 mL) were added HATU (107 mg, 0.28 mmol), DIPEA (45.5 mg, 0.35 mmol) and intermediate 17A (75 mg, 0.23 mmol) sequentially under ice bath conditions. The reaction solution was stirred at room temperature for 1 hour and directly purified by Flash column chromatography (C18, eluent 0%-60% acetonitrile in a 0.1% aqueous trifluoroacetic acid solution) to give intermediate 17B (126 mg) as a yellow solid. m/z: $[M+H]^+$ 717.2.

Step 3: To a solution of intermediate 17B (126 mg, 0.18 mmol) in dichloromethane (2 mL) was added trifluoroacetic acid (1 mL). The reaction was stirred at room temperature for 1 hour and concentrated under reduced pressure, and the residue was purified by Flash column chromatography (C18, eluent acetonitrile in a 0.1% aqueous trifluoroacetic acid solution) to give intermediate 17C (60 mg) as a yellow solid. m/z: $[M+H]^+$ 661.2.

Step 4: To a solution of intermediate 17C (18 mg, 0.03 mmol) in DMF (1 mL) were added 2-mercaptopyridine (63 mg, 0.06 mmol) and triethylamine (4.8 mg, 0.08 mmol) were sequentially under ice bath conditions. The reaction solution was stirred at room temperature for 1 hour and directly purified by Flash column chromatography (C18, eluent 0%-60% acetonitrile in a 0.1% aqueous trifluoroacetic acid solution) to give intermediate L-17 (16 mg) as a yellow solid. m/z: $[M+H]^+$ 723.2.

Synthesis of **L-18**: 3-(2-(2-(3-(2,5-dioxo-3,4-bis(pyridin-2-ylmercapto)-2,5-dihydro-1H-pyrrol-1-yl)propionylamino) ethoxy)ethoxy)propionic acid

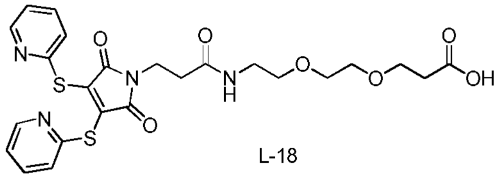

L-18

Using the synthesis method of L-17, L-18 was synthesized. m/z: $[M+H]^+$ 547.2.

Synthesis of L-19: (24S,27S)-34-(2,5-dioxo-3,4-bis(pyridin-2-ylmercapto)-2,5-dihydro-1H-pyrrol-1-yl)-27-isopropyl-23,26,29-trioxo-24-(3-ureidopropyl)-4,7,10,13,16,19-hexaoxa-22,25,28-triazatetratriacontan-1-oic acid

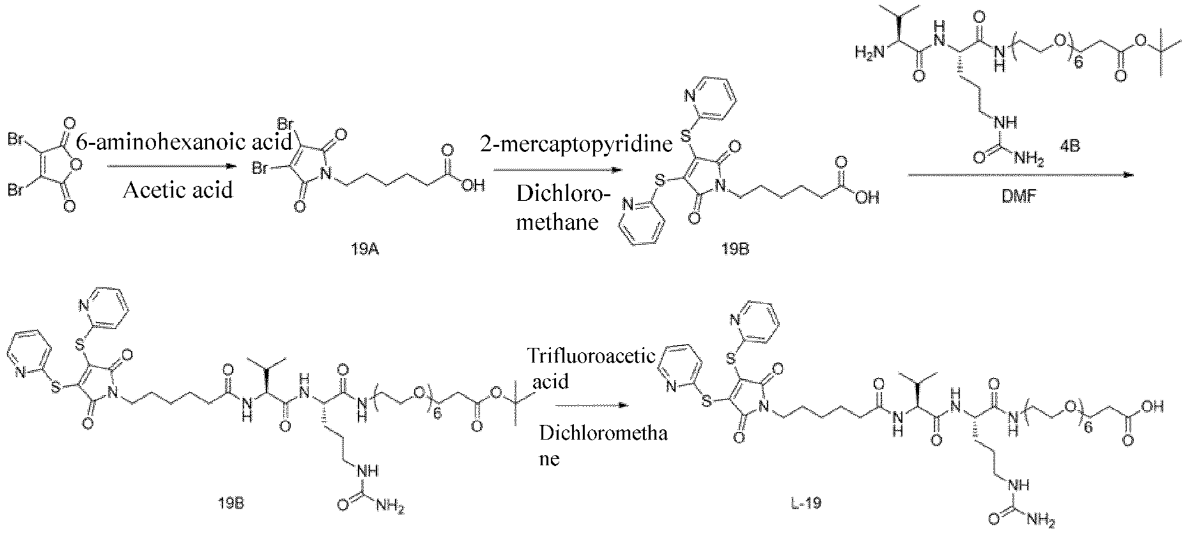


Step 1: Using the synthesis method of 17A, 19A was obtained by reaction with 3,4-dibromofuran-2,5-dione and 6-aminocaproic acid.

Step 2: To a solution of intermediate 19A (300 mg, 0.81 mmol) and 2-sulphidylpyridine (189 mg, 1.7 mmol) in dichloromethane (5 mL) was added triethylamine (180 mg, 1.78 mmol) under ice bath conditions, and the reaction solution was stirred at room temperature for 1 hour. The reaction solution was concentrated under reduced pressure at low temperature and then purified by Flash column chromatography (C18, eluent 0%-49% acetonitrile in a 0.1% aqueous trifluoroacetic acid solution) to give intermediate 19B (370 mg) as a yellow solid. m/z:$[M+H]^+$ 430.0.

Steps 3 & 4: Using the synthesis method of 17C, L-19 was obtained as a white solid by the reaction with 19B. m/z:$[1/2M+H]^+$ 511.3.

Synthesis of **L-20:** (2R,3S,4S,5R,6S)-2-(acetoxymethyl)-6-(2-(2-(6-(2,5-dioxo-2,5-dihydro-1H-pyrrol-1-yl)hexanoylamino)acetylamino)-4-((((4-nitrophenoxy)carbonyl)oxy)methyl)phenoxy)tetrahydro-2H-pyran-3,4,5-triyl triacetate

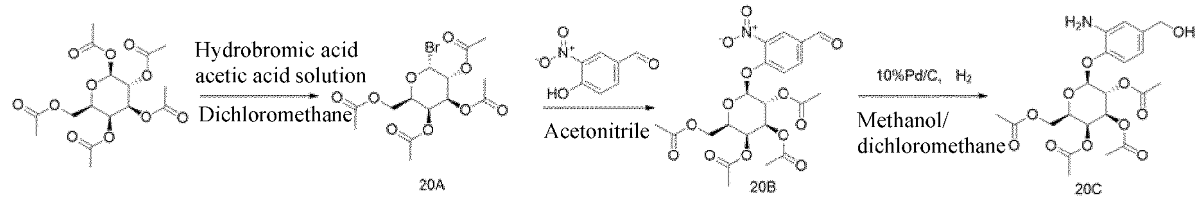


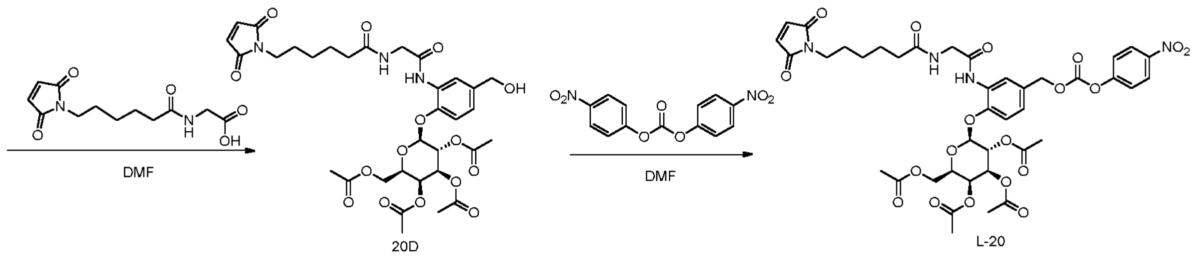


Step 1: To a solution of β-D-galactose pentaacetate (3.1 g, 7.94 mmol) in dichloromethane (30 mL) was added dropwise a hydrobromic acetic acid solution (33%, 15 mL) under ice bath conditions. The reaction solution was stirred at room temperature for 2 hours. The reaction mixture was then poured into ice water (300 mL) and extracted with ice dichloromethane extraction, the organic phases were combined and then washed with a saturated aqueous sodium bicarbonate solution, and the organic phases were separated and concentrated under reduced pressure to give intermediate 20A (3.2 g) as a white solid. m/z:$[M+Na]^+$ 433.0.

Step 2: To a solution of intermediate 20A (1 g, 2.4 mmol) and 4-hydroxy-3-nitrobenzaldehyde (0.45 g, 2.67 mmol) in acetonitrile (15 mL) was added silver oxide (2 g, 8.6 mmol). The reaction system was stirred at room temperature overnight in the dark. The reaction mixture was then filtered through diatomaceous earth, the filtrate was concentrated under reduced pressure, and the residue was purified by Flash column chromatography (dichloromethane/methanol = 20/1) to give intermediate 20B (1.10 g) as a white solid. m/z:$[M+Na]^+$ 520.2.

Step 3: Intermediate 20B (1.1 g, 2.21 mmol) was dissolved in methanol (110 mL) solution, then palladium on carbon (550 mg, 10%wt) was added, and the reaction system was replaced with hydrogen three times and stirred under hydrogen atmosphere for 3 hours. The reaction mixture was then filtered through diatomaceous earth and the filtrate was concentrated under reduced pressure to give intermediate 20C (1.02 g) as a white solid. m/z:$[M+H]^+$ 470.2.

Step 4: To a solution of 2-(6-(2,5-dioxo-2,5-dihydro-1H-pyrrol-1-yl)hexanoylamino)acetic acid (200 mg, 0.75 mmol) in DMF (3 mL) were added HATU (314 mg, 0.83 mmol), DIPEA (194 mg, 1.50 mmol) and intermediate 20C (352 mg, 0.75 mmol) each, and the reaction system was stirred at room temperature overnight. The reaction solution was directly purified by Flash column chromatography (C18, eluent 0%-37% acetonitrile in a 0.1% aqueous trifluoroacetic acid solution) to give intermediate 20D (160 mg) as a white solid. m/z: $[M+Na]^+$ 742.2.

Step 5: To a solution of intermediate 20D (160 mg, 0.22 mmol) in DMF (3 mL) were added bis(p-nitrobenzene) carbonate (134 mg, 0.44 mmol) and DIPEA (85.3 mg, 0.66 mmol) and the reaction system was stirred at room temperature for 2 hours. The reaction solution was directly purified by Flash column chromatography (C18, eluent 0%-58% acetonitrile in a 10 mM aqueous ammonium bicarbonate solution) to give L-20 (130 mg) as a white solid. m/z:$[M+Na]^+$ 907.2.

Synthesis of **L-21:** (2S,3R,4S,5S,6S)-2-(2-(2-((tert-butoxycarbonyl)amino)acetylamino)-4-((((4-nitrophenoxy)carbonyl) oxy)methyl)phenoxy)-6-(methoxycarbonyl)tetrahydro-2H-pyran-3,4,5-triyl triacetate

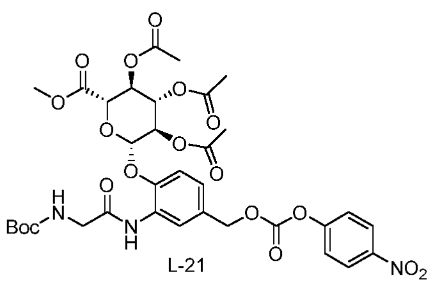

L-21

Using the synthesis method of intermediate L-20, L-21 was synthesized by replacing 2-(6-(2,5-dioxo-2,5-dihydro-1H-pyrrol-1-yl)hexanoylamino)acetic acid in step 4 with N-tert-butoxycarbonylglycine with methyl 1,2,3,4-tetra-O-acetyl-β-D-glucuronate as the starting material. m/z: $[M+H]^+$ 778.2.

Synthesis of **L-22:** 1-(2,5-dioxo-2,5-dihydro-1H-pyrrol-1-yl)-2-oxo-6,9,12,15,18,21-hexaoxa-3-azatetracosan-24-oic acid

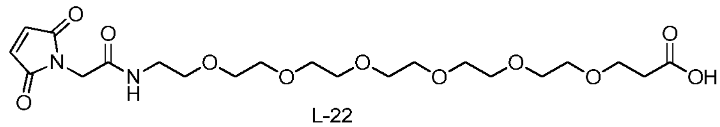

L-22

Using the synthesis method of L-7, L-22 was synthesized from 2-(2,5-dioxo-2,5-dihydro-1H-pyrrol-1-yl)acetic acid. m/z: $[M+H]^+$ 491.2.

Synthesis of L-23: 4-((S)-2-((S)-2-(6-(2,5-dioxo-2,5-dihydro-1H-pyrrol-1-yl)hexanoylamino)-3-methylbutanoylamino)-5-ureidopentanoylamino)benzyl(4-nitrobenzene)carbonate

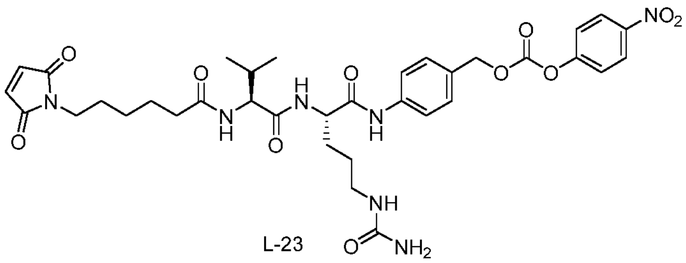

L-23

Using the synthesis method in L-14 step 2, L-23 was synthesized using Mc-Val-Cit-PAB-OH. m/z: $[M+H]^+$738.1.

Synthesis of **L-24:** (E)-4,7,11-trioxo-3,15,18,21,24,27,30-heptaoxa-8,12-diazatritriacont-5-ene-33-oic acid

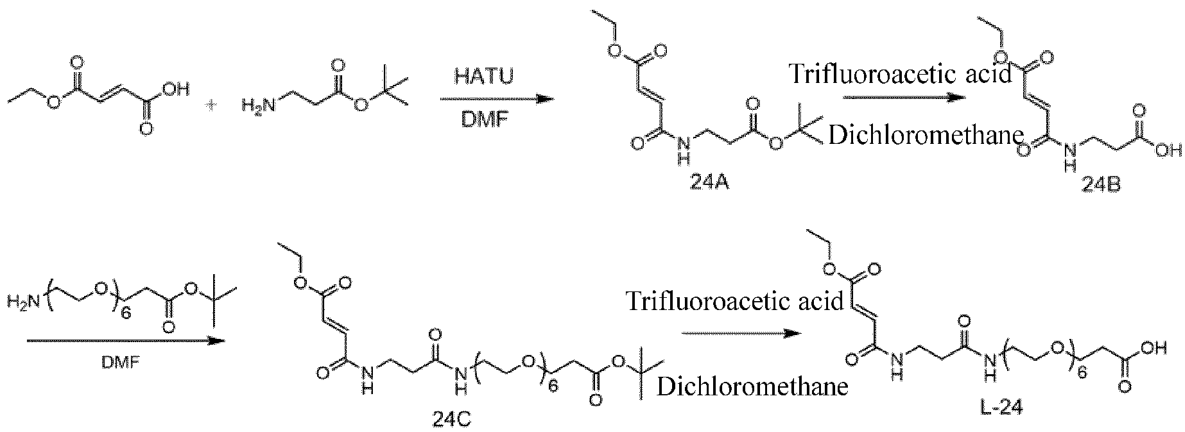


Step 1: To a solution of monoethyl fumarate (900 mg, 6.24 mmol) and tert-butyl 3-aminopropionate (906 mg, 6.24 mmol) in DMF (10 mL) were added HATU (3.6 g, 9.36 mmol) and DIPEA (1.6 g, 12.5 mmol) sequentially under ice bath conditions. The reaction solution was stirred at room temperature for 1.5 hours and then directly purified by Flash column chromatography (C18, eluent 0%-40% acetonitrile in a 0.1% aqueous trifluoroacetic acid solution) to give intermediate 24A (1.2 g) as a light yellow solid. m/z: $[M+H]^+$ 294.2.

Step 2: To a solution of intermediate 24A (1 g, 3.69 mmol) in dichloromethane (15 mL) was added trifluoroacetic acid (5 mL) under ice bath conditions. After addition, the reaction solution was stirred at room temperature for 1 hour and

then directly purified by Flash column chromatography (C18, eluent 0%-30% acetonitrile in a 0.1% aqueous trifluoroacetic acid solution) to give intermediate 24B (1.2 g) as a yellow solid. m/z: $[M+H]^+$ 216.2.

Steps 3-4: Using the synthesis method of L-7, L-24 was obtained by reaction with 24B. m/z: $[1/2M+H]^+$ 551.2.

Synthesis of **L-25:** 2-(4,6-divinylpyridin-2-yl)-4-oxo-8,11,14,17,20,23-hexaoxa-2,5-diazahexacosan-26-oic acid

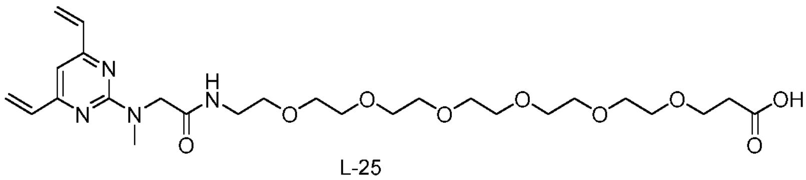

L-25

Using the synthesis method of L-7, L-25 was synthesized from N-(4,6-divinylpyridin-2-yl)-N-methylglycine. m/z: $[M+H]^+$ 555.3.

Synthesis of L-26: 1-(4-(2,5-dioxo-2,5-dihydro-1H-pyrrol-1-yl)phenyl)-1-oxo-5,8,11,14,17,20-hexaoxa-2-azatricosan-23-oic acid

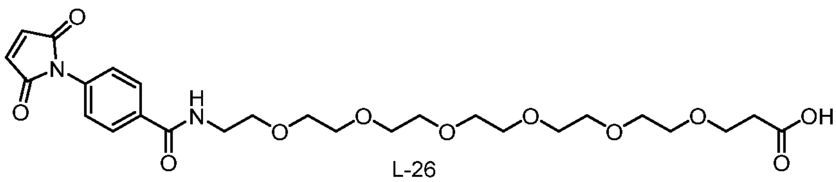

L-26

Using the synthesis method of L-7, L-26 was synthesized from 4-(2,5-dioxo-2,5-dihydro-1H-pyrrol-1-yl)benzoic acid. m/z: $[M+H]^+$ 553.2.

Synthesis of **L-27:** 2-(4,6-divinylpyridin-2-yl)-4-oxo-8,11-dioxa-2,5-diazatetradecan-14-oic acid

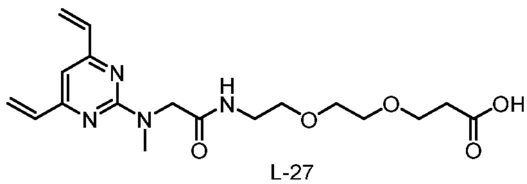

L-27

Using the synthesis method of L-7, L-27 was synthesized from N-(4,6-divinylpyridin-2-yl)-N-methylglycine. m/z: $[M+H]^+$ 379.2.

Synthesis of **L-28:** (3-(2-(2-(2-(2,5-dioxo-2,5-dihydro-1H-pyrrol-1-yl)acetylamino)ethoxy)ethoxy)propionyl)glycine

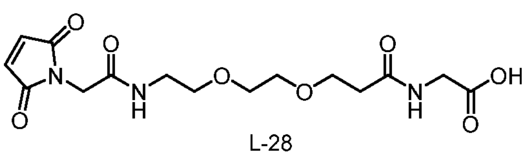

L-28

Using the synthesis method of L-7, L-28 was synthesized from L-8. m/z:$[M+H]^+$ 372.2.

Synthesis of **L-29:** 3-(2-(6-(2,5-dioxo-2,5-dihydro-1H-pyrrol-1-yl)hexanoylamino)acetylamino)-4-((1-methyl-2-nitro-1H-imidazol-5-yl)methoxy)benzyl(4-nitrophenyl)carbonate

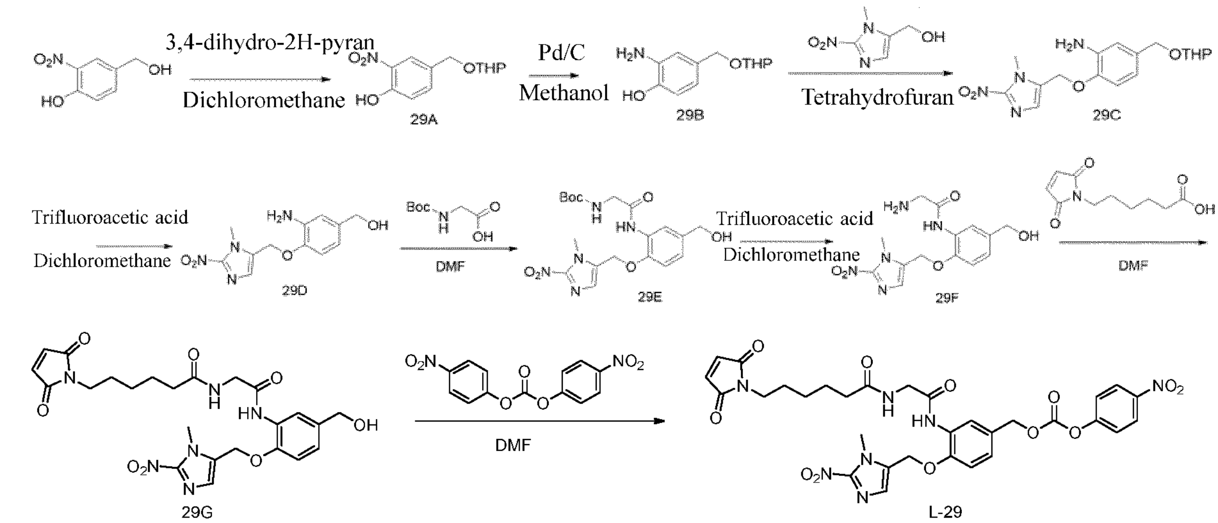


Step 1: To a solution of 4-hydroxy-3-nitrobenzyl alcohol (2 g, 11.8 mmol) in dichloromethane (100 mL) were added 3,4-dihydro-2H-pyran (1.02 g, 12.2 mmol) and pyridinium p-toluenesulfonate (0.3 g, 1.18 mmol). The reaction solution was stirred at room temperature for 16 hours. Then the reaction solution was washed with a saturated aqueous sodium bicarbonate solution and water, the organic phase was concentrated under reduced pressure, and the residue was purified by Flash column chromatography (petroleum ether/ethyl acetate = 4/1) to give intermediate 29A (2.5 g) as a yellow oil. m/z:$[M+Na]^+$ 276.0.

Step 2: To a solution of intermediate 29A (1.25 g, 4.94 mmol) in methanol (120 mL) under nitrogen was added palladium on carbon (10%wt, 165 mg), the system was replaced with hydrogen three times, and the reaction was stirred at room temperature under hydrogen atmosphere for 2 hours. The reaction mixture was then filtered through diatomaceous earth and the filtrate was concentrated under reduced pressure to give intermediate 29B (1 g) as a yellow solid. m/z:$[M+Na]^+$ 246.2.

Step 3: To a solution of 3-methyl-2-nitroimidazole-4-methanol (600 mg, 3.82 mmol), intermediate 29B (929 mg, 4.16 mmol) and triphenylphosphine (1.5 g, 5.73 mmol) in tetrahydrofuran (10 mL) was added DBAD (1.32 g, 5.73 mmol) under ice bath conditions, and the reaction system was stirred at room temperature for 2 hours. The reaction solution was directly purified by Flash column chromatography (C18, eluent 0%-40% acetonitrile in a 0.1% aqueous trifluoroacetic acid solution) to give intermediate 29C (0.51 g) as a yellow solid. m/z:$[M+Na]^+$ 385.2.

Step 4: To a solution of intermediate 29C (510 mg, 1.41 mmol) in dichloromethane (6 mL) was added trifluoroacetic acid (2 mL) dropwise under ice bath conditions, and the reaction system was stirred at room temperature for 1 hour. The reaction solution was concentrated under reduced pressure and purified by Flash column chromatography (C18, eluent 0%-25% acetonitrile in a 0.1% aqueous trifluoroacetic acid solution) to give intermediate 29D (391 mg) as a yellow solid. m/z:$[M+H]^+$ 279.0.

Step 5: To a solution of N-tert-butoxycarbonylglycine (214 mg, 1.22 mmol) in DMF (5 mL) were added HATU (510 mg, 1.34 mmol), DIPEA (315 mg, 2.44 mmol) and intermediate 29D (340 mg, 1.22 mmol) each under ice bath conditions, and the reaction system was stirred at room temperature for 3 hours. The reaction solution was directly purified by Flash column chromatography (C18, eluent 0%-44% acetonitrile in a 0.1% aqueous trifluoroacetic acid solution) to give intermediate 29E (150 mg) as a yellow solid. m/z:$[M+Na]^+$ 458.2.

Step 6: To a solution of intermediate 29E (150 mg, 0.34 mmol) in dichloromethane (3 mL) was added trifluoroacetic acid (1 mL) dropwise under ice bath conditions. The reaction solution was stirred at room temperature for 1 hour. The reaction solution was concentrated under reduced pressure and purified by Flash column chromatography (C18, eluent 0%-27% acetonitrile in a 0.1% aqueous trifluoroacetic acid solution) to give intermediate 29F (70 mg) as a yellow solid. m/z:$[M+H]^+$336.2.

Step 7: To a solution of 6-(2,5-dioxo-2,5-dihydro-1H-pyrrol-1-yl)hexanoic acid (38 mg, 0.18 mmol) in DMF (2 mL) was added HATU (75.3 mg, 0.20 mmol), DIPEA (46.5 mg, 0.36 mmol) and intermediate 29F (60 mg, 0.18 mmol) each under ice bath conditions. The reaction solution was stirred at room temperature for 1 hour and directly purified by Flash column chromatography (C18, eluent 0%-41% acetonitrile in a 0.1% aqueous trifluoroacetic acid solution) to give

intermediate 29G (60 mg) as a yellow solid. m/z:[M+H]$^+$ 529.2.

Step 8: To a solution of intermediate 29G (50 mg, 0.1 mmol) in DMF (2 mL) were added bis(p-nitrobenzene) carbonate (57.8 mg, 0.19 mmol) and DIPEA (36.8 mg, 0.29 mmol). The reaction solution was stirred at room temperature for 2 hours and directly purified by Flash column chromatography (C18, eluent 0%-54% acetonitrile in a 0.1% aqueous trifluoroacetic acid solution) to give L-29 (60 mg) as a white solid. m/z: [M+H]$^+$ 694.2.

Synthesis of **L-30:** 1-(4,5-dibromo-2-ethyl-3,6-dioxo-3,6-dihydropyridazin-1(2H)-yl)-3-oxo-7,10,13,16,19,22-hexaoxa-4-azapentacosan-25-oic acid

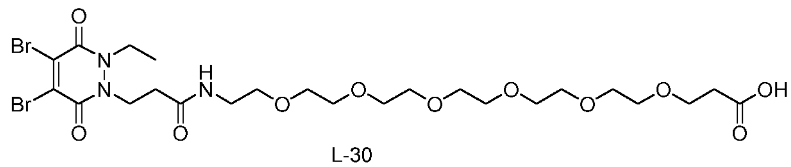

L-30

Using the synthesis method of L-7, L-30 was synthesized from 3-(4,5-dibromo-2-ethyl-3,6-dioxo-3,6-dihydropyridazin-1(2H)-yl)propionic acid. m/z: [M+H]$^+$ 704.0.

Synthesis of L-31: 2-((2-(3-(2,5-dioxo-2,5-dihydro-1H-pyrrol-1-yl)propionylamino)ethyl)dimercapto)-2-methylpropyl(4-nitrophenyl)carbonate

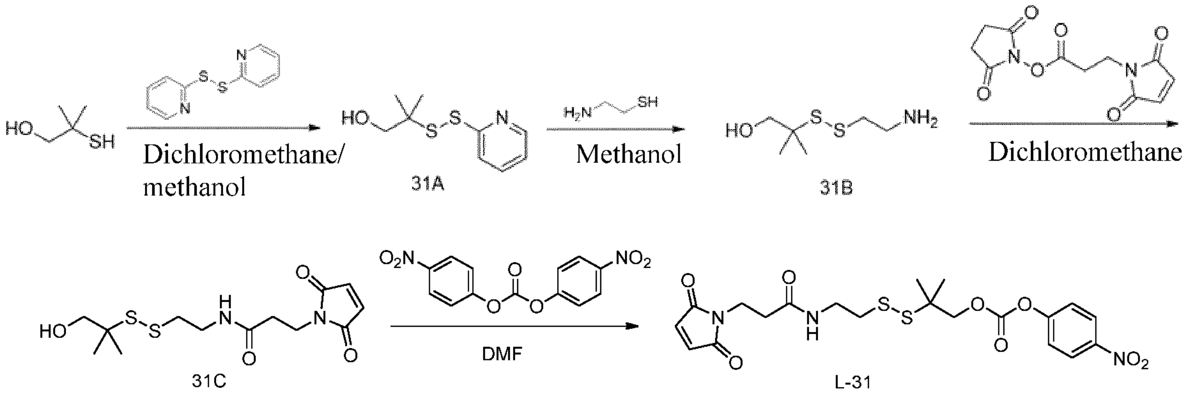


Step 1: To a mixed solution of 2-mercapto-2-methylpropan-1-ol (100 mg, 0.83 mmol) in dichloromethane (4 mL) and methanol (0.2 mL) was added 1,2-bis(pyridin-2-yl)disulfide (274 mg, 1.24 mmol). The reaction solution was stirred at room temperature for 16 hours and concentrated under reduced pressure, and the residue was purified by Flash column chromatography (petroleum ether/ethyl acetate = 3/1-1/3) to give intermediate 31A (300 mg) as a colorless liquid. m/z: [M+H]$^+$ 215.6.

Step 2: To a solution of intermediate 31A (1.9 g, 8.82 mmol) in methanol (15 mL) was added 2-aminoethanethiol (820 mg, 10.6 mmol). The reaction solution was stirred at 70°C for 4 hours and concentrated under reduced pressure. The residue was purified by Flash column chromatography (dichloromethane/methanol = 10/1 to 4/1) to give intermediate 31B (1.3 g) as a light yellow oil. m/z: [M+H]$^+$ 181.9.

Step 3: A solution of intermediate 31B (500 mg, 1.88 mmol), BMPS (310 mg, 1.71 mmol) and N-methylmorpholine (360 mg, 2.82 mmol) in dichloromethane (4 mL) was stirred at room temperature for 4 hours, the reaction solution was diluted with ethyl acetate and washed with saturated brine, the organic phase was separated and concentrated under reduced pressure, and the residue was purified by Flash column chromatography (dichloromethane/methanol = 20/1) to give intermediate 31C (232 mg) as a yellow oil.

Step 4: To a solution of intermediate 31C (50 mg, 0.15 mmol) in DMF (2 mL) were added bis(p-nitrobenzene) carbonate (68.5 mg, 0.22 mmol) and DIPEA (48.5 mg, 0.38 mmol). The reaction solution was stirred at room temperature for 5 hours and concentrated under reduced pressure. The residue was purified by Flash column chromatography (C18, eluent 0%-60% acetonitrile in a 0.1% aqueous trifluoroacetic acid solution) to give L-31 (38 mg) as a white solid. m/z:[M+H]$^+$ 498.0.

Synthesis of L-32: (E)-3,6,10-trioxo-2,14,17,20,23,26,29-heptaoxa-7,11-diazadotriacont-4-ene-32-oic acid

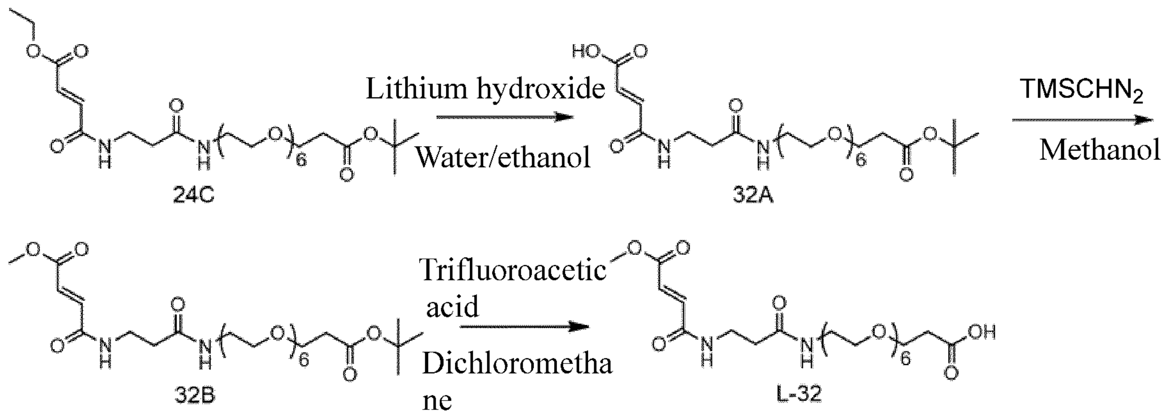

24C 32A 32B L-32

Step 1: To a solution of intermediate 24C (300 mg, 0.49 mmol) in ethanol (5 mL) was added an aqueous lithium hydroxide solution (1 M, 1.5 mL) under ice bath conditions, and the reaction solution was stirred at room temperature for 2 hours. At 0°C, the reaction solution was adjusted to pH = 2-3 with hydrochloric acid (3 M) and concentrated under reduced pressure, and the residue was purified by Flash column chromatography (C18, eluent 0-30% acetonitrile in a 0.1% aqueous trifluoroacetic acid solution) to give intermediate 32A (230 mg) as a white solid. m/z:$[M+H]^+$ 579.4.

Step 2: To a solution of intermediate 32A (230 mg, 0.4 mmol) in methanol (8 mL) was slowly added a solution of trimethylsilyldiazomethane in n-hexane (2 M, 3 mL) under ice bath conditions, and after the addition was completed, the reaction solution was stirred at room temperature for 8 hours and then concentrated directly under reduced pressure to give intermediate 32B (250 mg) as a yellow solid.

Step 3: To a solution of intermediate 32B (250 mg, 0.42 mmol) in dichloromethane (5 mL) was added trifluoroacetic acid (2 mL) under ice bath conditions, and the reaction solution was stirred at room temperature for 1 hour and concentrated directly under reduced pressure. The residue was purified by Flash column chromatography (C18, eluent 0-30% acetonitrile in a 0.1% aqueous trifluoroacetic acid solution) to give L-32 (110 mg) as a light yellow solid. m/z:$[M+H]^+$ 537.2.

Synthesis of **L-33:** 2,5-dioxopyrrolidin-1-yl 6-(3-bromo-5-methylene-2-oxo-2,5-dihydro-1H-pyrrol-1-yl)hexanoate

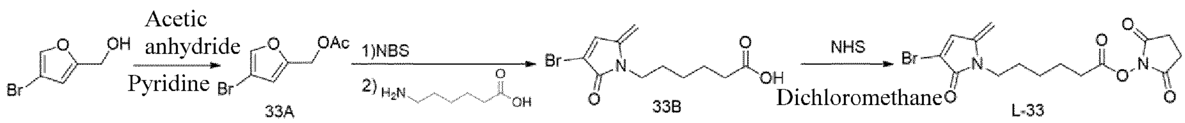

33A 33B L-33

Step 1: To a solution of (4-bromofuran-2-yl) methanol (1.5 g, 8.47 mmol) in pyridine (12 mL) was added acetic anhydride (3.46 g, 33.9 mmol) dropwise under ice bath conditions. The reaction solution was stirred at room temperature for 2 hours, the reaction solution was diluted with ethyl acetate, the organic phase was washed with saturated brine, and the organic phase was separated and concentrated under reduced pressure. The residue was purified by Flash column chromatography (petroleum ether/ethyl acetate = 5/1) to give intermediate 33A (1.4 g) as a yellow oil. $^1$H NMR (400 MHz, $CDCl_3$): $\delta$ 7.35 (d, *J* = 0.7Hz, 1H), 6.39 (s, 1H), 4.94 (s, 2H), 2.01 (s, 3H).

Step 2: To a mixed solution of intermediate 33A (1.4 g, 6.39 mmol) in tetrahydrofuran (9 mL) and water (1 mL) was added NBS (1.36 g, 7.67 mmol) under ice bath conditions. The reaction solution was stirred at 0°C for 4 hours, 6-aminocaproic acid (1.01 g, 7.67 mmol) was added to the reaction system, and the reaction solution was stirred at room temperature for 4 hours. The system was extracted with ethyl acetate, the organic phases were combined and washed with saturated brine, and the organic phases were separated and concentrated under reduced pressure. The residue was purified by Flash column chromatography (petroleum ether/ethyl acetate = 1/1) to give intermediate 33B (600 mg) as a yellow oil. m/z:$[M+H]^+$ 288.0.

Step 3: To a solution of intermediate 33B (600 mg, 2.08 mmol) in dichloromethane (4 mL) were added NHS (250 mg, 2.18 mmol) and DIC (280 mg, 2.18 mmol) sequentially under ice bath conditions. The reaction solution was stirred at 0°C for 3 hours. The reaction solution was diluted with ethyl acetate, the organic phase was washed with saturated brine,

and the organic phase was separated and concentrated under reduced pressure. The residue was purified by Flash column chromatography (petroleum ether/ethyl acetate = 1/1) to give L-33 (176 mg) as a yellow oil. m/z: $[M+H]^+$ 384.8.

Synthesis of L-34: 6-(4-(3-toluenesulfonyl-2-(toluenesulfonylmethyl)propionyl)benzoylamino)hexanoic acid

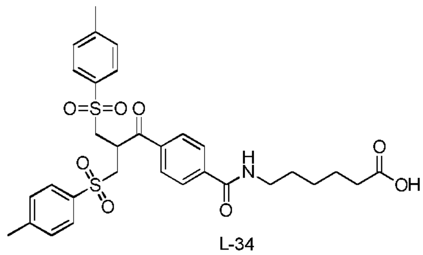

L-34

Using the synthesis method of L-7, L-34 was synthesized from 4-(3-p-toluenesulfonyl-2-(toluenesulfonylmethyl) propionyl)benzoic acid. m/z: $[M+H]^+$ 614.2.

Synthesis of L-35: 1-(4-(2,5-dioxo-2,5-dihydro-1H-pyrrol-1-yl)phenyl)-2-oxo-6,9,12,15,18,21-hexaoxa-3-azatetracosan-24-oic acid

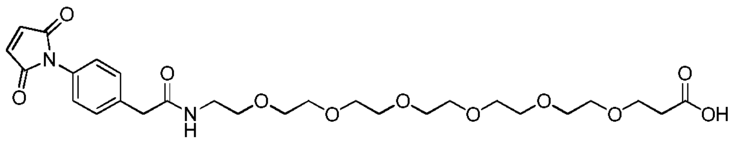

L-35

Using the synthesis method of L-7, L-35 was synthesized from 2-(4-(2,5-dioxo-2,5-dihydro-1H-pyrrol-1-yl) phenyl)acetic acid. m/z:$[M+H]^+$ 567.2.

Synthesis of L-36: 2-(2-((tert-butoxycarbonyl) amino) acetylamino)-4-((((4-nitrophenoxy) carbonyl) oxy) methyl) phenyl neopentyl sulfate

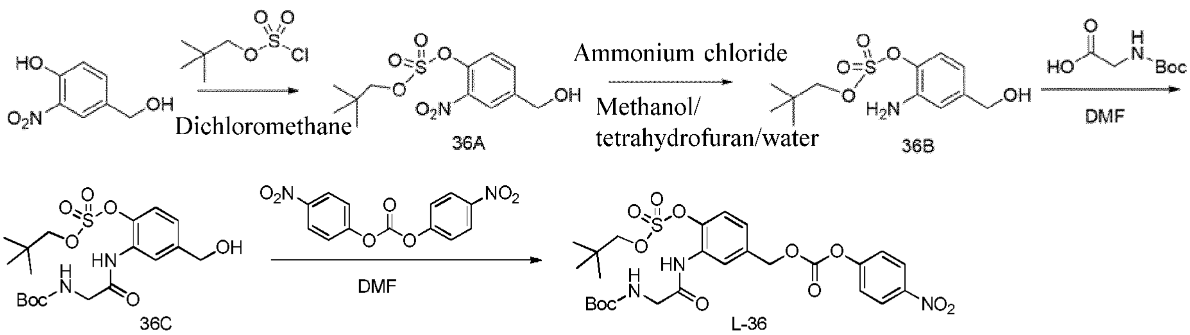


Step 1: To a solution of 4-(hydroxymethyl)-2-nitrophenol (2 g, 11.8 mmol) and 1,8-diazabicyclo[5.4.0]undec-7-ene (2.3 g, 15.4 mmol) in dichloromethane (30 mL) was slowly added neopentyl chlorosulfonate (2.87 g, 15.4 mmol) dropwise under ice bath conditions. The reaction solution was stirred at room temperature for 6 hours. The reaction was quenched by the addition of a saturated aqueous ammonium chloride solution, the aqueous phase was extracted with ethyl acetate, the organic phases were combined and washed with saturated brine, and the organic phases were concentrated under reduced pressure. The residue was purified by silica gel column chromatography (petroleum ether/ethyl acetate = 100-2/1) to give intermediate 36A (960 mg) as a yellow oil. m/z:$[M+Na]^+$ 342.0.

Step 2: To a mixed solution of intermediate 36A (700 mg, 2.19 mmol) and ammonium chloride (1.17 g, 21.9 mmol)

in methanol (3 mL), tetrahydrofuran (6 mL) and water (8 mL) was slowly added zinc powder (1.4 g, 21.9 mmol) under ice bath conditions. The reaction system was stirred at room temperature for 3 hours. The mixture was concentrated under reduced pressure and the residue was extracted with ethyl acetate. The organic phases were combined and concentrated under reduced pressure, and the residue was purified by Flash column chromatography (C18, eluent 0%-60% acetonitrile in a 0.1% aqueous trifluoroacetic acid solution) to give intermediate 36B (240 mg) as a yellow solid. m/z:[M+H]+ 290.1.

Steps 3-4: Using the synthesis method of steps 7-8 of L-29, L-36 was obtained as a white solid by reaction with intermediate 36B. m/z:[M+Na]+ 634.2.

Synthesis of L-37: (S)-2-(6-(2,5-dioxo-2,5-dihydro-1H-pyrrol-1-yl)hexanoylamino)-5-ureidopentanoic acid

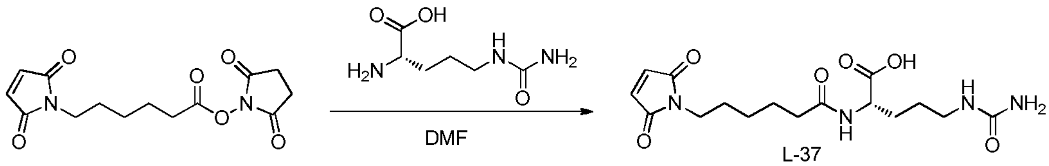

L-37

To a solution of EMCS (300 mg, 0.97 mmol) and L-citrulline (170 mg, 0.97 mmol) in DMF (3 mL) was added DIPEA (251 mg, 1.94 mmol). The reaction solution was stirred at room temperature for 3 days and then purified by Flash column chromatography (C18, eluent 0% to 45% acetonitrile in a 0.1% aqueous trifluoroacetic acid solution) to give L-37 ((169 mg) as a white solid. m/z:[M+H]+ 369.2.

Synthesis of L-38: 2-((S)-2-((S)-2-(6-(2,5-dioxo-2,5-dihydro-1H-pyrrol-1-yl)hexanoylamino)-3-methylbutanoylamino)-5-ureidopentanoylamino)acetic acid

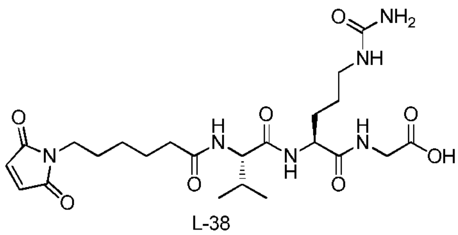
L-38

Using the synthesis method of L-3, L-38 was synthesized from Fmoc-Val-Cit-OH and tert-butyl 2-aminoacetate. m/z:[M+H]+ 525.2.

Synthesis of L-39: 1-(3,5-diacryloyl-1,3,5-triazin-1-yl)-1,6-dioxo-10,13,16,19,22,25-hexaoxa-4-thia-7-azaoctacosan-28-oic acid

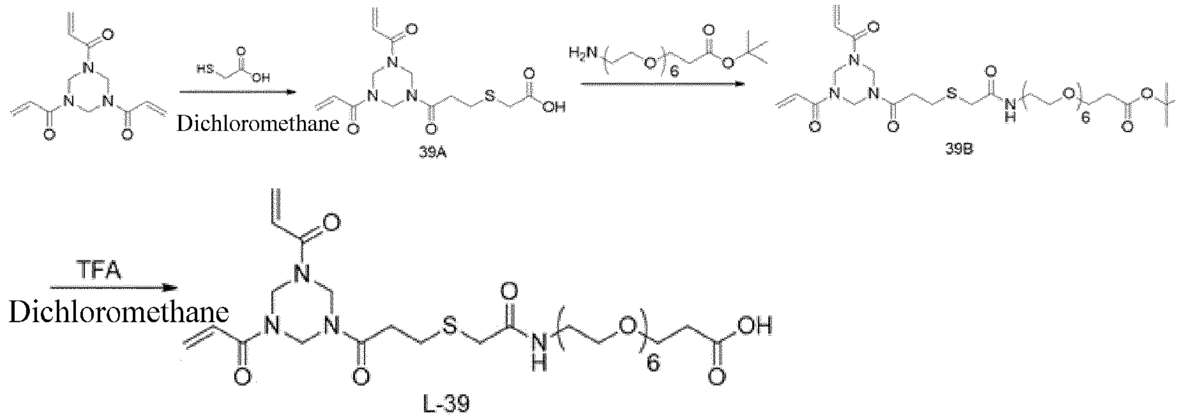


39A 39B L-39

Step 1: To a solution of compound 1,1',1"-(1,3,5-triazine-1,3,5-triyl)tris(prop-2-en-1-one) (3.74 g, 15.0 mmol) in dichloromethane (100 mL) were added triethylamine (0.15 g, 1.5 mmol) and 2-mercaptoacetic acid (280 mg, 3 mmol) under ice bath conditions and under nitrogen atmosphere. The reaction solution was stirred at room temperature for 16 hours and concentrated under reduced pressure, and the residue was purified by Flash column chromatography (dichloromethane/methanol = 10/1) to give intermediate 39A (400 mg) as a white solid. m/z: $[M+H]^+$ 342.0.

Step 2: To a solution of intermediate 39A (80 mg, 0.23 mmol) and tert-butyl 1-amino-3,6,9,12,15,18-hexaoxaeicosan-21-oate (94 mg, 0.23 mmol) in DMF (3 mL) were added DIPEA (44.6 mg, 0.35 mmol), EDCI (52.9 mg, 0.28 mmol) and HOBT (37.3 mg, 0.28 mmol) sequentially under ice bath conditions. The reaction solution was stirred at room temperature overnight and filtered, and the filtrate was purified by Flash column chromatography (C18, eluent 0-50% acetonitrile in a 0.1% aqueous trifluoroacetic acid solution) to give intermediate 39B (56 mg) as a colorless oil. m/z: $[M+H]^+$ 733.4.

Step 3: To a solution of intermediate 39B (56 mg, 0.08 mmol) in dichloromethane (3 mL) was added trifluoroacetic acid (1 mL) under ice bath conditions. After addition, the reaction solution was stirred at room temperature for 1 hour and then directly purified by Flash column chromatography (C18, eluent 0%-60% acetonitrile in a 0.1% aqueous trifluoroacetic acid solution) to give L-39 (30 mg) as a colorless oil. m/z: $[M+H]^+$ 677.3.

Synthesis of **L-40:** (9H-fluoren -9-yl)methyl(2-(3-((1-((((2,5-dioxopyrrolidin-1-yl)oxy)carbonyl)oxy)-2-methylpropan-2-yl)dimercapto)propanamido)ethyl)carbamate

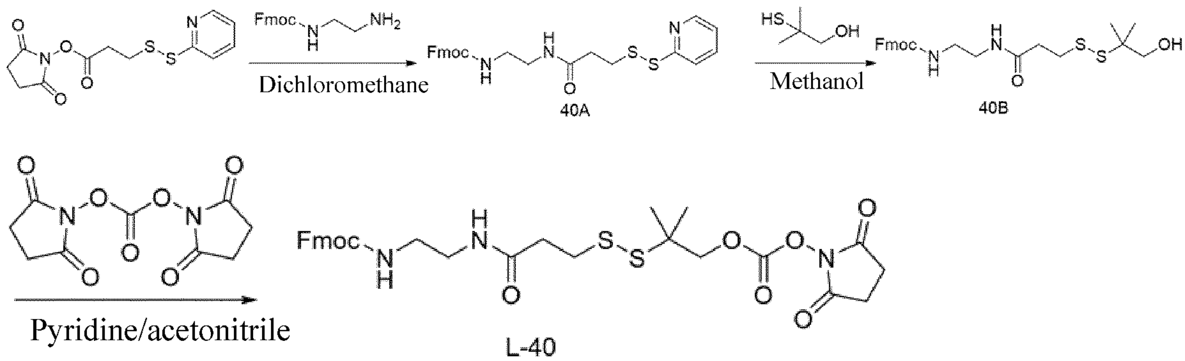


40A 40B L-40

Step 1: A solution of N-hydroxysuccinimide 3-(2-pyridyldimercapto)propionate (1.8 g, 5.76 mmol), Fmoc-ethylenediamine (1.79 g, 6.34 mmol), N-methylmorpholine (1.17 g, 11.5 mmol) in dichloromethane (15 mL) was stirred at room temperature for 4 hours. After dilution with addition of water and extraction with dichloromethane, the organic phases were combined and concentrated under reduced pressure, and the residue was purified by Flash column chromatography (dichloromethane/methanol = 20/1) to give intermediate 40A (2.01 g) as a white solid.

Step 2: A solution of intermediate 40A (1.0 g, 2.09 mmol), 2-mercapto-2-methylpropan-1-ol (0.21 g, 1.99 mmol) in methanol (12 mL) was stirred at 60°C for 6 hours. Methanol was removed by concentration under reduced pressure, diluted with water and extracted with dichloromethane, the organic phases were combined and concentrated under reduced pressure, and the residue was purified by silica gel column chromatography (ethyl acetate) to give intermediate 40B (686 mg) as a white solid. m/z:$[M+Na]^+$ 497.0.

Step 3: To a solution of intermediate 40B (100 mg, 0.21 mmol) in acetonitrile (3 mL) were added N,N'-disuccinimidyl carbonate (64.6 mg, 0.25 mmol) and pyridine (20.8 mg, 0.26 mmol) sequentially under nitrogen atmosphere, and after the addition was completed, the reaction system was replaced with nitrogen three times and stirred at room temperature overnight. The reaction solution was concentrated directly under reduced pressure and purified by *prep*-TLC (dichloromethane/methanol = 10/1) to give L-40 (61 mg) as a white solid. m/z:$[M+Na]^+$ 638.2.

Synthesis of **L-41A:** 2-((2,5-dioxo-2,5-dihydro-1H-pyrrol-1-yl)methyl)-1,3-dioxan-5-carboxylic acid, isomer 1 and **L-41B:** 2-((2,5-dioxo-2,5-dihydro-1H-pyrrol-1-yl)methyl)-1,3-dioxan-5-carboxylic acid, isomer 2

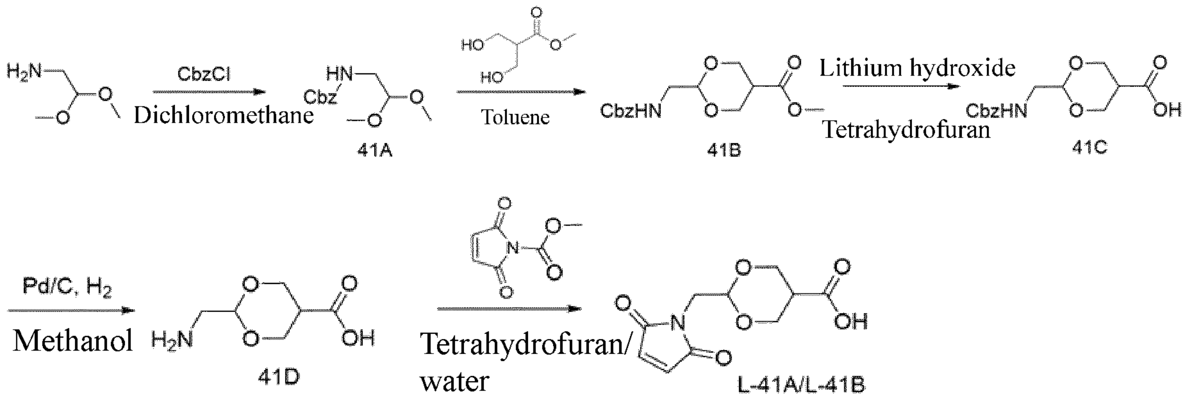


Step 1: To a solution of 2,2-dimethoxyethan-1-amine (2 g, 19.0 mmol) and DIPEA (7.37 g, 57.1 mmol) in dichloromethane (20 mL) was added benzyl chloroformate (3.41 g, 20.0 mmol) dropwise under ice bath conditions. The reaction solution was stirred at room temperature for 3 hours, the reaction system was washed with water and saturated brine, and the organic phase was separated and concentrated under reduced pressure to give intermediate 41A (2.7 g) as a colorless oil.

Step 2: To a solution of intermediate 41A (1.3 g, 5.43 mmol) in toluene (40 mL) were added methyl 3-hydroxy-2-(hydroxymethyl) propionate (1.18 g, 7.06 mmol) and p-toluenesulfonic acid (18.7 mg, 0.11 mmol). The reaction solution was stirred at 110°C for 2 hours, sodium bicarbonate (100 mg) was added, and stirring was continued for 0.5 hours. The reaction solution was filtered and the filtrate was concentrated under reduced pressure to give 41B (2.1 g) as a light yellow oil. m/z:$[M+H]^+$ 310.1.

Step 3: To a mixed solution of lithium hydroxide in tetrahydrofuran and water (0.75%, 60 mL, 1/1) was added intermediate 41B (2.1 g, 5.43 mmol). The reaction solution was stirred at room temperature for 6 hours, the reaction system was adjusted to pH = 5-6 with hydrochloric acid (1 M) and extracted with dichloromethane, and the organic phases were combined and concentrated under reduced pressure to give intermediate 41C (1.25 g) as a light yellow solid.

Step 4: To a solution of intermediate 41C (380 mg, 1.29 mmol) in methanol (20 mL) was added Pd/C (10%wt, 38 mg), and the reaction system was replaced three times with nitrogen and three times with hydrogen and pressurized to 14.5 psi, stirred at room temperature for 1 hour, and concentrated under reduced pressure to give intermediate 41D (200 mg) as a black solid. m/z:$[M+H]^+$ 162.0.

Step 5: To a mixed solution of intermediate 41D (200 mg, 1.24 mmol) and methyl 2,5-dioxo-2,5-dihydro-1H-pyrrole-1-carboxylate (190 mg, 1.24 mmol) in water (2 mL) and tetrahydrofuran (4 mL) was added sodium bicarbonate (210 mg, 2.48 mmol) under ice bath conditions. The reaction solution was stirred at 0°C for 2 hours, the aqueous phase was extracted with ethyl acetate, the organic phases were combined and concentrated under reduced pressure to give L-41, L-41 was separated by *prep*-HPLC (separation condition 4, eluent 0%-28% acetonitrile in a 0.1% aqueous formic acid solution, 20 minutes) to give L-41A (14 mg) and L-41B (16.5 mg), both as white solids.

L-41A: $^1H$ NMR (400 MHz, DMSO-$d_6$): δ 12.45 (s, 1H), 7.04 (s, 2H), 4.72 (t, *J* = 5.2Hz, 1H), 4.31 (d, *J* = 11.2Hz, 2H), 3.80-3.84 (m, 2H), 3.41 (d, *J* = 5.2Hz, 2H), 3.31-3.25 (m, 1H).

L-41B: $^1H$ NMR (400 MHz, $CDCl_3$): δ 6.73 (s, 1H), 4.70 (t, *J* = 5.2Hz, 1H), 4.30-4.34 (m, 1H), 3.71-3.75 (m, 2H), 3.07 (m, 1H).

Synthesis of **L-42:** 1-(2-((2,5-dioxo-2,5-dihydro-1H-pyrrol-1-yl)methyl)-1,3-dioxan-5-yl)-1-oxo-5,8,11,14,17,20-hexaoxa-2-azatricosan-23-oic acid

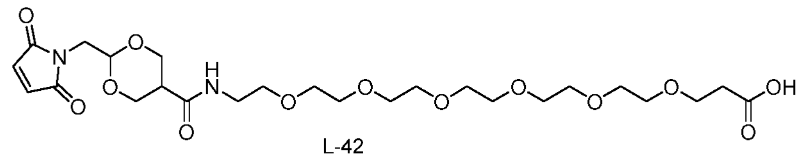

L-42

Using the synthesis method of L-7, L-42 was synthesized from L-41. m/z: $[M+H]^+$ 577.2.

Synthesis of **L-43:** tert-butyl 2-(3-((1-((((2,5-dioxopyrrolidin-1-yl)oxy)carbonyl)oxy)-2-methylpropan-2-yl)dimercapto) propionyl)hydrazine carbamate

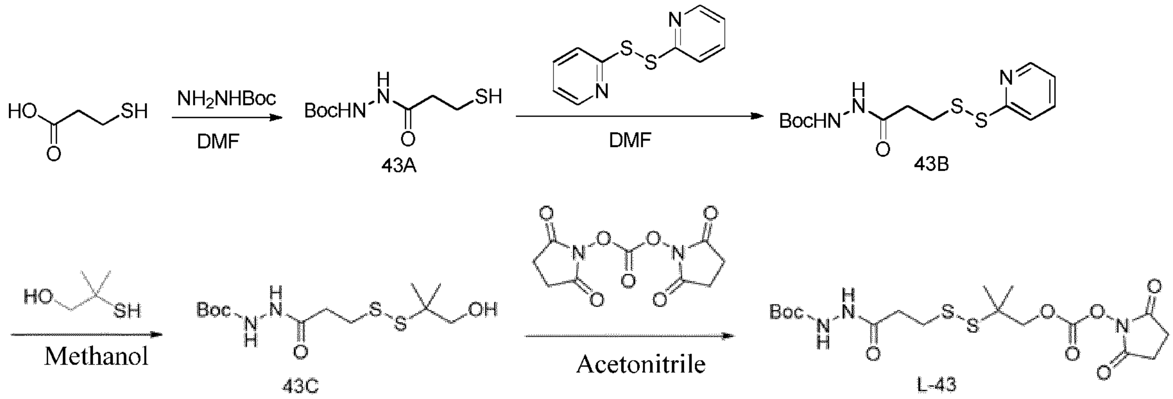


43A

43B

43C

L-43

Step 1: To a solution of 3-mercaptopropionic acid (5.0 g, 47.1 mmol) in DMF (50 mL) were added HOBT (8.28 g, 61.2 mmol), EDCI (9.93 g, 51.8 mmol), tert-butyl hydrazinocarbamate (9.34 g, 70.7 mmol) and DIPEA (12.2 g, 94.2 mmol) sequentially under ice bath conditions. The reaction system was replaced with nitrogen three times and stirred at room temperature overnight. The reaction solution was concentrated under reduced pressure, and the residue was purified by Flash column chromatography (dichloromethane/methanol = 10/1) to give intermediate 43A (3.0 g) as a colorless oil. m/z: $[M-56+H]^+$ 164.9.

Step 2: To a solution of intermediate 43A (3.0 g, 8.17 mmol) in DMF (10 mL) was added 1,2-bis(pyridin-2-yl) disulfide (2.34 g, 10.6 mmol) under nitrogen atmosphere, and the reaction solution was stirred at room temperature for 16 hours. The reaction solution was poured into water, the aqueous phase was extracted with ethyl acetate, the organic phases were combined and washed with saturated brine, the organic phase was concentrated under reduced pressure, and the residue was purified by silica gel column chromatography (petroleum ether/ethyl acetate = 10/1-1/1) to give intermediate 43B (1.0 g) as a colorless oil. m/z: $[M+H]^+$ 330.0.

Steps 3-4: Using the synthesis method in steps 2-3 of L-40, L-43 was obtained by reaction with 43B. m/z:$[M+Na]^+$ 488.0.

Synthesis of **L-44:** (5)-2-(1-(ethoxycarbonyl)cyclobutylformylamino)-5-ureidopentanoic acid

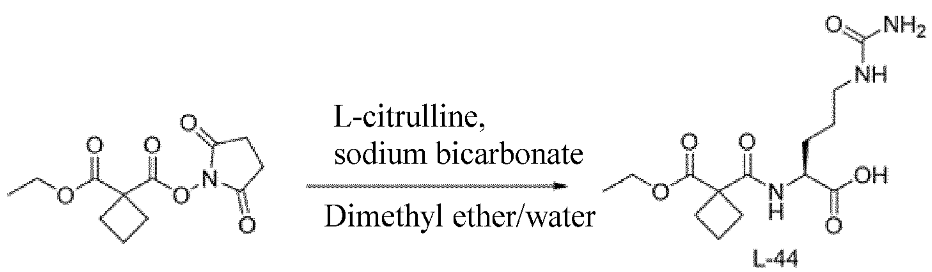


L-44

A mixed solution of L-citrulline (1.5 g, 8.56 mmol) and sodium bicarbonate (1.5 g, 8.56 mmol) in dimethyl ether (20 mL) and water (40 mL) was stirred at room temperature for 15 minutes, after the reaction system became turbid, 1-(2,5-dioxopyrrolidin-1-yl)1-ethylcyclobutane-1,1-dicarboxylate (3.46 g, 12.8 mmol) was added, and the reaction system was

stirred at room temperature for 4 days. The reaction solution was concentrated under reduced pressure and the residue was then purified by Flash column chromatography (C18, eluent 0-30% acetonitrile in a 0.1% aqueous trifluoroacetic acid solution) to give L-44 (2.3 g) as a white solid. m/z: $[M+H]^+$ 330.2.

Synthesis of **L-45:** N-(2-(4-((2,5-dioxo-2,5-dihydro-1H-pyrrol-1-yl)methyl)cyclohexane-1-amido) ethyl)-4-formylbenzamide

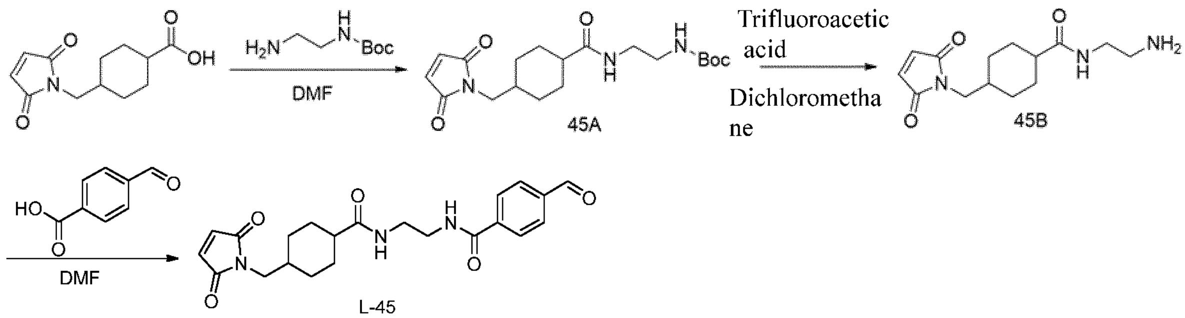


Steps 1 & 2: Using the synthesis method of L-7, 45B was synthesized from 4-((2,5-dioxo-2,5-dihydro-1H-pyrrol-1-yl)methyl)cyclohexanecarboxylic acid and tert-butyl(2-aminoethyl)carbamate. m/z: $[M+H]^+$ 280.2.

Step 3: To a solution of 4-formylbenzoic acid (41.3 mg, 0.28 mmol), HATU (124 mg, 0.33 mmol) and DIPEA (97 mg, 0.75 mmol) in DMF (3 mL) was added intermediate 45B (100 mg, 0.25 mmol). The reaction solution was stirred at room temperature for 3 hours and then directly purified by Flash column chromatography (C18, eluent 0%-45% acetonitrile in a 10 mmol/L aqueous ammonium bicarbonate solution) to give L-45 (85 mg) as a white solid. m/z: $[M+H]^+$ 412.2.

Synthesis of **L-46:** 3-(2-(2-(2-(2,5-dioxo-2,5-dihydro-1H-pyrrol-1-yl)-N-methylacetylamino)-N-methylacetylamino)acetylamino)propionic acid

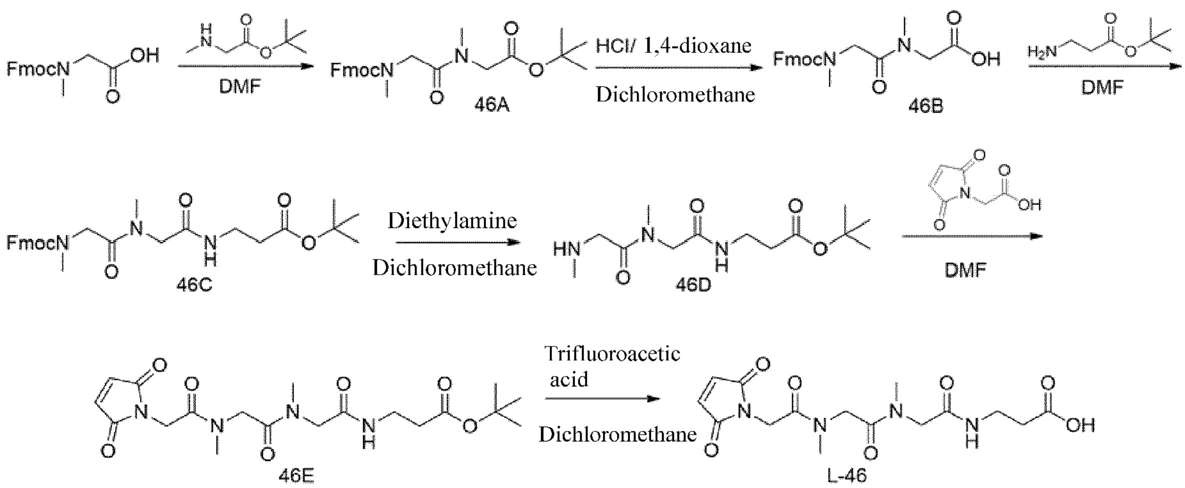


Step 1: To a solution of 2-((((9H-fluoren-9-yl)methoxy)carbonyl)(methyl)amino)acetic acid (1.5 g, 4.82 mmol) in DMF (20 mL) were added DIPEA (1.8 g, 14.5 mmol) and HATU (1.83 g, 4.82 mmol), the reaction solution was stirred at room temperature for 10 minutes, then tert-butyl 2-(methylamino)acetate (1.06 g, 5.78 mmol) was added, the reaction system was stirred at room temperature for 40 minutes and diluted with ethyl acetate, the organic phase was washed with water and saturated brine, the organic phase was separated and concentrated under reduced pressure, and the residue was purified by Flash column chromatography (ethyl acetate/petroleum ether = 1/9-3/7) to give intermediate 46A (1.5 g) as a yellow solid. m/z:$[M+Na]^+$ 461.1.

Step 2: To a solution of intermediate 46A (3 g, 6.84 mmol) in dichloromethane (5 mL) was added a solution of hydrogen chloride-1,4-dioxane (2 M, 30 mL), and the reaction solution was stirred at room temperature for 6 hours and then

concentrated directly under reduced pressure to give intermediate 46B (3 g) as a yellow solid. m/z: $[M+H]^+$ 383.0.

Step 3: Using the synthesis method of 46A, 46C was obained as a yellow solid by reaction with 46B. m/z:$[M+H]^+$ 510.1.

Step 4: Intermediate 46C (900 mg, 1.77 mmol) was added to a solution of diethylamine in dichloromethane (30%, 3 mL), the reaction solution was stirred at room temperature for 2 hours and then concentrated directly under reduced pressure, and the residue was purified by Flash column chromatography (methanol/dichloromethane = 0-1/10) to give intermediate 46D (490 mg) as a yellow solid.

Step 5: To a solution of intermediate 46D (490 mg, 1.7 mmol) and 2-(2,5-dioxo-2,5-dihydro-1H-pyrrol-1-yl)acetic acid (330 mg, 2.13 mmol) in DMF (4 mL) were added HOBT (370 mg, 2.77 mmol), EDCI (530 mg, 2.77 mmol) and DIPEA (830 mg, 6.39 mmol) sequentially. The reaction solution was stirred at room temperature for 2 hours and diluted with ethyl acetate, the organic phase was washed with water and saturated brine, the organic phase was separated and concentrated under reduced pressure, and the residue was purified by Flash column chromatography (C18, eluent 0-30% acetonitrile in a 0.1 aqueous trifluoroacetic acid solution) to give intermediate 46E (310 mg) as a yellow solid. m/z:$[M+H]^+$ 425.1.

Step 6: To a solution of intermediate 46E (310 mg, 0.73 mmol) in dichloromethane (3.5 mL) was added trifluoroacetic acid (0.5 mL), and the reaction solution was stirred at room temperature for 6 hours and concentrated directly under reduced pressure. The residue was purified by Flash column chromatography (C18, eluent 0-60% acetonitrile in a 0.1% aqueous trifluoroacetic acid solution) to give L-46 (60 mg) as a white solid. $^1H$ NMR (400 MHz, DMSO-$d_6$): $\delta$ 12.27 (s, 1H), 8.25-7.74 (m,1H), 7.10 (d, $J$ = 2.4Hz, 2H), 4.43-4.29 (m, 1H), 4.17 (d, $J$ = 15.8Hz, 1H), 3.91 (dd, $J$ = 19.8, 13.4Hz, 2H), 3.29 (d, $J$ = 10.2Hz, 4H), 2.98 (dd, $J$ = 27.4, 15.2Hz, 2H), 2.82-2.76 (m, 4H), 2.40 (dd, $J$ = 13.6, 6.0Hz, 3H).

Synthesis of **L-47:** 4-((S)-2-((S)-2-(2-((3-(3,5-diacryloyl-1,3,5-triazin-1-yl)-3-oxopropyl)mercapto)acetylamino)-3-methylbutanoylamino)-5-ureidopentanoylamino)benzyl(4-nitrobenzene)carbonate

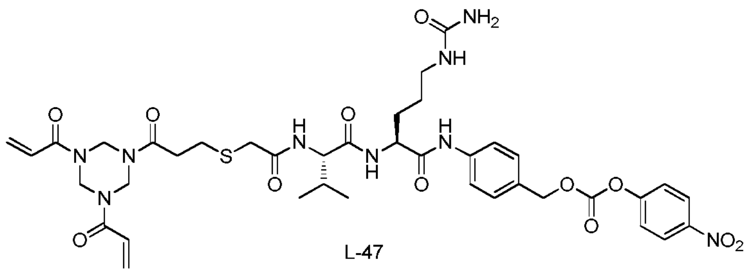

L-47

Using the synthesis method of L-5, L-47 was synthesized with Val-Cit-PAB-OH and 39A. m/z:$[M+H]^+$ 868.2.

Synthesis of L-48: 4-((S)-2-((S)-2-(2-((2,5-dioxo-2,5-dihydro-1H-pyrrol-1-yl)methyl)-1,3-dioxan-5 amido)-3-methylbutanoylamino)-5-ureidopentanoylamino)benzyl(4-nitrobenzene)carbonate

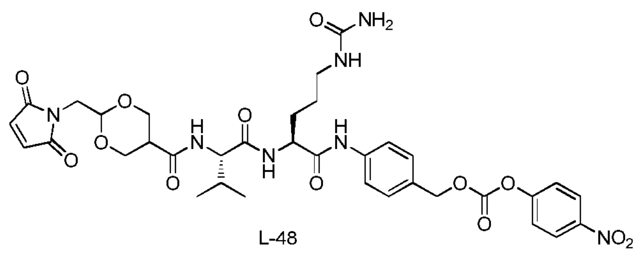

L-48

Using the synthesis method of L-5, L-47 was synthesized from Val-Cit-PAB-OH and L-41. m/z:$[M+H]^+$ 768.2.

Synthesis of **L-49:** 4-((S)-2-((S)-2-(2-(4-(2,5-dioxo-2,5-dihydro-1H-pyrrol-1-yl)phenyl)acetylamino)-3-methylbutanoylamino)-5-ureidopentanoylamino)benzyl(4-nitrobenzene)carbonate

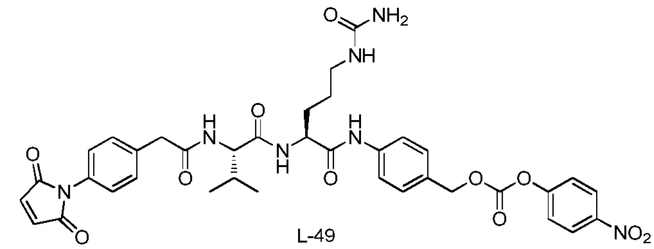

L-49

Using the synthesis method of L-5, L-49 was synthesized using Val-Cit-PAB-OH and 2-(4-(2,5-dioxo-2,5-dihydro-1H-pyrrol-1-yl)phenyl)acetic acid. m/z:$[M+H]^+$ 758.2.

Synthesis of **L-50:** 4-((S)-2-((S)-2-(2-(4-(2,5-dioxo-2,5-dihydro-1H-pyrrol-1-yl)benzoylamino)-3-methylbutanoylamino)-5-ureidopentanoylamino)benzyl(4-nitrobenzene)carbonate

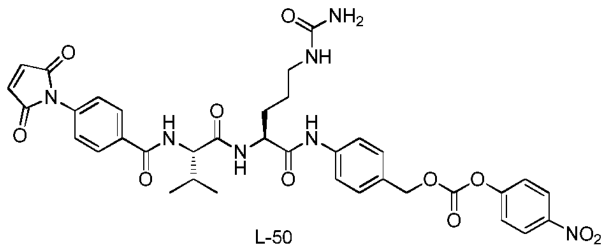

L-50

Using the synthesis method of L-5, L-50 was synthesized from Val-Cit-PAB-OH and 2-(4-(2,5-dioxo-2,5-dihydro-1H-pyrrol-1-yl)benzoic acid. m/z:$[M+H]^+$ 744.2.

Synthesis of **L-51:** (R)-2-((tert-butoxycarbonyl)amino)-3-(2-(2,5-dioxo-2,5-dihydro-1H-pyrrol-1-yl)acetylamino)propionic acid

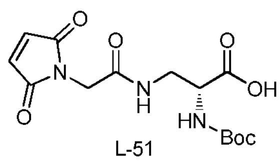

L-51

Using the synthesis method in step 1 of L-7, L-51 was synthesized from 2-(2,5-dioxo-2,5-dihydro-1H-pyrrol-1-yl) acetic acid and (R)-3-amino-2-((tert-butoxycarbonyl)amino)propionic acid. m/z: $[M+Na]^+$ 364.0.

Synthesis of L-52: 4-((S)-4-amino-2-((S)-2-((S)-2-(6-(2,5-dioxo-2,5-dihydro-1H-pyrrol-1-yl)hexanoylamino) propionylamino)propionylamino)-4-oxobutanoylamino)benzyl(4-nitrobenzene)carbonate

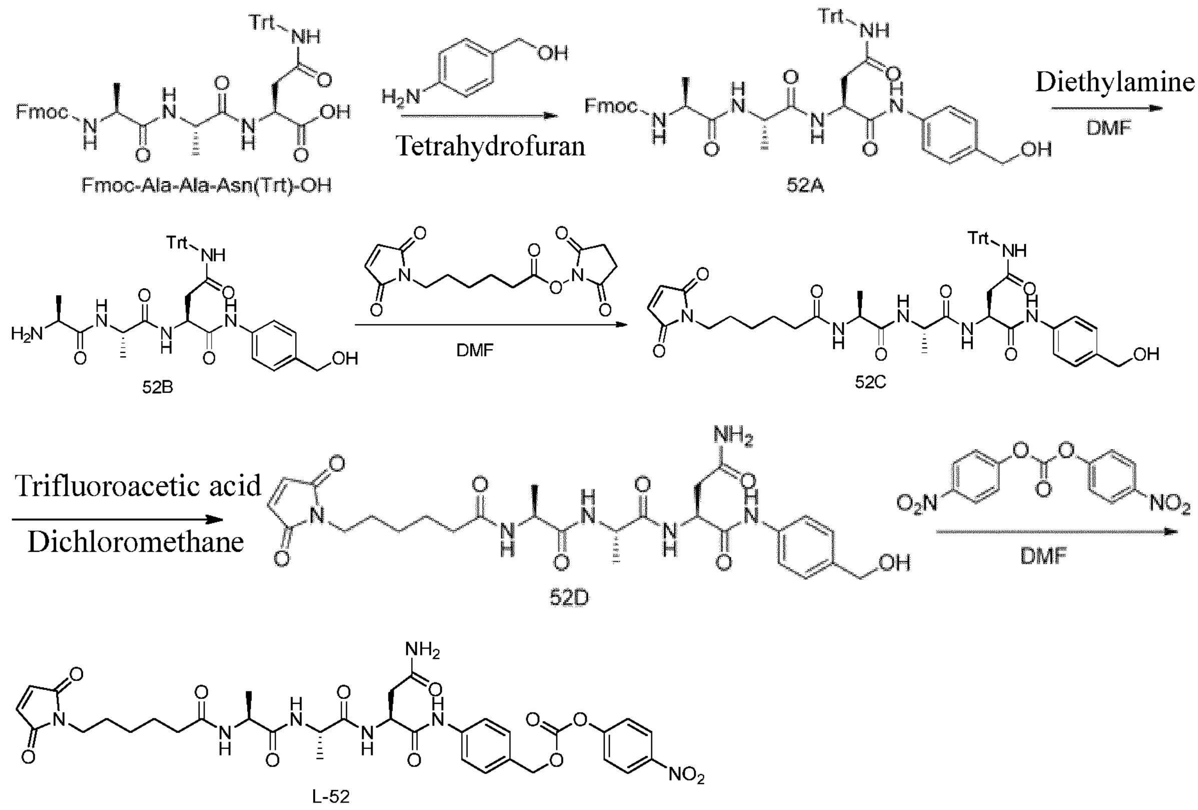


Step 1: To a solution of Fmoc-Ala-Ala-Asn (Trt)-OH (150 mg, 0.2 mmol) in tetrahydrofuran (2 mL) were added (4-aminophenyl) methanol (29.6 mg, 0.24 mmol) and EEDQ (79.1 mg, 0.32 mmol) each. The reaction solution was stirred at room temperature overnight and concentrated under reduced pressure to give intermediate 52A (171 mg) as a yellow solid. m/z: $[M+H]^+$ 844.3.

Steps 2-4: Using the synthesis method in steps 2-4 of L-3, 52D was obtained by reaction with 52A. m/z: $[M+H]^+$573.2.

Step 5: Using the synthesis method in step 2 of L-14, L-52 was obtained by reaction with 52D. m/z:$[M+H]^+$738.2.

Synthesis of **L-53:** (10S,135)-20-(2,5-dioxo-2,5-dihydro-1H-pyrrol-1-yl)-13-isopropyl-6,9,12,15-tetraoxo-10-(3-ureidopropyl)-3-oxa-5,8,11,14-tetraazaeicosan-1-oic acid

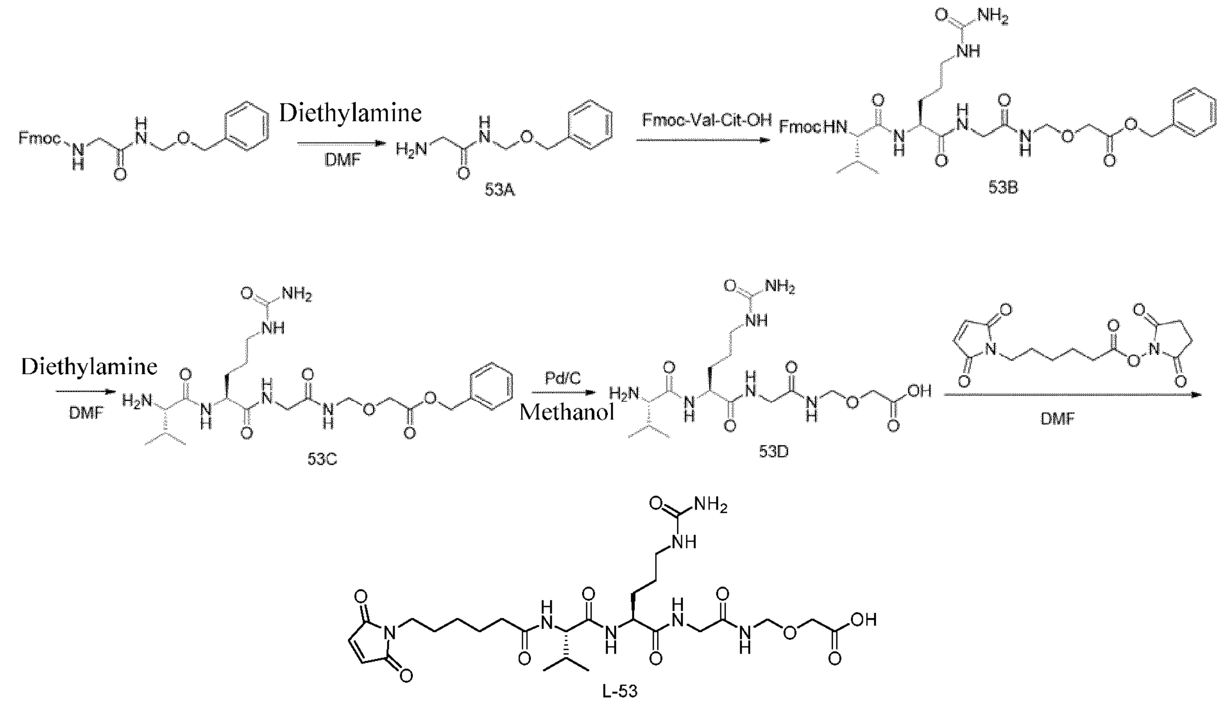


53A 53B 53C 53D L-53

Step 1: Using the synthesis method in step 2 of L-3, 53A was obtained by reaction with (9H-fluoren-9-yl) methyl(2-(((benzyloxy)methyl)amino)-2-oxoethyl)carbamate. m/z: $[M+H]^+$253.2.

Steps 2-3: Using the synthesis method in steps 1-2 of L-3, 53C was obtained by reaction with 53A. m/z: $[M+H]^+$509.2.

Step 4: To a solution of intermediate 53C (30 mg, 0.06 mmol) in methanol (3 mL) was added palladium on carbon (10%wt, 10 mg). The reaction system was replaced with nitrogen and then three times with hydrogen, and then stirred at room temperature for 1 hour under hydrogen atmosphere. The reaction solution was filtered and the filtrate was concentrated directly under reduced pressure to give intermediate 53D (25 mg) as a yellow solid. m/z:$[M+H]^+$419.2.

Step 5: Using the synthesis method in step 3 of L-3, L-53 was obtained by reaction with 53D. m/z: $[M+Na]^+$ 634.2.

Synthesis of **L-54:** (2S,3R,4S,5S,6S)-2-(2-((S)-2-((S)-2-((((9H-fluoren-9-yl)methoxy)carbonyl)amino)-3-methylbutanamido)-5-ureidopentanoylamino)-4-((((4-nitrophenoxy)carbonyl)oxy)methyl)phenoxy)-6-(methoxycarbonyl)tetrahydro-2H-pyran-3,4,5-triyl triacetate

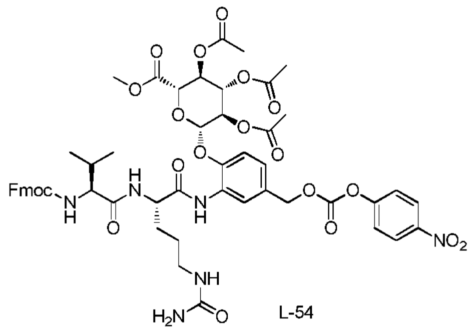

L-54

Using the synthesis method of intermediate L-20, L-54 was synthesized by replacing 2-(6-(2,5-dioxo-2,5-dihydro-1H-pyrrol-1-yl)hexanamido)acetic acid in step 4 with Fmoc-Val-Cit-OH with methyl 1,2,3,4-tetra-O-acetyl-β-D-glucuronate as the starting material. m/z: $[M+H]^+$ 1099.2.

Synthesis of **L-55:** $N^2$-N-((((9H-fluoren-9-yl)methoxy)carbonyl)-L-leucyl)-O-(tert-butyl)-L-serylglycyl-$N^6$-((allyloxy)carbonyl)-L-lysine

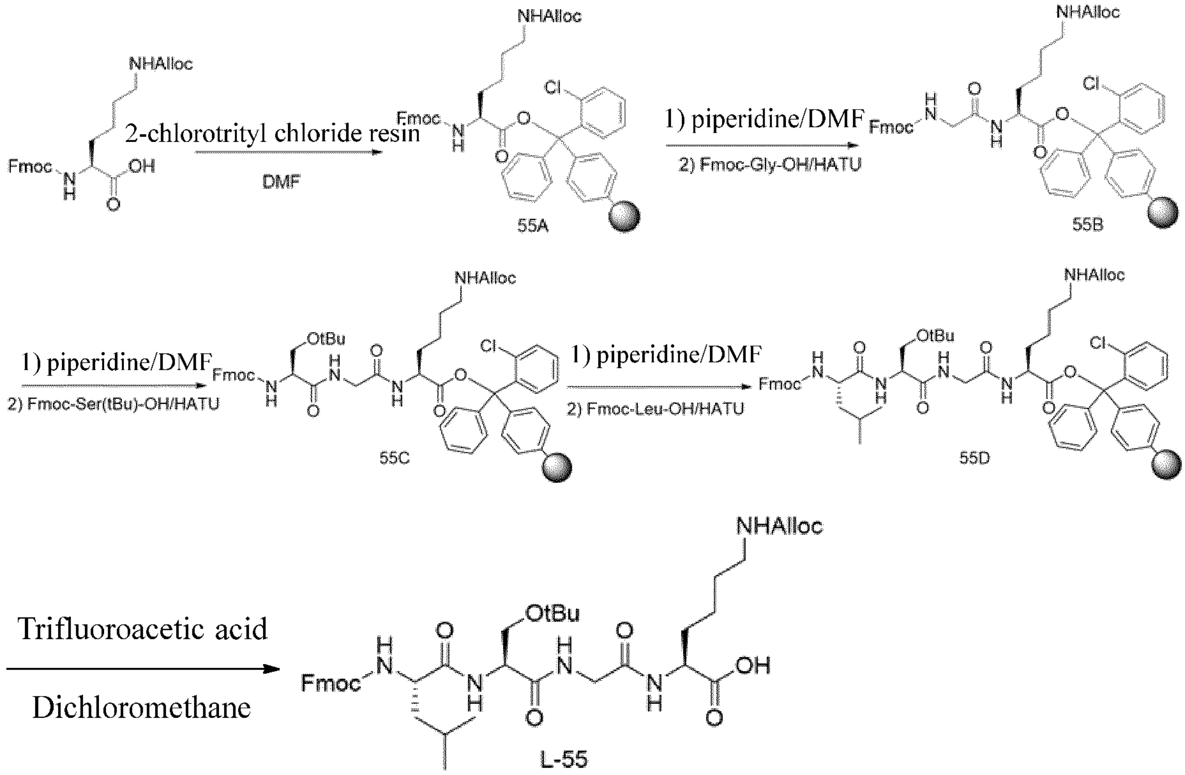

L-55

Step 1: 2-chlorotrityl chloride resin (1.0 g) was weighed and added to the reaction column, then dichloromethane (15 mL) was added until the reaction column swelled for 10 minutes, the waste liquid was drained, and the reaction column was repeatedly washed twice with dichloromethane.

A solution of Fmoc-Lys (Alloc)-OH (1.1 g, 2.5 mmol) and DIPEA (2 mL) in DMF (8 mL) was added to the reaction column obtained in step 1, and the reaction column was placed flat on a shaker for shaking at 150-180 r, and mixed with the resin and allowed to react at room temperature for 2 hours. The reaction solution was removed, the reaction column was washed 2-3 times with DMF and dichloromethane, and the waste liquid was drained until no liquid flowed out. DIPEA (1 mL), methanol (3 mL) and dichloromethane (12 mL) were added for termination and washed to give 55A.

Step 2: A piperidine DMF solution (20%, 10 mL) was added to the reaction column, which was repeated twice for 10 minutes each. DIPEA and dichloromethane (10 mL) were added for washing 3 times and the waste liquid was drained. Fmoc-Gly-OH (742.5 mg, 2.5 mmol) and HATU (1.07 g, 2.75 mmol) were weighed, DIPEA (2 mL) was added, DMF (10 mL) was additionally added to the reaction column, the shaker was shaken, and the reaction was carried out at room temperature for 30 minutes. The reaction solution was removed, the reaction column was washed 3 times with DMF and dichloromethane (10 mL) respectively, and the waste liquid was drained until no liquid flowed out to give 55B.

Step 3: As in Step 2, Fmoc-Gly-OH was replaced with Fmoc-Ser (tBu)-OH to give 55C.

Step 4: As in Step 2, Fmoc-Gly-OH was replaced with Fmoc-Leu-OH to give 55D.

Step 5: The dried reaction resin 55D was added to a cutting solution (trifluoroacetic acid/dichloromethane mixture, 8 mL, 1/100), shaken on a shaker for 30 minutes and filtered, and the resin was washed once with the cutting solution. The filtrates were combined, most of dichloromethane were removed from the filtrates under reduced pressure, and then the filtrates were added to 10 times the volume of ice ether, centrifuged, and washed twice with ether. The solid was dried under vacuum for 2 hours to give L-55 as a white solid. m/z: $[M+H]^+$ 766.4.

Synthesis of **L-56:** allyl((5S,11S,14S)-11-(tert-butoxymethyl)-21-(2,5-dioxo-2,5-dihydro-1H-pyrrol-1-yl)-14-isobutyl-5-((4-((((4-nitrophenoxy)carbonyl)oxy)methyl)phenyl)carbamoyl)-7,10,13,16-tetraoxa-6,9,12,15-tetraazahenicosyl) carbamate

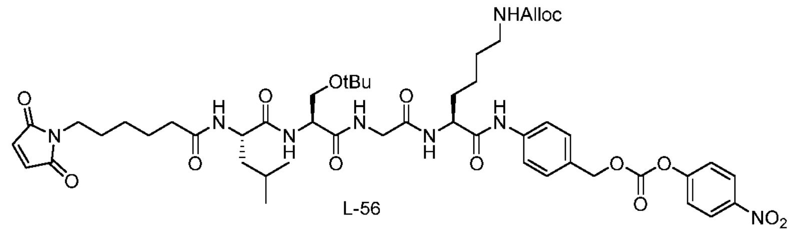

L-56

Using the synthesis method in steps 1-3 and 5 of L-52, L-56 was obtained by reaction with L-55. m/z:[M+H]+1007.3.

Synthesis of **L-57:** 4-((S)-2-((S)-2-((R)-2-((dimethoxyphosphoryl)amino)-3-(2-(2,5-dioxo-2,5-dihydro-1H-pyrrol-1-yl) acetamido)propionylamino)-3-methylbutanoylamino)-3-ureidopentanoylamino)benzyl(4-nitrobenzene)carbonate

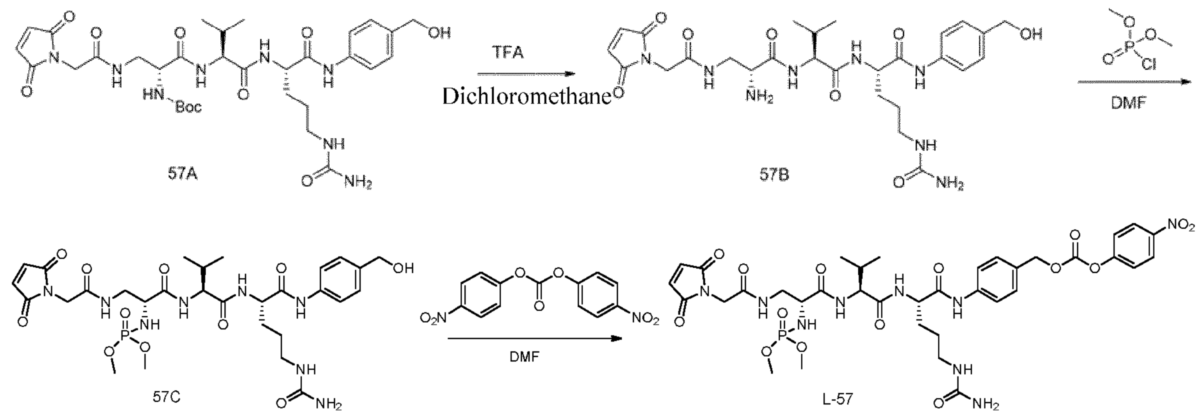


57A 57B 57C L-57

Step 1: To a solution of intermediate 57A (700 mg, 1 mmol) (obtained by reacting L-51 with Val-Cit-PAB-OH using the synthesis method in step 1 of L-5) in dichloromethane (2 mL) was added trifluoroacetic acid (1 mL), the reaction solution was stirred at room temperature for 2 hours and then concentrated directly under reduced pressure, the residue was diluted with methanol and the pH was adjusted to neutral with a methanol solution of ammonia (7 M), and then the residue was purified by *prep*-HPLC (eluent 0-35% acetonitrile in a 0.1% aqueous trifluoroacetic acid solution, 25 minutes) to give intermediate 57B (250 mg) as a yellow solid. m/z:[M+H]+ 603.2.

Step 2: To a solution of intermediate 57B (250 mg, 0.41 mmol) and triethylamine (104 mg, 1.02 mmol) in DMF (8 mL) was added O,O-dimethylphosphoryl chloride (65.2 mg, 0.45 mmol) under ice bath conditions. The reaction solution was stirred at 0°C for 2 hours and concentrated directly under reduced pressure, and the residue was purified by *prep*-HPLC (eluent 10-65% acetonitrile in a 0.1% aqueous trifluoroacetic acid solution, 20 minutes) to give intermediate 57C (200 mg) as a white solid. m/z: [M+H]+711.2.

Step 3: Using the synthesis method in step 2 of L-5 , L-57 was obtained as a white solid by reaction with 57C. m/z: [M+H]+ 876.2.

Synthesis of **L-58:** (9H-fluoren-9-yl)methyl((S)-1-(((S)-1-((4-(aminomethyl)phenyl)amino)-1-oxo-5-ureidopentan-2-yl) amino)-3-methyl-1-oxobutan 2-yl)carbamate

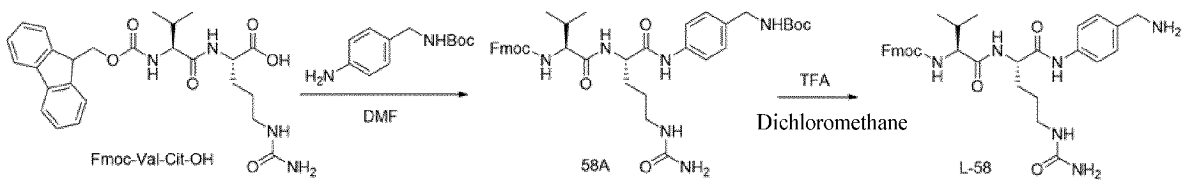


Fmoc-Val-Cit-OH 58A L-58

Step 1: To a solution of Fmoc-Val-Cit-OH (500 mg, 1.01 mmol) and 4-(N-Boc-aminomethyl) aniline (200 mg, 1.01

mmol) in DMF (5 mL) were added EDCI (232 mg, 1.21 mmol) and HOBT (164 mg, 1.21 mmol), and the reaction solution was stirred at room temperature for 1 hour. The reaction solution was dropped into water (40 mL) and stirred for 20 minutes, and then the precipitated solid was filtered, filtered, and the filter cake was dried to give intermediate 58A (1 g) as a yellow solid. m/z: $[M+H]^+$701.4.

Step 2: To a solution of intermediate 58A (350 mg, 0.5 mmol) in dichloromethane (3 mL) was added trifluoroacetic acid (1 mL), the reaction solution was stirred at room temperature for 2 hours and then concentrated directly under reduced pressure, the residue was concentrated under reduced pressure after being adjusted to pH = 7 with an ammonia methanol (7 M) solution, and the residue was purified by Flash column chromatography (C18, eluent 0%-50% acetonitrile in a 10 mM aqueous ammonium bicarbonate solution) to give L-58 (60 mg) as a white solid. m/z:$[M+H]^+$ 601.4.

Synthesis of **L-59:** $N^2$-(((9H-fluoren-9-yl)methoxy)carbonyl)-L-alanyl-L-alanyl-$N^4$-trityl-L-asparagine

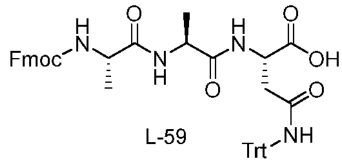

L-59

Using the L-55 solid phase synthesis method, the 2-chlorotrityl chloride resin was pretreated by the method in step 1, then the method in step 2 was repeated to add Fmoc-Asn(Trt)-OH, Fmoc-Ala-OH and Fmoc-Ala-OH sequentially, and then the cutting method in step 5 of L-55 was followed to give L-59 as a white solid. m/z:$[M+Na]^+$ 761.3.

Synthesis of **L-60:** (10S,13S)-20-(2,5-dioxo-2,5-dihydro-1H-pyrrol-1-yl)-13-isopropyl-6,9,12,15-tetraoxo-10-(3-ureidopropyl)-3-oxa-5,8,11,14-tetraazaeicosan-1-oic acid

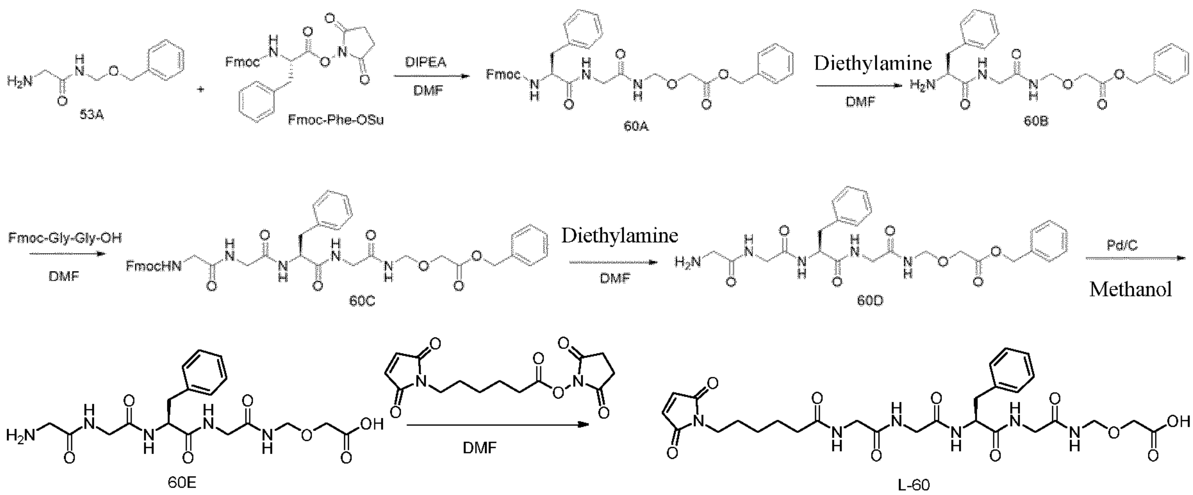


Step 1: To a solution of intermediate 53A (150 mg, 0.59 mmol) and Fmoc-Phe-OSu (314 mg, 0.65 mmol) in DMF (3 mL) was added DIPEA (114 mg, 0.89 mmol). The reaction solution was stirred at room temperature for 1 hour and then directly purified by *prep*-HPLC (eluent 10%-75% acetonitrile in a 0.1% aqueous trifluoroacetic acid solution, 20 minutes) to give intermediate 60A (280 mg) as a white solid. m/z:$[M+Na]^+$ 644.2.

Steps 2-6: Using the synthesis method of L-53, L-60 was obtained by reaction with 60A. m/z:$[M+Na]^+$639.2.

Synthesis of **L-61:** (9H-fluoren-9-yl)methyl((S)-1-(((S)-1-((3-(aminomethyl)phenyl)amino)-1-oxo-5-ureidopentan-2-yl) amino)-3-methyl-1-oxobutan-2-yl)carbamate

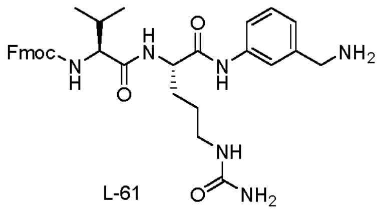

L-61

Using the synthesis method of L-58, L-61 was obtained as a white solid by reacting Fmoc-Val-Cit-OH with 3-(N-Boc-aminomethyl)aniline. m/z:$[M+H]^+$ 601.3.

Synthesis of **L-62:** (S)-11-benzyl-1-(9H-fluoren-9-yl)-3,6,9,12,15-pentaoxo-2,18,21,24-tetraoxa-4,7,10,13,16-pentaazaheptacosan-27-oic acid

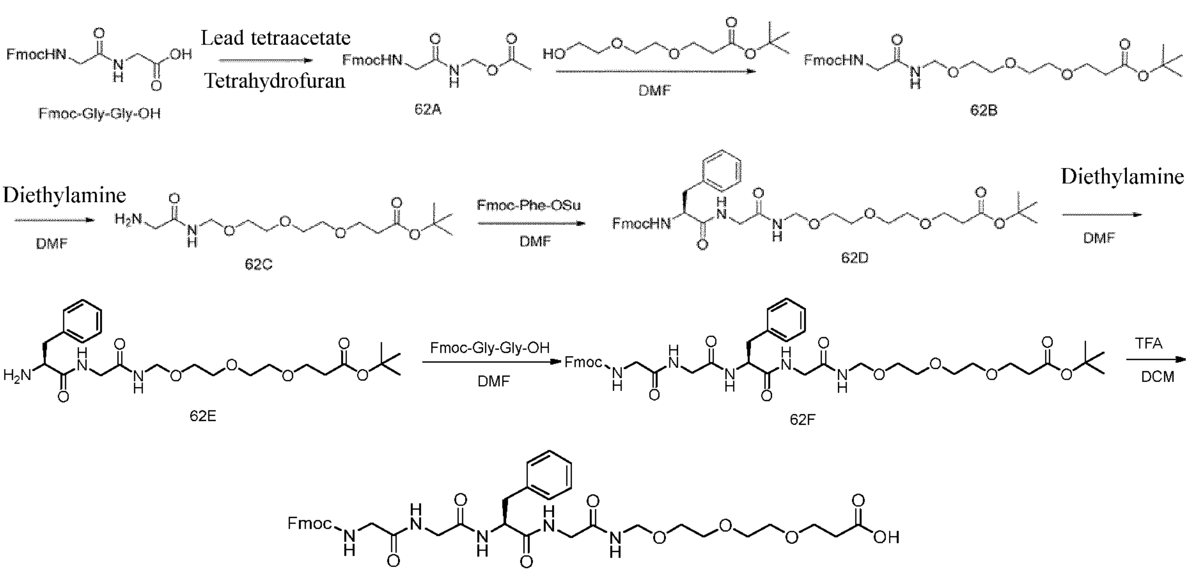


L-62

Step 1: A solution of Fmoc-Gly-Gly-OH (10 g, 28.2 mmol) and lead tetraacetate (17.5 g, 39.5 mmol) in tetrahydrofuran (240 mL) and toluene (80 mL) was stirred at 80°C for 2 hours. The reaction solution was filtered, the filtrate was concentrated under reduced pressure, the residue was triturate with an ethyl acetate/methyl tert-butyl ether solution (20%, 300 mL) and filtered, and the filter cake was dried in vacuum to give intermediate 62A (9.5 g) as a white solid. m/z: $[M+Na]^+$ 391.2.

Steps 2-3: Using the synthesis method in steps 1-2 of L-60, 62C was obtained as a colorless liquid by reaction with 62A. m/z:$[M+H]^+$ 321.2.

Steps 4-6: Using the synthesis method in steps 1-3 of L-60, 62F was obtained as a colorless liquid by reaction with 62C. m/z:$[M+Na]^+$ 826.3.

Step 7: A mixed solution of trifluoroacetic acid (2 mL) and dichloromethane (10 mL) was slowly added to a solution of intermediate 62F (400 mg, 0.5 mmol) in dichloromethane (10 mL) under ice bath conditions. The reaction solution was stirred at room temperature for 1 hour and then concentrated directly under reduced pressure to give L-62 (350 mg) as a colorless oil. m/z: $[M+Na]^+$ 770.2.

Synthesis of **L-63:** (4S,7S)-1-(4-(2,5-dioxo-2,5-dihydro-1H-pyrrol-1-yl)phenyl)-4-isopropyl-2,5,8-trioxo-7-(3-ureidopropyl)-12,15,18,21,24,27-hexaoxa-3,6,9-triazatriacontan-30-oic acid

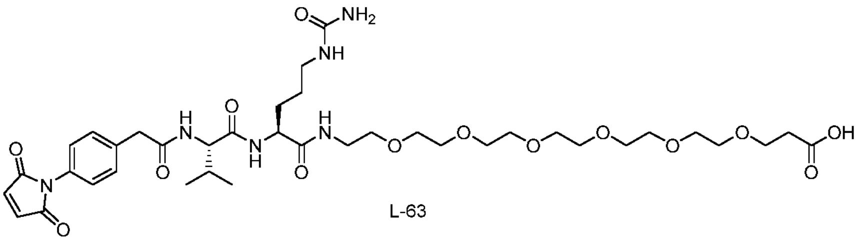

L-63

Using the synthesis method of L-7, L-63 was synthesized from 4-((2,5-dioxo-2,5-dihydro-1H-pyrrol-1-yl)methyl) benzoic acid and intermediate 3B. m/z: $[M+H]^+$ 823.4.

Synthesis of **L-64:** 4-((26S,29S)-1-(4-(2,5-dioxo-2,5-dihydro-1H-pyrrol-1-yl) phenyl)-26-isopropyl-2,24,27-trioxo-29-(3-ureidopropyl)-6,9,12,15,18,21-hexaoxa-3,25,28-triazatriacontane-30-amido)benzyl(4-nitrobenzene)carbonate

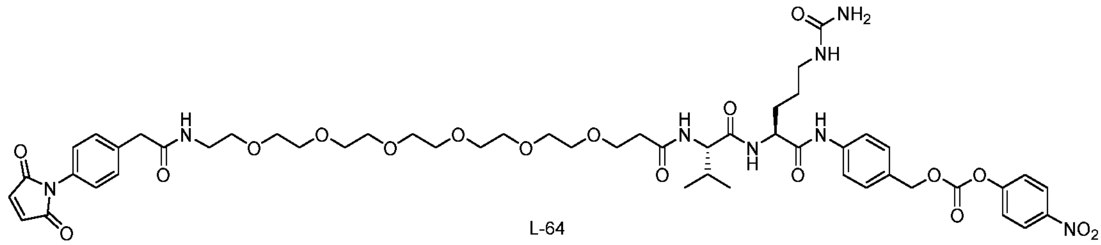

L-64

Using the synthesis method of L-5, L-64 was synthesized from Val-Cit-PAB-OH and L-35. m/z: $[M+H]^+$ 1093.5.

Synthesis of **L-65:** 4-((25S,28S)-1-(2-((2,5-dioxo-2,5-dihydro-1H-pyrrol-1-yl)methyl)-1,3-dioxan-5-yl)-25-isopropyl-1,23,26-trioxo-28-(3-ureidopropyl)-5,8,11,14,17,20-hexaoxa-2,24,27-triazanonacosane-29-amido)benzyl(4-nitrobenzene)carbonate

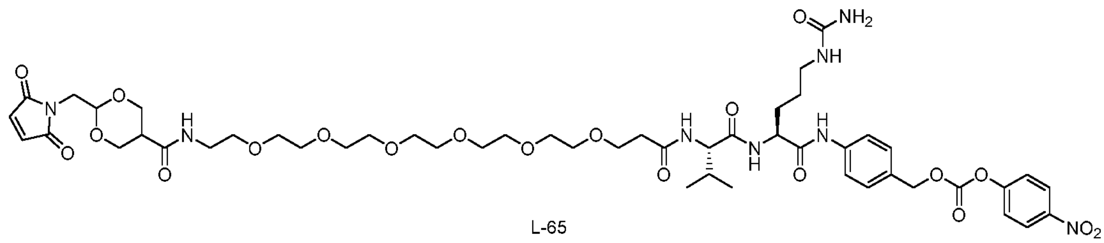

L-65

Using the synthesis method of L-5, L-65 was synthesized from Val-Cit-PAB-OH and L-42. m/z: $[1/2M+H]^+$ 552.3.

Synthesis of **L-66:** (2-(4-(2,5-dioxo-2,5-dihydro-1H-pyrrol-1-yl)phenyl)ethylacyl)glycylglycyl-L-phenylalanylglycine

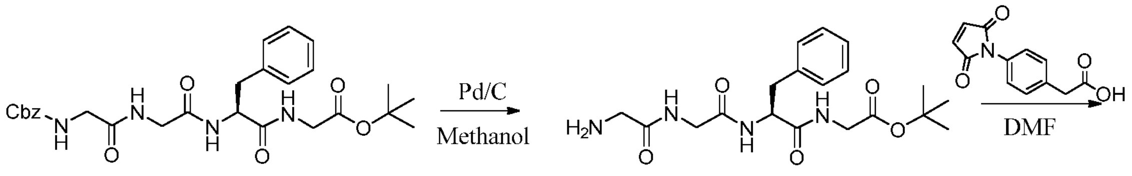


Cbz-Gly-Gly-Phe-Gly-OtBu 66A

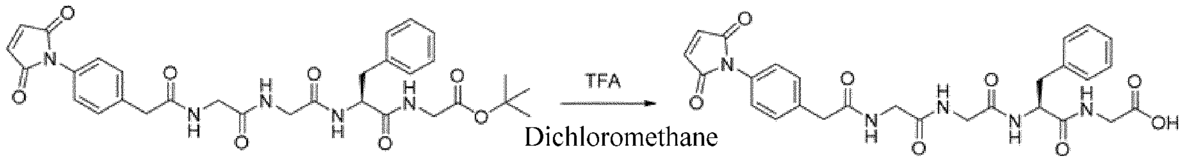


66B L-66

Step 1: To a mixed solution of Cbz-Gly-Gly-Phe-Gly-OtBu (350 mg, 0.66 mmol) in methanol (15 mL) and dichloromethane (5 mL) was added palladium on carbon (70 mg, 10%wt). The reaction system was replaced with hydrogen three times and then stirred under hydrogen atmosphere for 2 hours, the reaction solution was filtered through diatomaceous earth, and the filtrate was concentrated under reduced pressure to give 66A (250 mg) as a yellow oil. m/z: $[M+H]^+$ 393.2.

Steps 2-3: Using the synthesis method of L-58, L-66 was obtained by reaction with 66A. m/z: $[M+H]^+$ 550.2.

Synthesis of **L-67:** tert-butyl(S)-4-(6-(2,5-dioxo-2,5-dihydro-1H-pyrrol-1-yl)hexanoylamino)-5-(((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)amino)-5-oxopentanoate

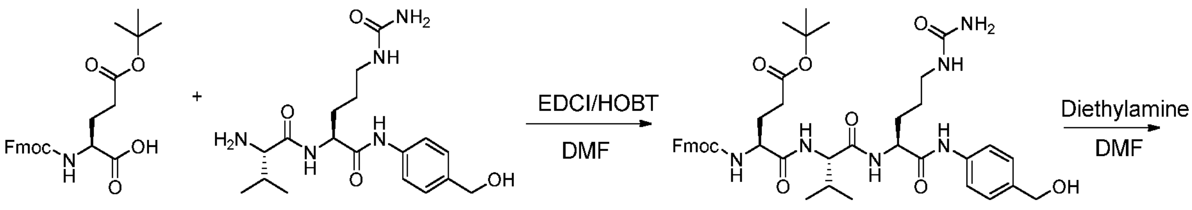


Fmoc-Glu(OtBu)-OH Val-Cit-PAB-OH 67A

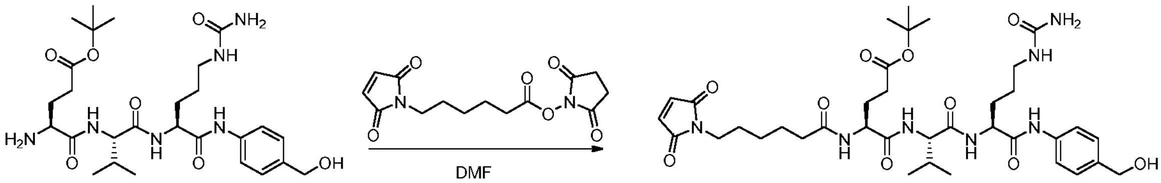


67B 67C

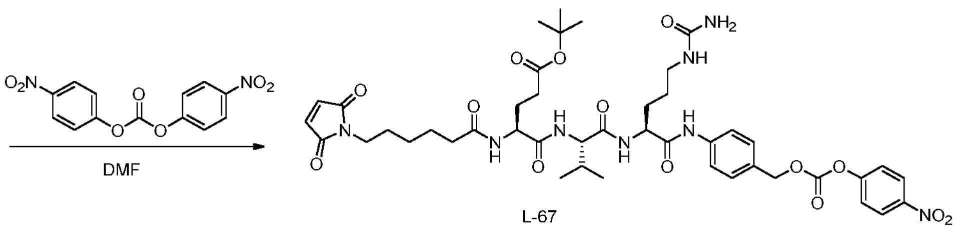


L-67

Steps 1-2: To a solution of Fmoc-Glu (OtBu)-OH (1.0 g, 2.35 mmol) and Val-Cit-PAB-OH (0.89 g, 2.35 mmol) in DMF (10 mL) were added EDCI (0.68 g, 3.53 mmol) and HOBT (0.48 g, 3.53 mmol) under ice bath conditions, and the reaction system was stirred at room temperature for 2 hours. To the above mixture was added diethylamine (2 mL), and the reaction system was stirred at room temperature for additional 1 hour. The reaction solution was directly purified by Flash column chromatography (C18, eluent 0%-35% acetonitrile in a 0.05% aqueous trifluoroacetic acid solution) to give

Glu(OtBu)-Val-Cit-PAB-OH (67B, 1.02 g) as a white solid. m/z:$[M+H]^+$ 565.4.

Step 3: Using the synthesis method in step 3 of L-3, 67C was obtained by reaction with 67B. m/z: $[M+H]^+$ 758.4.

Step 4: Using the synthesis method in step 2 of L-5, L-67 was obtained by reaction with 67C. m/z: $[M+H]^+$ 923.4.

Synthesis of **L-68:** 4-((S)-4-amino-2-((S)-2-((S)-2-(2-(4-(2,5-dioxo-2,5-dihydro-1H-pyrrol-1-yl)phenyl)acetamido)propionamido)propionamido)-4-oxobutanamido)benzyl(4-nitrobenzene)carbonate

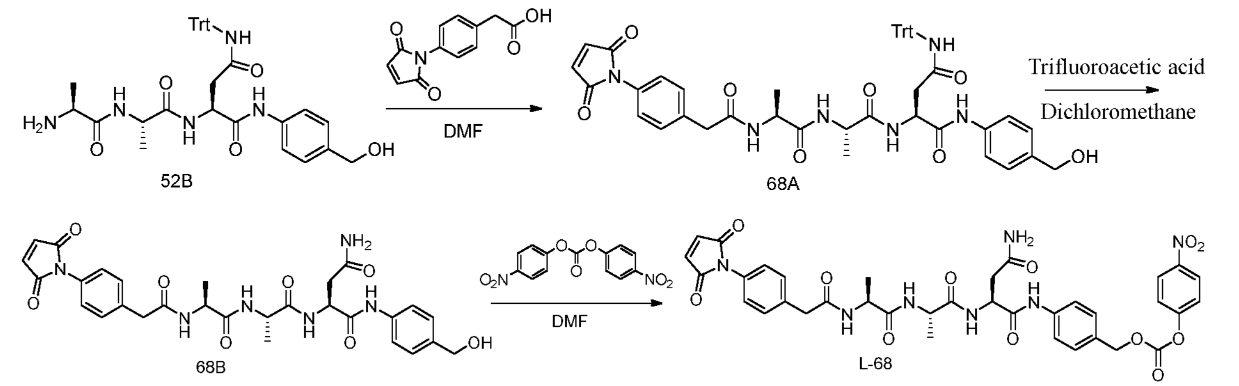

52B 68A 68B L-68

Step 1: To a solution of 52B (153 mg, 0.25 mmol) in DMF (2 mL) were added EDCI (71.9 mg, 0.38 mmol) and HOBT (50.7 mg, 0.38 mmol) sequentially under ice bath conditions, and a solution of 4-((2,5-dioxo-2,5-dihydro-1H-pyrrol-1-yl)methyl)benzoic acid (57.8 mg, 0.25 mmol) in DMF (0.4 mL) was then slowly added dropwise. The reaction solution was stirred at room temperature for 5 hours and directly purified by Flash column chromatography (C18, eluent 0%-57% acetonitrile in a 0.05% aqueous trifluoroacetic acid solution) to give 68A (40 mg) as a white solid. m/z:$[M+H]^+$ 835.3.

Steps 2-3: Using the synthesis method in steps 4-5 of L-52, L-68 was obtained by reaction with 68A. m/z: $[M+H]^+$ 758.2.

L-69: (Z)-3,6-dioxo-2,10,13,16,19,22,25-heptaoxa-7-azaoctacosa-4-en-28-oic acid

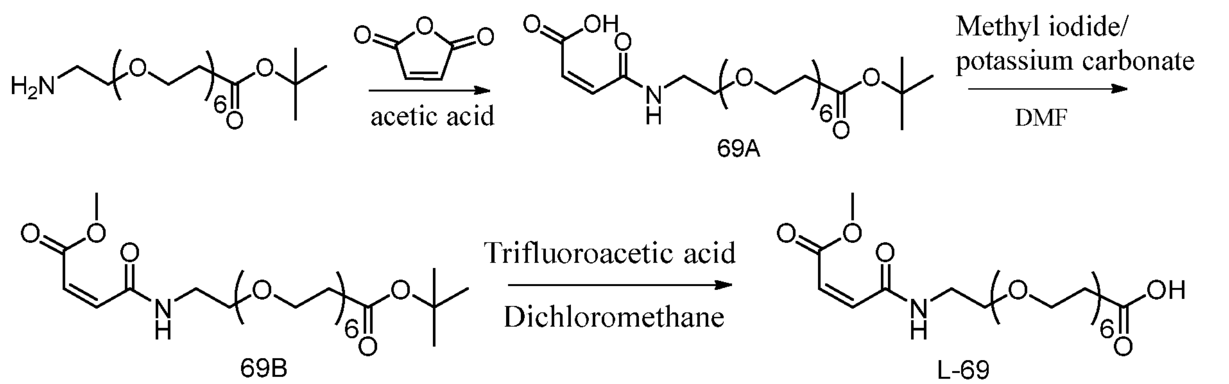

69A 69B L-69

Step 1: To a solution of tert-butyl 1-amino-3,6,9,12,15,18-hexaoxaeicosane-21-acid (300 mg, 0.85 mmol) in acetic acid (5 mL) was added 2,5-dihydrofuran-2,5-dione (125 mg, 1.27 mmol) under ice bath conditions. After the addition was completed, the reaction solution was stirred at room temperature for 2 hours and concentrated under reduced pressure. The residue was purified by Flash column chromatography (C18, eluent 0-40% acetonitrile in a 0.1% aqueous trifluoroacetic acid solution) to give 69A (400 mg) as a white solid. m/z: $[M+H]^+$ 508.2.

Step 2: To a solution of 69A (350 mg, 0.69 mmol) and potassium carbonate (190 mg, 1.38 mmol) in DMF (5 mL) was added iodomethane (147 mg, 1.03 mmol) dropwise under ice bath conditions. The reaction solution was stirred at room temperature for 3 hours and directly purified by Flash column chromatography (C18, eluent 0%-45% acetonitrile in a 0.1% aqueous trifluoroacetic acid solution) to give 69B (300 mg) as a white solid. m/z:$[M+H]^+$ 522.2.

Step 3: A mixed solution of 69B (300 mg, 0.58 mmol) in dichloromethane (8 mL) and trifluoroacetic acid (3 mL)

was stirred at room temperature for 2 hours under ice bath conditions. The reaction solution was concentrated directly under reduced pressure, and the residue was purified by Flash column chromatography (C18, eluent 0%-30% acetonitrile in a 0.1% aqueous trifluoroacetic acid solution) to give L-69 (120 mg) as a white solid. m/z:[M+H]+ 466.2.

Synthesis of **L-70:** tert-butyl(S)-1-(4-(2,5-dioxo-2,5-dihydro-1H-pyrrol-1-yl) phenyl)-26-(((S)-3-methyl-1-(((S)-1-((4-((((4-nitrophenoxy)carbonyl)oxy)methyl)phenyl)amino)-1-oxopropan-2-yl)amino)-1-oxobutan-2-yl) carbamoyl)-2,24-dioxo-6,9,12,15,18,21-hexaoxa-3,25-diazanonacosan-29-oate

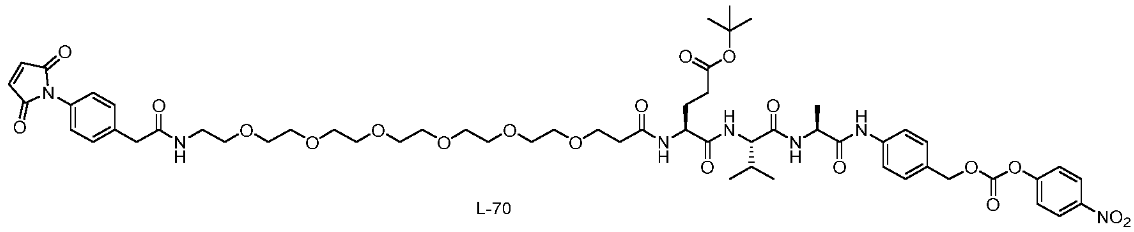

L-70

Synthesis of intermediate 70A: Using the synthesis method of intermediate 67B, Glu(OtBu)-Val-Ala-PAB-OH (Intermediate 70A) was obtained as a white solid by reacting Fmoc-Glu(OtBu)-OH with Val-Ala-PAB-OH. m/z:[M+H]+479.2.

Using the synthesis method of L-5, L-70 was synthesized from intermediates 70A and L-35. m/z:[M+H]+ 1192.4.

Synthesis of **L-71:** 4-((26S,29S,32S)-1-(4-(2,5-dioxo-2,5-dihydro-1H-pyrrol-1-yl)phenyl)phenyl)-26,29-dimethyl-2,24,27,3 0-tetrahydrooxo-3 2-(2-oxo-2-(tiitylamino)ethyl)-6,9,12,15,18,21-hexaoxa-3,25,28,31-tetraazatritriacontane-33-amido)benzyl(4-nitrophenyl)carbonate

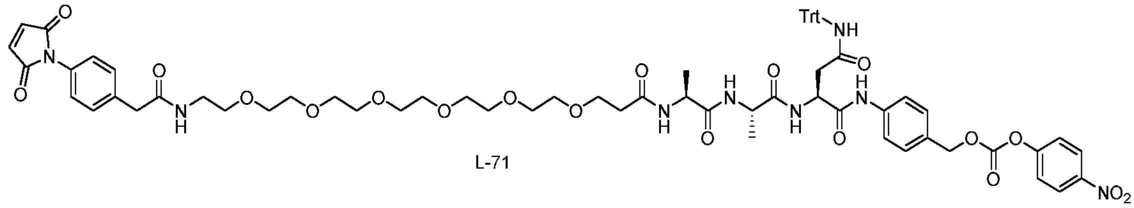

L-71

Using the synthesis method of L-5, L-71 was synthesized from intermediates 52B and L-35. m/z:[M+H]+ 1192.4.

Synthesis of **L-72:** (2-((2,5-dioxo-2,5-dihydro-1H-pyrrol-1-yl)methyl)-1,3-dioxan-5-carbonyl)glycine

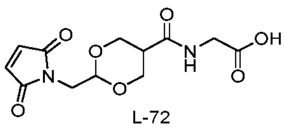

L-72

Using the synthesis method of L-7, L-72 was synthesized from L-41 and tert-butyl glycine ester. m/z: [M+H]+ 299.0.

Synthesis of **L-73:** 1-(9H-fluoren-9-yl)-3,10-dioxo-2,14,17,20,23,26,29-heptaoxa-4,11-diazadotriacontan-32-oic acid

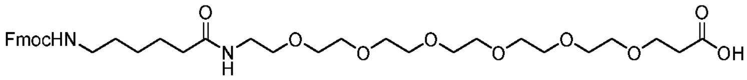

L-73

Using the synthesis method of L-7, L-73 was synthesized from tert-butyl 1-amino-3,6,9,12,15,18-hexaoxa-21-acid and 6-((((9H-fluoren-9-yl)methoxy)carbonyl)amino)hexanoic acid. m/z:[M+H]+ 688.9.

Synthesis of **L-74:** 3-(2-(2-(6-(11,12-didehydrodibenzo[b,f]azacyclooct-5(6H)-yl)-6-oxohexanamido)ethoxy)ethoxy) propionic acid

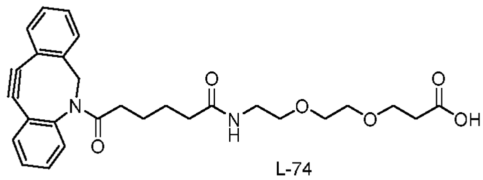

L-74

Using the synthesis method of L-7, L-74 was synthesized from DBCO-$C_6$-COOH (CAS: 1425485-72-8) and tert-butyl 3-(2-(2-aminoethoxy)ethoxy)propionate. m/z: [M+H]+ 493.2.

Synthesis of **L-75:** 2-(4-(2,5-dioxo-2,5-dihydro-1H-pyrrol-1-yl)phenyl)-1,3-dioxan-5-carboxylic acid

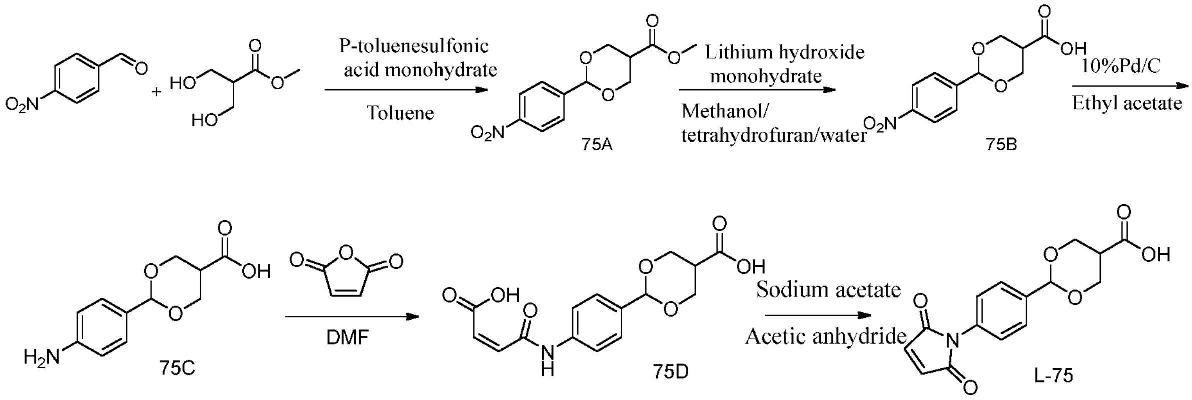


Step 1: To a solution of methyl 3-hydroxy-2-(hydroxymethyl) propionate (1.5 g, 11.2 mmol) and 4-nitrobenzaldehyde (1.69 g, 11.2 mmol) in toluene (50 mL) was added p-toluenesulfonic acid monohydrate (210 mg, 1.12 mmol). The reaction solution was stirred at 110°C for 2 hours, then cooled to room temperature and filtered, the filtrate was concentrated under reduced pressure, and the residue was purified by Flash column chromatography (petroleum ether/ethyl acetate = 1/1) to give 75A (1.5 g) as a light yellow solid. m/z:[M+H]+ 268.2.

Step 2: To a mixed solution of 75A (1.4 g, 5.24 mmol) in tetrahydrofuran (20 mL) and water (10 mL) was added lithium hydroxide monohydrate (440 mg, 10.5 mmol), the reaction solution was stirred at room temperature for 2 hours and concentrated under reduced pressure, the residue was adjusted to pH = 3 with hydrochloric acid (2 M) and filtered, and the filter cake was dried in vacuum to give 75B (1.42 g) as a light yellow solid. m/z:[M+H]+ 254.1.

Step 3: To a solution of 75B (300 mg, 1.18 mmol) in ethyl acetate (20 mL) was added palladium on carbon (10%wt, 299 mg). After the reaction system was replaced with hydrogen, it was stirred at room temperature for 1 hour under hydrogen atmosphere and filtered, and the filtrate was concentrated under reduced pressure to give 75C (300 mg) as a yellow solid. m/z:[M+H]+ 224.2.

Step 4: To a solution of 75C (300 mg, 1.34 mmol) in DMF (3 mL) was added 2,5-dihydrofuran-2,5-dione (171 mg, 1.74 mmol). The reaction solution was stirred at room temperature for 1 hour and concentrated under reduced pressure, and the residue was purified by Flash column chromatography (C18, eluent 10%-60% acetonitrile in a 0.1% aqueous trifluoroacetic acid solution) to give 75D (150 mg) as an orange solid. m/z:[M+H]+ 322.2.

Step 5: To a solution of 75D (150 mg, 0.47 mmol) in acetic anhydride (480 mg, 4.7 mmol) was added sodium

acetate (38.6 mg, 0.47 mmol). The reaction solution was stirred at 50°C for 0.5 h, and the reaction system was cooled to room temperature and then directly purified by Flash column chromatography (C18, eluent 10%-60% acetonitrile in a 0.1% aqueous trifluoroacetic acid solution) to give L-75 (30 mg) as a yellow solid. m/z:$[M+H]^+$ 304.2.

Synthesis of **L-76:** (32S,35S,38S)-32-(2-(4-(2,5-dioxo-2,5-dihydro-1H-pyrrol-1-yl)phenyl)acetylamino)-35-isopropyl-38-methyl-26,33,36,39,42-pentaoxo-2,5,8,11,14,17,20,23,45-nonaoxa-27,34,37,40,43-pentaazaheptateteracontan-47-oic acid

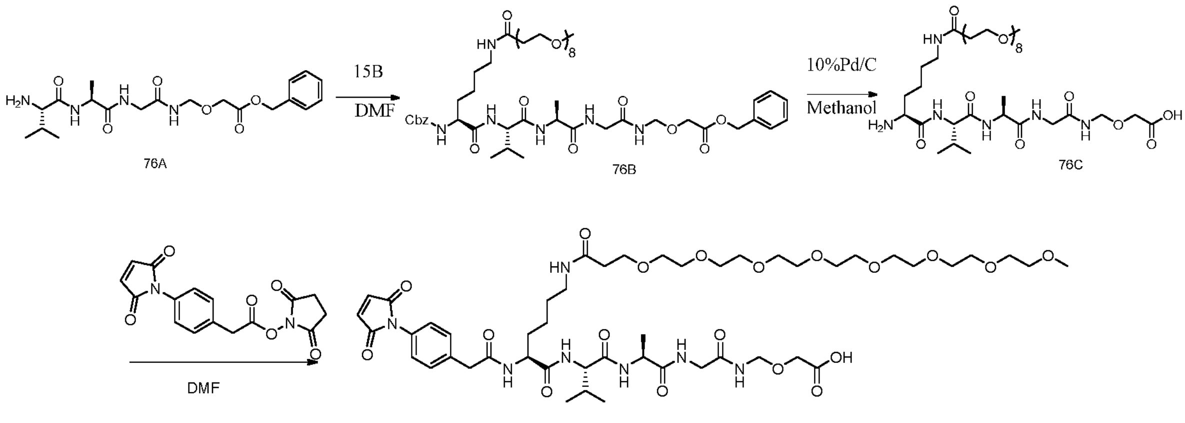


76A 76B 76C

L-76

Step 1: To a solution of 15B (195 mg, 0.29 mmol) and 76A (replacing Fmoc-Val-Cit-OH with Fmoc-Val-Ala-OH with reference to the synthesis method of intermediate 53C) (123 mg, 0.29 mmol) in DMF (3 mL) were added HATU (132 mg, 0.35 mmol) and DIPEA (56.2 mg, 0.43 mmol) under ice bath conditions, and the reaction system was stirred at 0°C for 1 hour. The reaction solution was directly purified by Flash column chromatography (C18, eluent 0%-52% acetonitrile in a 0.05% aqueous trifluoroacetic acid solution) to give 76B (177 mg) as a white solid. m/z:$[\ 1M+H]^+$ 1079.5.

Step 2: To a solution of 76B (100 mg, 0.093 mmol) in methanol (5 mL) was added palladium on carbon (29.7 mg, 10%wt), and the reaction system was stirred under hydrogen atmosphere for 4 hours. The reaction mixture was then filtered through diatomaceous earth and concentrated under reduced pressure to give 76C (76.4 mg) as a white solid. m/z:$[M+H]^+$855.6.

Step 3: To a solution of 76C (66 mg, 0.077 mmol) in anhydrous DMF (2 mL) were added 2-(4-maleimidophenyl) acetic acid(N-hydroxysuccinimide)ester (27.8 mg, 0.085 mmol) and DIPEA (19.9 mg, 0.15 mmol) under ice bath conditions, and the reaction system was stirred at room temperature for 1 hour. The reaction solution was directly purified by Flash column chromatography (C18, eluent 0%-41% acetonitrile in a 0.05% aqueous trifluoroacetic acid solution) to give L-76 (18.2 mg) as a white solid. m/z:$[M+Na]^+$ 1090.5.

Synthesis of **L-77:** 2-(4-(3-methylbenzenesulfonyl-2-(methylbenzenesulfonylmethyl)propionyl)phenyl)-1,3-dioxan-5-carboxylic acid

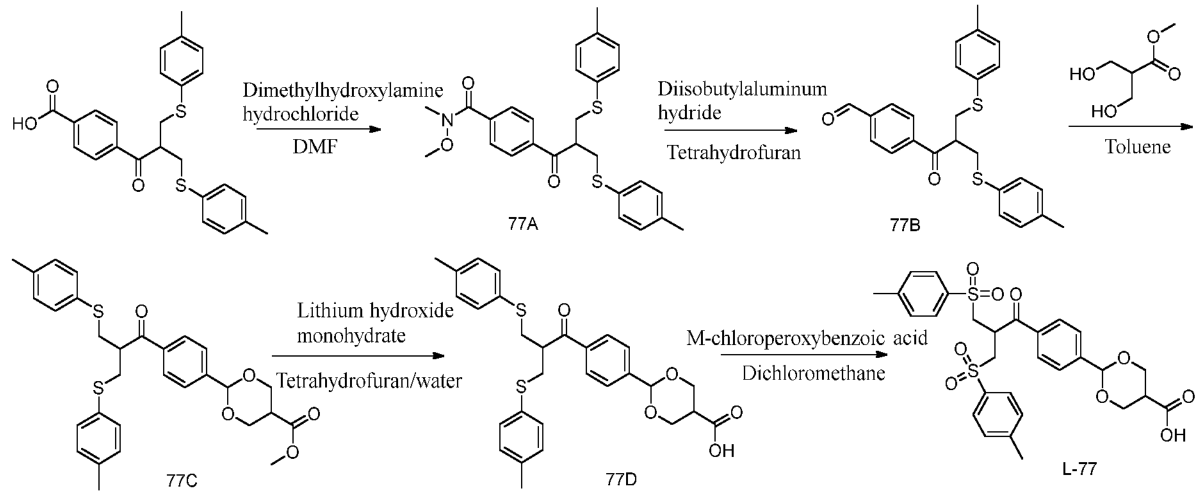


77A 77B 77C 77D L-77

Step 1: To a solution of 4-(3-(p-tolylmercapto)-2-((p-tolylmercapto)methyl)propionyl)benzoic acid (CAS: 124242-93-9) (1 g, 2.29 mmol) and dimethylhydroxylamine hydrochloride (335 mg, 3.44 mmol) in DMF (1 mL) were added HATU (1.1 g, 2.98 mmol) and DIPEA (592 mg, 4.58 mmol). The reaction solution was stirred at room temperature for 1 hour, the reaction was quenched by adding water, the aqueous phase was extracted with ethyl acetate, the organic phases were combined and concentrated under reduced pressure, and the residue was purified by Flash column chromatography (petroleum ether/ethyl acetate = 4/1) to give 77A (1.1 g) as a colorless oil. m/z:$[M+H]^+$ 480.2.

Step 2: To a solution of 77A (620 mg, 1.29 mmol) in tetrahydrofuran (8 mL) was added diisobutylaluminum hydride (290 mg, 2.06 mmol) at -78°C under nitrogen atmosphere. The reaction solution was stirred at -78°C for 1 hour and slowly warmed to room temperature, the reaction was quenched with a saturated aqueous ammonium chloride solution, and the aqueous phase was extracted with ethyl acetate. The organic phases were combined and concentrated under reduced pressure, and the residue was purified by Flash column chromatography (petroleum ether/ethyl acetate = 2/1) to give 77B (420 mg) as a yellow oil. m/z:$[M+H]^+$ 421.0.

Step 3: To a solution of 77B (320 mg, 0.76 mmol) and methyl 3-hydroxy-2-(hydroxymethyl) propionate (133 mg, 0.99 mmol) in toluene (20 mL) was added p-toluenesulfonic acid monohydrate (14.5 mg, 0.08 mmol). The reaction solution was stirred at 110°C for 2 hours, cooled to room temperature, and then concentrated under reduced pressure. The residue was purified by Flash column chromatography (petroleum ether/ethyl acetate = 4/1) to give 77C (450 mg) as a light yellow solid. m/z:$[M+H]^+$ 537.2.

Step 4: To a mixed solution of 77C (320 mg, 0.6 mmol) in tetrahydrofuran (2 mL) and water (1 mL) was added lithium hydroxide monohydrate (32.7 mg, 0.78 mmol) under ice bath conditions. The reaction solution was stirred at 0°C for 1 hour, adjusted to pH = 3 with hydrochloric acid (1M), and then directly purified by Flash column chromatography (C18, eluent 10%-95% acetonitrile in a 0.05% aqueous trifluoroacetic acid solution) to give 77D (40 mg) as a white solid. m/z:$[M+H]^+$ 523.2.

Step 5: To a solution of 77D (120 mg, 0.23 mmol) in dichloromethane (10 mL) was added m-chloroperoxybenzoic acid (234 mg, 1.15 mmol) under ice bath conditions. The reaction solution was stirred at room temperature for 1 hour and then concentrated at low temperature under reduced pressure. The residue was purified by Flash column chromatography (C18, eluent 10%-60% acetonitrile in a 0.05% aqueous trifluoroacetic acid solution) to give L-77 (120 mg) as a white solid. m/z:$[M+H]^+$ 587.2.

Synthesis of L-78: 6-(3,5-dichloro-2,6-bis(methylsulfonyl)isonicotinamido)hexanoic acid

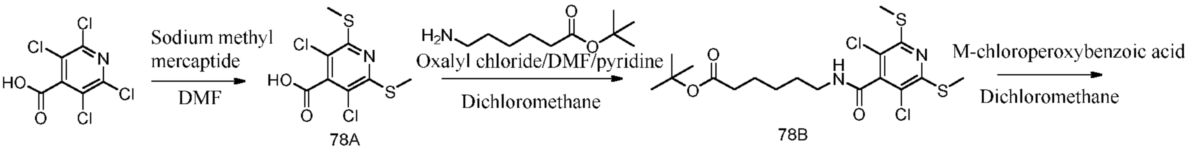

78A 78B

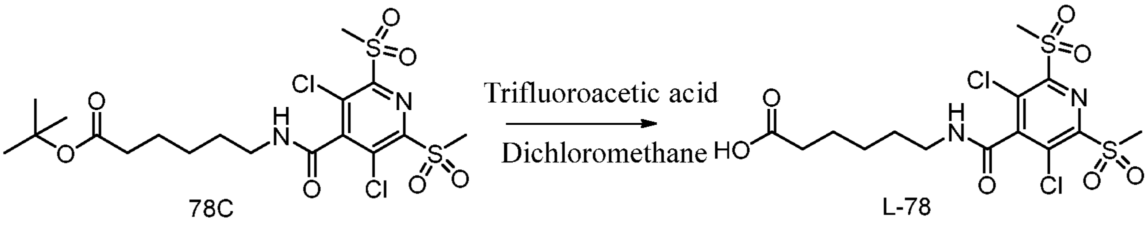

78C L-78

Step 1: To a solution of 2,3,5,6-tetrachloroisonicotinic acid (1 g, 3.83 mmol) in DMF (10 mL) was added sodium methyl mercaptide (670 mg, 9.57 mmol) in portions, and the reaction solution was stirred at 50°C overnight under nitrogen atmosphere, and then directly purified by Flash column chromatography (C18, eluent 0%-55% acetonitrile in a 0.05% aqueous trifluoroacetic acid solution) to give 78A (480 mg) as a white solid. m/z:[M+H]$^+$ 284.0.

Step 2: To a solution of 78A (75.4 mg, 0.27 mmol) in dichloromethane (3 mL) were added DMF (4 mg, 0.054 mmol) and oxalyl chloride (41.1 mg, 0.32 mmol) sequentially under ice bath conditions, and the reaction solution was stirred at room temperature for 2 hours. After the reaction solution was cooled to 0°C, pyridine (70.5 mg, 0.89 mmol) and tert-butyl 6-aminocaproate (55.6 mg, 0.3 mmol) were added, the reaction solution was stirred at room temperature for 2 hours, saturated aqueous sodium bicarbonate solution was added to quench the reaction, the aqueous phase was extracted with ethyl acetate, the organic phases were combined and concentrated under reduced pressure, and the residue was purified by Flash column chromatography (petroleum ether/ethyl acetate = 2/1) to give 78B (42.3 mg) as a white solid. m/z:[M+Na]$^+$ 475.0.

Step 3: To a solution of 78B (42.3 mg, 0.093 mmol) in dichloromethane (10 mL) was added m-chloroperoxybenzoic acid (113 mg, 0.56 mmol) under ice bath conditions. The reaction solution was stirred at room temperature for 2 days, ice water was added to quench the reaction, the aqueous phase was extracted with dichloromethane, and the organic phases were combined and concentrated under reduced pressure to give 78C (48 mg) as a white solid. m/z: [M+Na]$^+$ 539.0.

Step 4: To a solution of 78C (48 mg, 0.093 mmol) in dichloromethane (10 mL) was added trifluoroacetic acid (0.5 mL) under ice bath conditions. The reaction solution was stirred at 0°C for 2 hours and then directly purified by Flash column chromatography (C18, eluent 0%-40% acetonitrile in a 0.05% aqueous trifluoroacetic acid solution) to give L-78 (37.5 mg) as a white solid. m/z:[M+H]$^+$ 461.0.

Synthesis of **L-79:** 4-((S)-2-((S)-2-(2-(4-(3-bromo-2,5-dioxo-2,5-dihydro-1H-pyrrol-1-yl)phenyl)acetamido)-3-methylbutanoylamino)-5-ureidopentanoylamino)benzyl(4 nitrobenzene)carbonate

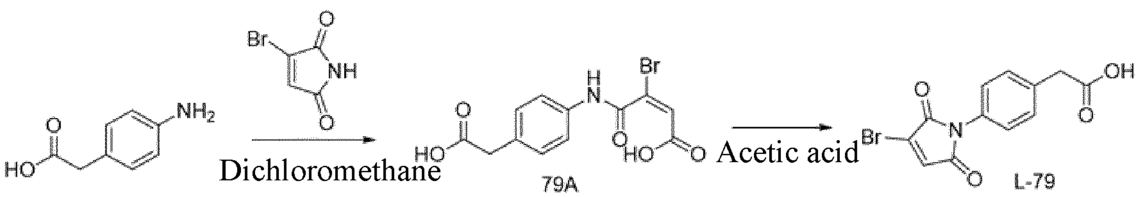

79A L-79

Step 1: To a solution of p-aminophenylacetic acid (300 mg, 1.98 mmol) and DIEPA (380 mg, 2.97 mmol) in dichloromethane (5 mL) was added 3-bromo-1H-pyrrole-2,5-dione (350 mg, 1.98 mmol), and the reaction solution was stirred at room temperature overnight and then directly purified by Flash column chromatography (C18, eluent 0%-35% acetonitrile in a 0.05% aqueous trifluoroacetic acid solution) to give 79A (350 mg) as a yellow solid. m/z:[M+H]$^+$ 328.0.

Step 2: A solution of 79A (200 mg, 0.61 mmol) in acetic acid (3 mL) was stirred in a sealed tube at 80°C for 4 hours, and the reaction solution was cooled to room temperature and directly purified by Flash column chromatography (C18, eluent 10%-50% acetonitrile in a 0.1% aqueous trifluoroacetic acid solution) to give L-79 (120 mg) as a yellow solid. m/z:[M+H]$^+$ 310.1.

Synthesis of **L-80:** 4-((S)-2-((S)-2-(2-(4-(3-bromo-2,5-dioxo-2,5-dihydro-1H-pyrrol-1-yl)phenyl)acetamido)-3-methylbutanoylamino)-5-ureidopentanoylamino)benzyl(4 nitrobenzene)carbonate

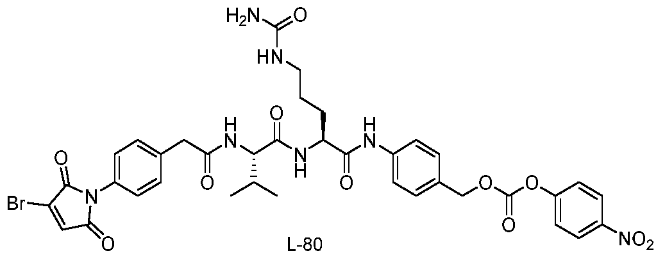

L-80

Using the synthesis method of L-5, L-80 was synthesized from Val-Cit-PAB-OH and L-79. m/z:$[M+H]^+$ 836.2.

Synthesis of **L-81:** 1-oxo-1-(4-(3-toluenesulfonyl-2-(toluenesulfonylmethyl) propionyl) phenyl)-5,8,11,14,17,20-hexaoxa-2-azatricosane-23-acid

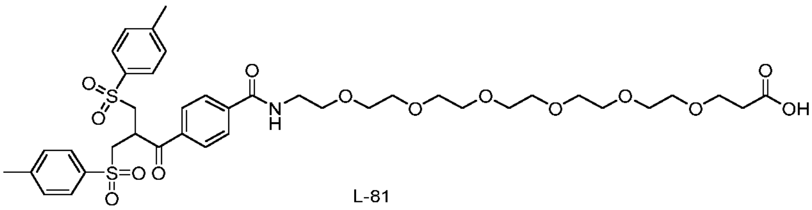

L-81

Using the synthesis method of L-7, L-81 was synthesized from 4-(3-p-toluenesulfonyl-2-(toluenesulfonylmethyl) propionyl)benzoic acid. m/z: $[M+H]^+$ 836.2.

Synthesis of **L-82:** tert-butyl(S)-4-((((9H-fluoren-9-yl)methoxy)carbonyl)amino)-5-(((S)-3-methyl-1-(((S)-1-((4-((((4-nitrophenoxy)carbonyl)oxy)methyl)phenyl)amino)-1-oxopropan-2-yl)amino)-1-oxobutan-2-yl)amino)-5-oxopentanoate

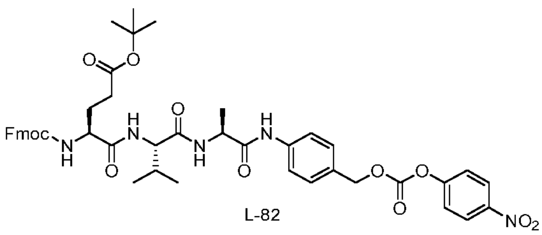

L-82

Using the synthesis method in step 2 of L-5, L-82 was synthesized from intermediate 70A. m/z:$[M+H]^+$ 866.2.

Synthesis of **L-83:** 3-(2-(2-(((1,3-diacryloyltetrahydropyrimidin-5(2H)-methylene)amino)oxy)ethoxy)ethoxy)propionic acid

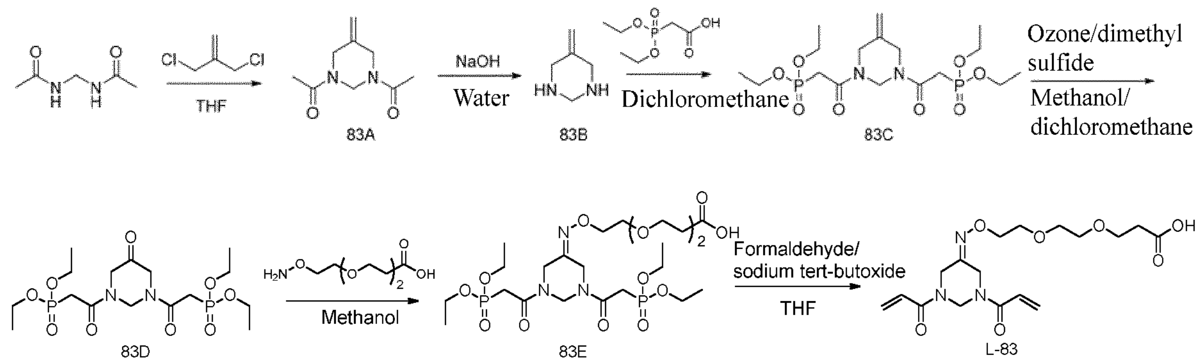


83A 83B 83C 83D 83E L-83

Step 1: To a suspension of sodium hydrogen (21.9 g, 549 mmol) in tetrahydrofuran (1.5 L) was added N,N'-methylenediacetamide (34 g, 261 mmol) followed by dropwise addition of 3-chloro-2-(chloromethyl)prop-1-ene (34.3 g, 274 mmol). The reaction was stirred at reflux for 48 hours, cooled to room temperature and filtered, and the filtrate was concentrated under reduced pressure and purified by silica gel column chromatography (dichloromethane/methanol = 50/1) to give 83A (31.7 g) as a yellow oil. m/z:$[M+H]^+$ 183.2.

Steps 2 & 3: An aqueous solution (75 mL) of 83A (14.4 g, 79 mmol) in sodium hydroxide (15.8 g, 395 mmol) was stirred at 110°C overnight, the reaction solution was cooled to room temperature, added with sodium hydroxide solid (1.6 g) and extracted with dichloromethane, and the organic phases were combined and dried over anhydrous sodium sulfate and concentrated under reduced pressure to a volume of 250 mL. To the above solution containing 83B in dichloromethane were added HOBT (39.1 g, 289 mmol), EDCI (55.5 g, 289 mmol), DIPEA (46.8 g, 362 mmol) and 2-(diethoxyphosphoryl) acetic acid (46.3 g, 236 mmol) sequentially under ice bath conditions. The reaction solution was stirred at room temperature overnight, diluted with dichloromethane (200 mL) and washed with water, the organic phase was separated and concentrated under reduced pressure, and the residue was purified by Flash column chromatography (C18, eluent 0%-40% acetonitrile in a 0.05% aqueous trifluoroacetic acid solution) to give 83C (4.5 g) as a colorless oil. m/z:$[M+H]^+$ 455.2.

Step 4: To a mixed solution of 83C (1 g, 2.2 mmol) in dichloromethane (20 mL) and methanol (5 mL) was passed ozone until the reaction solution turned light blue (about 5 minutes) under dry ice-ethyl acetate bath conditions, and after the reaction system was stirred for 5 minutes, nitrogen was bubbled in to purge the excess ozone. To the reaction was added dimethyl sulfide (3.58 g, 57.6 mmol). The reaction solution was warmed to room temperature with stirring and concentrated under reduced pressure to give 83D (1 g) as a yellow liquid. m/z:$[M+H]^+$ 457.2.

Step 5: To a solution of 83D (1 g, 2.19 mmol) in methanol (10 mL) were added 3-(2-(2-(aminooxy)ethoxy)ethoxy) propionic acid (800 mg, 4.16 mmol) and sodium acetate (900 mg, 11 mmol) sequentially. The reaction solution was stirred at room temperature overnight. Water (3 mL) was added and directly purified by *prep*-HPLC (eluent 0%-35% acetonitrile in a 0.1% aqueous trifluoroacetic acid solution, 20 minutes) to give 83E (412 mg) as a yellow oil. m/z:$[M+H]^+$ 632.2.

Step 6: To a solution of 83E (150 mg, 0.24 mmol) in tetrahydrofuran (2.5 mL) was added sodium tert-butoxide (92.3 mg, 0.96 mmol) under ice bath conditions. The reaction solution was stirred at 0°C for 30 minutes. A solution of formaldehyde (13 mg) in tetrahydrofuran was added dropwise to the above reaction solution, the obtained mixture was directly freeze-dried to give a crude product, and the crude product was further purified by *prep*-HPLC (eluent: 0%-35% acetonitrile in a 0.1% aqueous trifluoroacetic acid solution, 20 minutes) to give L-83 (90 mg) as a colorless oil. m/z:$[M+H]^+$ 384.2.

Synthesis of **L-84:** 6-(2-(methanesulfonyl)pyrimidine-5-formylamino)hexanoic acid

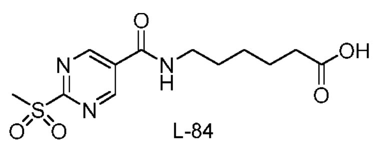

L-84

Using the synthesis method in steps 2-4 of L-78, L-84 was synthesized from 2-(methylthio)pyrimidine-5-carboxylic acid. m/z:$[M+H]^+$ 316.0.

Synthesis of **L-85:** 3-((2-(2,5-dioxo-2,5-dihydro-1H-pyrrol-1-yl)ethyl)(methyl)amino)propionic acid

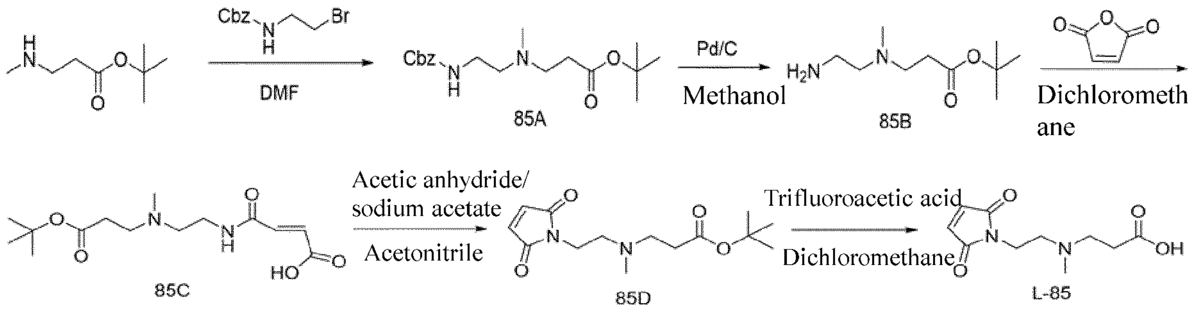


Step 1: To a solution of tert-butyl 3-(methylamino)propionate (300 mg, 1.88 mmol) and benzyl(2-bromoethyl) carbamate (437 mg, 1.69 mmol) in DMF (8 mL) were added potassium carbonate (260 mg, 1.88 mmol) and sodium iodide (28 mg, 0.19 mmol). The reaction solution was stirred at room temperature overnight, and the reaction was quenched by the addition of water (6 mL). The mixture was directly purified by *prep*-HPLC (eluent 10-50% acetonitrile in a 0.1% aqueous trifluoroacetic acid solution, 20 minutes) to give 85A (550 mg) as a colorless oil. m/z:$[M+H]^+$ 337.2.

Step 2: To a solution of 85A (550 mg, 1.63 mmol) in methanol (8 mL) was added Pd/C (10%, 170 mg). The reaction solution was stirred at room temperature for 1 hour under hydrogen atmosphere. The solid was removed by filtration, and the filtrate was concentrated under reduced pressure to give 85B (300 mg) as a colorless oil. m/z:$[M+H]^+$ 203.2.

Step 3: To a solution of 85B (270 mg, 1.33 mmol) and triethylamine (270 mg, 2.66 mmol) in dichloromethane (3 mL) was added maleic anhydride (196 mg, 2.0 mmol). The reaction solution was stirred at room temperature for 1 hour and directly purified by *prep*-HPLC (eluent 10%-60% acetonitrile in a 0.1% aqueous trifluoroacetic acid solution for 20 minutes) to give 85C (220 mg) as a colorless oil. m/z:$[M+H]^+$ 301.2.

Step 4: To a solution of 85C (190 mg, 0.63 mmol) in acetonitrile (5 mL) were added acetic anhydride (129 mg, 1.26 mmol) and sodium acetate (8 mg, 0.1 mmol). The reaction solution was stirred at 85°C for 2 hours, cooled to room temperature, and then directly purified by *prep*-HPLC (eluent: 10%-45% acetonitrile in a 0.1% aqueous trifluoroacetic acid solution, 20 minutes) to give 85D (160 mg) as a colorless oil. m/z: $[M+H]^+$ 283.2.

Step 5: To a solution of 85D (110 mg, 0.39 mmol) in dichloromethane (2 mL) was added trifluoroacetic acid (1 mL). The reaction solution was stirred at room temperature for 1 hour, concentrated under reduced pressure, and then directly purified by *prep*-HPLC (eluent: 10%-30% acetonitrile in a 0.1% aqueous trifluoroacetic acid solution, 20 minutes) to give L-85 (100 mg) as a colorless oil. m/z:$[M+H]^+$ 227.2.

Synthesis of **L-86:** 2-((3-bromo-2,5-dioxo-2,5-dihydro-1H-pyrrol-1-yl)methyl)-1,3-dioxan-5-carboxylic acid

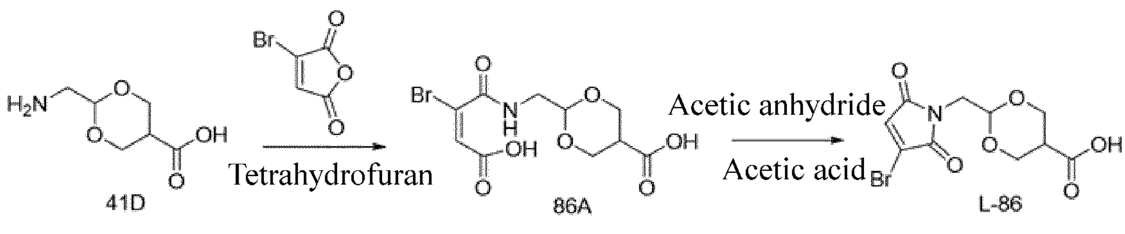


Step 1: To a mixed solution of 41D (450 mg, 2.79 mmol) and DIPEA (721 mg, 5.58 mmol) in tetrahydrofuran and water (20 mL, 1/1) was slowly added bromomaleic anhydride (642 mg, 3.63 mmol). The reaction solution was slowly warmed to room temperature, continued to be stirred for 1 hour, and then directly purified by *prep*-HPLC (eluent 10%-45% acetonitrile in a 0.1% aqueous trifluoroacetic acid solution, 20 minutes) to give 86A (450 mg) as a yellow oil. m/z:$[M+H]^+$ 338.2.

Step 2: To a solution of 86A (200 mg, 0.59 mmol) in acetic acid (4 mL) was added acetic anhydride (60.2 mg, 0.59 mmol). The reaction solution was stirred in a sealed tube at 110°C for 3 hours, cooled to room temperature, and then directly purified by *prep*-HPLC (eluent 10%-45% acetonitrile in a 0.1% aqueous trifluoroacetic acid solution, 20 minutes) to give L-86 (45 mg) as a brown solid. m/z: $[M+H]^+$ 320.2.

Synthesis of **L-87:** 3-(2-(4-(3-toluenesulfonyl-2-(toluenesulfonylmethyl)propionyl)benzoylamino)ethoxy)propionic acid

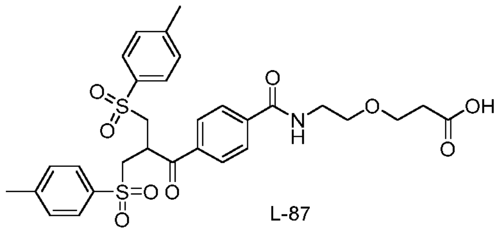

L-87

Using the synthesis method of L-7, L-87 was synthesized from 4-(3-p-toluenesulfonyl-2-(toluenesulfonylmethyl)propionyl)benzoic acid. m/z:$[M+H]^+$ 616.2.

**Synthesis of compounds**

**Example 1:** Synthesis of 2-amino-N,N-dipropyl-8-(1-((5,6,7,8-tetrahydro-1,6-naphthyridin-3-yl)carbamoyl)cyclopropyl)-3H-benzo[b]azepine-4-formamide (compound 1)

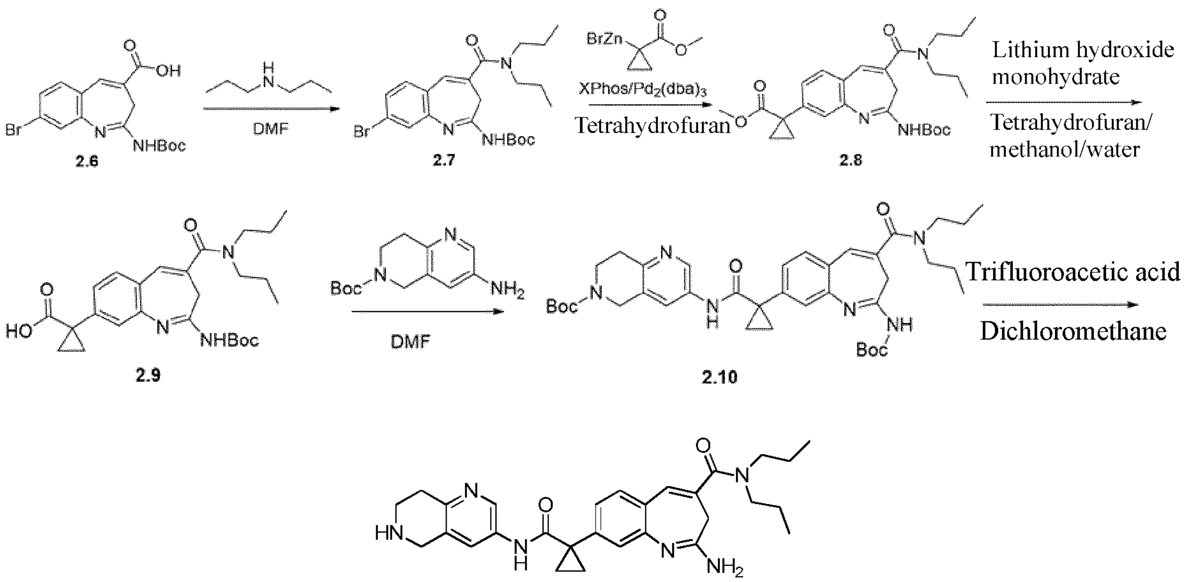


2.6, 2.7, 2.8, 2.9, 2.10, 1

Step 1: A solution of compound 2.6 (1.1 g, 2.8 mmol), HATU (1.6 g, 4.33 mmol), dipropylamine (580 mg, 22 mmol) and DIPEA (560 mg, 4.37 mmol) in DMF (10 mL) was stirred at room temperature for 3 hours. The reaction system was diluted with ethyl acetate (100 mL) and washed with water and brine each, the organic phase was separated, dried over anhydrous sodium sulfate and filtered, the filtrate was concentrated under reduced pressure, and the residue was purified by Flash column chromatography (methanol/dichloromethane = 1/20) to give compound 2.7 (500 mg, yield: 39%) as a light yellow solid.

Step 2: Compound 2.7 (2 g, 4.31 mmol), Xphos (0.2 g), $Pd_2(dba)_3$ (0.2 g) were dissolved in a freshly prepared solution of (1-(methoxycarbonyl) cyclopropyl)zinc bromide in tetrahydrofuran (30 mL) (refer to WO2018/138356A1), the reaction system was replaced with nitrogen three times, then stirred for 2 h at 75°C under nitrogen atmosphere and cooled to room temperature, the reaction was quenched by adding ice water, the aqueous phase was extracted with ethyl acetate, the organic phases were combined and washed with saturated brine, and the residue was purified by Flash column chromatography (petroleum ether/ethyl acetate = 3/1) to give compound 2.8 (500 mg, yield: 24%) as a light yellow solid. m/z: $[M+H]^+$484.2.

Step 3: To a mixed solution of compound 2.8 (500 mg, 1.03 mmol) in tetrahydrofuran (5 mL), methanol (0.5 mL) and water (0.5 mL) was added lithium hydroxide monohydrate (130 mg, 3.1 mmol), the reaction system was stirred at room

temperature for 16 hours, then the reaction solution was neutralized with hydrochloric acid (1 M), the aqueous phase was extracted with ethyl acetate, the organic phases were combined and washed with saturated brine, dried over anhydrous sodium sulfate and filtered, and the filtrate was concentrated under reduced pressure to give compound 2.9 (400 mg, yield: 82%) as an off-white solid. m/z: $[M+H]^+$ 484.2.

Step 4: To a solution of compound 2.9 (100 mg, 0.21 mmol) in DMF (1 mL) were added tert-butyl 3-amino-7,8-dihydro-1,6-naphthyridine-6(5H)-carboxylate (64 mg, 0.25 mmol), HATU (97 mg, 0.25 mmol) and DIPEA (55 mg, 0.42 mmol) sequentially, and the reaction solution was stirred at room temperature for 4 hours. The reaction solution was then directly purified by Flash column chromatography (C18, eluent 0-85% acetonitrile in a 10 mM aqueous ammonium bicarbonate solution) to give compound 2.10 (28 mg, yield: 19%) as a white solid. m/z: $[M+H]^+$ 701.3.

Step 5: To a solution of compound 2.10 (28 mg, 0.4 mmol) in dichloromethane (2 mL) was added trifluoroacetic acid (1 mL), the reaction solution was stirred at room temperature for 2 hours and then concentrated directly under reduced pressure, and the residue was neutralized with an ammonia methanol solution (7 M) and purified by Flash column chromatography (C18, eluent 0%-60% acetonitrile in a 10 mM aqueous ammonium bicarbonate solution) to give compound 1 (17 mg, yield: 85%) as a white solid. m/z: $[M+H]^+$ 501.3; $^1H$ NMR (400 MHz, DMSO-$d_6$): δ 9.19 (s, 1H), 8.42 (s, 1H), 7.63 (s, 1H), 7.25 (d, $J$ = 7.6Hz, 1H), 7.00 (s, 1H), 6.91 (d, $J$ = 8.4Hz, 1H), 6.74 (s, 2H), 6.69 (s, 1H) , 3.78 (s, 2H), 2.97 (br. s, 2H), 2.63 (s, 6H), 1.55-0.74 (m, 17H).

**Example 2:** Synthesis of 2-amino-N-(2-hydroxyethyl)-N-propyl-8-(1-((5,6,7,8-tetrahydro-1,6-naphthyridin-3-yl)carbamoyl)cyclopropyl)-3H-benzo[b]azepine-4-formamide (compound 2)

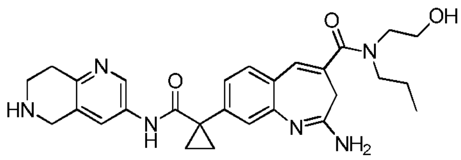

2

Using the synthesis method of compound 1, compound 2 was obtained as a white solid by replacing dipropylamine in step 1 with 2-(propylamino)ethanol. m/z: $[M+H]^+$ 503.3; $^1H$ NMR (400 MHz, $CD_3OD$): δ 8.40 (s, 1H), 7.68 (s, 1H), 7.38 (d, $J$ = 8.0Hz, 1H), 7.26 (s, 1H), 7.16 (d, $J$ = 8.0Hz, 1H), 6.94 (s, 1H), 3.94 (s, 2H), 3.77 (br. s, 2H), 3.62 (t, $J$ = 8.0Hz, 2H), 3.50 (t, $J$ = 8.0Hz, 2H), 3.16 (t, $J$ = 8.0Hz, 2H), 2.93-2.80 (m, 3H), 1.78- 1.50 (m, 4H), 1.44-1.15 (m, 3H), 0.90 (br. s, 3H).

**Example 3:** Synthesis of 2-amino-6-fluoro-N,N-dipropyl-8-(1-((5,6,7,8-tetrahydro-1,6-naphthyridin-3-yl)carbamoyl)cyclopropyl)-3H-benzo[b]azepine-4-formamide (compound 3)

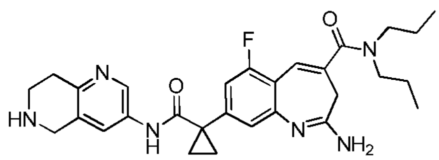

3

Using the synthesis method of compound 1, compound 3 was obtained as a white solid by replacing intermediate 2.6 in step 1 with intermediate 3.1. m/z: $[M+H]^+$ 519.1.

**Example 4:** Synthesis of 2-amino-8-(2-oxo-2-((5,6,7,8-tetrahydro-1,6-naphthyridin-3-yl)amino)acetyl)-N,N-dipropyl-3H-benzo[b]azepine-4-formamide (compound 4)

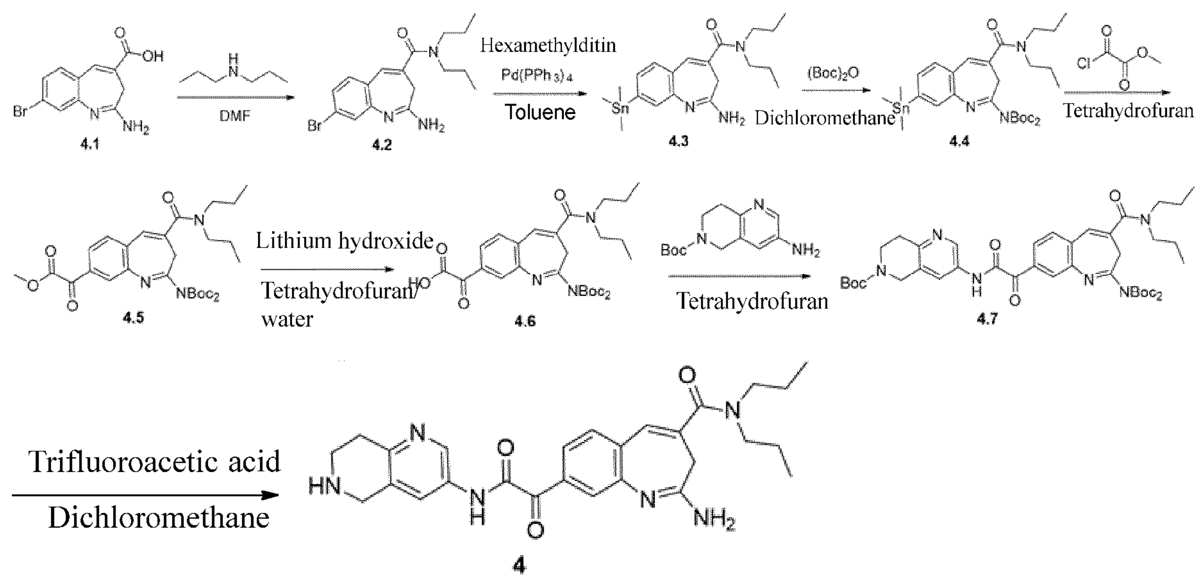


Step 1: Using the synthesis method of compound 2.7, compound 4.2 was obtained by reaction with compound 4.1.

Step 2: A mixture of compound 4.2 (2 g, 5.49 mmol), hexamethylditin (1.89 g, 5.76 mmol) and $Pd(PPh_3)_4$ (127 mg, 0.11 mmol) in toluene (25 mL) was replaced three times with nitrogen, and the reaction system was stirred at 100°C for 6 hours under nitrogen. Then, the reaction system was cooled to room temperature and filtered, the filtrate was concentrated under reduced pressure, and the residue was purified by Flash column chromatography (petroleum ether/ethyl acetate = 1/1) to give compound 4.3 (1.45 g, yield: 59%) as a light brown solid. m/z: $[M+H]^+$ 450.2.

Step 3: To a solution of compound 4.3 (1.45 g, 3.24 mmol) in dichloromethane (30 mL) were added triethylamine (0.98 g, 9.71 mmol), 4-dimethylaminopyridine (79 mg, 0.65 mmol) and $(Boc)_2O$ (2.12 g, 9.71 mmol) each. The reaction solution was stirred at room temperature overnight and then directly concentrated under reduced pressure, and the residue was purified by Flash column chromatography (petroleum ether/ethyl acetate = 10/1) to give compound 4.4 (1.2 g, yield: 57%) as a light yellow solid. m/z: $[M+H]^+$ 650.0.

Step 4: To a solution of compound 4.4 (400 mg, 0.62 mmol), DIPEA (120 mg, 0.92 mmol) and potassium carbonate (17 mg, 0.12 mmol) in anhydrous tetrahydrofuran (6 mL) was added $Pd_2(dba)_3$ (33 mg, 0.06 mmol) under ice bath conditions, the reaction system was displaced three times with nitrogen, and then a solution of monomethyl oxalyl chloride (113 mg, 0.92 mmol) in tetrahydrofuran (1 mL) was added dropwise to the system. After the addition was completed, the reaction system was warmed to room temperature and stirred overnight, the reaction was quenched by adding water, the aqueous phase was extracted with ethyl acetate, the organic phases were combined and concentrated under reduced pressure, and the residue was purified by Flash column chromatography (petroleum ether/ethyl acetate = 2/1) to give compound 4.5 (145 mg, yield: 58%) as a light yellow solid. m/z: $[M+H]^+$594.3.

Step 5: To a solution of compound 4.5 (145 mg, 0.44 mmol) in tetrahydrofuran (5 mL) was added an aqueous solution of lithium hydroxide monohydrate (289 mg, 0.88 mmol) (2 mL) under ice bath conditions. The reaction system was stirred at 0°C for 1 hour. Then, the reaction system was adjusted to pH = 7 with hydrochloric acid (1 M), and the mixture was directly purified by Flash column chromatography (C18, eluent 0-85% acetonitrile in a 10 mM aqueous ammonium bicarbonate solution) to give compound 4.6 (90 mg, yield: 31%) as a light brown solid. m/z: $[M+H]^+$558.3.

Step 6: To a solution of compound 4.6 (80 mg, 0.14 mmol) in DMF (4 mL) were added tert-butyl 3-amino-7,8-dihydro-1,6-naphthyridine-6 (5H)-carboxylate (43 mg, 0.17 mmol), HATU (65 mg, 0.17 mmol) and DIPEA (22 mg, 0.17 mmol) sequentially, and the reaction solution was subjected to microwave reaction at 50°C for 1 hour. The reaction was then quenched by adding water, and the aqueous phase was extracted with ethyl acetate. The organic phases were combined and concentrated under reduced pressure, and the residue was purified by *prep*-TLC (petroleum ether/ethyl acetate = 1/2) to give compound 4.7 (39 mg, yield: 34%) as a light yellow solid. m/z: $[M+H]^+$ 789.5.

Step 7: To a solution of compound 4.7 (39 mg, 0.05 mmol) in dichloromethane (4 mL) was added trifluoroacetic acid (2 mL), the reaction solution was stirred at room temperature for 2 hours and then concentrated directly under reduced pressure, and the residue was neutralized with an ammonia methanol solution (7M) and purified by Flash column chromatography (C18, eluent 0%-60% acetonitrile in a 10 mM aqueous ammonium bicarbonate solution) to give compound 4 (15 mg, yield: 62%) as a yellow solid. m/z: $[M+H]^+$489.3.

Example 5: Synthesis of 2-amino-8-(2-methyl-1-oxo-1-((5,6,7,8-tetrahydro-1,6-naphthyridin-3-yl)amino)propan-2-yl)-N,N-dipropyl-3H-benzo[b]azepine-4-formamide (compound 5)

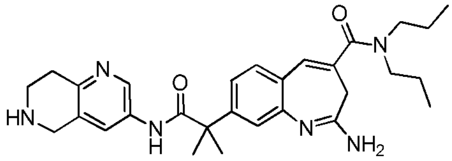

**5**

Using the synthesis method of compound 1, compound 5 was obtained as a white solid by replacing (1-(methoxycarbonyl)cyclopropyl)zinc bromide in step 2 with (1-methoxy-2-methyl-1-oxopropan-2-yl)zinc bromide. m/z:[M+H]$^+$ 503.3; $^1$H NMR (400 MHz, $CD_3OD$): δ 8.45 (d, *J*= 2.0Hz, 1H), 7.77 (d, *J*= 2.0Hz, 1 H), 7.31 (d, *J*= 8.4Hz, 1H), 7.20 (d, *J* = 1.6Hz, 1H), 7.09-7.05 (m, 1H), 6.82 (s, 1H), 3.96 (s, 2H), 3.44-3.37 (m, 4H), 3.19-3.14 (m, 2H), 2.94-2.73 (m, 4H), 1.75-1.56 (m, 10H), 1.05-0.78 (m, 6H).

**Example** 6: Synthesis of 2-amino-6-fluoro-N-(2-hydroxyethyl)-N-propyl-8-(1-((5,6,7,8-tetrahydro-1,6-naphthyridin-3-yl)carbamoyl)cyclopropyl)-3H-benzo[b]azepine-4-formamide (compound 6)

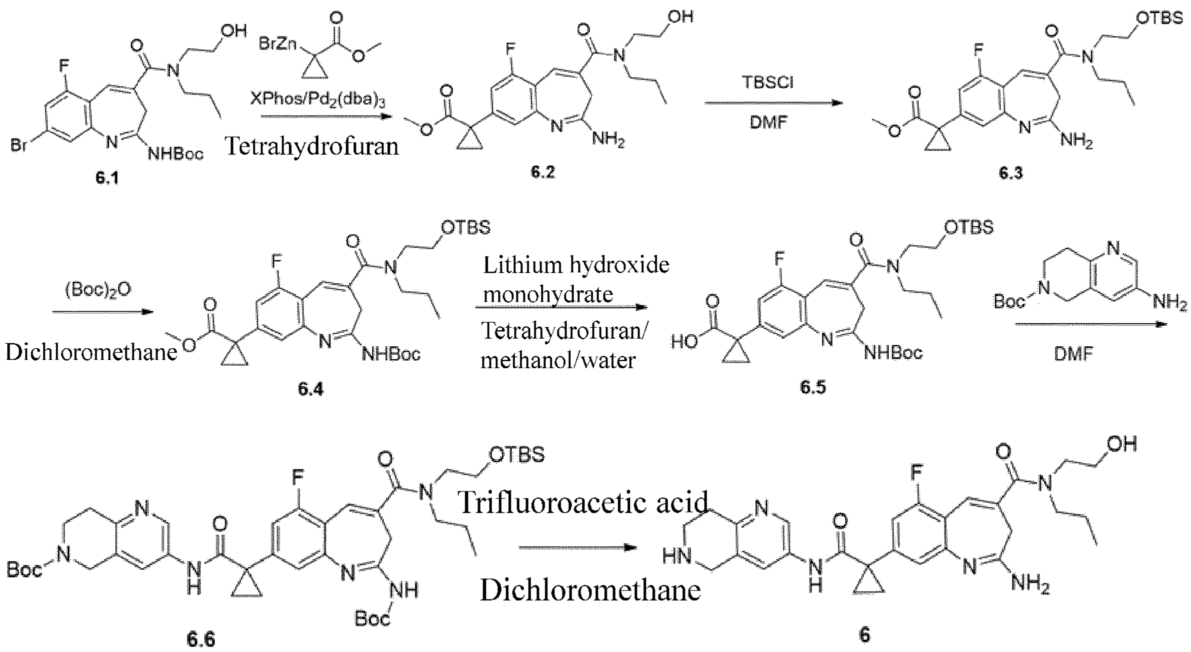

6.1 6.2 6.3 6.4 6.5 6.6 6

Step 1: Compound 6.1 (obtained by reacting intermediate 3.1 with 2-(propylamino)ethanol using the synthesis method of compound 2.7) (1 g, 2.06 mmol), Xphos (0.1 g), $Pd_2(dba)_3$ (0.2 g) were dissolved in a freshly prepared solution of (1-(methoxycarbonyl)cyclopropyl)zinc bromide in tetrahydrofuran (35 mL), the reaction system was replaced three times with nitrogen, then stirred at 75°C under nitrogen atmosphere for 2 hours and cooled to room temperature, a methanol solution of hydrogen chloride (1 mL, 4 M) was added to quench the reaction, and the reaction system was directly purified by Flash column chromatography (C18, eluent 0%-70% acetonitrile in a 10 mM aqueous ammonium bicarbonate solution) to give compound 6.2 (600 mg, yield: 72%) as an off-white solid. m/z: [M+H]$^+$ 404.2.

Step 2: To a solution of compound 6.2 (500 mg, 1.24 mmol) in DMF (5 mL) were added imidazole (422 mg, 6.2 mmol) and TBSCl (561 mg, 3.72 mmol) sequentially, and the reaction system was stirred at room temperature for 3 hours and then directly purified by Flash column chromatography (C18, eluent 0-85% acetonitrile in a 10 mM aqueous ammonium bicarbonate solution) to give compound 6.3 (510 mg, yield: 79%) as a white solid. m/z: [M+H]$^+$ 518.2.

Step 3: To a solution of compound 6.3 (500 mg, 0.97 mmol) in dichloromethane (15 mL) were added triethylamine

(220 mg, 2.18 mmol) and $(Boc)_2O$ (285 mg, 1.31 mmol) sequentially, and the reaction system was stirred at room temperature for 12 hours. The reaction was quenched by adding water, the aqueous phase was extracted with dichloromethane, the organic phases were combined and washed with saturated brine, dried over anhydrous sodium sulfate and filtered, the filtrate was concentrated under reduced pressure, and the residue was purified by Flash column chromatography (petroleum ether/ethyl acetate = 10/1) to give compound 6.4 (510 mg, yield: 85%) as an off-white solid. m/z: $[M+H]^+$ 618.3.

Step 4: To a mixed solution of compound 6.4 (500 mg, 0.81 mmol) in tetrahydrofuran (2 mL), methanol (1 mL) and water (1 mL) was added lithium hydroxide monohydrate (102 mg, 2.43 mmol), the reaction system was stirred at room temperature for 3 hours, and then the reaction solution was adjusted to pH = 6 with hydrochloric acid (1M) and then directly purified by Flash column chromatography (C18, eluent 0%-55% acetonitrile in a 10 mM aqueous ammonium bicarbonate solution) to give compound 6.5 (330 mg, yield: 68%) as a white solid. m/z: $[M+H]^+$ 604.3.

Step 5: To a solution of compound 6.5 (50 mg, 0.08 mmol) in DMF (5 mL) were added HATU (46 mg, 0.12 mmol) and DIPEA (31 mg, 0.24 mmol) sequentially, the reaction solution was stirred at room temperature for 30 minutes, and then tert-butyl 3-amino-7,8-dihydro-1,6-naphthyridine-6(5H)-carboxylate (25 mg, 0.1 mmol) was added. The resulting reaction solution was continuously stirred at room temperature for 16 hours and then directly purified by Flash column chromatography (C18, eluent 0-85% acetonitrile in a 10 mM aqueous ammonium bicarbonate solution) to give compound 6.6 (35 mg, yield: 52%) as a white solid. m/z: $[M+H]^+$ 835.4.

Step 6: To a solution of compound 6.6 (30 mg, 0.04 mmol) in dichloromethane (2 mL) was added trifluoroacetic acid (1 mL). The reaction system was stirred at room temperature for 1 hour, the reaction solution was concentrated under reduced pressure, and then ammonia methanol (1 mL, 7M) solution was added and then directly purified by Flash column chromatography (C18, eluent 0%-35% acetonitrile in a 10 mM ammonium bicarbonate solution) to give compound 6 (12 mg, yield: 58%) as a white solid. m/z: $[M+H]^+$ 521.3; $^1H$ NMR (400 MHz, $CD_3OD$): $\delta$ 8.40 (d, $J$ = 2.4Hz, 1H), 7.69 (d, $J$ = 2.0Hz, 1H), 7.06 (s,1H), 7.01 (d, $J$ = 1.2Hz, 1H), 6.90 (dd, $J$ = 1.6, 10.8Hz, 1H), 3.94 (s, 2H), 3.45-3.75 (m, 6H), 3.13 (t, $J$ = 6.4Hz, 2H), 2.85-2.90 (m, 3H), 1.58-1.70 (m, 4H), 1.21-1.28 (m, 3H), 0.84-0.96 (m, 3H).

**Example 7:** Synthesis of 2-amino-8-(1-((5-(aminomethyl)pyridin-3-yl)carbamoyl) cyclopropyl)-N-(2-hydroxyethyl)-N-propyl-3H-benzo[b]azepine-4-formamide (compound 7)

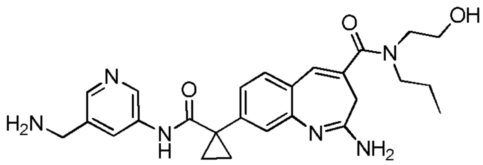

**7**

Using the synthesis method of compound 1, compound 7 was obtained as a white solid by replacing dipropylamine in step 1 with 2-(propylamino)ethanol and replacing tert-butyl 3-amino-7,8-dihydro-1,6-naphthyridine-6(5H)-carboxylate in step 3 with tert-butyl ((5-aminopyridin-3-yl)methyl)carbamate. m/z: $[M+H]^+$ 477.3; $^1H$ NMR (400 MHz, $CD_3OD$): $\delta$ 8.59 (d, $J$ = 2.0Hz, 1H), 8.38 (d, $J$ = 2.0 Hz, 1H), 8.33 (t, $J$ = 2.0Hz, 1H), 7.60-7.54 (m, 1H), 7.52-7.44 (m, 2H), 7.11 (s, 1 H), 4.90 (overlapping with solvent, 2H), 4.18 (s, 2H), 3.83-3.42 (m, 6H), 1.77- 1.60 (m, 4H), 1.33-1.27 (m, 2H), 1.06-0.81 (m, 3H).

**Example 8:** Synthesis of 2-amino-$N^4$-(2-hydroxyethyl)-$N^4$-propyl-$N^8$-(5,6,7,8-tetrahydro-1,6-naphthyridin-3-yl)-3H-benzo[b]azepine-4,8-diacetamide (compound 8)

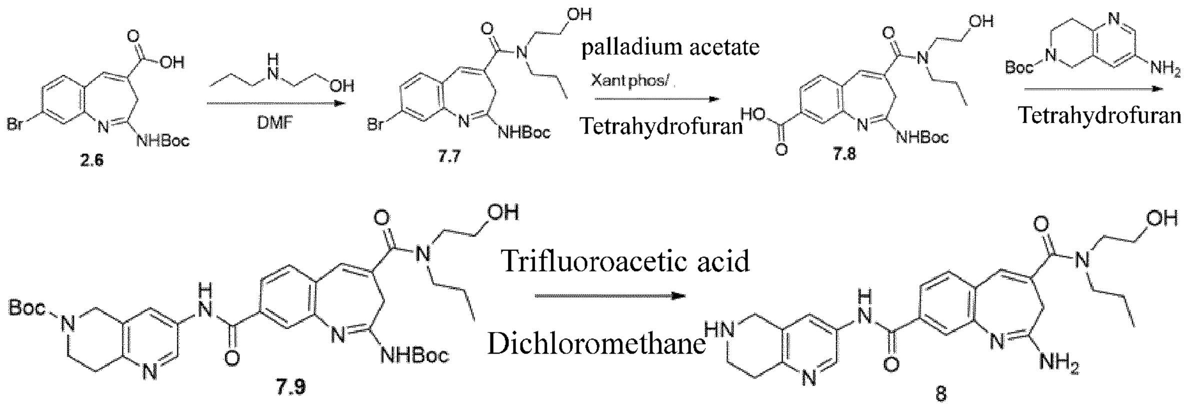


2.6 7.7 7.8 7.9 8

Step 1: To a solution of compound 2.6 (8.8 g, 23.1 mmol) in DMF (100 mL) were added DIPEA (8.9 g, 69.2 mmol), 2-(propylamino) ethanol (2.9 g, 27.7 mmol) and HATU (13.2 g, 34.63 mmol) sequentially. After the addition was completed, the reaction system was stirred at room temperature for 2 hours, then the reaction was quenched by adding water, the aqueous phase was extracted with ethyl acetate (100 mL x 2), the organic phases were combined and washed with saturated brine, the organic phases were separated, dried over anhydrous sodium sulfate, filtered and concentrated, and the residue was purified by silica gel column chromatography (petroleum ether/ethyl acetate = 1/1) to give compound 7.7 (10 g, yield: 93%) as an off-white solid. m/z: $[M+H]^+$466.2.

Step 2: A mixed solution of compound 7.7 (600 mg, 1.29 mmol), potassium phosphate (820 mg, 3.86 mmol), palladium acetate (29 mg, 0.13 mmol) and Xantphos (37 mg, 0.06 mmol) in tetrahydrofuran (10 mL) and water (5 mL) was replaced three times with nitrogen and carbon monoxide each, and then stirred at 70°C for 4 hours under carbon monoxide atmosphere. The reaction system was cooled to room temperature and concentrated under reduced pressure, the crude product was dispersed in methanol and filtered, the filter cake was washed with methanol, and the filtrate was concentrated under reduced pressure and directly purified by Flash column chromatography (C18, eluent 0%-70% acetonitrile in a 10 mM aqueous ammonium bicarbonate solution) to give compound 7.8 (100 mg, yield: 18%) as a light brown solid. m/z: $[M+H]^+$ 432.2.

Step 3: To a solution of compound 7.8 (50 mg, 0.11 mmol), HATU (49 mg, 0.13 mmol) and DIPEA (17 mg, 0.13 mmol) in DMF (3 mL) was added tert-butyl 3-amino-7,8-dihydro-1,6-naphthyridine-6(5H)-carboxylate (32 mg, 0.13 mmol), and the reaction solution was stirred at room temperature overnight. The reaction solution was poured into water to quench the reaction, the aqueous phase was extracted with ethyl acetate, and the organic phases were combined and concentrated under reduced pressure. The residue was purified by Flash column chromatography (C18, eluent 0-70% acetonitrile in a 10 mM aqueous ammonium bicarbonate solution) to give compound 7.9 (25 mg, yield: 32%) as a light brown solid. m/z: $[M+H]^+$ 663.2.

Step 4: A solution of compound 7.9 (25 mg, 0.04 mmol) and trifluoroacetic acid (2 mL) in dichloromethane (5 mL) was stirred at room temperature for 2 hours and concentrated directly under reduced pressure, and the residue was prepared by *prep*-HPLC (isolation condition 2) to give compound 8 (16 mg, yield: 94%) as a white solid. m/z: $[M+H]^+$ 463.2; $^1$H NMR (400 MHz, DMSO-$d_6$): δ 10.36 (s, 1H), 8.69 (d, *J* = 2.0 Hz, 1H), 8.00 (s, 1H), 7.69 (s, 1H), 7.53-7.45 (m, 1H), 7.42 (d, *J* = 8.4Hz, 1H), 6.99-6.76 (m, 2H), 4.81 (s, 1H), 4.01 (s, 2H), 3.55 (br. s, 4H), 3.38 (m, 5H), 3.17 (t, *J* = 6.0 Hz, 3H), 2.83 (t, *J* = 6.0Hz, 2H), 2.73 (s, 2H), 1.67-1.43 (m, 2H), 0.86 (s, 3H).

**Example 9:** Synthesis of 2-amino-N,N-bis(2-hydroxyethyl)-8-(1-((5,6,7,8-tetrahydro-1,6-naphthyridin-3-yl)carbamoyl) cyclopropyl)-3H-benzo[b]azepine-4-formamide (compound 9)

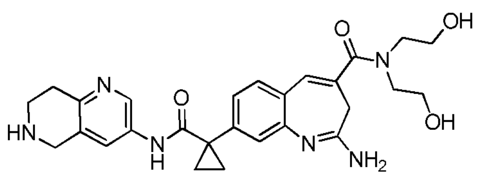

9

Using the synthesis method of compound 1, compound 9 was obtained as a white solid by replacing dipropylamine in step 1 with bis(2-((tert-butyldimethylsilyl)oxy)ethyl)amine. m/z:$[M+H]^+$505.2; $^1$H NMR (400 MHz, $CD_3OD$): δ 8.38

(d, $J$ = 2.4Hz, 1H), 7.68 (d, $J$ = 2.0Hz, 1H), 7.37 (d, $J$ = 8.0Hz, 1H), 7.24 (d, $J$ = 1.6Hz, 1H), 7.17- 7.11 (m, 1H), 7.00 (s, 1H), 3.94 (s, 2H), 3.86-3.69 (m, 4H), 3.69-3.57 (m, 4 H), 3.18-3.11 (m, 2H), 3.07-2.95 (m, 1H), 2.90-2.79 (m, 3H), 1.63-1.56 (m, 2 H), 1.26-1.19 (m, 2H).

**Example 10:** Synthesis of 2-amino-6-fluoro-N,N-bis(2-hydroxyethyl)-8-(1-((5,6,7,8-tetrahydro-1,6-naphthyridin-3-yl) carbamoyl)cyclopropyl)-3H-benzo[b]azepine-4-formamide (compound 10)

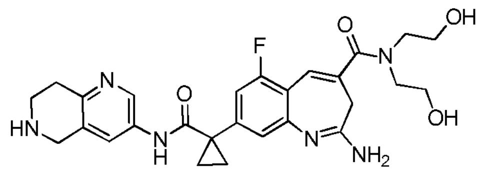

**10**

Using the synthesis method of compound 1, compound 10 was obtained as a white solid with bis(2-((tert-butyldimethylsilyl)oxy)ethyl)amine and 6.1 as starting materials. m/z: $[M+H]^+$ 523.1; $^1H$ NMR (400 MHz, $CD_3OD$): δ 8.42 (d, $J$ = 2.4Hz, 1H), 7.72 (d, $J$ = 2.4Hz, 1H), 7.12 (d, $J$ = 1.6Hz, 1H), 7.08 (s, 1H), 6.92 (dd, $J$ = 1.2, 10.8Hz, 1H), 3.97 (s, 2H), 3.68-3.81 (m, 8H), 3.32-3.34 (m, 2H), 3.17-3.20 (m, 2H), 2.88-2.91 (m, 2H), 1.60- 1.62 (m, 2H), 1.23-1.26 (m, 2H).

**Example 11:** Synthesis of 2-amino-8-(1-((5-(aminomethyl)pyridin-3-yl)carbamoyl)cyclopropyl)-N,N-bis(2-hydroxyethyl)-3H-benzo[b]azepine-4-formamide (compound 11)

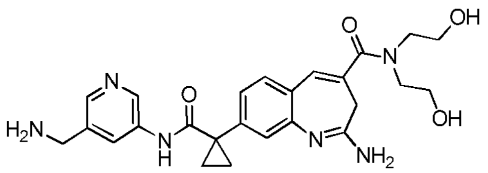

**11**

Using the synthesis method of compound 1, compound 11 was obtained as a white solid by replacing dipropylamine in step 1 with bis(2-((tert-butyldimethylsilyl)oxy)ethyl)amine and replacing tert-butyl 3-amino-7,8-dihydro-1,6-naphthyridine-6(5H)-carboxylate in step 3 with tert-butyl((5-aminopyridin-3-yl)methyl)carbamate. m/z:$[M+H]^+$479.2; $^1H$ NMR (400 MHz, $CD_3OD$): δ 8.53 (d, $J$ = 2.4Hz, 1H), 8.24 (d, $J$ = 2.0Hz, 1H), 7.93 (t, $J$ = 2.4Hz, 1H), 7.37 (d, $J$ = 8.0Hz, 1H), 7.24 (d, $J$ = 2.0Hz, 1H), 7.14 (dd, $J$ = 1.8, 8.0Hz, 1H), 7.00 (s, 1H), 4.62 (s, 2H), 3.81 (s, 2 H), 3.79-3.61 (m, 8H), 1.65-1.58 (m, 2H), 1.27-1.19 (m, 2H).

**Example 12:** Synthesis of 2-amino-8-(1-((5-(aminomethyl)pyridin-3-yl)carbamoyl)cyclopropyl)-6-fluoro-N-(2-hydroxyethyl)-N-propyl-3H-benzo[b]azepine-4-formamide (compound 12)

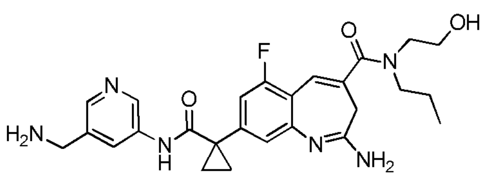

**12**

Using the synthesis method of compound 6, compound 12 was obtained as a white solid by replacing tert-butyl 3-amino-7,8-dihydro-1,6-naphthyridine-6(5H)-carboxylate in step 5 with tert-butyl((5-aminopyridin-3-yl)methyl)carbamate. m/z:$[M+H]^+$495.2; $^1H$ NMR (400 MHz, $CD_3OD$): δ 8.55 (d,J= 2.4Hz, 1H), 8.24 (d, $J$ = 1.6Hz, 1H), 7.93 (t, $J$ = 2.4Hz, 1H),

7.07(s, 1H), 7.01(d, $J$ = 2.0Hz, 1H), 6.90 (dd, $J$ = 2.0, 10.8Hz, 1H), 4.64 (br. s, 1H) 3.84-3.64 (m, 4H), 3.63-3.55 (m, 2H), 3.51-3.42 (m, 2H), 2.91(s,1H), 1.73-1.63 (m, 2H), 1.62-1.58 (m, 2H), 1.26-1.20 (m, 2H), 1.02-0.77 (m, 3H).

**Example 13:** Synthesis of 2-amino-8-(1-((5-(aminomethyl)pyridin-3-yl)carbamoyl)cyclopropyl)-6-fluoro-N,N-dipropyl-3H-benzo[b]azepine-4-formamide (compound 13)

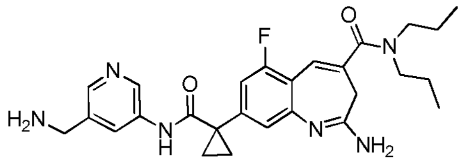

**13**

Using the synthesis method of compound 1, compound 13 was obtained as a white solid by replacing tert-butyl 3-amino-7,8-dihydro-1,6-naphthyridine-6(5H)-carboxylate in step 3 with tert-butyl((5-aminopyridin-3-yl)methyl)carbamate with intermediate 3.2 as the starting material. m/z: $[M+H]^+$ 493.1; $^1H$ NMR (400 MHz, $CD_3OD$): δ 8.45 (d, $J$ = 4.0Hz, 1H), 8.15 (d, $J$ = 4.0Hz, 1H), 7.84 (s, 1H), 6.98 (s, 1H), 6.83-6.80 (m, 2H), 3.71 (s, 2H), 3.31-3.29 (m, 4H), 2.80 (s, 2H), 1.60-1.50 (m, 6H), 1.16-1.13 (m, 2H), 0.80 (br. s, 6H).

**Example 14:** Synthesis of 2-amino-N-(2-hydroxyethyl)-8-(2-oxo-2-((5,6,7,8-tetrahydro-1,6-naphthyridin-3-yl)amino)acetyl)-N-propyl-3H-benzo[b]azepine-4-formamide (compound 14)

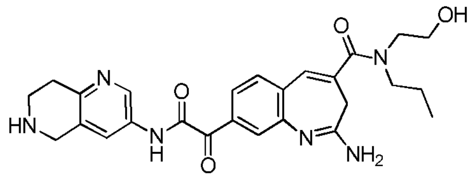

**14**

Using the synthesis method of compound 4, compound 14 was obtained as a white solid by reacting intermediate 4.1 with N-(2-((tert-butyldimethylsilyl)oxy)ethyl)propan-1-amine as starting material. m/z: $[M+H]^+$ 491.2; $^1H$ NMR (400 MHz, $CD_3OD$): δ 8.67 (s, 1H), 7.97 (s, 1H), 7.78 (d, $J$ = 8.0Hz, 1H), 7.47 (d, $J$ = 8.0Hz, 1H), 6.97 (s, 1H), 4.00 (s, 2H), 3.78-3.75 (m, 2H), 3.61- 3.58 (m, 2H), 3.49-3.45 (m, 2H), 1.67-1.60 (m, 2H), 1.00-0.85 (m, 3H).

**Example 15:** Synthesis of 2-amino-N,N-dipropyl-8-(1-((1,2,3,4-tetrahydroisoquinolin-7-yl)carbamoyl)cyclopropyl)-3H-benzo[b]azepine-4-formamide (compound 15)

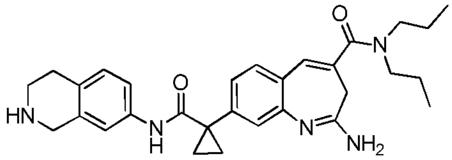

**15**

Using the synthesis method of compound 1, compound 15 was obtained as a white solid by replacing tert-butyl 3-amino-7,8-dihydro-1,6-naphthyridine-6(5H)-carboxylate in step 3 with tert-butyl 7-amino-3,4-dihydroisoquinoline-2(1H)-carboxylate. m/z: $[M+H]^+$500.3; $^1H$ NMR (400 MHz, $CD_3OD$): δ 7.36 (d, $J$ = 8.4Hz, 1H), 7.24 (d, $J$ = 1.6Hz, 1H), 7.16-7.11 (m, 3H), 7.02-7.00 (m, 1 H), 6.84 (s, 1H), 3.91 (s, 2H), 3.41 (t, $J$ = 7.6Hz, 4H), 3.08 (t, $J$ = 6.0Hz, 2H), 2.78 (t, $J$ = 6.0Hz, 2H), 1.69- 1.62 (m, 4H), 1.58-1.55 (m, 2H), 1.31-1.28 (m, 1H), 1.20-1.17 (m, 2H), 1.00-0.76 (m, 7H).

**Example 16:** Synthesis of 2-amino-8-(1-((3-(aminomethyl)pyridin-3-yl)carbamoyl)cyclopropyl)-N,N-dipropyl-3H-benzo[b]azepine-4-formamide (compound 16)

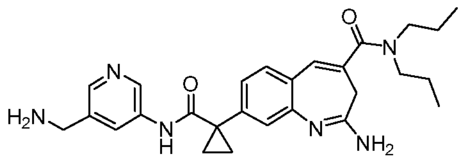

**16**

Using the synthesis method of compound 1, compound 16 was obtained as a white solid by replacing tert-butyl 3-amino-7,8-dihydro-1,6-naphthyridine-6(5H)-carboxylate in step 3 with tert-butyl((5-aminopyridin-3-yl)methyl)carbamate. m/z:[M+H]$^+$ 475.2. $^1$H NMR (400 MHz, $CD_3OD$): δ 8.53 (d, *J* = 2.4Hz, 1H), 8.24 (d, *J* = 1.6Hz, 1H), 7.93-7.90 (m, 1H), 7.36 (d, *J* = 8.0Hz, 1 H), 7.24 (d, *J* = 1.6Hz, 1H), 7.16-7.13 (m, 1H), 6.84 (s, 1H), 3.80 (s, 2H), 3.41 (t, *J* = 7.6Hz, 4H), 2.85 (d, *J* = 16.0Hz, 1H), 1.70-1.60 (m, 6H), 1.28-1.22 (m, 3H), 1.03-0.78 (m, 6H).

**Example 17:** Synthesis of 2-amino-8-(1-((4-aminobenzyl)carbamoyl)cyclopropyl)-N-(2-hydroxyethyl)-N-propyl-3H-benzo[b]azepine-4-formamide (compound 17)

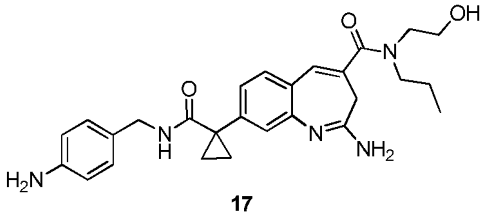

**17**

Using the synthesis method of compound 1, compound 17 was obtained as a white solid by replacing dipropylamine in step 1 with N-(2-((tert-butyldimethylsilyl)oxy)ethyl)propan-1-amine and replacing tert-butyl 3-amino-7,8-dihydro-1,6-naphthyridine-6(5H)-carboxylate in step 3 with 4-(aminomethyl)benzylamine. m/z: [M+H]$^+$ 476.2.

**Example 18:** Synthesis of 2-amino-8-(1-((3-aminobenzyl)carbamoyl)cyclopropyl)-N-(2-hydroxyethyl)-N-propyl-3H-benzo[b]azepine-4-formamide (compound 18)

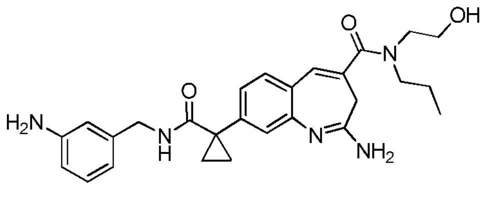

**18**

Using the synthesis method of compound 1, compound 18 was obtained as a white solid by replacing dipropylamine in step 1 with N-(2-((tert-butyldimethylsilyl)oxy)ethyl)propan-1-amine and replacing tert-butyl 3-amino-7,8-dihydro-1,6-naphthyridine-6(5H)-carboxylate in step 3 with 3-(aminomethyl)benzylamine. m/z:[M+H]$^+$ 476.2; $^1$H NMR (400 MHz, $CD_3OD$): δ 7.70-7.57 (m, 1H), 7.53 (d, *J* = 8.0Hz, 1H), 7.49-7.36 (m, 3H), 7.26-7.12 (m, 3H), 7.09 (s, 1H), 4.34 (d, *J* = 4.8Hz, 2H), 3.83-3.55 (m, 4H), 3.56- 3.40 (m, 2H), 1.75-1.61 (m, 2H), 1.60-1.52 (m, 2H), 1.37-1.24 (m, 1H), 1.23-1.13 (m, 2H), 1.05-0.76 (m, 4H).

**Example 19:** Synthesis of 2-amino-8-(1-((3-aminophenyl)carbamoyl)cyclopropyl)-N-(2-hydroxyethyl)-N-propyl-3H-benzo[b]azepine-4-formamide (compound 19)

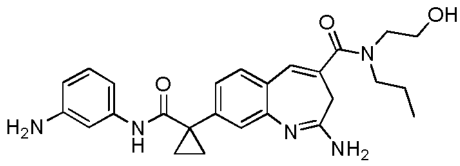

19

Using the synthesis method of compound 1, compound 19 was obtained as a white solid by replacing dipropylamine in step 1 with N-(2-((tert-butyldimethylsilyl)oxy)ethyl)propan-1-amine and replacing tert-butyl 3-amino-7,8-dihydro-1,6-naphthyridine-6(5H)-carboxylate in step 3 with benzene-1,3-diamine. m/z:$[M+H]^+$ 462.2; $^1H$ NMR (400 MHz, $CD_3OD$): δ 7.38 (d, $J$ = 8.0Hz, 1H), 7.25 (s, 1H), 7.16 (d, $J$= 8.0Hz, 1H), 7.03-6.88 (m, 2H), 6.79 (s, 1H), 6.63 (d, $J$ = 8.0Hz, 1H), 6.45 (d, $J$ = 8.0Hz, 1H), 3.75 (s, 2H), 3.65-3.39 (m, 4H), 1.79-1.47 (m, 5H), 1.38-1.24 (m, 1H), 1.23-1.11 (m, 2H), 1.00-0.76 (m, 3H).

**Example 20:** Synthesis of 2-amino-8-(1-((5-aminopyridin-3-yl)carbamoyl)cyclopropyl)-N-(2-hydroxyethyl)-N-propyl-3H-benzo[b]azepine-4-formamide (compound 20)

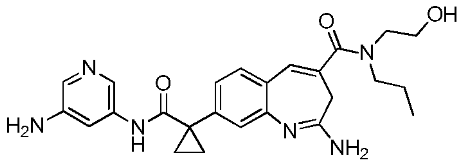

20

Using the synthesis method of compound 1, compound 20 was obtained as a white solid by replacing dipropylamine in step 1 with N-(2-((tert-butyldimethylsilyl)oxy)ethyl)propan-1-amine and replacing tert-butyl 3-amino-7,8-dihydro-1,6-naphthyridine-6(5H)-carboxylate in step 3 with pyridine-3,5-diamine. m/z:$[M+H]^+$ 463.2; $^1H$ NMR (400 MHz, $CD_3OD$): δ 8.36-8.21 (m, 1H), 7.78-7.67 (m, 2H), 7.55 (d, $J$ = 8.0Hz, 1H), 7.45 (d, $J$ = 9.0Hz, 2H), 7.10 (s, 1H), 3.84-3.55 (m, 4H), 3.54-3.39 (m, 2H), 1.77- 1.60 (m, 4H), 1.45-1.21 (m, 3H), 1.09-0.68 (m, 4H).

**Example 21:** Synthesis of 2-amino-8-(1-(3-amino-5,6,7,8-tetrahydro-1,6-naphthyridine-6-carbonyl)cyclopropyl)-N-(2-hydroxyethyl)-N-propyl-3H-benzo[b]azepine-4-formamide (compound 21)

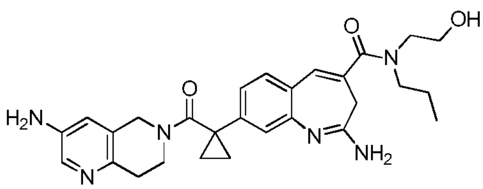

21

Using the synthesis method of compound 1, compound 21 was obtained as a white solid by replacing dipropylamine in step 1 with N-(2-((tert-butyldimethylsilyl)oxy)ethyl)propan-1-amine and replacing tert-butyl 3-amino-7,8-dihydro-1,6-naphthyridine-6(5H)-carboxylate in step 3 with 5,6,7,8-tetrahydro-1,6-naphthyridin-3-amine. m/z: $[M+H]^+$ 503.2.

**Example 22:** Synthesis of 2-amino-8-(1-(((5-aminopyridin-3-yl)methyl)carbamoyl)cyclopropyl)-N-(2-hydroxyethyl)-N-propyl-3H-benzo[b]azepine-4-formamide (compound 22)

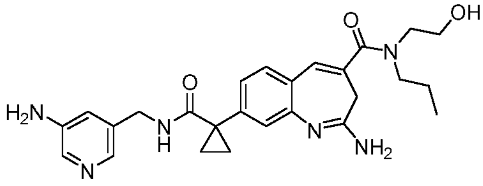

**22**

Using the synthesis method of compound 1, compound 22 was obtained as a white solid by replacing dipropylamine in step 1 with N-(2-((tert-butyldimethylsilyl)oxy)ethyl)propan-1-amine and replacing tert-butyl 3-amino-7,8-dihydro-1,6-naphthyridine-6(5H)-carboxylate in step 3 with 5-(aminomethyl)pyridin-3-amine. m/z: $[M+H]^+$477.2; $^1H$ NMR (400 MHz, $CD_3OD$): δ 7.92-7.84 (m, 1H), 7.77 (s, 1H), 7.55 (d, *J* = 8.0 Hz, 1H), 7.52-7.42 (m, 3H), 7.11 (s, 1H), 4.33 (s, 2H), 3.93-3.57 (m, 4H), 3.57-3.41 (m, 2H), 1.77-1.63 (m, 2H), 1.62-1.54 (m, 2H), 1.39-1.10 (m, 3H), 1.05-0.78 (m, 4H).

**Example 23:** Synthesis of 2-amino-N-ethoxy-N-propyl-8-(1-((5,6,7,8-tetrahydro-1,6-naphthyridin-3-yl)carbamoyl)cyclopropyl)-3H-benzo[b]azepine-4-formamide (compound 23)

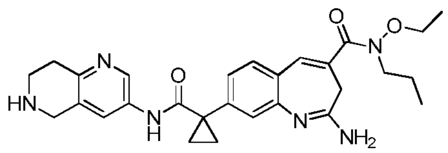

**23**

Using the synthesis method of compound 1, compound 23 was obtained as a yellow solid by replacing dipropylamine in step 1 with O-ethyl-N-propylhydroxylamine hydrochloride. m/z: $[M+H]^+$ 503.2; $^1H$ NMR (400 MHz, DMSO-$d_6$): δ 12.15 (s, 1H), 9.82 (s, 1H), 9.50 (s, 1H), 9.15 (s, 1H), 9.08 (s, 2H), 8.53 (d, *J* = 2.4Hz, 1H), 7.90 (d, *J* = 2.4Hz, 1H), 7.59-7.57 (m, 1H), 7.39- 7.36 (m, 2H), 7.28 (s, 1H), 4.29 (s, 2H), 3.89-3.84 (m, 2H), 3.66-3.62 (m, 3H), 3.32 (s, 2H), 3.02-2.99 (m, 2H), 1.67-1.62 (m, 2H), 1.56-1.53 (m, 2H), 1.23-1.19 (m, 3H), 1.09-1.05 (m, 3H), 0.93-0.89 (m, 3H).

**Example 24:** Synthesis of 2-(2-amino-N-propyl-8-(1-((5,6,7,8-tetrahydro-1,6-naphthyridin-3-yl)carbamoyl)cyclopropyl)-3H-benzo[b]azepine-4-formylamino)ethyl cyclobutylcarbamate (compound 24)

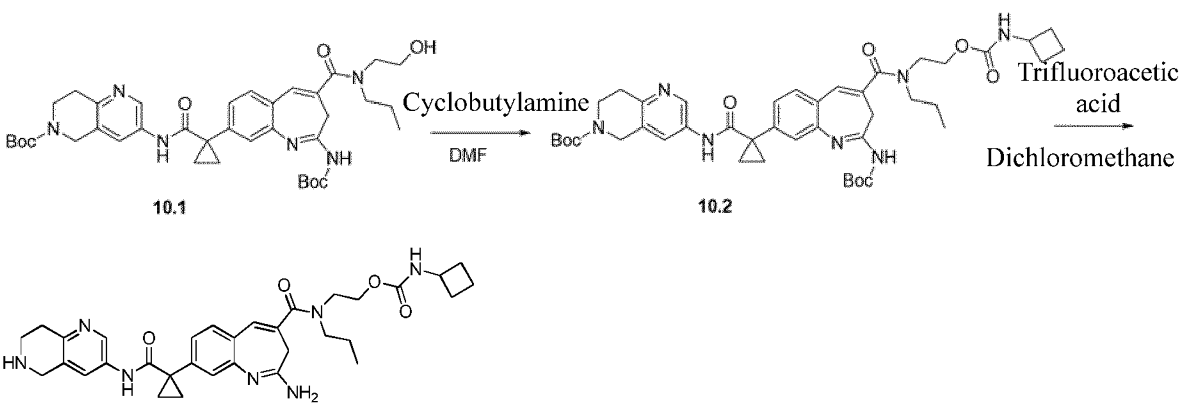


**24**

Step 1: Bis(p-nitrobenzene)carbonate (27 mg, 0.09 mmol) was added to a solution of compound 10.1 (25 mg,

0.04 mmol) and DIPEA (14 mg, 0.11 mmol) in DMF (1.0 mL) under ice bath conditions. The reaction mixture was stirred at room temperature for 1 hour, then cyclobutylamine (7.7 mg, 0.11 mmol) was added to the above reaction mixture, stirring was continued for 1 hour, the reaction was quenched by adding water, the aqueous phase was extracted with ethyl acetate, and the organic phases were combined and concentrated under reduced pressure. The residue was purified by *prep*-TLC (dichloromethane/methanol = 10/1) to give compound 10.2 (20 mg, yield: 70%) as a pale yellow solid. m/z: $[M+H]^+$ 801.4.

Step 2: To a solution of compound 10.2 (15 mg, 0.02 mmmol) in dichloromethane (1 mL) was added trifluoroacetic acid (0.5 mL), the reaction solution was stirred at room temperature for 1 hour and then directly concentrated under reduced pressure, and the residue was dissolved in acetonitrile, adjusted to pH = 8 with concentrated ammonia and then purified by Flash column chromatography (C18, eluent 0%-70% acetonitrile in a 10 mM aqueous ammonium bicarbonate solution) to give compound 24 (3.2 mg, yield: 26%) as an off-white solid. m/z: $[M+H]^+$ 600.4.

**Example 25:** Synthesis of (2S)-benzyl 2-(((2-(2-amino-N-propyl-8-(1-((5,6,7,8-tetrahydro-1,6-naphthyridin-3-oyl)carbamoyl)cyclopropyl)-3H-benzo[b]azepine-4-formylamino)ethoxy)(phenoxy)phosphoryl)amino)propanoate, isomer 1 (compound 25-1) and (2S)-benzyl 2-(((2-(2-amino-N-propyl-8-(1-((5,6,7,8-tetrahydro-1,6-naphthyridin-3-acyl)carbamoyl)cyclopropyl)-3H-benzo[b]azepine-4-fonnylamino)ethoxy)(phenoxy)phosphoryl)amino)propionate, isomer 2 (compound 25-2)

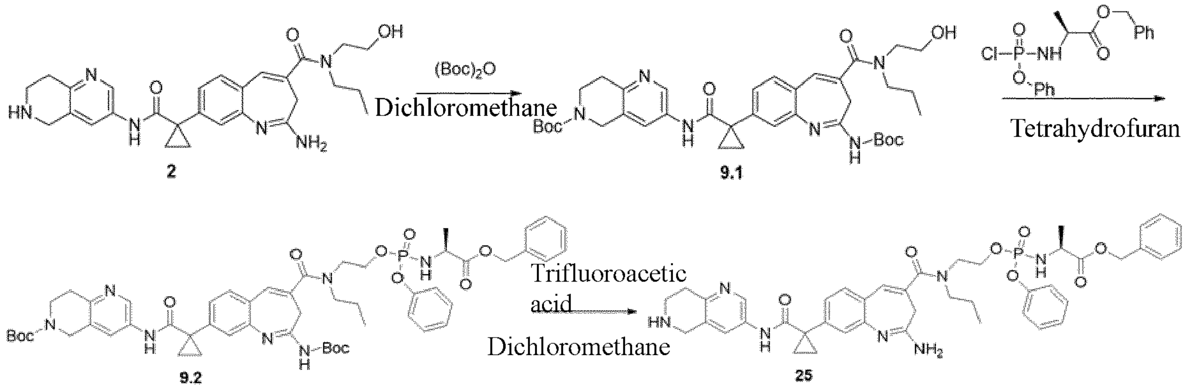

2
9.1
9.2
25

Step 1: To a solution of compound 2 (300 mg, 0.6 mmol) in dichloromethane (10 mL) were added DIPEA (390 mg, 3 mmol) and $(Boc)_2O$ (327 mg, 1.5 mmol). The reaction was stirred at room temperature overnight, the reaction was quenched with adding water, the aqueous phase was extracted with ethyl acetate, the organic phases were combined and washed with saturated brine, the organic phase was separated and concentrated under reduced pressure, and the residue was subjected to Flash column chromatography (methanol/dichloromethane = 1/10) to give compound 9.1 (294 mg, yield: 70%) as a white solid. m/z: $[M+H]^+$ 703.3.

Step 2: To a solution of compound 9.1 (100 mg, 0.14 mmol) in tetrahydrofuran (1 mL) was added a solution of isopropylmagnesium chloride in tetrahydrofuran (0.14 mL, 2 M) under ice bath conditions under nitrogen atmosphere, the reaction solution was stirred at 0°C for 20 minutes, and benzyl(2S)-2-((chloro(phenoxy)phosphoryl)amino)propionate (149 mg, 0.42 mmol) was added to the above reaction solution. The resulting mixture was stirred at room temperature for 2 hours, the reaction was quenched by adding a saturated aqueous ammonium chloride solution, the aqueous phase was extracted with ethyl acetate, and the organic phases were combined and concentrated under reduced pressure. The residue was subjected to Flash column chromatography (methanol/dichloromethane = 1/10) to give compound 9.2 (120 mg, yield: 83%) as a white solid. m/z: $[M+H]^+$ 703.3.

Step 3: To a solution of compound 9.2 (100 mg, 0.1 mmol) in dichloromethane (1 mL) was added trifluoroacetic acid (0.5 mL). The reaction solution was stirred at room temperature for 1 hour and concentrated under reduced pressure, and the residue was added with methanol (1.5 mL) and ammonia methanol solution (1 mL, 7 M) and then purified by Flash column chromatography (C18, eluent 0%-60% acetonitrile in a 10 mM aqueous ammonium bicarbonate solution) to give compound 25 (74 mg, yield: 92%) as an off-white solid. m/z: [M+H]+ 699.4.

Compound 25 was purified by *prep*-HPLC (eluent 15-85% acetonitrile in a 10 mM aqueous ammonium bicarbonate solution, elution 20 minutes) to give compounds 25-1 (4.5 mg) and 25-2 (12.0 mg), both as white solids. 25-1: m/z: $[M+H]^+$ 820.4; UPLC retention time: 5.280 minutes; and 25-2: m/z: $[M+H]^+$ 820.4; UPLC retention time: 5.388 minutes.

**Example 26:** Synthesis of 2-amino-8-(1-((6-(2-hydroxyacetyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound 26)

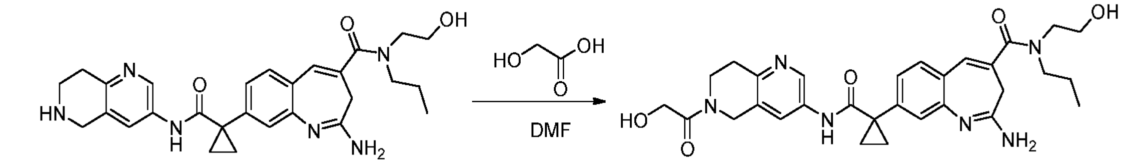


2 26

To a solution of compound 2 (19.6 mg, 0.04 mmol) and 2-glycoacetic acid (3 mg, 0.04 mmol) in DMF (2 mL) were added HATU (17.8 mg, 0.05 mmol) and DIPEA (7.6 mg, 0.06 mmol), and the reaction solution was stirred at room temperature for 1 hour and then directly purified by *prep*-HPLC (eluent 5-40% acetonitrile in a 0.1% aqueous trifluoroacetic acid solution, 20 minutes) to give compound 26 (6.85 mg, yield: 31%) as a white solid m/z: $[M+H]^+$ 561.2.

**Example 27:** Synthesis of 2-amino-N,N-dipropyl-8-(1-((5,6,7,8-tetrahydropyrido[4,3-c]pyrazin-3-yl)carbamoyl)cyclopropyl)-3H-benzo[b]azepine-4-formamide (compound 27)

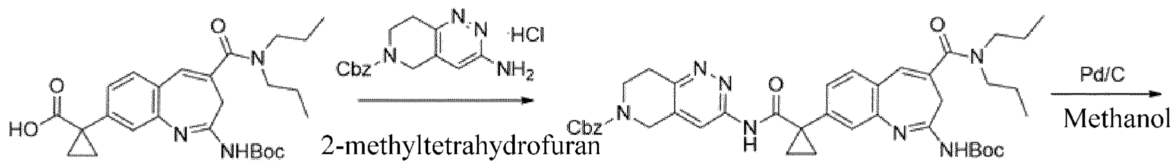


2.9 11.1

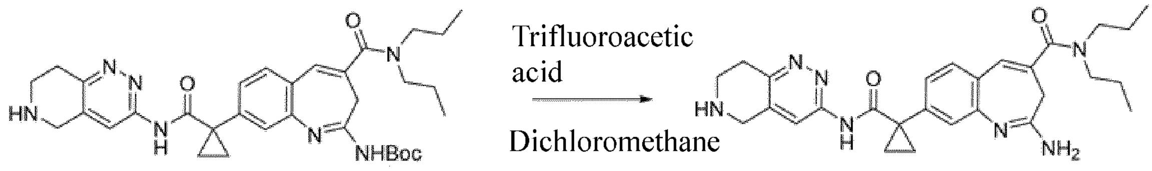


11.2 27

Step 1: A solution of compound 2.9 (200 mg, 0.43 mmol), benzyl 3-amino-7,8-dihydropyrido[4,3-c]pyrazine-6(5H)-carboxylate hydrochloride (135 mg, 0.47 mmol), 2-chloro-1-methylpyridine iodide (330 mg, 1.29 mmol) and DIPEA (278 mg, 2.15 mmol) in 2-methyltetrahydrofuran (10 mL) was stirred at room temperature for 2 days, the reaction was quenched with a protected aqueous ammonium chloride solution, the aqueous phase was extracted with ethyl acetate, and the organic phases were combined and concentrated under reduced pressure. The residue was purified by Flash column chromatography (C18, eluent 0-70% acetonitrile in a 0.1% aqueous trifluoroacetic acid solution) to give compound 11.1 (25 mg, yield: 8%) as a yellow solid. m/z: $[M+H]^+$ 736.4.

Step 2: To a solution of compound 11.1 (20 mg, 0.03 mmol) and Pd/C (10%wt, 5 mg) in methanol was added 1 drop of acetic acid, and the reaction system was replaced three times with nitrogen and hydrogen each and then stirred at room temperature under hydrogen atmosphere for 2 hours. The reaction system was directly concentrated under reduced pressure, and the residue was purified by Flash column chromatography (C18, eluent 0-50% acetonitrile in a 10 mM aqueous ammonium bicarbonate solution) to give compound 11.2 (10 mg, yield: 61%) as a yellow solid. m/z: $[M+H]^+$ 602.3.

Step 3: To a solution of compound 11.2 (10 mg, 0.02 mmol) in dichloromethane (2 mL) was added trifluoroacetic acid (1 mL). The reaction solution was stirred at room temperature for 1 hour and concentrated directly under reduced pressure, and the residue was purified by *prep*-HPLC (eluent 10%-45% acetonitrile in a 0.1% aqueous trifluoroacetic acid solution, 20 minutes) to give compound 27 (1.98 mg, yield: 18%) as a yellow solid. m/z: $[M+H]^+$ 502.2; $^1H$ NMR (400 MHz,$CD_3OD$): δ 8.26 (s, 1H), 7.52 (d, *J* = 8.0Hz, 1H), 7.47-7.43 (m, 2H), 6.97 (s, 1H), 4.40 (s, 1H), 3.55 (t, *J* = 6.4Hz, 2H), 3.86-3.32 (m, 4H), 3.26-3.22 (m, 3H), 2.99 (s, 1H), 1.66-1.55 (m, 6H), 1.27-1.19 (m, 3H), 0.87-0.73 (m, 6H).

**Example 28:** Synthesis of 2-amino-8-(1-((6-(3-(2-(2-aminoethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound 28)

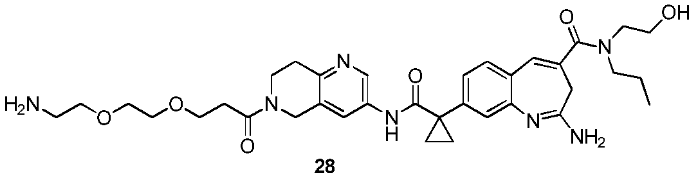

28

Using the synthesis method of compound 26, compound 28 was obtained as a white solid by reacting compound 2 with 2,2-dimethyl-4-oxo-3,8,11-trioxa-5-azatetradecan-14-oic acid. m/z: $[M+H]^+$ 662.4.

**Example 29:** Synthesis of 2-amino-8-(1-((6-(3-(2-(2-hydroxyethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound 28)

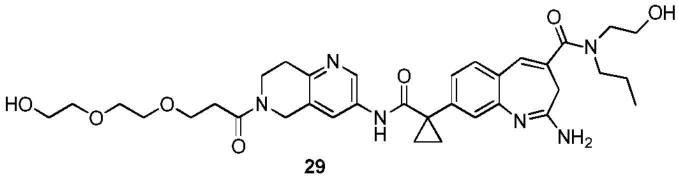

29

Using the synthesis method of step 1 of compound 26, compound 29 was obtained as a white solid by reacting compound 2 with 3-(2-(2-hydroxyethoxy)ethoxy)propionic acid. m/z: $[M+H]^+$ 663.4.

**Example 30:** Synthesis of 2-amino-8-(1-((6-(1-amino-3,6,9,12,15,18-hexaoxaheneicosanyl-21-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound 30)

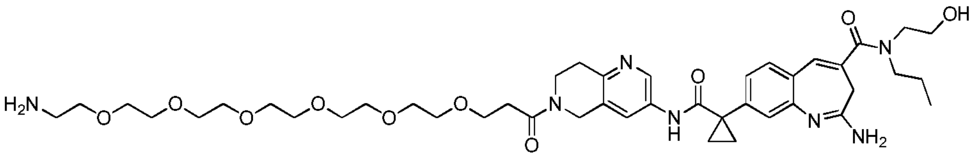

30

Using the synthesis method of compound 26, compound 30 was obtained as a white solid by reacting compound 2 with 2,2-dimethyl-4-oxo-3,8,11,14,17,20,23-hexaoxa-5-azahexacosyl-26-oic acid. m/z: $[M+H]^+$ 838.4.

**Example 31:** Synthesis of 22-(3-(1-(2-amino-4-((2-hydroxyethyl)(propyl)carbamoyl)-3H-benzo[b]azepin-8-yl)cyclopropylcarboxamido)-7,8-dihydro-1,6-naphthyridin-6(5H)-yl)-22-oxo-4,7,10,13,16,19-hexaoxadocosyl-1-carboxylic acid (compound 31)

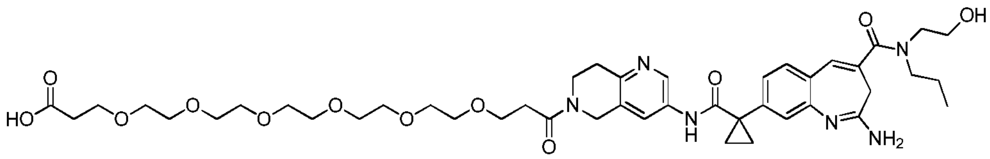

31

Using the synthesis method in step 1 of compound 26, compound 31 was obtained as a white solid by reacting compound 2 with 4,7,10,13,16,19-hexaoxadocosyl-1,22-dicarboxylic acid. m/z: $[M+H]^+$ 868.4.

**Example 32:** Synthesis of 2-amino-8-(1-((6-(32-amino-3,6,9,12,15,18,21,24,27,30-decaoxadotriacontyl)-3,6,7,8-tetrahydro-1,6-naphthyridin-3-yl)carbamoyl)cyclopropyl)-N-(2-hydroxyethyl)-N-propyl-3H-benzo[b]azepine-4-formamide (compound 32)

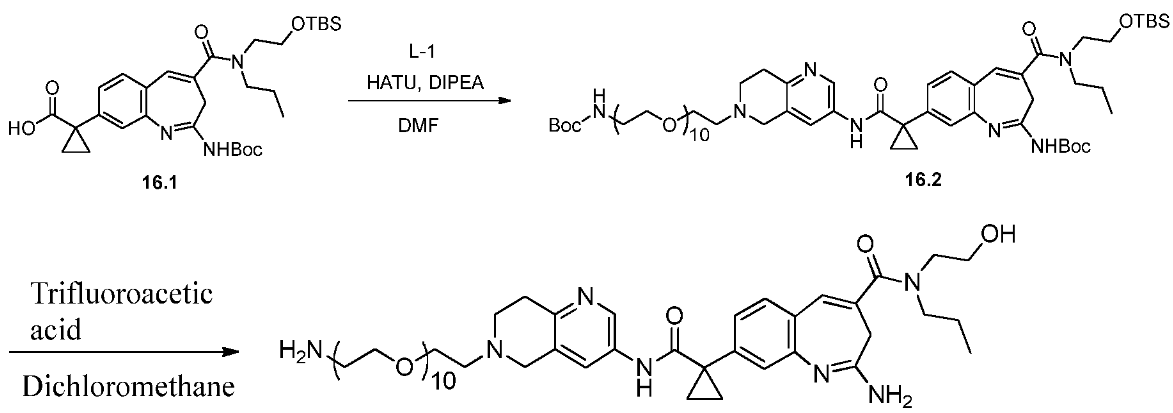


16.1

16.2

32

Step 1: To a solution of compound L-1 (110 mg, 0.15 mmol) in DMF (2 mL) were added 16.1 (refer to the synthesis method of intermediate 6.5) (87 mg, 0.15 mmol), DIEPA (58 mg, 0.45 mmol) and HATU (85 mg, 0.22 mmol) sequentially. The reaction solution was stirred at room temperature for 2 hours and then directly purified by Flash column chromatography (C18, eluent 0%-75% acetonitrile in a 10 mM aqueous ammonium bicarbonate solution) to give compound 16.2 (64 mg, yield: 33%) as a light yellow oil. m/z: $[M+H]^+$ 1300.7.

Step 2: To a solution of intermediate 16.2 (64 mg, 0.05 mmol) in dichloromethane (2 mL) was added trifluoroacetic acid (1 mL). The reaction solution was stirred at room temperature for 2 hours and concentrated directly under reduced pressure, and the residue was neutralized with an ammonia methanol solution (7 M), concentrated under reduced pressure and then purified by Flash column chromatography (C18, eluent 0%-55% acetonitrile in a 10 mM aqueous ammonium bicarbonate solution) to give compound 32 (42 mg, yield: 87%) as an off-white solid. m/z: $[M+H]^+$ 986.3.

**Example 33:** Synthesis of 2-amino-8-(1-((6-((S)-2-((S)-2-amino-3-methylbutanoylamino)-5-ureidopentanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound A-1)

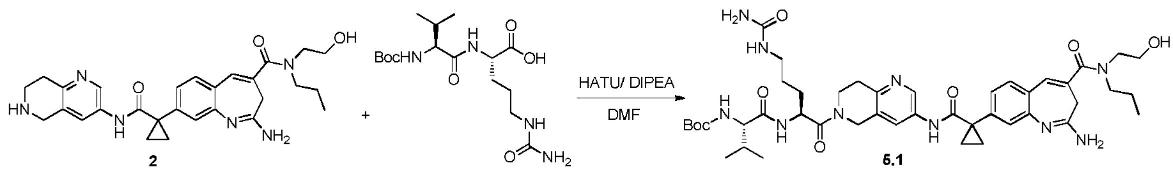


2

5.1

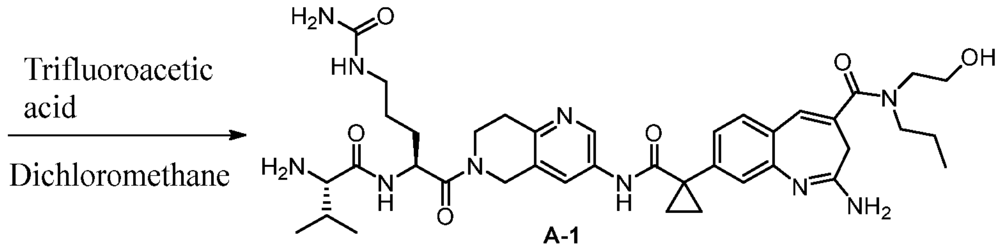


A-1

Step 1: To a solution of Boc-Val-Cit-OH (100 mg, 0.27 mmol) in DMF (3 mL) were added HATU (120 mg, 0.32 mmol), compound 2 (140 mg, 0.27 mmol) and DIPEA (52 mg, 0.41 mmol) sequentially. The reaction solution was stirred at room temperature for 1.5 hours and then directly purified by Flash column chromatography (C18, eluent 0%-55% acetonitrile in a 10 mM aqueous ammonium bicarbonate solution) to give compound 5.1 (130 mg, yield: 57%) as a white solid. m/z: $[M+H]^+$ 859.4.

Step 2: To a solution of compound 5.1 (80 mg, 0.09 mmol) in dichloromethane (3 mL) was added trifluoroacetic acid (1 mL) dropwise under ice bath conditions. The reaction solution was stirred at room temperature for 2 hours and concentrated under reduced pressure, and the residue was purified by Flash column chromatography (C18, eluent 0%-30% acetonitrile in a 10 mM aqueous ammonium bicarbonate solution) to give compound A-1 (53 mg, yield: 75%) as a white solid. m/z: $[M+H]^+$ 759.4.

**Example 34:** Synthesis of 2-amino-N-(2-hydroxyethyl)-8-(1-((6-((S)-3-methyl-2-((R)-pyrrolidine-2-formamide)butanoyl)-5,6,7,8-tetrahydro-1,6-naphthyridin-3-yl)carbamoyl)cyclopropyl)-N-propyl-3H-benzo[b]azepine-4-formamide (compound A-2)

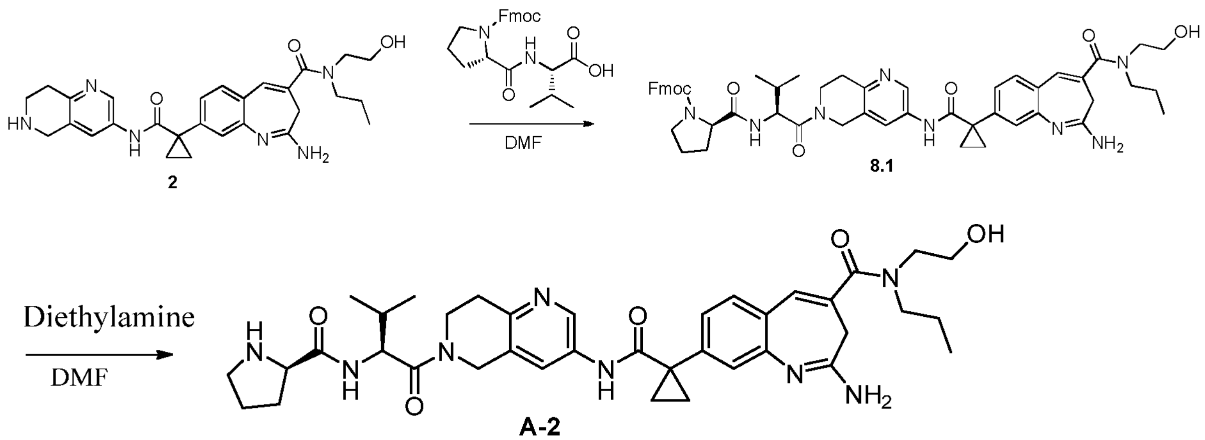


2

8.1

A-2

Step 1: To a solution of (S)-2-((S)-1-(((9H-fluoren-9-yl)methoxy)carbonyl)pyrrolidine-2-formylamino)-3-methylbutanoic acid (90 mg, 0.21 mmol) in DMF (2 mL) were added HATU (104 mg, 0.27 mmol), compound 2 (106 mg, 0.21 mmol) and DIPEA (48.9 mg, 0.38 mmol) sequentially. The reaction solution was stirred at room temperature for 2 hours, the reaction was quenched with adding water, the organic phases were extracted with ethyl acetate, the organic phases were combined and concentrated under reduced pressure, and the residue was purified by *prep*-TLC (methanol/dichloromethane = 1/10) to give compound 8.1 (120 mg, yield: 63%) as a white solid. m/z: $[1/2M+H]^+$ 461.4.

Step 2: To a solution of compound 8.1 (120 mg, 0.13 mmol) in dichloromethane (5 mL) was added diethylamine (1 mL) dropwise under ice bath conditions. The reaction solution was stirred at room temperature for 3 hours and concentrated under reduced pressure, and the residue was purified by Flash column chromatography (C18, eluent 0%-60% acetonitrile in a 0.1% aqueous trifluoroacetic acid solution) to give compound A-2 (55 mg, yield: 60%) as a white solid. m/z: $[M+H]^+$ 699.4.

**Example 35:** Synthesis of (S)-1-((1-(3-(1-(2-amino-4-((2-hydroxyethyl)(propyl)carbamoyl)-3H-benzo[b]azepin-8-yl)cyclopropyl-1-amido)-7,8-dihydro-1,6-naphthyridin-6(5H)-yl)-1-oxo-5-ureidopentan-2-yl)carbamoylamino)cyclobutyl-1-carboxylic acid (A-3)

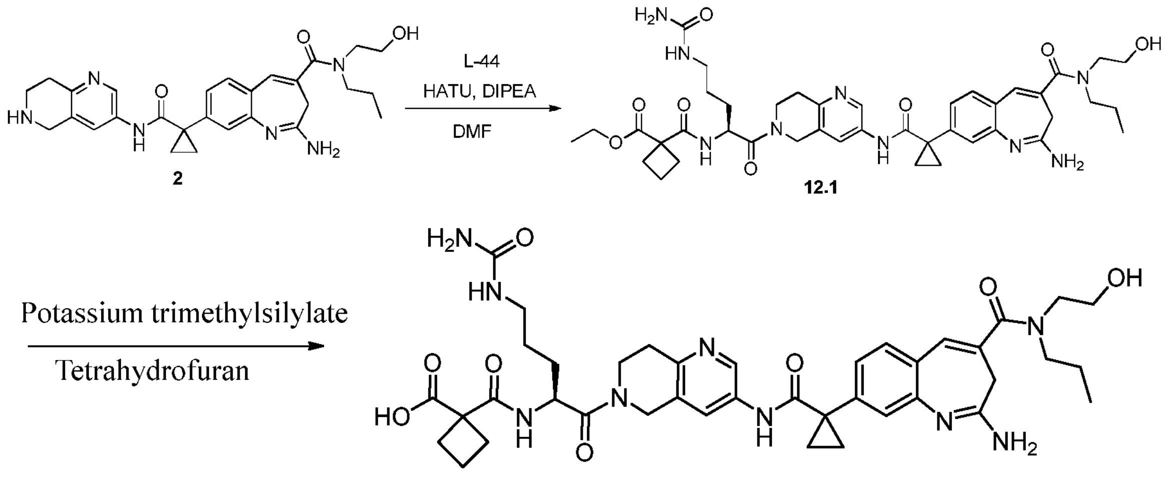


2 12.1 A-3

Step 1: To a solution of compound L-44 (150 mg, 0.46 mmol) in DMF (2 mL) were added compound 2 (231 mg, 0.46 mmol), DIEPA (178 mg, 1.38 mmol) and HATU (359 mg, 0.69 mmol) sequentially. The reaction solution was stirred at room temperature for 3 hours and then directly purified by Flash column chromatography (C18, eluent 0% to 30% acetonitrile in a 0.1% aqueous trifluoroacetic acid solution) to give compound 12.1 (230 mg, yield: 62%) as a yellow solid. m/z: $[M+H]^+$ 814.4.

Step 2: To a solution of compound 12.1 (210 mg, 0.26 mmol) in tetrahydrofuran (5 mL) was added potassium trimethylsilylate (100 mg, 0.78 mmol). The reaction solution was stirred at room temperature for 2 hours and concentrated directly under reduced pressure, and the residue was purified by Flash column chromatography (C18, eluent 0% to 30% acetonitrile in a 0.1% aqueous trifluoroacetic acid solution) to give compound A-3 (100 mg, yield: 49%) as a white solid. m/z: $[M+H]^+$ 786.4.

**Example 36:** Synthesis of 2-amino-8-(1-((4-((S)-2-((S)-2-amino-3-methylbutanoylamino)-5-ureidopentanoylamino) benzyl)carbamoyl)cyclopropyl)-N-(2-hydroxyethyl)-N-propyl-3H-benzo[b]azepine-4-formamide (compound A-4)

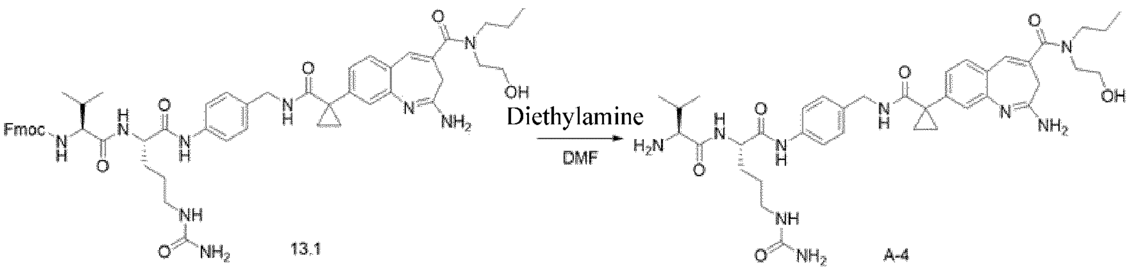


13.1 A-4

To a solution of compound 13.1 (40.8 mg, 0.04 mmol) (obtained by reacting L-58 with compound 16.1 using the synthesis method of compound 32) in DMF (2 mL) was added diethylamine (0.4 mL), and the reaction system was stirred at room temperature for 1 hour. The reaction solution was directly purified by Flash column chromatography (C18, eluent 0% to 34% acetonitrile in a 0.05% aqueous trifluoroacetic acid solution) to give compound A-4 (21.7 mg, yield: 69%) as a white solid. m/z:$[M+H]^+$ 732.4.

**Example 37:** Synthesis of (S)-2-amino-8-(1-((6-(2-amino-5-ureidopyvaloyl)-5,6,7,8-tetrahydro-1,6-naphthyridin-3-yl) carbamoyl)cyclopropyl)-N-(2-hydroxyethyl)-N-propyl-3H-benzo[b]azepine-4-formamide (compound A-5)

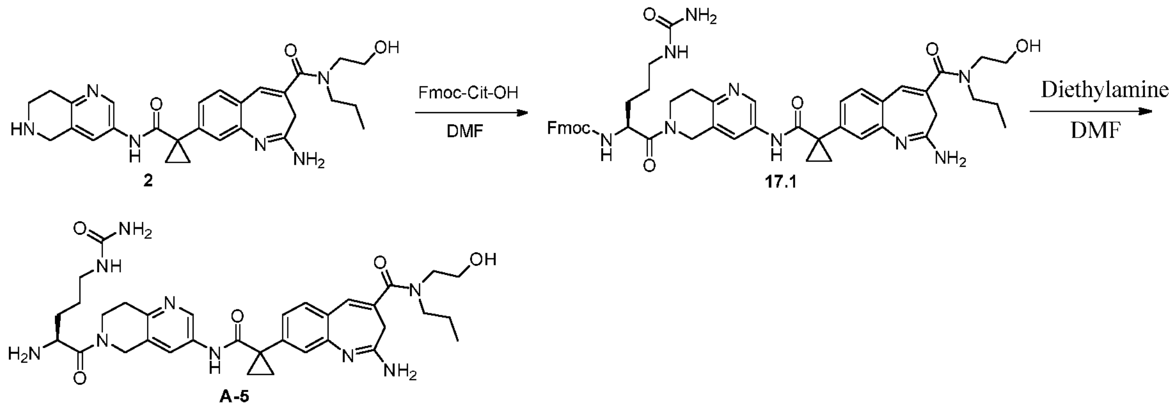


2 17.1 A-5

Step 1: To a solution of Fmoc-Cit-OH (60 mg, 0.15 mmol) and compound 2 (75.4 mg, 0.15 mmol) in DMF (3 mL) were added HOBT (24.3 mg, 0.18 mmol) and EDCI (34.5 mg, 0.18 mmol) sequentially under ice bath conditions. The reaction solution was stirred at room temperature overnight and directly purified by Flash column chromatography (C18, eluent 0-50% acetonitrile in a 0.1% aqueous trifluoroacetic acid solution) to give compound 17.1 (40 mg, yield: 30%) as a white solid. m/z: $[1/2M+H]^+$ 441.8.

Step 2: To a solution of compound 17.1 (40 mg, 0.05 mmol) in DMF (2 mL) was added diethylamine (0.5 mL). The reaction solution was stirred at room temperature for 1 hour and concentrated under reduced pressure. The residue was purified by Flash column chromatography (C18, eluent 0-60% acetonitrile in a 10 mM aqueous ammonium bicarbonate solution) to give compound A-5 (13 mg, yield: 43%) as a white solid. m/z: $[1/2M+H]^+$ 330.8.

**Example 38:** Synthesis of 8-(1-((6-($N^2$-L-alanyl-L-alanyl-$N^4$-trityl-L-asparagyl)-5,6,7,8-tetrahydro-1,6-naphthyridin-3-yl)carbamoyl)cyclopropyl)-2-amino-N-(2-hydroxyethyl)-N-propyl-3H-benzo[b]azepine-4-formamide (compound A-6)

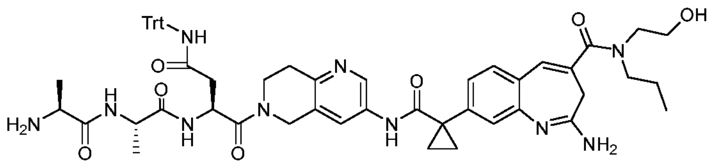

A-6

Using the synthesis method of compound A-5, compound A-6 was obtained as a white solid by reacting L-59 with compound 2. m/z:$[M+H]^+$ 1001.6.

**Example 39:** Synthesis of 2-amino-8-(1-((3-((S)-2-((S)-2-amino-3-methylbutanoylamino)-5-ureidopentanoylamino) benzyl)carbamoyl)cyclopropyl)-N-(2-hydroxyethyl)-N-propyl-3H-benzo[b]azepine-4-formamide (compound A-7)

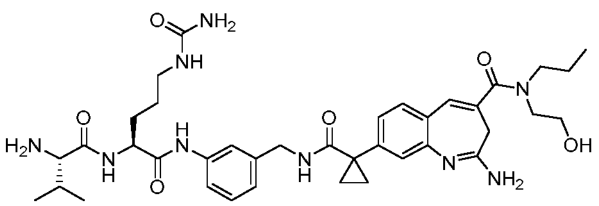

A-7

Using the synthesis method of compound A-4, compound A-7 was obtained as a white solid by reacting L-61 with compound 16.1. m/z: $[M+H]^+$ 732.5.

**Example 40:** Synthesis of (S)-2-amino-8-(1-((6-(1-amino-7-benzyl-2,5,8,11-tetraoxo-14,17,20-trioxa-3,6,9,12-tetraazatricosan-23-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound A-8)

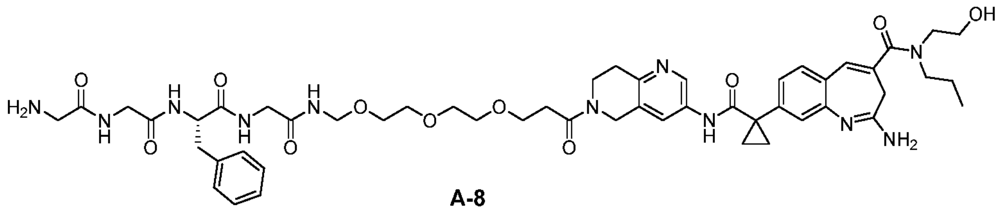

**A-8**

Using the synthesis method of compound A-4, compound A-8 was obtained as a white solid by reacting L-62 with compound 2. m/z:[1/2M+H]$^+$ 505.4.

**Example 41:** Synthesis of 2-amino-8-(1-((6-(glycylglycyl-L-phenylalanylglycyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound A-9)

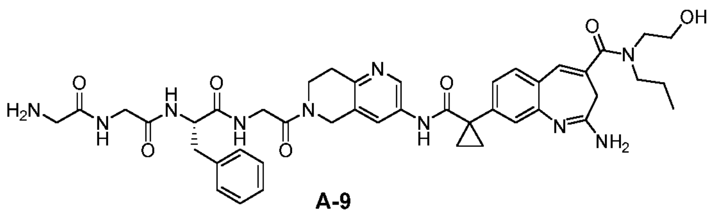

**A-9**

Using the synthesis method of compound A-2, compound A-9 was obtained as a white solid by reacting Fmoc-Gly-Gly-Phe-Gly-OH with compound 2. m/z:[M+H]$^+$821.4.

**Example 42:** Synthesis of 2-(3-(1-(2-amino-4-((2-hydroxyethyl)(propyl)carbamoyl)-3H-benzo[b]azepin-8-yl)cyclopropane-1-formamide)-7,8-dihydro-1,6-naphthyridin-6(5H)-yl)-2-oxoethyl(S)-(14-amino-11-benzyl-4,7,10,13-tetraoxo-3,6,9,12-tetraazamyristyl)(2-(methylsulfonyl)ethyl)carbamate (compound A-10) and synthesis of 2-(3-(1-(2-amino-4-((2-hydroxyethyl)(propyl)carbamoyl)-3H-benzo[b]azepin-8-yl)cyclopropane-1-formamide)-7,8-dihydro-1,6-naphthyridin-6(5H)-yl)-2-oxoethyl((41S)-32-amino-41-benzyl-26,33,36,39,42,45-hexaoxo-2,5,8,11,14,17,20,23-decazepin-27,34,37,40,43,46-hexaazaoctatetracontan-48-yl)(2-(methylsulfonyl)ethyl)carbamate (compound A-12)

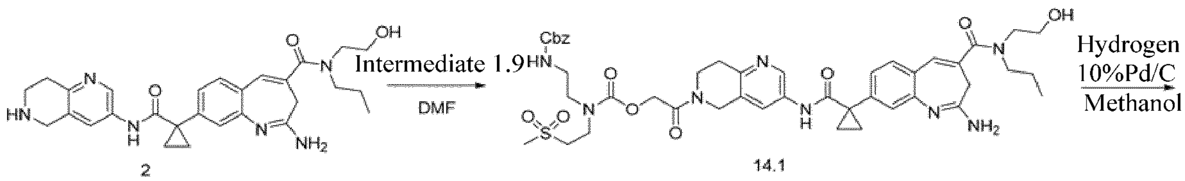


2

14.1

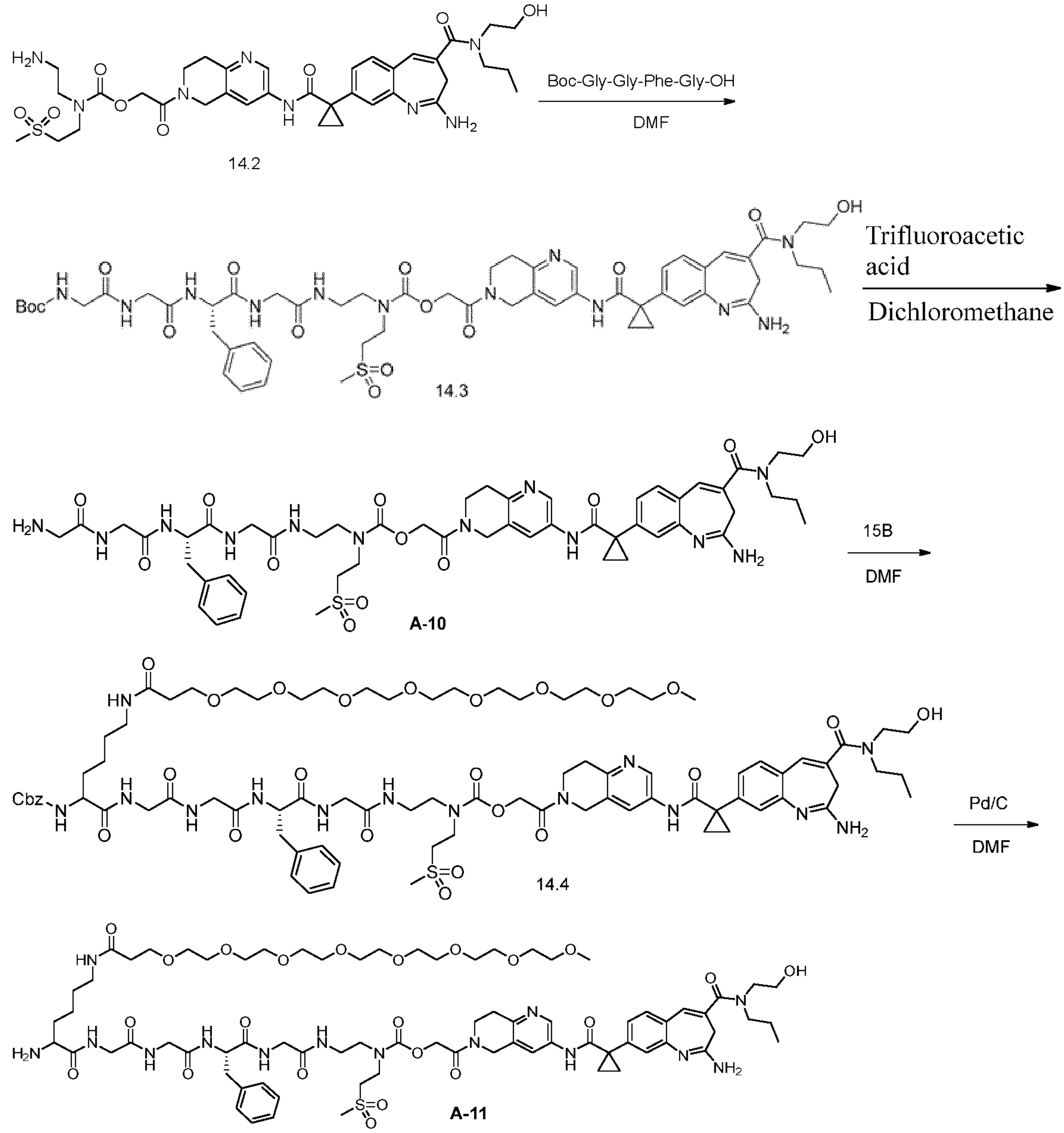


Step 1: To a solution of compound 2 (106 mg, 0.21 mmol) and intermediate 1.9 (80 mg, 0.2 mmol) in DMF (3 mL) were added HATU (99 mg, 0.26 mmol) and DIEPA (39 mg, 0.3 mmol). The reaction solution was stirred at room temperature for 1 hour and then concentrated directly under reduced pressure, and the residue was purified by Flash column chromatography (C18, eluent 10% to 40% acetonitrile in a 0.1% aqueous trifluoroacetic acid solution) to give compound 14.1 (175 mg, yield: 100%) as a white solid. m/z: $[1/2M+H]^+$ 444.3.

Step 2: To a solution of compound 14.1 (140 mg, 0.16 mmol) in methanol (4 mL) was added palladium on carbon (10%wt, 30 mg). The reaction system was stirred under hydrogen atmosphere at room temperature for 1 hour and filtered through diatomaceous earth, and the filtrate was concentrated under reduced pressure to give compound 14.2 (100 mg, yield: 84%) as a yellow solid. m/z: $[M+H]^+$ 753.2.

Steps 3 & 4: Using the synthesis method of compound 26, compound A-10 was obtained as a white solid by reacting compound 14.2 with Boc-Gly-Gly-Phe-Gly-OH. m/z:$[1/2M+H]^+$536.3. Steps 5 & 6: Using the synthesis method of compound 14.2, A-11 was obtained as a white solid by reacting compounds A-10 with 15B. m/z:$[1/2M+H]^+$798.0.

**Example 43:** Synthesis of 2-amino-8-(1-((6-(2-(aminomethyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compoundA-12)

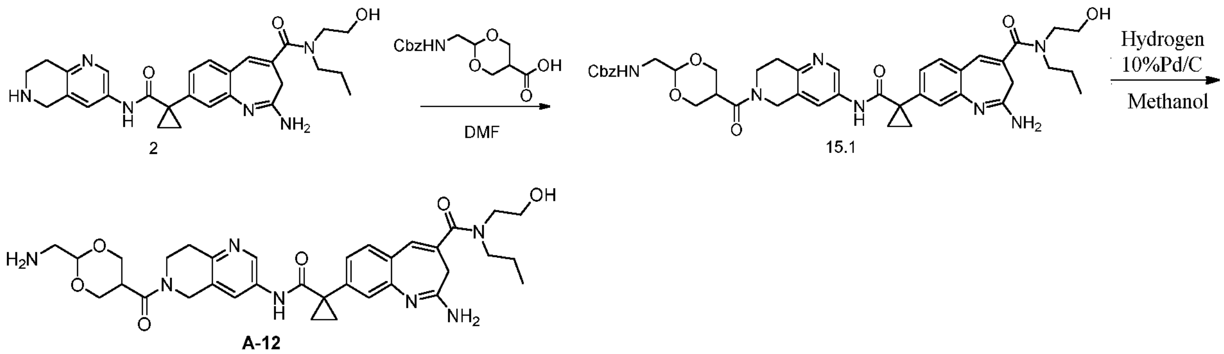


2

15.1

A-12

Using the synthesis method in steps 1 and 2 of compound A-10, compound A-12 was obtained as a white solid by reacting compound 2 with 2-((((benzyloxy)carbonyl)amino)methyl)-1,3-dioxane-5-carboxylic acid. m/z: $[M+H]^+$ 646.2.

**Example 44:** Synthesis of 2-amino-8-(1-((6-glycyl-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound A-23)

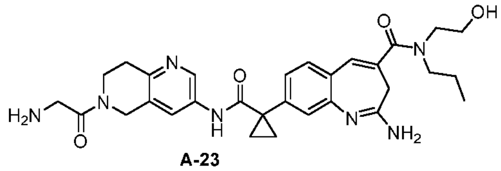

A-23

Using the synthesis method of compound A-2, compound A-23 was obtained as a white solid by reacting compound 2 with Fmoc-Gly-OH. m/z: $[M+H]^+$ 560.2.

**Example 45:** Synthesis of 8-(1-((6-(L-valyl-L-alanylglycyl)-5,6,7,8-tetrahydro-1,6-naphthyridin-3-yl)carbamoyl)cyclopropyl)-2-amino-N-(2-hydroxyethyl)-N-propyl-3H-benzo[b]azepine-4-formamide (compound A-24)

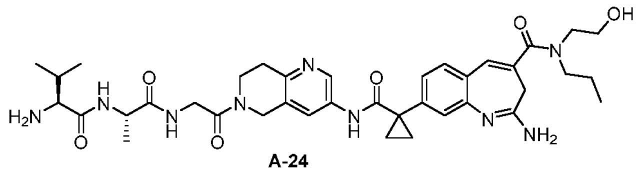

A-24

Using the synthesis method of compound A-2, compound A-24 was obtained as a white solid by reacting compound A-23 with Fmoc-Val-Ala-OH. m/z: $[M+H]^+$ 730.4.

**Example 46:** Synthesis of tert-butyl(S)-4-amino-5-(((S)-1-(((S)-1-((2-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)-2-oxoethyl)amino)-1-oxopropyl-2-yl)amino)-3-methyl-1-oxobutan-2-yl)amino)-5-oxopentanoate (compound A-25)

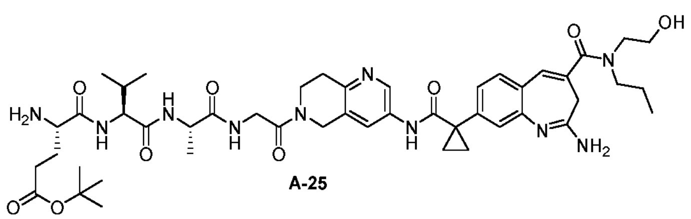

A-25

Using the synthesis method of compound A-2, compound A-25 was obtained as a white solid by reacting compound A-24 with Fmoc-Glu(OtBu)-OH. m/z: $[M+H]^+$ 915.2.

**Example 47:** Synthesis of tert-butyl(S)-4-amino-5-((2-(3-(1-(2-amino-4-((2-hydroxyethyl)(propyl)carbamoyl)-3H-benzo [b]azepin-8-yl)cyclopropyl-1-formylamino)-7,8-dihydro-1,6-naphthyridin-6(5H)-yl)-2-oxoethyl)amino)-5-oxopentanoate (compound A-26)

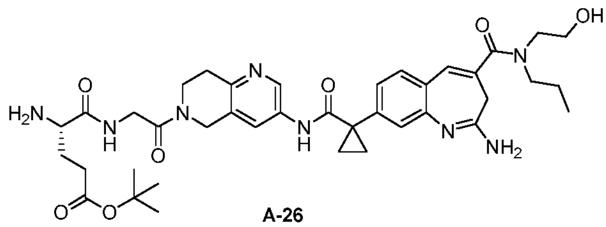

A-26

Using the synthesis method of compound A-2, compound A-26 was obtained as a white solid by reacting compound A-23 with Fmoc-Glu(OtBu)-OH. m/z: $[M+H]^+$ 745.4.

**Example 48:** Synthesis of tert-butyl(S)-5-((2-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)-2-oxoethyl)amino)-4-((S)-2-((R)-2-amino-4-methylpentanoylamino)propionylamino)-5-oxopentanoate (compound A-27)

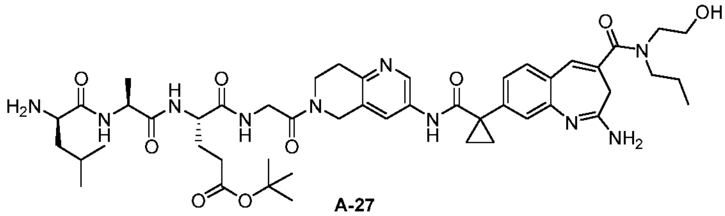

A-27

Using the synthesis method of compound A-2, compound A-27 was obtained as a white solid by reacting compound A-26 with (((9H-fluoren-9-yl)methoxy)carbonyl)-D-leucine-L-alanine. m/z: $[M+H]^+$ 929.6.

**Example 49:** Synthesis of 2-amino-8-(1-((6-(2-(2,5-dioxo-2,5-dihydro-1H-pyrrol-1-yl)ethyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (compound II-1)

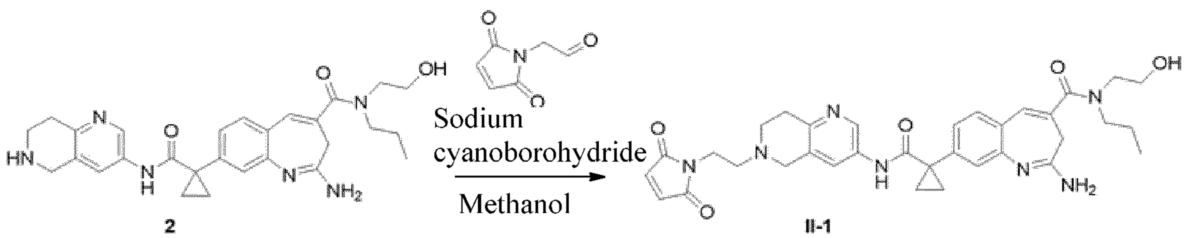


2

II-1

To a solution of compound 2 (108 mg, 0.21 mmol) in methanol (3 mL) were added 2-(2,5-dioxo-2,5-dihydro-1H-pyrrol-1-yl) acetaldehyde (58 mg, 0.42 mmol), a drop of acetic acid and sodium cyanoborohydride (19 mg, 0.32 mmol) sequentially, the reaction solution was stirred at room temperature overnight, and the reaction solution was directly purified by Flash column chromatography (C18, eluent 0%-20% acetonitrile in a 10 mM aqueous ammonium bicarbonate solution) to give compound II-1 (70 mg, yield: 32%) as a white solid. m/z: $[M+H]^+$ 626.4.

**Example 50:** Synthesis of 2-amino-8-(1-((6-(2-(2,5-dioxo-2,5-dihydro-1H-pyrrol-1-yl)acetyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-2)

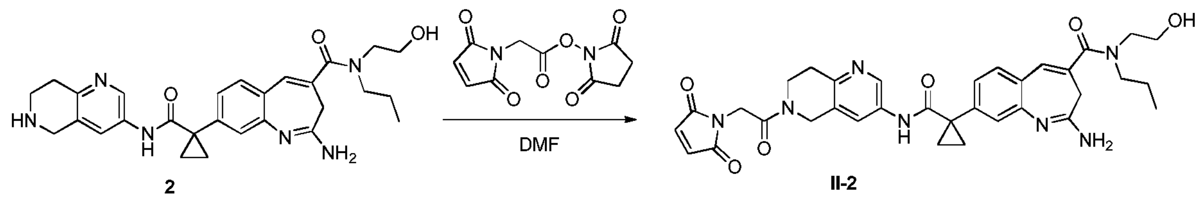


2

II-2

To a solution of compound 2 (43 mg, 0.09 mmol) in DMF (1 mL) were added AMAS (24 mg, 0.09 mmol) and DIPEA (33 mg, 0.26 mmol), the reaction solution was stirred at room temperature for 2 hours, and the reaction solution was directly purified by Flash column chromatography (C18, eluent 0%-40% acetonitrile in a 0.1% aqueous trifluoroacetic acid solution) to give compound II-2 (32 mg, yield: 56%) as a white solid. m/z: $[M+H]^+$ 640.1.

**Example 51:** Synthesis of 2-amino-8-(1-((6-(32-(2,5-dioxo-2,5-dihydro-1H-pyrrol-1-yl)-3,6,9,12,15,18,21,24,27,30-decaoxadotriacontyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-3)

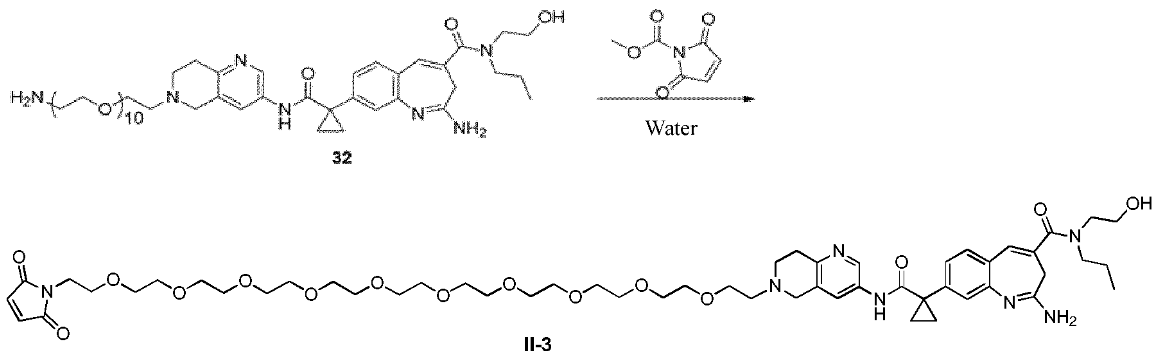


32

II-3

To a solution of compound 32 (42 mg, 0.04 mmol) in water (1 mL) were added sodium bicarbonate (15 mg, 0.17 mmol) and methyl 2,5-dioxo-2,5-dihydro-1H-pyrrole-1-carboxylate (10 mg, 0.07 mmol) sequentially under ice bath conditions. The reaction solution was stirred at 0°C for 3 hours and then directly purified by Flash column chromatography (C18, eluent 0%-55% acetonitrile in a 10 mM aqueous ammonium bicarbonate solution) to give compound II-3 (6 mg, yield: 13%) as a white solid. m/z: $[1/2M+H]^+$ 533.8.

**Example 52:** Synthesis of 2-amino-8-(1-((6-(4-((2,5-dioxo-2,5-dihydro-1H-pyrrol-1-yl)methyl)cyclohexane-1-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-4)

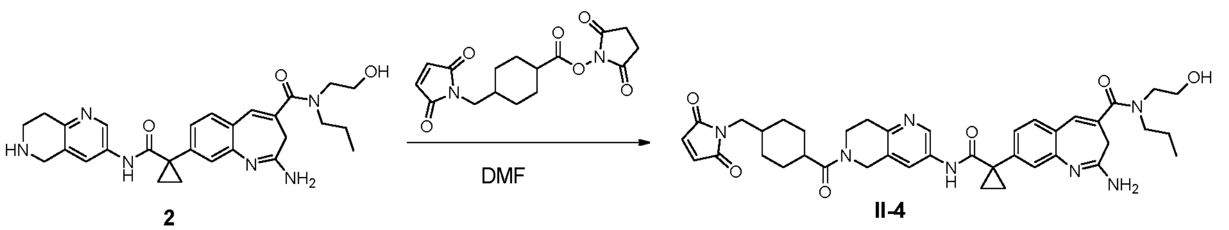


2

II-4

To a solution of 2,5-dioxopyrrolidin-1-yl 4-((2,5-dioxo-2,5-dihydro-1H-pyrrol-1-yl)methyl)cyclohexanecarboxylate (32 mg, 0.09 mmol) in DMF (3 mL) were added DIPEA (21 mg, 0.16 mmol) and compound 2 (40 mg, 0.08 mmol)

sequentially. The reaction solution was stirred at room temperature for 2 hours and then directly purified by *prep*-HPLC (eluent 5%-45% acetonitrile in a 0.1% aqueous trifluoroacetic acid solution, 20 minutes) to give compound II-4 (10.6 mg, yield: 18%) as a white solid. m/z: $[M+H]^+$ 722.3.

**Example 53:** Synthesis of 2-amino-8-(1-((6-(3-(2,5-dioxo-2,5-dihydro-1H-pyrrol-1-yl)propionyl)-5,6,7, 8-tetrahydro-1,6-naphthyridin-3-yl)carbamoyl)cyclopropyl)-N-(2-hydroxyethyl)-N-propyl-3H-benzo[b]azepine-4-formamide (II-5)

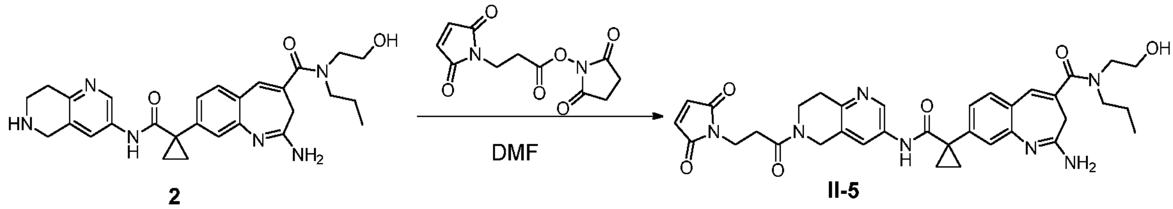


2

II-5

To a solution of BMPS (22 mg, 0.08 mmol) in DMF (3 mL) was added compound 2 (35 mg, 0.07 mmol). The reaction solution was stirred at room temperature for 1 hour and then directly purified by *prep*-HPLC (eluent 5%-45% acetonitrile in a 0.1% aqueous trifluoroacetic acid solution, 20 minutes) to give compound II-5 (23.6 mg, yield: 52%) as a white solid. m/z: $[M+H]^+$ 722.3. m/z: $[M+H]^+$ 654.2.

**Example 54:** Synthesis of 2-amino-8-(1-((6-(3-(2-(2-(2,5-dioxo-2,5-dihydro-1H-pyrrol-1-yl)ethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-6)

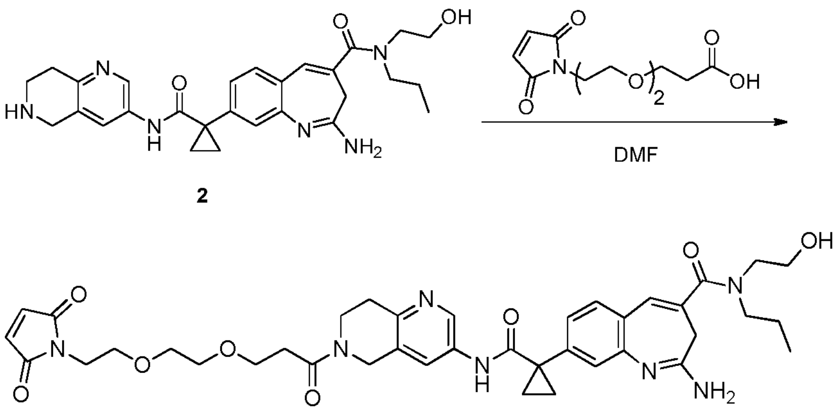


2

II-6

To a solution of 3-(2-(2-(2,5-dioxo-2,5-dihydro-1H-pyrrol-1-yl)ethoxy)ethoxy)propionic acid (36.8 mg, 0.14 mmol) in DMF (3 mL) were added HATU (81 mg, 0.21 mmol), DIPEA (37 mg, 0.28 mmol) and compound 2 (74 mg, 0.14 mmol) sequentially. The reaction solution was stirred at room temperature for 2 hours, and the residue was directly purified by *prep*-HPLC (eluent 5%-45% acetonitrile in a 0.1% aqueous trifluoroacetic acid solution, 20 minutes) to give compound II-6 (20 mg, yield: 19%) as a white solid. m/z: $[M+H]^+$ 742.3.

**Example 55:** Synthesis of 2-amino-8-(1-((6-(3-(2-(2-(3-(2,5-dioxo-2,5-dihydro-1H-pyrrol-1-yl)propionylamino)ethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-7)

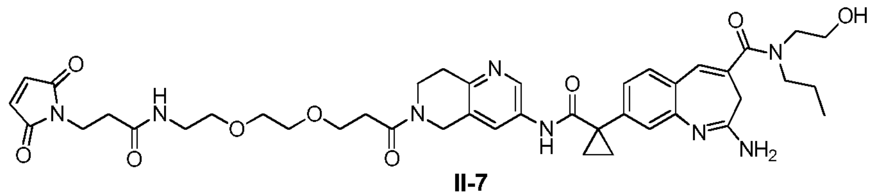

II-7

Using the synthesis method of compound II-5, compound II-7 was obtained as a white solid by reacting 2,5-dioxopyrrolidin-1-yl 3-(2-(2-(3-(2,5-dioxo-2,5-dihydro-1H-pyrrol-1-yl)propionylamino)ethoxy)ethoxy)propionate with compound 2. m/z: $[M+H]^+$ 813.3.

**Example 56:** Synthesis of 2-amino-8-(1-((6-(3-(2-(2-(2-(2,5-dioxo-2,5-dihydro-1H-pyrrol-1-yl)ethoxy)ethoxy)ethoxy) propionyl)-5,6,7,8-tetrahydro-1,6-naphthyridin-3-yl)carbamoyl)cyclopropyl)-N-(2-hydroxyethyl)-N-propyl-3H-benzo[b] azepine-4-formamide (II-8)

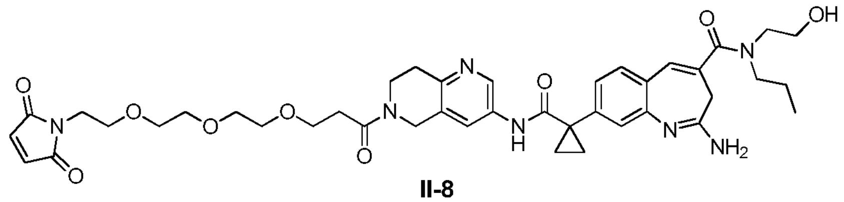

II-8

Using the synthesis method of compound II-6, compound II-8 was obtained as a white solid by reacting 3-(2-(2-(2-(2,5-dioxo-2,5-dihydro-1H-pyrrol-1-yl)ethoxy)ethoxy)ethoxy)propionic acid with compound 2. m/z: $[M+H]^+$ 786.2.

**Example 57:** Synthesis of 2-amino-8-(1-((6-(1-(2,5-dioxo-2,5-dihydro-1H-pyrrol-1-yl)-3,6,9,12,15,18-hexaoxaheneicosan-21-acyl)-5,6,7, 8-tetrahydro-1,6-naphthyridin-3-yl)carbamoyl)cyclopropyl)-N-(2-hydroxyethyl)-N-propyl-3H-benzo [b]azepine-4-formamide (II-9)

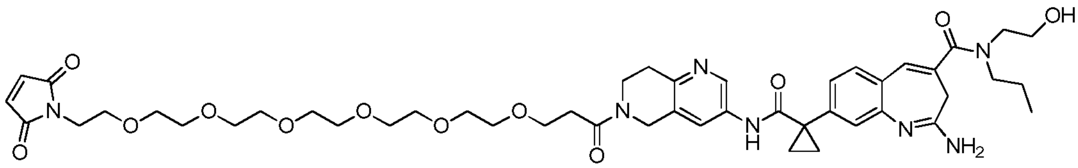

II-9

Using the synthesis method of compound II-6, compound II-9 was obtained as a white solid by reacting 1-(2,5-dioxo-2,5-dihydro-1H-pyrrol-1-yl)-3,6,9,12,15,18-hexaoxaheneicosan-21-oic acid with compound 2. m/z: $[M+H]^+$ 918.1.

**Example 58:** Synthesis of 2-amino-8-(1-((6-(1-(2,5-dioxo-2,5-dihydro-1H-pyrrol-1-yl)-3,6,9,12,15,18,21.,24,27,30-decaoxatritriacontan-33-acyl)-3,6,7,8-tetrahydro-1,6-naphthyridin-3-yl)carbamoyl)cyclopropyl)-N-(2-hydroxyethyl)-N-propyl-3H-benzo[b]azepine-4-formamide (II-10)

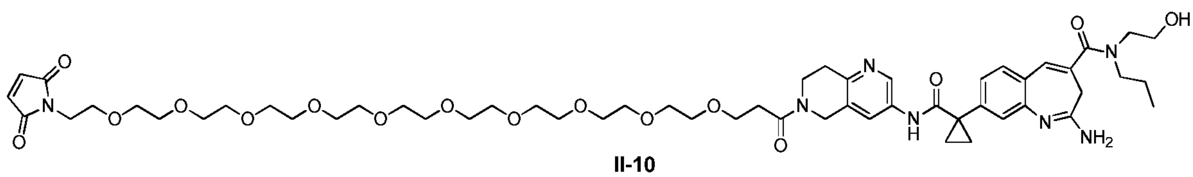

II-10

Using the synthesis method of compound II-6, compound II-10 was obtained as a white solid by reacting L-2 with compound 2. m/z: $[1/2M+H]^+$ 547.9.

**Example 59:** Synthesis of 2-amino-8-(1-((6-(1-(2,5-dioxo-2,5-dihydro-1H-pyrrol-1-yl)-3-oxo-7,10,13,16-tetraoxa-4-azanonadecan-19-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-11)

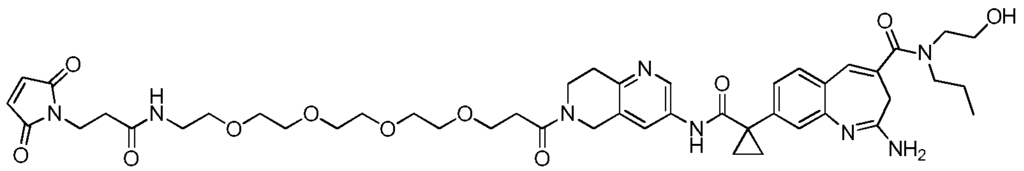

**II-11**

Using the synthesis method of compound II-5, compound II-11 was obtained as a white solid by reacting 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate with compound 2. m/z: $[1/2M+H]^+$451.4.

**Example 60:** Synthesis of 2-amino-8-(1-((6-(1-(2,5-dioxo-2,5-dihydro-1H-pyrrol-1-yl)-3-oxo-7,10,13,16,19,22-hexaoxa-4-azapentacosan-25-acyl)-5,6,7, 8-tetrahydro-1,6-naphthyridin-3-yl)carbamoyl)cyclopropyl)-N-(2-hydroxyethyl)-N-propyl-3H-benzo[b]azepine-4-formamide (II-12)

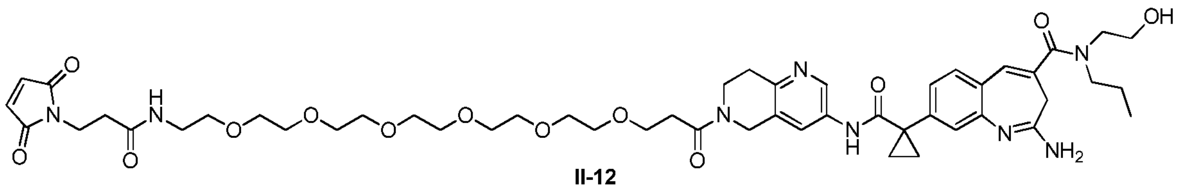

**II-12**

Using the synthesis method of compound II-6, compound II-12 was obtained as a white solid by reacting 1-(2,5-dioxo-2,5-dihydro-1H-pyrrol-1-yl)-3-oxo-7,10,13,16,19,22-hexaoxa-4-azapentacosan-25-acid with compound 2. m/z: $[1/2M+H]^+$ 495.4.

**Example 61:** Synthesis of 2-amino-8-(1-((6-(1-(2,5-dioxo-2,5-dihydro-1H-pyrrol-1-yl)-3-oxo-7,10,13,16,19,22,25,28,31,34-decaoxa-4-azaheptatriacontan-37-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-13)

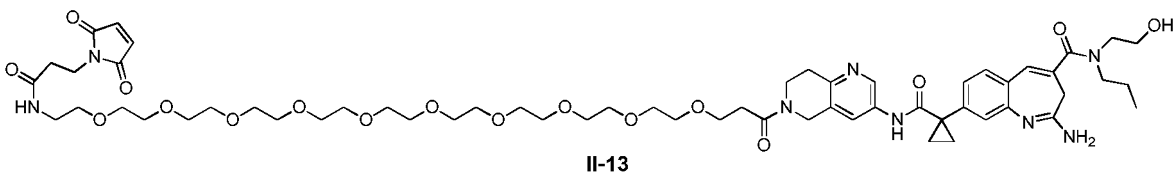

**II-13**

Using the synthesis method of compound II-5, compound II-13 was obtained as a white solid by reacting 2,5-dioxopyrrolidin-1-yl 1-(2,5-dioxo-2,5-dihydro-1H-pyrrol-1-yl)-3-oxo-7,10,13,16,19,22,25,28,31,34-decaoxa-4-azaheptatriacontan-37-oate with compound 2. m/z: $[1/2M+H]^+$ 583.4.

**Example 62:** Synthesis of 2-amino-8-(1-((6-(1-(2,5-dioxo-2,5-dihydro-1H-pyrrol-1-yl)-3-oxo-7,10,13,16,19,22,25,28,31,34,37,40-dodeca-4-azatritetracontan-43-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-14)

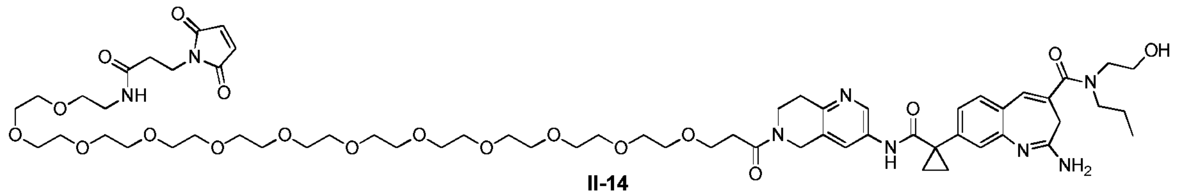

II-14

Using the synthesis method of compound II-5, compound II-14 was obtained as a white solid by reacting 2,5-dioxopyrrolidin-1-yl 1-(2,5-dioxo-2,5-dihydro-1H-pyrrol-1-yl)-3-oxo-7,10,13,16,19,22,25,28,31,34,37,40-dodeca-4-azatritetracontan-43-oate with compound 2. m/z: $[1/2M+H]^+$ 627.4.

**Example 63:** Synthesis of 2-amino-8-(1-((6-(1-(2,5-dioxo-2,5-dihydro-1H-pyrrol-1-yl)-3-oxo-7,10,13,16,19,22,25,28-octaoxa-4-azahentriacontan-31-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-15)

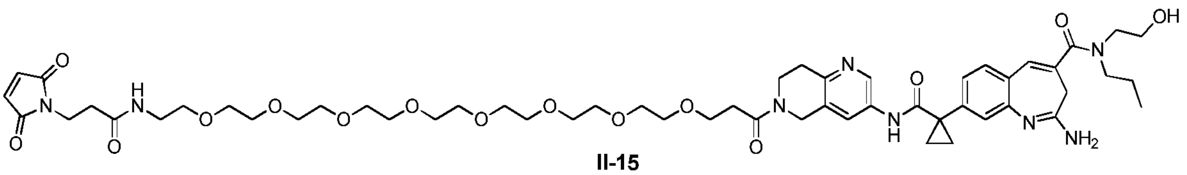

II-15

Using the synthesis method of compound II-6, compound II-15 was obtained as a white solid by reacting 1-(2,5-dioxo-2,5-dihydro-1H-pyrrol-1-yl)-3 -oxo-7,10,13,16,19,22,25,28-octaoxa-4-azahentriacontan-31-acid with compound 2. m/z: $[1/2M+H]^+$ 539.4.

**Example 64:** Synthesis of 2-amino-8-(1-((6-(1-(2,5-dioxo-2,5-dihydro-1H-pyrrol-1-yl)-3-oxo-7,10,13,16,19,22,25,28,31,34,37,40,43,46,49,52,55,58,61,64,67,70,73,76-tetracosaoxa-4-azaheptacontan-79-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-16)

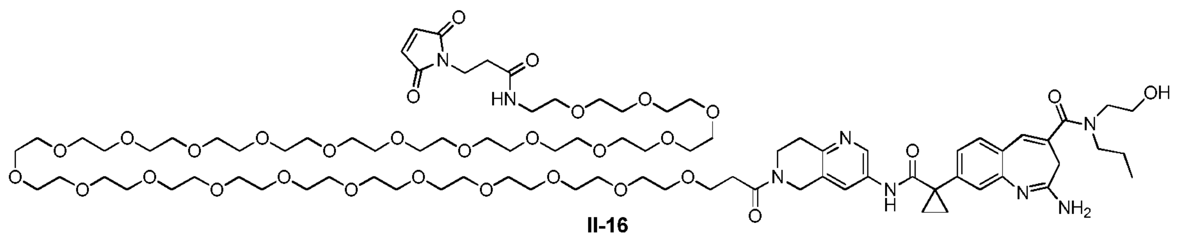

II-16

Using the synthesis method of compound II-5, compound II-16 was obtained as a white solid by reacting 2,5-dioxopyrrolidin-1-yl 1-(2,5-dioxo-2,5-dihydro-1H-pyrrol-1-yl)-3-oxo-7,10,13,16,19,22,25,28,31,34,37,40,43,46,49,52,55,58,61,64,67,70,73,76-tetracosaoxa-4-azaheptacontan-79-oate with compound 2. m/z: $[1/2M+H]^+$ 891.6.

**Example 65:** Synthesis of 2-amino-8-(1-((6-((12S,15S)-22-(2,5-dioxo-2,5-dihydro-1H-pyrrol-1-yl)-15-isopropyl-11,14,17-trioxo-12-(3-ureidopropyl)-4,7-dioxa-10,13,16-triazabehenoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-17)

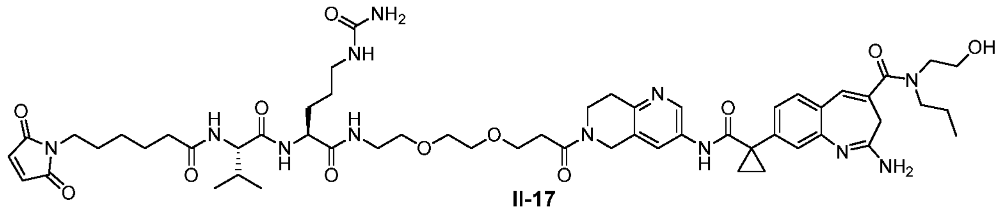

II-17

Using the synthesis method of compound II-6, compound II-17 was obtained as a white solid by reacting L-3 with compound 2. m/z:$[M+H]^+$ 1111.7.

**Example 66:** Synthesis of 2-amino-8-(1-((6-((24S,27S)-34-(2,5-dioxo-2,5-dihydro-1H-pyrrol-1-yl)-27-isopropyl-23,26,29-trioxo-24-(3-ureidopropyl)-4,7,10,13,16,19-hexaoxa-22,25,28-triazatetratriacontanacyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-18)

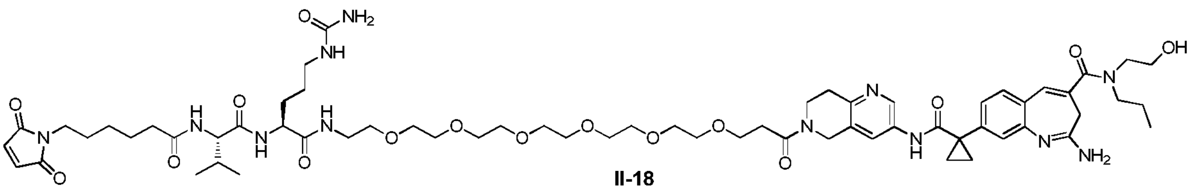

II-18

Using the synthesis method of compound II-6, compound II-18 was obtained as a white solid by reacting L-4 with compound 2. m/z:$[M+H]^+$ 1287.8.

**Example 67:** Synthesis of 2-amino-8-(1-((6-((S)-2-((S)-2-(6-(2,5-dioxo-2,5-dihydro-1H-pyrrol-1-yl)hexanoylamino)-3-methylbutanoylamino)-5-ureidopentanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-19)

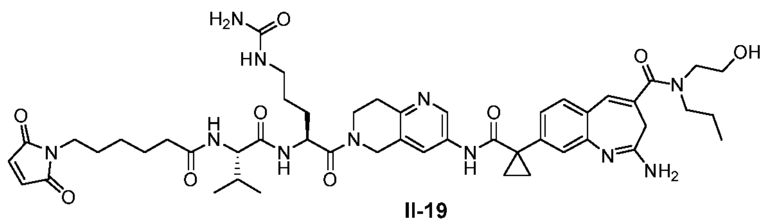

II-19

Using the synthesis method of compound II-5, compound II-19 was obtained as a white solid by reacting EMCS with compound A-1. m/z: $[1/2M+H]^+$476.8.

**Example 68:** Synthesis of 4-((2S, 5S)-31-(2,5-dioxo-2,5-dihydro-1H-pyrrol-1-yl)-5-isopropyl-4,7,29-trioxo-2-(3-ureidopropyl)-10,13,16,19,22,25hexaoxa-3,6,28-triazahentriacontanamido)benzyl 3-(1-(2-amino-4-((2-hydroxyethyl)(propyl)carbamoyl)-3H-benzo[b]azepin-8-yl)cyclopropane-1-formylamino)-7,8-dihydro-1,6-naphthyridine-6(5H)-carboxylate (II-20)

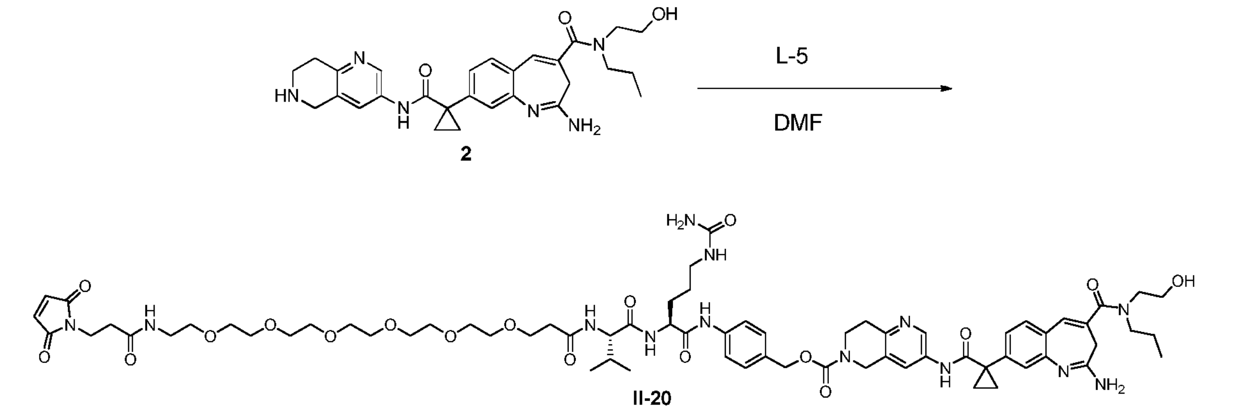

2

II-20

To a solution of compound 2 (20 mg, 0.019 mmol) in DMF were added DIPEA (4.91 mg, 0.038 mmol) and L-5 (20 mg, 0.019 mmol) sequentially under ice bath conditions. The reaction solution was stirred at room temperature for 1 hour and then directly purified by *prep-HPLC* (separation condition 3, eluent 15%-85% acetonitrile in a 0.1% aqueous trifluoroacetic acid solution, elution 20 minutes) to give compound II-20 (4.8 mg) as a white solid. m/z: $[1/2M+H]^+$ 697.9.

**Example 69:** Synthesis of 2-amino-8-(1-((6-(1-(4-((2,5-dioxo-2,5-dihydro-1H-pyrrol-1-yl)methyl)cyclohexyl)-1-oxo-5,8,11,14,17,20-hexaoxa-2-azatricosyl-23-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-21)

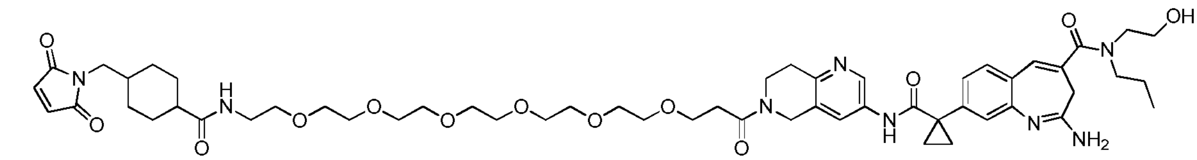

II-21

Using the synthesis method of compound II-6, compound II-21 was obtained as a white solid by reacting L-6 with compound 2. m/z: $[1/2M+H]^+$ 529.4.

**Example 70:** Synthesis of 2-amino-8-(1-((6-(3-(2-(2-(4-((2,5-dioxo-2,5-dihydro-1H-pyrrol-1-yl)methyl)cyclohexane-1-formylamino)ethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-22)

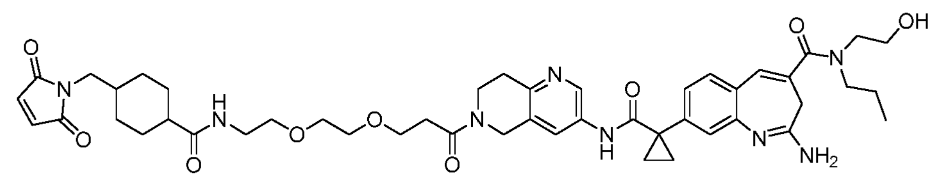

II-22

Using the synthesis method of compound II-6, compound II-22 was obtained as a white solid by reacting L-7 with compound 2. m/z: $[1/2M+H]^+$ 441.3.

**Example 71:** Synthesis of 2-amino-8-(1-((6-(3-(2-(2-(2-(2,5-dioxo-2,5-dihydro-1H-pyrrol-1-yl)acetylamino)ethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-23)

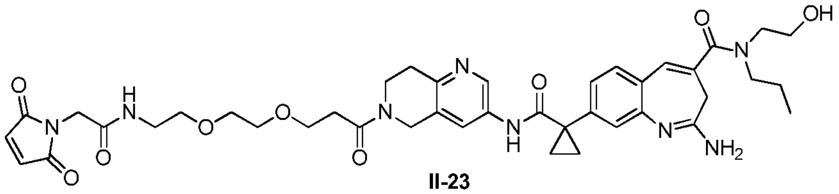

II-23

Using the synthesis method of compound II-6, compound II-23 was obtained as a white solid by reacting L-8 with compound 2. m/z: $[1/2M+H]^+$ 400.2.

**Example 72:** Synthesis of 2-amino-8-(1-((6-(20-(2,5-dioxo-2,5-dihydro-1H-pyrrol-1-yl)-4,14,19-trioxo-7,10-dioxa-3,13,18-triazaeicosanacyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-24)

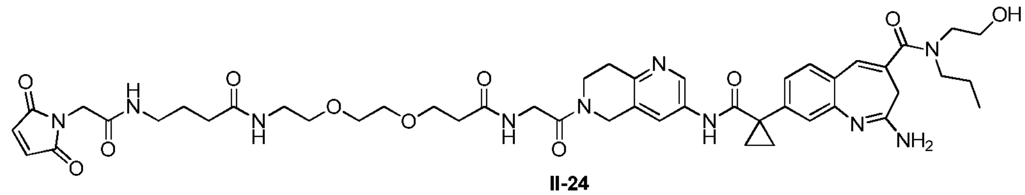

II-24

Using the synthesis method of compound II-6, compound II-24 was obtained as a white solid by reacting L-11 with compound 2. m/z: $[M+Na]^+$963.4.

**Example 73:** Synthesis of 2-amino-8-(1-((6-(3-(2-(2-(3-(2,5-dioxo-2,5-dihydro-1H-pyrrol-1-yl)propionylamino)ethoxy) ethoxy)propionyl)-5,6,7,8-tetrahydro-1,6-naphthyridin-3-yl)carbamoyl)cyclopropyl)-N,N-dipropyl-3H-benzo[b]azepine-4-formamide (II-25)

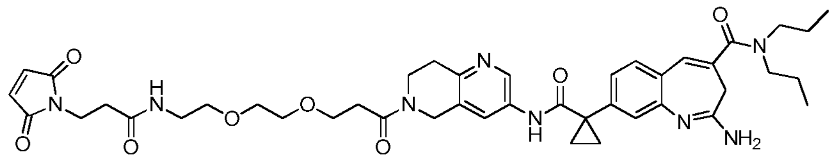

II-25

Using the synthesis method of compound II-5, compound II-25 was obtained as a white solid by reacting 2,5-dioxopyrrolidin-1-yl 3-(2-(2-(3-(2,5-dioxo-2,5-dihydro-1H-pyrrol-1-yl)propionylamino)ethoxy)ethoxy)propionate with compound 1. m/z: $[M+H]^+$ 811.4.

**Example 74:** Synthesis of 2-amino-8-(1-((6-(3-(2-(2-(4-((2,5-dioxo-2,5-dihydro-1H-pyrrol-1-yl)methyl)benzoylamino) ethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-26)

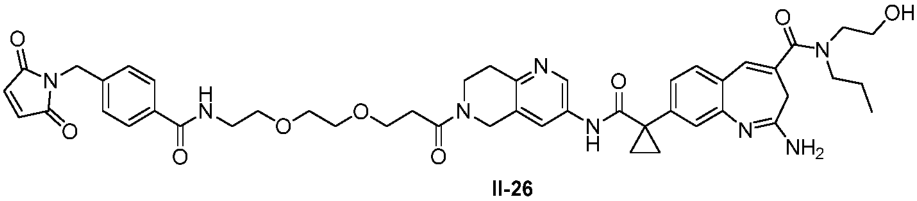

II-26

Using the synthesis method of compound II-6, compound II-26 was obtained as a white solid by reacting L-12 with

compound 2. m/z: $[M+H]^+$ 875.3.

**Example 75:** Synthesis of 2-amino-8-(1-((6-(1-(4-((2,5-dioxo-2,5-dihydro-1H-pyrrol-1-yl)methyl)phenyl)-1-oxo-5,8,11,14,17,20-hexaoxa-2-azatricosan-23-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-27)

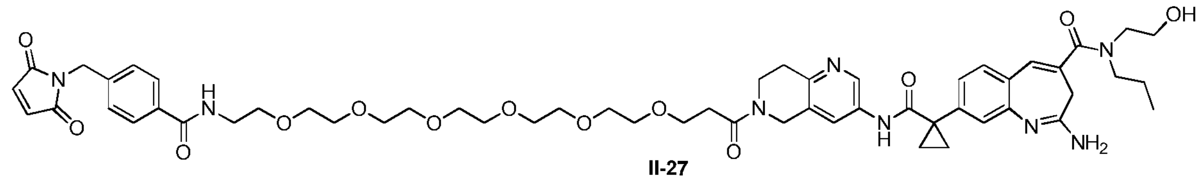

II-27

Using the synthesis method of compound II-6, compound II-27 was obtained as a white solid by reacting L-13 with compound 2. m/z: $[1/2M+H]^+$ 527.1.

**Example 76:** Synthesis of 4-(3-(2-(2-(3-(2,5-dioxo-2,5-dihydro-1H-pyrrol-1-yl)propionylamino)ethoxy)ethoxy)propionylamino)benzyl 3-(1-(2-amino-4-((2-hydroxyethyl)(propyl)carbamoyl)-3H-benzo[b]azepin-8-yl)cyclopropane-1-formylamino)-7,8-dihydro-1,6-naphthyridine-6(5H)-carboxylate (II-28)

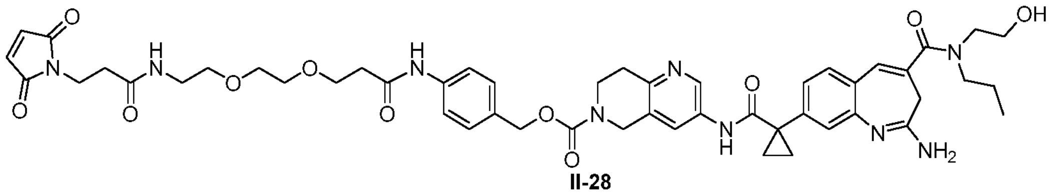

II-28

Using the synthesis method of compound II-20, compound II-28 was obtained as a white solid by reacting L-14 with compound 2. m/z: $[M+H]^+$962.4.

**Example 77:** Synthesis of 2-amino-8-(1-((6-((6-(2,5-dioxo-2,5-dihydro-1H-pyrrol-1-yl)hexanoyl)-L-prolyl-L-valyl)-5,6,7,8-tetrahydro-1,6-naphthyridin-3-yl)carbamoyl)cyclopropyl)-N-(2-hydroxyethyl)-N-propyl-3H-benzo[b]azepine-4-carboxylate (II-29)

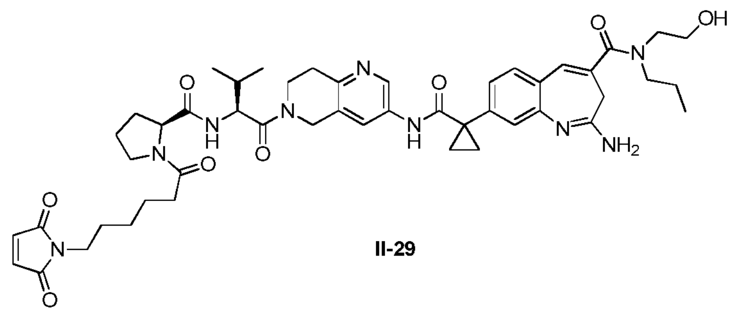

II-29

Using the synthesis method of compound II-6, compound II-29 was obtained as a white solid by reacting 6-(2,5-dioxo-2,5-dihydro-1H pyrrol-1-yl)hexanoic acid with compound A-2. m/z: $[M+H]^+$ 892.5.

**Example 78:** Synthesis of 2-amino-8-(1-((6-(32-(3-(2,5-dioxo-2,5-dihydro-1H-pyrrol-1-yl)propionylamino)-26,33-dioxo-2,5,8,11,14,17,20,23-octaoxa-27,34-diazeptadecan-37-acyl)-5,6,7,8-tetrahydro-1,6-naphthyridin-3-yl)carbamoyl)cyclopropyl)-N-(2-hydroxyethyl)-N-propyl-3H-benzo[b]azepine-4-carboxylate (II-30)

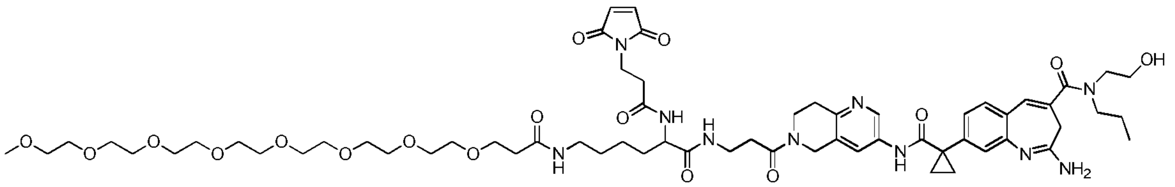

II-30

Using the synthesis method of compound II-6, compound II-30 was obtained as a white solid by reacting L-15 with compound 2. m/z: $[M+H]^+$ 1248.0.

**Example 79:** Synthesis of 2-amino-8-(1-((6-(32-(3-(2,5-dioxo-2,5-dihydro-1H-pyrrol-1-yl)propionylamino)-26,33-dioxo-2,5,8,11,14,17,20,23,37,40-decaoxa-27,34-diazatritetracontan-43-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-31)

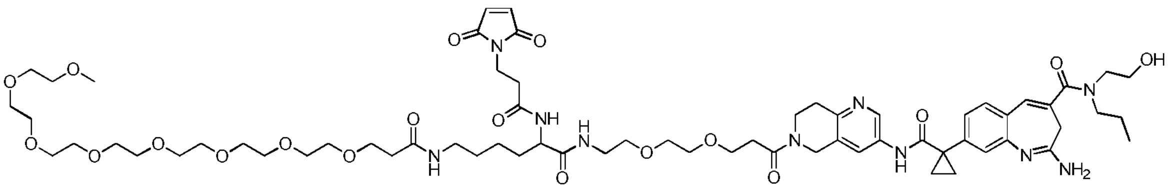

II-31

Using the synthesis method of compound II-6, compound II-31 was obtained as a white solid by reacting L-16 with compound 2. m/z: $[1/2M+H]^+$ 668.4.

**Example 80:** Synthesis of 2-amino-8-(1-((6-(1-(2,5-dioxo-3,4-bis(pyridin-2-ylthio)-2,5-dihydro-1H-pyrrol-1-yl)-3-oxo-7,10,13,16,19,22-hexaoxa-4-azapentacosan-25-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-32)

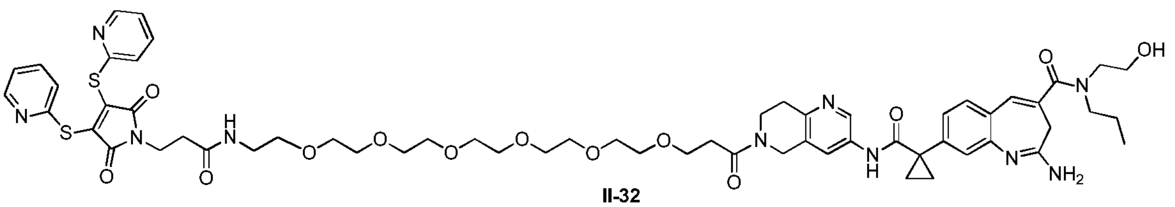

II-32

Using the synthesis method of compound II-6, compound II-32 was obtained as a white solid by reacting L-17 with compound 2. m/z: $[1/2M+H]^+$ 604.4.

**Example 81:** Synthesis of 2-amino-8-(1-((6-(3-(2-(2-(3-(2,5-dioxo-3,4-bis(pyridin-2-ylthio)-2,5-dihydro-1H-pyrrol-1-yl)propionylamino)ethoxy)ethoxy)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-33)

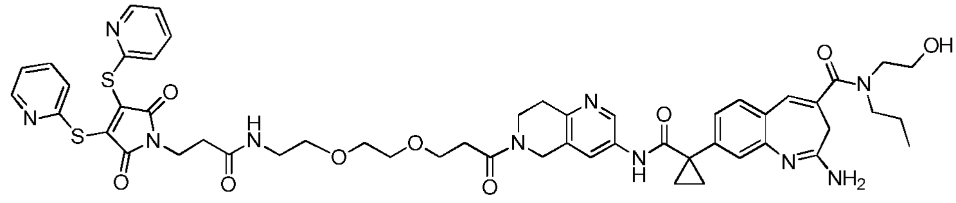

II-33

Using the synthesis method of compound II-6, compound II-33 was obtained as a white solid by reacting L-18 with compound 2. m/z: $[1/2M+H]^+$ 516.2.

**Example 82:** Synthesis of 2-amino-8-(1-((6-((24S,27S)-34-(2,5-dioxo-3,4-bis(pyridin-2-ylthio)-2,5-dihydro-1H-pyrrol-1-yl)-27-isopropyl-23,26,29-trioxo-24-(3-ureidopropyl)-4,7,10,13,16,19-hexaoxa-22,25,28-triazatetratriacontanacyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-34)

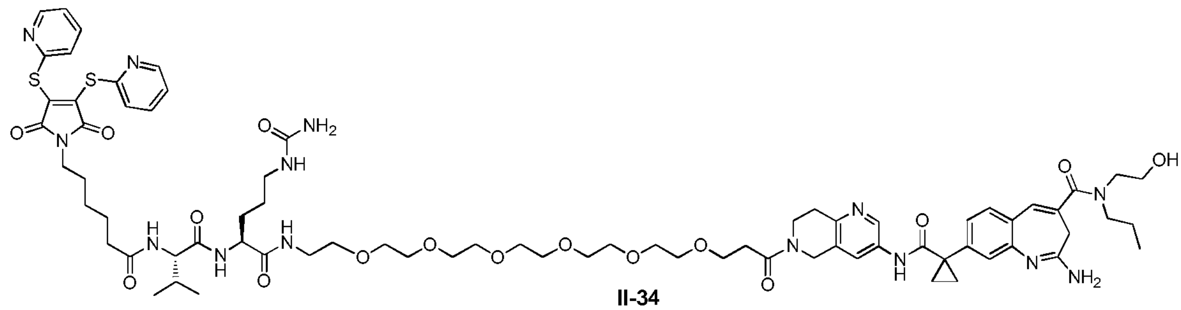

II-34

Using the synthesis method of compound II-6, compound II-34 was obtained as a white solid by reacting L-19 with compound 2. m/z: $[1/2M+H]^+$ 753.4.

**Example 83:** Synthesis of 3-(2-(6-(2,5-dioxo-2,5-dihydro-1H-pyrrol-1-yl)hexanoylamino)acetylamino)-4-(((2S,3R,4S,5R,6R)-3,4,5-trihydroxy-6-(hydroxymethyl) tetrahydro-2H-pyran-2-yl)oxy)benzyl 3-(1-(2-amino-4-((2-hydroxyethyl)(propyl)carbamoyl)-3H-benzo[b]azepin-8-yl)cyclopropane-1-formylamino)-7,8-dihydro-1,6-naphthyridine-6(5H)-carboxylate (II-35)

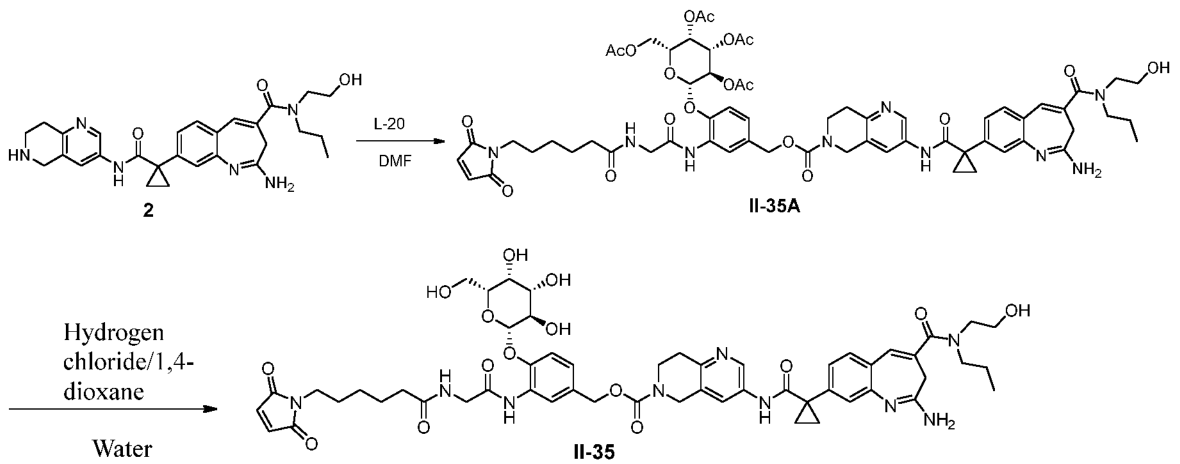


2

II-35A

II-35

Step 1: Using the synthesis method of compound II-20, compound II-35A was obtained as a white solid by reacting L-20 with compound 2. m/z: $[1/2M+H]^+$ 624.8.

Step 2: To a solution of compound II-35A (25 mg, 0.02 mmol) in water (1 mL) was added a solution of hydrogen

chloride in 1,4-dioxane (4 M, 1 mL). The reaction solution was stirred at 30°C for 6 hours. The reaction solution was then directly purified by *prep-HPLC* (eluent 5%-45% acetonitrile in a 0.1% aqueous trifluoroacetic acid solution, 20 minutes) to give compound II-35 (1.71 mg, yield: 6%) as a white solid. m/z: $[1/2M+H]^+$ 540.9.

**Example 84:** Synthesis of (2S,3S,4S,5R,6S)-6-(4-(((3-(1-(2-amino-4-((2-hydroxyethyl)(propyl)carbamoyl)-3H-benzo[b]azepin-8-yl)cyclopropyl-1-formylamino)-5,6,7,8-tetrahydro-1,6-naphthyridine-6-carbonyl)oxy)methyl)-2-(2-(6-(2,5-dioxo-2,5-dihydro-1H-pyrrol-1-yl)hexanoylamino)acetylamino)phenoxy)-3,4,5-trihydroxytetrahydro-2H-pyran-2-carboxylic acid (II-36)

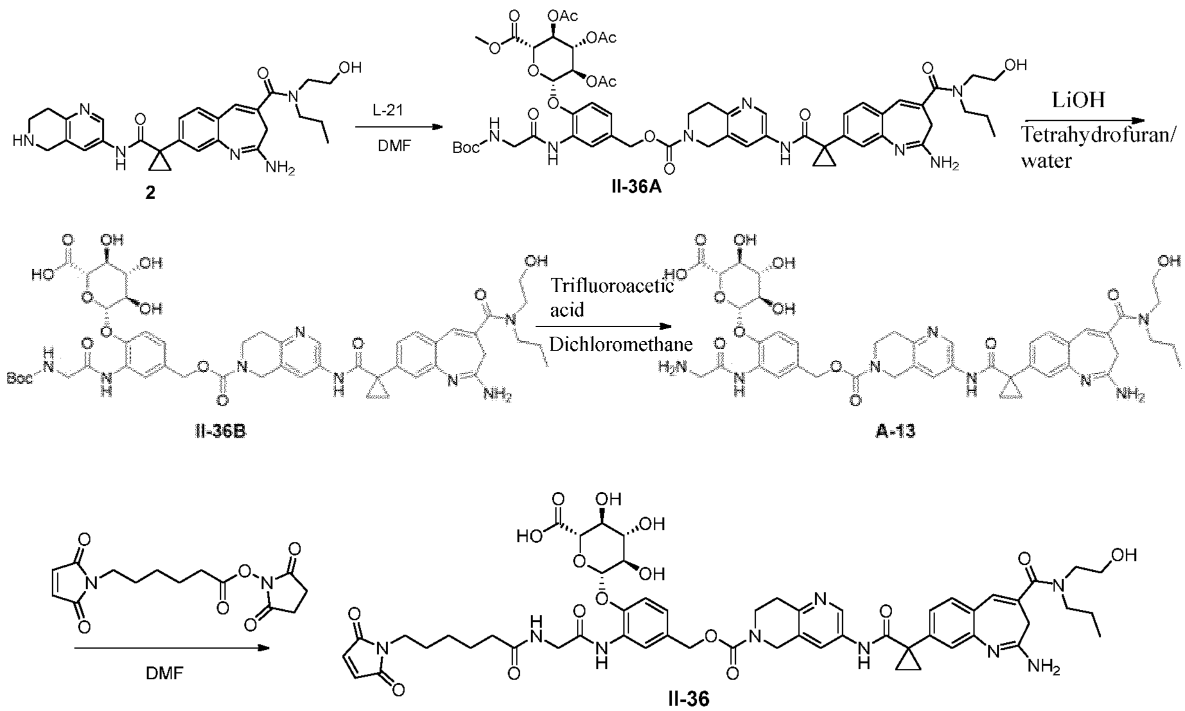


2

II-36A

II-36B

A-13

II-36

Step 1: Using the synthesis method of compound II-20, compound II-36A was obtained as a white solid by reacting L-21 with compound 2. m/z: $[M+H]^+$ 1141.2.

Step 2: To a mixed solution of compound II-36A (120 mg, 0.11 mmol) in tetrahydrofuran (2 mL) and water (1 mL) was added lithium hydroxide monohydrate (14 mg, 0.33 mmol). The reaction solution was stirred at room temperature for 1.5 hours and directly purified by Flash column chromatography (C18, eluent 0-85% acetonitrile in a 10 mM aqueous ammonium bicarbonate solution) to give compound II-36B (105 mg, yield: 100%) as a white solid. m/z: $[1/2M+H]^+$ 501.2.

Step 3: To a solution of compound II-36B (95 mg, 0.1 mmol) in dichloromethane (2 mL) was added trifluoroacetic acid (0.5 mL). The reaction solution was stirred at room temperature for 1 hour and directly purified by Flash column chromatography (C18, eluent 5-85% acetonitrile in a 0.1% aqueous ammonium bicarbonate solution) to give compound A-13 (75 mg, yield: 88%) as a white solid. m/z: $[1/2M+H]^+$ 451.2.

Step 4: Using the synthesis method of compound II-5, compound II-36 was obtained as a white solid by reaction with A-13. m/z: $[1/2M+H]^+$ 547.8.

**Example 85:** Synthesis of 2-amino-8-(1-((6-(1-(2,5-dioxo-2,5-dihydro-1H-pyrrol-1-yl)-2-oxo-6,9,12,15,18,21-hexaoxa-3-azatetracosan-24-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-37)

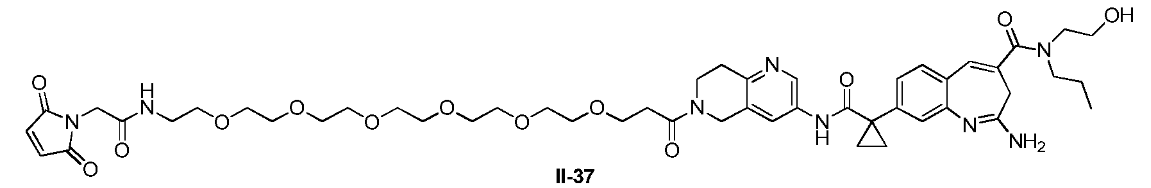

II-37

Using the synthesis method of compound II-6, compound II-37 was obtained as a white solid by reacting L-22 with compound 2. m/z: [1/2M+H]$^+$ 488.4.

**Example 86:** Synthesis of (E)-1-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-1,23,27-trioxo-4,7,10,13,16,19-hexaoxa-22,26-diazahentriacont-28-en-30-oic acid (II-38)

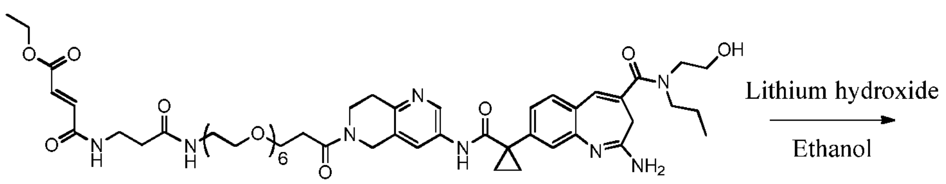


II-38A

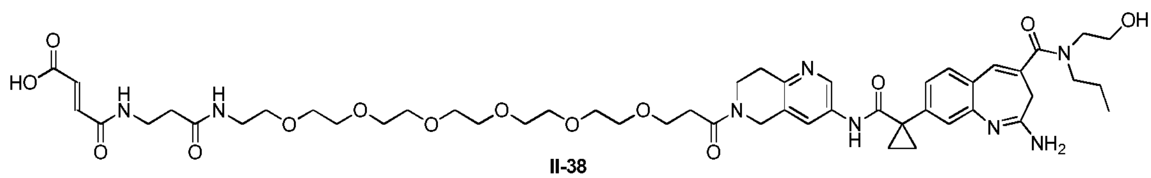

II-38

To a solution of II-38A (obtained by reacting L-24 with compound 2 using the synthesis method of compound II-6) (80 mg, 0.08 mmol) in ethanol (1 mL) was added an aqueous lithium hydroxide solution (0.23 mL, 1 M) under ice bath conditions. The reaction solution was stirred at 0°C for 2 hours and then directly purified by *prep-HPLC* (separation condition 3, eluent 5%-45% acetonitrile in a 0.1% aqueous trifluoroacetic acid solution, 20 minutes) to give compound II-38 (11.2 mg, yield: 12%) as a yellow solid. m/z: [1/2M+H]$^+$504.4.

**Example 87:** Synthesis of 2-amino-8-(1-((6-(2-(4,6-divinylpyrimidin-2-yl)-4-oxo-8,11,14,17,20,23-hexaoxa-2,5-diazahexacosan-26-acyl)-5,6,7, 8-tetrahydro-1,6-naphthyridin-3-yl)carbamoyl)cyclopropyl)-N-(2-hydroxyethyl)-N-propyl-3H-benzo[b]azepine-4-formamide (II-39)

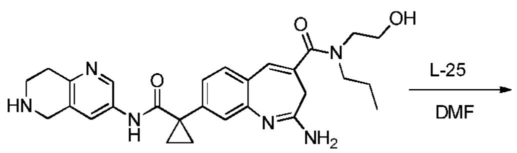


2

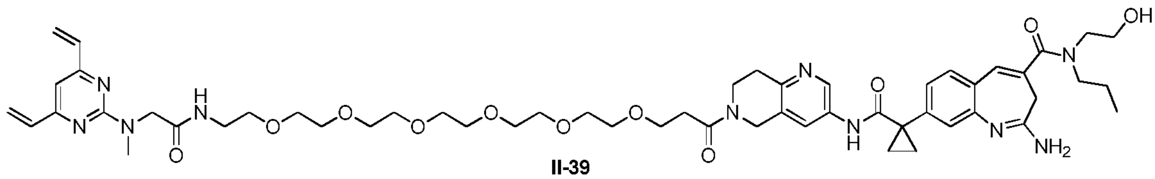

II-39

To a solution of compound 2 (32 mg, 0.06 mmol) and L-25 (35 mg, 0.06 mmol) in DMF (2 mL) were added HOBT (10.2 mg, 0.08 mmol), EDCI (14.5 mg, 0.08 mmol) and DIPEA (16.3 mg, 0.13 mmol) under ice bath conditions. The

reaction solution was stirred at room temperature overnight and directly purified by *prep-HPLC* (eluent 5%-45% acetonitrile in a 0.1% aqueous trifluoroacetic acid solution, 20 minutes) to give compound II-39 (22.4 mg, yield: 29%) as a white solid. m/z: $[1/2M+H]^+$ 520.4.

**Example 88:** Synthesis of Benzyl((2-(2-amino-8-(1-((6-(3-(2-(2-(3-(2,5-dioxo-2,5-dihydro-1H-pyrrol-1-yl)propionylamino)ethoxy)ethoxy)propionyl)-5,6,7,8-tetrahydro-1,6-naphthyridin-3-yl)carbamoyl)cyclopropyl)-N-propyl-3H-benzo[b]azepine-4-formylamino)ethoxy)(phenoxy)phosphoryl)-L-alanine, isomer 1 (II-40-1) and benzyl((2-(2-amino-8-(1-((6-(3-(2-(2-(3-(2,5-dioxo-2,5-dihydro-1H-pyrrol-1-yl)propionylamino)ethoxy)ethoxy)propionyl)-5,6,7,8-tetrahydro-1,6-naphthyridin-3-yl)carbamoyl)cyclopropyl)-N-propyl-3H-benzo[b]azepine-4-formylamino)ethoxy)(phenoxy)phosphoryl)-L-alanine, isomer 2 (II-40-2)

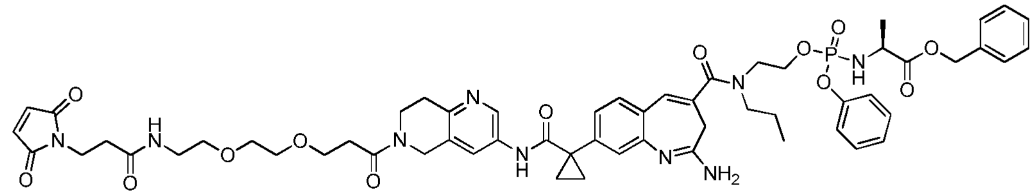

**II-40**

Using the synthesis method of compound II-5, compound II-40 was obtained as a white solid by reacting 2,5-dioxopyrrolidin-1-yl 3-(2-(2-(3-(2,5-dioxo-2,5-dihydro-1H-pyrrol-1-yl)propylamino)ethoxy)ethoxy)propionate with compound 25. m/z: $[M+H]^+$ 1130.3. II-40 was purified by *prep-HPLC* (separation condition 3, eluent 15%-70% acetonitrile in a 0.1% aqueous trifluoroacetic acid solution, elution 20 minutes) to give compound II-40-1 (3.3 mg) and compound II-40-2 (5.2 mg). II-40-1: m/z: $[M+H]^+$ 1130.3, UPLC retention time: 6.218 minutes; and II-40-2: m/z: $[M+H]^+$1130.3, UPLC retention time: 6.313 minutes.

**Example 89:** Synthesis of 4-((S)-2-((S)-2-(6-(2,5-dioxo-2,5-dihydro-1H-pyrrol-1-yl)hexanoylamino)-3-methylbutanoylamino)-5-ureidopentanoylamino)benzyl 3-(1-(2-amino-4-((2-(((((S)-1-(benzyloxy)-1-oxopropyl-2-yl)amino)(phenoxy)phosphoryl)oxy)ethyl)(propyl)carbamoyl)-3H-benzo[b]azepin-8-yl)cyclopropane-1-formylamino)-7,8-dihydro-1,6-naphthyridine-6(5H)-carboxylate, isomers 1 (II-41-1) and 4-((S)-2-((S)-2-(6-(2,5-dioxo-2,5-dihydro-1H-pyrrol-1-yl)hexanoylamino)-3-methylbutanoylamino)-5-ureidopentanoylamino)benzyl 3-(1-(2-amino-4-((2-(((((S)-1-(benzyloxy)-1-oxopropyl-2-yl)amino)(phenoxy)phosphoryl)oxy)ethyl)(propyl)carbamoyl)-3H-benzo[b]azepin-8-yl)cyclopropane-1-formylamino)-7,8-dihydro-1,6-naphthyridine-6(5H)-carboxylate, isomer 2 (II-41-2)

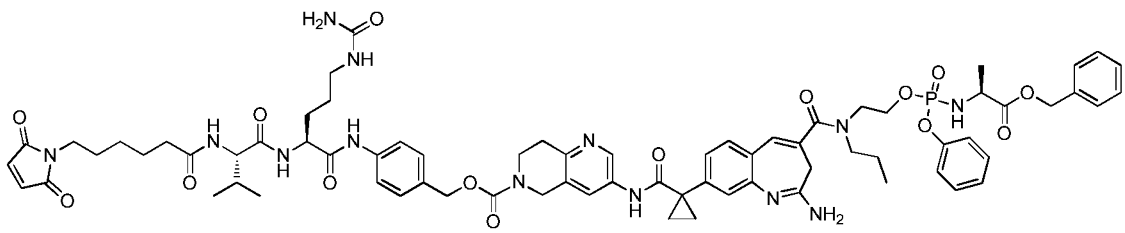

**II-41**

Using the synthesis method of II-20, compound II-41 was obtained as a white solid by reacting L-23 with compound 25. II-41 was purified by *prep-HPLC* (eluent 15-70% acetonitrile in a 0.1% aqueous trifluoroacetic acid solution, 20 minutes) to give compounds II-41-1 (4.51 mg) and II-41-2 (4.05 mg). II-41-1: m/z: $[M+H]^+$1418.5, UPLC retention time: 6.816 minutes; II-41-2: m/z: $[M+H]^+$1418.5, UPLC retention time: 6.845 minutes.

**Example 90:** Synthesis of 4-((S)-2-((S)-2-(6-(2,5-dioxo-2,5-dihydro-1H-pyrrol-1-yl)hexanoylamino)-3-methylbutanoylamino)-5-ureidopentanoylamino)benzyl 3-(1-(4-((2-hydroxyethyl)(propyl)carbamoyl)-2-((((1-methyl-2-nitro-1H-imidazol-5-yl)methoxy)carbonyl)amino)-3H-benzo[b]azepin-8-yl)cyclopropyl-1-formylamino)-7,8-dihydro-1,6-naphthyridine-6(5H)-carboxylate (II-42)

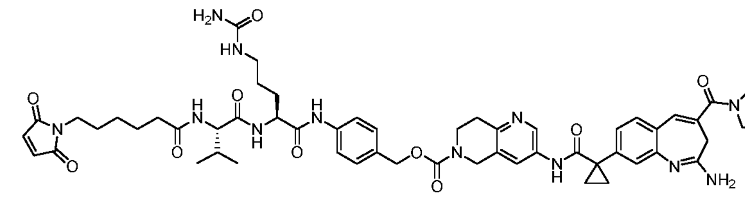

II-106

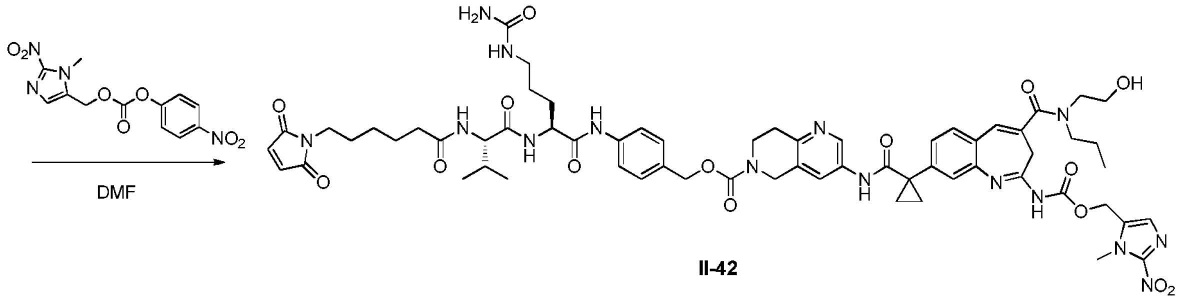


II-42

Compound II-106 (20.1 mg, 0.02 mmol) and (1-methyl-2-nitro-1H-imidazol-5-yl)methyl(4-nitrophenyl)carbonate (6.4 mg, 0.020 mmol) were dissolved in anhydrous DMF (1.5 mL), and HOBT (3.2 mg, 0.023 mmol), pyridine (2.4 mg, 0.03 mmol) and DIPEA (9.3 mg, 0.07 mmol) were added sequentially. The reaction solution was stirred at room temperature for 1 hour and directly purified by *prep-HPLC* (eluent 15%-65% acetonitrile in a 10 mM aqueous ammonium bicarbonate solution, 20 minutes) to give compound II-42 (5.1 mg, yield: 16%) as a white solid. m/z: [1/2M+H] $^+$ 642.8; $^1$H NMR (400 MHz, $CD_3OD$): δ 8.41(s, 1H), 7.74 (s, 1H), 7.58 (d, *J* = 8.4Hz, 2H), 7.47 (d, *J* = 8.2Hz, 1H), 7.41 (s, 1H), 7.33 (d, *J* = 8.4Hz, 3H), 7.25 (s, 1H), 6.99 (s, 1H), 6.77 (s, 2H), 5.49 (s, 2H), 5.31 (s, 2H), 5.11 (s, 2H), 4.68-4.57 (m, 4H), 4.54-4.46 (m, 2H), 4.15 (d, *J* = 7.4Hz, 2H), 4.06 (s, 3H), 3.79 (s, 3H), 3.55 (s, 2H), 3.49-3.43 (m, 3H), 3.19-3.10 (m, 4H), 2.89 (s, 2H), 2.27 (t, *J* = 7.4Hz, 2H), 2.09-2.04 (m, 1H), 1.91-1.86 (m, 1H), 1.78-1.73 (m, 1H), 1.66-1.52 (m, 8H), 1.36-1.22 (m, 6H), 1.01-0.93 (m, 6H).

**Example 91:** Synthesis of 2-amino-8-(1-((6-(1-(4-(2,5-dioxo-2,5-dihydro-1H-pyrrol-1-yl)phenyl)-1-oxo-5,8,11,14,17,20-hexaoxa-2-azatricosan-23-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-43)

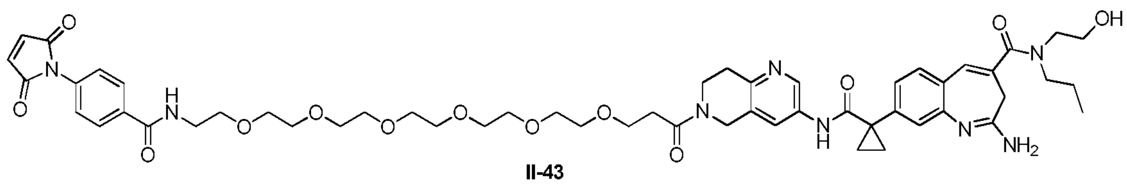

II-43

Using the synthesis method of compound II-6, compound II-43 was obtained as a white solid by reacting L-26 with compound 2. m/z: [1/2M+H]$^+$ 519.3.

**Example 92:** Synthesis of 2-amino-8-(1-((6-(2-(4,6-divinylpyrimidin-2-yl)-4-oxo-8,11-dioxa-2,5-diazatetradecan-14-acyl)-5,6,7,8-tetrahydro-1,6-naphthyridin-3-yl)carbatnoyl)cyclopropyl)-N-(2-hydroxyethyl)-N-propyl-3H-benzo[b]azepine-4-formamide (II-44)

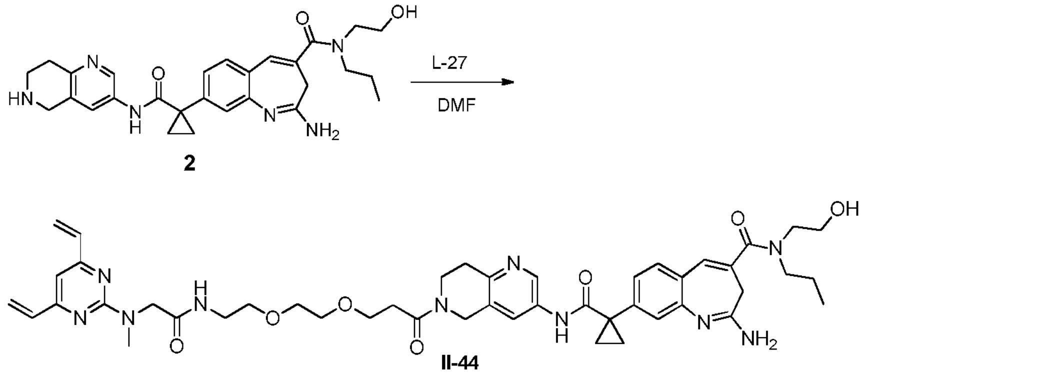


2

II-44

To a solution of L-27 (25 mg, 0.06 mmol), HOBT (13 mg, 0.1 mmol), HATU (19 mg, 0.1 mmol) and DIPEA (17 mg, 0.13 mmol) in DMF (2 mL) was added compound 2 (33 mg, 0.07 mmol) under ice bath conditions. The reaction solution was stirred at room temperature overnight and directly purified by *prep*-HPLC (eluent 10%-50% acetonitrile in a 0.1% aqueous trifluoroacetic acid solution, 20 minutes) to give compound II-44 (4.3 mg, yield: 6%) as a white solid. m/z: $[M+H]^+$ 863.5.

**Example 93:** Synthesis of 2-(2-amino-8-(1-((6-(1-(2,5-dioxo-2,5-dihydro-1H-pyrrol-1-yl)-3-oxo-7,10,13,16,19,22-hexaoxa-4-azapentacosan-25-acyl)-5,6,7,8-tetrahydro-1,6-naphthyridin-3-yl)carbamoyl)cyclopropyl)-N-propyl-3H-benzo [b]azepine-4-formylamino)ethyl cyclobutylcarbamate (II-45)

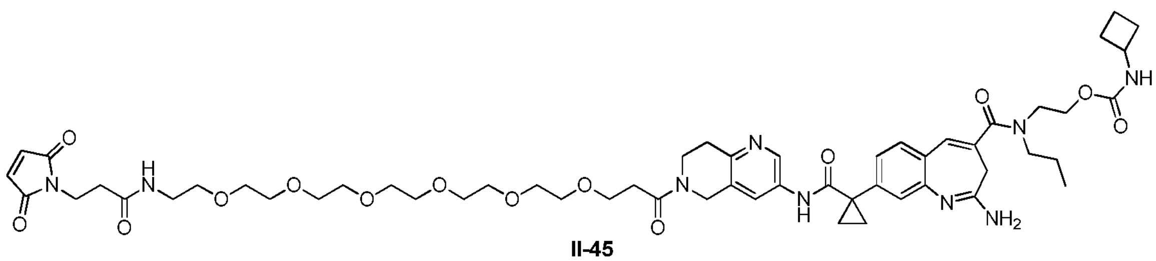

II-45

Using the synthesis method of compound II-6, compound II-45 was obtained as a white solid by reacting 1-(2,5-dioxo-2,5-dihydro-1H-pyrrol-1-yl)-3-oxo-7,10,13,16,19,22-hexaoxa-4-azapentacosan-25-acid with compound 24. m/z: $[1/2M+H]^+$ 543.8.

**Example 94:** Synthesis of 2-amino-8-(1-((6-(1-(2,5-dioxo-2,5-dihydro-1H-pyrrol-1-yl)-2,12-dioxo-6,9-dioxa-3,13-diazapentadecan-15-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-46)

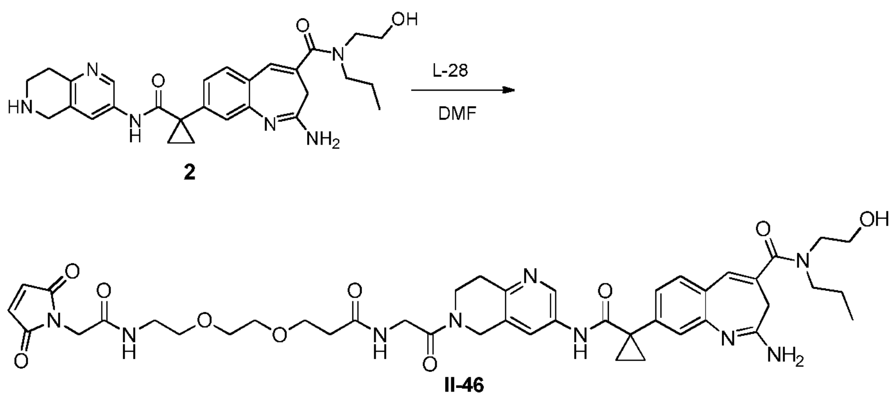


2

II-46

To a solution of L-28 (25 mg, 0.07 mmol) in DMF (3 mL) were added compound 2 (35.4 mg, 0.07 mmol), PyBOP

(41.8 mg, 0.08 mmol) and DIPEA (21.7 mg, 0.17 mmol) sequentially under ice bath conditions. The reaction solution was stirred at room temperature for 1 hour and directly purified by *prep*-HPLC (eluent 5%-45% acetonitrile in a 0.1% aqueous trifluoroacetic acid solution, 20 minutes) to give compound II-46 (9.5 mg, yield: 14%) as a white solid. m/z: $[1/2M+H]^+$ 428.8.

**Example 95:** Synthesis of 2-amino-8-(1-((5-(27-(2,5-dioxo-2,5-dihydro-1H-pyrrol-1-yl)-3,25-dioxo-6,9,12,15,18,21-hexaoxa-2,24-diazaheptacosyl)pyridin-3-yl)carbamoyl)cyclopropyl)-N-(2-hydroxyethyl)-N-propyl-3H-benzo[b]azepine-4-formamide (II-47)

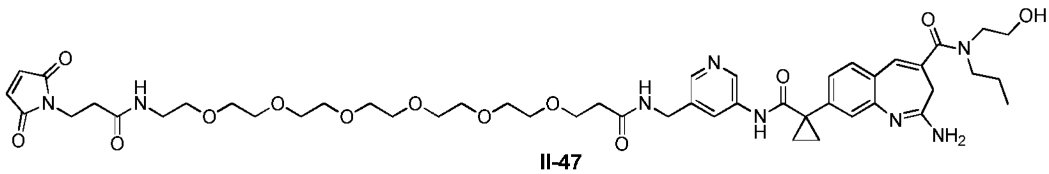

II-47

Using the synthesis method of compound II-6, compound II-47 was obtained as a white solid by reacting 1-(2,5-dioxo-2,5-dihydro-1H-pyrrol-1-yl)-3-oxo-7,10,13,16,19,22-hexaoxa-4-azapentacosan-25-acid with compound 7. m/z: $[M+H]^+$ 963.5.

**Example 96:** Synthesis of 3-(2-(6-(2,5-dioxo-2,5-dihydro-1H-pyrrol-1-yl)hexanoylamino)acetylamino)-4-((1-methyl-2-nitro-1H-imidazol-5-yl)methoxy)benzyl 3-(1-(2-amino-4-((2-hydroxyethyl)(propyl)carbamoyl)-3H-benzo[b]azepin-8-yl) cyclopropane-1-formylamino)-7,8-dihydro-1,6-naphthyridine-6(5H)-carboxylate (II-48)

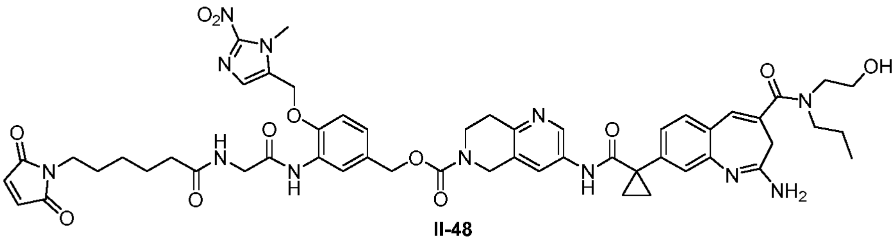

II-48

Using the synthesis method of compound II-20, compound II-48 was obtained as a white solid by reacting L-29 with compound 2. m/z: $[M+H]^+$ 1057.4.

**Example 97:** Synthesis of 2-amino-8-(1-((6-((S)-2-((S)-2-(6-(2,5-dioxo-2,5-dihydro-1H-pyrrol-1-yl)hexanoylamino)-3-methylbutanoylamino)-5-ureidopentanoyl)-5,6,7,8-tetrahydro-1,6-naphthyridin-3-yl)carbamoyl)cyclopropyl)-6-fluoro-N-(2-hydroxyethyl)-N-propyl-3H-benzo[b]azepine-4-formamide (II-49)

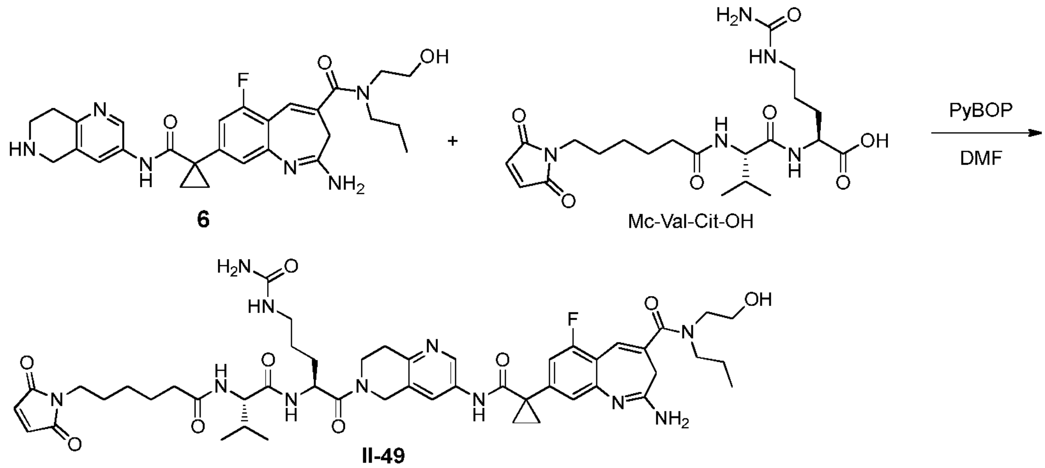


6 + Mc-Val-Cit-OH

II-49

To a solution of Mc-Val-Cit-OH (8 mg, 0.02 mmol) in DMF (2 mL) were added compound 6 (8.9 mg, 0.02 mmol) and PyBOP (11 mg, 0.02 mmol) sequentially under ice bath conditions. The reaction solution was stirred at room temperature for 1 hour and directly purified by *prep-HPLC* (eluent 10%-45% acetonitrile in a 0.1% aqueous trifluoroacetic acid solution, 20 minutes) to give compound II-49 (3.9 mg, yield: 22%) as a white solid. m/z: $[1/2M+H]^+$ 485.8.

**Example 98:** Synthesis of 2-amino-8-(1-((6-(1-(2,5-dioxo-2,5-dihydro-1H-pyrrol-1-yl)-3-oxo-7,10,13,16,19,22-hexaoxa-4-azapentacosan-25-acyl)-5,6,7,8-tetrahydro-1,6-naphthyridin-3-yl)carbamoyl)cyclopropyl)-6-fluoro-N-(2-hydroxyethyl)-N-propyl-3H-benzo[b]azepine-4-formamide (II-50)

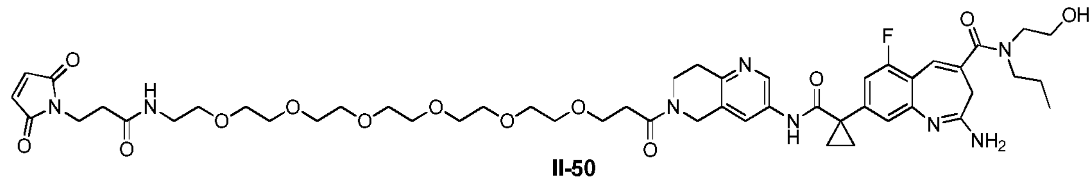

II-50

Using the synthesis method of compound II-6, compound II-50 was obtained as a white solid by reacting 1-(2,5-dioxo-2,5-dihydro-1H-pyrrol-1-yl)-3-oxo-7,10,13,16,19,22-hexaoxa-4-azapentacosan-25-acid with compound 6. m/z: $[1/2M+H]^+$ 504.2.

**Example 99:** Synthesis of 2-amino-8-(1-((6-(1-(4,5-dibromo-2-ethyl-3,6-dioxo-3,6-dihydropyridazin-1(2H)-yl)-3-oxo-7,10,13,16,19,22-hexaoxa-4-azapentacosan-25-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-51)

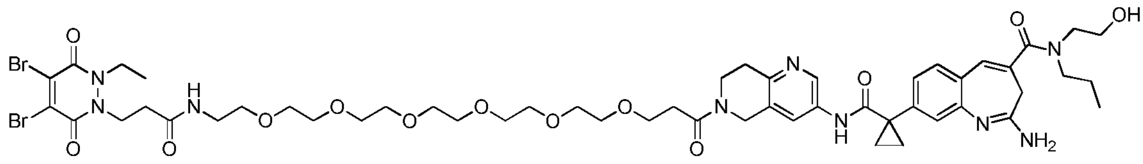

II-51

Using the synthesis method of compound II-49, compound II-51 was obtained as a light yellow solid by reacting L-30 with compound 2. m/z: $[1/2M+H]^+$ 595.4.

**Example 100:** Synthesis of 2-((2-(3-(2,5-dioxo-2,5-dihydro-1H-pyrrol-1-yl)propionylamino)ethyl)dimercapto)-2-methylpropyl 3-(1-(2-amino-4-((2-hydroxyethyl)(propyl)carbamoyl)-3H-benzo[b]azepin-8-yl)cyclopropylformamido)-7,8-dihydro-1,6-naphthyridine-6(5H)-carboxylate (II-52)

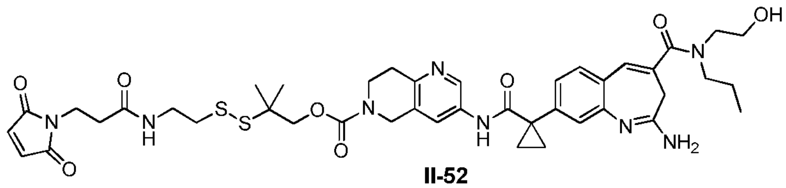

II-52

Using the synthesis method of compound II-20, compound II-52 was obtained as a white solid by reacting L-31 with compound 2. m/z: $[M+H]^+$ 861.2.

**Example 101:** Synthesis of (E)-methyl 1-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-1,23,27-trioxo-4,7,10,13,16,19-hexaoxa-22,26-diazahentriacont-28-en-30-oate (II-53)

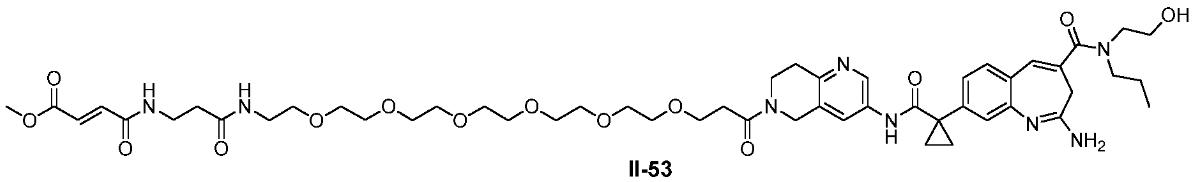

II-53

Using the synthesis method of compound II-6, compound II-53 was obtained as a white solid by reacting L-32 with compound 2. m/z: $[1/2M+H]^+$ 511.4.

**Example 102:** Synthesis of 2-amino-8-(1-((5-(((S)-2-((S)-2-(6-(2,5-dioxo-2,5-dihydro-1H-pyrrol-1-yl)hexanoylamino)-3-methylbutanoylamino)-5-ureidopentanoylamino)methyl)pyridin-3-yl)carbamoyl)cyclopropyl)-N-(2-hydroxyethyl)-N-propyl-3H-benzo[b]azepine-4-formamide (II-54)

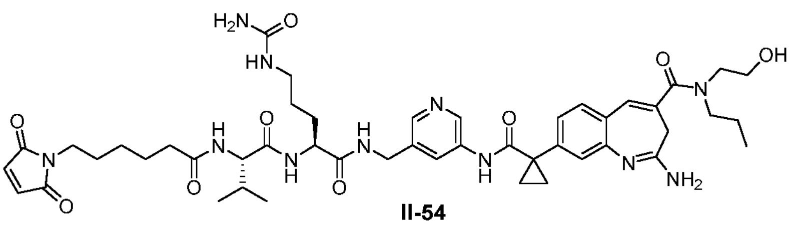

II-54

Using the synthesis method of compound II-49, compound II-54 was obtained as a white solid by reacting Mc-Val-Cit-OH with compound 7. m/z: $[M+Na]^+$ 948.4.

**Example 103:** Synthesis of 2-amino-8-(1-((6-((S)-2-((S)-2-(4-(2,5-dioxo-2,5-dihydro-1H-pyrrol-1-yl)benzoylatnino)-3-methylbutanoylatnino)-5-ureidopentanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-55)

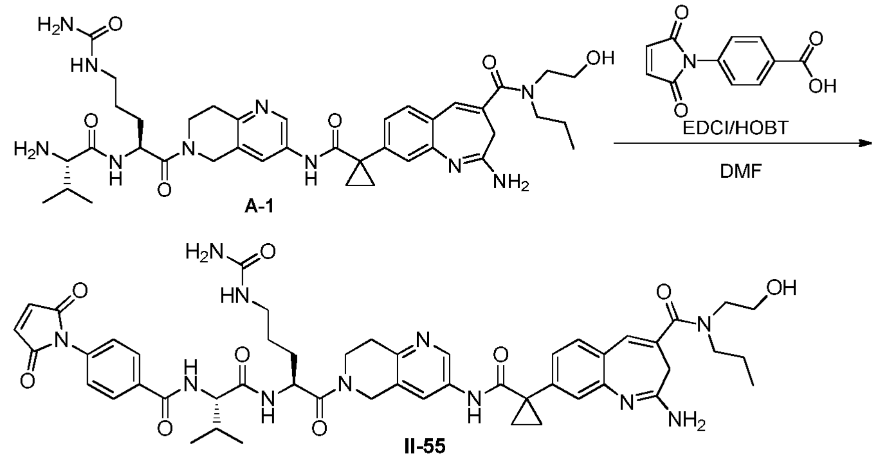


A-1

II-55

To a solution of A-1 (15 mg, 0.02 mmol), EDCI (6 mg, 0.03 mmol) and HOBT (4 mg, 0.03 mmol) in DMF (1.5 mL) was added 4-(2,5-dioxo-2,5-dihydro-1H-pyrrol-1-yl)benzoic acid (4 mg, 0.02 mmol) under ice bath conditions. The reaction solution was stirred at room temperature for 1 hour, then DIPEA (8 mg, 0.06 mmol) was added, and the reaction solution was continued to be stirred at room temperature for 1 hour and then directly purified by *prep-HPLC* (eluent 5%-45% acetonitrile in a 0.1% aqueous trifluoroacetic acid solution, 20 minutes) to give II-55 (2.5 mg, yield 10%) as a white solid. m/z: $[1/2M+H]^+$ 479.8.

**Example 104:** Synthesis of 2-amino-8-(1-((6-((S)-2-((S)-2-(6-(3-bromo-5-methylene-2-oxo-2,5-dihydro-1H-pyrrol-1-yl)hexanoylamino)-3-methylbutanoylamino)-5-ureidopentanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-56)

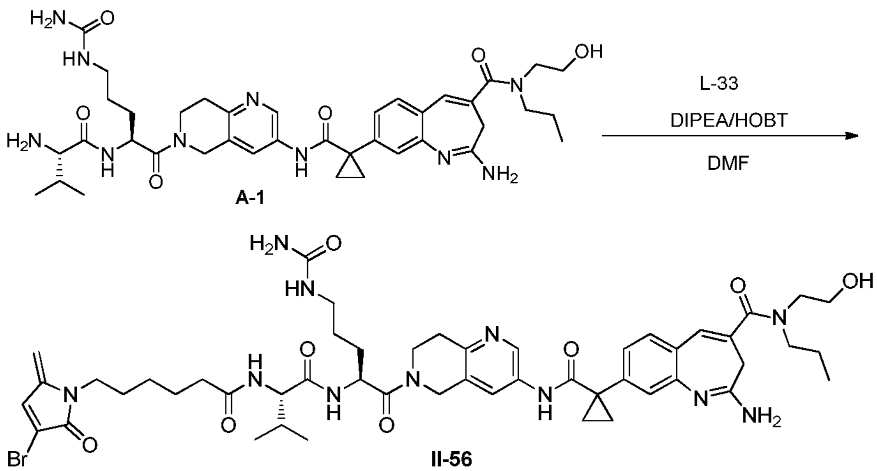


A-1

II-56

To a solution of compound A-1 (8.4 mg, 0.02 mmol) and L-33 (15 mg, 0.02 mmol) in DMF (1.5 mL) were added HOBT (3.2 mg, 0.02 mmol) and DIPEA (3.9 mg, 0.03 mmol) under ice bath conditions. The reaction solution was stirred at room temperature for 1 hour and then directly purified by *prep-HPLC* (eluent 10%-50% acetonitrile in a 0.1% aqueous trifluoroacetic acid solution, 20 minutes) to give II-56 (1.48 mg, yield: 6%) as a yellow solid. m/z: $[1/2M+H]^+$ 514.8.

**Example 105:** Synthesis of 2-amino-8-(1-((6-((S)-2-((S)-2-(2-(2,5-dioxo-2,5-dihydro-1H-pyrrol-1-yl)acetylamino)-3-methylbutanoylamino)-5-ureidopentanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-57)

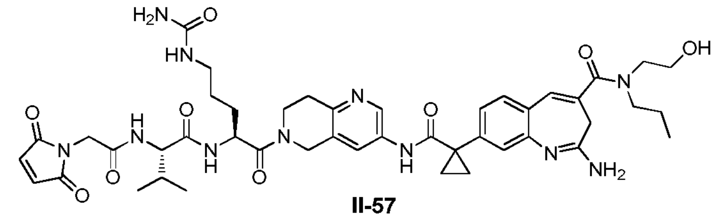

II-57

Using the synthesis method of compound II-55, compound II-57 was obtained as a white solid by reacting 2-(2,5-dioxo-2,5-dihydro-1H-pyrrol-1-yl)acetic acid with compound A-1. m/z: $[M+H]^+$ 896.4.

**Example 106:** Synthesis of 2-amino-N-(2-hydroxyethyl)-8-(1-((6-((S)-2-((S)-3-methyl-2-(6-(4-(3-tosyl-2-(tosylmethyl)propionyl)benzoylamino)hexanoylamino)butanoylamino)-3-ureidopentanoyl)-5,6,7,8-tetrahydro-1,6-naphthyridin-3-yl)carbamoyl)cyclopropyl)-N-propyl-3H-benzo[b]azepine-4-formamide (II-58)

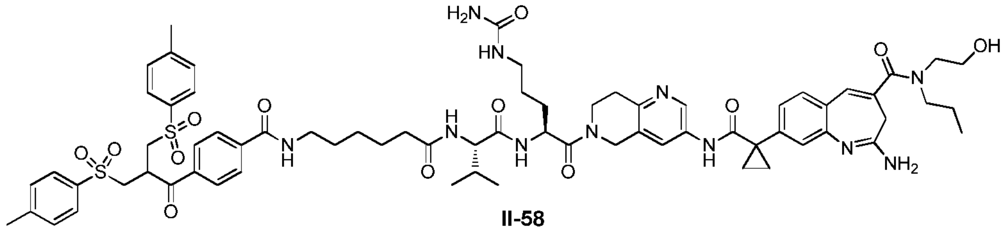

II-58

Using the synthesis method of compound II-6, compound II-58 was obtained as a white solid by reacting L-34 with compound A-1. m/z: $[1/2M+H]^+$ 677.8.

**Example 107:** Synthesis of 2-amino-8-(1-((6-(1-(4-(2,5-dioxo-2,5-dihydro-1H-pyrrol-1-yl)phenyl)-2-oxo-6,9,12,15,18,21-hexaoxa-3-azatetracosan-24-acyl)-5,6,7, 8-tetrahydro-1,6-naphthyridin-3-yl)carbamoyl)cyclopropyl)-N-(2-hydroxyethyl)-N-propyl-3H-benzo[b]azepine-4-formamide (II-59)

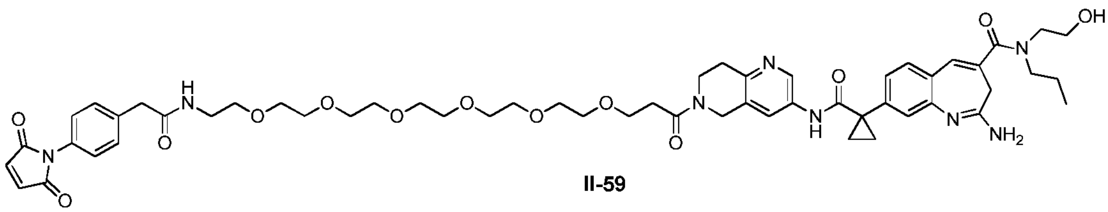

II-59

Using the synthesis method of compound II-6, compound II-59 was obtained as a white solid by reacting L-35 with compound **2.** m/z: $[1/2M+H]^+$ 526.3.

**Example 108:** Synthesis of 3-(2-(6-(2,5-dioxo-2,5-dihydro-1H-pyrrol-1-yl)hexanoylamino)acetylamino)-4-(sulfonyloxy)benzyl 3-(1-(2-amino-4-((2-hydroxyethyl)(propyl)amino)-3H-benzo[b]azepin-8-yl)cyclopropylformamido)-7,8-dihydro-1,6-naphthyridine-6(5H)-carboxylate (II-60)

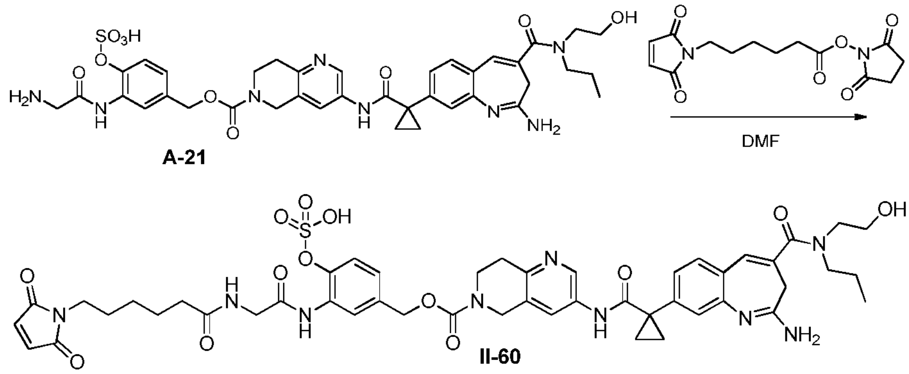


A-21

II-60

Using the synthesis method in steps 1 and 3 of compound A-13, compound A-21 was obtained as a white solid by reacting L-36 with compound 2. m/z: $[M+H]^+$ 805.2.

Using the synthesis method in step 4 of compound II-36, compound II-60 was obtained as a white solid by reaction with A-21. m/z: $[M+H]^+$ 998.2.

**Example 109:** Synthesis of 2-amino-8-(1-((5-(17-(2,5-dioxo-2,5-dihydro-1H-pyrrol-1-yl)-3,6,16-trioxo-9,12-dioxa-2,5,15-triazaheptadecyl)pyridin-3-yl)carbamoyl)cyclopropyl)-N-(2-hydroxyethyl)-N-propyl-3H-benzo[b]azepine-4-formamide (II-61)

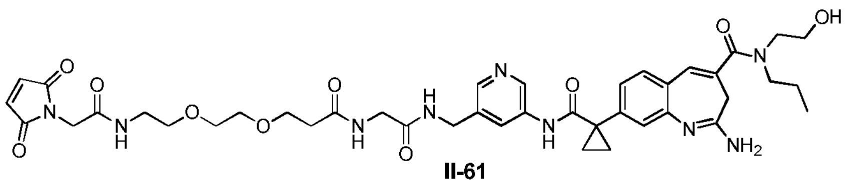

II-61

Using the synthesis method of compound II-46, compound II-61 was obtained as a white solid by reacting L-28 with compound 7. m/z: $[M+H]^+$ 830.4.

**Example 110:** Synthesis of 2-amino-8-(1-((6-(28-(3-bromo-5-methylene-2-oxo-2,5-dihydro-1H-pyrrol-1-yl)-23-oxo-4,7,10,13,16,19-hexaoxa-22-azaoctacosan-1-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-62)

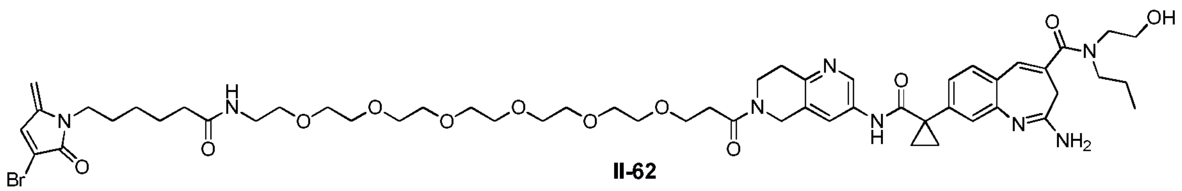

II-62

Using the synthesis method of compound II-56, compound II-62 was obtained as a yellow solid by reacting L-33 with compound 30. m/z: $[1/2M+H]^+$ 554.2.

**Example 111:** Synthesis of 2-amino-8-(1-((6-(1-(2,5-dioxo-2,5-dihydro-1H-pyrrol-1-yl)-2,12-dioxo-6,9-dioxa-3,13-diazapentadecan-15-acyl)-5,6,7,8-tetrahydro-1,6-naphthyridin-3-yl)carbamoyl)cyclopropyl)-6-fluoro-N-(2-hydroxyethyl)-N-propyl-3H-benzo[b]azepine-4-formamide (II-63)

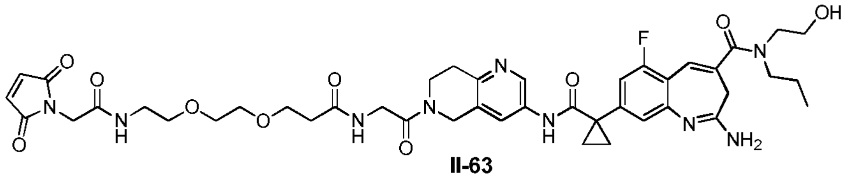

II-63

Using the synthesis method of compound II-46, compound II-63 was obtained as a white solid by reacting L-28 with compound 6. m/z: [M+H]+ 874.3.

**Example 112:** Synthesis of 2-amino-8-(1-((5-(17-(2,5-dioxo-2,5-dihydro-1H-pyrrol-1-yl)-3,6,16-trioxo-9,12-dioxa-2,5,15-triazaheptadecyl)pyridin-3-yl)carbamoyl)cyclopropyl)-N-(2-hydroxyethyl)-6-fluoro-N-propyl-3H-benzo[b]azepine-4-formamide (II-64)

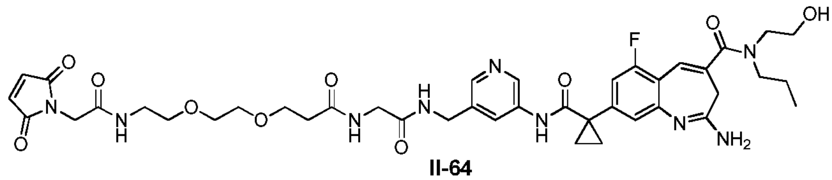

II-64

Using the synthesis method of compound II-46, compound II-64 was obtained as a white solid by reacting L-28 with compound 12. m/z: [M+H]+ 848.4.

**Example 113:** Synthesis of 2-amino-8-(1-((6-(((S)-2-((S)-2-(6-(2,5-dioxo-2,5-dihydro-1H-pyrrol-1-yl)hexanoylamino)-3-methylbutanoylamino)-5-ureidopentanoyl)-5,6,7,8-tetrahydro-1,6-naphthyridin-3-yl)amino)-2-methyl-1-oxopropan-2-yl)-N,N-dipropyl-3H-benzo[b]azepine-4-formamide (II-65)

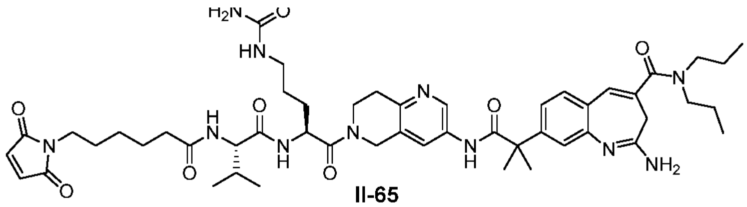

II-65

Using the synthesis method of compound II-49, compound II-65 was obtained as a white solid by reacting Mc-Val-Cit-OH with compound 5. m/z: [M+H]+ 952.4.

**Example 114:** Synthesis of 2-amino-8-(1-((6-(1-(2,5-dioxo-2,5-dihydro-1H-pyrrol-1-yl)-3-oxo-7,10,13,16,19,22-hexaoxa-4-azapentacosan-25-acyl)-5,6,7,8-tetrahydro-1,6-naphthyridin-3-yl)carbamoyl)cyclopropyl)-N-ethoxy-N-propyl-3H-benzo[b]azepine-4-formamide (II-66)

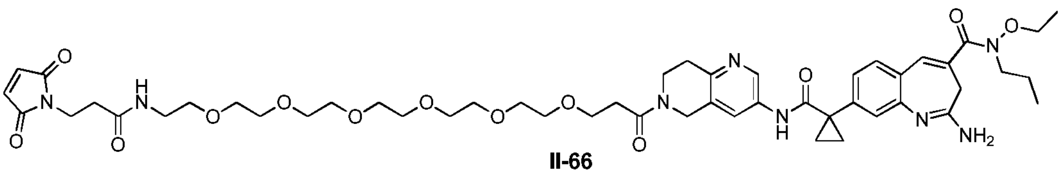

II-66

Using the synthesis method of compound II-6, compound II-66 was obtained as a white solid by reacting 1-(2,5-dioxo-2,5-dihydro-1H-pyrrol-1-yl)-3-oxo-7,10,13,16,19,22-hexaoxa-4-azapentacosan-25-acid with compound 23. m/z: [1/2M+H]+495.3.

**Example 115:** Synthesis of 2-amino-8-(1-((5-(26-(2,5-dioxo-2,5-dihydro-1H-pyrrol-1-yl)-3,25-dioxo-6,9,12,15,18,21-hexaoxa-2,24-diazahexacosyl)pyridin-3-yl)carbamoyl)cyclopropyl)-6-fluoro-N,N-dipropyl-3H-benzo[b]azepine-4-formamide (II-67)

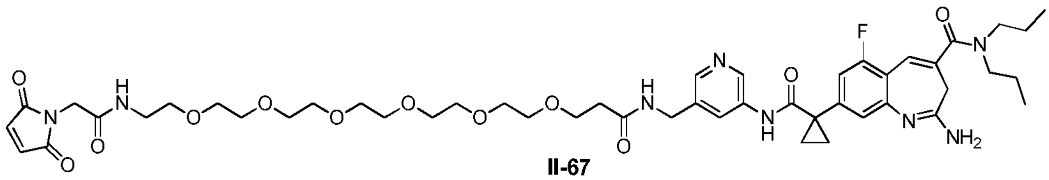

**II-67**

Using the synthesis method of compound II-6, compound II-67 was obtained as a white solid by reacting L-22 with compound 13. m/z: $[1/2M+H]^+$ 483.3.

**Example 116:** Synthesis of (S)-2-amino-8-(1-((6-(2-(6-(2,5-dioxo-2,5-dihydro-1H-pyrrol-1-yl)hexanoylamino)-5-ureidopentanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-68)

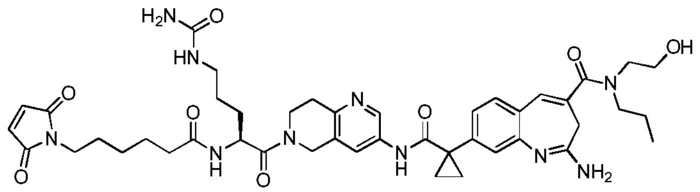

**II-68**

Using the synthesis method of compound II-49, compound II-68 was obtained as a white solid by reacting L-37 with compound 2. m/z: $[M+H]^+$ 853.3.

**Example 117:** Synthesis of 4-(3-(2-(2-(3-(2,5-dioxo-2,5-dihydro-1H-pyrrol-1-yl)propionylamino)ethoxy)ethoxy)propionylamino)benzyl 7-(1-(2-amino-4-(dipropylformamido)-3H-benzo[b]azepin-8-yl)cyclopropane-1-formylamino)-3,4-dihydroisoquinoline-2(1H)-carboxylate (II-69)

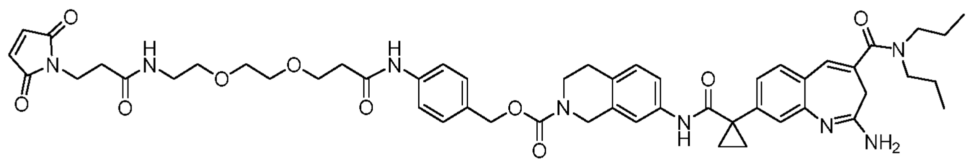

**II-69**

Using the synthesis method of compound II-20, compound II-69 was obtained as a white solid by reacting L-14 with compound 15. m/z: $[M+H]^+$959.3.

**Example 118:** Synthesis of 2-amino-8-(1-((6-(2-((S)-2-((S)-2-(6-(2,5-dioxo-2,5-dihydro-1H-pyrrol-1-yl)hexanoylamino)-3-methylbutanoylamino)-5-ureopentanoylamino)acetyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-70)

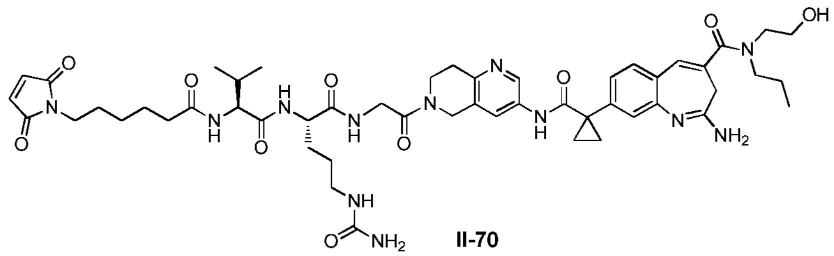

II-70

Using the synthesis method of compound II-46, compound II-70 was obtained as a white solid by reacting L-38 with compound 2. m/z: $[1/2M+H]^+$ 505.4.

**Example 119:** Synthesis of 2-amino-8-(2-((6-(3-(2-(2-(2-(2,5-dioxo-2,5-dihydro-1H-pyrrol-1-yl)acetylamino)ethoxy) ethoxy)propionyl)-5,6,7,8-tetrahydro-1,6-naphthyridin-3-yl)amino)-2-oxoacetyl)-N,N-dipropyl-3H-benzo[b]azepine-4-formamide (II-71)

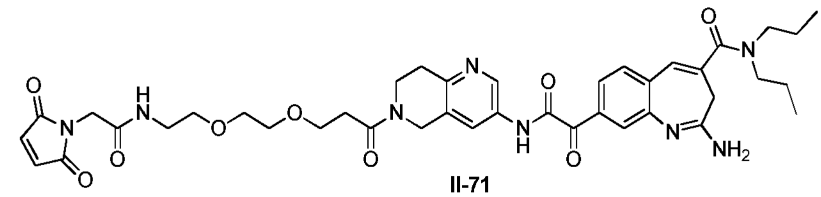

II-71

Using the synthesis method of compound II-6, compound II-71 was obtained as a white solid by reacting L-8 with compound 4. m/z: $[M+H]^+$ 785.4.

**Example 120:** Synthesis of (S)-2-amino-8-(1-((6-(5-benzyl-18-(2,5-dioxo-2,5-dihydro-1H-pyrrol-1-yl)-4,7,10,13-tetraoxo-3,6,9,12-tetraoxaoctadecan-1-yl)-5,6,7, 8-tetrahydro-1,6-naphthyridin-3-yl)carbamoyl)cyclopropyl)-N-(2-hydroxyethyl)-N-propyl-3H-benzo[b]azepine-4-formamide (II-72)

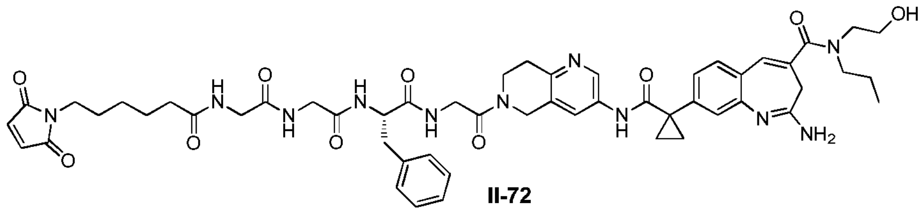

II-72

Using the synthesis method of compound II-49, compound II-72 was obtained as a white solid by reacting Mc-Gly-Gly-Phe-Gly-OH with compound 2. m/z: $[M+H]^+$ 507.8.

**Example 121:** Synthesis of 2-amino-8-(1-((6-((S)-2-((S)-2-(6-(2,5-dioxo-2,5-dihydro-1H-pyrrol-1-yl)hexanoylamino)-3-methylbutanoylamino)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-73)

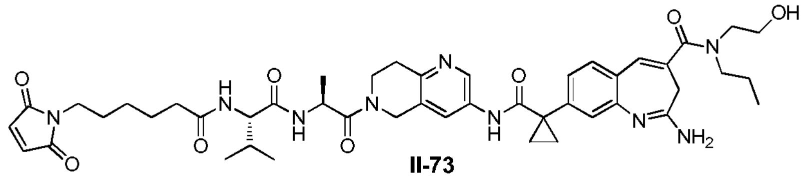

II-73

Using the synthesis method of compound II-49, compound II-73 was obtained as a white solid by reacting Mc-Val-Ala-OH with compound 2. m/z: [1/2M+H]$^+$433.8.

**Example 122:** Synthesis of 2-amino-8-(1-((6-(1-(3,5-diacryloyl-1,3,5-triazin-1-yl)-1,6-dioxo-10,13,16,19,22,25-hexaoxa-4-thia-7-azaoctacosan-28-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-74)

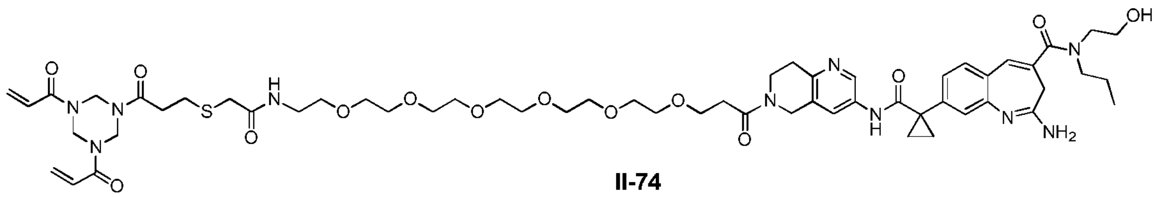

II-74

Using the synthesis method of compound II-39, compound II-74 was obtained as a white solid by reacting L-39 with compound 2. m/z: [1/2M+H]$^+$ 581.4.

**Example 123:** Synthesis of 2-((3-((2-(3-(2,5-dioxo-2,5-dihydro-1H-pyrrol-1-yl)propionylamino)ethyl)amino)-3-oxopropyl)dimercapto)-2-methylpropyl 3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridine-6(5H)-carboxylate (II-75)

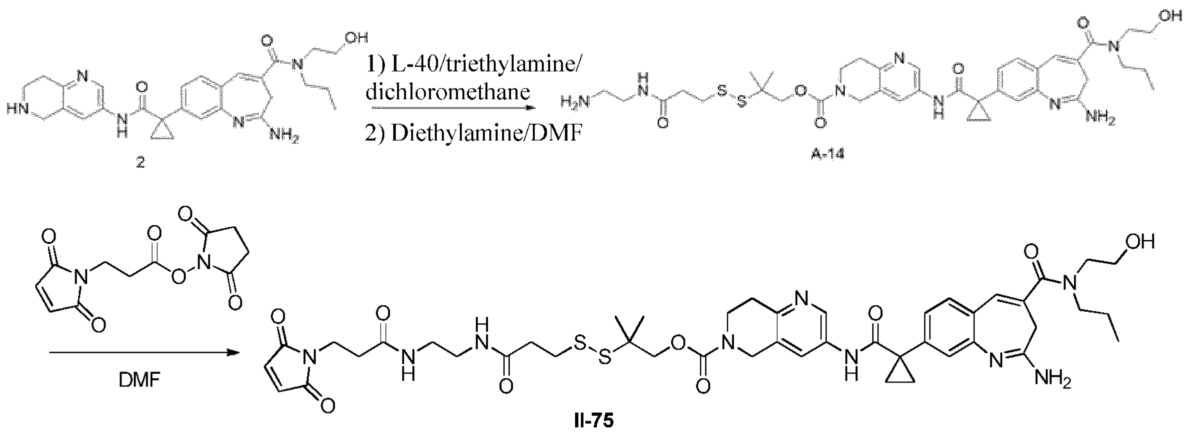


2

A-14

II-75

Step 1: To a solution of L-40 (61 mg, 0.1 mmol) in dichloromethane (2 mL) were added compound 2 (40 mg, 0.08 mmol) and triethylamine (30 mg, 0.3 mmol) under ice bath conditions, and the reaction solution was stirred at room temperature for 1 hour. The reaction solution was concentrated under reduced pressure, the crude product was dissolved in DMF (3 mL), then diethylamine (0.6 mL) was added, and the resulting mixture was stirred at room temperature for 35 minutes. The reaction solution was directly purified by Flash column chromatography (C18, eluent 0%-47% acetonitrile in a 10 mM aqueous ammonium bicarbonate solution) to give compound A-14 (65 mg, yield: 83%) as a white solid. m/z:[M+H]$^+$ 781.3.

Step 2: Using the synthesis method of compound II-2, II-75 was obtained as a white solid by reaction with A-14. m/z:[1/2M+H]$^+$ 466.8.

**Example 124:** Synthesis of 2-amino-8-(1-((6-(2-((2,5-dioxo-2,5-dihydro-1H-pyrrol-1-yl)methyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide, isomer 1 (II-76)

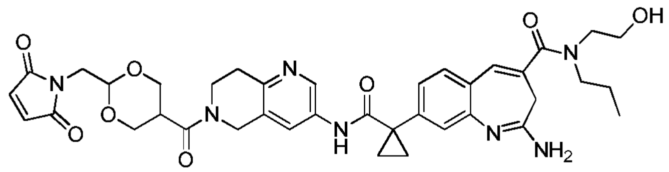

**II-76**

Using the synthesis method of compound II-6, compound II-76 was obtained as a white solid by reacting L-41A with compound 2. UPLC retention time 3.600 minutes; m/z: $[M+H]^+$ 726.3.

**Example 125:** Synthesis of 2-amino-8-(1-((6-(2-((2,5-dioxo-2,5-dihydro-1H-pyrrol-1-yl)methyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide, isomer 2 (II-77)

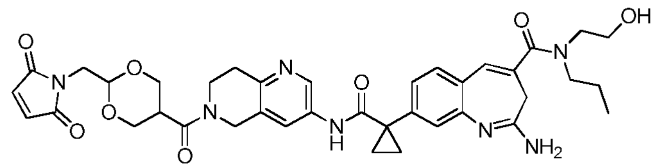

**II-77**

Using the synthesis method of compound II-6, compound II-77 was obtained as a white solid by reacting L-41B with compound 2. UPLC retention time 3.998 minutes; m/z: $[M+H]^+$ 726.3.

**Example 126:** Synthesis of 2-amino-8-(1-((6-(1-(2-((2,5-dioxo-2,5-dihydro-1H-pyrrol-1-yl)methyl)-1,3-dioxan-5-yl)-1-oxo-5,8,11,14,17,20-hexaoxa-2-azatricosan-23-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-78)

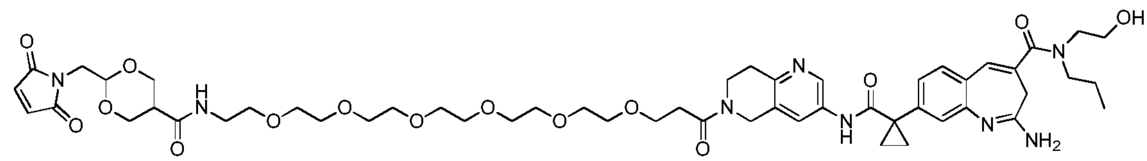

**II-78**

Using the synthesis method of compound II-6, compound II-78 was obtained as a white solid by reacting L-42 with compound 2. UPLC retention times 4.196 and 4.280 minutes; m/z: $[1/2M+H]^+$ 531.3.

Example 127: Synthesis of 2-amino-8-(1-((6-(4-((2-(4-((2,5-dioxo-2,5-dihydro-1H-pyrrol-1-yl)methyl)cyclohexane-1-formylamino)ethyl)carbamoyl)benzyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-79)

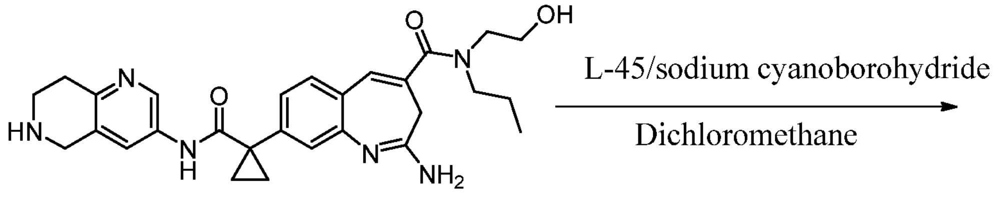


2

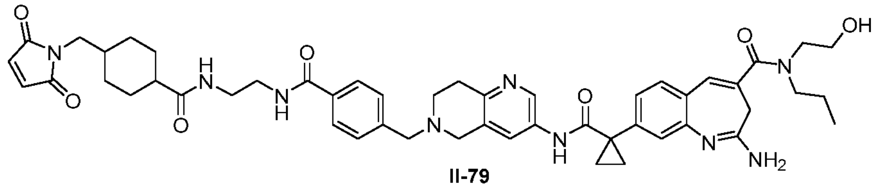

II-79

A solution of L-45 (15 mg, 0.036 mmol) and compound 2 (21.7 mg, 0.043 mmol) in dichloromethane (2.5 mL) was stirred at room temperature for 6 hours, then sodium cyanoborohydride (3.4 mg, 0.054 mmol) was added, the reaction system continued to be stirred at room temperature for 16 hours, and the reaction solution was concentrated under reduced pressure and then purified by prep-HPLC (eluent 20%-80% acetonitrile in a 0.1% aqueous trifluoroacetic acid solution, 20 minutes) to give II-79 (6.3 mg, yield: 16%) as a white solid. m/z: $[1/2M+H]^+$449.8.

**Example 128:** Synthesis of amino-8-(1-((6-(1-(2,5-dioxo-2,5-dihydro-1H-pyrrol-1-yl)-2-oxo-6,9,12,15,18,21-hexaoxa-3-azatetracosan-24-acyl)-5,6,7,8-tetrahydro-1,6-naphthyridin-3-yl)-2-methyl-1-oxopropan-2-yl)-N,N-dipropyl-3H-benzo[b]azepine-4-formamide (II-80)

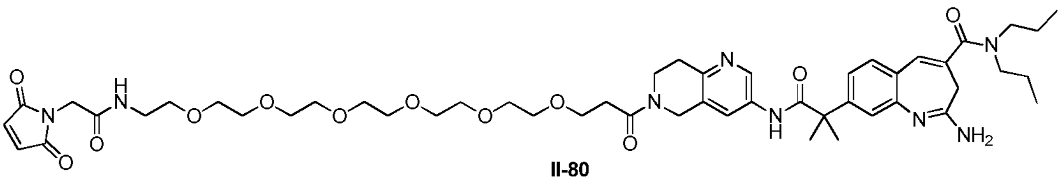

II-80

Using the synthesis method of compound II-6, compound II-80 was obtained as a white solid by reacting L-22 with compound 5. m/z: $[1/2M+H]^+$ 488.3.

**Example 129:** Synthesis of (E)-2-((3-(2-(1-(4-(2,5-dioxo-2,5-dihydro-1H-pyrrol-1-yl)phenyl)ethylidene)hydrazino)-3-oxopropyl)dimercapto)-2-methylpropyl 3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridine-6(5H)-carboxylate (II-81)

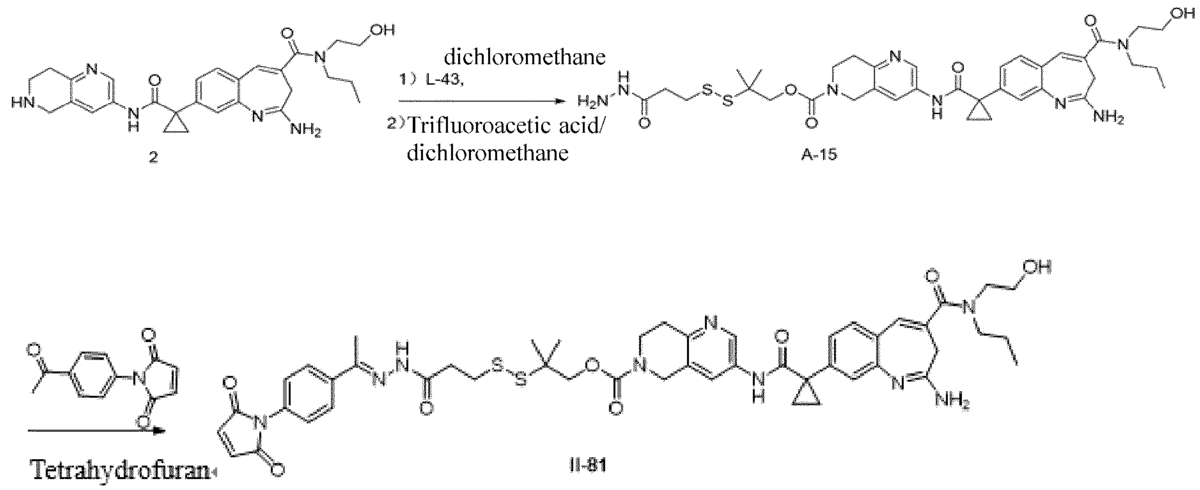


2

A-15

II-81

Step 1: To a solution of L-43 (61.0 mg, 0.04 mmol) in dichloromethane (2 mL) were added compound 2 (22.1 mg, 0.04 mmol) and triethylamine (13.4 mg, 0.13 mmol) under ice bath conditions, and the reaction system was stirred at room temperature for 1 hour and directly concentrated under reduced pressure. To the residue were added dichloromethane (3 mL) and trifluoroacetic acid (1 mL) under ice bath conditions, and the reaction system was stirred at room temperature for 1 hour. The reaction solution was concentrated under reduced pressure and then purified by Flash column chromatography (C18, eluent 0%-40% acetonitrile in a 0.02% aqueous trifluoroacetic acid solution) to give compound A-15 (21.8 mg, yield: 95%) as a white solid. m/z: $[M+H]^+$753.3.

Step 2: To a solution of compound A-15 (16.8 mg, 0.02 mmol) in tetrahydrofuran (2 mL) was added trifluoroacetic acid (2.5 mg, 0.02 mmol), the reaction system was stirred at room temperature for 10 minutes, then 1-(4-acetophenyl)-1H-pyrrole-2,5-dione (5.0 mg, 0.02 mmol) was added, and stirring was continued for 1 hour. The reaction system was then directly purified by *prep-HPLC* (eluent 20-80% acetonitrile in a 10 mM aqueous ammonium bicarbonate solution, 20 minutes) to give compound II-81 (0.83 mg, yield: 3%) as a white solid. m/z: $[M+H]^+$ 950.2.

**Example 130:** Synthesis of (S)-N-(1-(3-(1-(2-amino-4-((2-hydroxyethyl)(propyl)carbamoyl)-3H-benzo[b]azepin-8-yl) cyclopropyl-1-formylamino)-7,8-dihydro-1,6-naphthyridin-6(5H)-yl)-1-oxo-5-ureidopentan-2-yl)-N-(5-(2,5-dioxo-2,5-dihydro-1H-pyrrol-1-yl)pentyl)cyclobutane-1,1-dimethylamide (II-82)

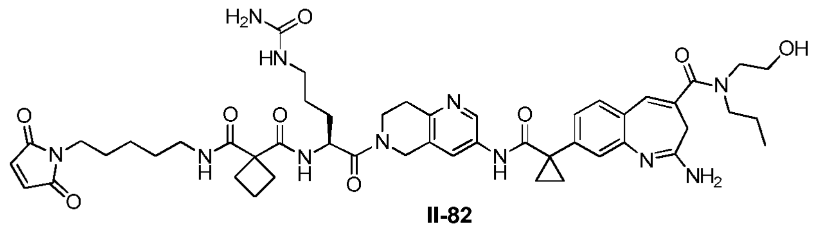

**II-82**

Using the synthesis method of compound II-6, compound II-82 was obtained as a white solid by reacting 1-(5-aminopentyl)-1H-pyrrole-2,5-dione with compoundA-3. m/z: $[1/2M+H]^+$475.8.

**Example 131:** Synthesis of 2-amino-8-(1-((6-(3-(2-(2-(2-(2,5-dioxo-2,5-dihydro-1H-pyrrol-1-yl)-N-methylacetylamino)-N-methylacetylamino)acetylamino)propionyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-83)

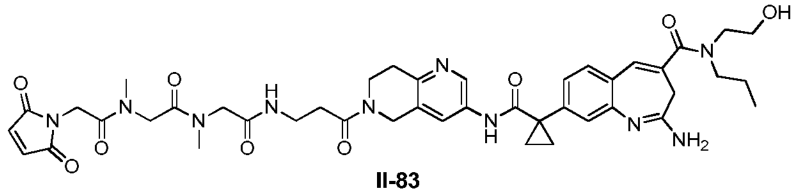

**II-83**

Using the synthesis method of compound II-46, compound II-83 was obtained as a white solid by reacting L-46 with compound 2. m/z: $[M+H]^+$ 853.2.

Example 132: Synthesis of pentafluorophenol 22-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)-22-oxo-4,7,10,13,16,19-hexaoxadocosan-1-oate (II-84)

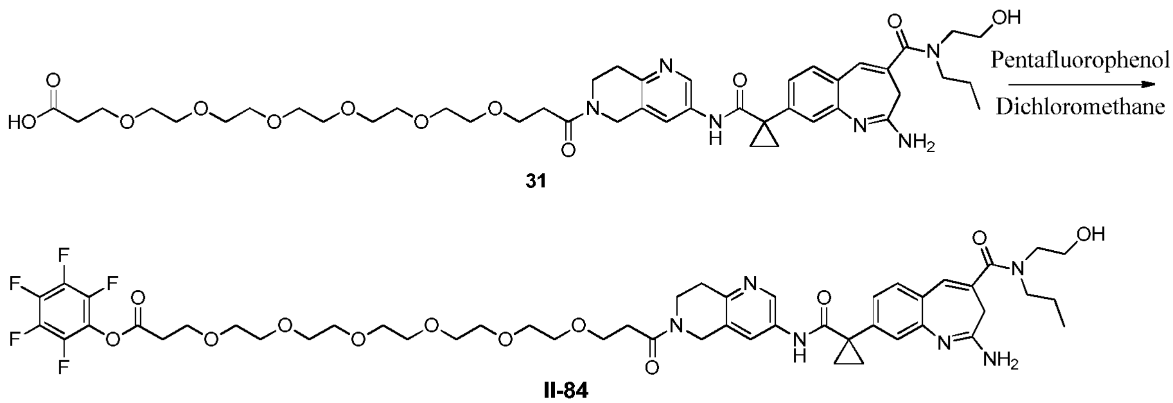


**31**

**II-84**

To a solution of compound 31 (10 mg, 0.012 mmol) in dichloromethane (3 mL) were added 2,3,4,5,6-pentafluorophenol (4 mg, 0.02 mmol) and EDCI (5 mg, 0.03 mmol) sequentially. The reaction solution was stirred at room temperature for 2 hours and concentrated under reduced pressure, and the residue was purified by *prep-HPLC* (eluent 15%-60% acetonitrile in a 0.1% aqueous trifluoroacetic acid solution, 20 minutes) to give compound II-84 (3.5 mg, yield: 11%) as a white solid. m/z: $[1/2\ M+H]^+$ 517.2.

**Example 133:** Synthesis of 2-amino-8-(1-((6-((S)-2-((R)-2-amino-3-(2-(2,5-dioxo-2,5-dihydro-1H-pyrrol-1-yl)acetylamino)propionylamino)-5-ureidopentanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-85)

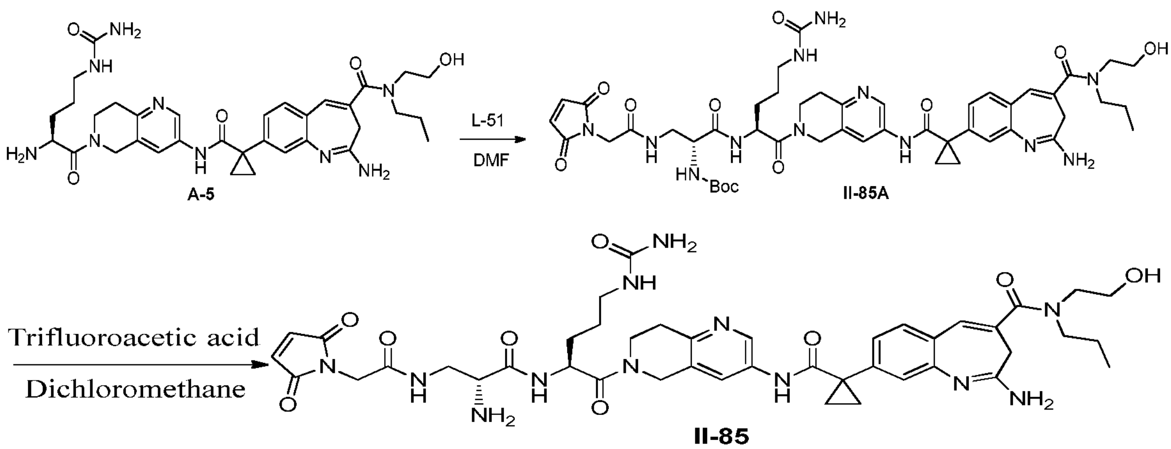


**A-5**

**II-85A**

**II-85**

Step 1: To a solution of compound A-5 (13 mg, 0.02 mmol) and L-51 (6.8 mg, 0.02 mmol) in DMF (1.5 mL) were

added HOBT (3.2 mg, 0.02 mmol) and EDCI (4.6 mg, 0.02 mmol) sequentially. The reaction solution was stirred at room temperature overnight and directly purified by Flash column chromatography (C18, eluent 0-45% acetonitrile in a 0.1% aqueous trifluoroacetic acid solution) to give II-85A (8 mg, yield: 41%) as a white solid. m/z: $[1/2M+H]^+$ 492.3.

Step 2: To a solution of II-85A(8 mg, 8.1 μM) in dichloromethane (2 mL) was added trifluoroacetic acid (0.5 mL). The reaction solution was stirred at room temperature for 0.5 h and then directly purified by *prep-HPLC* (eluent 10%-45% acetonitrile in a 0.1% aqueous trifluoroacetic acid solution, 20 minutes) to give II-85 (4.1 mg, yield: 49%) as a white solid. m/z: $[1/2M+H]^+$ 442.2.

**Example 134:** Synthesis of 2-amino-8-(1-((6-((10S, 13S)-20-(2,5-dioxo-2,5-dihydro-1H-pyrrol-1-yl)-13-isopropyl-6,9,12, 15-tetraoxo-10-(3-ureidopropyl)-3-oxa-5,8,11,14-tetraazaeicosan-1-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-86)

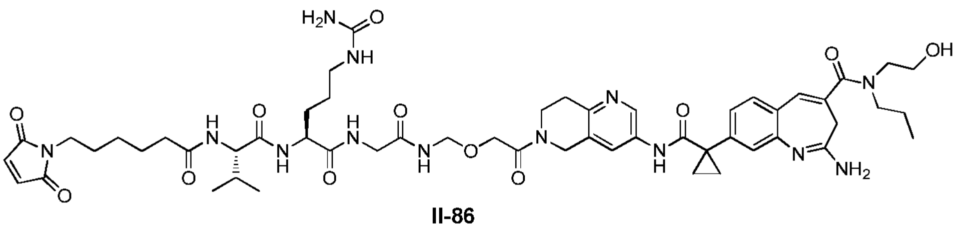

II-86

Using the synthesis method of compound II-6, compound II-86 was obtained as a white solid by reacting L-53 with compound 2. m/z: $[1/2M+H]^+$ 548.8.

**Example 135:** Synthesis of (2S,3S,4S,5R,6S)-6-(4-(((3-(1-(2-amino-4-((2-hydroxyethyl)(propyl)carbamoyl)-3H-benzo [b]azepin-8-yl)cyclopropyl-1-formylamino)-5,6,7,8-tetrahydro-1,6-naphthyridine-6-carbonyl)oxy) methyl)-2-((S)-2-((S)-2-(6-(2,5-dioxo-2,5-dihydro-1H-pyrrol-1-yl)hexanoylamino)-3-methylbutanoylamino)-5-ureidopentanoylamino) phenoxy)-3,4,5-trihydroxytetrahydro-2H-pyran-2-carboxylic acid (II-87)

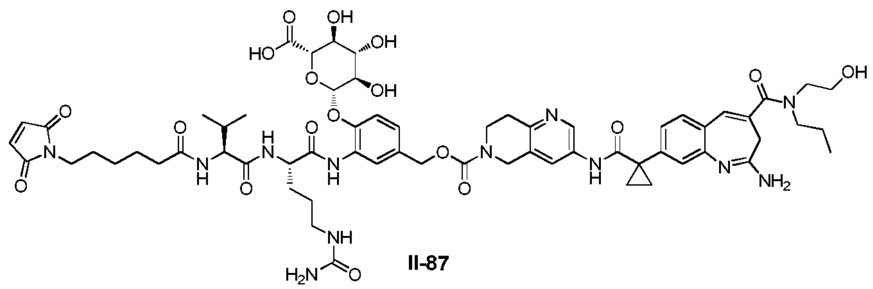

II-87

Using the synthesis method in steps 1, 2 and 4 of compound II-36, compound II-87 was obtained as a white solid by reacting L-54 with compound 2. m/z: $[M+H]^+$ 1293.6.

**Example 136:** Synthesis of 2-amino-8-(1-((4-((S)-2-((S)-2-(6-(2,5-dioxo-2,5-dihydro-1H-pyrrol-1-yl)hexanoylamino)-3-methylbutanoylamino)-5-ureidopentanoylamino)benzyl)carbamoyl)cyclopropyl)-N-(2-hydroxyethyl)-N-propyl-3H-benzo[b]azepine-4-formamide (II-88)

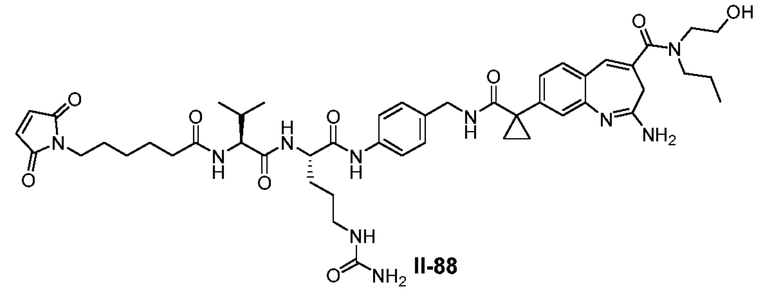

II-88

Using the synthesis method of compound II-2, II-88 was obtained as a white solid by reacting compound A-4 with EMCS. m/z: $[1/2M+H]^+$ 925.4.

**Example 137:** Synthesis of 2-amino-8-(1-((6-((6-(2,5-dioxo-2,5-dihydro-1H-pyrrol-1-yl)hexanoyl)-L-alanyl-L-alanyl-L-asparagyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-89)

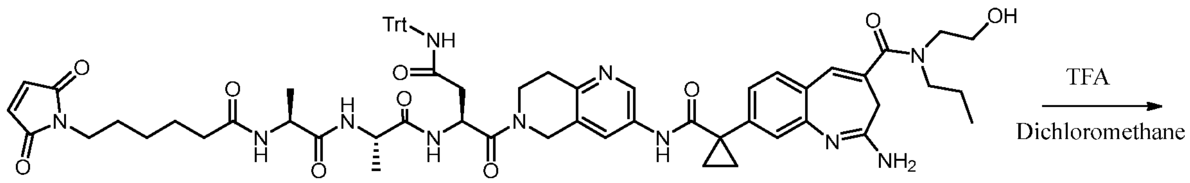


II-89A

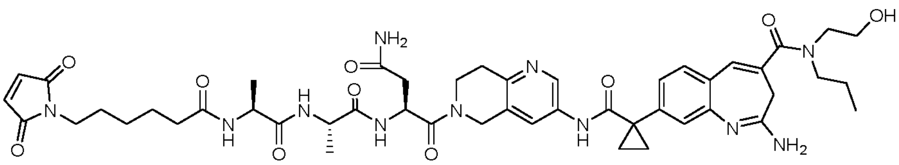

II-89

To a solution of compound II-89A (20 mg, 0.02 mmol) (obtained by reacting compound A-6 with EMCS using the synthesis method of compound II-2) in dichloromethane (2.5 mL) was added trifluoroacetic acid (1 mL) dropwise under ice bath conditions, and the reaction system was stirred at room temperature for 1 hour. The reaction system was concentrated under reduced pressure and then directly purified by *prep*-HPLC (eluent 5-45% acetonitrile in a 0.1% aqueous trifluoroacetic acid solution, 20 minutes) to give compound II-89 (9.1 mg, yield: 50%) as a white solid. m/z:$[1/2M+H]^+$ 952.4.

**Example 138:** Synthesis of (S)-2-amino-8-(1-((6-(10-benzyl-23-(2,5-dioxo-2,5-dihydro-1H-pyrrol-1-yl)-6,9,12,15,18-pentaoxo-3-oxa-5,8,11,14,17-pentaazatricosanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-90)

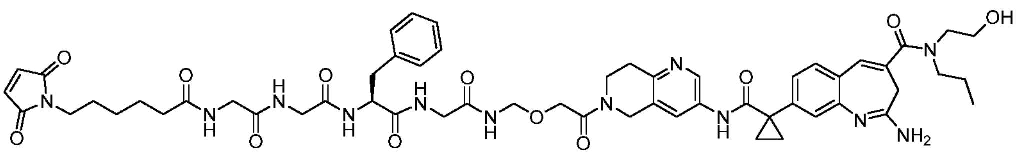

II-90

Using the synthesis method of compound II-6, compound II-90 was obtained as a white solid by reacting L-60 with compound 2. m/z: $[1/2M+H]^+$ 551.4.

**Example 139:** Synthesis of 2-amino-8-(1-((3-((S)-2-((S)-2-(6-(2,5-dioxo-2,5-dihydro-1H-pyrrol-1-yl)hexanoylamino)-3-methylbutanoylamino)-5-ureidopentanoylamino)benzyl)carbamoyl)cyclopropyl)-N-(2-hydroxyethyl)-N-propyl-3H-benzo[b]azepine-4-formamide (II-91)

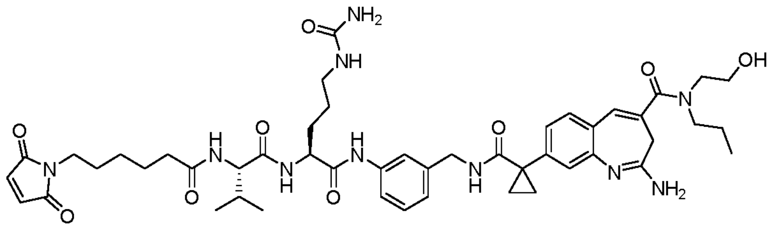

II-91

Using the synthesis method of compound II-2, II-91 was obtained as a white solid by reacting compound A-7 with EMCS. m/z: $[M+H]^+$ 925.4.

**Example 140:** Synthesis of (S)-2-amino-8-(1-((6-(17-benzyl-30-(2,5-dioxo-2,5-dihydro-1H-pyrrol-1-yl)-13,16,19,22,25-pentaoxo-4,7,10-oxa-12,15,18,21,24-pentaazatriacontanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-92)

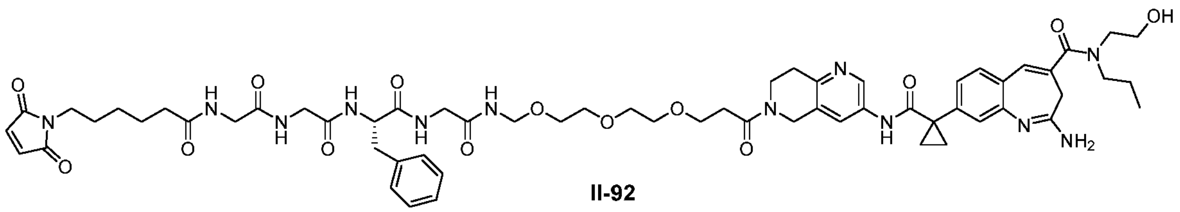

II-92

Using the synthesis method of compound II-2, II-92 was obtained as a white solid by reacting compound A-8 with EMCS. m/z: $[1/2M+H]^+$ 602.4.

**Example 141:** Synthesis of 2-amino-8-(1-((6-((4S,7S)-1-(4-(2,5-dioxo-2,5-dihydro-1H-pyrrol-1-yl)phenyl)-4-isopropyl-2,5,8-trioxo-7-(3-ureidopropyl)-12,15,18,21,24,27-hexaoxa-3,6,9-triazatriaconan-30-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-93)

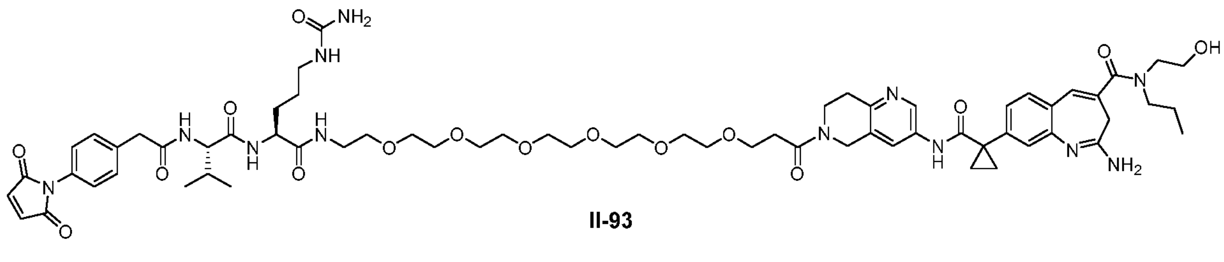

II-93

Using the synthesis method of compound II-6, compound II-93 was obtained as a white solid by reacting L-63 with compound 2. m/z: $[1/2M+H]^+$ 654.4.

**Example 142:** Synthesis of 2-amino-$N^8$-(6-(1-(2,5-dioxo-2,5-dihydro-1H-pyrrol-1-yl)-3-oxo-7,10,13,16,19,22-hexaoxa-4-azapentacosan-25-acyl)-5,6,7,8-tetrahydro-1,6-naphthyridin-3-yl)-$N^4$-(2-hydroxyethyl)-$N^4$-propyl-3H-benzo[b]azepine-4,8-diformylamino (II-94)

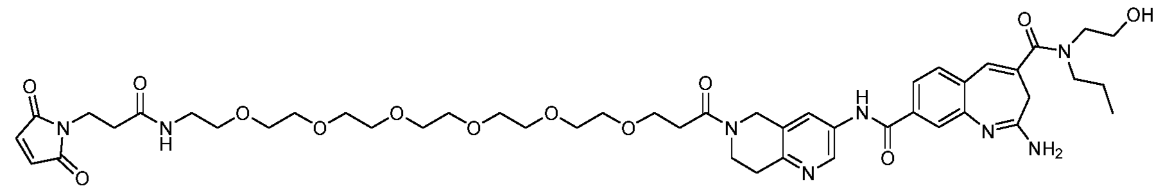

**II-94**

Using the synthesis method of compound II-6, compound II-94 was obtained as a white solid by reacting 1-(2,5-dioxo-2,5-dihydro-1H-pyrrol-1-yl)-3-oxo-7,10,13,16,19,22-hexaoxa-4-azapentacosan-25-acid with compound 8. m/z: $[1/2M+H]^+$ 475.2.

**Examples 143-163:** Using the synthesis method of II-20, the compounds listed in Table 8 were synthesized:

Table 8

| Number | Structure | MS |
|---|---|---|
| II-95 | 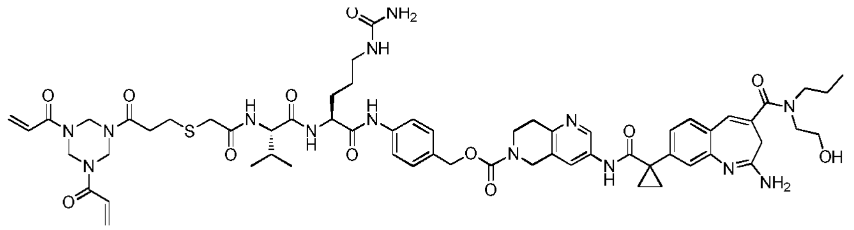 | m/z:$[1/2M +H]^+$ 616.4 |
| II-96 | 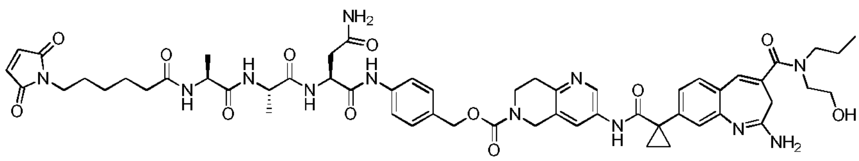 | m/z:$[1/2M +H]^+$ 551.3 |
| II-97 | 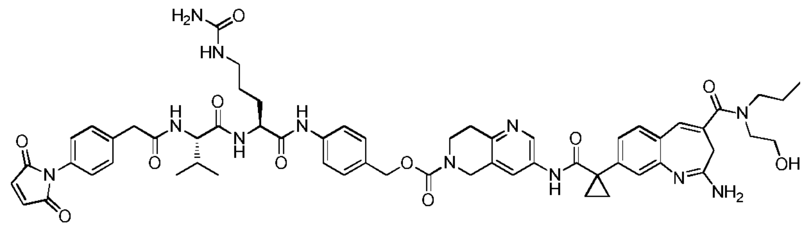 | m/z:$[1/2M +H]^+$ 561.4 |
| II-98 | 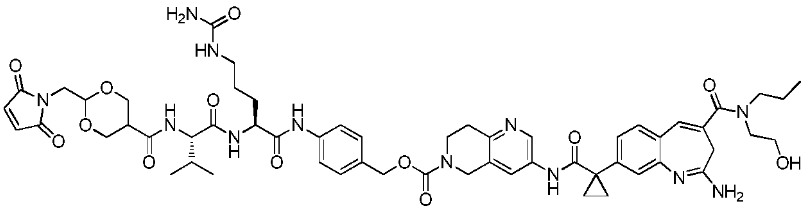 | m/z:$[1/2M +H]^+$ 566.3 |
| II-99 | 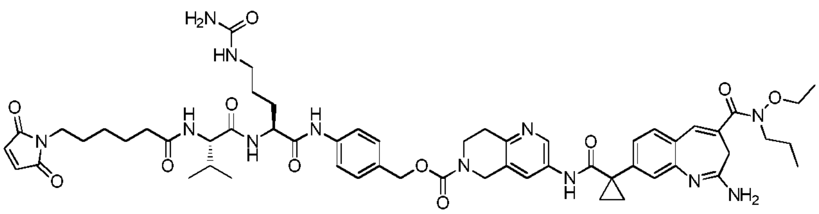 | m/z:$[1/2M +H]^+$ 551.4 |

(continued)

| Number | Structure | MS |
|---|---|---|
| II-100 | 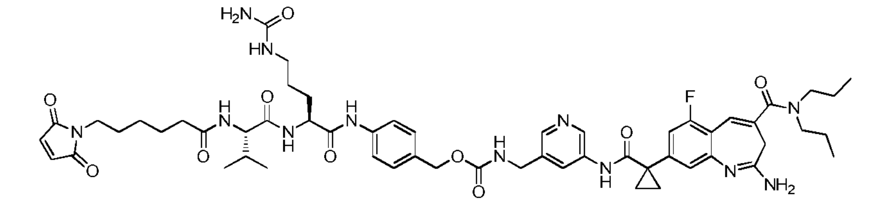 | m/z:[1/2M +H]$^+$ 546.3 |
| II-101 | 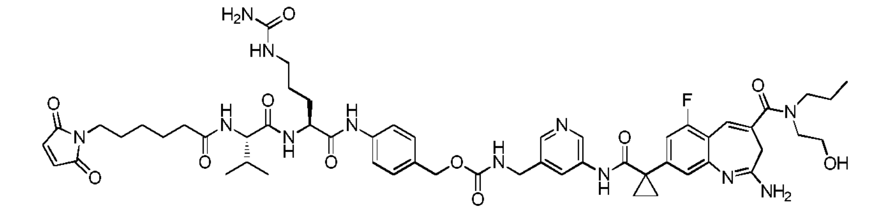 | m/z:[1/2M +H]$^+$ 547.3 |
| II-102 | 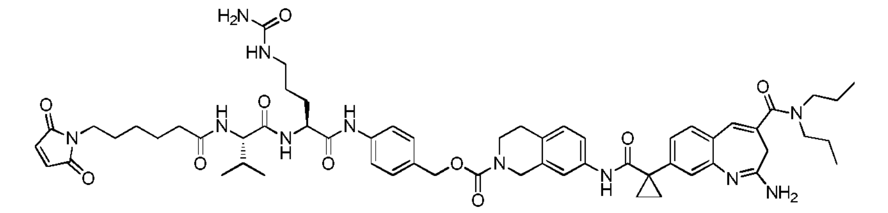 | m/z:[M+H] $^+$ 1098.5 |
| II-103 | 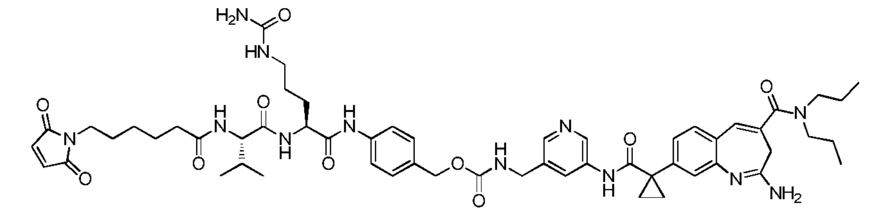 | m/z:[1/2M +H]$^+$ 537.4 |
| II-104 | 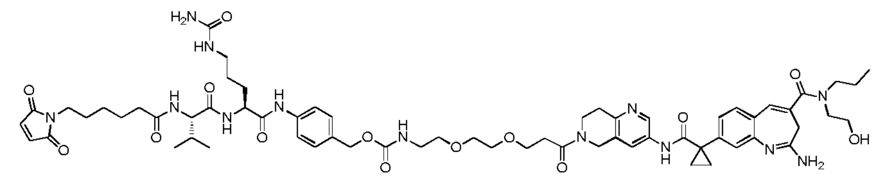 | m/z:[1/2M +H]$^+$ 630.8 |
| II-105 | 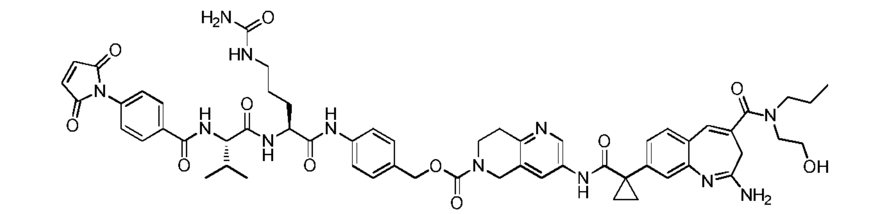 | m/z:[M+H] $^+$ 1107.7 |
| II-106 | 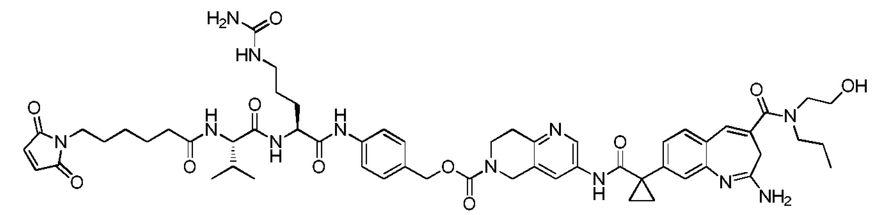 | m/z:[M+H] $^+$ 1101.6 |

(continued)

| Number | Structure | MS |
|---|---|---|
| II-107 | 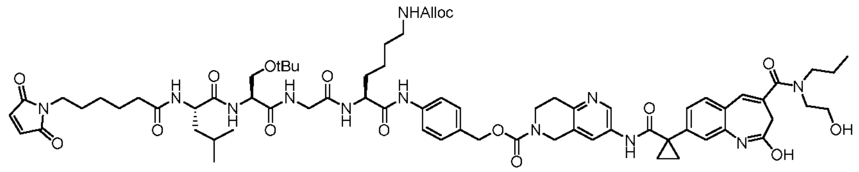 | m/z:[1/2M +H]+ 686.0 |
| II-110 | 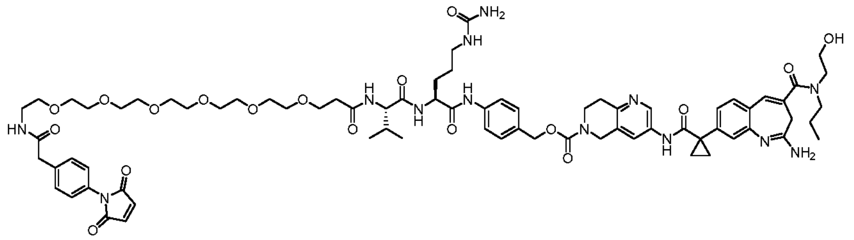 | m/z:[1/2M +H]+ 729.0 |
| II-111 | 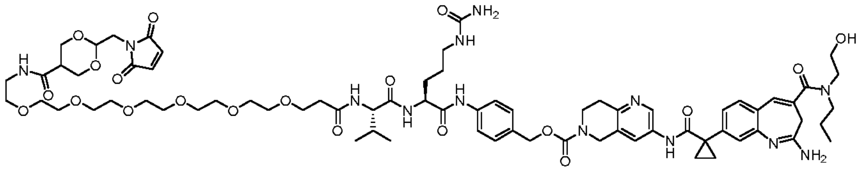 | m/z:[1/2M +H]+ 734.0 |
| II-113 | 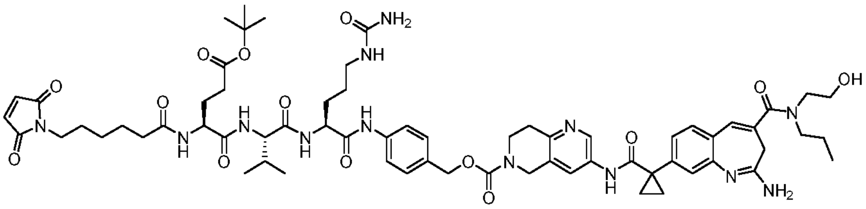 | m/z:[1/2M +H]+ 643.9 |
| II-114 | 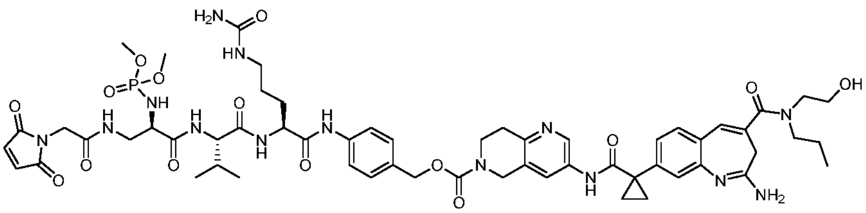 | m/z:[1/2M +H]+ 620.4 |
| II-117 | 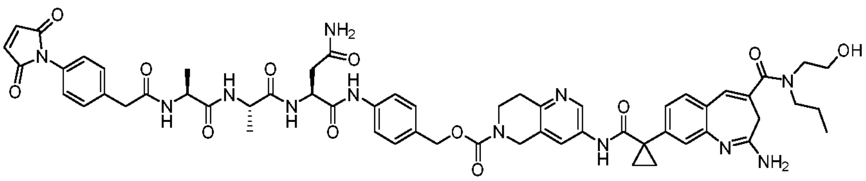 | m/z:[1/2M +H]+ 561.4 |
| II-118 | 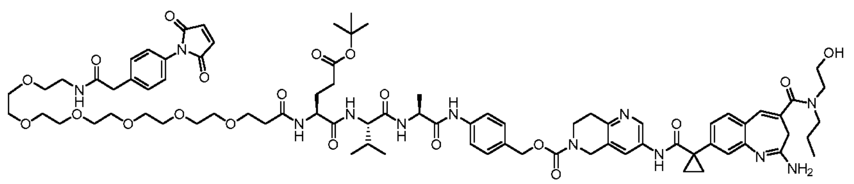 | m/z:[M+H] + 1555.6 |
| II-119 | 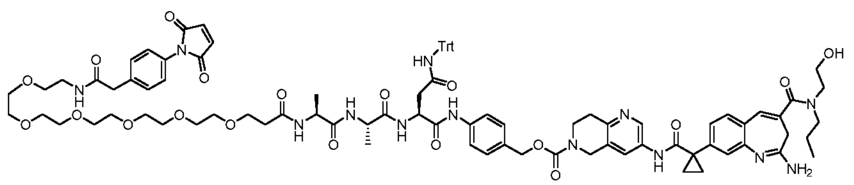 | m/z:[M+H] + 1698.6 |

(continued)

| Number | Structure | MS |
|---|---|---|
| II-120 | 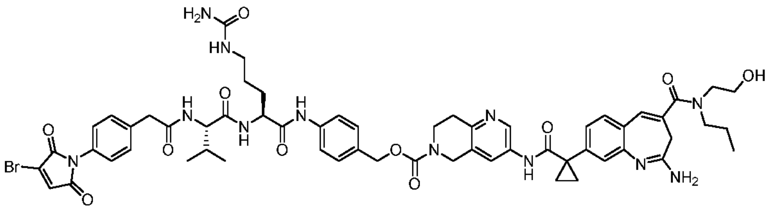 | m/z:[M+1/ 2H]+ 600.3 |

**Example 164:** Synthesis of 4-((25,85,115)-2-(4-aminobuty1)-18-(2,5-dioxo-2,5-dihydro-1H-pyrrol-1-yl)-8-(hydroxymethyl)-11-isobutyl-4,7,10,13-tetraoxa-3,6,9,12-tetraazaoctadecanoylatnino)benzyl 3-(1-(2-amino-4-((2-hydroxyethyl)(propyl)carbamoyl)-3H-benzo[b]azepin-8-yl)cyclopropane-1-formylamino)-7,8-dihydro-1,6-naphthyridine-6(5H)-carboxylate (II-109)

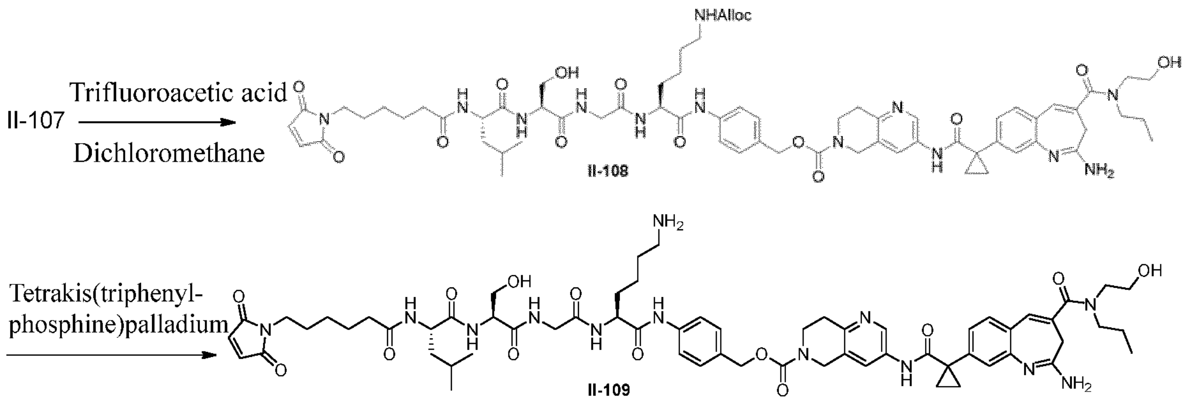


Step 1: To a solution of L-107 (21.1 mg, 0.02 mmol) in dichloromethane (3 mL) was added trifluoroacetic acid (1.5 mL) dropwise under ice bath conditions, and the reaction solution was stirred at room temperature for 1 hour. The reaction solution was concentrated under reduced pressure and then purified by Flash column chromatography (C18, eluent 0%-51% acetonitrile in a 0.05% aqueous trifluoroacetic acid solution) to give II-108 (17.6 mg, yield: 87%) as a white solid. m/z:[1/2M+H]+ 658.0.

Step 2: To a solution of II-108 (14.6 mg, 0.01 mmol) in anhydrous DMF (2 mL) were added tetrakis(triphenylphosphine)palladium (7.6 mg, 0.01 mmol), acetic acid (9.9 mg, 0.16 mmol) and tri-n-butyltin hydride (41.6 mg, 0.14 mmol) sequentially under nitrogen atmosphere, and the reaction system was replaced with nitrogen three times and stirred at room temperature for 2 hours. The reaction solution was directly purified by *prep*-HPLCC (eluent: 5%-45% acetonitrile in a 0.1% aqueous trifluoroacetic acid solution, 20 minutes) to give compound II-109 (0.80 mg, yield: 5%) as a colorless oil. m/z:[1/2M+H]+615.9.

**Example 165:** Synthesis of (S)-2-amino-8-(1-((6-(5-benzyl-14-(4-(2,5-dioxo-2,5-dihydro-1H-pyrrol-1-yl)phenyl)-4,7,10,13-tetraoxo-3,6,9,12-tetraazatetradecanoyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-112)

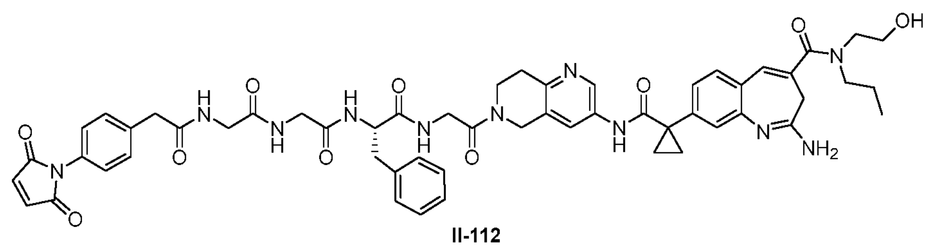

II-112

Using the synthesis method of compound II-49, compound II-112 was obtained as a white solid by reacting L-66 with compound 2. m/z: [1/2M+H]+ 517.8.

**Example 166:** Synthesis of (S)-5-(((S)-1-(((S)-1-((4-(((3-(1-(2-amino-4-((2-hydroxyethyl)(propyl)carbamoyl)-3H-benzo [b]azepin-8-yl)cyclopropane-1-formylamino)-5,6,7,8-tetrahydro-1,6-naphthyridine-6-carbonyl)oxy)methyl)phenyl)amino)-1-oxo-5-ureidopentan-2-yl)amino)-3-methyl-1-oxobutan-2-yl)amino)-4-(6-(2,5-dioxo-2,5-dihydro-1H-pyrrol-1-yl) hexanoylamino)-5-oxo n-pentanoic acid (II-115)

II-113
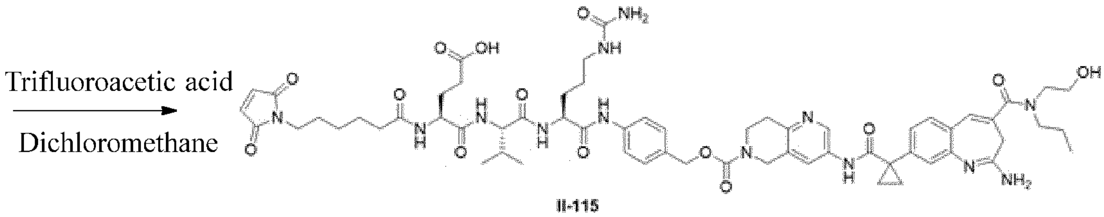

II-115

Using the synthesis method of compound II-108, compound II-115 was obtained as a white solid by reaction with II-113. m/z: [1/2M+H]+ 615.8.

**Example 167:** Synthesis of methyl(Z)-1-(3-(1-(2-amino-4-((2 hydroxyethyl)(propyl)carbamoyl)-3H-benzo[b]azepin-8-yl)cyclopropane-1-formylamino)-7,8-dihydro-1,6-naphthyridin-6(5H)-yl)-1,23-dioxo-4,7,10,13,16,19-hexaoxa-22-azahexacosa-24-en-26-oate (II-116)

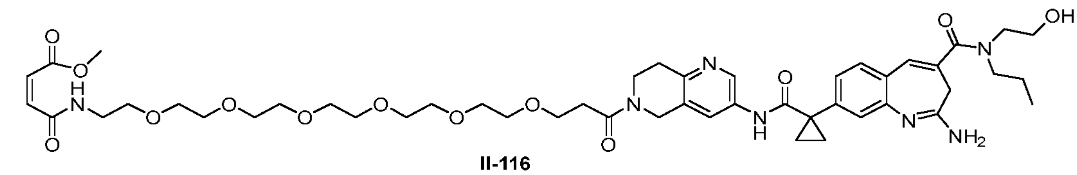
II-116

Using the synthesis method of compound II-6, compound II-116 was obtained as a white solid by reacting L-69 with compound 2.

**Example 168:** Synthesis of 2-amino-8-(1-((6-((5-((2,5-dioxo-2,5-dihydro-1H-pyrrol-1-yl)methyl)-1,3-dioxan-2-yl) methyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-121)

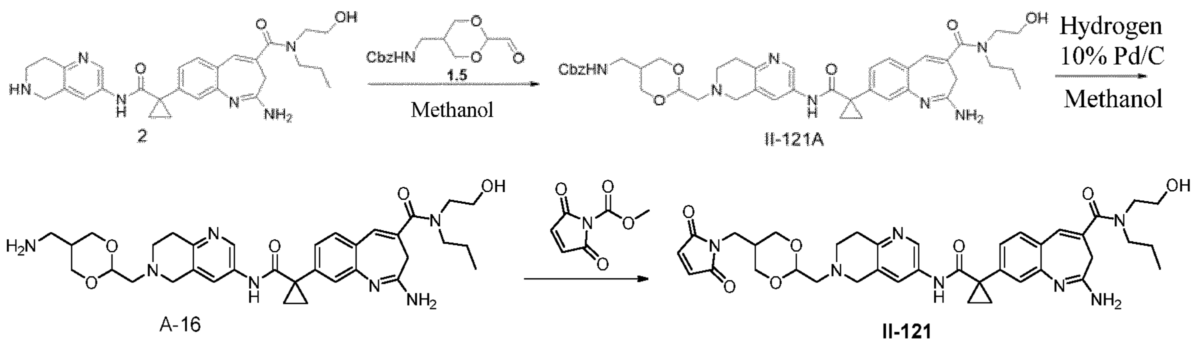

2
II-121A
A-16
II-121

Step 1: To a solution of compound 2 (40 mg, 0.08 mmol) in methanol (3 mL) was added compound 1.5 (62.2 mg, 0.22 mmol), and the reaction system was stirred at room temperature for 5 minutes. To the above reaction system was added sodium cyanoborohydride (15.1 mg, 0.24 mmol), and the resulting mixture was stirred at room temperature for 16 hours. The reaction solution was directly purified by Flash column chromatography (C18, eluent 0-50% acetonitrile in a

0.05% aqueous trifluoroacetic acid solution) to give II-121A (50.7 mg, yield: 83%) as a white solid. m/z: $[M+H]^+$766.3.

Step 2: To a solution of II-121A (50.7 mg, 0.066 mmol) in methanol (5 mL) was added palladium on carbon (21.1 mg, 10%wt), and the reaction system was replaced with hydrogen and stirred under hydrogen atmosphere for 16 hours. The reaction mixture was then filtered through diatomaceous earth and concentrated under reduced pressure to give A-16 (39.1 mg) as a white solid. m/z:$[M+H]^+$632.3.

Step 3: To a saturated aqueous sodium bicarbonate solution (2 mL) of A-16 (39.1 mg, 0.06 mmol) was added methyl 2,5-dioxo-2,5-dihydro-1H-pyrrole-1-carboxylate (14.4 mg, 0.093 mmol) under an ice methanol bath. The reaction system was stirred at room temperature for 1 hour. At -5°C, dilute hydrochloric acid (1 M) was added dropwise slowly to the reaction solution to adjust the latter to pH = 2-3. The reaction mixture was then directly purified by *prep-HPLC* (eluent 10%-40% acetonitrile in a 0.1% aqueous trifluoroacetic acid solution, 20 minutes) to give II-121 (9.01 mg, yield: 16%) as a white solid. m/z:$[M+H]^+$712.4.

**Example 169:** Synthesis of (S)-26-(((S)-1-(((S)-1-((4-(((3-(1-(2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)carbamoyl)-1-(4-(2,5-dioxo-2,5-dihydro-1H-pyrrol-1-yl)phenyl)-2,24-dioxo-6,9,12,15,18,21-hexaoxa-3,25-azanonacosan-29-oic acid (II-122)

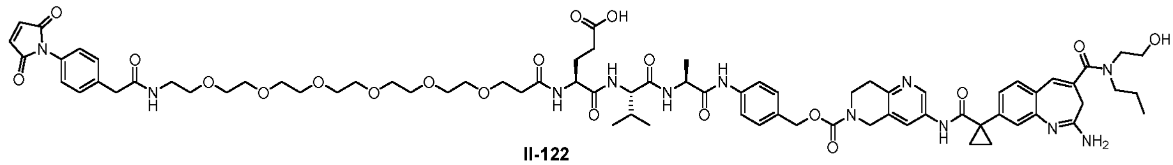

II-122

Using the synthesis method of compound II-108, compound II-125 was obtained as a white solid by reaction with II-118. m/z: $[M+H]^+$ 1499.4.

**Example 170:** Synthesis of 4-((26S,29S,32S)-32-(2-amino-2-oxoethyl)-1-(4-(2,5-dioxo-2,5-dihydro-1H-pyrrol-1-yl)phenyl)-26,29-dimethyl-2,24,27,30-tetraoxo-6,9,12,15,18,21-hexaoxa-3,25,28,31-tetraazatritriacontan-33-amido)benzyl-3-(1-(2-amino-4-((2-hydroxyethyl) (propyl)carbamoyl)-3H-benzo[b]azepin-8-yl)cyclopropane-1-formylamino)-7,8-dihydro-1,6-naphthyridine-6(5H)-formamide (II-123)

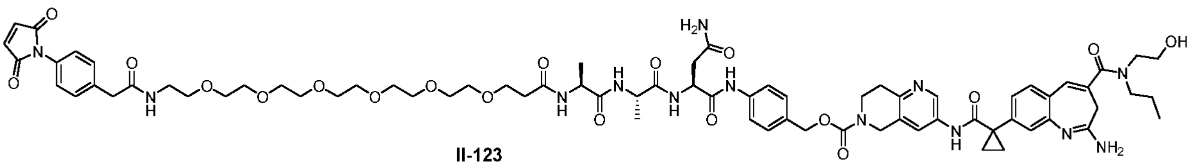

II-123

Using the synthesis method of compound II-108, compound II-126 was obtained as a white solid by reaction with II-119. m/z: $[1/2M+H]^+$ 729.0.

**Example 171:** Synthesis of (S)-5-(((S)-1-(((S)-1-((4-(((3-(1-(2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)amino)-5-oxo-4-(6-(4-(3-p-toluenesulfonyl-2-(p-toluenesulfonylmethyl)propionyl)benzoylamino)hexacarboxamido)n-pentanoic acid (II-124)

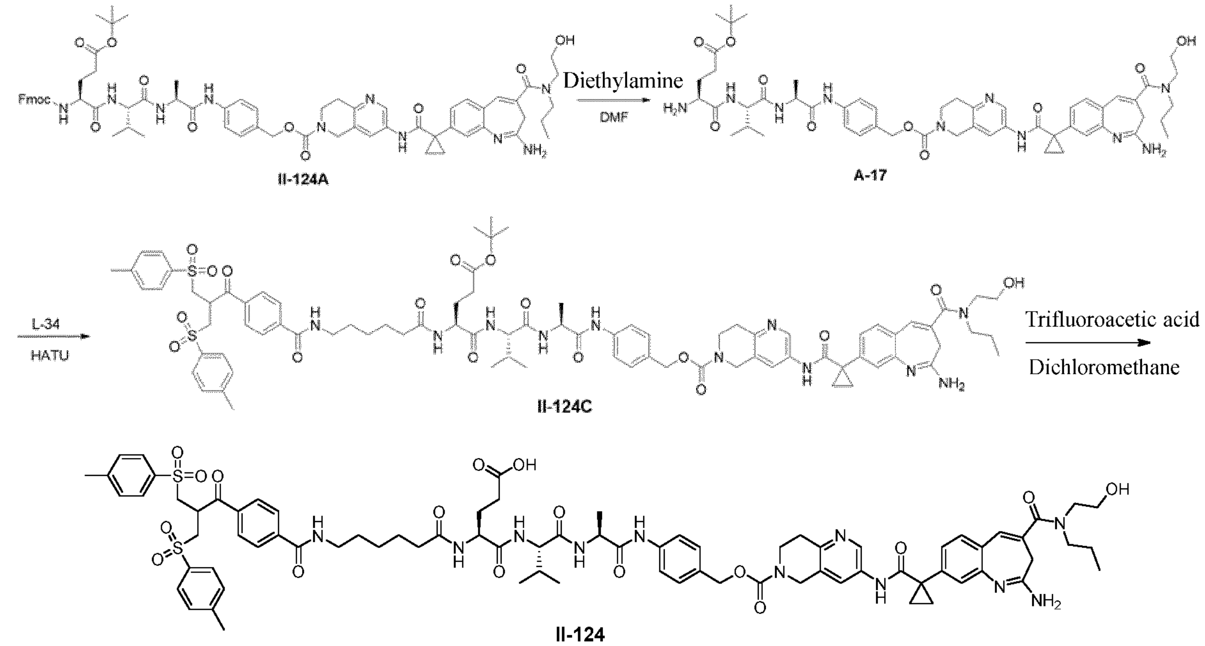


II-124A

A-17

II-124C

II-124

Step 1: To a solution of II-124A (70 mg, 0.06 mmol) in DMF (2 mL) was added diethylamine (0.4 mL), and the reaction system was stirred at room temperature for 1 hour. The reaction solution was directly purified by Flash column chromatography (C18, eluent 0%-40% acetonitrile in a 10 mM aqueous ammonium bicarbonate solution) to give A-17 (42.3 mg, yield: 74%) as a white solid. m/z: $[M+H]^+$ 1007.0.

Step 2: Using the synthesis method of compound II-6, II-124C was obtained as a white solid by reacting L-34 with A-17. m/z: $[1/2M+H]^+$ 802.0.

Step 3: Using the synthesis method of compound II-108, compound II-124 was obtained as a white solid by reaction with II-124C. m/z: m/z:$[1/2M+H]^+$ 774.0.

**Examples 172-180:** Using the synthetic procedure of II-6, the compounds listed in Table 9 were synthesized from compound A-9:

Table 9

| Number | Structure | MS |
|---|---|---|
| II-125 | 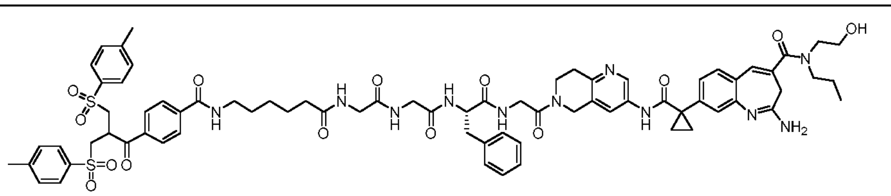 | m/z:$[1/2M+ H]^+$ 708.8 |
| II-126 | 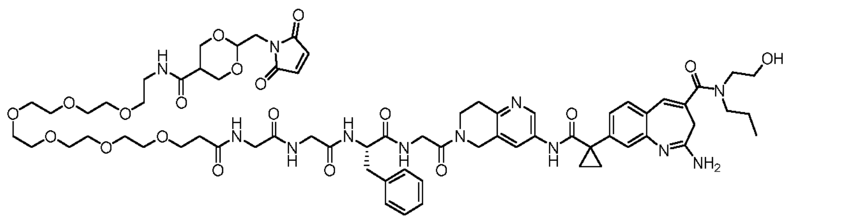 | m/z:$[1/2M+ H]^+$ 690.4 |
| II-127 | 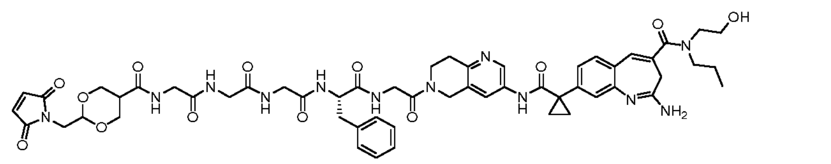 | m/z:$[1/2M+ H]^+$ 551.3 |

(continued)

| Number | Structure | MS |
|---|---|---|
| II-128 | 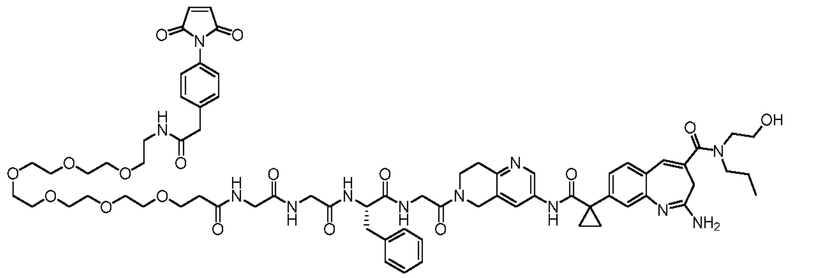 | m/z:[1/2M+ H]+ 685.4 |
| II-129 | 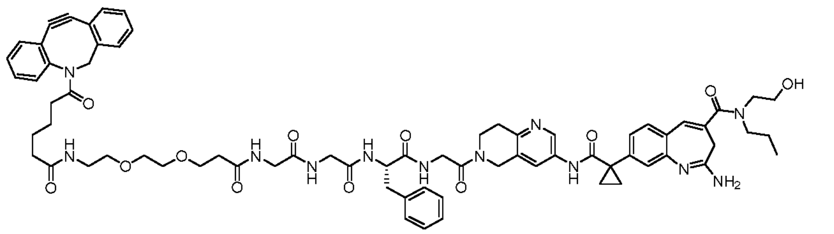 | m/z:[1/2M+ H]+ 648.4 |
| II-130 | 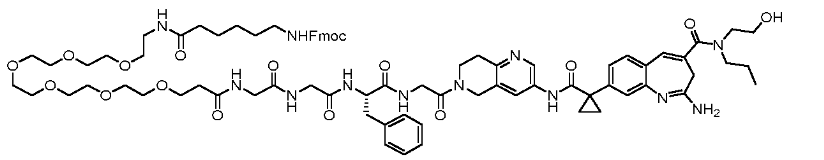 | m/z:[1/2M+ H]+ 746.4 |
| II-131 | 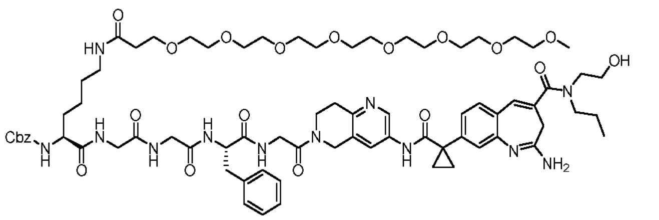 | m/z:[1/2M+ H]+ 739.5 |
| II-132 | 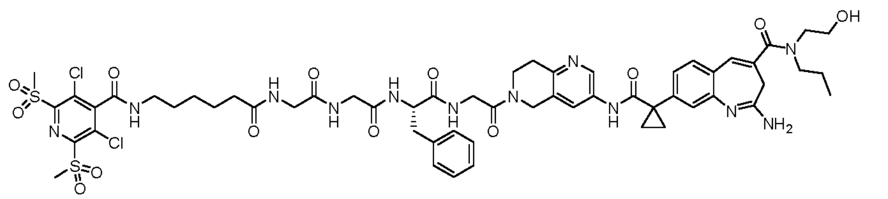 | m/z:[1/2M+ H]+ 632.4 |
| II-133 | 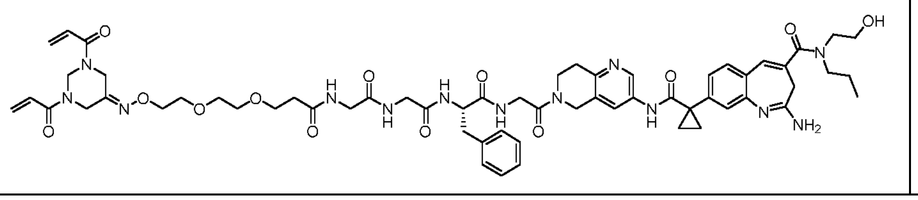 | m/z:[1/2M+ H]+ 594.0 |

**Example 181:** Synthesis of (S)-2-amino-8-(1-((6-(5-benzyl-43-(4-(2,5-dioxo-2,5-dihydro-1H-pyrrol-1-yl)phenyl)-4,7,10,13,35,42-hexaoxo-16,19,22,25,28,31-hexaoxa-3,6,9,12,34,41-hexaazattritetracontanacyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-134)

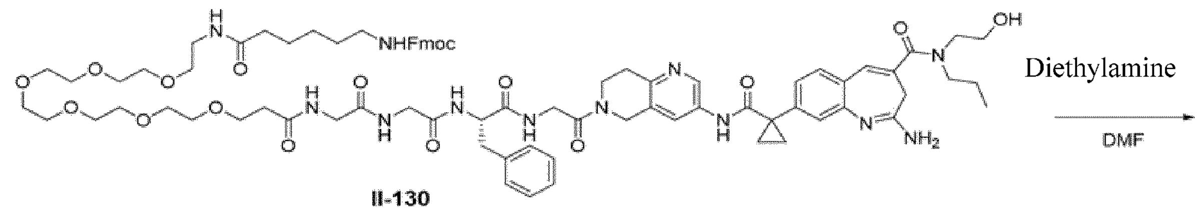


II-130

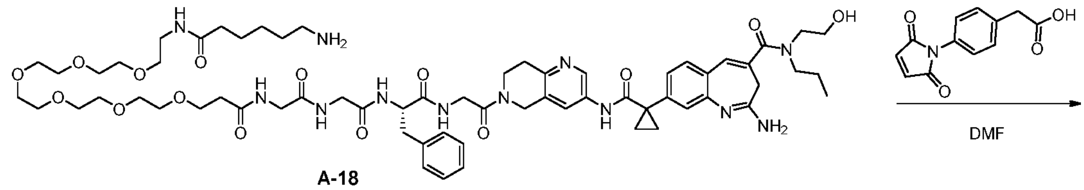


A-18

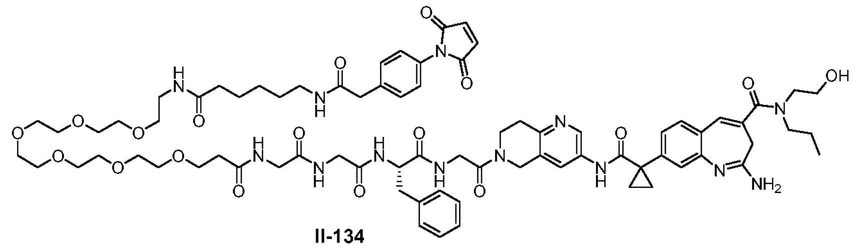

II-134

Step 1: To a solution of II-130 (90 mg, 0.06 mmol) in DMF (4 mL) was added diethylamine (26 mg, 0.36 mmol). The reaction mixture was stirred at room temperature for 1 h and then directly purified by Flash column chromatography (C18, 0-55% acetonitrile in a 10 mM aqueous ammonia bicarbonate solution) to give A-18 (21 mg, yield: 25%) as a white solid. m/z: $[M+H]^+$ 1270.2.

Step 2: To a solution of A-18 (2 mg, 8.7 μmol) and 2-(4-(2,5-dioxo-2,5-dihydro-1H-pyrrol-1-yl)phenyl)acetic acid (2 mg, 8.7 μmol) in DMF (2 mL) were added EDCI (3.3 mg, 17 μM) and HOBT (2.4 mg, 17 μmol) under ice bath conditions. The reaction was stirred at 0°C for 2 hours and then directly purified by *prep-HPLC* (eluent 0-55% acetonitrile in a 10 mM aqueous ammonia bicarbonate solution, 25 minutes) to give II-134 (2.8 mg, yield: 21%) as a white solid. m/z: $[1/2M+H]^+$ 742.0.

**Example 182:** Synthesis of 2-amino-8-(1-((6-((32S,41S)-41-benzyl-32-(2-(4-(2,5-dioxo-2,5-dihydro-1H-pyrrol-1-yl)phenyl)acetylamino)-26,33,36,39,42-pentaoxo-2,5,8,11,14,17,20,23-octaoxa-27,34,37,40,43-pentaazapentatetracontan-45-acyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-136)

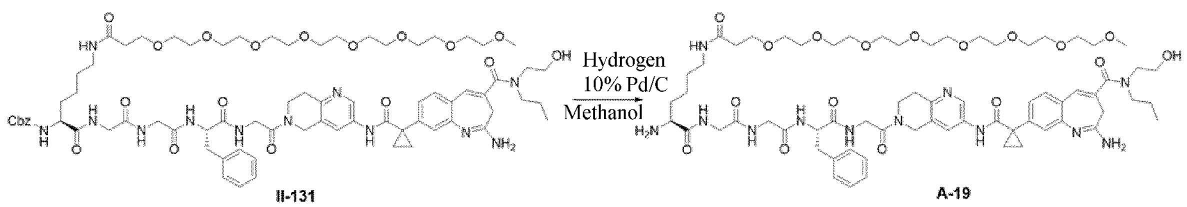


II-131

A-19

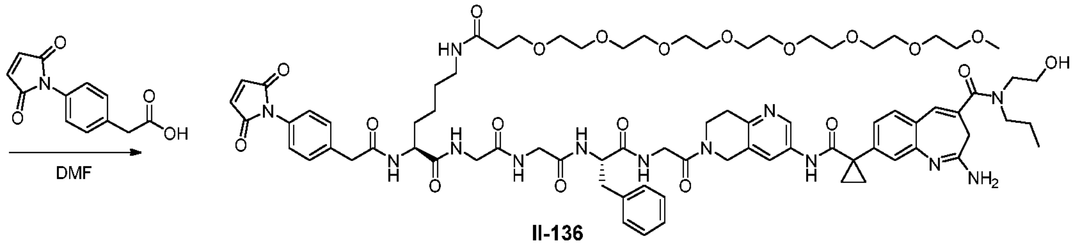


II-136

Step 1: To a solution of II-131 (25 mg, 0.017 mmol) in methanol (5 mL) was added palladium on carbon (5.4 mg, 10%wt), and the reaction system was stirred under hydrogen atmosphere for 4 h. The reaction mixture was then filtered

through diatomaceous earth, and the filtrate was concentrated under reduced pressure to give A-19 (13.5 mg, yield: 59%) as a white solid. m/z:[1/2M+H]$^+$672.5.

Step 2: To a solution of A-19 (13.5 mg, 0.010 mmol) and 2-(4-(2,5-dioxo-2,5-dihydro-1H-pyrrol-1-yl)phenyl)acetic acid (2.3 mg, 0.01 mmol) in DMF (2 mL) were added HATU (4.6 mg, 0.012 mmol) and DIPEA (1.9 mg, 0.015 mmol) under ice bath conditions, and the reaction system was stirred at 0°C for 1 hour. The reaction solution was directly purified by *prep-HPLC* (eluent 10%-45% acetonitrile in a 0.1% aqueous trifluoroacetic acid solution, 20 minutes) to give II-136 (3 mg, yield: 17%) as a white solid. m/z:[1/2M+H]$^+$ 779.0.

**Example 183:** Synthesis of 2-amino-8-(1-((6-((32S,35S,38S)-32-(2-(4-(2,5-dioxo-2,5-dihydro-1H-pyrrol-1-yl)phenyl) acetylamino)-35-isopropyl-38-methyl-26,33,36,39,42-pentaoxo-2,5,8,11,14,17,20,23,45-nonaoxa-27,34,37,40,43-pentaazaheptateteracontan-47-acyl)-5,6,7,8-tetrahydro-1,6-naphthyridin-3-yl)carbamoyl)cyclopropyl)-N-(2-hydroxyethyl))-N-propyl-3H-benzo[b]azepine-4-formamide (II-137)

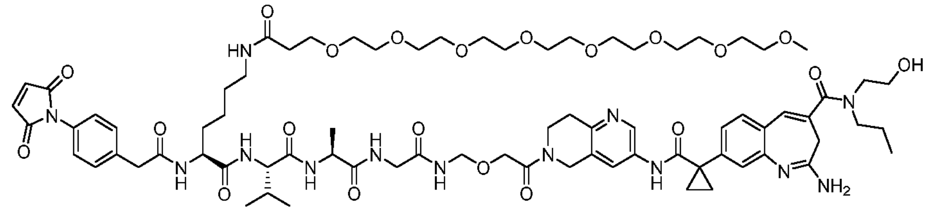

II-137

Using the synthesis method of compound II-6, compound II-137 was obtained as a white solid by reacting L-76 with compound 2. m/z: [M+H]$^+$ 777.0.

**Example 184:** 2-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-2-oxoethyl((32S,38S)-38-benzyl-32-(2-(4-(2,5-dioxo-2,5-dihydro-1H-pyrrol-1-yl)phenyl)acetamido)-26,33,36,39,42,45-hexaoxo-2,5,8,11,14,17,20,23-octaoxa-27,34,37,40,43,46-hexaazaoctatetracontan-48-yl)(2-(methylsulfonyl)ethyl)carbamate (II-138)

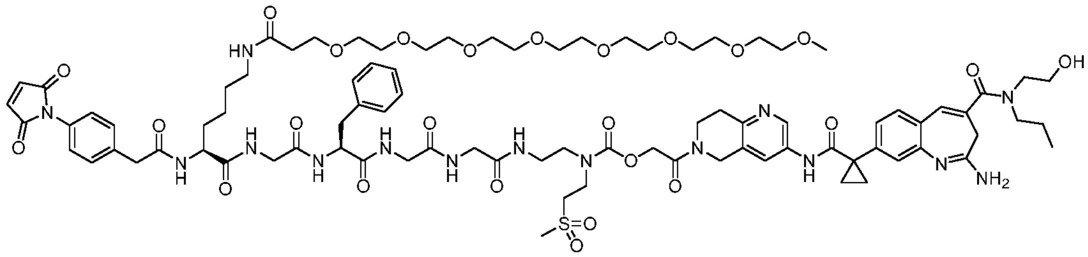

II-138

Using the synthesis method in step 2 of compound II-136, II-138 was obtained as a white solid by reaction with compound A-11. m/z: [1/2M+H]$^+$ 904.0.

**Example 185:** Synthesis of 2-amino-N-(2-hydroxyethyl)-N-propyl-8-(1-((6-(2-(4-(3-p-methylbenzenesulfonyl-2-(p-methylbenzenesulfonylmethyl)propionyl)phenyl)-1,3-dioxan-5-carbonyl)-5,6,7,8-tetrahydro-1,6-naphthyridin-3-yl)carbamoyl)cyclopropyl)-3H-benzo[b]azepine-4-formamide (II-139)

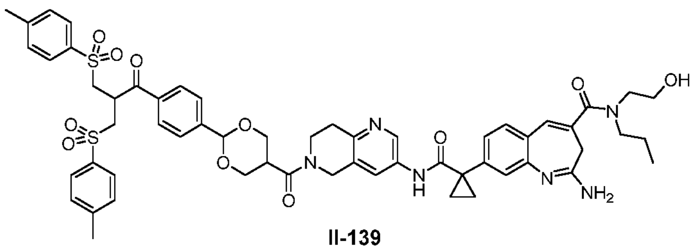

II-139

Using the synthesis method of compound II-6, compound II-139 was obtained as a white solid by reacting L-77 with compound 2. m/z: $[M+H]^+$ 1071.5.

**Example 186:** Synthesis of 2-amino-8-(1-((6-(2-(4-(2,5-dioxo-2,5-dihydro-1H-pyrrol-1-yl)phenyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-140)

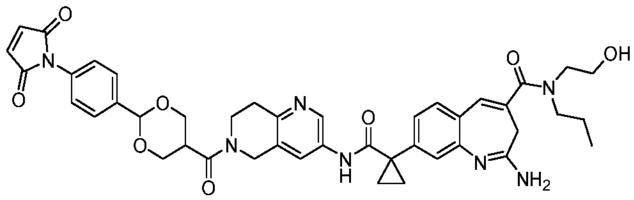

II-140

Using the synthesis method of compound II-6, compound II-140 was obtained as a white solid by reacting L-75 with compound 2. m/z: $[M+H]^+$ 788.4.

**Example 187:** Synthesis of (S)-5-(((S)-1-(((S)-1-((4-(((3-(1-(2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)amino)-4-(6-(2,5-dioxo-2,5-dihydro-1H-pyrrol-1-yl)hexanoylamino)-5-oxopentanoic acid (II-141)

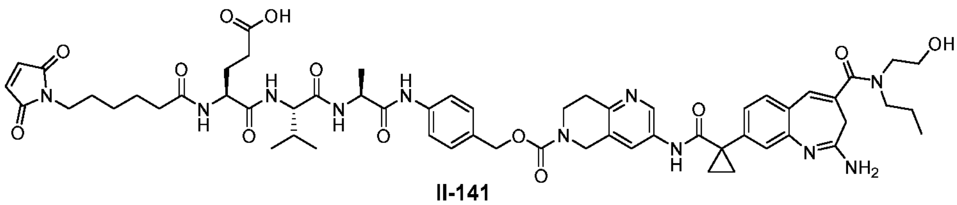

II-141

Using the synthesis method of compound II-124, compound II-141 was obtained as a white solid by reacting A-17 with 6-(2,5-dioxo-2,5-dihydro-1H-pyrrol-1-yl)hexanoic acid. m/z: $[1/2M+H]^+$ 572.8.

**Example 188:** Synthesis of (S)-5-(((S)-1-(((S)-1-((4-(((3-(1-(4-((2-hydroxyethyl)(propyl)carbamoyl)-2-((((1-methyl-2-nitro-1H-imidazol-5-yl)methoxy)carbonyl)amino)-3H-benzo[b]azepin-8-yl)cyclopropane-1-formylatnino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)amino)-5-oxo-4-(6-(4-(3-p-toluenesulfonyl-2-(p-toluenesulfonylmethyl)propionyl)benzoylamino)hexacarboxamido)n-pentanoic acid (II-142)

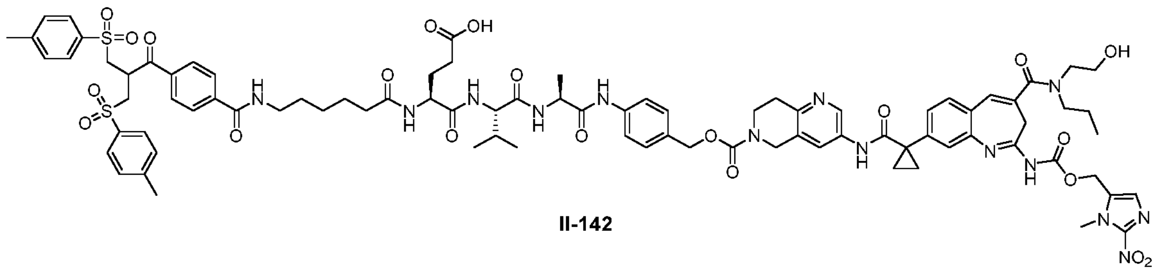

II-142

Using the synthesis method of compound II-42, compound II-142 was obtained as a white solid by reaction with II-124. m/z: $[1/2M+H]^+$ 865.4.

**Example 189:** Synthesis of 2,5-dioxopyrrolidin-1-yl(S)-1-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-5-benzyl-1,4,7,10,13-pentaoxo-3,6,9,12-tetraazahexadecan-16-oate (II-143)

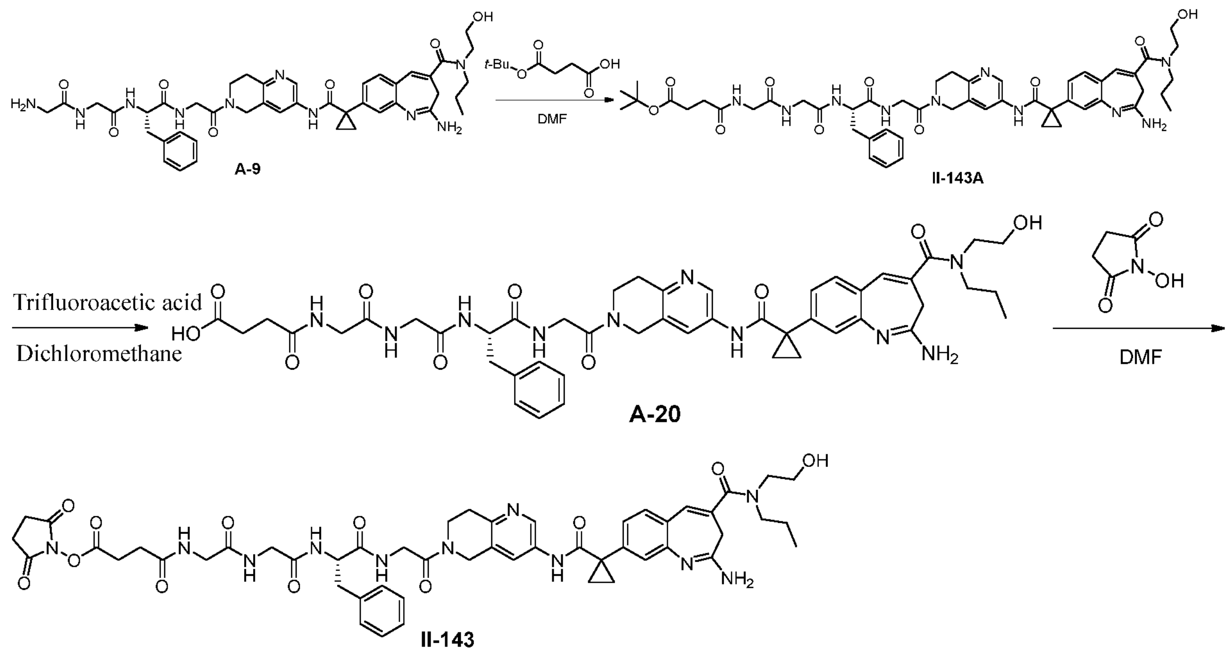


A-9, II-143A, A-20, II-143

Step 1: To a solution of compound A-9 (55 mg, 0.067 mmol) and mono-tert-butyl succinate (11.7 mg, 0.067 mmol) in DMF (2 mL) were added HATU (30.6 mg, 0.080 mmol) and DIPEA (13 mg, 0.1 mmol) under ice bath conditions, and the reaction system was stirred at room temperature for 1 hour. The reaction solution was directly purified by Flash column chromatography (C18, eluent 0% to 41% acetonitrile in a 0.05% aqueous trifluoroacetic acid solution) to give compound II-143A (33.0 mg, yield: 50%) as a white solid. m/z:$[M+H]^+$ 977.5.

Step 2: To a solution of compound II-143A (33.0 mg, 0.034 mmol) in dichloromethane (2 mL) was added trifluoroacetic acid (0.5 mL) dropwise under ice bath conditions, and the reaction system was stirred at room temperature for 1 hour. The reaction solution was directly purified by Flash column chromatography (C18, eluent 0%-35% acetonitrile in a 0.05% aqueous trifluoroacetic acid solution) to give compound A-20 (26 mg, yield: 84%) as a white solid. m/z:$[M+H]^+$ 921.4.

Step 3: To a solution of compound A-20 (26 mg, 0.028 mmol) in DMF (2 mL) were added *N*-hydroxysuccinimide (9.7 mg, 0.084 mmol) and EDCI (16.1 mg, 0.084 mmol), and the reaction system was stirred at room temperature for 3 hours. The reaction solution was directly purified by *prep-HPLC* (eluent 5%-45% acetonitrile in a 0.1% aqueous trifluoroacetic acid solution, 20 minutes) to give compound II-143 (5.15 mg, yield: 14%) as a white solid. m/z:$[1/2M+H]^+$ 509.8.

**Example 190:** Synthesis of 2,3,5,6-tetrafluorophenyl 4-(((5-(3-(1-(2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)-1,3-dioxan-2-yl)methyl)amino)-4-oxobutanoate (II-144)

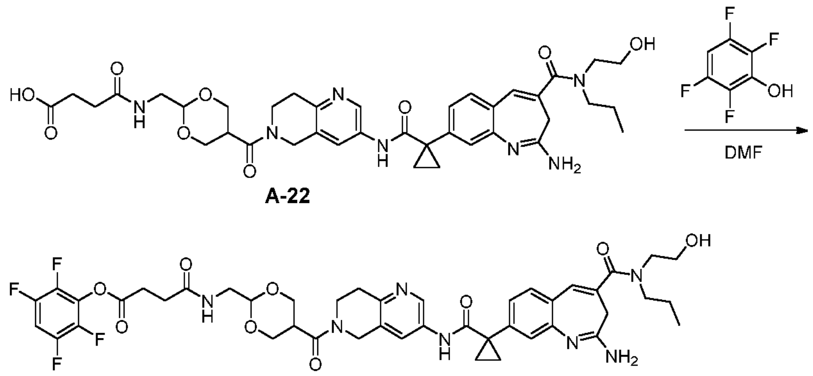


A-22

II-144

Using the synthesis method of compound A-20, compound A-22 was obtained as a white solid by reacting compound A-12 with mono-tert-butyl succinate as starting materials. m/z: $[M+H]^+$ 746.2.

To a solution of compound A-22 (10 mg, 0.013 mmol) in DMF (1 mL) were added 2,3,5,6-tetrafluorophenol (11 mg, 0.065 mmol), EDCI (25 mg, 0.13 mmol) and DMAP (3.2 mg, 0.026 mmol), and the reaction system was stirred at room temperature for 2 hours. The reaction solution was directly purified by *prep-HPLC* (eluent 10%-70% acetonitrile in a 0.1% aqueous trifluoroacetic acid solution, 20 minutes) to give compound II-144 (4.96 mg, yield: 41%) as a white solid. m/z: $[M+H]^+$ 894.4.

**Example 191:** Synthesis of pentafluorophenyl 4-(((5-(3-(1-(2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)-1,3-dioxan-2-yl)methyl)amino)-4-oxobutanoate (II-145)

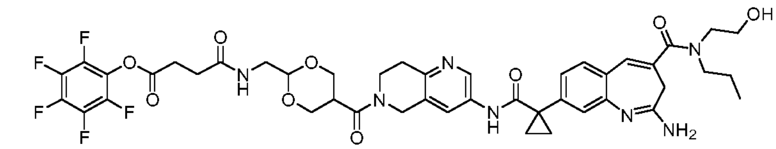

II-145

Using the synthesis method of compound II-144, compound II-145 was obtained as a white solid by reacting compound A-22 with 2,3,4,5,6-pentafluorophenol. m/z: $[M+H]^+$ 912.3.

**Example 192:** Synthesis of (S)-25-(((S)-1-(((S)-1-((4-(((3-(1-(2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)carbamoyl)-1,23-dioxo-1-(4-(3-p-toluenesulfonyl-2-(p-toluenesulfonylmethyl)propionyl)phenyl)-5,8,11,14,17,20-hexaoxa-2,24-diazaoctacosan-28-oic acid (II-146)

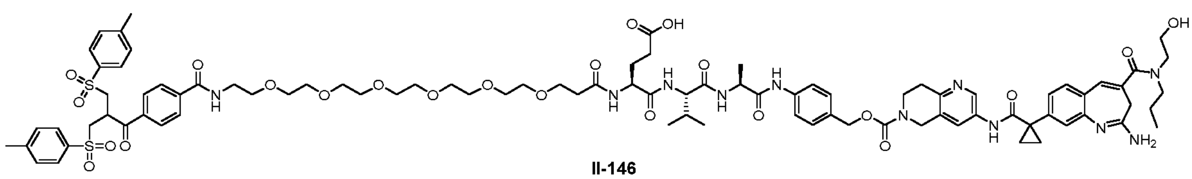

II-146

Using the synthesis method of compound II-124, compound II-146 was obtained as a white solid by reacting A-17

with L-81. m/z: [1/2M+H]+ 885.0.

**Example 193:** Synthesis of (S)-25-(((S)-1-(((S)-1-((4-(((3-(1-(4-((2-hydroxyethyl)(propyl)carbamoyl)-2-((((1-methyl-2-nitro-1H-imidazol-5-yl)methoxy)carbonyl)amino)-3H-benzo[b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)carbamoyl)-1,23-dioxo-1-(4-(3-p-toluenesulfonyl-2-(p-toluenesulfonylmethyl)propionyl)phenyl)-5,8,11,14,17,20-hexaoxa-2,24-diazaoctacosan-28-oic acid (II-147)

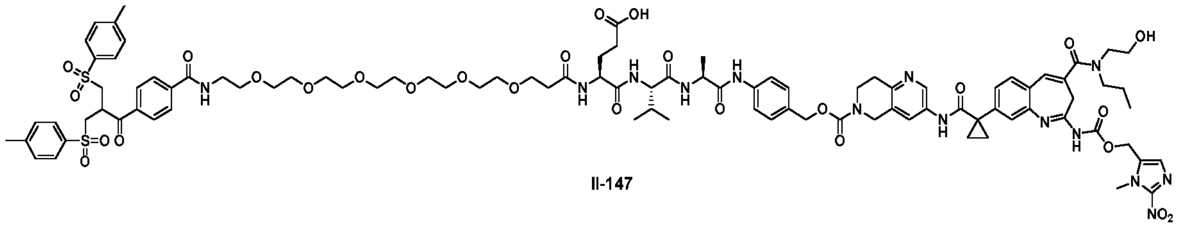

II-147

Using the synthesis method of compound II-42, compound II-147 was obtained as a white solid by reaction with II-146. m/z: [1/2M+H]+ 976.8.

**Example 194:** Synthesis of (1-methyl-2-nitro-1H-imidazol-5-yl)methyl(8-(1-((6-(2-((2,5-dioxo-2,5-dihydro-1H-pyrrol-1-yl)methyl)-1,3-dioxan-5-carbonyl)-5,6,7,8-tetrahydro-1,6-naphthyridin-3-yl)carbamoyl)cyclopropyl)-4-((2-hydroxyethyl)(propyl)carbamoyl)-3H-benzo[b]azepin-2-yl)carbamate (II-148)

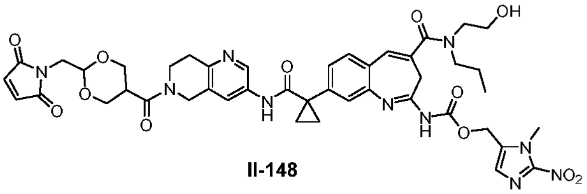

II-148

Using the synthesis method of compound II-42, compound II-148 was obtained as a white solid by reaction with II-77. UPLC retention time 5.690 minutes; m/z: [M+H]+909.4.

**Example 195:** Synthesis of 2-amino-8-(1-((6-(2-((2-bromoacetylamino)methyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-149)

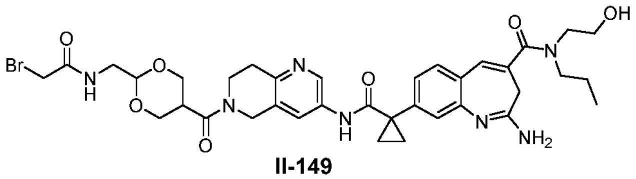

II-149

To a solution of compound A-12 (20 mg, 0.03 mmol) in DMF (1 mL) were added triethylamine (4.7 mg, 0.05 mmol) and 2-bromoacetyl bromide (5.6 mg, 0.03 mmol) under ice bath conditions. The reaction solution was stirred at room temperature for 1 hour and then directly purified by *prep-HPLC* (eluent 10%-30% acetonitrile in a 0.1% aqueous trifluoroacetic acid solution, 20 minutes) to give compound II-149 (3.12 mg, yield: 94%) as a white solid. m/z: [M+H]+ 766.2.

**Example 196:** Synthesis of 2-amino-N-(2-hydroxyethyl)-8-(1-((6-(2-((2-(methanesulfonyl)pyrimidine-5-formylamino) methyl)-1,3-dioxan-5-carbonyl)-5,6,7,8-tetrahydro-1,6-naphthyridin-3-yl)carbamoyl)cyclopropyl)-N-propyl-3H-benzo[b] azepine-4-formamide (II-150)

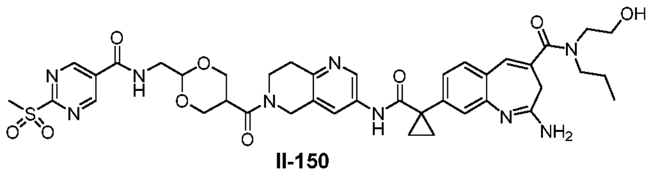

**II-150**

Using the synthesis method in step 2 of compound II-136, compound II-150 was obtained as a white solid by reacting compound A-12 with 2-(methanesulfonyl)pyrimidine-5-carboxylic acid as starting materials. m/z: $[M+H]^+$ 830.3.

**Example 197:** Synthesis of (S)-5-(((S)-1-(((S)-1-((4-(((3-(1-(2-amino-4-((2-hydroxyethyl)(propyl)carbamoyl)-3H-benzo [b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)amino)-4-(6-(2-(methanesulfonyl)pyrimidine-5-formylamino) hexanoylamino)-5-oxopentanoic acid (II-151)

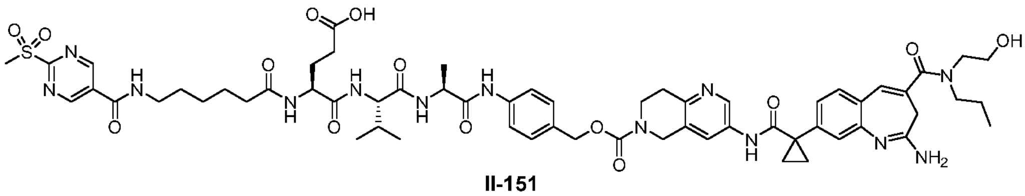

**II-151**

Using the synthesis method of compound II-124, compound II-151 was obtained as a white solid by replacing L-34 in step 2 with L-84. m/z: $[M+H]^+$ 1248.6.

**Example 198:** Synthesis of (S)-5-(((S)-1-(((S)-1-((4-(((3-(1-(2-amino-4-((2-hydroxyethyl)(propyl)carbamoyl)-3H-benzo [b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)amino)-4-(4-(bis(2-(2,5-dioxo-2,5-dihydro-1H-pyrrol-1-yl)ethyl) amino)-4-oxobutanamido)-5-oxo-n-pentanoic acid (II-152)

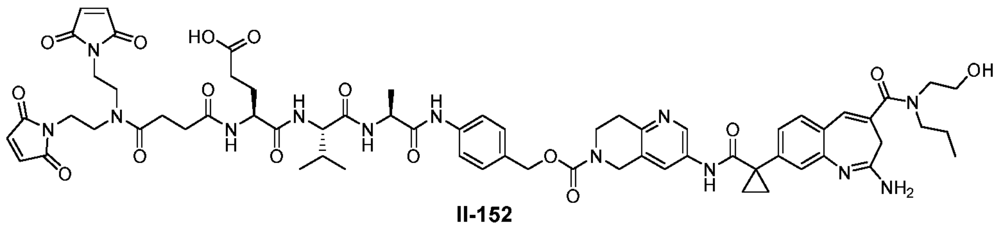

**II-152**

Using the synthesis method of compound II-124, compound II-152 was obtained as a white solid by replacing L-34 in step 2 with 4-(bis(2-(2,5-dioxo-2,5-dihydro-1H-pyrrol-1-yl)ethyl)amino)-4-oxobutanoic acid (obtained according to the synthesis method of US10960082B2 Compound 6). m/z: $[1/2M+H]^+$ 649.0.

**Example 199:** Synthesis of (S)-5-(((S)-1-(((S)-1-((2-(3-(1-(2-amino-4-((2-hydroxyethyl)(propyl)carbamoyl)-3H-benzo [b]azepin-8-yl)cyclopropane-1-formylamino)-7,8-dihydro-1,6-naphthyridin-6(5H)-yl)-2-oxoethyl)amino)-1-oxopropan-2-yl)amino)-3-methyl-1-oxobutan-2-yl)amino)-4-(6-(2,5-dioxo-2,5-dihydro-1H-pyrrol-1-yl)hexanoylamino)-5-oxo-n-pentanoic acid (II-153)

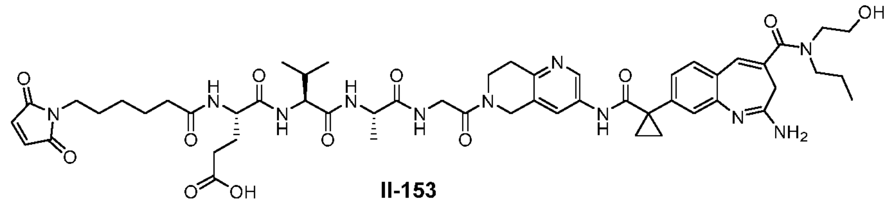

II-153

Using the synthesis method of compound II-124, compound II-153 was obtained as a white solid by reacting A-23 with 6-(2,5-dioxo-2,5-dihydro-1H-pyrrol-1-yl)hexanoic acid. m/z: $[1/2M+H]^+$ 526.8.

**Example 200:** Synthesis of (S)-5-(((S)-1-(((S)-1-((2-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-2-oxoethyl)amino)-1-oxopropan-2-yl)amino)-3-methyl-1-oxobutan-2-yl)amino)-5-oxo-4-(6-(4-(3-p-toluenesulfonyl-2-(p-toluenesulfonylmethyl)propionyl)benzoylamino)hexanoylamino)n-pentanoic acid (II-154)

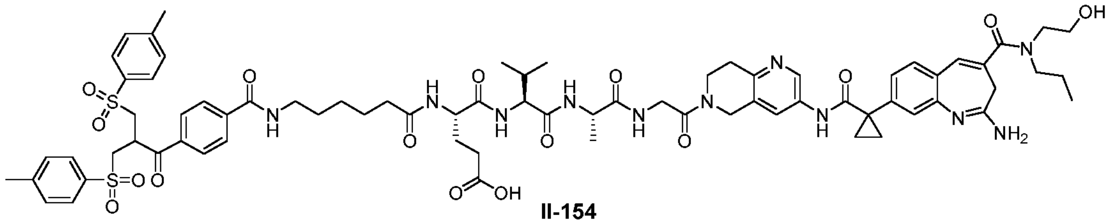

II-154

Using the synthesis method of compound II-124, compound II-154 was obtained as a white solid by reacting A-23 with L-34. m/z: $[1/2M+H]^+$ 728.1.

**Example 201:** Synthesis of (S)-5-(((S)-1-(((S)-1-((2-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-2-oxoethyl)amino)-1-oxopropan-2-yl)amino)-3-methyl-1-oxobutan-2-yl)amino)-4-(6-(2-(methanesulfonyl)pyrimidine-5-formylamino)hexanoylamino)-5-oxo-n-pentanoic acid (II-155)

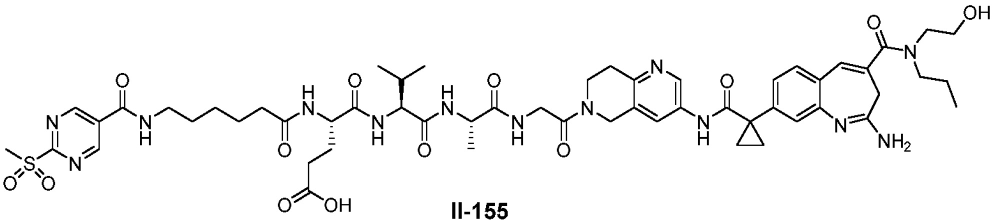

II-155

Using the synthesis method of compound II-124, compound II-155 was obtained as a white solid by reacting A-23 with L-84. m/z: $[1/2M+H]^+$ 578.8.

**Example 202:** Synthesis of (S)-5-(((S)-1-(((S)-1-((4-(((3-(1-(2-amino-4-((2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)amino)-4-(4-(bis(2-(3-bromo-2,5-dioxo-2,5-dihydro-1H-pyrrol-1-yl)ethyl)amino)-4-oxobutanoylamino)-5-oxo-n-pentanoic acid (II-156)

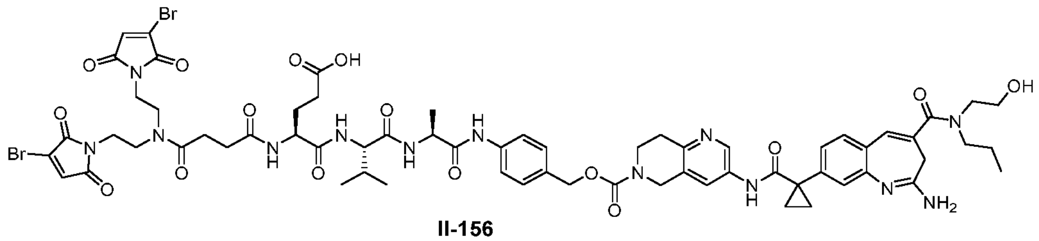

**II-156**

Using the synthesis method of compound II-124, compound II-152 was obtained as a white solid by replacing L-34 in step 2 with 4-(bis(2-(2,5-dioxo-2,5-dihydro-1H-pyrrol-1-yl)ethyl)amino)-4-oxobutanoic acid (obtained according to the synthesis method of CN111433188B compound A'-7). m/z: $[1/2M+H]^+$ 727.0.

**Example 203:** Synthesis of 2-amino-8-(1-((6-(2-((3-((2-(2,5-dioxo-2,5-dihydro-1H-pyrrol-1-yl)ethyl)(methyl)amino)propionylamino)methyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-157)

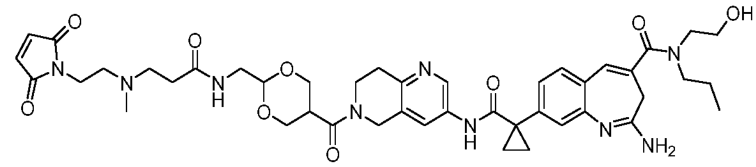

**II-157**

Using the synthesis method in step 2 of compound II-136, compound II-157 was obtained as a white solid by reacting compounds A-12 and L-85 as starting materials. m/z: $[M+H]^+$ 854.6.

**Example 204:** Synthesis of (S)-2-amino-8-(1-((6-(2-((3-amino-2-(2,5-dioxo-2,5-dihydro-1H-pyrrol-1-yl)propionylamino)methyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-158)

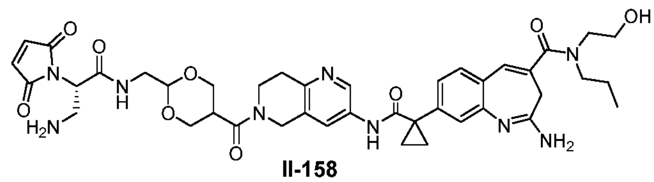

**II-158**

Using the synthesis method in steps 2 & 3 of compound II-124, compound II-158 was obtained as a white solid by reacting compound A-12 with (S)-3-((tert-butoxycarbonyl)amino)-2-(2,5-dioxo-2,5-dihydro-1H-pyrrol-1-yl)propionic acid as starting materials. m/z: $[M+H]^+$ 812.3.

**Example 205:** Synthesis of (8R,11S,14S)-14-((2-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-2-oxoethyl)carbamoyl)-1-(2,5-dioxo-2,5-dihydro-1H-pyrrol-1-yl)-8-isobutyl-11-methyl-6,9,12-trioxo-3-oxa-7,10,13-triazaheptadecan-17-oic acid (II-159)

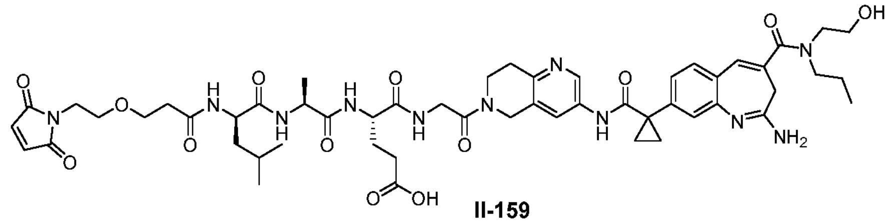

II-159

Using the synthesis method of compound II-124, compound II-159 was obtained as a white solid by reacting A-27 with 3-(2-(2,5-dioxo-2,5-dihydro-1H-pyrrol-1-yl)ethoxy)propionic acid. m/z: $[1/2M+H]^+$ 534.8.

**Example 206:** Synthesis of (S)-5-(((S)-1-(((S)-1-((2-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-2-oxoethyl)amino)-1-oxopropan-2-yl)amino)-3-methyl-1-oxobutan-2-yl)amino)-4-(3-(2-(2,5-dioxo-2,5-dihydro-1H-pyrrol-1-yl)ethoxy)propionylatnino)-5-oxo-n-pentanoic acid (II-160)

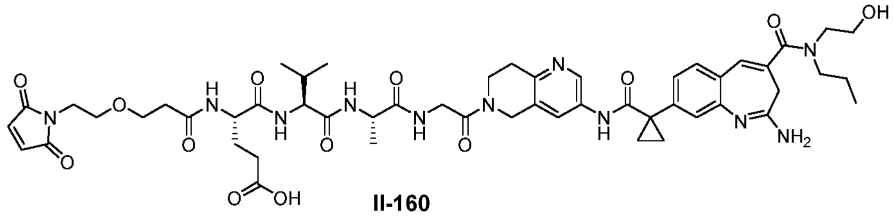

II-160

Using the synthesis method of compound II-124, compound II-160 was obtained as a white solid by reacting A-23 with 3-(2-(2,5-dioxo-2,5-dihydro-1H-pyrrol-1-yl)ethoxy)propionic acid. m/z: $[1/2M+H]^+$ 527.8.

**Example 207:** Synthesis of (S)-5-(((S)-1-(((S)-1-((2-(3-(1-(2-amino-4-((2-hydroxyethyl)(propyl)carbamoyl)-3H-benzo[b]azepin-8-yl)cyclopropane-1-formylamino)-7,8-dihydro-1,6-naphthyridin-6(5H)-yl)-2-oxoethyl)amino)-1-oxopropan-2-yl)amino)-3-methyl-1-oxobutan-2-yl)amino)-5-oxo-4-(6-(4-(3-p-toluenesulfonyl-2-(p-toluenesulfonylmethyl)propionyl)benzoylamino)ethoxy)propionylamino)n-pentanoic acid (II-161)

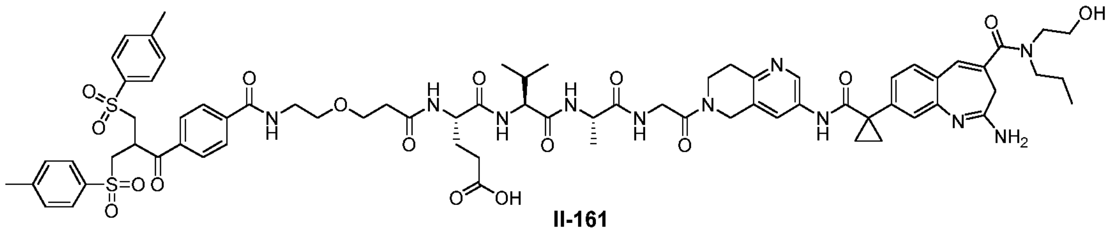

II-161

Using the synthesis method of compound II-124, compound II-161 was obtained as a white solid by reacting A-23 with L-87. m/z: $[1/2M+H]^+$ 728.8.

**Example 208:** Synthesis of 2-amino-8-(1-((6-(2-((3-bromo-2,5-dioxo-2,5-dihydro-1H-pyrrol-1-yl)methyl)-1,3-dioxan-5-carbonyl)-5,6,7,8-tetrahydro-1,6-naphthyridin-3-yl)carbamoyl)cyclopropyl)-N-(2-hydroxyethyl)-N-propyl-3H-benzo[b]azepine-4-formamide (II-162)

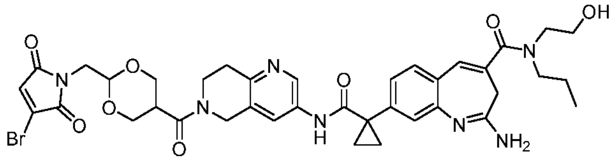

II-162

To a solution of L-86 (17 mg, 0.05 mmol) and triethylamine (19 mg, 0.19 mmol) in DMF (2 mL) was added 1-propylphosphoric anhydride (84 mg, 0.13 mmol) under ice bath conditions. The reaction solution was stirred at 0°C for 1 hour, and compound 2 (24 mg, 0.05 mmol) was added. The reaction solution was continued to be stirred at 0°C for 2 hours and then directly purified by *prep-HPLC* (eluent 10%-50% acetonitrile in a 0.1% aqueous trifluoroacetic acid solution, 20 minutes) to give II-162 (2.3 mg, yield: 96%) as a white solid. m/z:$[1/2M+H]^+$ 804.2.

**Example 209:** Synthesis of S-(1-(2-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)ethyl)-2,5-dioxopyrrolidin-3-yl)-L-cysteine (compound B-1)

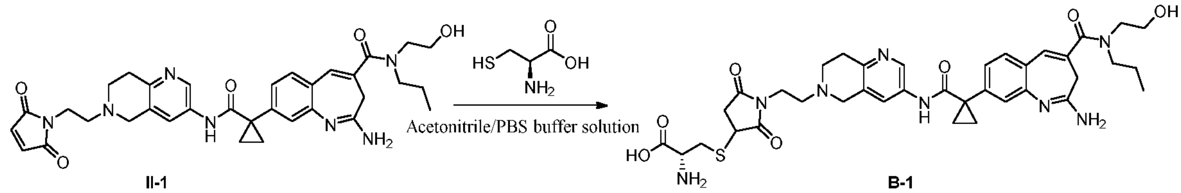


II-1 B-1

Compound 11-1 (32 mg, 0.051 mmol) was dissolved in acetonitrile (0.5 mL) and PBS buffer solution (1 mL), and L-cysteine (12 mg, 0.10 mmol) in PBS buffer solution (0.5 mL) was added dropwise to the above system. After the addition was completed, the reaction solution was stirred at room temperature for 1 hour and directly purified by *prep-HPLC* (eluent: 10% to 30% acetonitrile in a 0.1% aqueous trifluoroacetic acid solution, 20 minutes) to give compound B-1 (12 mg, yield: 27%) as a white solid. m/z: $[M+H]^+$ 747.4.

**Example 210:** Synthesis of S-(1-(2-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)-2-oxoethyl)-2,5-dioxopyrrolidin-3-yl)-L-cysteine (compound B-2)

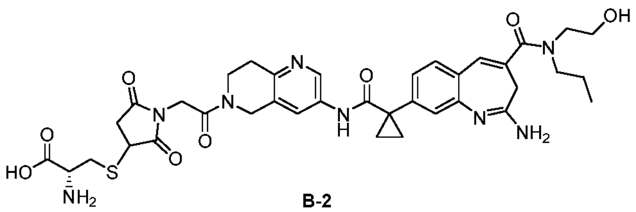

B-2

Using the synthesis method of B-1, compound B-2 was obtained as a white solid by reaction with compound II-2. m/z: $[M+H]^+$ 761.4.

**Example 211:** Synthesis of S-(1-(6-(((S)-1-(((S)-1-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)-1-oxo-5-ureidopentan-2-yl)amino)-3-methyl-1-oxobutan-2-yl)amino)-6-oxohexyl)-2,5-dioxopyrrolidin-3-yl)-L-cysteine (compound B-3)

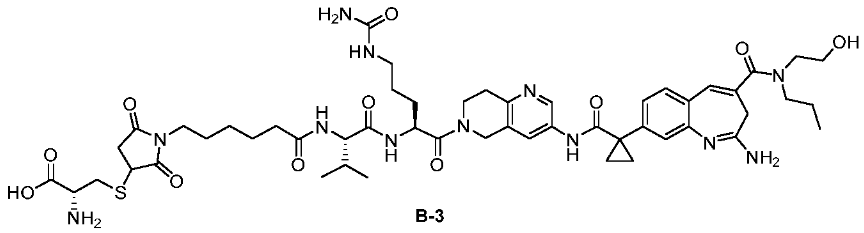

B-3

To a solution of compound II-19 (8 mg, 8.4 μM) in DMF (1 mL) was added L-cysteine (1.5 mg, 0.013 mmol). The reaction solution was stirred at room temperature for 1 hour and then directly purified by *prep-HPLC* (eluent 5%-45% acetonitrile in a 0.1% aqueous trifluoroacetic acid solution, 20 minutes) to obtain B-3 (3.5 mg, yield: 36%) as a white solid. m/z: $[1/2M+H]^+$ 537.2.

**Example 212:** Synthesis of S-(1-(25-(3-(1-(2-amino-4-((2-hydroxyethyl)(propyl)carbamoyl)-3H-benzo[b]azepin-8-yl) cyclopropyl-1-formylamino)-7,8-dihydro-1,6-naphthyridin-6(5H)-yl)-3,25-dioxo-7,10,13,16,19,22-hexaoxa-4-azapentacosyl)-2,5-dioxopyrrolidin-3-yl)-L-cysteine (compound B-4)

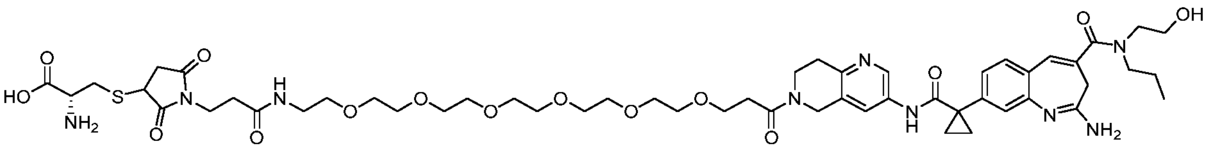

B-4

Using the synthesis method of B-3, compound B-4 was obtained as a white solid by reaction with compound II-12. m/z: $[1/2M+H]^+$555.8.

**Example 213:** Synthesis of S-(1-(2-((2-(2-(3-(3-(1-(2-amino-4-((2-hydroxyethyl)(propyl)carbamoyl)-3H-benzo[b]azepin-8-yl)cyclopropyl-1-formylamino)-7, 8-dihydro-1,6-naphthyridin-6(5H)-yl)-3-oxopropoxy)ethoxy)ethyl)amino)-2-oxoethyl)-2,5-dioxopyrrolidin-3-yl)-L-cysteine (compound B-5)

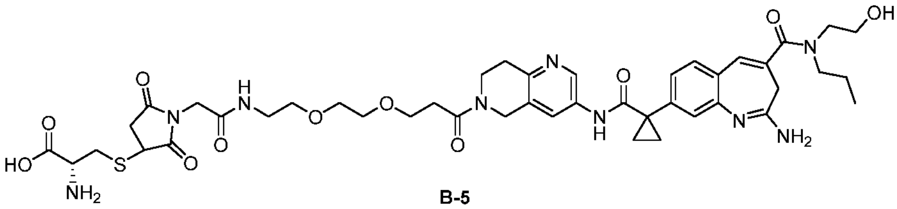

B-5

Using the synthesis method of B-3, compound B-5 was obtained as a white solid by reaction with compound II-23. m/z: $[1/2M+H]^+$460.2.

**Example 214:** Synthesis of (2R,2'R)-3,3'-(((5-(28-(3-(1-(2-amino-4-((2-hydroxyethyl)(propyl)carbamoyl)-3H-benzo[b] azepin-8-yl)cyclopropane-1-formylamino)-7,8-dihydro-1,6-naphthyridin-6(5H)-yl)-6,28-dioxo-10,13,16,19,22,25-hexaoxa-4-thia-7-azaoctacosanoyl)-1,3,5-triazine-1,3-diyl)bis(3-oxopropane-3,1-diyl))bis(sulfanediyl))bis(2-aminopropionic acid) (compound B-6)

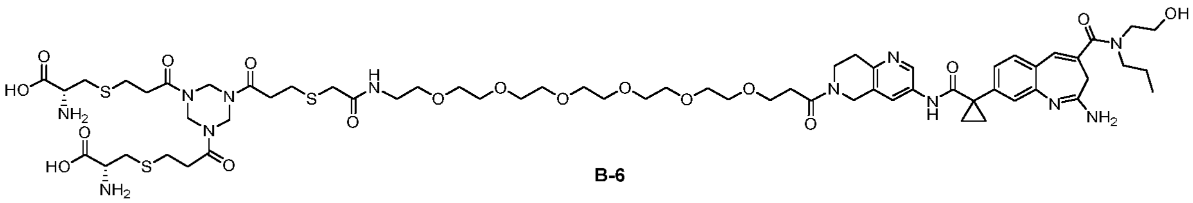

B-6

Using the synthesis method of B-3, compound B-6 was obtained as a white solid by reaction with compound II-74. m/z: $[1/2M+H]^+$702.4.

**Example 215:** Synthesis of S-(1-(4-(24-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)-2,24-dioxo-6,9,12,15,18,21-hexaoxa-3-azatetracosyl)phenyl)-2,5-dioxopyrrolidin-3-yl)-L-cysteine (compound B-7)

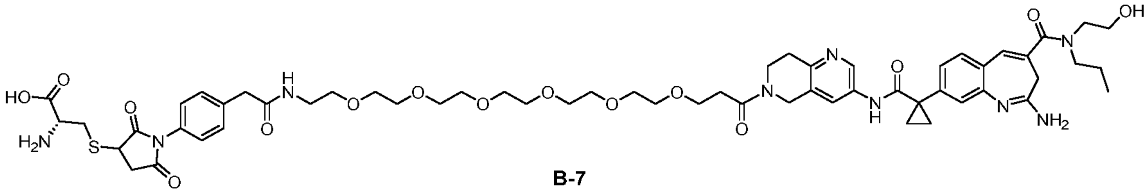

B-7

Using the synthesis method of B-3, compound B-7 was obtained as a white solid by reaction with compound II-59. m/z: $[1/2M+H]^+$586.8.

**Example 216:** Synthesis of S-(1-((5-((21-(3-(1-(2-amino-4-((2-hydroxyethyl)(propyl)carbamoyl)-3H-benzo[b]azepin-8-yl)cyclopropyl-1-formylamino)-7,8-dihydro-1,6-naphthyridin-6(5H)-yl)-21-oxo-3,6,9,12,15,18-hexaoxaheneicosyl)carbamoyl)-1,3-dioxan-2-yl)methyl)-2,5-dioxopyrrolidin-3-yl)-L-cysteine (compound B-8)

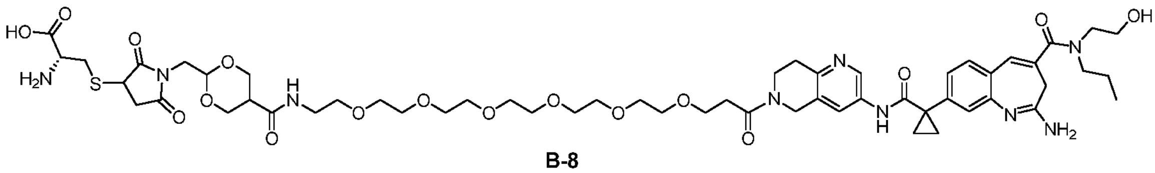

B-8

Using the synthesis method of B-3, compound B-8 was obtained as a white solid by reaction with compound II-78. m/z: $[1/2M+H]^+$591.8.

**Example 217:** Synthesis of S-(1-((5-(3-(1-(2-amino-4-((2-hydroxyethyl)(propyl)carbamoyl)-3H-benzo[b]azepin-8-yl)cyclopropyl-1-formylamino)-5,6,7,8-tetrahydro-1,6-naphthyridine-6-carbonyl)-1,3-dioxan-2-yl)methyl)-2,5-dioxopyrrolidin-3-yl)-L-cysteine (compound B-9)

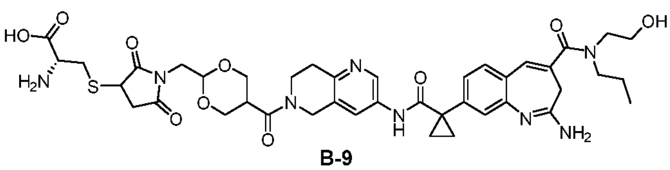

B-9

Using the synthesis method of B-3, compound B-9 was obtained as a white solid by reaction with compound II-77. m/z: $[1/2M+H]^+$424.2.

**Example 218:** Synthesis of S-(1-(4-(5-(3-(1-(2-amino-4-((2-hydroxyethyl)(propyl)carbamoyl)-3H-benzo[b]azepin-8-yl) cyclopropyl-1-formylamino)-5,6,7,8-tetrahydro-1,6-naphthyridine-6-carbonyl)-1,3-dioxan-2-yl)phenyl)-2,5-dioxopyrrolidin-3-yl)-L-cysteine (compound B-10)

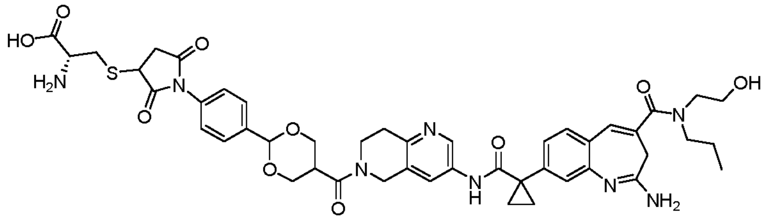

B-10

Using the synthesis method of B-3, compound B-10 was obtained as a white solid by reaction with compound II-140. m/z: $[M+H]^+$909.7.

**Example** 219: Synthesis of (2R,2'R)-3,3'-((2-(4-((6-(((S)-1-(((S)-1-(((S)-1-((4-(((3-(1-(2-amino-4-((2-hydroxyethyl)(propyl)carbamoyl)-3H-benzo[b]azepin-8-yl)cyclopropane-1-formylamino)-5,6,7,8-tetrahydro-1,6-naphthyridine-6-carbonyl)oxy)methyl)phenyl)amino)-1-oxopropan-2-yl)amino)-3-methyl-1-oxobutan-2-yl)amino)-4-carboxy-1-oxobutan-2-yl) amino)-6-oxohexyl)carbamoyl)benzoyl)propane-3,1-diyl))bis(sulfanediyl))bis(2-aminopropionic acid) (compound B-11)

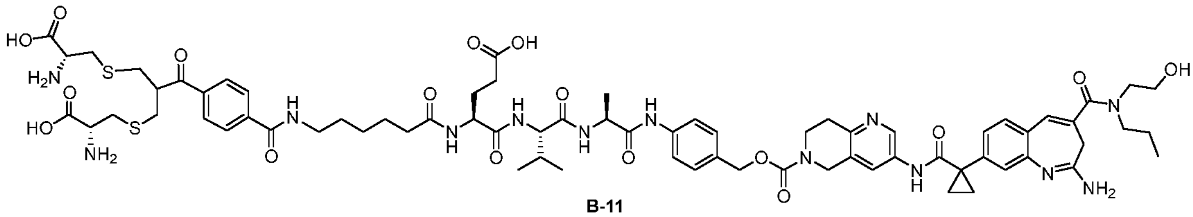

B-11

Using the synthesis method of B-3, compound B-11 was obtained as a white solid by reaction with compound II-124. m/z: $[1/2M+H]^+$ 739.4.

**Example 220: Antibody-drug conjugation reaction**

General method of antibody-drug conjugation reaction:

Method 1. Cysteine conjugation (general formula of the reaction is shown in formula 1 or formula 13): 2 mL (10.4 mg/mL) of an antibody solution was placed in a centrifuge tube and centrifuged using a centrifuge (centrifuged at 3800 G for 5 minutes) to concentrate the antibody solution. The NAP-25 chromatographic columns (5 mL/12 mL) of a Sephadex G-25 carrier were equilibrated with a phosphate buffer solution (50 mM, pH 6.5; PBS6.5/EDTA) containing sodium chloride (50 mM) and EDTA (2 mM). For one of the NAP-25 columns, 1.27 mL of the aqueous antibody solution was loaded, and the fractions eluted with PBS6.5/EDTA were separated. The concentration of the antibody solution was determined to be 13 mg/mL, and then the concentration of the antibody was adjusted to 10 mg/mL using PBS6.5/EDTA. This solution was taken into a 15 mL test tube, a TCEP aqueous solution (5 equivalents relative to one molecule of the antibody) was added and incubated at 25°C for 2 hours. The concentration of the reduced antibody was adjusted to 5 mg/mL using 3 mL of the PBS6.5/EDTA solution. 179 μL of N,N-dimethylacetamide (DMA) was added, followed by a dimethyl sulfoxide solution of the compound of formula II (Linker-payload) (10 equivalents relative to one molecule of the antibody), and the final proportion of DMA was controlled to about 10%. The solution was continued to be stirred at room temperature for 60 minutes. The NAP-25 chromatographic columns were equilibrated with the phosphate buffer solution (PB 7.4), 6.6 mL of the aqueous antibody-drug conjugate solution was loaded for one of the NAP-25 columns, and the fractions eluted with PB7.4 were separated. After this operation was repeated 2-3 times, and the eluted components were collected, concentrated, sterilized and filtered to obtain an antibody-immunostimulatory conjugate.

Method 2. Cysteine conjugation (general formula of the reaction is shown in formula 1): the antibody was adjusted to a concentration of 3 mg/mL with reaction Buffer (50 mM PBS, 2 mM EDTA, pH 6.5). Antibody, 8 volumes of the reaction

Buffer, and 20 equivalents (TCEP/antibody) of an aqueous TCEP solution were added to a centrifuge tube. The sample collected in the previous step was added with 30 equivalents (DHAA/antibody) of a DHAA oxidant in the centrifuge tube. The mixture was mixed evenly, placed in a thermostatic mixer at room temperature (22°C) and stirred for 2 hours, and then centrifuged with a 30 KD ultrafiltration tube (Millipore UFC803096) at 4300 rpm for 10 minutes. The ultrafiltration step was repeated three times. The sample was cooled to about 4°C, and a dimethyl sulfoxide solution of the compound (Linker-payload) of formula II (10 equivalents relative to one molecule of the antibody) was added to the reaction system. The mixture was mixed evenly, and placed in the thermostatic mixer (4°C) and stirred for reaction for 1 hour. The mixture was centrifuged with the ultrafiltration tube at 4300 rpm for 10 minutes, and the ultrafiltration step was repeated three times to obtain an antibody-immunostimulatory conjugate.

Method 3. Hydrolysis reaction (general formula of the reaction is shown in formula 1'): the antibody-immunostimulatory conjugate obtained by general method 1 or 2 with an ultrafiltration tube (MWCO 30KD, 4 mL, manufacturer: Millipore) was replaced into a buffer solution of 50 mM PBS, 2 mM EDTA, PH = 8 and reacted at 37°C overnight. 30 mM His-HAc without dimethyl sulfoxide at pH 5.5 was ultrafiltrated 10 times to obtain an antibody-immunostimulatory conjugate.

Method 4. Lysine random conjugation (general formula of the reaction is shown in formula 11): 15 mg (8.35 mg/mL) of an antibody solution was put into a centrifuge tube, a buffer solution of 50 mM PBS, 2 mM EDTA, pH 7.4 was added to dilute the antibody concentration to 5 mg/mL, a dimethyl sulfoxide solution of the compound (Linker-payload) of formula II was added according to the molar ratio of the final concentration of the drug and antibody of 15:1, and at the same time dimethyl sulfoxide was supplemented according to 15% of the total volume of the reaction solution. After being mixed evenly by shaking, the mixture was placed on a refrigerated thermostatic mixer for reaction at 22°C for 2 hours. The sample was replaced with an ultrafiltration tube (MWCO 30KD, 4 mL, manufacturer: Millipore) and preserved in the buffer, and ultrafiltrated with 30 mM His-HAc without dimethyl sulfoxide at pH 5.5 for 10 times to obtain an antibody-immunostimulatory conjugate.

The antibody used in methods 1 to 4 was antibody 1: Trastuzumab, Roche; antibody 2: Pertuzumab, Roche; antibody 3: Trastuzumab (L234A/L235A/P329G), MHDDD001, Shanghai Biointron Biotechnology Co., Ltd.; antibody 4: Trastuzumab (HC-s239.5) (engineered Trastuzumab with cysteine inserted between heavy chain positions 239 and 240 can be prepared by the method disclosed in Molecular Pharmaceutics. 2017, 14, 1501-1516), LPDDFD001, Shanghai Biointron Biotechnology Co., Ltd.; antibody 5: Anti-HEL human IgG1-Kappa Isotype control, B117901, Shanghai Biointron Biotechnology Co., Ltd.; antibody 6: Cetuximab, Merck; antibody 7: huA1 ($V_H$v2.0+$V_L$v2.4) (can be expressed and purified by the sequence information table disclosed in US8044178B2 for preparation); antibody 8: Sacituzumab (hRS7) (can be expressed and purified by the sequence information table disclosed in CN100360567C for preparation); antibody 9: Zolbetuximab (IMAB362) (can be expressed and purified by the sequence information table disclosed in US10738108B2 for preparation); antibody 10: Labetuzumab (hMN-14) (can be expressed and purified by the sequence information sheet disclosed in US5874540A for preparation); and antibody 11: Enfortumab (Ha22-2) (can be expressed and purified by the sequence information sheet disclosed in US10894090B2 for preparation).

2: Determination of the antibody concentration in an antibody-drug conjugate

The concentration of the conjugated drug in the antibody-drug conjugate can be calculated in the following way: measuring the UV absorbance of the aqueous solution of the antibody-drug conjugate at a wavelength of 280 nm, and then performing the following calculation to obtain the result.

Since the total absorbance at a certain wavelength is equal to the sum of the absorbances of all chemical-absorbing species present in the system (additivity of absorbance), it is assumed that the molar absorption coeffcients of the antibody and the drug do not change before and after conjugation of the antibody with the drug, and the antibody concentration and the drug concentration in the antibody-drug conjugate are shown in the following relational expressions.

$$A_{280} = A_{Drug,280} + A_{ab,280} = \varepsilon_{Drug}^{280} \times c_{Drug} + \varepsilon_{ab}^{280} \times c_{ab} = \varepsilon_{Drug}^{280} \times c_{ab} \times DAR + \varepsilon_{ab}^{280} \times c_{ab}$$

Thus, the concentration of the antibody-immunostimulatory conjugate is

$$c_{ab} = \frac{A_{280}}{\left(\varepsilon_{ab}^{280} + \varepsilon_{Drug}^{280} \times DAR\right)}.$$

3: Determination of the average number of drugs conjugated (DAR) per molecule of antibody in an antibody-drug conjugate

The average number of drugs conjugated per molecule of antibody in the antibody-drug conjugate can be

determined by high performance liquid chromatography (HPLC) analysis using the following method. HPLC apparatus: Waters/Waters e2695; mobile phase A: 1.5 M $(NH_4)_2SO_4$ + 50 mM potassium phosphate salt (pH 7.0); mobile phase B: 50 mM sodium phosphate salt (pH 7.0)/isopropanol (75:25 V/V); analytical column: Thermo MabPac™ HIC-Butyl 5μm 4.6×100mm, PN. 088558; injection volume: 5 μL; flow rate: 1 mL/min; column temperature: 30°C; detector: PDA detector; detection wavelength: 280 nm; and elution gradient:

| Time | Mobile phase A/% | Mobile phase B/% |
|---|---|---|
| 0.00 | 100 | 0 |
| 2.00 | 80 | 20 |
| 22.00 | 20 | 80 |
| 24.00 | 0 | 100 |
| 26.00 | 100 | 0 |
| 30.00 | 100 | 0 |

Hydrophobic interaction chromatography can be used to determine the drug-to-antibody ratio (DAR) in the antibody-conjugated drug. The unconjugated antibody drug has the weakest hydrophobicity and is eluted first; and the antibody conjugated with 8 drugs has the strongest hydrophobicity and is eluted last. The percentage of peak area represents the relative distribution of the ADC with a specific number of drugs conjugated, and the weighted average DAR can be calculated by using the percentage of peak area and the number of conjugated drugs through the formula: the weighted average DAR = Σ (relative peak area × number of drugs conjugated)/total peak area.

4: Analysis of the molecular size heterogeneity (SEC) of an antibody-immunostimulatory conjugate

HPLC apparatus: Waters Acquity Arc; Mobile phase: 100 mM PB + 200 mM Arg·HCl + 5% IPA, pH 6.8; analytical column: TOSOH TSKgel G3000 SWxl, 7.8 × 300 mm, 5 μM, 450 Å, PN0008541; injection volume: 10 μL; flow rate: 0.6 mL/min; column temperature: 30°C; detector: PDA detector; detection wavelength: 280 nm; and gradient: isocratic elution.

The antibody-immunostimulatory conjugates shown in Table 10 were prepared from antibodies and corresponding compounds using the general preparation method 1 in Example 220.

Table 10

| Antibody conjugate number | Compound number | Antibody | DAR | SEC/% |
|---|---|---|---|---|
| C-1 | II-1 | 1 | 3.54 | 99.07 |
| C-2 | II-2 | 1 | 2.98 | 99.10 |
| C-3 | II-3 | 1 | 1.34 | 99.07 |
| C-4 | II-4 | 1 | 5.69 | 99.55 |
| C-5 | II-5 | 1 | 5.07 | 99.08 |
| C-6 | II-6 | 1 | 5.22 | 99.20 |
| C-7 | II-7 | 1 | 5.53 | 97.22 |
| C-8 | II-8 | 1 | 5.07 | 99.13 |
| C-9 | II-9 | 1 | 5.21 | 99.16 |
| C-10 | II-10 | 1 | 5.06 | 99.12 |
| C-11 | II-11 | 1 | 4 | 98.70 |
| C-12 | II-12 | 1 | 3.93 | 99.34 |
| C-13 | II-13 | 1 | 3.84 | 99.31 |
| C-14 | II-14 | 1 | 3.93 | 99.35 |
| C-15 | II-15 | 1 | 3.95 | 99.23 |
| C-16 | II-16 | 1 | 3.39 | / |

(continued)

| Antibody conjugate number | Compound number | Antibody | DAR | SEC/% |
|---|---|---|---|---|
| C-17 | II-17 | 1 | 4.17 | 99.76 |
| C-18 | II-18 | 1 | 3.48 | 99.42 |
| C-19 | II-19 | 1 | 4.57 | 99.63 |
| C-20 | II-20 | 1 | 3.46 | 99.42 |
| C-21 | II-21 | 1 | 4.39 | 99.78 |
| C-22 | II-22 | 1 | 4.52 | 99.73 |
| C-23 | II-23 | 1 | 4.34 | 99.75 |
| C-24 | II-24 | 1 | 3.47 | 99.67 |
| C-25 | II-25 | 1 | 3.50 | 100 |
| C-26 | II-26 | 1 | 4.36 | 99.47 |
| C-27 | II-27 | 1 | 3.54 | 99.42 |
| C-28 | II-28 | 1 | 3.89 | 99.46 |
| C-29 | II-35 | 1 | / | 100 |
| C-30 | II-36 | 1 | 3.69 | 99.58 |
| C-31 | II-40-1 | 1 | 4.48 | 100 |
| C-32 | II-40-2 | 1 | 3.69 | 100 |
| C-33 | II-41-1 | 1 | 3.25 | 99.78 |
| C-34 | II-41-2 | 1 | 2.99 | 100 |
| C-35 | II-30 | 1 | 3.58 | 99.61 |
| C-36 | II-31 | 1 | 3.72 | 99.64 |
| C-37 | II-29 | 1 | 3.67 | 99.64 |
| C-38 | II-42 | 1 | 4.16 | 97.97 |
| C-39 | II-37 | 1 | 3.76 | 99.60 |
| C-40 | II-43 | 1 | 3.81 | 99.50 |
| C-41 | II-45 | 1 | 4.11 | 99.57 |
| C-42 | II-46 | 1 | 4.21 | 99.52 |
| C-43 | II-47 | 1 | 4.11 | 99.57 |
| C-44 | II-48 | 1 | 4.08 | 98.69 |
| C-45 | II-50 | 1 | 4.14 | 99.50 |
| C-46 | II-49 | 1 | 4.53 | 99.45 |
| C-47 | II-52 | 1 | 3.81 | 99.50 |
| C-48 | II-54 | 1 | 3.97 | 99.56 |
| C-49 | II-57 | 1 | 3.58 | 99.27 |
| C-50 | II-59 | 1 | 3.91 | 99.56 |
| C-51 | II-60 | 1 | 3.96 | 99.32 |
| C-52 | II-61 | 1 | 3.91 | 99.53 |
| C-53 | II-63 | 1 | 3.76 | 99.68 |
| C-54 | II-64 | 1 | 4.25 | 99.62 |
| C-55 | II-65 | 1 | 3.97 | 99.55 |

(continued)

| Antibody conjugate number | Compound number | Antibody | DAR | SEC/% |
|---|---|---|---|---|
| C-56 | II-66 | 1 | 4.07 | 99.59 |
| C-57 | II-67 | 1 | 4.02 | 99.53 |
| C-58 | II-68 | 1 | 4.00 | 99.47 |
| C-59 | II-69 | 1 | 4.08 | 94.74 |
| C-60 | II-70 | 1 | 3.85 | 99.57 |
| C-61 | II-71 | 1 | 3.85 | 99.53 |
| C-62 | II-72 | 1 | 3.98 | 99.66 |
| C-63 | II-73 | 1 | 3.70 | 99.63 |
| C-64 | II-75 | 1 | 3.78 | 99.60 |
| C-65 | II-77 | 1 | 3.93 | 99.61 |
| C-66 | II-78 | 1 | 4.01 | 99.67 |
| C-67 | II-79 | 1 | 3.95 | 99.64 |
| C-68 | II-80 | 1 | 4.06 | 99.50 |
| C-69 | II-83 | 1 | 4.20 | 99.52 |
| C-70 | II-85 | 1 | 4.14 | 99.38 |
| C-71 | II-86 | 1 | 3.82 | 99.65 |
| C-72 | II-82 | 1 | 3.79 | 99.11 |
| C-73 | II-87 | 1 | 3.50 | 98.64 |
| C-74 | II-88 | 1 | 3.86 | 98.92 |
| C-75 | II-89 | 1 | 4.03 | 98.90 |
| C-76 | II-109 | 1 | 3.53 | 99.52 |
| C-77 | II-96 | 1 | 3.91 | 99.60 |
| C-78 | II-97 | 1 | 3.84 | 99.35 |
| C-79 | II-98 | 1 | 3.87 | 99.50 |
| C-80 | II-99 | 1 | 4.03 | 99.56 |
| C-81 | II-100 | 1 | 3.99 | 99.51 |
| C-82 | II-101 | 1 | 4.03 | 99.50 |
| C-83 | II-102 | 1 | 4.15 | 97.81 |
| C-84 | II-103 | 1 | 3.86 | 99.59 |
| C-85 | II-104 | 1 | 3.81 | 99.44 |
| C-86 | II-105 | 1 | 3.98 | 99.08 |
| C-87 | II-106 | 1 | 4.16 | 99.33 |
| C-91 | II-90 | 1 | 3.91 | 98.58 |
| C-92 | II-91 | 1 | 3.93 | 98.96 |
| C-93 | II-94 | 1 | 4.45 | 98.90 |
| C-94 | II-53 | 1 | 2.21 | 94.27 |
| C-95 | II-92 | 1 | 3.86 | 98.59 |
| C-96 | II-93 | 1 | 3.86 | 98.62 |
| C-97 | II-87 | 1 | 3.50 | 98.64 |

(continued)

| Antibody conjugate number | Compound number | Antibody | DAR | SEC/% |
|---|---|---|---|---|
| C-98 | II-88 | 1 | 3.86 | 98.92 |
| C-99 | II-89 | 1 | 4.03 | 98.90 |
| C-105 | II-110 | 1 | 3.25 | 97.63 |
| C-106 | II-112 | 1 | 4.06 | 98.88 |
| C-107 | II-111 | 1 | 3.94 | 98.92 |
| C-108 | II-115 | 1 | 3.95 | 98.96 |
| C-109 | II-117 | 1 | 3.86 | 98.97 |
| C-110 | II-122 | 1 | 3.93 | 98.96 |
| C-111 | II-123 | 1 | 3.98 | 98.95 |
| C-115 | II-126 | 1 | 4.00 | 98.98 |
| C-116 | II-127 | 1 | 4.02 | 98.96 |
| C-117 | II-140 | 1 | 3.80 | 98.84 |
| C-118 | II-140 | 1 | 7.11 | 98.63 |
| C-119 | II-121 | 1 | 4.45 | 98.89 |
| C-120 | II-128 | 1 | 7.30 | 99.13 |
| C-121 | II-137 | 1 | 7.64 | 100 |
| C-122 | II-77 | 1 | 7.76 | 99.16 |
| C-123 | II-72 | 1 | 6.85 | 100 |
| C-124 | II-59 | 1 | 8.0 | 98.60 |
| C-125 | II-128 | 1 | 4.26 | 99.42 |
| C-137 | II-72 | 6 | 4.45 | 97.05 |
| C-138 | II-106 | 6 | 4.35 | 99.77 |
| C-139 | II-77 | 6 | 4.46 | 99.89 |
| C-140 | II-12 | 6 | 4.40 | 99.73 |
| C-141 | II-72 | 7 | 4.03 | 98.91 |
| C-142 | II-77 | 7 | 4.02 | 98.58 |
| C-143 | II-12 | 7 | 3.89 | 98.99 |
| C-144 | II-115 | 7 | 3.91 | 98.79 |
| C-145 | II-78 | 5 | 3.57 | 98.81 |
| C-146 | II-72 | 11 | 4.16 | 97.94 |
| C-147 | II-106 | 11 | 3.98 | 98.17 |
| C-148 | II-12 | 11 | 4.30 | 97.76 |
| C-149 | II-30 | 11 | 4.44 | / |
| C-150 | II-30 | 10 | 4.54 | / |
| C-151 | II-12 | 10 | 3.70 | 97.77 |
| C-152 | II-72 | 10 | 3.77 | 97.58 |
| C-153 | II-106 | 10 | 3.87 | 98.11 |
| C-154 | II-106 | 7 | 3.91 | 97.03 |
| C-155 | II-72 | 9 | 3.40 | 98.36 |

(continued)

| Antibody conjugate number | Compound number | Antibody | DAR | SEC/% |
|---|---|---|---|---|
| C-156 | II-106 | 9 | 3.40 | 98.58 |
| C-157 | II-7 | 9 | 3.20 | 98.14 |
| C-158 | II-140 | 9 | 3.30 | 98.46 |
| C-159 | II-106 | 8 | 3.50 | 98.43 |
| C-160 | II-72 | 8 | 3.67 | 98.62 |
| C-161 | II-7 | 8 | 3.60 | 98.54 |
| C-162 | II-140 | 8 | 4.20 | 98.64 |
| C-163 | II-157 | 1 | 3.79 | 99.52 |
| C-164 | II-153 | 1 | 3.72 | 99.59 |
| C-165 | II-159 | 1 | 3.74 | 99.55 |
| C-166 | II-160 | 1 | 3.75 | 99.58 |
| C-167 | II-151 | 1 | 4.32 | 98.19 |
| C-168 | II-155 | 1 | 4.11 | 98.53 |

The antibody-immunostimulatory conjugates shown in Table 11 were prepared from antibodies and corresponding compounds using the general preparation method 2 in Example 220.

Table 11

| Antibody conjugate number | Compound number | Antibody | DAR | SEC/% |
|---|---|---|---|---|
| C-127 | II-72 | 4 | 2 | 99.53 |

The antibody-immunostimulatory conjugates shown in Table 12 were prepared from antibodies and corresponding compounds using general methods 1 and 3 in Example 220.

Table 12

| Antibody conjugate number | Compound number | Antibody | DAR | SEC/% |
|---|---|---|---|---|
| C-126 | II-140 | 2 | 4.41 | 99.34 |
| C-128 | II-128 | 1 | 7.30 | 99.13 |
| C-129 | II-128 | 1 | 4.03 | 98.76 |
| C-130 | II-140 | 1 | 4.70 | 98.64 |

The antibody-immunostimulatory conjugates shown in Table 13 were prepared from antibodies and corresponding compounds using the general preparation method 4 in Example 220.

Table 13

| Antibody conjugate number | Compound number | Antibody | DAR | SEC/% |
|---|---|---|---|---|
| C-90 | II-84 | 1 | 3.44 | 98.45 |

**Example 221: Antibody-drug conjugation reaction (cysteine bridging)**

1. General method of antibody-drug conjugation reaction (general formula of the reaction is shown in formula 5, formula 6, formula 14, formula 15 or formula 16):

15 mg of a 15.8 mg/mL antibody was taken and placed in a centrifuge tube, and 25 mM PBS buffer (pH7.4)

containing 1 mM EDTA was added to dilute the antibody concentration to 5 mg/mL. The mixture was mixed evenly by shaking, and then a 2 mg/mL TCEP aqueous solution (8 equivalents relative to one molecule of antibody) was added to reduce the antibody. After being mixed evenly by shaking, the mixture was placed on a refrigerated thermostatic mixer for reaction at 37°C for 1-24 hours. The mixture was ultrafiltrated with a buffer solution of 50 mM BBS, pH 8.0 4 times, the ultrafiltrated antibody was recovered, a dimethyl sulfoxide solution of the compound (Linker-payload) of formula II (6-8 equivalents relative to one molecule of antibody) was added, and the dimethyl sulfoxide solution was supplemented according to 10% of the total volume of the reaction solution. After being mixed evenly by shaking, the mixture was placed on a refrigerated thermostatic mixer for reaction at 4-20°C for 1 hour. The sample was replaced with an ultrafiltration tube and preserved in the buffer solution, and ultrafiltrated with 30 mM His-HAc at pH 5.5 for 10 times to obtain an antibody-immunostimulatory conjugate.

The antibody used in the general method was antibody 1: Trastuzumab, Roche; antibody 2: Pertuzumab, Roche; antibody 6: Cetuximab, Merck; antibody 7: huA1($V_H$v2.0+$V_L$v2.4); antibody 8: Sacituzumab (hRS7); antibody 9: Zolbetuximab (IMAB362); antibody 10: Labetuzumab (hMN-14); and antibody 11: Enfortumab (Ha22-2).

2. Determination of the average number of drugs conjugated (DAR) per molecule of antibody in an antibody-drug conjugate

HPLC apparatus: Waters/Waters e2695; mobile phase A: 1.5 M $(NH_4)_2SO_4$ + 50 mM potassium phosphate salt (pH 7.0); mobile phase B: 50 mM sodium phosphate salt (pH 7.0)/isopropanol (75:25 V/V); analytical column: Thermo MabPac™ HIC-Butyl 5μm 4.6×100mm, PN. 088558; injection volume: 5 μL; flow rate: 1 mL/min; column temperature: 30°C; detector: PDA detector; detection wavelength: 280 nm; and elution gradient:

| Time | Mobile phase A/% | Mobile phase B/% |
|---|---|---|
| 0.00 | 100 | 0 |
| 2.00 | 90 | 10 |
| 22.00 | 35 | 65 |
| 24.00 | 0 | 100 |
| 26.00 | 100 | 0 |
| 30.00 | 100 | 0 |

Hydrophobic interaction chromatography can be used to determine the drug-to-antibody ratio (DAR) in the antibody-conjugated drug. The unconjugated antibody drug has the weakest hydrophobicity and is eluted first; and the antibody conjugated with 8 drugs has the strongest hydrophobicity and is eluted last. The percentage of peak area represents the relative distribution of the ADC with a specific number of drugs conjugated, and the weighted average DAR can be calculated by using the percentage of peak area and the number of conjugated drugs through the formula: the weighted average DAR = Σ (relative peak area × number of drugs conjugated)/total peak area.

4. Analysis of the molecular size heterogeneity (SEC) of an antibody-immunostimulatory conjugate

HPLC apparatus: Waters/Waters e2695; mobile phase: 50 mM PB + 200 mM Arg·HCl + 10% IPA, pH 6.8; analytical column: Waters Xbridge BEH200 SEC (7.8×300 mm, 3.5 μm); injection volume: 10 μL; flow rate: 0.5 mL/min; column temperature: 30°C; detector: PDA detector; detection wavelength: 280 nm; and gradient: isocratic elution.

The antibody-immunostimulatory conjugates shown in Table 14 were prepared from antibodies and corresponding compounds using the general preparation method in Example 221.

Table 14

| Antibody conjugate number | Compound number | Antibody | DAR | SEC/% |
|---|---|---|---|---|
| C-88 | II-74 | 1 | ~3.3 | 99.68 |
| C-89 | II-95 | 1 | ~4.1 | 97.64 |
| C-112 | II-125 | 1 | 4.0 | 96.60 |
| C-113 | II-125 | 1 | 2.33 | 97.88 |
| C-114 | II-124 | 1 | 3.85 | 96.48 |

(continued)

| Antibody conjugate number | Compound number | Antibody | DAR | SEC/% |
|---|---|---|---|---|
| C-131 | II-139 | 1 | 4.0 | 97.42 |
| C-132 | II-133 | 1 | 4.0 | 98.54 |
| C-133 | II-124 | 7 | 4.03 | 98.91 |
| C-134 | II-124 | 6 | 4.0 | 94.30 |
| C-135 | II-124 | 2 | 3.82 | 96.70 |
| C-136 | II-124 | 11 | 3.10 | 97.02 |
| C-170 | II-124 | 10 | 3.51 | 96.97 |
| C-171 | II-154 | 1 | 4.19 | 98.12 |
| C-172 | II-146 | 1 | 3.75 | 96.34 |
| C-173 | II-152 | 1 | 4.0 | 90.95 |
| C-174 | II-156 | 1 | 3.58 | 94.51 |
| C-175 | II-161 | 1 | 4.10 | 97.00 |

**Effect Example 1: Detection of the cell activity of TLR7 and TLR8**

In this experiment, the biological activity of TLR7 and TLR8 in the compound of formula I was detected using cell tests. This method was conducted in human embryonic kidney cells (HEK293) expressing TLR family members such as TLR4, TLR7, TLR8 or TLR9. TLR agonists activate TLRs, triggering the activation of downstream NF-kB, which in turn activates the secreted embryonic alkaline phosphatase (SEAP) reporter gene. The activity of SEAP was detected using the Quanti-Blue (InvivoGen) reagent, thereby reflecting the activities of TLR7 and TLR8 agonists.

The detailed experimental method was as follows:
HEK-BLUE-hTLR7 and HEK-BLUE-hTLR8 cell lines were purchased from Invivogen Company and cultured in DMEM medium containing 4.5 g/L glucose (Sigma-Aldrich) and 10% fetal bovine serum under the conditions of temperature 37°C, humidity 95% and 5% $CO_2$.

The compound was tested at a concentration ranging from 0.5 nM to 15 μM over a total of 10 concentration gradients. TLR7 or TLR8 agonists with known activity were added as positive controls, and 1 μL of DMSO was added as a negative control.

The cells were treated as follows: the cells were removed from the culture dish and centrifuged to remove the medium, and resuspended in a T-150 bottle with 10 mL of preheated PBS, 12 mL of preheated medium was added, and the cells were gently pipetted up and down and counted under a microscope. A 200,000/mL single-cell suspension was immediately prepared with medium and added to a 96-well plate at 200 μL/well (40,000/well). The final concentration of DMSO was 0.5%.

The compound was added and incubated in an incubator at 37°C, 5% CO2 for 24 hours.

A supernatant at 20 μL/well was aspirated into 180 μL of preheated Quanti-Blue at 37°C, incubated at 37°C for 1.5 hours, and detected for the absorbance (OD value) with a spectrophotometer at 650 nm. The formula for calculating the agonistic effect is as follows:

Effect % = (average OD value of the drug-administered group - average OD value of the DMSO group) / (average OD value of the positive drug group - average OD value of the DMSO group) × 100

Graphpad software was used to fit the concentration-effect curve, and $EC_{50}$ was calculated. The test results are shown in Table 15 (A denotes < 250 nM, B denotes 250 nM-1 μM, C denotes 1 μM-2 μM, D denotes > 2 μM, and - denotes not listed or not tested):

Table 15

| Compound number | TLR8 $EC_{50}$ | TLR7 $EC_{50}$ | Compound number | TLR8 $EC_{50}$ | TLR7 $EC_{50}$ |
|---|---|---|---|---|---|
| 1 | A | D | II-7 | B | - |
| 2 | B | D | II-12 | B | - |
| 3 | A | D | II-17 | B | - |
| 6 | B | D | II-18 | B | - |

(continued)

| Compound number | TLR8 $EC_{50}$ | TLR7 $EC_{50}$ | Compound number | TLR8 $EC_{50}$ | TLR7 $EC_{50}$ |
|---|---|---|---|---|---|
| 7 | A | D | II-19 | B | - |
| 8 | B | D | II-23 | A | - |
| 12 | A | - | II-24 | B | - |
| 13 | A | - | II-28 | B | - |
| 15 | A | - | II-36 | A | - |
| 16 | A | - | II-46 | A | - |
| 17 | A | - | II-64 | B | - |
| 18 | A | - | II-66 | B | - |
| 19 | A | - | II-72 | B | - |
| 20 | B | - | II-77 | B | - |
| 22 | B | - | II-96 | B | - |
| 23 | A | - | II-97 | B | - |
| 25-1 | A | - | II-98 | B | - |
| 33 | A | - | II-104 | B | - |
| 35 | B | - | | | |

**Effect Example 2: Detection of cytokines**

The cryopreserved human peripheral blood mononuclear cells (Allcells) were rapidly thawed in a 37°C water bath and then added into 9 mL of RPMI 1640 medium containing 10% fetal bovine serum, 100 U/mL penicillin, 100 μg/mL streptomycin, and 1 mM sodium pyruvate (all from Gibco). The cell culture medium was centrifuged at 400 × g for 5 minutes at room temperature, the supernatant was discarded, the cells were resuspended in the medium, and the cell density was adjusted to 4 × $10^6$/mL. The cells were added to a 96-well flat-bottom plate (Corning), with 50 μL per well.

System 1: 50 μL of medium was added to the above culture system.

System 2: human breast cancer epithelial cells BT474 (Nanjing Cobioer Biosciences Co., Ltd.), human breast cancer cells MCF-7 (Nanjing Cobioer Biosciences Co., Ltd.), human breast cancer cells MDA-MB-468 (Nanjing Cobioer Biosciences Co., Ltd.), human gastric cancer cells NUGC-4 (Nanjing Cobioer Biosciences Co., Ltd.), human breast ductal carcinoma cells MDA-MB-175VII (Nanjing Cobioer Biosciences Co., Ltd.), human colon adenocarcinoma cells LS180 (Nanjing Cobioer Biosciences Co., Ltd.), or human metastatic pancreatic cancer cell AsPC-1 (Nanjing Cobioer Biosciences Co., Ltd.) were collected and resuspended in RPMI 1640 complete medium, and the cell density was adjusted to 4 × $10^5$/mL. The cells were added to a 96-well plate, with 50 μL per well, and mixed evenly with hPBMCs.

A dilution of the sample to be tested at 3-fold working concentration was prepared using the medium, and added to the cell suspension of the above system 1 or system 2, with 50 μL per well. The total system was 150 μL and the final concentration of the compound was 500 nM. The blank control was supplemented with 50 μL of the medium and incubated in an incubator at 37°C, 5%$CO_2$ for 20 hours.

Detection of human TNFα:

The cell culture plate was centrifuged at 2000 rpm for 5 minutes at room temperature, and 100 μL of the supernatant was transferred to a clean 96-well plate. The TNFα detection reagent (Cisbio, 62HTNFAPEG) was placed at room temperature until complete dissolution. Two antibodies (TNFα Eu Cryptate antibody and TNFα d2 antibody) were mixed with a buffer solution (detection buffer) at a ratio of 1:40. 4 μL of the antibody mixture and 16 μL of the supernatant were added to the detection plate and incubated at room temperature in the dark for 2 hours. The signal was read in HTRF mode on the Infinite M1000 PRO (TECAN).

Detection of human IL6:

The cell culture plate was centrifuged at 2000 rpm for 5 minutes at room temperature, and 100 μL of the supernatant was transferred to a clean 96-well plate. The IL6 detection reagent (Cisbio, 62HIL6PEG) was placed at room

temperature until complete dissolution. Two antibodies (IL6 Eu Cryptate antibody and IL6 d2 antibody) were mixed with a buffer solution (detection buffer) at a ratio of 1:40. 4 μL of the antibody mixture and 16 μL of the supernatant were added to the detection plate and incubated at room temperature in the dark for 2 hours. The signal was read in HTRF mode on the Infinite M1000 PRO (TECAN).

Detection of human IFNγ

The cell culture plate was centrifuged at 2000 rpm for 5 minutes at room temperature, and 100 μL of the supernatant was transferred to a clean 96-well plate. The IFNγ detection reagent (Cisbio, 62HIFNGPEG) was placed at room temperature until complete dissolution. Two antibodies (IFNγ Eu Cryptate antibody and IFNγ d2 antibody) were mixed with a buffer solution (detection buffer) at a ratio of 1:40. 4 μL of the antibody mixture and 16 μL of the supernatant were added to the detection plate and incubated at room temperature in the dark overnight. The signal was read in HTRF mode on the Infinite M1000 PRO (TECAN).

Detection of human IL12/IL23 (P40)

100 μL of the capture antibody was added to a high-binding 96-well plate (Corning, 9018) and incubated at 4°C overnight. The plate was washed 4 times with a washing solution, a blocking solution (1 × Assay Diluent A) was added with 200 μL per well and incubated at room temperature for 1 h, the blocking solution was discarded, the diluted sample or standard was added and incubated at room temperature for 2 h, then the plate was washed 4 times, 100 μL of the detection antibody was added and incubated at room temperature for 1 h, then the plate was washed 4 times, an Avidin-HRP working solution was added and incubated at room temperature for 0.5 h, then the plate was washed 5 times, 100 μL of a TMB mixture was added, and the absorbance value OD450nm was read after 15 minutes at room temperature in the dark.

The test results of system 1 showed: when C-7, C-10, C-11, C-12, C-13, C-14, C-15, C-16, C-17, C-18, C-19, C-21, C-22, C-23, C-24, C-25, C-26, C-27, C-28, C-29, C-30, C-35, C-36, C-37, C-38, C-39, C-40, C-41, C-42, C-43, C-44, C-45, C-46, C-47, C-48, C-49, C-50, C-51, C-52, C-53, C-54, C-55, C-56, C-58, C-59, C-60, C-61, C-62, C-63, C-64, C-65, C-66, C-67, C-68, C-69, C-70, C-71, C-72, C-73, C-75, C-77, C-78, C-79, C-81, C-82, C-84, C-85, C-86, C-88, C-89, C-97, C-99, C-117, C-118, C-119, C-127, C-128, C-129, C-131, C-163, C-164, C-165, C-166, C-167, C-168 or C-174 at the concentration of 500 nM, TNFα secretion values were all less than 500 pg/mL.

The test results of system 2 are shown in Table 16 (A denotes < 1 nM, B denotes 1-10 nM, C denotes > 10 nM and ≤ 100 nM, D denotes > 100 nM, and - denotes not listed or not tested):

Table 16

| Number | TNFα (BT474/hPBMC) $EC_{50}$ | IL12 (BT474/hPBMC) $EC_{50}$ |
|---|---|---|
| C-4 | A | A |
| C-5 | A | A |
| C-6 | A | A |
| C-7 | A | A |
| C-8 | A | A |
| C-9 | A | A |
| C-10 | A | A |
| C-11 | A | A |
| C-12 | A | A |
| C-13 | A | A |
| C-14 | A | A |
| C-15 | A | A |
| C-16 | A | A |
| C-17 | A | A |
| C-18 | A | A |
| C-19 | A | A |
| C-20 | A | A |

(continued)

| Number | TNFα (BT474/hPBMC) $EC_{50}$ | IL12 (BT474/hPBMC) $EC_{50}$ |
|---|---|---|
| C-21 | A | A |
| C-22 | A | A |
| C-23 | A | A |
| C-24 | A | A |
| C-25 | A | - |
| C-26 | A | - |
| C-27 | A | - |
| C-28 | A | - |
| C-29 | A | A |
| C-30 | A | - |
| C-31 | A | A |
| C-32 | A | A |
| C-33 | A | A |
| C-34 | A | A |
| C-35 | B | - |
| C-36 | B | - |
| C-37 | A | - |
| C-39 | A | - |
| C-40 | A | - |
| C-41 | B | - |
| C-42 | A | - |
| C-43 | A | - |
| C-44 | A | - |
| C-45 | A | - |
| C-46 | A | - |
| C-47 | A | - |
| C-48 | A | - |
| C-49 | A | - |
| C-50 | A | - |
| C-51 | A | - |
| C-52 | A | - |
| C-53 | A | - |
| C-54 | A | - |
| C-56 | A | - |
| C-57 | A | - |
| C-58 | A | - |
| C-60 | A | - |
| C-62 | A | - |
| C-63 | A | - |

(continued)

| Number | TNFα (BT474/hPBMC) $EC_{50}$ | IL12 (BT474/hPBMC) $EC_{50}$ |
|---|---|---|
| C-64 | B | - |
| C-65 | A | - |
| C-66 | A | - |
| C-67 | A | - |
| C-69 | A | - |
| C-70 | A | - |
| C-71 | A | - |
| C-72 | A | - |
| C-73 | A | - |
| C-74 | A | - |
| C-75 | A | - |
| C-76 | A | - |
| C-77 | A | - |
| C-78 | A | - |
| C-79 | A | - |
| C-80 | A | - |
| C-81 | A | - |
| C-82 | A | - |
| C-83 | B | - |
| C-84 | A | - |
| C-85 | A | - |
| C-86 | A | - |
| C-87 | A | - |
| C-88 | A | - |
| C-89 | A | - |
| C-91 | A | - |
| C-92 | A | - |
| C-93 | A | - |
| C-94 | A | - |
| C-95 | A | - |
| C-96 | A | - |
| C-97 | A | - |
| C-98 | A | - |
| C-99 | A | - |
| C-105 | A | - |
| C-106 | A | - |
| C-107 | A | - |
| C-108 | A | - |
| C-109 | A | - |

(continued)

| Number | TNFα (BT474/hPBMC) $EC_{50}$ | IL12 (BT474/hPBMC) $EC_{50}$ |
|---|---|---|
| C-110 | A | - |
| C-111 | A | - |
| C-112 | A | - |
| C-114 | A | - |
| C-115 | A | - |
| C-116 | A | - |
| C-117 | A | - |
| C-118 | A | - |
| C-119 | A | - |
| C-120 | A | - |
| C-121 | A | - |
| C-122 | A | - |
| C-123 | A | - |
| C-124 | A | - |
| C-125 | A | - |
| C-126 | A | - |
| C-128 | A | - |
| C-129 | A | - |
| C-130 | A | - |
| C-131 | A | - |
| C-132 | A | - |
| C-135 | A | - |
| C-145 | B | - |
| C-163 | A | - |
| C-164 | A | - |
| C-165 | A | - |
| C-166 | A | - |
| C-167 | A | - |
| C-168 | A | - |
| C-171 | A | - |
| C-172 | A | - |
| C-173 | A | - |
| C-174 | A | - |
| C-175 | A | - |

Table 17

| Number | TNFα (MCF-7/hPBMC) EC50 | Number | TNFα (MCF-7/hPBMC) EC50 |
|---|---|---|---|
| huA1 | D | C-142 | B |
| C-133 | A | C-143 | B |

(continued)

| Number | TNFα (MCF-7/hPBMC) EC50 | Number | TNFα (MCF-7/hPBMC) EC50 |
|---|---|---|---|
| C-141 | A | C-144 | A |

Table 18

| Number | TNFα (MDA-MB-468/hPBMC) $EC_{50}$ | TNFα (MDA-MB-468/hPBMC) (100 nM, pg/mL) |
|---|---|---|
| Cetuximab | - | 345 |
| C-134 | A | 12734 |
| C-137 | A | 12010 |
| C-138 | A | 13660 |
| C-139 | A | 12911 |
| C-140 | A | 9437 |

Table 19

| Number | TNFα (MDAMB175VII/hPBMC) EC50 | Number | TNFα (MDAMB175VII/hPBMC) EC50 |
|---|---|---|---|
| Ha22-2 | D | hMN-14 | D |
| C-136 | A | C-150 | B |
| C-148 | A | C-152 | A |
| C-149 | A | C-170 | A |

Table 20

| Number | TNFα (NUGC-4/hPBMC) $EC_{50}$ | TNFα (NUGC-4/hPBMC) (100 nM, pg/mL) |
|---|---|---|
| IMAB362 | - | 261 |
| C-155 | B | 8241 |
| C-157 | - | 1210 |
| C-158 | B | 4376 |

**Effect Example 3:** Plasma stability experiment

The sample to be tested was added to human and mouse plasma, and the concentration of the sample to be tested was 0.5 mg/mL. On days 0, 1, 2, 3, 5 and 7, respectively, 50 μg/mL of the sample solution to be tested was taken into a new plate, 1 μg/mL of human Her2/ErbB2 protein (acro, Cat#HE2-H5225) and anti-small molecule antibody (4072-01) in a PBS buffer solution (137 mM NaCl,10 mM $Na_2HPO_4$, 2.7 mM KCl, 2 mM $KH_2PO_4$), and 30 μL was added to each well of a high-binding 96-well plate (High Bind Microplate, SpectraPlate, Cat#: 6007500) and incubated at 4°C overnight. The plate was washed three times with a washing solution (90 μL per well), 60 μL of a blocking buffer solution (PBS buffer solution containing 0.05% Tween-20 and 1% BSA) was added per well and incubated at 37°C for 1 hour, then 30 μL of the sample or standard was added and incubated at room temperature for 2 hours, the plate was washed three times with a washing solution (PBS buffer solution containing 0.05% Tween-20) (90 μL per well), 30 μL of the detection antibody (Anti-Human IgG (Fab specific)- peroxidase: Sigma, Cat# A0293) was added and incubated at room temperature for 2 hours, the plate was washed three times with a washing solution (90 μL per well), and 30 μL of a TMB solution (A + B) (Solarbio, Cat#: PR1210-2*50 mL) was added and incubated at room temperature in the dark for 5 minutes. 30 μL of a stop solution (2 M dilute sulfuric acid) was added, and the OD value was read at a wavelength of 450 nm. The results are shown in Table 21:

Table 21

| Number | Human T1/2 (day) | Mouse T1/2 (day) | Number | Human T1/2 (day) | Mouse T1/2 (day) |
|---|---|---|---|---|---|
| C-12 | >4.0 | >4.0 | C-108 | >4.0 | >4.0 |

(continued)

| Number | Human T1/2 (day) | Mouse T1/2 (day) | Number | Human T1/2 (day) | Mouse T1/2 (day) |
|---|---|---|---|---|---|
| C-19 | 4.0 | >4.0 | C-110 | >4.0 | >4.0 |
| C-21 | <4.0 | >4.0 | C-112 | >4.0 | >4.0 |
| C-23 | >4.0 | >4.0 | C-114 | >4.0 | >4.0 |
| C-30 | >4.0 | >4.0 | C-115 | >4.0 | >4.0 |
| C-50 | >4.0 | >4.0 | C-116 | >4.0 | >4.0 |
| C-62 | >4.0 | >4.0 | C-117 | >4.0 | >4.0 |
| C-65 | >4.0 | >4.0 | C-118 | >4.0 | >4.0 |
| C-66 | >4.0 | >4.0 | C-119 | >4.0 | >4.0 |
| C-78 | >4.0 | >4.0 | C-120 | >4.0 | >4.0 |
| C-79 | >4.0 | >4.0 | C-122 | >4.0 | >4.0 |
| C-88 | >4.0 | >4.0 | C-123 | >4.0 | >4.0 |
| C-89 | >4.0 | >4.0 | C-128 | >4.0 | >4.0 |
| C-105 | >4.0 | >4.0 | C-131 | >4.0 | >4.0 |
| C-106 | >4.0 | >4.0 | C-132 | >4.0 | >4.0 |
| C-107 | >4.0 | >4.0 | | | |

**Effect Example 4:** PK concentration detection

The 96-well plate was coated with the goat anti-human IgG antibody (Cat # 609-101-017) or anti-small molecule antibody (4072-01), placed in a refrigerator at 4°C overnight, and washed three times the next day with a washing solution (PBS buffer solution containing 0.05% Tween-20), then a blocking buffer solution (PBS buffer solution containing 0.05% Tween-20 and 1% BSA) was used to block the plate and incubated at 37°C for 1-2 hours, then the plate was washed three times with a washing solution, and the diluted standard and test sample were added to the wells and incubated at room temperature for 2 hours, and the plate was washed three times with a washing solution, and then the detection antibody (Anti-Human IgG (Fab specific)-peroxidase: Sigma, Cat# A0293) diluted with a PBS buffer solution was added and incubated at room temperature for 1 hour. Finally, a color-developing solution was added and incubated at room temperature for 10 minutes, then the reaction was terminated with a stop solution (2 M dilute sulfuric acid), and the OD value was read at a wavelength of 450 nm.

**Effect Example** 5: In vivo efficacy experiment in the mouse subcutaneous xenograft model of MC38-HER2 mouse colon cancer cells

Cell culture: mouse colon cancer MC38-HER2 cells were maintained in monolayer culture in the DMEM medium containing 10% fetal bovine serum and 4 μg/mL puromycin in a constant temperature incubator containing 5% CO2 at 37°C. The tumor cells were subcultured twice a week. The cells in the exponential growth phase were collected and counted for inoculation.

Experimental animals: B6-hTLR8-HO mice, 6-8 weeks, 18-22 g.
A) A total of 6 experimental groups were set up for Vehicle, C-87, C-12, C-30, C-23 and C-19, as shown in Table 22 below:

Table 22

| Group | Number of mice | Test compound | Dose | Route of administration | Schedule of administration |
|---|---|---|---|---|---|
| G1 | 6 | Vehicle | -- | i.p. | Single dose |
| G2 | 6 | C-87 | 10 mg/kg | i.p. | Single dose |
| G3 | 6 | C-12 | 10 mg/kg | i.p. | Single dose |
| G4 | 6 | C-30 | 10 mg/kg | i.p. | Single dose |

(continued)

| Group | Number of mice | Test compound | Dose | Route of administration | Schedule of administration |
|---|---|---|---|---|---|
| G5 | 6 | C-23 | 10 mg/kg | i.p. | Single dose |
| G6 | 6 | C-19 | 10 mg/kg | i.p. | Single dose |

B) A total of 4 experimental groups were set up for Vehicle, C-65, C-130 and C-135, as shown in Table 23 below:

Table 23

| Group | Number of mice | Test compound | Dose | Route of administration | Schedule of administration |
|---|---|---|---|---|---|
| G1 | 8 | Vehicle | -- | i.p. | Single dose |
| G2 | 8 | C-65 | 7.5 mg/kg | i.p. | Single dose |
| G3 | 8 | C-135 | 7.5 mg/kg | i.p. | Single dose |
| G4 | 8 | C-130 | 7.5 mg/kg | i.p. | Single dose |

C) A total of 2 experimental groups were set up for Vehicle, trastuzumab and C-62, as shown in Table 24 below:

Table 24

| Group | Number of mice | Test compound | Dose | Route of administration | Schedule of administration |
|---|---|---|---|---|---|
| G1 | 8 | Vehicle | -- | i.p. | QW*3 |
| G2 | 8 | Trastuzumab | 7.5 mg/kg | i.p. | QW*3 |
| G3 | 8 | C-62 | 7.5 mg/kg | i.p. | QW*3 |
| Note: i.p.: intraperitoneal administration; and QW: once a week | | | | | |

Experimental method: the MC38-HER2 cell line ($5.0 \times 10^6$ cells/mouse) was inoculated subcutaneously on the right back of the experimental mice, with an inoculation volume of 0.1 mL for each mouse. The growth of the tumor was observed regularly. When the tumor grew to approximately 100 $mm^3$, the mice were randomly grouped according to the tumor size and the body weight of the mice, and administered according to the schedule of administration shown in Tables 18-20. In the whole experimental process, the body weight and tumor size of the mice were measured twice a week.

The formula for calculating the tumor size: tumor volume ($mm^3$) = 0.5 $\times$ (tumor long diameter $\times$ tumor short diameter$^2$). The experimental results are shown in Tables 25, 26 and 27:

Table 25

| Group | Test compound | Tumor volume (day 15, $mm^3$) | **TGI** (%) | T/C(%) |
|---|---|---|---|---|
| G1 | Vehicle | 596.9±268.7 | N/A | N/A |
| G2 | C-87 | 108.2±83.6 | 82 | 18.13 |
| G3 | C-12 | 38.3±36.3 | 94 | 6.41 |
| G4 | C-30 | 14.5±8.2 | 98 | 2.43 |
| G5 | C-23 | 30.0±26.2 | 95 | 5.02 |
| G6 | C-19 | 23.6±17.4 | 96 | 3.95 |

Table 26

| Group | Test compound | Tumor volume (day 16, $mm^3$) | **TGI** (%) | T/C (%) |
|---|---|---|---|---|
| G1 | Vehicle | 2614.7±756.9 | N/A | N/A |
| G2 | C-65 | 76.2±23.6 | 97 | 2.9 |
| G3 | C-135 | 318.7±154.6 | 88 | 12.2 |

(continued)

| Group | Test compound | Tumor volume (day 16, $mm^3$) | **TGI** (%) | T/C (%) |
|---|---|---|---|---|
| G4 | C-130 | 162.0±125.0 | 94 | 6.2 |

Table 27

| Group | Test compound | Tumor volume (day 20, $mm^3$) | TGI (%) | T/C(%) |
|---|---|---|---|---|
| G1 | Vehicle | 2039.7±331.0 | N/A | N/A |
| G2 | Trastuzumab | 1100.4±311.3 | 46 | 53.9 |
| G3 | C-62 | 231.2±94.6 | 89 | 11.3 |

**Effect Example 6:** MTD experiment in the mouse subcutaneous xenograft model of MC38-HER2 mouse colon cancer cells

Cell culture: mouse colon cancer MC38-HER2 cells were maintained in monolayer culture in the DMEM medium containing 10% fetal bovine serum and 4 μg/mL puromycin in a constant temperature incubator containing 5% $CO_2$ at 37°C. The tumor cells were subcultured twice a week. The cells in the exponential growth phase were collected and counted for inoculation.

Experimental animals: B6-hTLR8-HO mice, 6-8 weeks, 18-22 g.

Experimental method: the MC38-HER2 cell line ($5.0 \times 10^6$ cells/animal) was inoculated subcutaneously on the right back of the experimental mice, with an inoculation volume of 0.1 mL for each mouse. The growth of the tumor was observed regularly. The mice were randomly grouped, with 3-5 mice in each group. On the day of grouping, the antibody-immunostimulatory conjugate of the present disclosure was administered by a single intraperitoneal dose at a dose of 50 mg/kg. The animals were examined for any effects of treatment on normal behaviors, such as mobility, visual estimation of food and water consumption, weight gain/loss, and any other abnormal effects. Deaths and observed clinical symptoms were recorded according to the number of the animals in each experimental group. In the whole experimental process, the body weight of the mice was measured twice a week.

The results showed that there was no animal death in all experimental groups, no obvious abnormality was observed clinically, and the maximum ratio of weight loss in the mice did not exceed 10%

## Claims

1. An antibody-immunostimulatory conjugate of formula I or a pharmaceutically acceptable salt thereof,

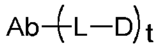

(I)

wherein Ab is an antibody;

t is any value from 1 to 8;
D is a benzazepine group as shown below:

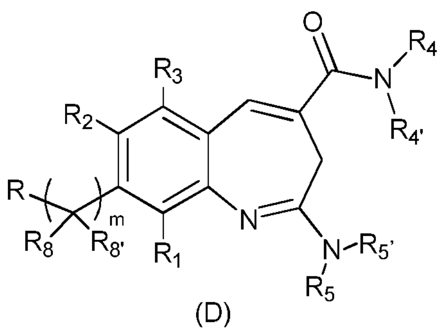

(D) ;

m is 0 or 1;
R is -C(O)-$NR_9$-$L_1$-$R_7$, -C(S)-$NR_9$-$L_1$-$R_7$ or -$NR_9$-C(O)-$L_1$-$R_7$;
$R_1$, $R_2$ and $R_3$ are each independently hydrogen, deuterium, halogen, hydroxyl, amino, cyano, alkyl, haloalkyl, -$L_2$-$OR_a$ or -$L_2$-$NR_aR_b$;
$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl or heteroarylalkyl; and the $R_4$ or $R_{4'}$ is unsubstituted or optionally substituted at any position with one or more substituents selected from halogen, cyano, -$L_2$-$OR_a$, -$L_2$-$OR_g$, -$L_2$-OC(O)$R_a$, -$L_2$-OC(O)$OR_a$, -$L_2$-OC(O)$NR_aR_b$, -$L_2$-$NR_aR_b$, -$L_2$-$NR_a$C(O)$OR_b$, -$L_2$-$NR_a$C(O)$NR_aR_b$, -$L_2$-$NR_b$C($NR_b$)$NR_aR_b$ and -$L_2$-C(O)$OR_b$; or $R_4$ and $R_{4'}$ together with the N atom to which they are attached form 3-8 membered heterocycloalkyl; and the 3-8 membered heterocycloalkyl is unsubstituted or further substituted at any position with 1 to 3 substituents selected from halogen, cyano, - $L_2$-$OR_a$, -$L_2$-OC(O)$R_a$, -$L_2$-$NR_aR_b$, -$L_2$-$NR_a$C(O)$OR_b$, -$L_2$-$NR_a$C(O)$NR_aR_b$, -$L_2$-$NR_b$C($NR_b$)$NR_aR_b$ and -$L_2$-C(O)$OR_b$;
$R_5$ is hydrogen, -$OR_a$, -C(O)$R_a$, -C(O)$OR_a$ or -C(O)$NR_aR_b$;
$R_{5'}$ is -$R_c$;
$R_7$ is phenyl, 5-6 membered heteroaryl, 3-8 membered heterocycloalkyl or an 8-12 membered fused ring group; and the $R_7$ is unsubstituted or optionally substituted at any position with one or more substituents selected from -$L_3$-W, -$R_c$, halogen, cyano, nitro, alkyl, alkenyl, alkynyl, alkoxy, haloalkyl, haloalkoxy and alkylamino;
$R_8$ and $R_{8'}$ are each independently hydrogen, halogen or alkyl, and the $R_8$ or $R_{8'}$ is unsubstituted or optionally substituted at any position with one or more substituents selected from -$L_3$-W, halogen, cyano, nitro, alkyl, alkenyl, alkynyl, alkoxy, haloalkyl, haloalkoxy and alkylamino; $R_8$ and $R_{8'}$ are each independently a substituent, or $R_8$ and $R_{8'}$ together with the carbon atom to which they are attached form oxo, thio, $C_{1-6}$ alkylene, $C_{3-10}$ cycloalkyl or 3-10 membered heterocycloalkyl; and the $C_{1-6}$ alkylene, $C_{3-10}$ cycloalkyl or 3-10 membered heterocycloalkyl is unsubstituted or optionally substituted at any position with one or more substituents selected from deuterium, halogen, hydroxyl, amino, cyano, oxo, alkyl, haloalkyl, -$L_2$-$OR_a$ and - $L_2$-$NR_aR_b$;
$R_9$ is hydrogen, alkyl, haloalkyl, -$L_2$-$OR_a$ or -$L_2$-$NR_aR_b$;
W is $Cy^1$, -$SR_d$, -$OR_d$, -OC(O)$R_e$, -OC(O)$NR_eR_{e'}$, -C(O)$OR_e$, -C(O)$R_e$, -C(O)$NR_eR_{e'}$, -C(O)$NR_e$S(O)$_2R_e$, -$NR_dR_e$, -$NR_d$C(O)$R_e$, -N($R_d$)C(O)$OR_e$, -N($R_d$)C(O)$NR_eR_{e'}$, -$NR_d$S(O)$_2R_e$, -$NR_d$S(O)$_2NR_eR_{e'}$, -S(O)$_{1-2}R_e$, -S(O)$_2NR_eR_{e'}$, -S(O)(=$NR_d$)$R_e$, -S(O)$_2$N($R_e$)C(O)$R_{e'}$, -P(O)($OR_e$)$_2$, -P(O)($OR_e$)$R_{e'}$, -OP(O)($OR_e$)$_2$ or - B($OR_e$)$_2$;
$Cy^1$ is cycloalkyl, heterocycloalkyl, aryl or heteroaryl; and the $Cy^1$ is unsubstituted or optionally substituted at any position with one or more substituents selected from halogen, alkyl, haloalkyl, haloalkoxy, alkenyl, alkynyl, cyano, -$R_c$, -$L_4$-$SR_d$, -$L_4$-OC(O)$R_e$, -$L_4$-C(O)$OR_e$, -$L_4$-C(O)$R_e$, -$L_4$-C(O)$NR_eR_{e'}$, -$L_4$-$NR_d$C(O)$R_e$, - $L_4$-$NR_d$S(O)$_2R_e$, -$L_4$-S(O)$_{1-2}R_e$, -$L_4$-S(O)$_2NR_eR_{e'}$, -$L_4$-$OR_d$ and -$L_4$-$NR_eR_{e'}$;
each $R_a$, $R_b$, $R_d$, $R_e$ and $R_{e'}$ is independently -$R_c$, amino, $C_{1-6}$ alkyl, $C_{2-6}$ alkenyl, $C_{2-6}$ alkynyl, $C_{3-10}$ cycloalkyl, 3-10 membered heterocycloalkyl, $C_{6-10}$ aryl, 5-10 membered heteroaryl, $C_{3-10}$ cycloalkyl $C_{1-6}$ alkyl, 3-10 membered heterocycloalkyl $C_{1-6}$ alkyl, phenyl $C_{1-6}$ alkyl or 5-10 membered heteroaryl $C_{1-6}$ alkyl; and the $R_a$, $R_b$, $R_d$, $R_e$ or $R_{e'}$ is unsubstituted or optionally substituted at any position with 1 to 3 substituents selected from -$L_4$-$OR_f$, -OC(O)-$L_4$-$R_f$, -$L_4$-$NR_fR_f$, halogen, cyano, nitro, $C_{1-6}$ alkyl, halo $C_{1-6}$ alkyl and halo $C_{1-6}$ alkoxy;
$L_1$, $L_2$, $L_3$ and $L_4$ are each independently a linkage bond, $C_{1-6}$ alkylene, $C_{2-6}$ alkenylene, $C_{2-6}$ alkynylene or a polyethylene glycol fragment comprising 1 to 20 -$OCH_2CH_2$- units; and the $L_1$, $L_2$, $L_3$ or $L_4$ is unsubstituted or optionally substituted at any position with one or more substituents selected from oxo, hydroxyl, amino, halogen, cyano, alkyl, haloalkyl, alkoxy and haloalkoxy;
each $R_f$ and $R_f$ is independently -$R_c$, -$NHR_c$ or $C_{1-6}$ alkyl;
$R_g$ is a prodrug group comprising phosphoryl; and
each $R_c$ is independently hydrogen or a linkage bond attached to L; and at least one $R_c$ in the D is a linkage bond attached to L;
and the benzazepine group of formula D satisfies 1 or 2 of conditions of:

(1) m is 1; and
(2) at least one of $R_4$ and $R_4$ is substituted at any position with one or more substituents selected from halogen, cyano, -$L_2$-$OR_a$, -$L_2$-$OR_g$, -$L_2$-OC(O)$R_a$, -$L_2$-OC(O)$OR_a$, -$L_2$-OC(O)$NR_aR_b$, -$L_2$-$NR_aR_b$, -$L_2$-$NR_a$-C(O)$OR_b$, -$L_2$-C(O)$OR_b$, -$L_2$-$NR_a$C(O)$NR_aR_b$ and -$L_2$-$NR_b$C($NR_b$)$NR_aR_b$;

L is -M-(T)$_w$-PEG-(T)$_o$-*, -M-PEG-*, -M-(T)$_w$-*, -M-(T)$_w$-(A)$_v$-(T)$_o$-PEG-*, -M-(T)$_w$-(A)$_v$-(T)$_o$-*, -M-(T)$_w$-$L_5$-*, -M-(T)$_w$-PEG-$L_5$-*, -M-(T)$_w$-PEG-(A)$_v$-$L_5$-*, -M-(T)$_w$-PEG-(A)$_v$-(T)$_o$-*, -M-PEG-(T)$_o$-PEG-* or -M-(T)$_w$-(A)$_v$-$L_5$-*; v is any integer from 1 to 5; w is any integer from 1 to 10; o is any integer from 0 to 10; and * is a linking site of the L that is attached to the D;
each T is independently -($CH_2$)$_x$-C(O)-, -$NR_h$-, -O-, -S-, -($CH_2$)$_x$-arylene-($CH_2$)$_y$-, -($CH_2$)$_x$-heteroarylene-($CH_2$)$_y$-, -($CH_2$)$_x$-cycloalkylene-($CH_2$)$_y$-, -($CH_2$)$_x$-heterocycloalkylene-($CH_2$)$_y$-, -$NR_h$-($CH_2$)$_x$-C(O)-,

-O-$(CH_2)_x$-C(O)-, -S-$(CH_2)_x$-C(O)-, -$(CH_2)_x$CH($NHR_h$)-C(O)-, -$(CH_2)_x$-S-S-alkylene-, alkylene or alkenylene;

and x and y are each independently any integer from 0 to 10;

$R_h$ is hydrogen, alkyl, -$PO(OH)_2$, -$PO(OCH_3)_2$, -C(O)-$(CH_2CH_2O)_n$-$CH_3$ or -$(CH_2CH_2O)_n$-$CH_3$;

PEG is -$(CH_2CH_2O)_n$-$(CH_2)_u$- or -$(CH_2CH_2O)_n$-$(CH_2)_u$-C(O)-; n is any integer from 1 to 50; and u is any integer from 0 to 5;

each A is independently an amino acid residue or an amino acid residue modified by -C(O)-$(CH_2CH_2O)_n$-$CH_3$ or -$(CH_2CH_2O)_n$-$CH_3$;

$L_5$ is a self-immolative group; and

M is a linkage bond or a linker head attached to an antibody.

**2.** The antibody-immunostimulatory conjugate of formula I or the pharmaceutically acceptable salt thereof according to claim 1,

$$Ab\!-\!\left(L-D\right)_t$$

(I)

wherein Ab is an antibody;

t is any numerical value from 1 to 8;

L is a linker attached to the Ab and the D; and

the D is a group formed by loss of one hydrogen atom in a compound of formula D';

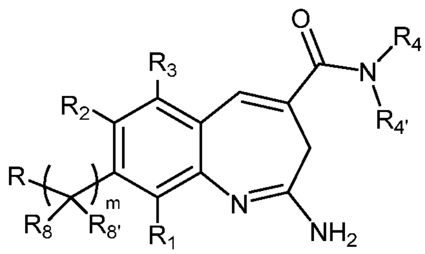

D'

wherein R is -L'-$L_1$-$R_7$; and L' is -C(=O)-, -C(=O)-$NR_9$- or -C(=S)-$NR_9$-;

$R_9$ is independently hydrogen, $C_{1-6}$ alkyl or halo $C_{1-6}$ alkyl;

$L_1$ is independently a linkage bond, $C_{1-6}$ alkylene or $C_{2-6}$ alkenylene;

$R_7$ is independently phenyl, 5-6 membered heteroaryl, 3-8 membered heterocycloalkyl, an 8-12 membered fused ring group, phenyl substituted with 1, 2 or 3 $R_{7-1}$, 5-6 membered heteroaryl substituted with 1, 2 or 3 $R_{7-2}$, 3-8 membered heterocycloalkyl substituted with 1, 2 or 3 $R_{7-3}$ or an 8-12 membered fused ring group substituted with 1, 2 or 3 $R_{7-4}$;

in $R_7$, in "5-6 membered heteroaryl" in the 5-6 membered heteroaryl and the 5-6 membered heteroaryl substituted with 1, 2 or 3 $R_{7-2}$, the heteroatom is independently selected from one or more of N, O and S, and the number of the heteroatom is independently 1, 2 or 3; and in "3-8 membered heterocycloalkyl" in the 3-8 membered heterocycloalkyl and the 3-8 membered heterocycloalkyl substituted with 1, 2 or 3 $R_{7-3}$, the heteroatom is independently selected from one or more of N, O and S, and the number of the heteroatom is independently 1, 2 or 3;

in the $R_7$, an 8-12 membered fused ring group in the 8-12 membered fused ring group and the 8-12 membered fused ring group substituted with 1, 2 or 3 $R_{7-4}$ is independently a fused ring of ring A and ring B, the ring A is a 5-6 membered heteroaryl ring or a phenyl ring, and the ring B is a 5-6 membered heteroalkenyl ring; in the 5-6 membered heteroalkenyl ring, the heteroatom is selected from one or more of N, O and S, and the number of the heteroatom is 1, 2 or 3; and in the 5-6 membered heteroalkenyl, the heteroatom is selected from one or more of N, O and S, and the number of the heteroatom is 1, 2 or 3;

$R_{7-1}$, $R_{7-2}$, $R_{7-3}$ and $R_{7-4}$ are independently amino, -$L_3$-$NH_2$, -$L_3$-OH, -C(=O)-$L_3$-OH or -C(=O)-$L_3$-$NH_2$, and $L_3$ is independently $C_{1-6}$ alkylene or -$(CH_2CH_2O)_{m'}$-$C_{1-6}$ alkylene-; and m' is 1, 2, 3, 4, 5, 6, 7, 8, 9 or 10; m is 0 or 1;

$R_8$ and $R_{8'}$ are independently hydrogen or $C_{1-6}$ alkyl, or $R_8$ and $R_{8'}$ together with the carbon atom to which they are attached form carbonyl, $C_{3-10}$ cycloalkylene or 3-10 membered heterocycloalkylene; and in the 3-10 membered heterocycloalkylene, the heteroatom is selected from one or more of N, O and S, and the number of the heteroatom is 1, 2 or 3;

$R_1$, $R_2$ and $R_3$ are independently hydrogen, deuterium or halogen;
$R_4$ and $R_{4'}$ are independently $C_{1-6}$ alkyl, $C_{1-6}$ alkoxy or $C_{1-6}$ alkyl substituted with 1, 2 or 3 $R_{4-1}$; and the $R_{4-1}$ is independently hydroxyl, amino, -OC(=O)NR$_a$R$_b$ or

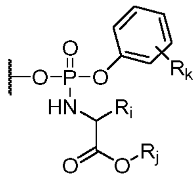
;

$R_a$ and $R_b$ are independently hydrogen, $C_{1-6}$ alkyl or $C_{3-10}$ cycloalkyl;
$R_i$ is hydrogen, $C_{1-6}$ alkyl or benzyl; $R_k$ is hydrogen, halogen or $C_{1-6}$ alkyl; and $R_j$ is $C_{1-6}$ alkyl, $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; and
in the $R_j$, in "3-10 membered heterocycloalkyl" in the 3-10 membered heterocycloalkyl and the 3-10 membered heterocycloalkyl-$C_{1-6}$ alkyl, the heteroatom is independently selected from one or more of N, O and S, and the number of the heteroatom is independently 1, 2 or 3; and in "5-10 membered heteroaryl" in the 5-10 membered heteroaryl and the 5-10 membered heteroaryl-$C_{1-6}$ alkyl, the heteroatom is independently selected from one or more of N, O and S, and the number of the heteroatom is independently 1, 2 or 3;
and the compound of formula D' satisfies 1 or 2 of conditions of:

(1) m is 1; and
(2) at least one of $R_4$ and $R_{4'}$ is $C_{1-6}$ alkoxy or $C_{1-6}$ alkyl substituted with 1, 2 or 3 $R_{4-1}$;

and the antibody-immunostimulatory conjugate of formula I is not an antibody-immunostimulatory conjugate shown in Table 1, wherein mAb is Trastuzumab:

➳Too large Table

Table 1

| Structure | t |
|---|---|
| 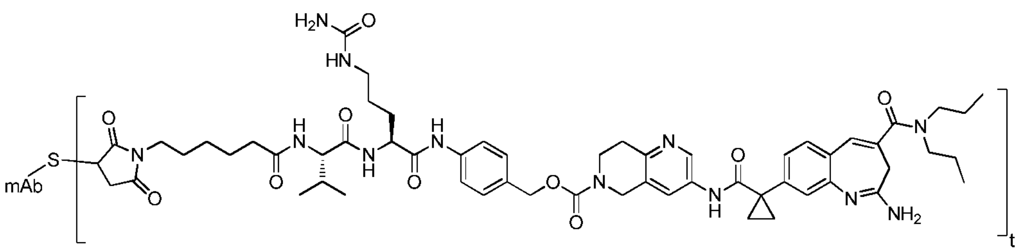 | 4.14 |
| 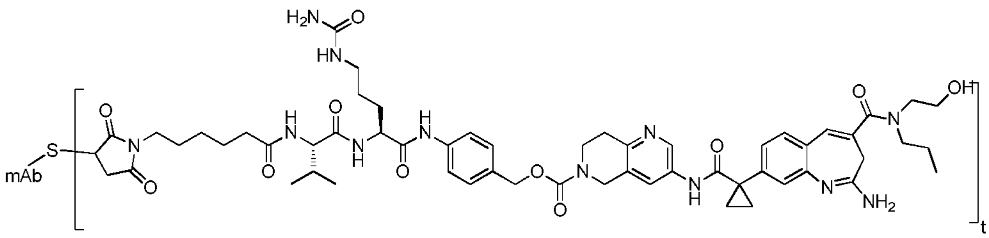 | 4.57 |
| 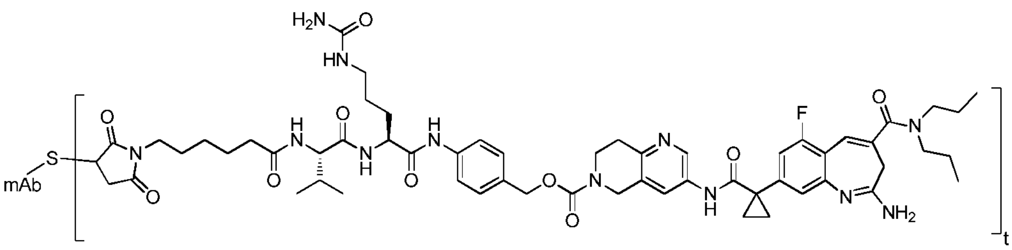 | 4.14 |

(continued)

| Structure | t |
|---|---|
| 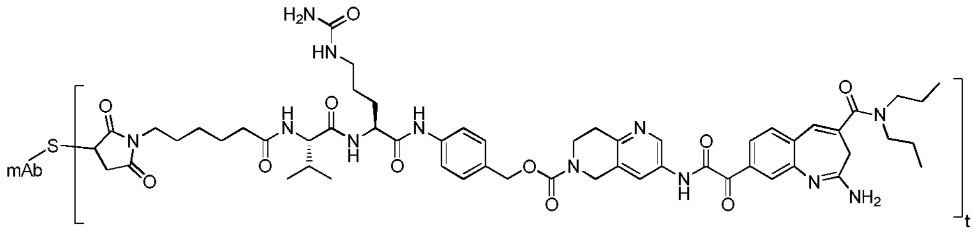 | 4.23 |
| 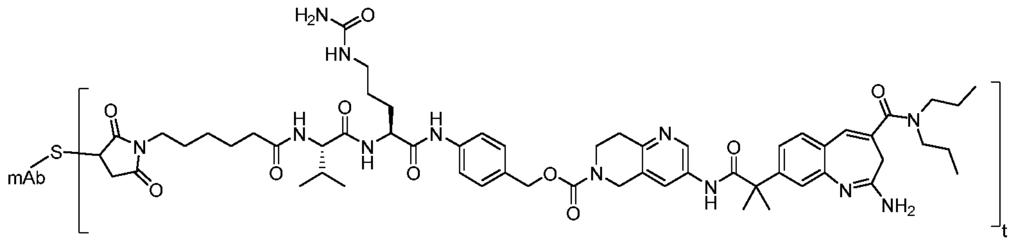 | 4.29 |
| 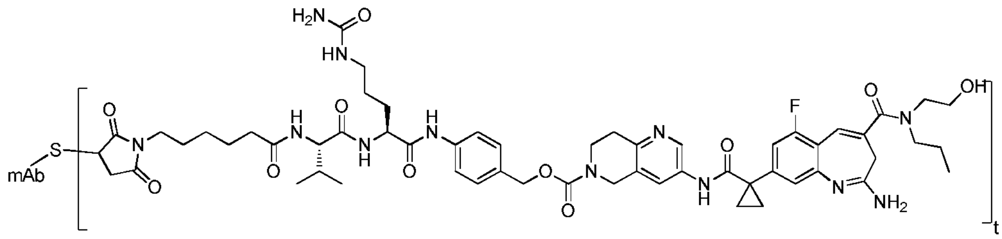 | 3.93 |
| 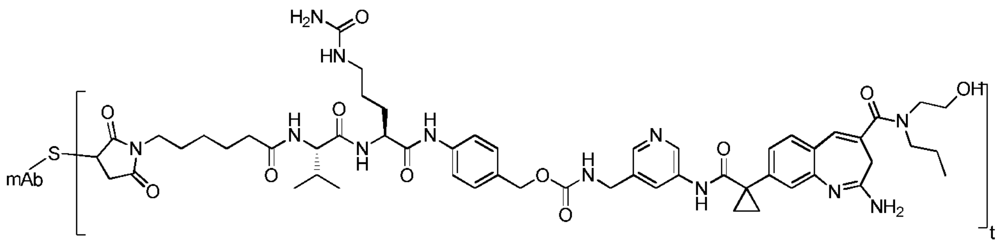 | 4.72 |
| 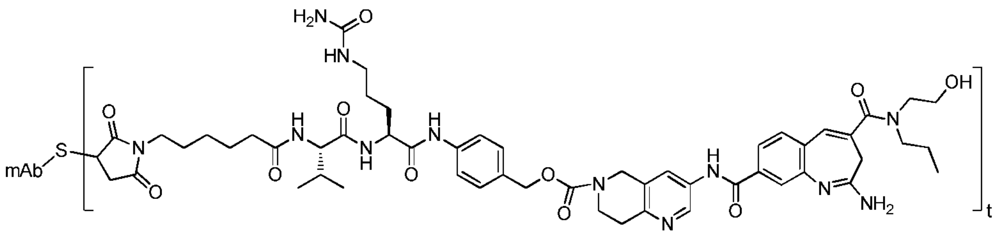 | 4.61 |
| 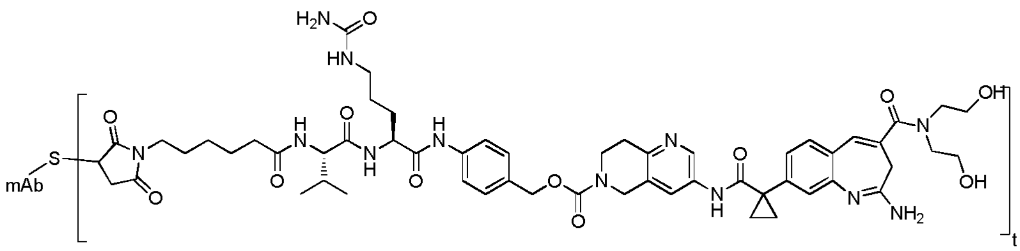 | 4.45 |

(continued)

| Structure | t |
|---|---|
| 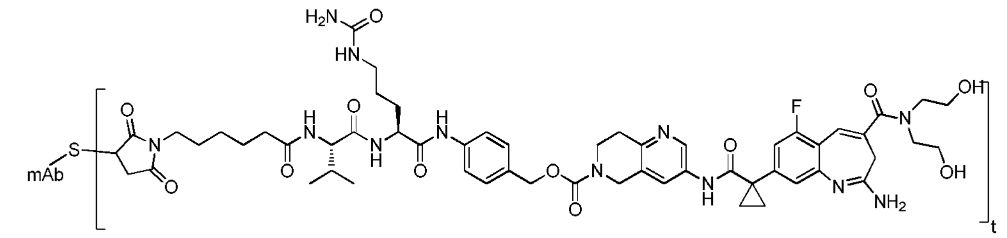 | 4.39 |
| 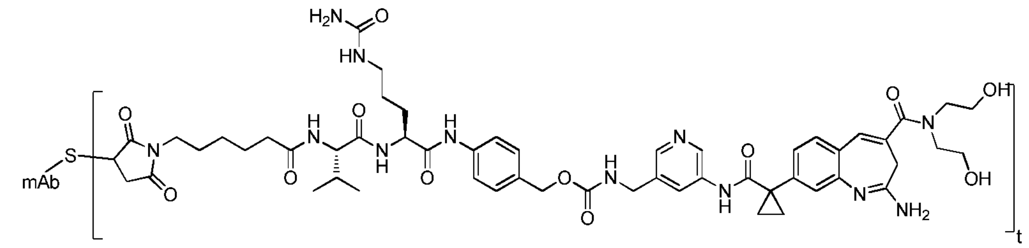 | 4.35 |
| 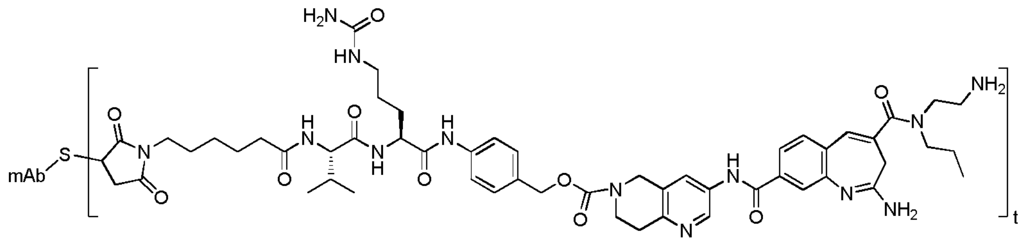 | 3.21 |
| 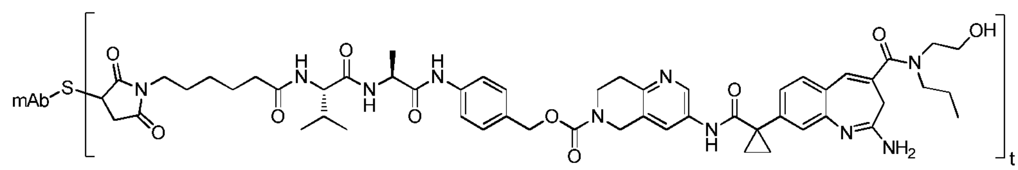 | 4.03 |
| 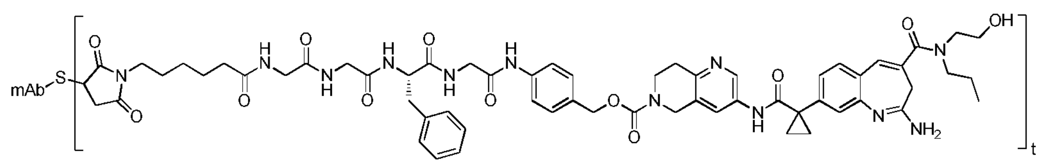 | 3.86 |
| 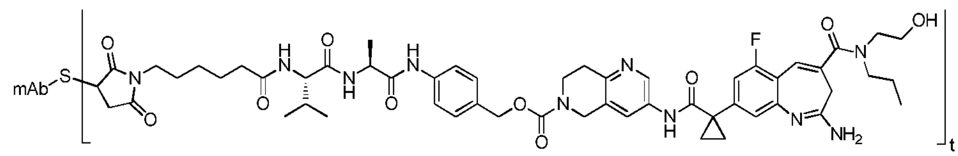 | 3.98 |
| 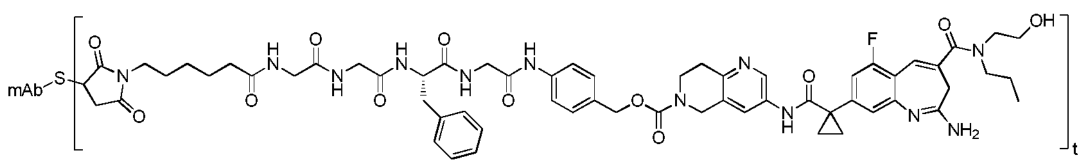 | 4.03 |
| 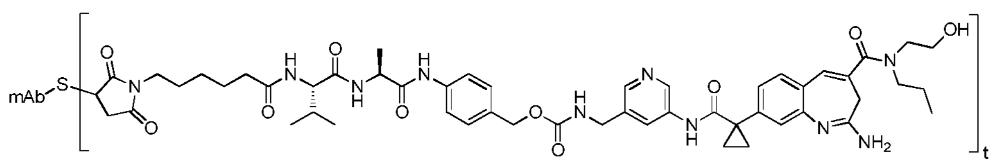 | 4.12 |

(continued)

| Structure | t |
|---|---|
| 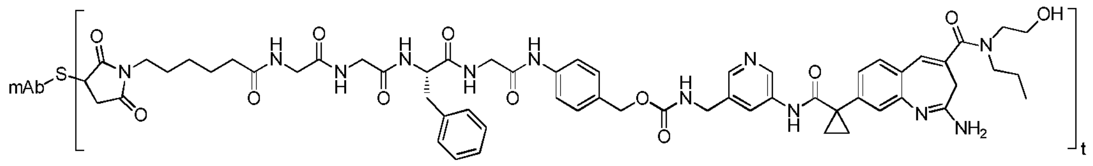 | 4.06 |

3. The antibody-immunostimulatory conjugate of formula I or the pharmaceutically acceptable salt thereof according to claim 1 or 2, satisfying one or more of conditions of:

(1) the antibody-immunostimulatory conjugate of formula I is not an antibody-immunostimulatory conjugate shown in Table 2, wherein mAb is a monoclonal antibody, and t is any value from 1 to 8:

Table 2

| |
|---|
| 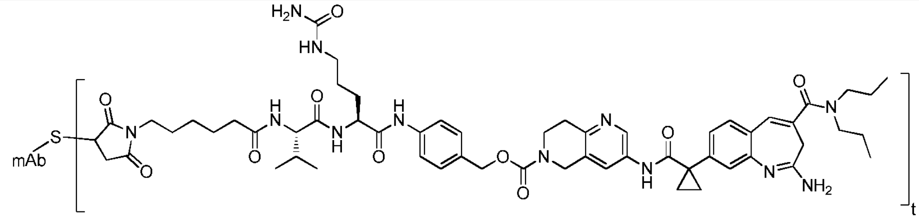 |
| 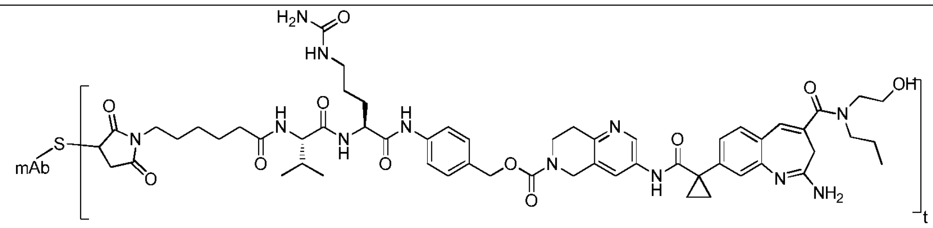 |

| |
|---|
| 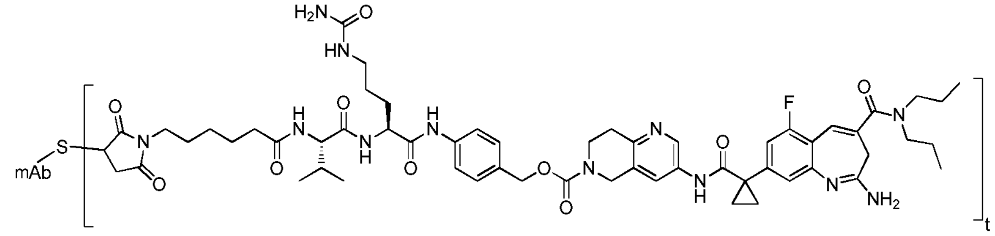 |
| 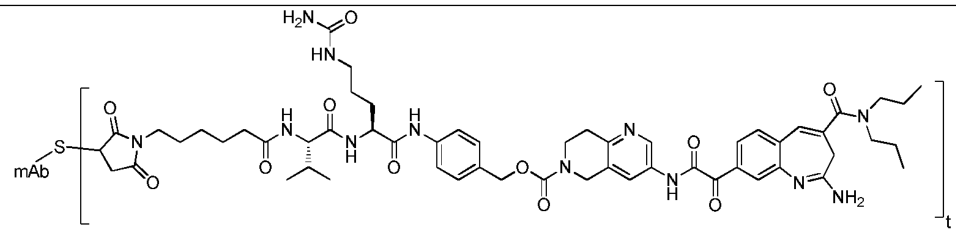 |
| 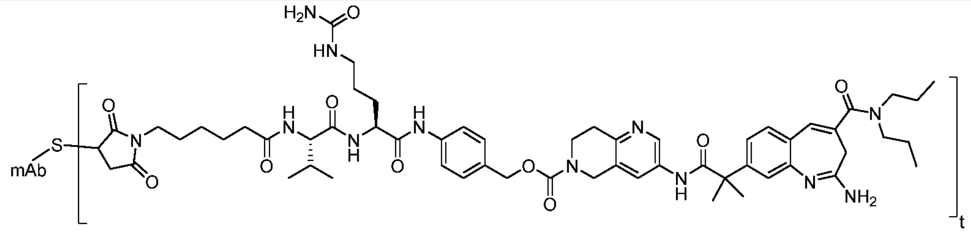 |
| 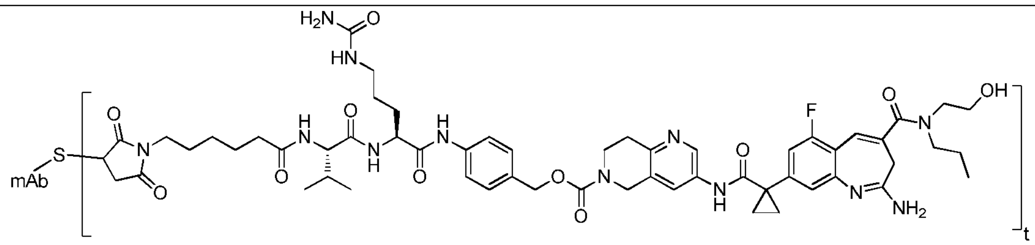 |
| 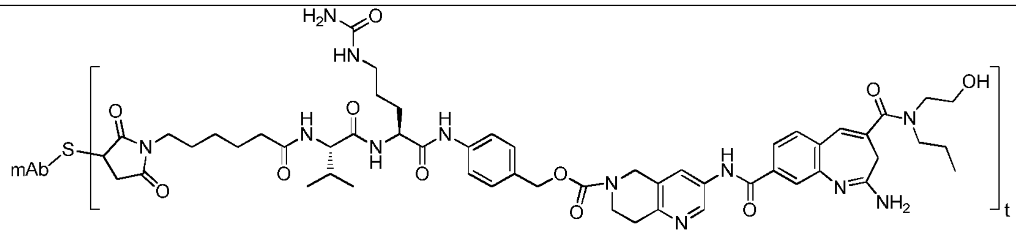 |
| 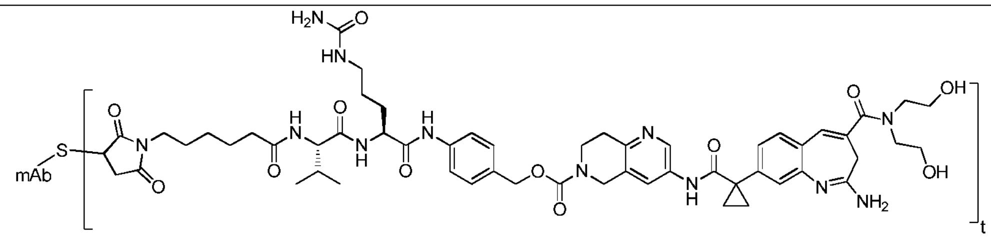 |
| 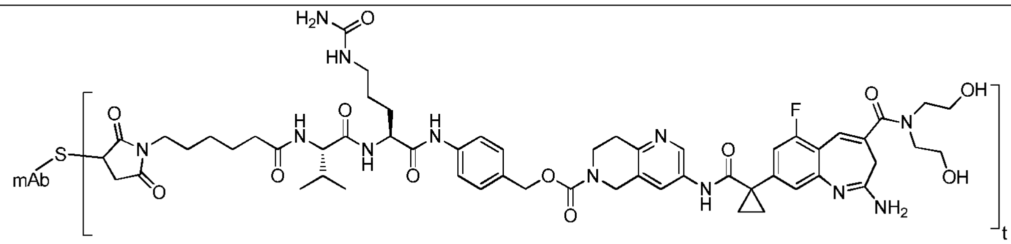 |

| |
|---|
| 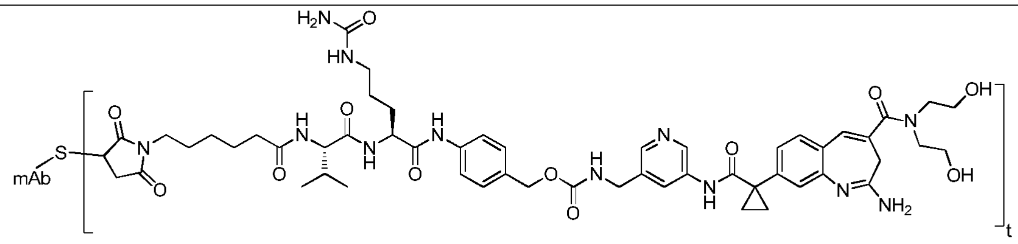 |
| 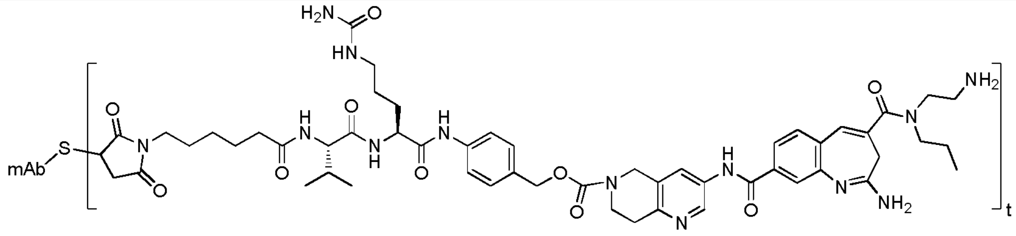 |
| 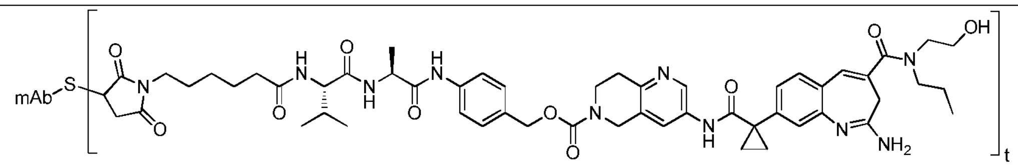 |
| 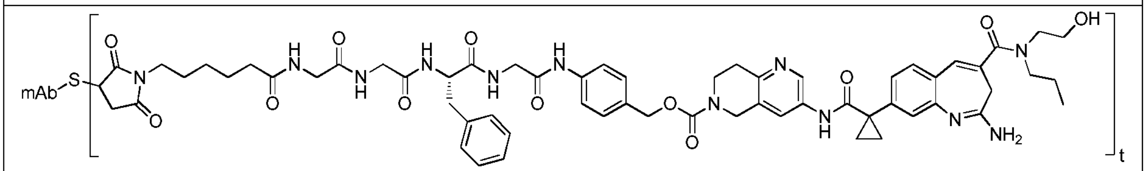 |
| 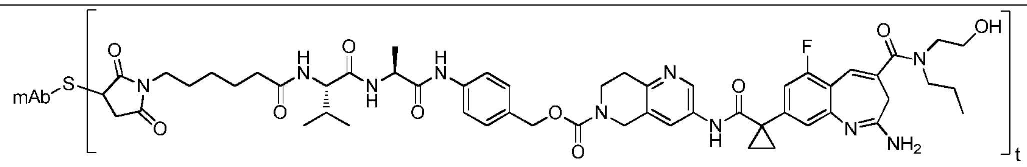 |
| 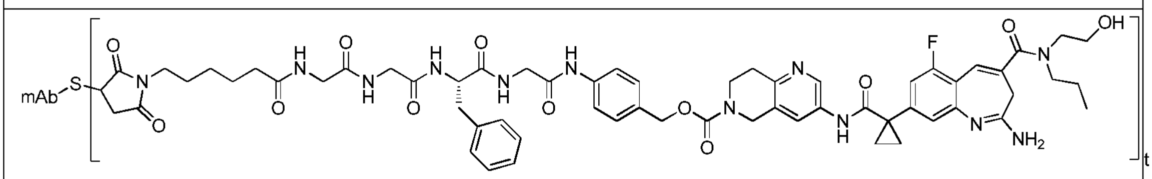 |
| 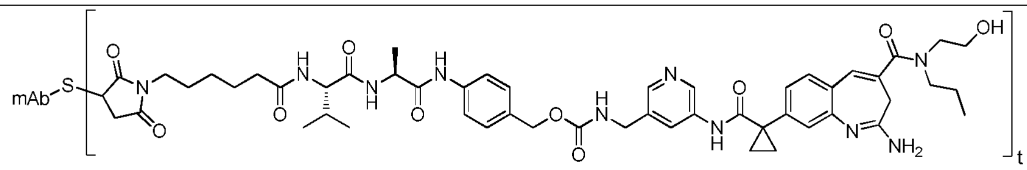 |

(2) the compound of formula D' is not a compound shown in Table 3:

Table 3

| | |
|---|---|
| 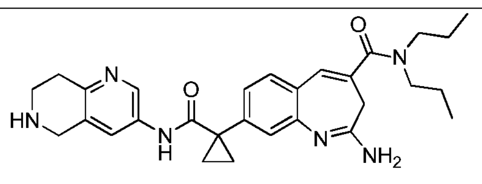 | 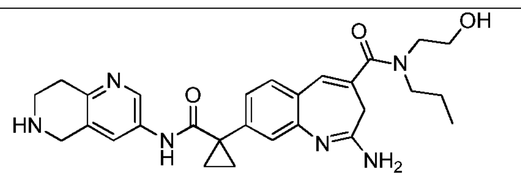 |

| | |
|---|---|
| 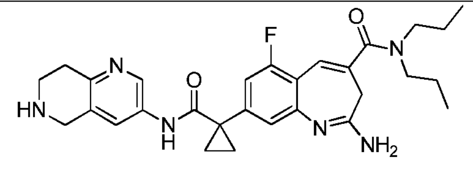 | 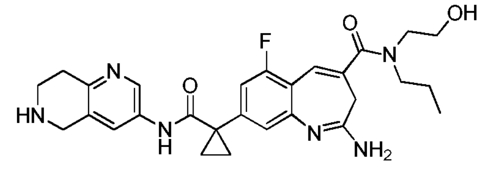 |
| 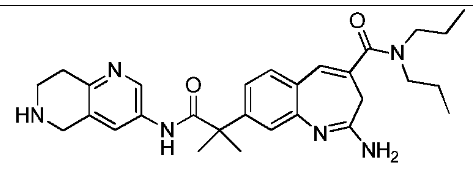 | 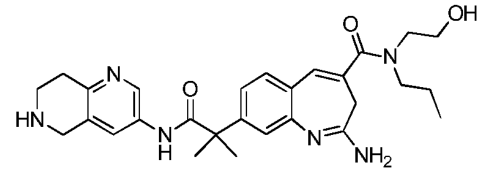 |
| 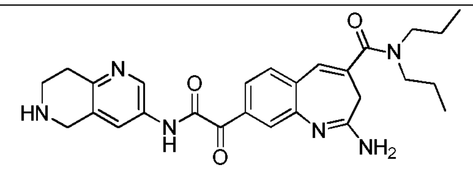 | 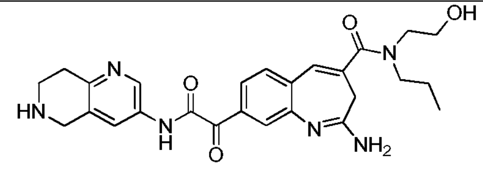 |
| 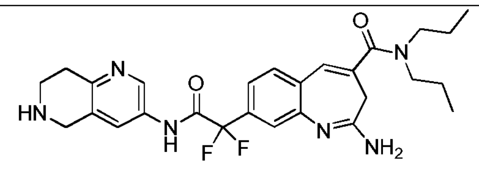 | 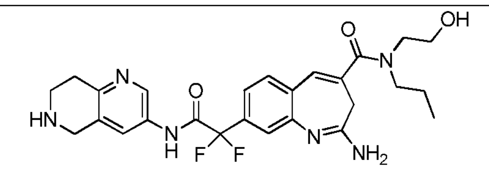 |
| 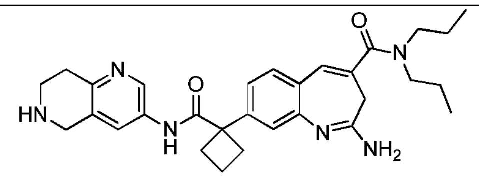 | 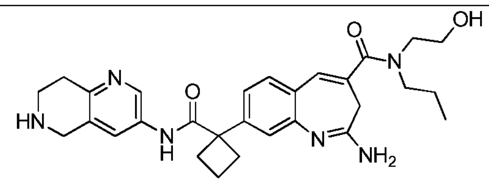 |
| 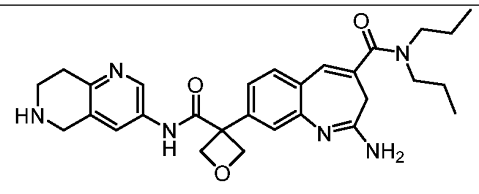 | 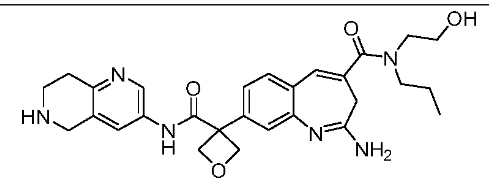 |
| 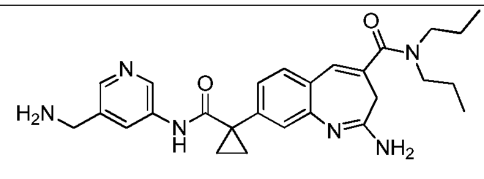 | 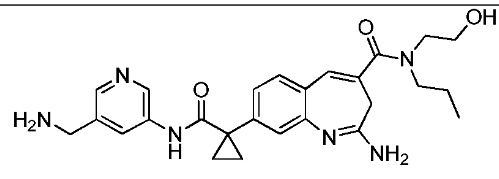 |
| 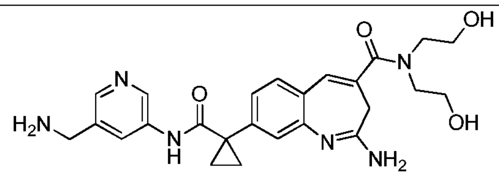 | 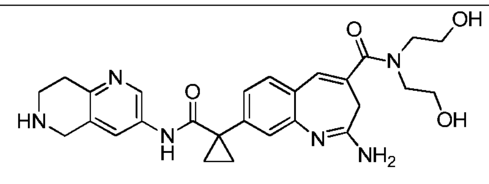 |
| 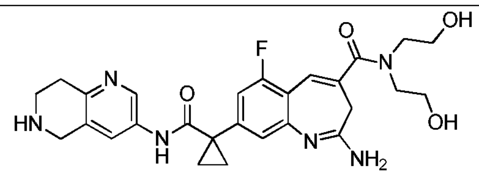 | 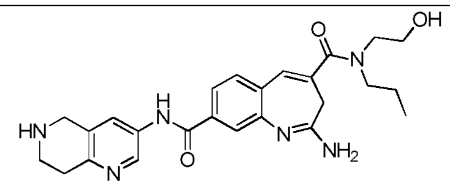 |

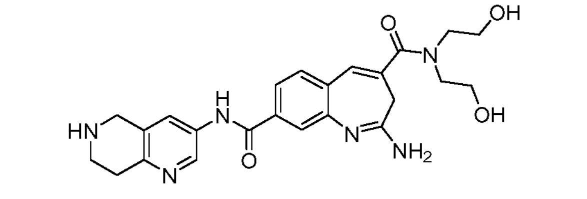

; and

(3) the linker is not of any one of structures shown in Table 4:

Table 4

| | |
|---|---|
| 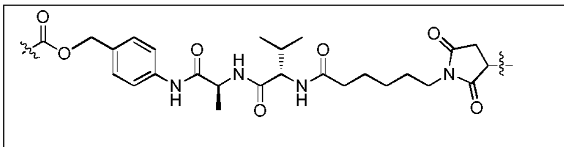 | 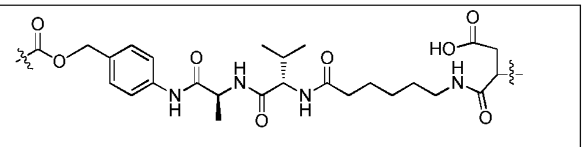 |
| 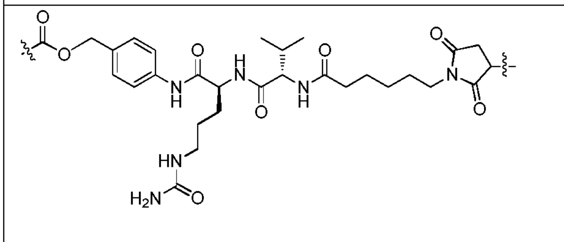 | 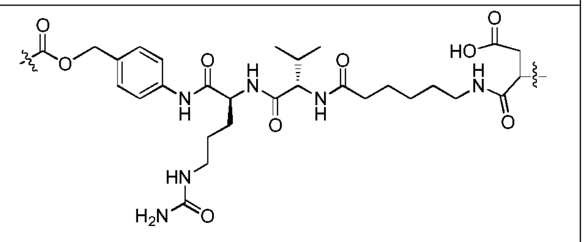 |
| 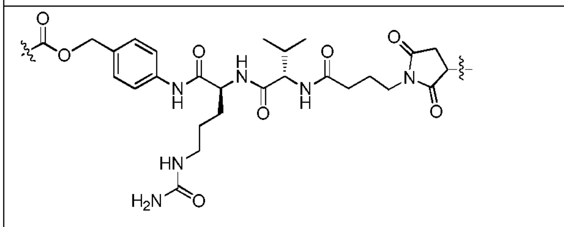 | 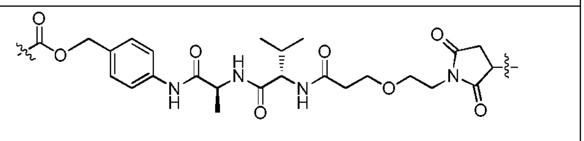 |
| 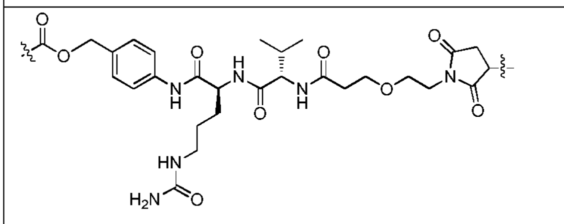 | 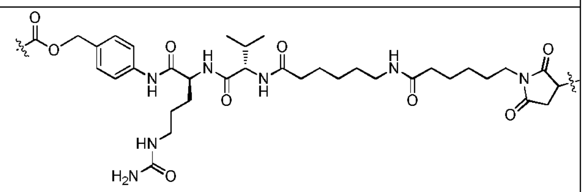 |
| 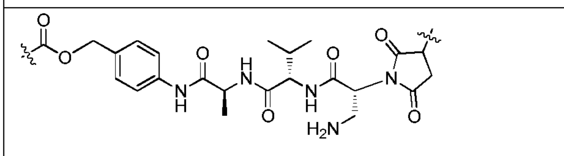 | 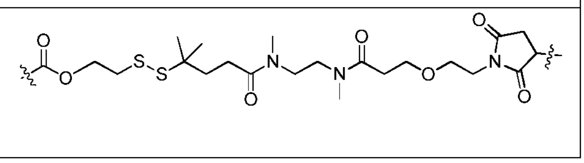 |
| 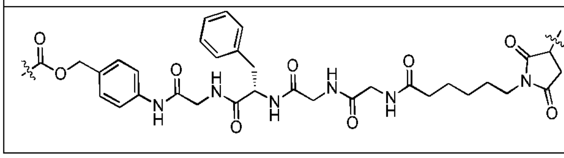 | |

**4.** The antibody-immunostimulatory conjugate of formula I or the pharmaceutically acceptable salt thereof according to claim 3, satisfying a condition (1) and/or a condition (2) of:

(1) the compound of formula D' satisfies 1, 2 or 3 of conditions of: (a) $L_1$ is independently $C_{1-6}$ alkylene or $C_{2-6}$ alkenylene; (b) $R_7$ is independently phenyl substituted with one amino, 5-6 membered heteroaryl substituted with one amino, 3-8 membered heterocycloalkyl substituted with 1 amino or an 8-12 membered fused ring group substituted with one amino; and (c) at least one of $R_4$ and $R_{4'}$ is $C_{1-6}$ alkoxy or $C_{1-6}$ alkyl substituted with 1, 2 or 3 $R_{4-1}$; and the $R_{4-1}$ is independently $-OC(=O)NR_aR_b$ or

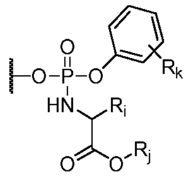

;

(2) the L is $-M-(T)_w-PEG-(T)_o-^*$, $-M-PEG-^*$, $-M-(T)_w-(A)_v-(T)_o-PEG-^*$, $-M-(T)_w-(A)_v-(T)_o-^*$, $-M-(T)_w-L_5-^*$, $-M-(T)_w-PEG-L_5-^*$, $-M-(T)_w-PEG-(A)_v-L_5-^*$, $-M-(T)_w-PEG-(A)_v-(T)_o-^*$ or $-M-PEG-(T)_o-PEG-^*$; and * is a linking site of the L that is attached to the D;

M is a linkage bond or a linker head attached to Ab;
w is independently 1, 2, 3, 4, 5, 6, 7, 8, 9 or 10;
o is independently 0, 1, 2, 3, 4, 5, 6, 7, 8, 9 or 10;
T is independently $-(CH_2)_x-C(O)-$, $-NR_h-$, -O-, -S-, $-(CH_2)_x-C_{6-14}$ arylene-$(CH_2)_y-$, $-(CH_2)_x$-5-6 membered heteroarylene-$(CH_2)_y-$, $-(CH_2)_x-C_{3-6}$ cycloalkylene-$(CH_2)_y-$, $-(CH_2)_x$-3-6 membered heterocycloalkylene-$(CH_2)_y-$, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x-S-S-C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the $-(CH_2)_x$-5-6 membered heteroarylene-$(CH_2)_y-$, the heteroatom is selected from one or more of N, O and S, and the number of the heteroatom is 1, 2 or 3; and in "3-6 membered heterocycloalkylene" in the $-(CH_2)_x$-3-6 membered heterocycloalkylene-$(CH_2)_y-$, the heteroatom is selected from one or more of N, O and S, and the number of the heteroatom is 1, 2 or 3;
$R_h$ is independently hydrogen, $C_{1-6}$ alkyl, $-PO(OH)_2$, $-PO(OCH_3)_2$, $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;
PEG is $-(CH_2CH_2O)_n-(CH_2)_u-$ or $-(CH_2CH_2O)_n-(CH_2)_u-C(O)-$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;
v is independently 1, 2, 3, 4 or 5;
A is independently an amino acid residue or an amino acid residue substituted with one $R_A$; the $R_A$ is independently $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and
$L_5$ is a self-immolative group.

**5.** The antibody-immunostimulatory conjugate of formula I or the pharmaceutically acceptable salt thereof according to claim 1 or 2, satisfying one or more of conditions of:

(1) the antibody comprises one or more antigen-binding domains capable of binding to an antigen;
(2) the antibody comprises one Fc-region;
(3) the antibody is a monoclonal antibody;
(4) the t is any value from 2 to 5;
(5) L' is -C(=O)-, $-C(=O)-NR_9-$ or $-C(=S)-NR_9-$, and a right end thereof is attached to $L_1$;
(6) $R_9$ is hydrogen;
(7) in the $L_1$, the $C_{1-6}$ alkylene is methylene or ethylene;
(8) in the $R_7$, the 8-12 membered fused ring group is a fused ring of ring A and ring B, the ring A is 5-6 membered heteroaryl or phenyl, and the ring B is a 5-6 membered heteroalkenyl ring attached to the $L_1$ through the ring A; in the 5-6 membered heteroaryl, the heteroatom is N, and the number of the heteroatom is 1 or 2; and in the 5-6 membered heteroalkenyl ring, the heteroatom is N, and the number of the heteroatom is 1 or 2;
(9) in the $R_7$, in "5-6 membered heteroaryl" in the 5-6 membered heteroaryl and the 5-6 membered heteroaryl substituted with 1, 2 or 3 $R_{7-2}$, the heteroatom is independently N;
(10) in the $R_7$, in "5-6 membered heteroaryl" in the 5-6 membered heteroaryl and the 5-6 membered heteroaryl substituted with 1, 2 or 3 $R_{7-2}$, the number of the heteroatom is independently 1 or 2;
(11) in the ring A, the heteroatom in the 5-6 membered heteroaryl ring is N;
(12) in the ring A, the number of the heteroatom in the 5-6 membered heteroaryl ring is 1 or 2;
(13) in the ring B, the heteroatom in the 5-6 membered heteroalkenyl ring is N;
(14) one hydrogen atom lost by the compound of formula D' is located on a secondary amine or a primary amine of

$R_7$; or one hydrogen atom lost by the compound of formula D' is located on hydroxyl of $R_7$;
(15) m is 1;
(16) in the $R_8$ and $R_{8'}$, the $C_{1-6}$ alkyl is methyl, ethyl, n-propyl or isopropyl;
(17) $R_8$ and $R_{8'}$ together with the carbon atom to which they are attached form $C_{3-10}$ cycloalkylene, and the $C_{3-10}$ cycloalkylene is cyclopropyl or cyclobutyl;
(18) $R_8$ and $R_{8'}$ together with the carbon atom to which they are attached form 3-10 membered heterocycloalkylene, and a heteroatom in the 3-10 membered heterocycloalkylene is N or O;
(19) $R_8$ and $R_{8'}$ together with the carbon atom to which they are attached form 3-10 membered heterocycloalkylene, and the number of the heteroatom in the 3-10 membered heterocycloalkylene is 1 or 2;
(20) in the $R_1$, $R_2$ and $R_3$, the halogen is F, Cl or Br;
(21) in the $R_4$ and $R_{4'}$, the $C_{1-6}$ alkyl is methyl, ethyl, n-propyl or isopropyl;
(22) in the $R_4$ and $R_{4'}$, the $C_{1-6}$ alkoxy is methoxy, ethoxy, n-propoxy or isopropoxy;
(23) in the $R_4$ and $R_{4'}$, $C_{1-6}$ alkyl in the $C_{1-6}$ alkyl substituted with 1, 2 or 3 $R_{4-1}$ is methyl, ethyl, n-propyl or isopropyl;
(24) in the $R_a$ and $R_b$, the $C_{1-6}$ alkyl is methyl, ethyl, n-propyl or isopropyl;
(25) in the $R_a$ and $R_b$, the $C_{3-10}$ cycloalkyl is cyclopropyl, cyclobutyl or cyclopentyl;
(26) in the $R_i$, the $C_{1-6}$ alkyl is methyl, ethyl, n-propyl or isopropyl;
(27) in the $R_k$, the $C_{1-6}$ alkyl is methyl, ethyl, n-propyl or isopropyl;
(28) in the $R_j$, the $C_{1-6}$ alkyl is methyl, ethyl, n-propyl or isopropyl;
(29) in the $R_j$, in the $C_{6-10}$ aryl-$C_{1-6}$ alkyl, the $C_{6-10}$ aryl is phenyl;
(30) in the $R_j$, in the $C_{6-10}$ aryl-$C_{1-6}$ alkyl, the $C_{1-6}$ alkyl is methyl, ethyl, n-propyl or isopropyl; and
(31) the L is $-M-(T)_w-PEG-(T)_o-^*$, $-M-PEG-^*$, $-M-(T)_w-^*$, $-M-(T)_w-(A)_v-(T)_o-PEG-^*$, $-M-(T)_w-(A)_v-(T)_o-^*$, $-M-(T)_w-L_5-^*$, $-M-(T)_w-PEG-L_5-^*$, $-M-(T)_w-PEG-(A)_v-L_5-^*$, $-M-(T)_w-PEG-(A)_v-(T)_o-^*$, $-M-PEG-(T)_o-PEG-^*$ or $-M-(T)_w-(A)_v-L_5-^*$; and * is a linking site of the L that is attached to the D;

M is a linkage bond or a linker head attached to Ab;
w is independently 1, 2, 3, 4, 5, 6, 7, 8, 9 or 10;
o is independently 0, 1, 2, 3, 4, 5, 6, 7, 8, 9 or 10;
T is independently $-(CH_2)_x-C(O)-$, $-NR_h-$, -O-, -S-, $-(CH_2)_x-C_{6-14}$ arylene-$(CH_2)_y-$, $-(CH_2)_x-$5-6 membered heteroarylene-$(CH_2)_y-$, $-(CH_2)_x-C_{3-6}$ cycloalkylene-$(CH_2)_y-$, $-(CH_2)_x-$3-6 membered heterocycloalkylene-$(CH_2)_y-$, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x-S-S-C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the $-(CH_2)_x-$5-6 membered heteroarylene-$(CH_2)_y-$, the heteroatom is selected from one or more of N, O and S, and the number of the heteroatom is 1, 2 or 3; and in "3-6 membered heterocycloalkylene" in the $-(CH_2)_x-$3-6 membered heterocycloalkylene-$(CH_2)_y-$, the heteroatom is selected from one or more of N, O and S, and the number of the heteroatom is 1, 2 or 3;
$R_h$ is independently hydrogen, $C_{1-6}$ alkyl, $-PO(OH)_2$, $-PO(OCH_3)_2$, $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;
PEG is $-(CH_2CH_2O)_n-(CH_2)_u-$ or $-(CH_2CH_2O)_n-(CH_2)_u-C(O)-$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;
v is independently 1, 2, 3, 4 or 5;
A is independently an amino acid residue or an amino acid residue substituted with one $R_A$; the $R_A$ is independently $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and
$L_5$ is a self-immolative group.

**6.** The antibody-immunostimulatory conjugate of formula I or the pharmaceutically acceptable salt thereof according to claim 5, satisfying one or more of conditions of:

(1) the antibody is an anti-HER2 antibody, for example, Trastuzumab, Trastuzumab biosimilar, Pertuzumab, Pertuzumab biosimilar, Margetuximab or HT-19;
(2) the antibody is an anti-5T4 antibody, for example, huA1;
(3) the antibody is an anti-EGFR antibody, for example, Cetuximab;
(4) the antibody is an anti-Trop-2 antibody, for example, Sacituzumab;
(5) the antibody is an anti-Claudin18.2 antibody, Zolbetuximab;

(6) the antibody is an anti-CEACAM5 antibody, Labetuzumab;
(7) the antibody is an anti-Nectin-4 antibody, for example, Enfortumab;
(8) $R_7$ is

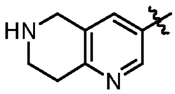, 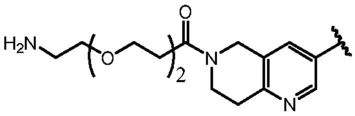, 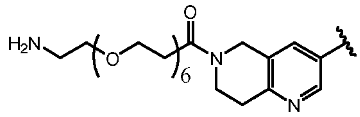,

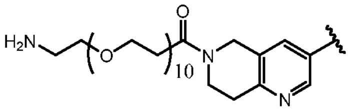, 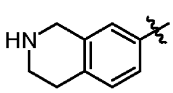, 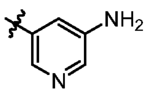, 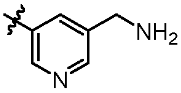, 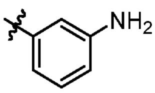,

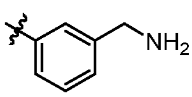, 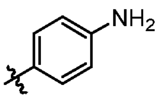, 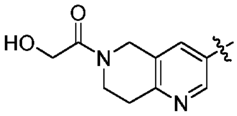 or 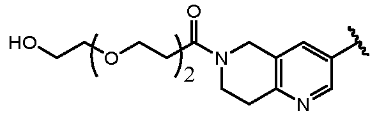;

(9) in the M, the linker head is

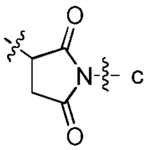, 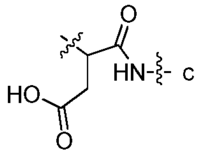, 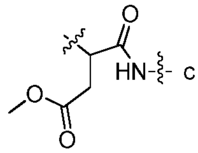, 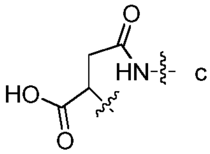,

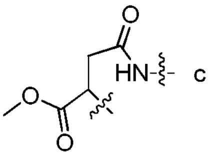, 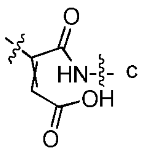, 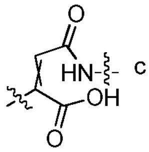, 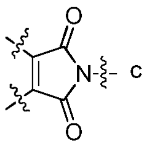, 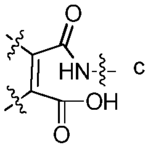, 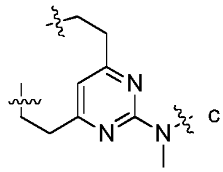,

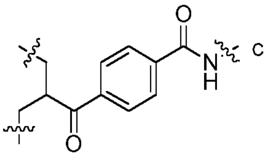, 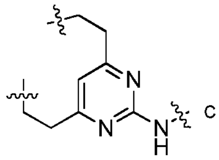, 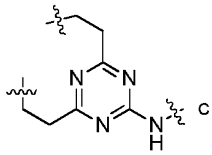, 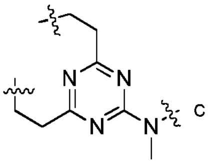, 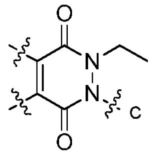,

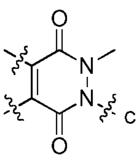, 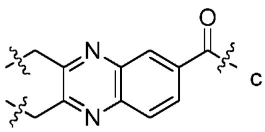, 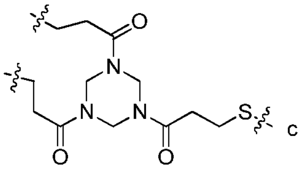, 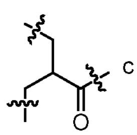, 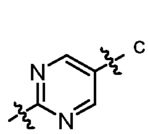,

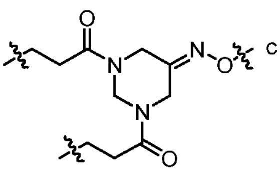, 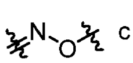, 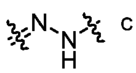, 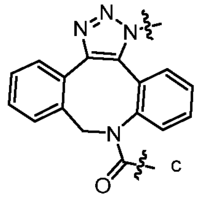, 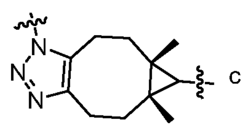,

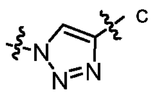, 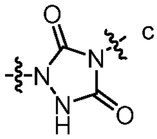 or 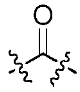;

and the linker head is attached to the rest of L through a c-terminus.

(10) $-(A)_v-$ is

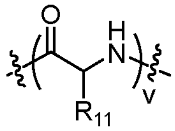;

each $R_{11}$ is independently hydrogen, methyl, ethyl, n-propyl, isopropyl, isobutyl, $-SO_3H$,

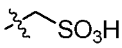, 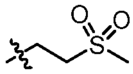, 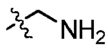, 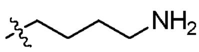, 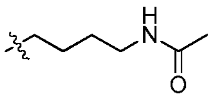,

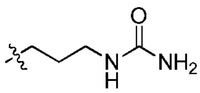, 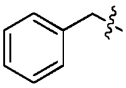, 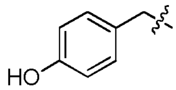, 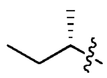, 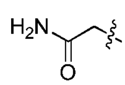, 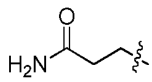, 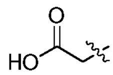,

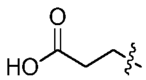 or 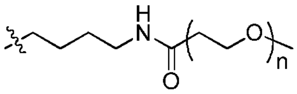;

or $R_{11}$ and an adjacent nitrogen atom form a five-membered heterocycloalkyl; and v is any integer from 1 to 4, and v is preferably 1 or 2, or 3 or 4;

(11) A is attached to $-(T)_o-$ or $-L_5-$ through a carbonyl terminus;

(12) the $L_5$ is

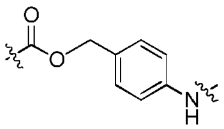, 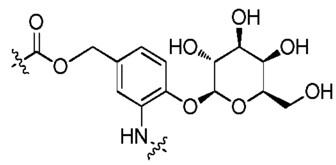, 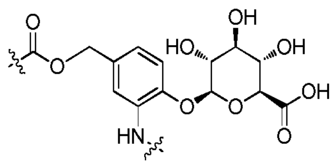,

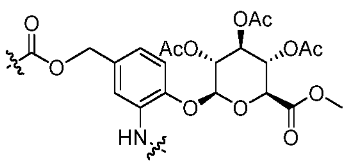, 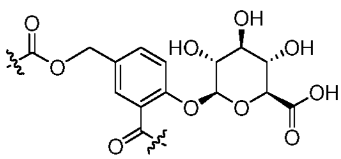, 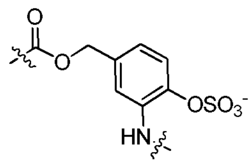

or

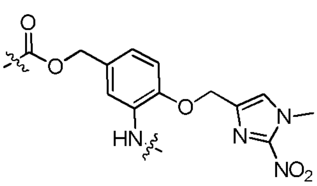;

(13) the $-L_5-$ is attached to the D through a carbonyl terminus;

(14) $-(T)_w-$ is

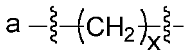, 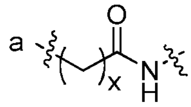, 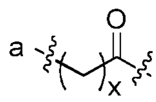, 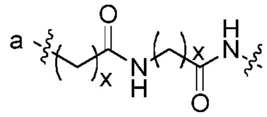,

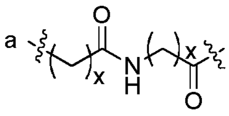, 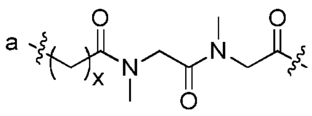, 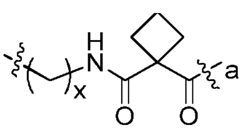, 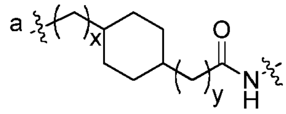,

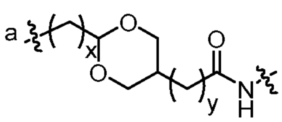, 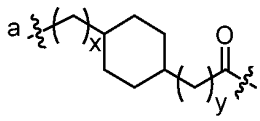, 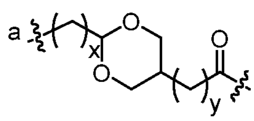, 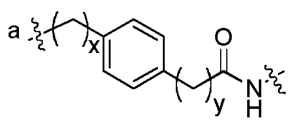,

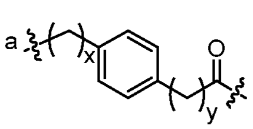, 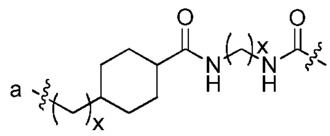, 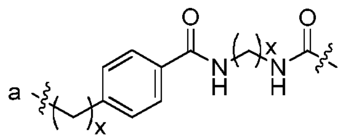,

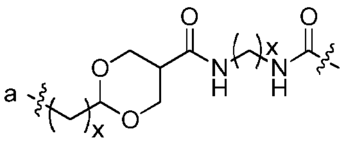, 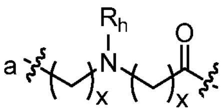 or 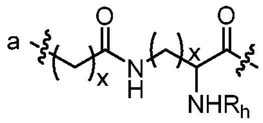;

and the $-(T)_w-$ is attached to the -M- through an a-terminus;

(15) the $-(T)_o-$ is

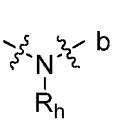, 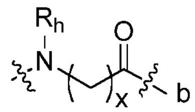, 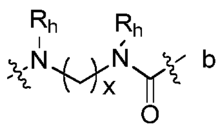, 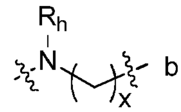

or

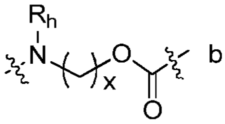;

and the $-(T)_o-$ is attached to the -PEG- or the D via a b-terminus; and

(16) L is any one of combinations of: a) -M-PEG-; b) $-M-(CH_2)_x-$; c) $-M-(CH_2)_x-C(O)-$; d) $-M-(CH_2)_x-(C_{5-6}$ cycloalkylene)$-(CH_2)_y-C(O)NH-PEG-$; e) $-M-(CH_2)_x-$(5-6 membered heterocyclylene)$-(CH_2)_y-C(O)NH-PEG-$; f) $-M-(CH_2)_x-$(phenylene)$-(CH_2)_y-C(O)NH-PEG-$; g) $-M-(CH_2)_x-C(O)NH-PEG-$; h) $-M-(CH_2)_x-C(O)NH-(CH_2)_x-C(O)NH-PEG-$; i) $-M-(CH_2)_x-C(O)NH-(CH_2)_x-C(O)NH-PEG-NH-(CH_2)_x-C(O)-$; j) $-M-(CH_2)_x-(C_{5-6}$ cycloalkylene)$-(CH_2)_y-C(O)NH-(CH_2)_x-NHC(O)-$; k) $-M-(CH_2)_x-$(5-6 membered heterocyclylene)$-(CH_2)_y-C(O)NH-(CH_2)_x-NHC(O)-$; l) $-M-(CH_2)_x-$(phenylene)$-(CH_2)_y-C(O)NH-(CH_2)_x-NHC(O)-$; m) $-M-(CH_2)_x-C(O)NH-(CH_2)_x-C(O)-L_5-$; n) $-M-(CH_2)_x-C(O)NH-PEG-L_5-$; o) $-M-(CH_2)_x-$(phenylene)$-(CH_2)_y-C(O)-(A)_v-$; p) $-M-(CH_2)_x-C(O)NH-(C_{3-6}$ cycloalkylene)$-C(O)-(A)_v$; q) $-M-(CH_2)_x-C(O)NH-PEG-(A)_v-$; r) $-M-(CH_2)_x-C(O)NH-PEG-(A)_v-L_5-$; s) $-M-(CH_2)_x-(C_{5-6}$ cycloalkylene)-C(O)-; t) $-M-(CH_2)_x-$(5-6 membered heterocyclylene)-C(O)-; u) -M-(phenylene)$-(CH_2)_y-C(O)-$; v) $-M-(CH_2)_x-C(O)-(A)_v-NH-PEG-$; w) $-M-(CH_2)_x-C(O)-(A)_v-NH-(CH_2)_x-C(O)-$;

x) -M-$(CH_2)_x$-C(O)-$(A)_v$-; and y) -M-$(CH_2)_x$-(5-6 membered heterocyclylene)-C(O)-$(A)_v$-, z) -M-$(CH_2)_x$-C(O)NH-$CH_2$-CH($NHR_h$)-C(O)-$(A)_v$-, aa) -M-(phenylene)-(5-6 membered heterocyclylene)-C(O)-, ab) -M-(phenylene)-$(CH_2)_y$-C(O)NH-$(CH_2)$x-PEG-$(A)_v$-, ac) -M-(phenylene)-C(O)NH-$(CH_2)$x-$(A)_v$-$L_5$- or ad) -M-$(CH_2)_x$-PEG-$(A)_v$-;
or L is -M-$(T)_w$-$(A)_v$-$L_5$-$^*$;
when L is -M-$(T)_w$-$(A)_v$-$L_5$-$^*$, a combination of -M-, -$(T)_w$-, -$(A)_v$- and -$L_5$- does not simultaneously satisfy conditions of: -M- is succinimidyl; -$(T)_w$- is hexanoyl; -$(A)_v$- is -Val-Cit-, -Val-Ala- or -Gly-Gly-Phe-Gly-, and -$L_5$- is PABC; and preferably, L is -M-$CH_2$-($C_{5-6}$ cycloalkylene)-C(=O)-$(A)_v$-$L_5$-, -M-$CH_2$-(5-6 membered heterocyclylene)-C(=O)-$(A)_v$-$L_5$-, -M-(phenylene)-$CH_2$-C(=O)-$(A)_v$-$L_5$- or -M-$(CH_2)_x$-C(=O)NH-$CH_2$-CH($NHR_h$)-C(O)-$(A)_v$-$L_5$-;
when L is -M-$(T)_w$-PEG-$(A)_v$-$L_5$-$^*$, the L is -M-$(CH_2)_x$-C(=O)NH-PEG-$(A)_v$-$L_5$-, -M-(phenylene)-$CH_2$-C(=O)NH-PEG-$(A)_v$-$L_5$-, -M-$CH_2$-($C_{5-6}$ cycloalkylene)-C(=O)NH-PEG-$(A)_v$-$L_5$- or -M-$CH_2$-(5-6 membered heterocyclylene)-C(=O)NH-PEG-$(A)_v$-$L_5$-; and
when L is -M-$(T)_w$-$(A)_v$-$(T)_o$-$^*$, the L is -M-$(CH_2)_x$-C(=O)NH-$(A)_v$-NH-$CH_2$-.

**7.** The antibody-immunostimulatory conjugate of formula I or the pharmaceutically acceptable salt thereof according to claim 6, satisfying one or more of conditions of:

(1) the antibody is Trastuzumab, Pertuzumab, Cetuximab, huA1, Sacituzumab, Zolbetuximab, Labetuzumab or Enfortumab;
(2) the D is a structure shown in Table 5:

Table 5

| | |
|---|---|
| 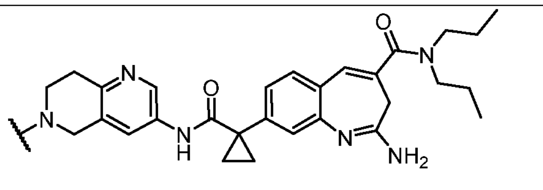 | 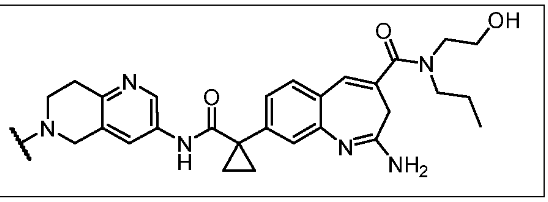 |

| | |
|---|---|
| 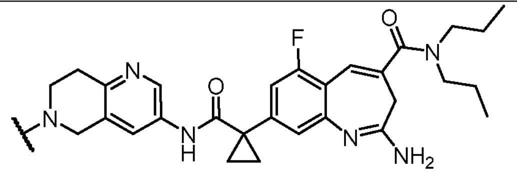 | 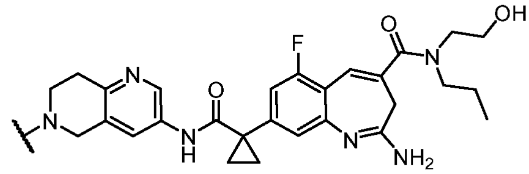 |
| 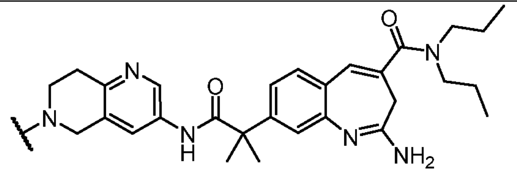 | 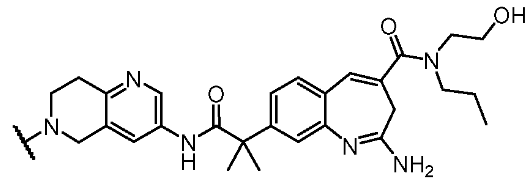 |
| 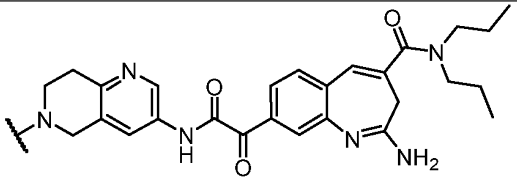 | 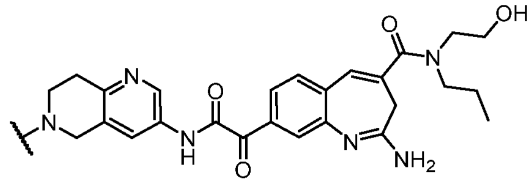 |
| 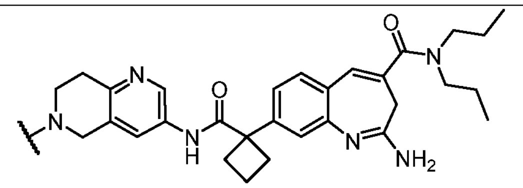 | 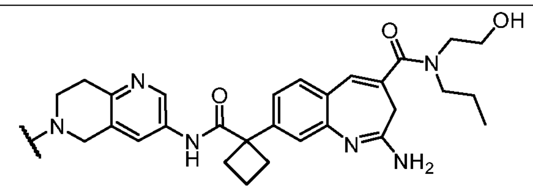 |
| 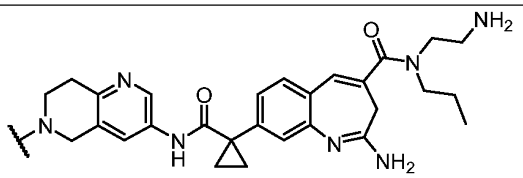 | 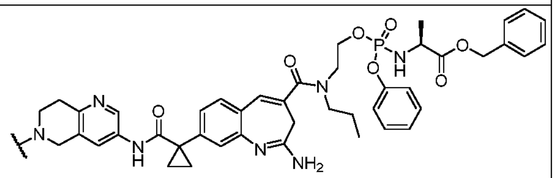 |
| 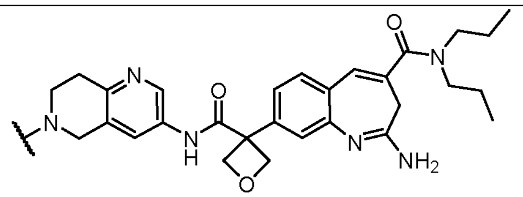 | 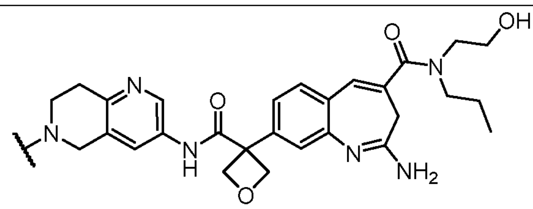 |
| 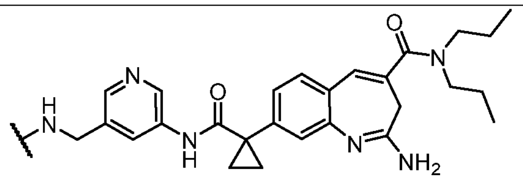 | 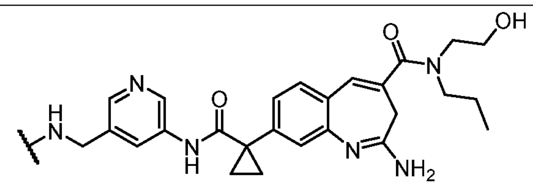 |
| 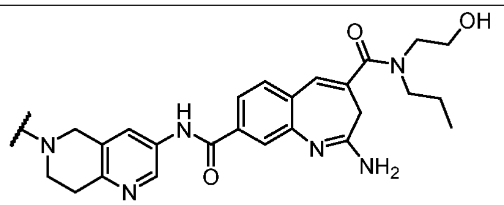 | 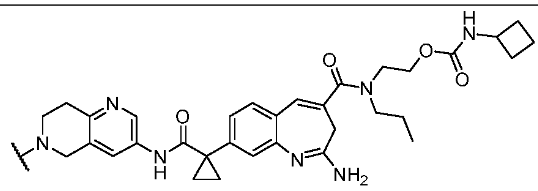 |

| | |
|---|---|
| 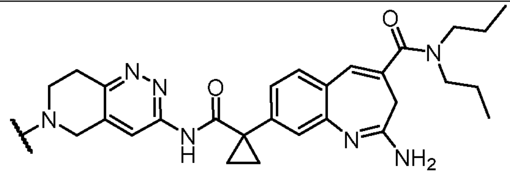 | 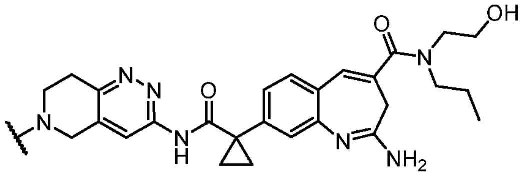 |
| 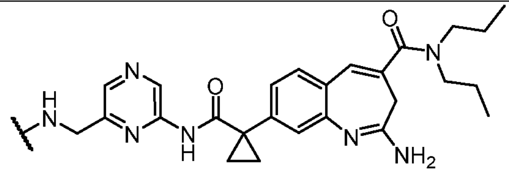 | 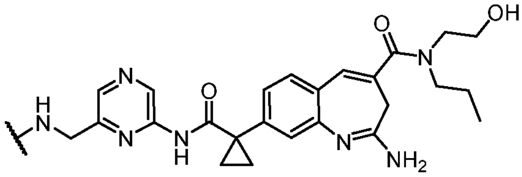 |
| 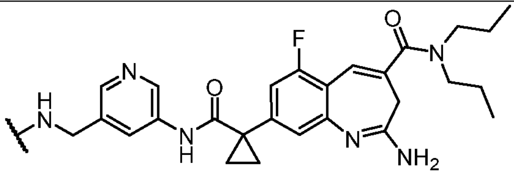 | 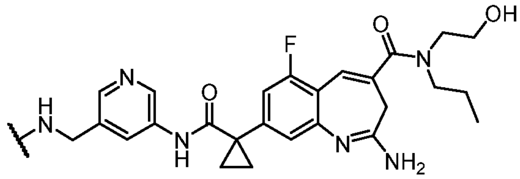 |
| 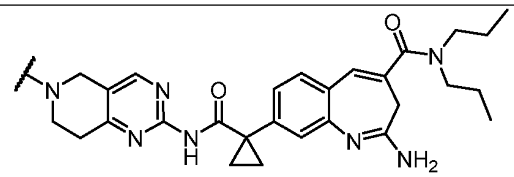 | 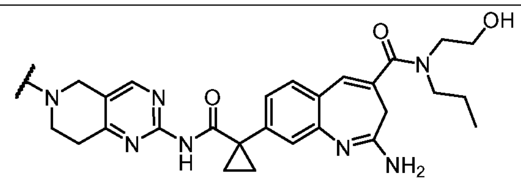 |
| 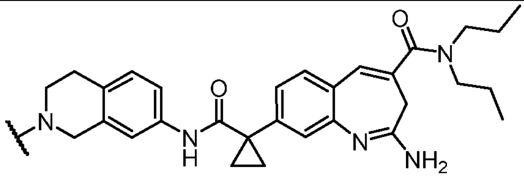 | 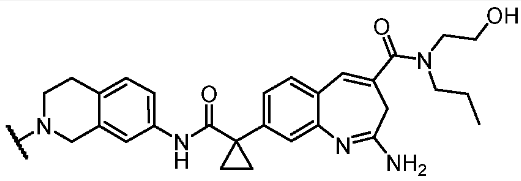 |
| 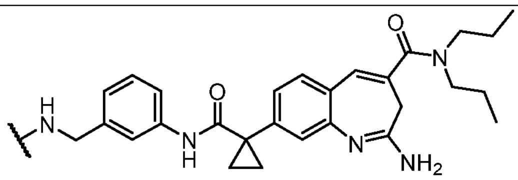 | 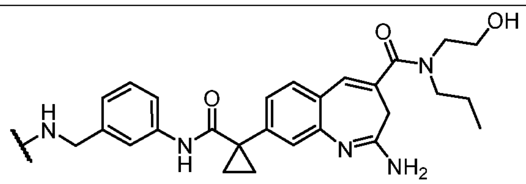 |
| 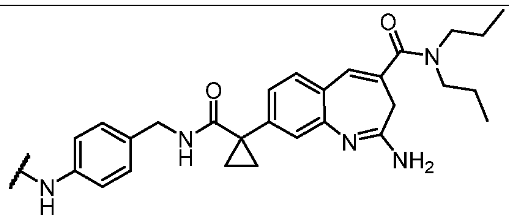 | 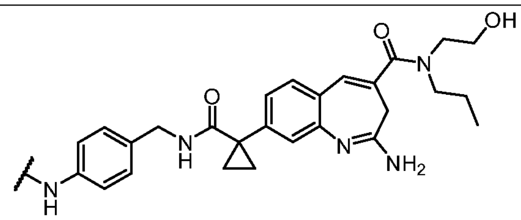 |
| 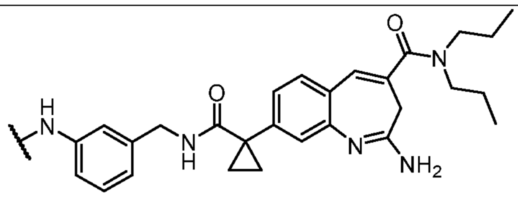 | 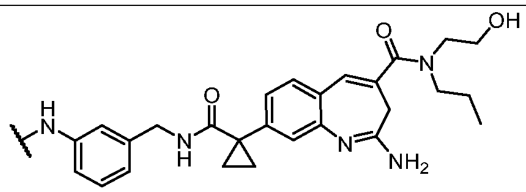 |

| | |
|---|---|
| 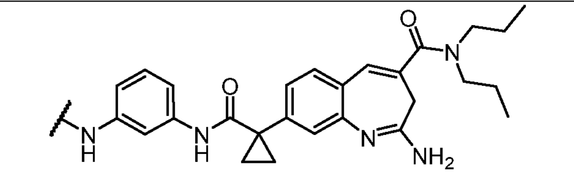 | 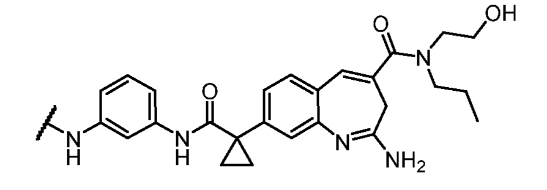 |
| 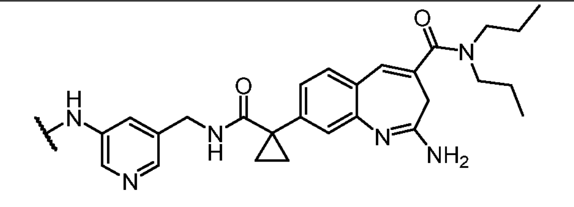 | 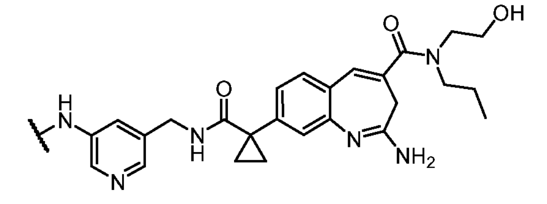 |
| 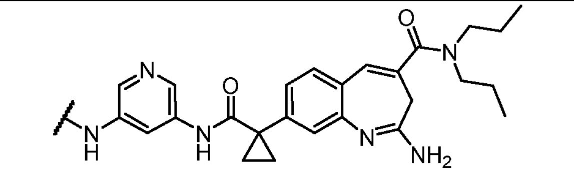 | 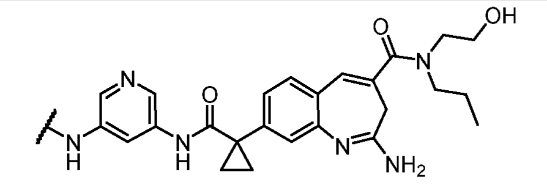 |
| 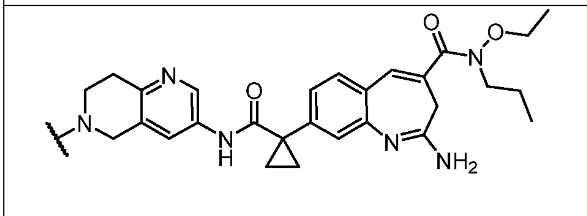 | 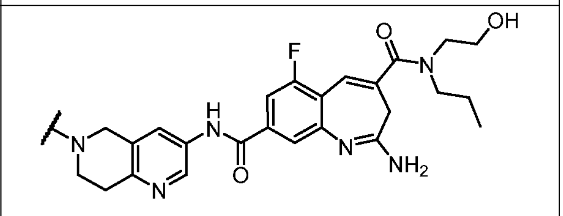 |
| 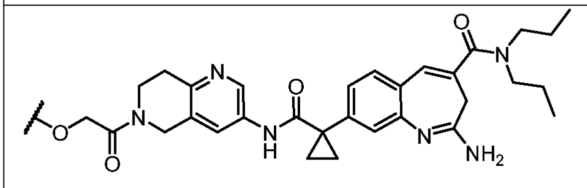 | 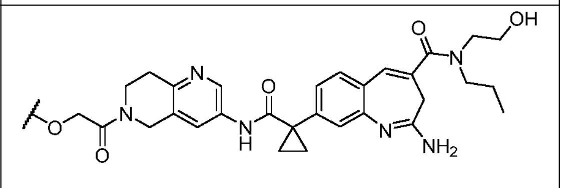 |
| 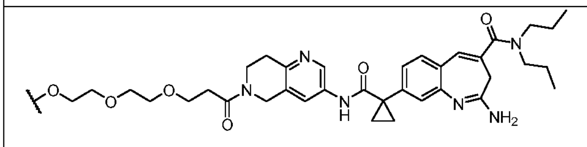 | 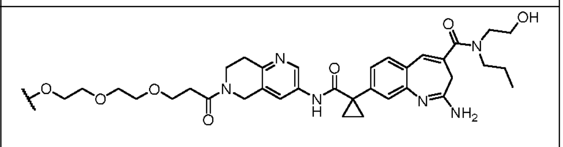 |
| 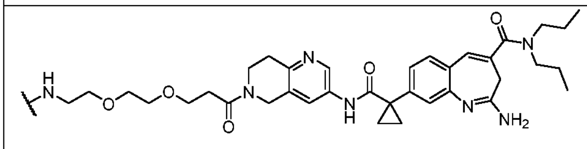 | 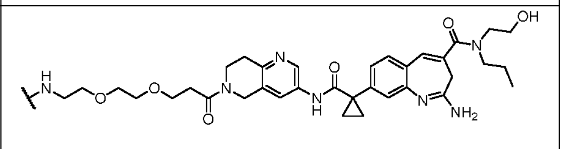 |
| 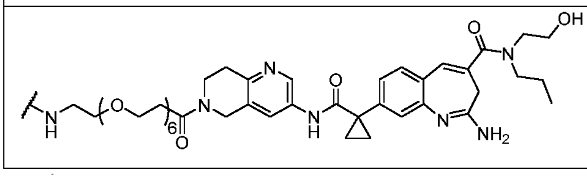 | 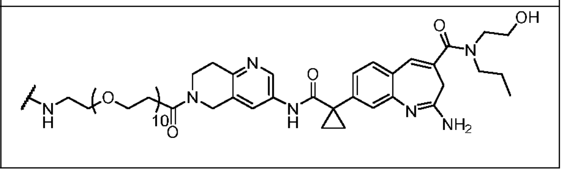 |

; and

(3) L is

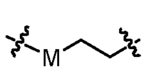, 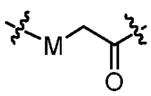, 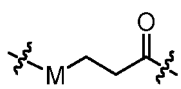, 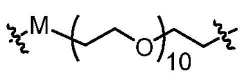, 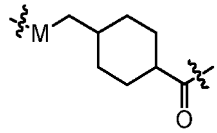,

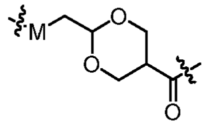, 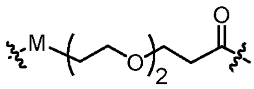, 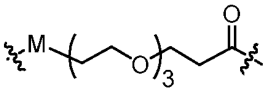, 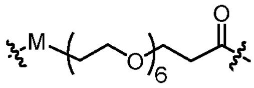,

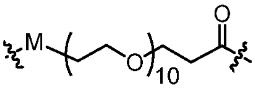, 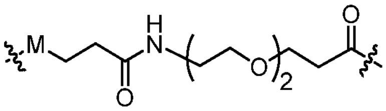, 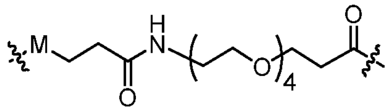,

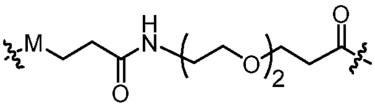, 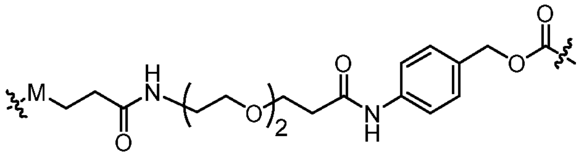,

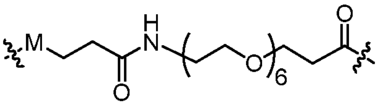, 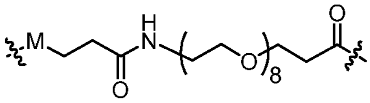,

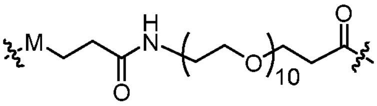, 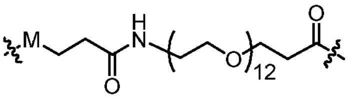, 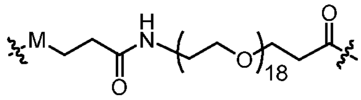,

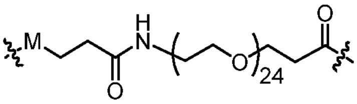, 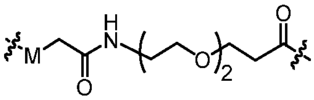, 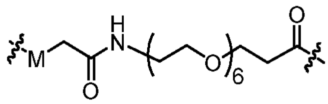,

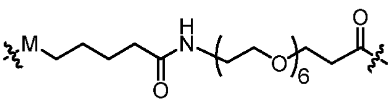, 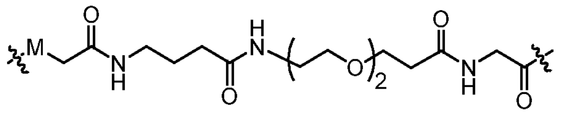,

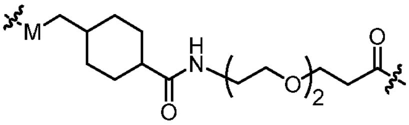, 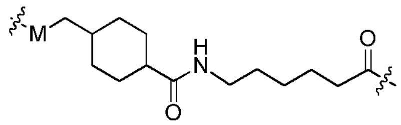,

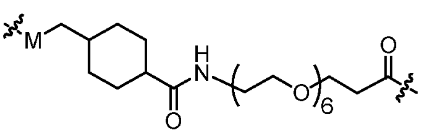, 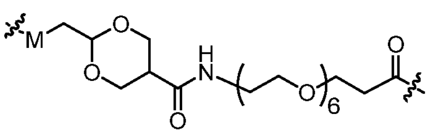,

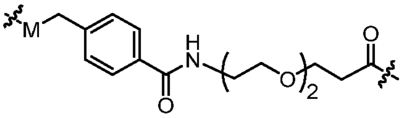, 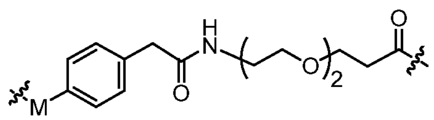,

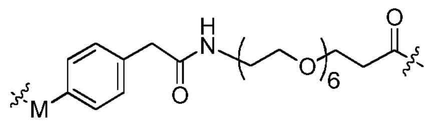
,
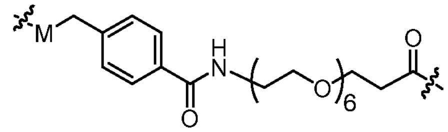
,
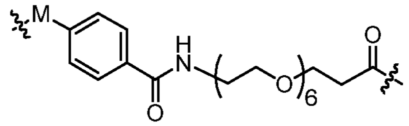
,
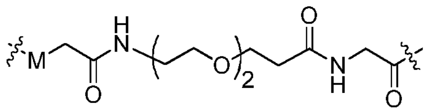
,
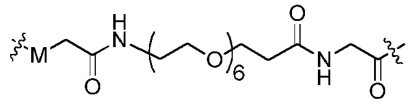
,
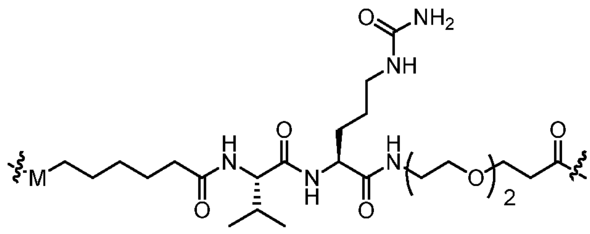
,
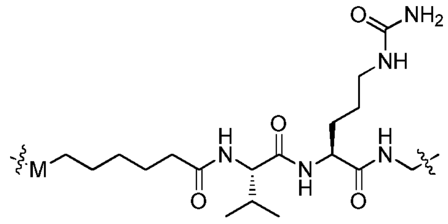
,
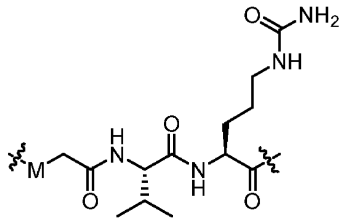
,
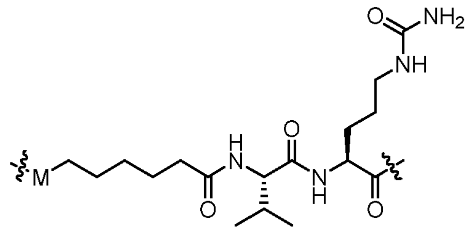
,
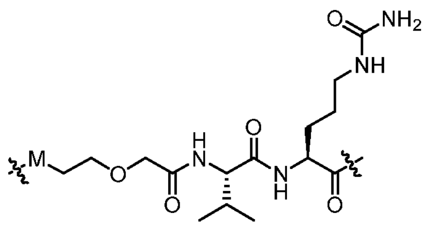
,
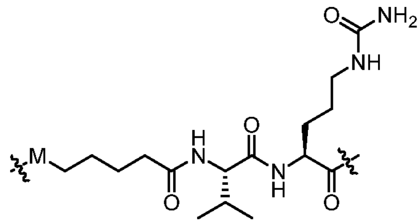
,
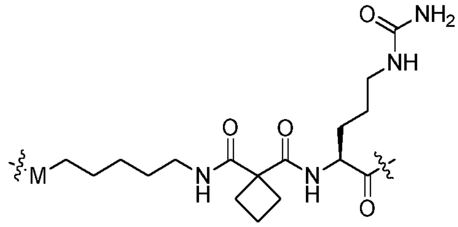
,
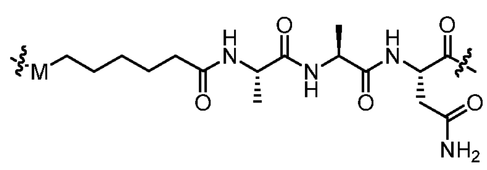
,
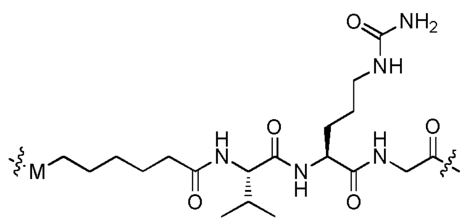
,

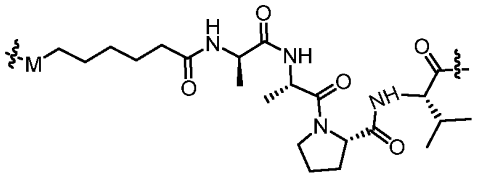
,
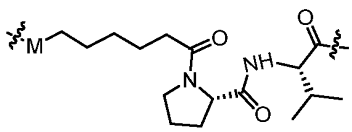
,

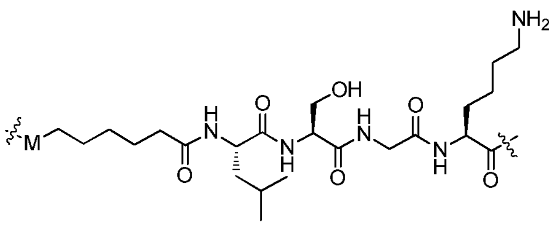
,
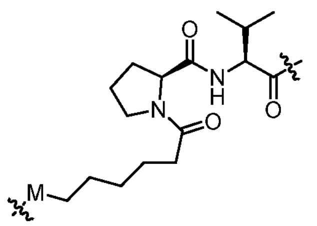
,

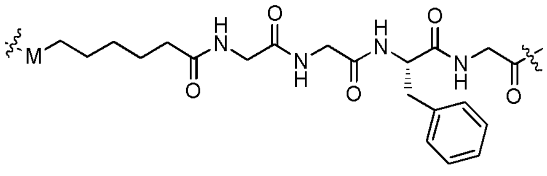
,
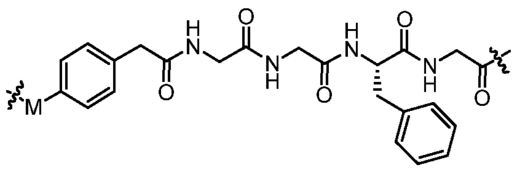
,

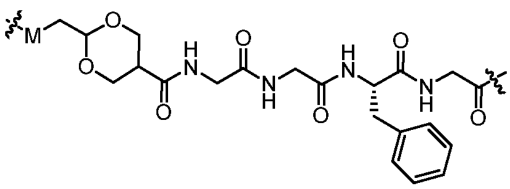
,

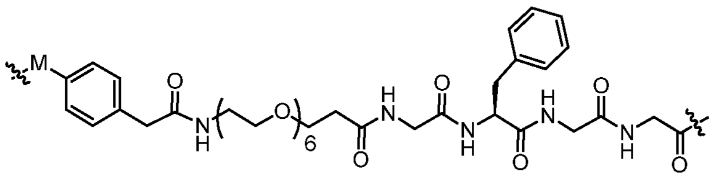
,

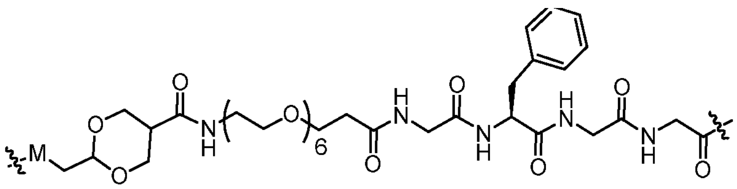
,
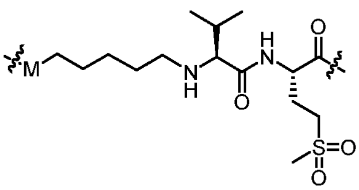
,

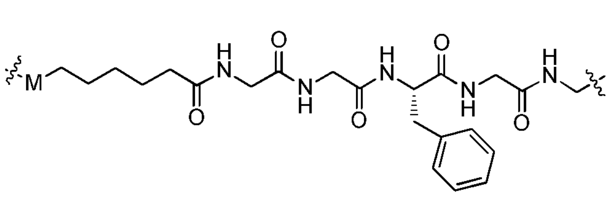
,
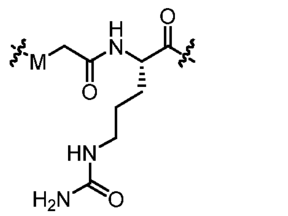
,

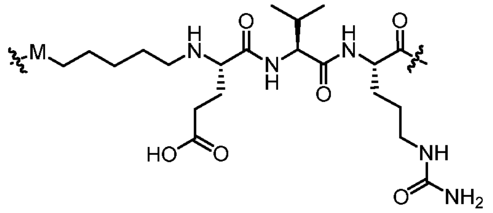

,

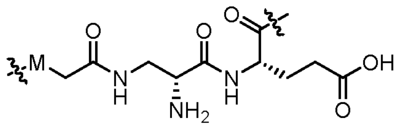

,

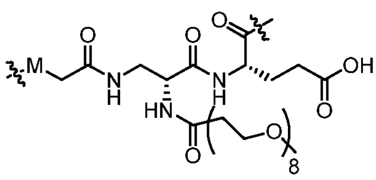

,

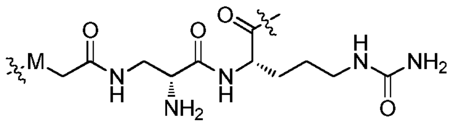

,

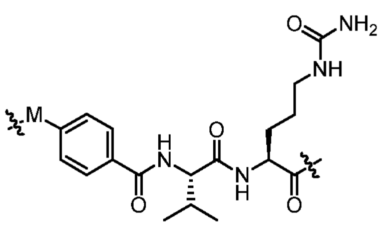

,

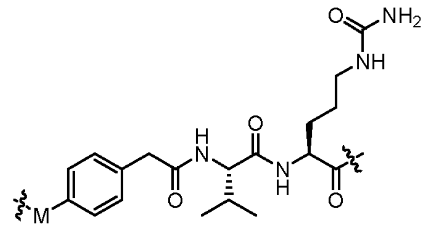

,

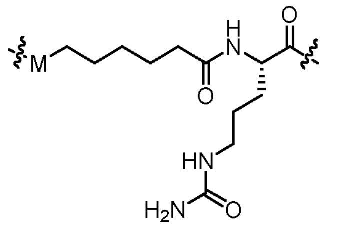

,

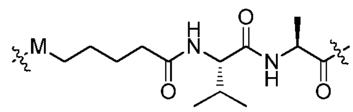

,

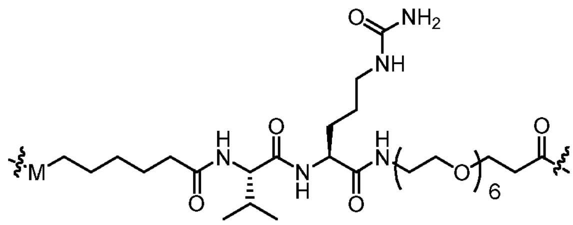

,

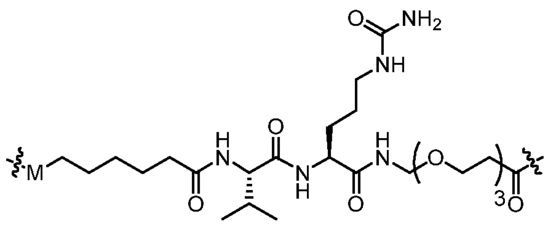

,

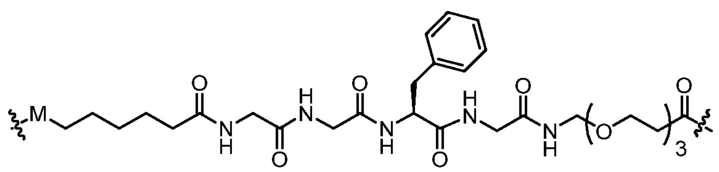

,

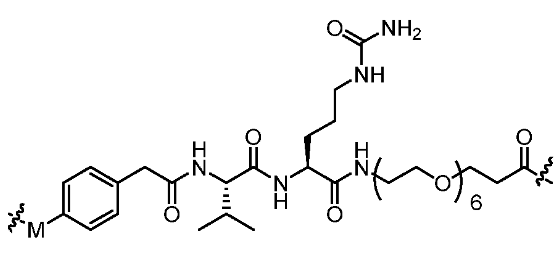

,

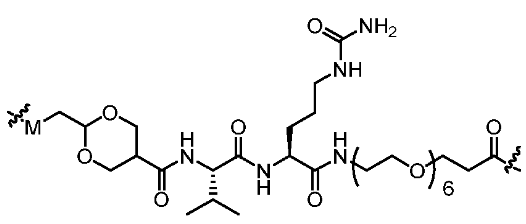

,

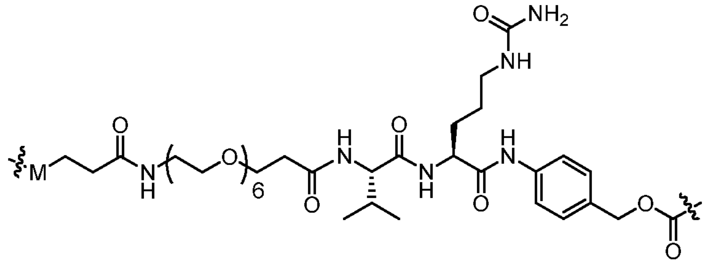

,

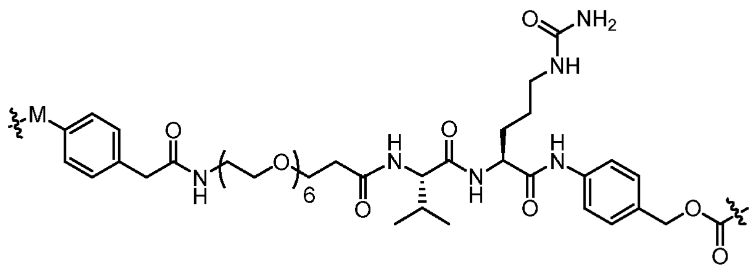

,

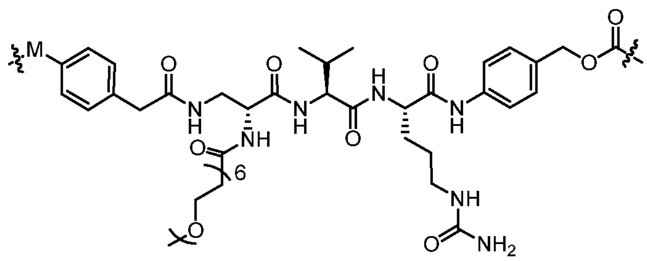

,

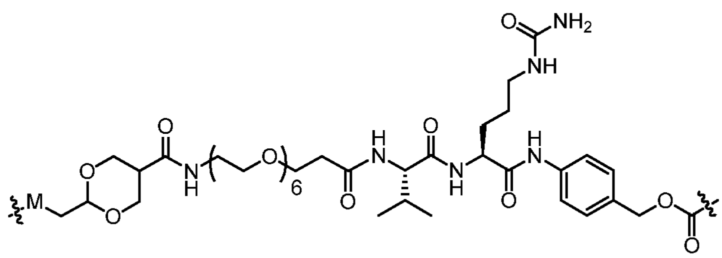

,

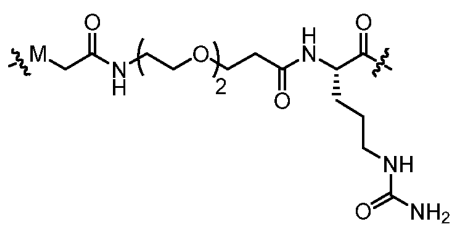

,

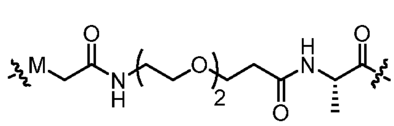

,

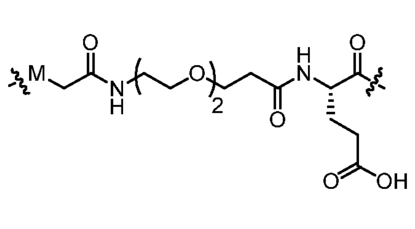

,

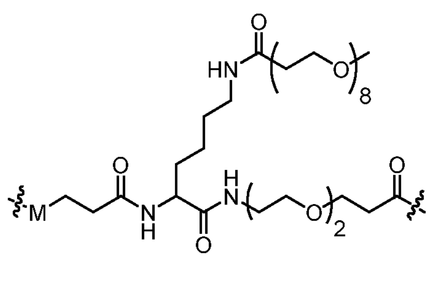

,

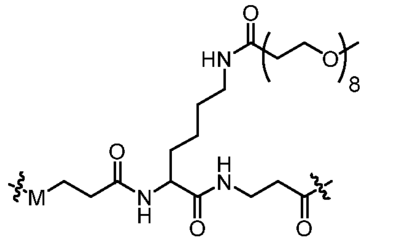

,

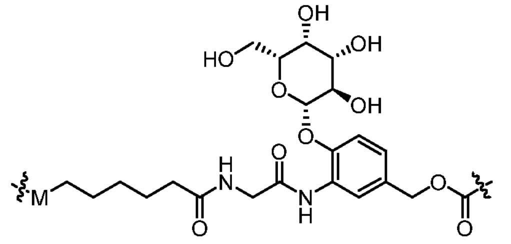

,

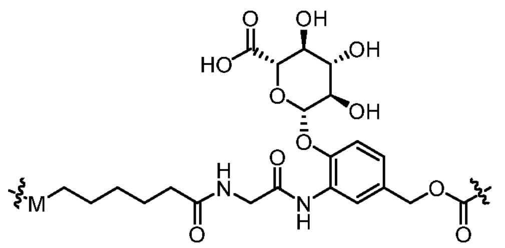

,

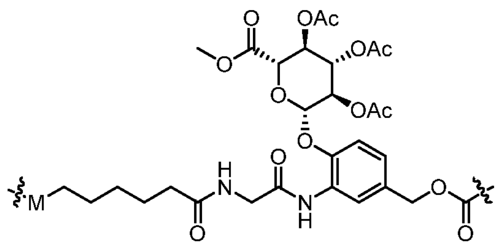

,

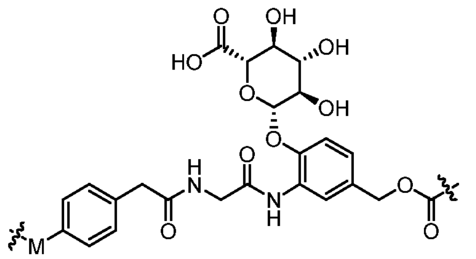

,

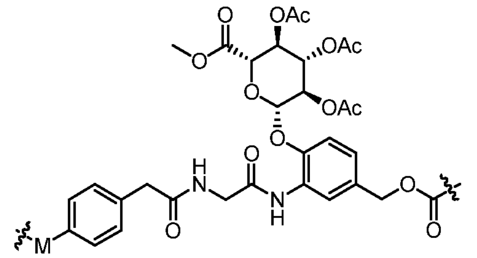

,

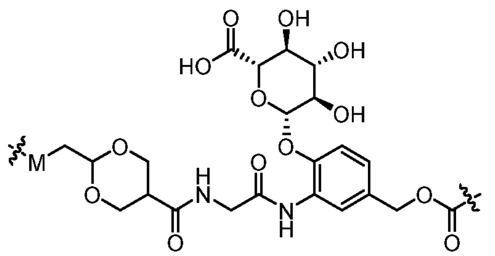

,

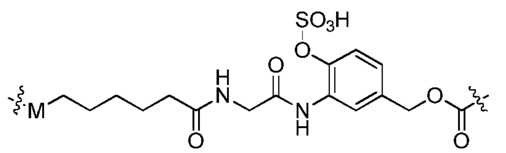

,

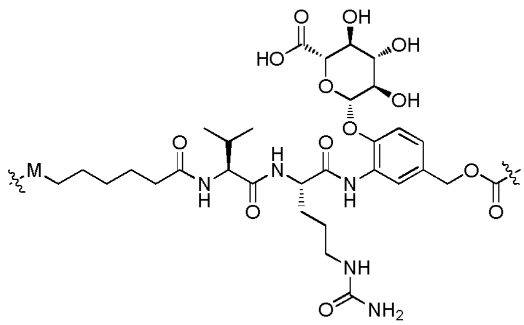

,

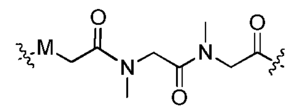

,

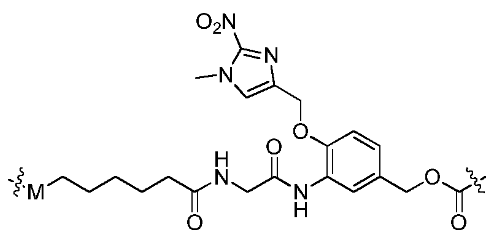

,

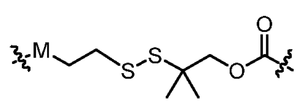

,

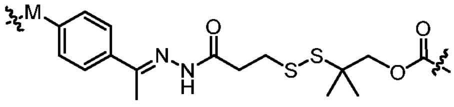,

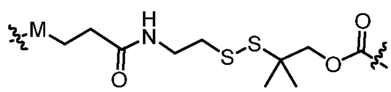,

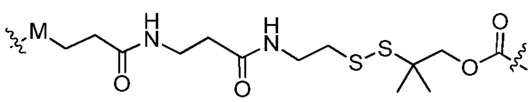,

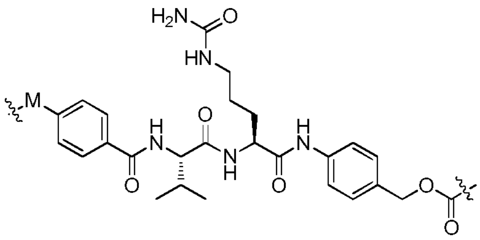,

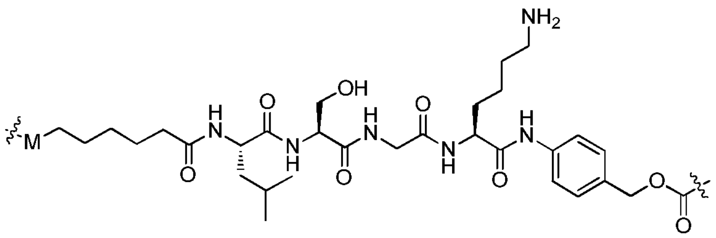,

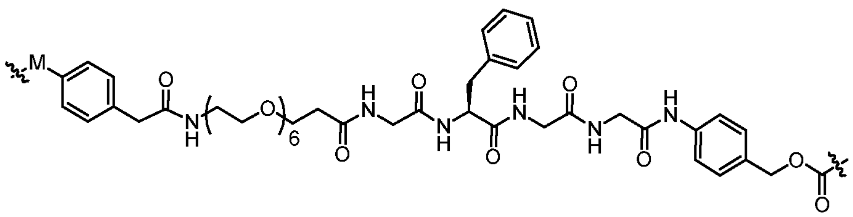,

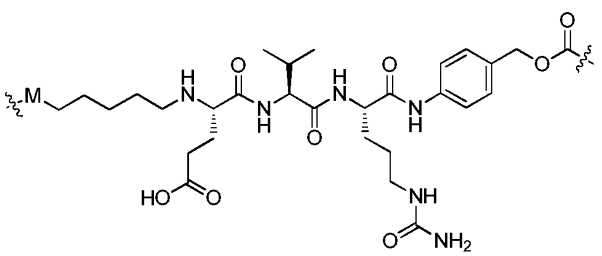,

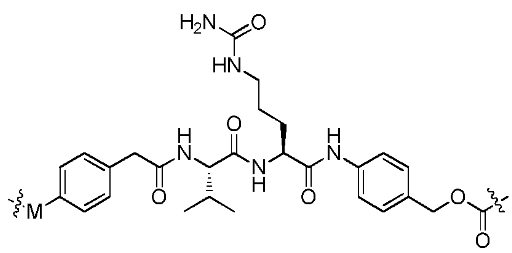,

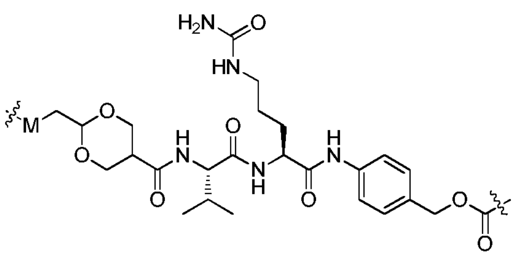,

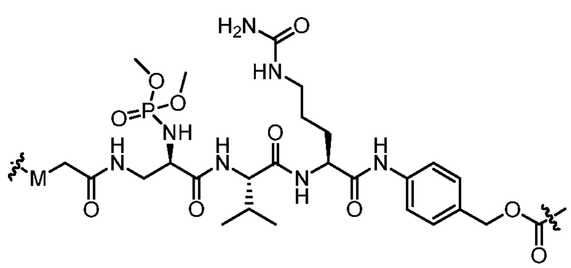,

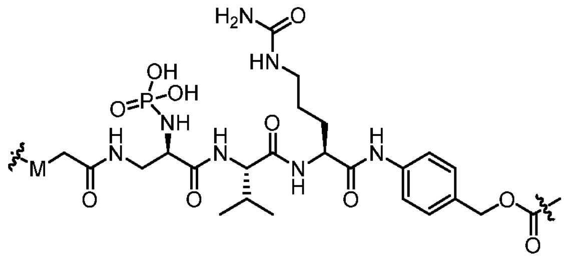

,

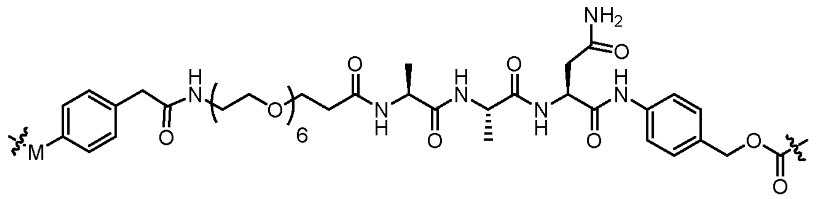

,

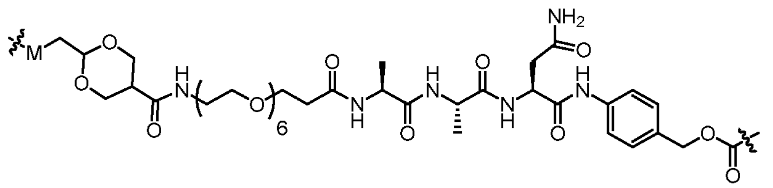

,

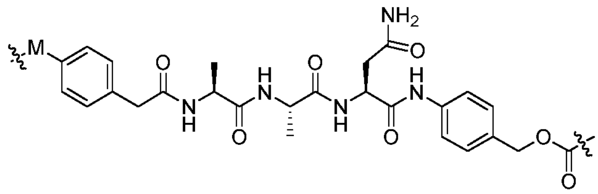

,

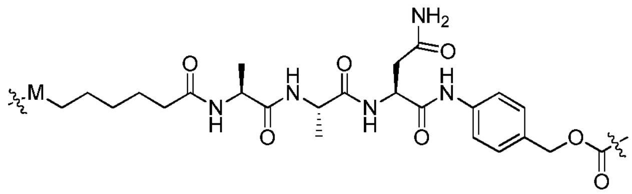

,

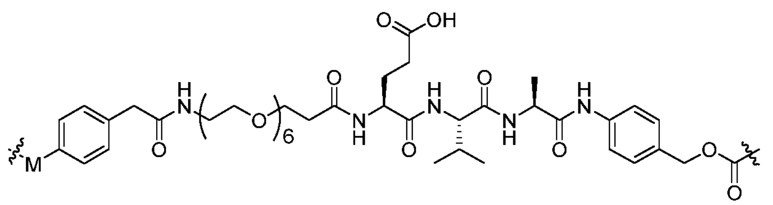

,

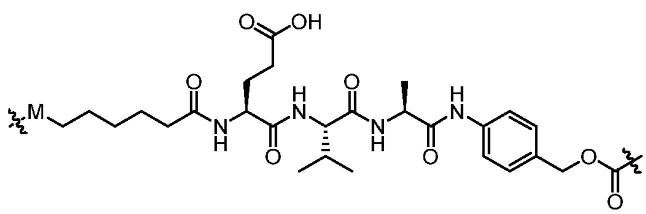

,

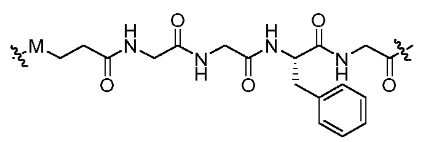

,

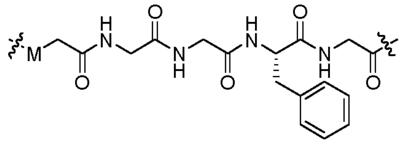,

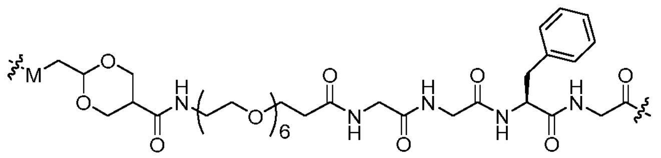,

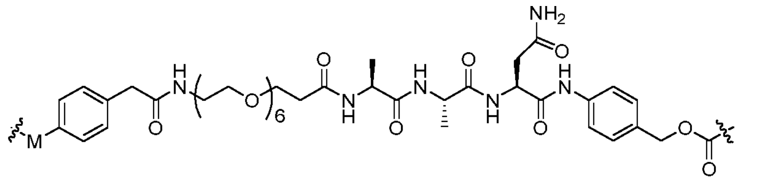,

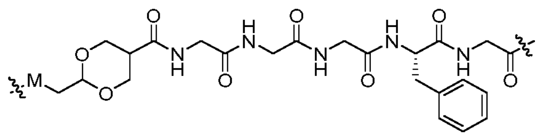,

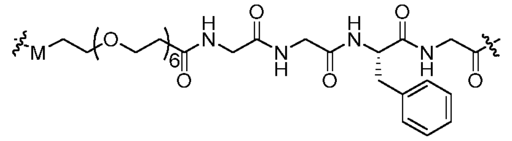,

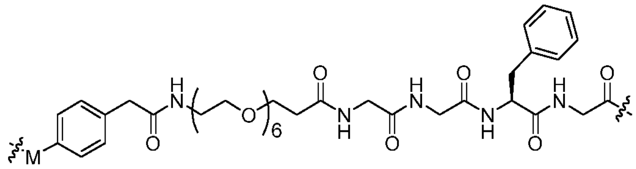,

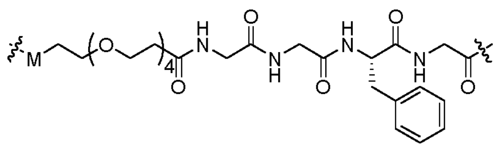,

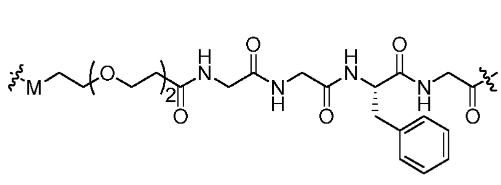,

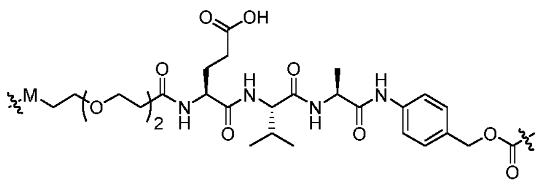,

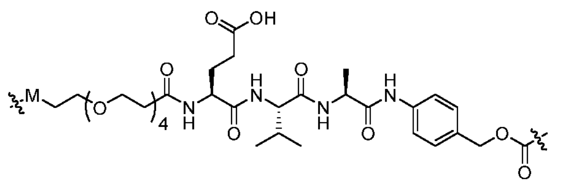,

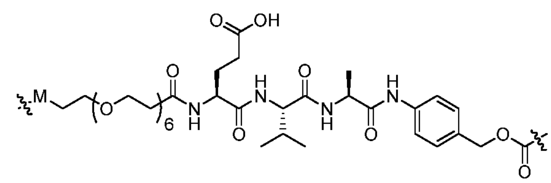,

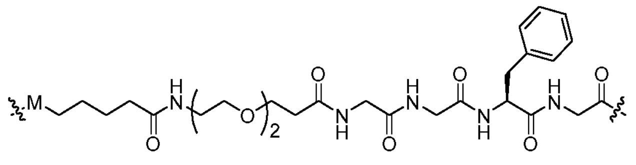,

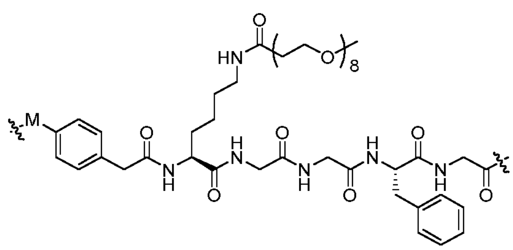,

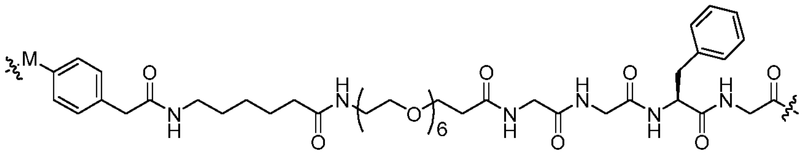, 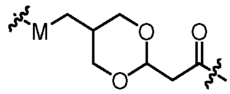,

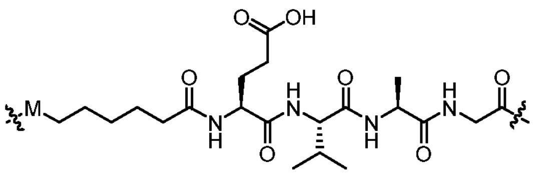, 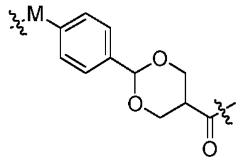, 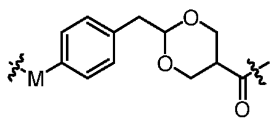,

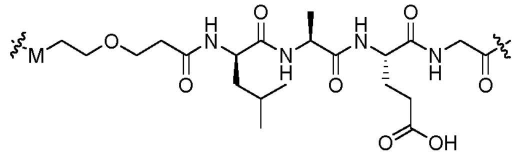, 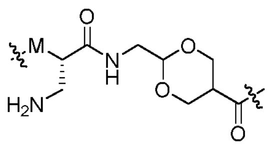,

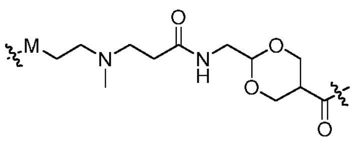, 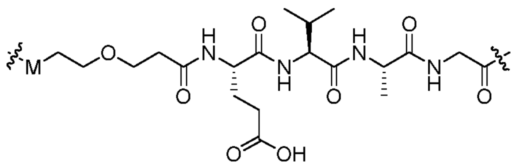, 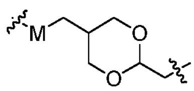,

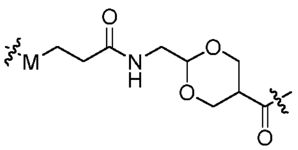 or 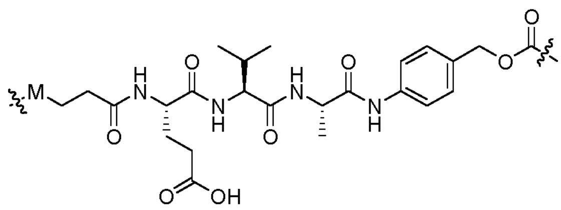.

8. The antibody-immunostimulatory conjugate of formula I or the pharmaceutically acceptable salt thereof according to any one of claims 1-7, wherein the D is case 1 or 2:

case 1: the D is formula (D-1) or formula (D-2):

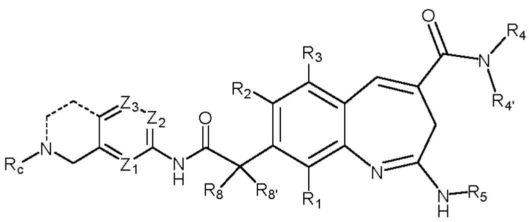

(D-1)

or

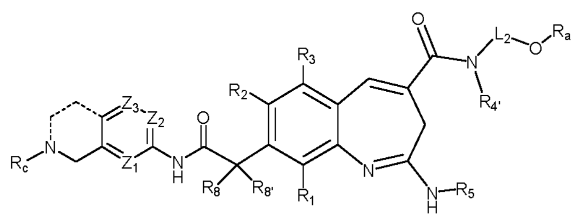

(D-2)

,

wherein

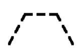

is absent, $-CH_2-$ or $-CH_2-CH_2-$;

$Z_1$, $Z_2$ and $Z_3$ are each independently N or CH;

$R_c$ is a linkage bond attached to L; and

definitions of $R_1$, $R_2$, $R_3$, $R_4$, $R_4$ , $R_5$, $R_8$, $R_8$, and $R_a$ are as described in any one of claims 1-7; and

case 2: the D is formula D', and the formula D' is any one of general formulas from D'-1 to D'-6:

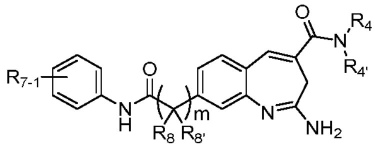

D'-1

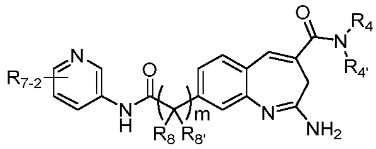

D'-2

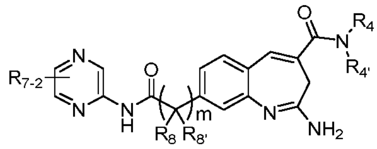

D'-3

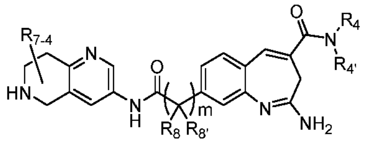

D'-4

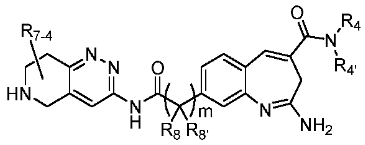

D'-5

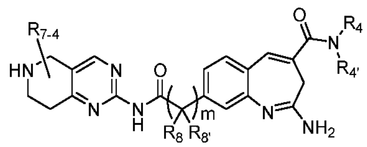

D'-6

wherein definitions of $R_4$, $R_{4'}$, $R_8$ and $R_{8'}$ are as described in any one of claims 1-7; and definitions of $R_{7-1}$, $R_{7-2}$, $R_{7-4}$ and $R_8$ are as described in any one of claims 2-7;
or in the antibody-immunostimulatory conjugate of formula I or the pharmaceutically acceptable salt thereof,

$$Ab\text{-}(L\text{—}D)_t$$

(I)

Ab is Pertuzumab, Cetuximab, huA1, hRS7, Zolbetuximab, Labetuzumab or Enfortumab; t is any value from 1 to 8;
L is a linker attached to the Ab and the D; and
the D is a group formed by loss of one hydrogen atom in a compound of formula D';

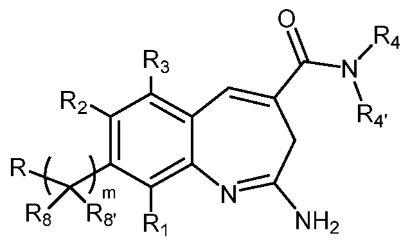

D'

wherein R is $-L'-L_1-R_7$; and L' is $-C(=O)-NR_9-$, $R_9$ is hydrogen, and $L_1$ is independently a linkage bond;
$R_7$ is independently an 8-12 membered fused ring group or an 8-12 membered fused ring group substituted with 1, 2 or 3 $R_{7-4}$; and
$R_{7-4}$ is independently $-C(=O)-L_3-NH_2$, $L_3$ is independently $C_{1-6}$ alkylene or $-(CH_2CH_2O)_{m'}-C_{1-6}$ alkylene-; and m' is 1, 2, 3, 4, 5, 6, 7, 8, 9 or 10.

**9.** The antibody-immunostimulatory conjugate of formula I or the pharmaceutically acceptable salt thereof according to claim 1 or 2, wherein the antibody-immunostimulatory conjugate is any one of the following conjugates, wherein antibody 1 is Trastuzumab; antibody 2 is Pertuzumab; antibody 4 is Trastuzumab (HC-s239.5); antibody 6 is Cetuximab; antibody 7 is huA1; antibody 8 is Sacituzumab; antibody 9 is Zolbetuximab; antibody 10 is Labetuzumab; and antibody 11 is Enfortumab:

Table 10

| Antibody conjugate number | L-D | Antibody | t |
|---|---|---|---|
| C-1 | II-1 | 1 | 3.54 |
| C-2 | II-2 | 1 | 2.98 |
| C-3 | II-3 | 1 | 1.34 |

(continued)

| Antibody conjugate number | L-D | Antibody | t |
|---|---|---|---|
| C-4 | II-4 | 1 | 5.69 |
| C-5 | II-5 | 1 | 5.07 |
| C-6 | II-6 | 1 | 5.22 |
| C-7 | II-7 | 1 | 5.53 |
| C-8 | II-8 | 1 | 5.07 |
| C-9 | II-9 | 1 | 5.21 |
| C-10 | II-10 | 1 | 5.06 |
| C-11 | II-11 | 1 | 4 |
| C-12 | II-12 | 1 | 3.93 |
| C-13 | II-13 | 1 | 3.84 |
| C-14 | II-14 | 1 | 3.93 |
| C-15 | II-15 | 1 | 3.95 |
| C-16 | II-16 | 1 | 3.39 |
| C-17 | II-17 | 1 | 4.17 |
| C-18 | II-18 | 1 | 3.48 |
| C-19 | II-19 | 1 | 4.57 |
| C-20 | II-20 | 1 | 3.46 |
| C-21 | II-21 | 1 | 4.39 |
| C-22 | II-22 | 1 | 4.52 |
| C-23 | II-23 | 1 | 4.34 |
| C-24 | II-24 | 1 | 3.47 |
| C-25 | II-25 | 1 | 3.5 |
| C-26 | II-26 | 1 | 4.36 |
| C-27 | II-27 | 1 | 3.54 |
| C-28 | II-28 | 1 | 3.89 |
| C-29 | II-35 | 1 | / |
| C-30 | II-36 | 1 | 3.69 |
| C-31 | II-40-1 | 1 | 4.48 |
| C-32 | II-40-2 | 1 | 3.69 |
| C-33 | II-41-1 | 1 | 3.25 |
| C-34 | II-41-2 | 1 | 2.99 |
| C-35 | II-30 | 1 | 3.58 |
| C-36 | II-31 | 1 | 3.72 |
| C-37 | II-29 | 1 | 3.67 |
| C-38 | II-42 | 1 | 4.16 |
| C-39 | II-37 | 1 | 3.76 |
| C-40 | II-43 | 1 | 3.81 |
| C-41 | II-45 | 1 | 4.11 |
| C-42 | II-46 | 1 | 4.21 |

(continued)

| Antibody conjugate number | L-D | Antibody | t |
|---|---|---|---|
| C-43 | II-47 | 1 | 4.11 |
| C-44 | II-48 | 1 | 4.08 |
| C-45 | II-50 | 1 | 4.14 |
| C-46 | II-49 | 1 | 4.53 |
| C-47 | II-52 | 1 | 3.81 |
| C-48 | II-54 | 1 | 3.97 |
| C-49 | II-57 | 1 | 3.58 |
| C-50 | II-59 | 1 | 3.91 |
| C-51 | II-60 | 1 | 3.96 |
| C-52 | II-61 | 1 | 3.91 |
| C-53 | II-63 | 1 | 3.76 |
| C-54 | II-64 | 1 | 4.25 |
| C-55 | II-65 | 1 | 3.97 |
| C-56 | II-66 | 1 | 4.07 |
| C-57 | II-67 | 1 | 4.02 |
| C-58 | II-68 | 1 | 4.00 |
| C-59 | II-69 | 1 | 4.08 |
| C-60 | II-70 | 1 | 3.85 |
| C-61 | II-71 | 1 | 3.85 |
| C-62 | II-72 | 1 | 3.98 |
| C-63 | II-73 | 1 | 3.70 |
| C-64 | II-75 | 1 | 3.78 |
| C-65 | II-77 | 1 | 3.93 |
| C-66 | II-78 | 1 | 4.01 |
| C-67 | II-79 | 1 | 3.95 |
| C-68 | II-80 | 1 | 4.06 |
| C-69 | II-83 | 1 | 4.20 |
| C-70 | II-85 | 1 | 4.14 |
| C-71 | II-86 | 1 | 3.82 |
| C-72 | II-82 | 1 | 3.79 |
| C-73 | II-87 | 1 | 3.50 |
| C-74 | II-88 | 1 | 3.86 |
| C-75 | II-89 | 1 | 4.03 |
| C-76 | II-109 | 1 | 3.53 |
| C-77 | II-96 | 1 | 3.91 |
| C-78 | II-97 | 1 | 3.84 |
| C-79 | II-98 | 1 | 3.87 |
| C-80 | II-99 | 1 | 4.03 |
| C-81 | II-100 | 1 | 3.99 |

(continued)

| Antibody conjugate number | L-D | Antibody | t |
|---|---|---|---|
| C-82 | II-101 | 1 | 4.03 |
| C-83 | II-102 | 1 | 4.15 |
| C-84 | II-103 | 1 | 3.86 |
| C-85 | II-104 | 1 | 3.81 |
| C-86 | II-105 | 1 | 3.98 |
| C-87 | II-106 | 1 | 4.16 |
| C-91 | II-90 | 1 | 3.91 |
| C-92 | II-91 | 1 | 3.93 |
| C-93 | II-94 | 1 | 4.45 |
| C-94 | II-53 | 1 | 2.21 |
| C-95 | II-92 | 1 | 3.86 |
| C-96 | II-93 | 1 | 3.86 |
| C-97 | II-87 | 1 | 3.50 |
| C-98 | II-88 | 1 | 3.86 |
| C-99 | II-89 | 1 | 4.03 |
| C-105 | II-110 | 1 | 3.25 |
| C-106 | II-112 | 1 | 4.06 |
| C-107 | II-111 | 1 | 3.94 |
| C-108 | II-115 | 1 | 3.95 |
| C-109 | II-117 | 1 | 3.86 |
| C-110 | II-122 | 1 | 3.93 |
| C-111 | II-123 | 1 | 3.98 |
| C-115 | II-126 | 1 | 4.00 |
| C-116 | II-127 | 1 | 4.02 |
| C-117 | II-140 | 1 | 3.80 |
| C-118 | II-140 | 1 | 7.11 |
| C-119 | II-121 | 1 | 4.45 |
| C-120 | II-128 | 1 | 7.30 |
| C-121 | II-137 | 1 | 7.64 |
| C-122 | II-77 | 1 | 7.76 |
| C-123 | II-72 | 1 | 6.85 |
| C-124 | II-59 | 1 | 8.0 |
| C-125 | II-128 | 1 | 4.26 |
| C-137 | II-72 | 6 | 4.45 |
| C-138 | II-106 | 6 | 4.35 |
| C-139 | II-77 | 6 | 4.46 |
| C-140 | II-12 | 6 | 4.40 |
| C-141 | II-72 | 7 | 4.03 |
| C-142 | II-77 | 7 | 4.02 |

(continued)

| Antibody conjugate number | L-D | Antibody | t |
|---|---|---|---|
| C-143 | II-12 | 7 | 3.89 |
| C-144 | II-115 | 7 | 3.91 |
| C-146 | II-72 | 11 | 4.16 |
| C-147 | II-106 | 11 | 3.98 |
| C-148 | II-12 | 11 | 4.30 |
| C-149 | II-30 | 11 | 4.44 |
| C-150 | II-30 | 10 | 4.54 |
| C-151 | II-12 | 10 | 3.70 |
| C-152 | II-72 | 10 | 3.77 |
| C-153 | II-106 | 10 | 3.87 |
| C-154 | II-106 | 7 | 3.91 |
| C-155 | II-72 | 9 | 3.40 |
| C-156 | II-106 | 9 | 3.40 |
| C-157 | II-7 | 9 | 3.20 |
| C-158 | II-140 | 9 | 3.30 |
| C-159 | II-106 | 8 | 3.50 |
| C-160 | II-72 | 8 | 3.67 |
| C-161 | II-7 | 8 | 3.60 |
| C-162 | II-140 | 8 | 4.20 |
| C-163 | II-157 | 1 | 3.79 |
| C-164 | II-153 | 1 | 3.72 |
| C-165 | II-159 | 1 | 3.74 |
| C-166 | II-160 | 1 | 3.75 |
| C-167 | II-151 | 1 | 4.32 |
| C-168 | II-155 | 1 | 4.11 |

Table 11

| Antibody conjugate number | L-D | Antibody | t |
|---|---|---|---|
| C-127 | II-72 | 4 | 2 |

Table 12

| Antibody conjugate number | L-D | Antibody | t |
|---|---|---|---|
| C-126 | II-140 | 2 | 4.41 |
| C-128 | II-128 | 1 | 7.30 |
| C-129 | II-128 | 1 | 4.03 |
| C-130 | II-140 | 1 | 4.70 |

Table 13

| Antibody conjugate number | L-D | Antibody | t |
|---|---|---|---|
| C-90 | II-84 | 1 | 3.44 |

Table 14

| Antibody conjugate number | L-D | Antibody | t |
|---|---|---|---|
| C-88 | II-74 | 1 | ~3.3 |
| C-89 | II-95 | 1 | ~4.1 |
| C-112 | II-125 | 1 | 4.0 |
| C-113 | II-125 | 1 | 2.33 |
| C-114 | II-124 | 1 | 3.85 |
| C-131 | II-139 | 1 | 4.0 |
| C-132 | II-133 | 1 | 4.0 |
| C-133 | II-124 | 7 | 4.03 |
| C-134 | II-124 | 6 | 4.0 |
| C-135 | II-124 | 2 | 3.82 |
| C-136 | II-124 | 11 | 3.10 |
| C-170 | II-124 | 10 | 3.51 |
| C-171 | II-154 | 1 | 4.19 |
| C-172 | II-146 | 1 | 3.75 |
| C-173 | II-152 | 1 | 4.0 |
| C-174 | II-156 | 1 | 3.58 |
| C-175 | II-161 | 1 | 4.10 |

**10.** A compound of formula II or a pharmaceutically acceptable salt thereof,

D-LX (II)

wherein D is a benzazepine group as shown below:

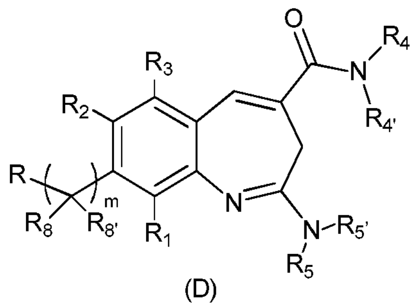

(D) ;

m is 0 or 1;

R is $-C(O)-NR_9-L_1-R_7$, $-C(S)-NR_9-L_1-R_7$ or $-NR_9-C(O)-L_1-R_7$;

$R_1$, $R_2$ and $R_3$ are each independently hydrogen, deuterium, halogen, hydroxyl, amino, cyano, alkyl, haloalkyl, $-L_2-OR_a$ or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl or heteroarylalkyl; and the $R_4$ or $R_{4'}$ is unsubstituted or optionally substituted at any position with one or more substituents selected from halogen, cyano, $-L_2-OR_a$, $-L_2-OR_g$,

$-L_2-OC(O)R_a$, $-L_2-OC(O)OR_a$, $-L_2-OC(O)NR_aR_b$, $-L_2-NR_aR_b$, $-L_2-NR_aC(O)OR_b$, $-L_2-NR_aC(O)NR_aR_b$, $-L_2-NR_bC(NR_b)NR_aR_b$ and $-L_2-C(O)OR_b$; or $R_4$ and $R_{4'}$ together with the N atom to which they are attached form 3-8 membered heterocycloalkyl; and the 3-8 membered heterocycloalkyl is unsubstituted or further substituted at any position with 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$ and $-L_2-C(O)OR_b$;
$R_5$ is hydrogen, $-OR_a$, $-C(O)R_a$, $-C(O)OR_a$ or $-C(O)NR_aR_b$;
$R_{5'}$ is $-R_c$;
$R_7$ is phenyl, 5-6 membered heteroaryl, 3-8 membered heterocycloalkyl or an 8-12 membered fused ring group; and the $R_7$ is unsubstituted or optionally substituted at any position with one or more substituents selected from $-L_3-W$, $-R_c$, halogen, cyano, nitro, alkyl, alkenyl, alkynyl, alkoxy, haloalkyl, haloalkoxy and alkylamino;
$R_8$ and $R_{8'}$ are each independently hydrogen, halogen or alkyl, and the $R_8$ or $R_{8'}$ is unsubstituted or optionally substituted at any position with one or more substituents selected from $-L_3-W$, halogen, cyano, nitro, alkyl, alkenyl, alkynyl, alkoxy, haloalkyl, haloalkoxy and alkylamino; $R_8$ and $R_{8'}$ are each independently a substituent, or $R_8$ and $R_{8'}$ together with the carbon atom to which they are attached form oxo, thio, $C_{1-6}$ alkylene, $C_{3-10}$ cycloalkyl or 3-10 membered heterocycloalkyl; and the $C_{1-6}$ alkylene, $C_{3-10}$ cycloalkyl or 3-10 membered heterocycloalkyl is unsubstituted or optionally substituted at any position with one or more substituents selected from deuterium, halogen, hydroxyl, amino, cyano, oxo, alkyl, haloalkyl, $-L_2-OR_a$ and $-L_2-NR_aR_b$;
$R_9$ is hydrogen, alkyl, haloalkyl, $-L_2-OR_a$ or $-L_2-NR_aR_b$;
W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$ $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$ or $-B(OR_e)_2$;
$Cy^1$ is cycloalkyl, heterocycloalkyl, aryl or heteroaryl; and the $Cy^1$ is unsubstituted or optionally substituted at any position with one or more substituents selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$ and $-L_4-NR_eR_{e'}$;
each $R_a$, $R_b$, $R_d$, $R_e$ and $R_{e'}$ is independently $-R_c$, amino, $C_{1-6}$ alkyl, $C_{2-6}$ alkenyl, $C_{2-6}$ alkynyl, $C_{3-10}$ cycloalkyl, 3-10 membered heterocycloalkyl, $C_{6-10}$ aryl, 5-10 membered heteroaryl, $C_{3-10}$ cycloalkyl $C_{1-6}$ alkyl, 3-10 membered heterocycloalkyl $C_{1-6}$ alkyl, phenyl $C_{1-6}$ alkyl or 5-10 membered heteroaryl $C_{1-6}$ alkyl; and the $R_a$, $R_b$, $R_d$, $R_e$ or $R_{e'}$ is unsubstituted or optionally substituted at any position with 1 to 3 substituents selected from $-L_4-OR_f$, $-OC(O)-L_4-R_f$, $-L_4-NR_fR_f$, halogen, cyano, nitro, $C_{1-6}$ alkyl, halo $C_{1-6}$ alkyl and halo $C_{1-6}$ alkoxy;
$L_1$, $L_2$, $L_3$ and $L_4$ are each independently a linkage bond, $C_{1-6}$ alkylene, $C_{2-6}$ alkenylene, or $C_{2-6}$ alkynylene; the $L_1$, $L_2$, $L_3$ or $L_4$ is unsubstituted or optionally substituted at any position with one or more substituents selected from oxo, hydroxyl, amino, halogen, cyano, alkyl, haloalkyl, alkoxy and haloalkoxy;
each $R_f$ and $R_f$ is independently $-R_c$, $-NHR_c$ or $C_{1-6}$ alkyl;
$R_g$ is a prodrug group comprising phosphoryl; and
each $R_c$ is independently hydrogen or a linkage bond attached to L; and at least one $R_c$ in the D is a linkage bond attached to LX;
and the benzazepine group of formula D satisfies 1 or 2 of conditions of:

(1) m is 1; and
(2) at least one of $R_4$ and $R_4$ is substituted at any position with one or more substituents selected from halogen, cyano, $-L_2-OR_a$, $-L_2-OR_g$, $-L_2-OC(O)R_a$, $-L_2-OC(O)OR_a$, $-L_2-OC(O)NR_aR_b$, $-L_2-NR_aR_b$, $-L_2-NR_aC(O)OR_b$, $-L_2-C(O)OR_b$, $-L_2-NR_aC(O)NR_aR_b$ and $-L_2-NR_bC(NR_b)NR_aR_b$;

LX is M'-$(T)_w$-PEG-$(T)_o$-*, M'-PEG-*, M'-$(T)_w$-*, M'-$(T)_w$-$(A)_v$-$(T)_o$-PEG-*, M'-$(T)_w$-$(A)_v$-$(T)_o$-*, M'-$(T)_w$-$L_5$-*, M'-$(T)_w$-PEG-$L_5$-*, M'-$(T)_w$-PEG-$(A)_v$-$L_5$-*, M'-$(T)_w$-PEG-$(A)_v$-$(T)_o$-*, M'-PEG-$(T)_o$-PEG-* or M'-$(T)_w$-$(A)_v$-$L_5$-*; v is any integer from 1 to 5; w is any integer from 1 to 10; o is any integer from 0 to 10; and * is a linking site of the LX that is attached to the D;
each T is independently $-(CH_2)_x-C(O)-$, $-NR_h-$, -O-, -S-, $-(CH_2)_x$-arylene-$(CH_2)_y$-, $-(CH_2)_x$-heteroarylene-$(CH_2)_y$-, $-(CH_2)_x$-cycloalkylene-$(CH_2)_y$-, $-(CH_2)_x$-heterocycloalkylene-$(CH_2)_y$-, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x$-S-S-alkylene-, alkylene or alkenylene; and x and y are each independently any integer from 0 to 10;
$R_h$ is hydrogen, alkyl, $-PO(OH)_2$, $-PO(OCH_3)_2$, $-C(O)-(CH_2CH_2O)_n-CH_3$ or $-(CH_2CH_2O)_n-CH_3$;
PEG is $-(CH_2CH_2O)_n-(CH_2)_u-$ or $-(CH_2CH_2O)_n-(CH_2)_u-C(O)-$, n is any integer from 1 to 50, and u is any integer from 0 to 5;
each A is independently an amino acid residue or an amino acid residue modified by $-C(O)-(CH_2CH_2O)_n-CH_3$ or $-(CH_2CH_2O)_n-CH_3$;
$L_5$ is a self-immolative group; and

M' is a linker head precursor or a linker head attached to 1 to 2 amino acids.

**11.** The compound of formula II or the pharmaceutically acceptable salt thereof according to claim 10,

D-LX (II)

wherein LX is a linker precursor; and
the D is a group formed by loss of one hydrogen atom in a compound of formula D';

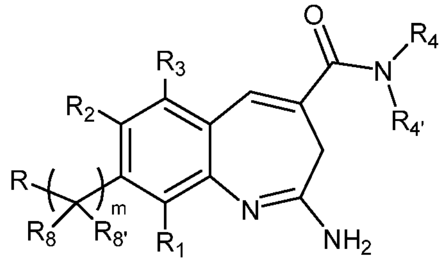

D'

wherein R is -L'-$L_1$-$R_7$; and L' is -C(=O)-, -C(=O)-$NR_9$- or -C(=S)-$NR_9$-;
$R_9$ is independently hydrogen, $C_{1-6}$ alkyl or halo $C_{1-6}$ alkyl;
$L_1$ is independently a linkage bond, $C_{1-6}$ alkylene or $C_{2-6}$ alkenylene;
$R_7$ is independently phenyl, 5-6 membered heteroaryl, 3-8 membered heterocycloalkyl, an 8-12 membered fused ring group, phenyl substituted with 1, 2 or 3 $R_{7-1}$, 5-6 membered heteroaryl substituted with 1, 2 or 3 $R_{7-2}$, 3-8 membered heterocycloalkyl substituted with 1, 2 or 3 $R_{7-3}$ or an 8-12 membered fused ring group substituted with 1, 2 or 3 $R_{7-4}$;
in the $R_7$, in "5-6 membered heteroaryl" in the 5-6 membered heteroaryl and the 5-6 membered heteroaryl substituted with 1, 2 or 3 $R_{7-2}$, the heteroatom is independently selected from one or more of N, O and S, and the number of the heteroatom is independently 1, 2 or 3; and in "3-8 membered heterocycloalkyl" in the 3-8 membered heterocycloalkyl and the 3-8 membered heterocycloalkyl substituted with 1, 2 or 3 $R_{7-3}$, the heteroatom is independently selected from one or more of N, O and S, and the number of the heteroatom is independently 1, 2 or 3;
in the $R_7$, an 8-12 membered fused ring group in the 8-12 membered fused ring group and the 8-12 membered fused ring group substituted with 1, 2 or 3 $R_{7-4}$ is independently a fused ring of ring A and ring B, the ring A is a 5-6 membered heteroaryl ring or a phenyl ring, and the ring B is a 5-6 membered heteroalkenyl ring; in the 5-6 membered heteroaryl ring, the heteroatom is selected from one or more of N, O and S, and the number of the heteroatom is 1, 2 or 3; and in the 5-6 membered heteroalkenyl ring, the heteroatom is selected from one or more of N, O and S, and the number of the heteroatom is 1, 2 or 3;
$R_{7-1}$, $R_{7-2}$, $R_{7-3}$ and $R_{7-4}$ are independently amino, -$L_3$-$NH_2$, -$L_3$-OH, -C(=O)-$L_3$-OH or -C(=O)-$L_3$-$NH_2$, and $L_3$ is independently $C_{1-6}$ alkylene or -$(CH_2CH_2O)_{m'}$-$C_{1-6}$ alkylene-; and m' is 1, 2, 3, 4, 5, 6, 7, 8, 9 or 10; m is 0 or 1;
$R_8$ and $R_{8'}$ are independently hydrogen or $C_{1-6}$ alkyl, or $R_8$ and $R_{8'}$ together with the carbon atom to which they are attached form carbonyl, $C_{3-10}$ cycloalkylene or 3-10 membered heterocycloalkylene; and in the 3-10 membered heterocycloalkylene, the heteroatom is selected from one or more of N, O and S, and the number of the heteroatom is 1, 2 or 3;
$R_1$, $R_2$ and $R_3$ are independently hydrogen, deuterium or halogen;
$R_4$ and $R_{4'}$ are independently $C_{1-6}$ alkyl, $C_{1-6}$ alkoxy or $C_{1-6}$ alkyl substituted with 1, 2 or 3 $R_{4-1}$; and the $R_{4-1}$ is independently hydroxyl, amino, -OC(=O)$NR_aR_b$ or

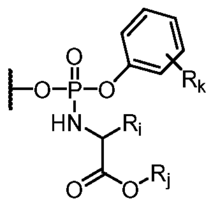
;

$R_a$ and $R_b$ are independently hydrogen, $C_{1-6}$ alkyl or $C_{3-10}$ cycloalkyl;
$R_i$ is hydrogen, $C_{1-6}$ alkyl or benzyl; $R_k$ is hydrogen, halogen or $C_{1-6}$ alkyl; and $R_j$ is $C_{1-6}$ alkyl, $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; and
in the $R_j$, in "3-10 membered heterocycloalkyl" in the 3-10 membered heterocycloalkyl and the 3-10 membered heterocycloalkyl-$C_{1-6}$ alkyl, the heteroatom is independently selected from one or more of N, O and S, and the number of the heteroatom is independently 1, 2 or 3; and in "5-10 membered heteroaryl" in the 5-10 membered heteroaryl and the 5-10 membered heteroaryl-$C_{1-6}$ alkyl, the heteroatom is independently selected from one or more of N, O and S, and the number of the heteroatom is independently 1, 2 or 3;
and the compound of formula D' satisfies 1 or 2 of conditions of:

(1) m is 1; and
(2) at least one of $R_4$ and $R_{4'}$ is $C_{1-6}$ alkoxy or $C_{1-6}$ alkyl substituted with 1, 2 or 3 $R_{4-1}$;

and the compound of formula II is not of a structure shown in Table 6:

Table 6

| |
|---|
| 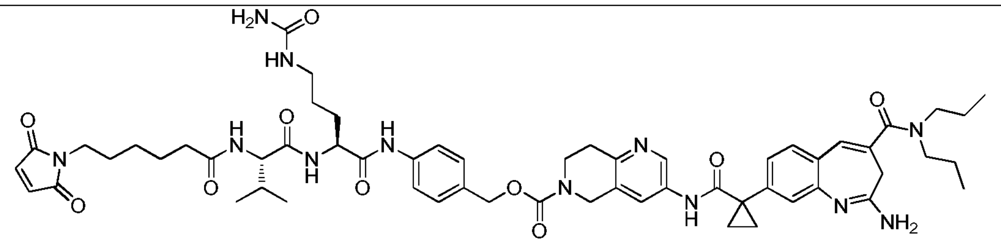 |
| 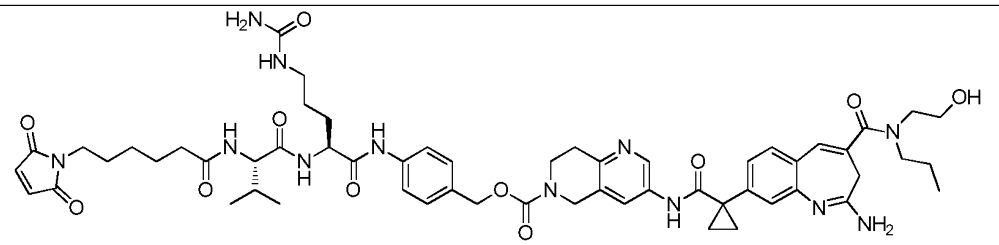 |
| 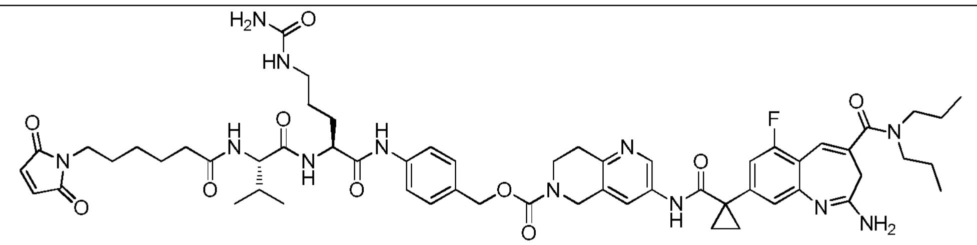 |

| |
|---|
| 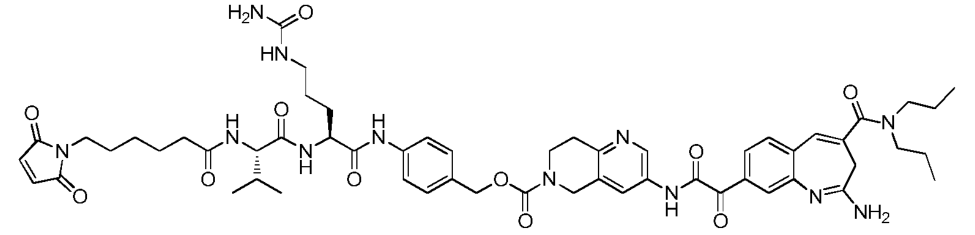 |
| 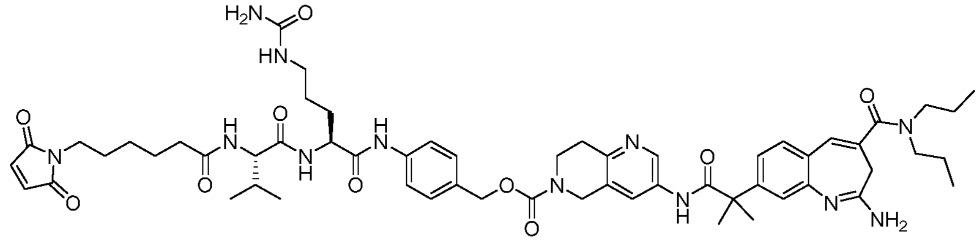 |
| 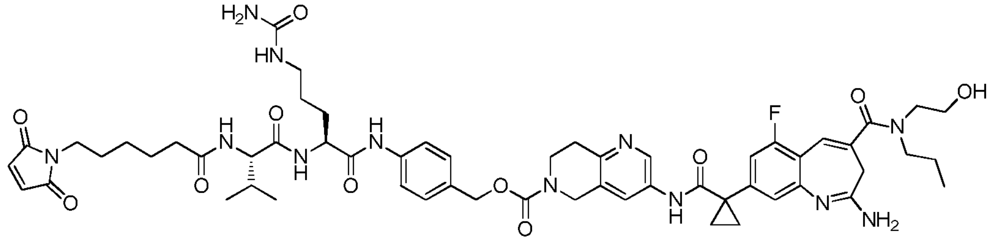 |
| 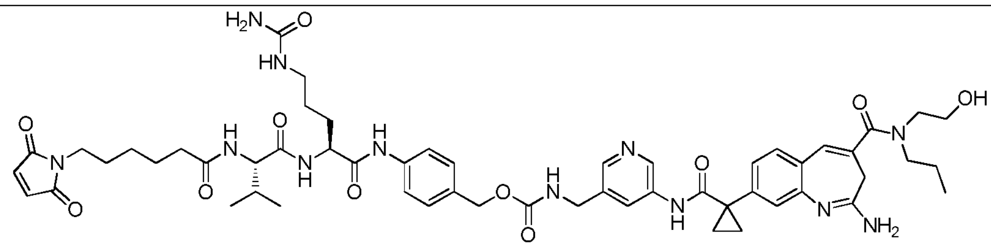 |
| 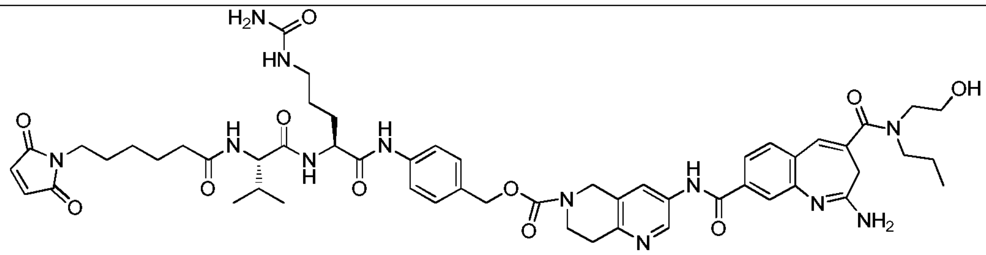 |
| 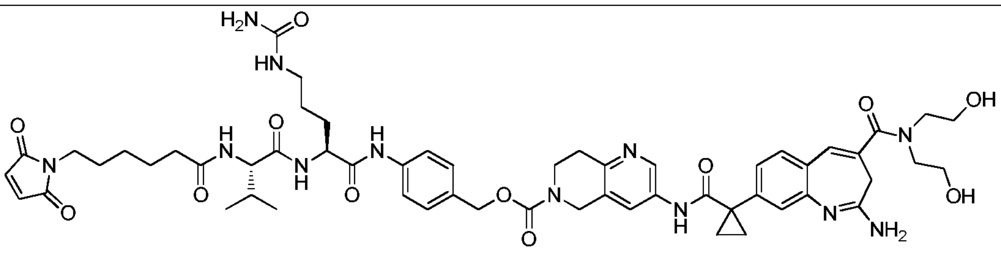 |

| |
|---|
| 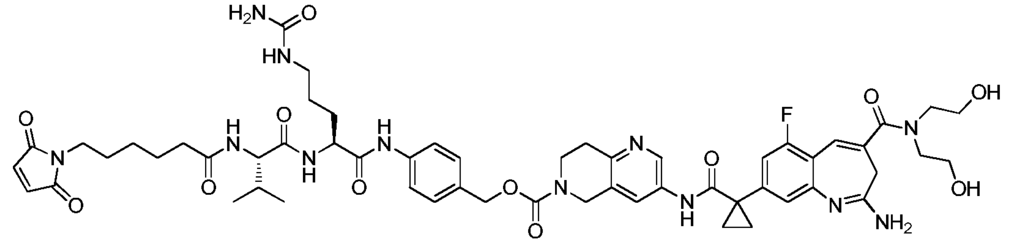 |
| 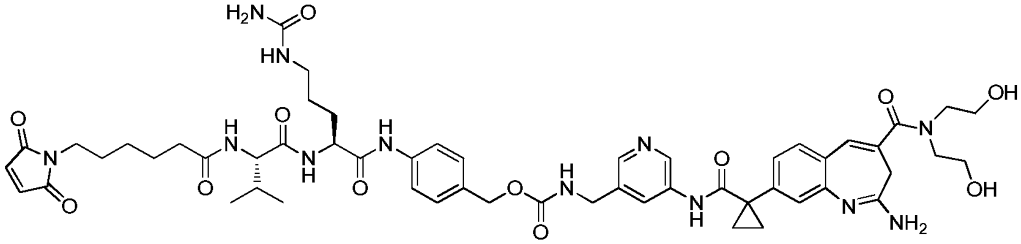 |
| 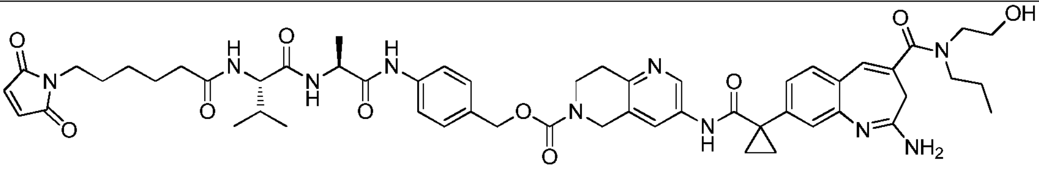 |
| 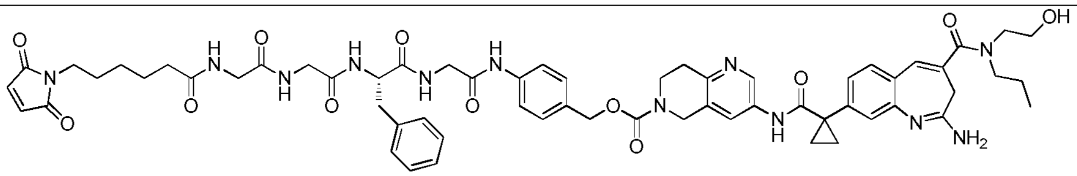 |
| 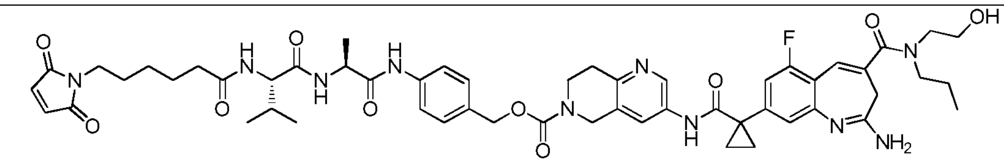 |
| 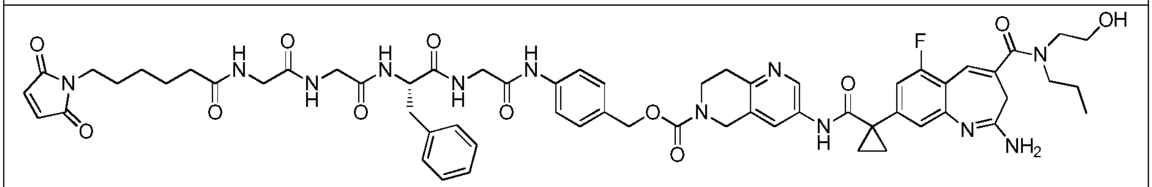 |
| 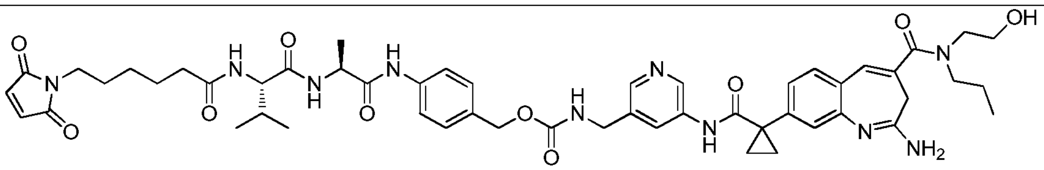 |
| 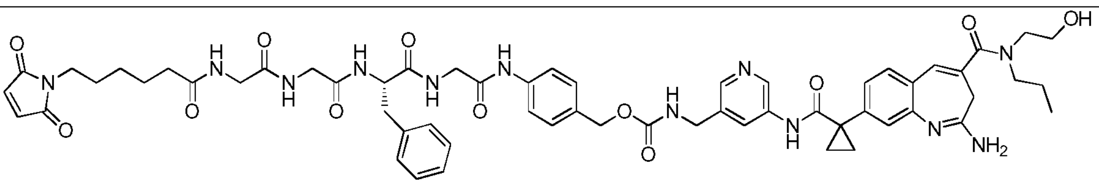 |

| |
|---|
| 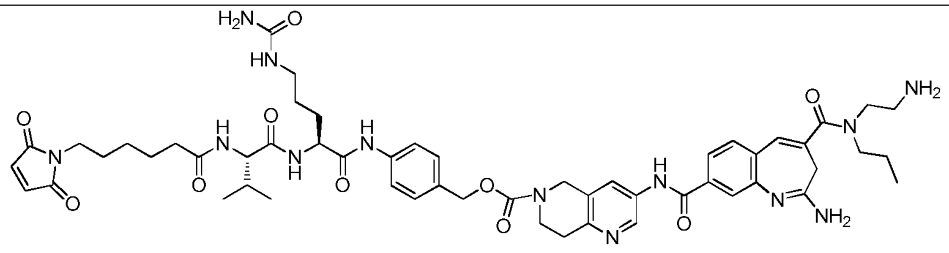 |
| 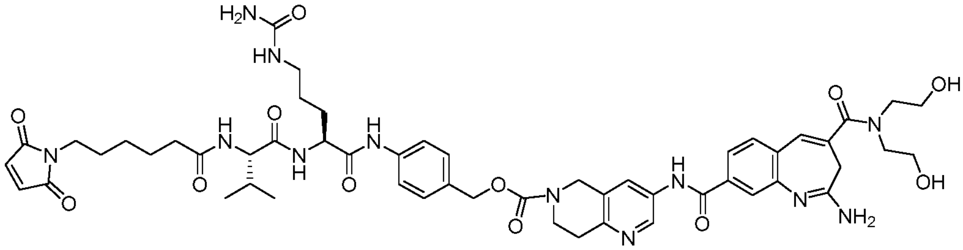 |

12. The compound of formula II or the pharmaceutically acceptable salt thereof according to claim 10 or 11, satisfying one or more of conditions of:

(1) the compound of formula D' is not a compound shown in Table 3:

Table 3

| | |
|---|---|
| 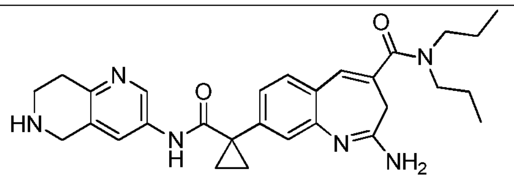 | 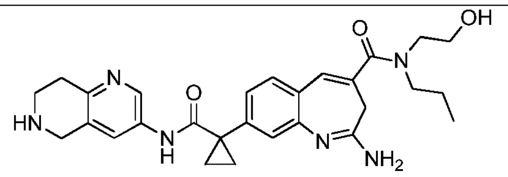 |
| 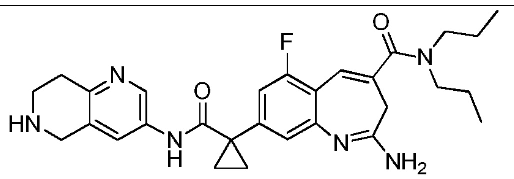 | 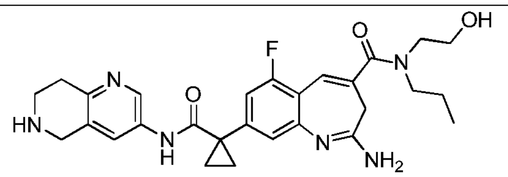 |
| 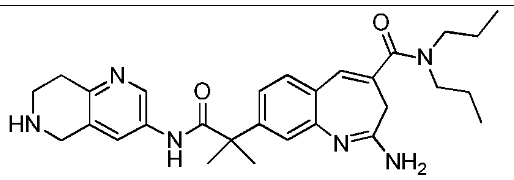 | 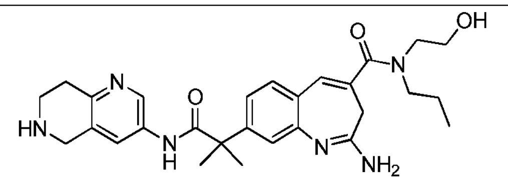 |
| 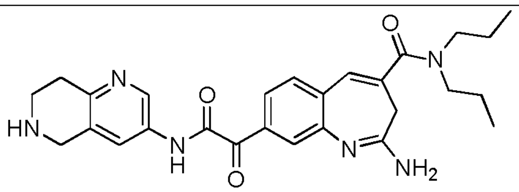 | 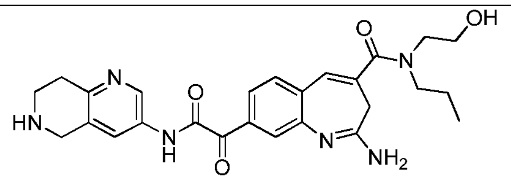 |
| 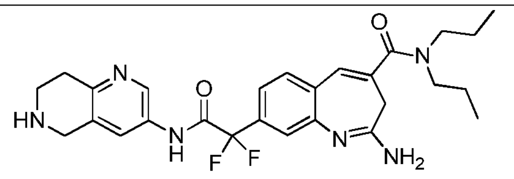 | 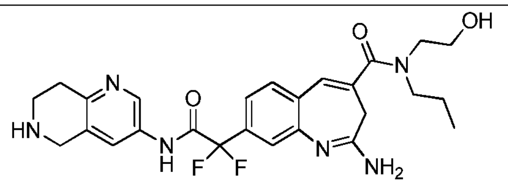 |

| | |
|---|---|
| 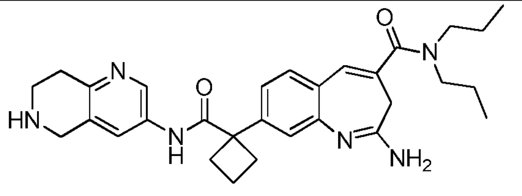 | 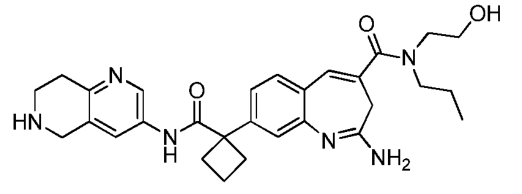 |
| 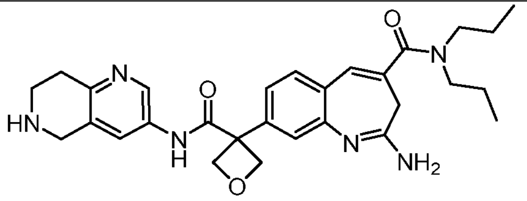 | 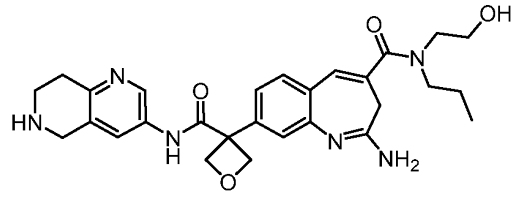 |
| 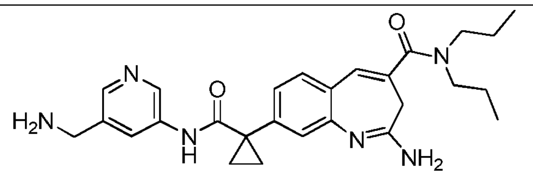 | 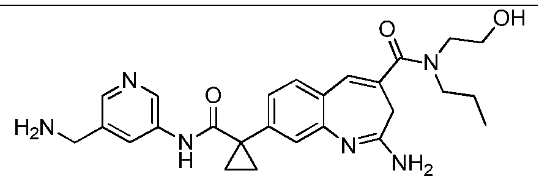 |
| 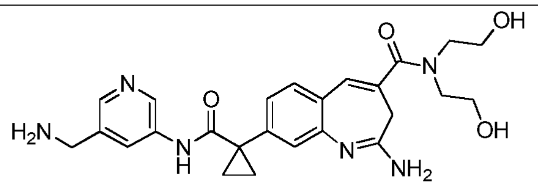 | 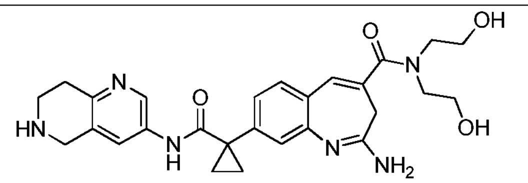 |
| 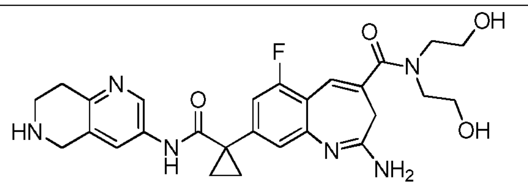 | 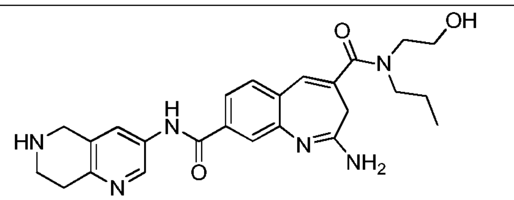 |
| 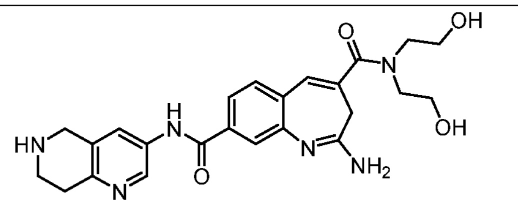 | |

; and

(2) the LX is not of a structure of:

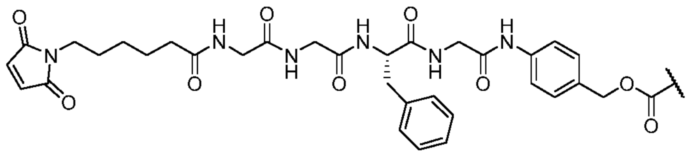

,

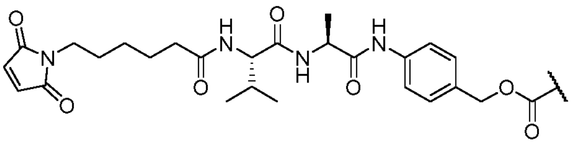

or

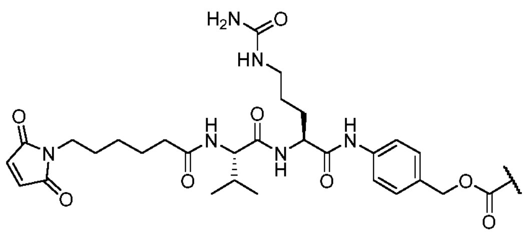

.

**13.** The compound of formula II or the pharmaceutically acceptable salt thereof according to claim 12, satisfying one or more of conditions: (1) the compound of formula D' satisfies 1, 2 or 3 of conditions of: (a) $L_1$ is independently $C_{1-6}$ alkylene or $C_{2-6}$ alkenylene; (b) $R_7$ is independently phenyl substituted with one amino, 5-6 membered heteroaryl substituted with one amino, 3-8 membered heterocycloalkyl substituted with one amino or an 8-12 membered fused ring group substituted with one amino; and (c) at least one of $R_4$ and $R_{4'}$ is $C_{1-6}$ alkoxy or $C_{1-6}$ alkyl substituted with 1, 2 or 3 $R_{4-1}$; and the $R_{4-1}$ is independently - $OC(=O)NR_aR_b$ or

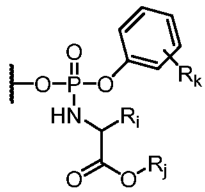
;

and

(2) the LX is M'-$(T)_w$-PEG-$(T)_o$-*, M'-PEG-*, M'-$(T)_w$-$(A)_v$-$(T)_o$-PEG-*, M'-$(T)_w$-$(A)_v$-$(T)_o$-*, M'-$(T)_w$-$L_5$-*, M'-$(T)_w$-PEG-$L_5$-*, M'-$(T)_w$-PEG-$(A)_v$-$L_5$-*, M'-$(T)_w$-PEG-$(A)_v$-$(T)_o$- * or M'-PEG-$(T)_o$-PEG-*; and * is a linking site of the LX that is attached the D;

M' is hydrogen, a linker head precursor or a linker head attached to 1 to 2 amino acids; and w is independently 1, 2, 3, 4, 5, 6, 7, 8, 9 or 10, or w is independently 0, 1, 2, 3, 4, 5, 6, 7, 8, 9 or 10;

o is independently 0, 1, 2, 3, 4, 5, 6, 7, 8, 9 or 10;

T is independently $-(CH_2)_x-C(O)-$, $-NR_h-$, -O-, -S-, $-(CH_2)_x-C_{6-14}$ arylene-$(CH_2)y$-, $-(CH_2)_x$-5-6 membered heteroarylene-$(CH_2)_y$-, $-(CH_2)_x-C_{3-6}$ cycloalkylene-$(CH_2)_y$-, $-(CH_2)_x$-3-6 membered heterocycloalkylene-$(CH_2)_y$-, $-NR_h-(CH_2)_x-C(O)-$, $-O-(CH_2)_x-C(O)-$, $-S-(CH_2)_x-C(O)-$, $-(CH_2)_xCH(NHR_h)-C(O)-$, $-(CH_2)_x-S-S-C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the $-(CH_2)_x$-5-6 membered heteroarylene-$(CH_2)_y$-, the heteroatom is selected from one or more of N, O and S, and the number of the heteroatom is 1, 2 or 3; and in "3-6 membered heterocycloalkylene" in the $-(CH_2)_x$-3-6 membered heterocycloalkylene-$(CH_2)y$-, the heteroatom is selected from one or more of N, O and S, and the number of the heteroatom is 1, 2 or 3;

$R_h$ is independently hydrogen, $C_{1-6}$ alkyl, $-PO(OH)_2$, $-PO(OCH_3)_2$, $-C(O)-(CH_2CH_2O)_n-CH_3$ or - $(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;

PEG is $-(CH_2CH_2O)_n-(CH_2)_u-$ or $-(CH_2CH_2O)_n-(CH_2)_u-C(O)-$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;

v is independently 1, 2, 3, 4 or 5;

A is independently an amino acid residue or an amino acid residue substituted with 1 $R_A$; the $R_A$ is independently $-C(O)-(CH_2CH_2O)_n-CH_3$ or $-(CH_2CH_2O)_n-CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and

$L_5$ is a self-immolative group.

**14.** The compound of formula II or the pharmaceutically acceptable salt thereof according to claim 10 or 11, satisfying one or more of conditions of:

(1) L' is -C(=O)-, $-C(=O)-NR_9-$ or $-C(=S)-NR_9-$, and a right end thereof is attached to $L_1$;

(2) $R_9$ is hydrogen;

(3) in the $L_1$, the $C_{1-6}$ alkylene is methylene or ethylene;

(4) in the $R_7$, the 8-12 membered fused ring group is a fused ring of ring A and ring B, the ring A is 5-6 membered heteroaryl or phenyl, and the ring B is a 5-6 membered heteroalkenyl ring attached to the $L_1$ through the ring A; in the 5-6 membered heteroaryl, the heteroatom is N, and the number of the heteroatom is 1 or 2; and in the 5-6 membered heteroalkene ring, the heteroatom is N, and the number of the heteroatom is 1 or 2;

(5) in the $R_7$, in "5-6 membered heteroaryl" in the 5-6 membered heteroaryl and the 5-6 membered heteroaryl substituted with 1, 2 or 3 $R_{7-2}$, the heteroatom is independently N;

(6) in the $R_7$, in "5-6 membered heteroaryl" in the 5-6 membered heteroaryl and the 5-6 membered heteroaryl substituted with 1, 2 or 3 $R_{7-2}$, the number of the heteroatom is independently 1 or 2;

(7) in the ring A, the heteroatom in the 5-6 membered heteroaryl ring is N;
(8) in the ring A, the number of the heteroatom in the 5-6 membered heteroaryl ring is 1 or 2;
(9) in the ring B, the heteroatom in the 5-6 membered heteroalkenyl ring is N;
(10) one hydrogen atom lost by the compound of formula D' is located on a secondary amine or a primary amine of $R_7$; or one hydrogen atom lost by the compound of formula D' is located on hydroxyl of $R_7$;
(11) m is 1;
(12) in the $R_8$ and $R_{8'}$, the $C_{1-6}$ alkyl is methyl, ethyl, n-propyl or isopropyl;
(13) $R_8$ and $R_{8'}$ together with the carbon atom to which they are attached form $C_{3-10}$ cycloalkylene, and the $C_{3-10}$ cycloalkylene is cyclopropyl or cyclobutyl;
(14) $R_8$ and $R_{8'}$ together with the carbon atom to which they are attached form 3-10 membered heterocycloalkylene, and a heteroatom in the 3-10 membered heterocycloalkylene is N or O;
(15) $R_8$ and $R_{8'}$ together with the carbon atom to which they are attached form 3-10 membered heterocycloalkylene, and the number of the heteroatom in the 3-10 membered heterocycloalkylene is 1 or 2;
(16) in the $R_1$, $R_2$ and $R_3$, the halogen is F, Cl or Br;
(17) in the $R_4$ and $R_{4'}$, the $C_{1-6}$ alkyl is methyl, ethyl, n-propyl or isopropyl;
(18) in the $R_4$ and $R_{4'}$, the $C_{1-6}$ alkoxy is methoxy, ethoxy, n-propoxy or isopropoxy;
(19) in the $R_4$ and $R_{4'}$, $C_{1-6}$ alkyl in the $C_{1-6}$ alkyl substituted with 1, 2 or 3 $R_{4-1}$ is methyl, ethyl, n-propyl or isopropyl;
(20) in the $R_a$ and $R_b$, the $C_{1-6}$ alkyl is methyl, ethyl, n-propyl or isopropyl;
(21) in the $R_a$ and $R_b$, the $C_{3-10}$ cycloalkyl is cyclopropyl, cyclobutyl or cyclopentyl;
(22) in the $R_i$, the $C_{1-6}$ alkyl is methyl, ethyl, n-propyl or isopropyl;
(23) in the $R_y$, the $C_{1-6}$ alkyl is methyl, ethyl, n-propyl or isopropyl;
(24) in the $R_j$, the $C_{1-6}$ alkyl is methyl, ethyl, n-propyl or isopropyl;
(25) in the $R_j$, in the $C_{6-10}$ aryl-$C_{1-6}$ alkyl, the $C_{6-10}$ aryl is phenyl;
(26) in the $R_j$, in the $C_{6-10}$ aryl-$C_{1-6}$ alkyl, the $C_{1-6}$ alkyl is methyl, ethyl, n-propyl or isopropyl; and
(27) the LX is M'-$(T)_w$-PEG-$(T)_o$-*, M'-PEG-*, M'-$(T)_w$-*, M'-$(T)_w$-$(A)_v$-$(T)_o$-PEG-*, M'-$(T)_w$-$(A)_v$-$(T)_o$-*, M'-$(T)_w$-$L_5$-*, M'-$(T)_w$-PEG-$L_5$-*, M'-$(T)_w$-PEG-$(A)_v$-$L_5$-*, M'-$(T)_w$-PEG-$(A)_v$-$(T)_o$-*, M'-PEG-$(T)_o$-PEG-* or M'-$(T)_w$-$(A)_v$-$L_5$-*; and * is a linking site of the LX that is attached to the D;

M' is hydrogen, a linker head precursor or a linker head attached to 1 to 2 amino acids;
w is independently 1, 2, 3, 4, 5, 6, 7, 8, 9 or 10, or w is independently 0, 1, 2, 3, 4, 5, 6, 7, 8, 9 or 10;
o is independently 0, 1, 2, 3, 4, 5, 6, 7, 8, 9 or 10;
T is independently -$(CH_2)_x$-C(O)-, -$NR_h$-, -O-, -S-, -$(CH_2)_x$-$C_{6-14}$ arylene-$(CH_2)_y$-, -$(CH_2)_x$-5-6 membered heteroarylene-$(CH_2)_y$-, -$(CH_2)_x$-$C_{3-6}$ cycloalkylene-$(CH_2)_y$-, -$(CH_2)_x$-3-6 membered heterocycloalkylene-$(CH_2)_y$-, -$NR_h$-$(CH_2)_x$-C(O)-, -O-(CH2)$_x$-C(O)-, -S-$(CH_2)_x$-C(O)-, -$(CH_2)_x$CH($NHR_h$)-C(O)-, -$(CH_2)_x$-S-S-$C_{1-6}$ alkylene-, $C_{1-6}$ alkylene or $C_{2-6}$ alkenylene; x and y are independently any integer of 0, 1, 2, 3, 4, 5, 6, 7, 8, 9 or 10; in "5-6 membered heteroarylene" in the -$(CH_2)_x$-5-6 membered heteroarylene-$(CH_2)_y$-, the heteroatom is selected from one or more of N, O and S, and the number of the heteroatom is 1, 2 or 3; and in "3-6 membered heterocycloalkylene" in the -$(CH_2)_x$-3-6 membered heterocycloalkylene-$(CH_2)_y$-, the heteroatom is selected from one or more of N, O and S, and the number of the heteroatom is 1, 2 or 3;
$R_h$ is independently hydrogen, $C_{1-6}$ alkyl, -$PO(OH)_2$, -$PO(OCH_3)_2$, -C(O)-$(CH_2CH_2O)_n$-$CH_3$ or -$(CH_2CH_2O)_n$-$CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;
PEG is -$(CH_2CH_2O)_n$-$(CH_2)_u$- or -$(CH_2CH_2O)_n$-$(CH_2)_u$-C(O)-; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and u is independently 0, 1, 2, 3, 4 or 5;
v is independently 1, 2, 3, 4 or 5;
A is independently an amino acid residue or an amino acid residue substituted with 1 $R_A$; the $R_A$ is independently -C(O)-$(CH_2CH_2O)_n$-$CH_3$ or -$(CH_2CH_2O)_n$-$CH_3$; and n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and
$L_5$ is a self-immolative group.

**15.** The compound of formula II or the pharmaceutically acceptable salt thereof according to claim 14, satisfying one or more of conditions of:

(1) $R_7$ is

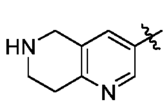, 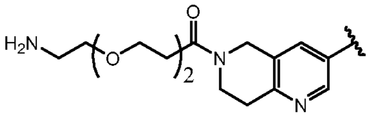, 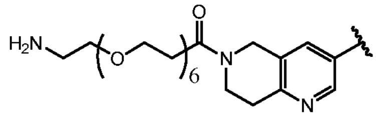,

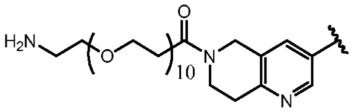, 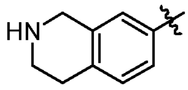, 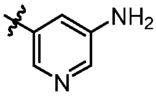, 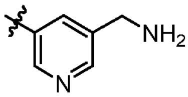, 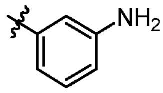,

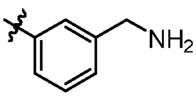, 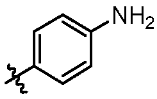, 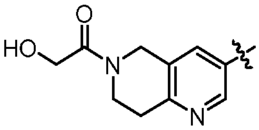 or 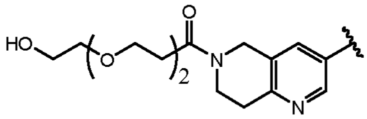;

(2) M' is

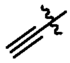, 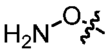, 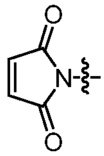, 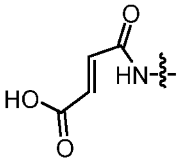, 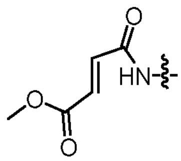, 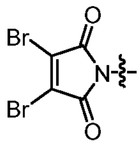,

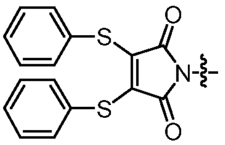, 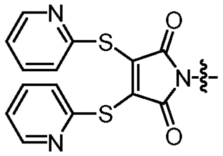, 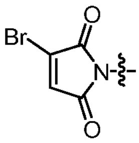, 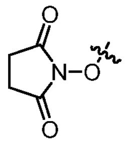, 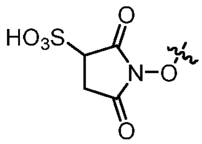,

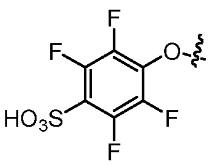, 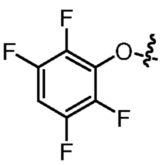, 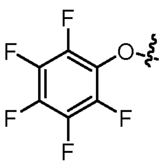, 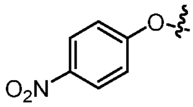, 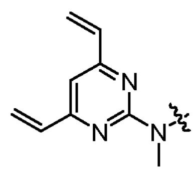,

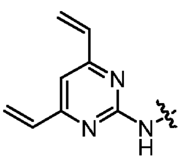, 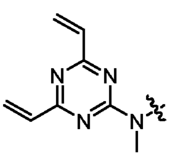, 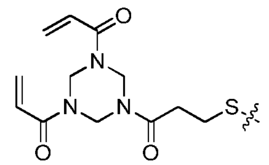, 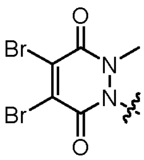, 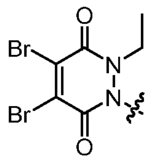,

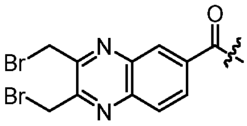, 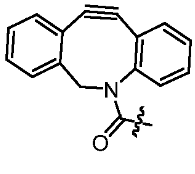, 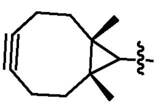, 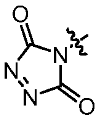, 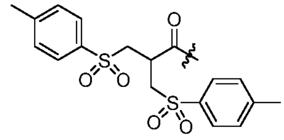,

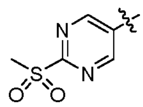, 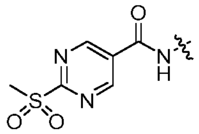, 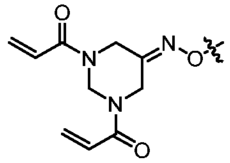, 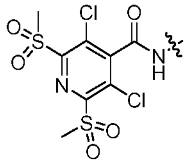, 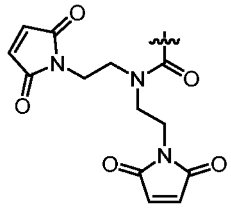,

or

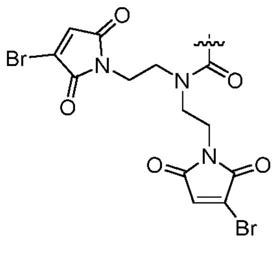;

(3) $-(A)_v-$ is

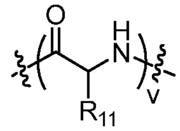;

each $R_{11}$ is independently hydrogen, methyl, ethyl, n-propyl, isopropyl, isobutyl, $-SO_3H$,

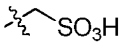, 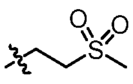, 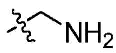, 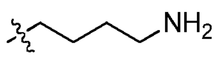, 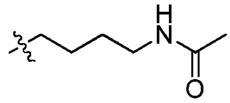,

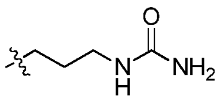, 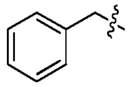, 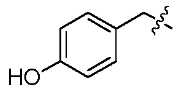, 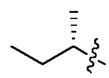, 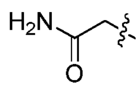, 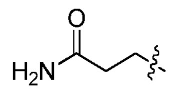,

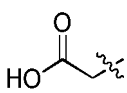, 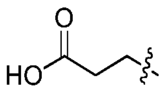 or 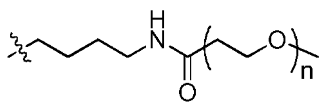;

or $R_{11}$; and an adjacent nitrogen atom form a five-membered heterocycloalkyl; and v is any integer from 1 to 4, and v is preferably 1 or 2;

(4) A is attached to $-(T)_o-$ or $-L_5-$ through a carbonyl terminus;

(5) the $L_5$ is

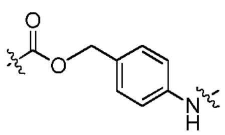, 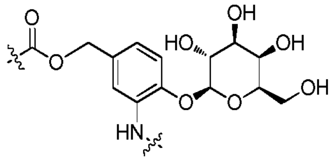, 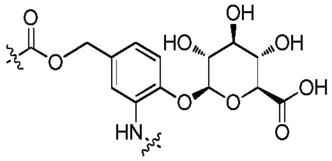,

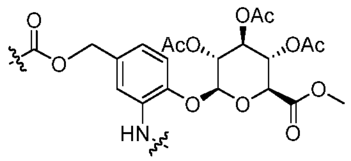,
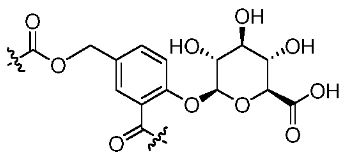,
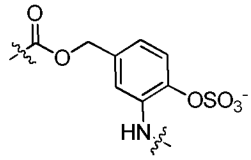

or

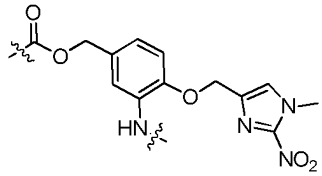;

(6) the $-L_5-$ is attached to the D through a carbonyl terminus;
(7) $-(T)_w-$ is

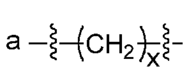,
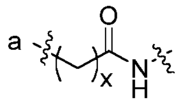,
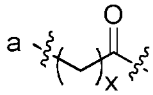,
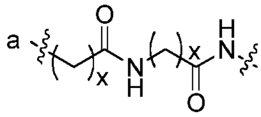,

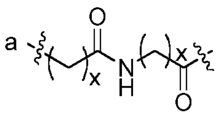,
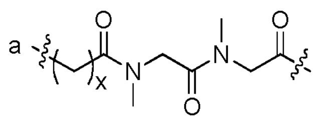,
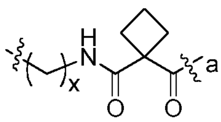,
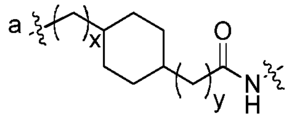,

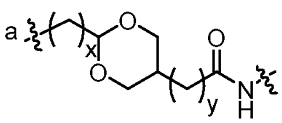,
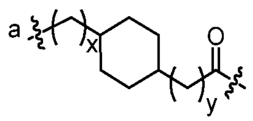,
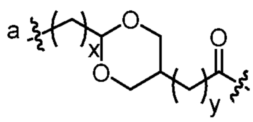,
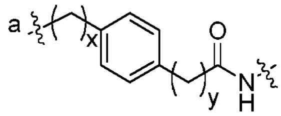,

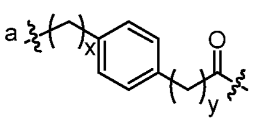,
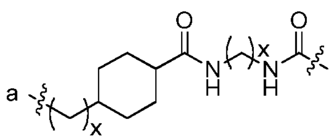,
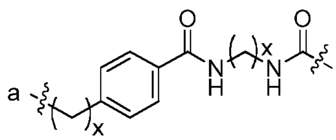,

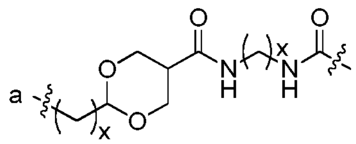,
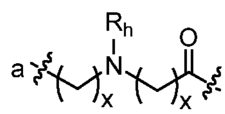
or
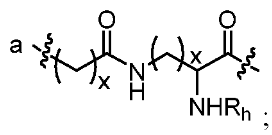;

and the $-(T)_w-$ is attached to the M' through an a-terminus;
(8) the $-(T)_o-$ is

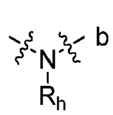,
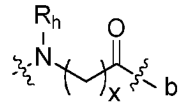,
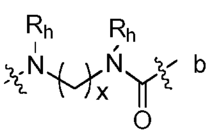,
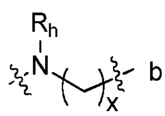
or
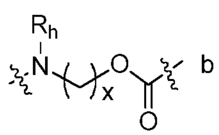;

and the $-(T)_o-$ is attached to the -PEG- or the D via a b-terminus; and
(9) LX is any one of combinations of: A) M'-PEG-; B) $M'-(CH_2)_x-$; C) $M'-(CH_2)_x-C(O)-$; D) $M'-(CH_2)_x-(C_{5-6}$ cycloalkylene)$-(CH_2)_y-C(O)NH-PEG-$; E) $M'-(CH_2)_x-$(5-6 membered heterocyclylene)$-(CH_2)_y-C(O)NH-PEG-$;

F) M'-$(CH_2)_x$-(phenylene)-$(CH_2)_y$-C(O)NH-PEG-; G) M'-$(CH_2)_x$-C(O)NH-PEG-; H) M'-$(CH_2)_x$-C(O)NH-$(CH_2)_x$-C(O)NH-PEG-; I) M'-$(CH_2)_x$-C(O)NH-$(CH_2)_x$-C(O)NH-PEG-NH-$(CH_2)_x$-C(O)-; J) M'-$(CH_2)_x$-($C_{5-6}$ cycloalkylene)-$(CH_2)$y-C(O)NH-$(CH_2)_x$-NHC(O)-; K) M'-$(CH_2)_x$-(5-6 membered heterocyclylene)-$(CH_2)_y$-C(O)NH-$(CH_2)_x$-NHC(O)-; L) M'-$(CH_2)_x$-(phenylene)-$(CH_2)_y$-C(O)NH-$(CH_2)_x$-NHC(O)-; M) M'-$(CH_2)_x$-C(O)NH-$(CH_2)_x$-C(O)-$L_5$-; N) M'-$(CH_2)_x$-C(O)NH-PEG-$L_5$-; O) M'-$(CH_2)_x$-(phenylene)-$(CH_2)_y$-C(O)-(A),-; P) M'-$(CH_2)_x$-C(O)NH-($C_{3-6}$ cycloalkylene)-C(O)-$(A)_v$-; Q) M'-$(CH_2)_x$-C(O)NH-PEG-$(A)_v$-; R) M'-$(CH_2)_x$-C(O)NH-PEG-$(A)_v$-$L_5$-; S) M'-$(CH_2)_x$-($C_{5-6}$ cycloalkylene)-C(O)-; T) M'-$(CH_2)_x$-(5-6 membered heterocyclylene)-C(O)-; U) M'-(phenylene)-$(CH_2)_y$-C(O)-; V) M'-$(CH_2)_x$-C(O)-$(A)_v$-NH-PEG-; W) M'-$(CH_2)_x$-C(O)-(A),-NH-$(CH_2)_x$-C(O)-; X) M'-$(CH_2)_x$-C(O)-(A),-; Y) M'-$(CH_2)_x$-(5-6 membered heterocyclylene)-C(O)-$(A)_v$-; or Z) M'-$(CH_2)_x$-C(O)NH-$CH_2$-CH($NHR_h$)-C(O)-(A),-; AA) M'-(phenylene)-(5-6 membered heterocyclylene)-C(O)-; AB) M'-(phenylene)-$(CH_2)_y$-C(O)NH-$(CH_2)$x-PEG-$(A)_v$-; AC) M'-(phenylene)-C(O)NH-$(CH_2)$x-(A),-$L_5$- or AD) M'-$(CH_2)$x-PEG-$(A)_v$-;

or LX is M'-$(T)_w$-$(A)_v$-$L_5$-*;
when LX is M'-$(T)_w$-$(A)_v$-$L_5$-*, a combination of M', -$(T)_w$-, -$(A)_v$- and -$L_5$- does not simultaneously satisfy conditions of: M' is maleimidyl; -$(T)_w$- is hexanoyl; -$(A)_v$- is -Val-Cit-, -Val-Ala or -Gly-Gly-Phe-Gly-; and -$L_5$- is PABC; and preferably, L is M'-$CH_2$-($C_{5-6}$ cycloalkylene)-C(=O)-$(A)_v$-$L_5$-, M'-$CH_2$-(5-6 membered heterocyclylene)-C(=O)-$(A)_v$-$L_5$-, M'-(phenylene)-$CH_2$-C(=O)-$(A)_v$-$L_5$- or M'-$(CH_2)_x$-C(=O)NH-$CH_2$-CH($NHR_h$)-C(O)-$(A)_v$-$L_5$-;
when LX is M'-$(T)_w$-PEG-$(A)_v$-$L_5$-*, the LX is M'-$(CH_2)_x$-C(=O)NH-PEG-$(A)_v$-$L_5$-, M'-(phenylene)-$CH_2$-C(=O)NH-PEG-$(A)_v$-$L_5$-, M'-$CH_2$-($C_{5-6}$ cycloalkylene)-C(=O)NH-PEG-$(A)_v$-$L_5$- or M'-$CH_2$-(5-6 membered heterocyclylene)-C(=O)NH-PEG-$(A)_v$-$L_5$-; and
when LX is M'-$(T)_w$-$(A)_v$-$(T)_o$-*, the LX is M'-$(CH_2)_x$-C(=O)NH-$(A)_v$-NH-$CH_2$-.

**16.** The compound of formula II or the pharmaceutically acceptable salt thereof according to claim 15, satisfying one or more of conditions of:

(1) the D is a structure shown in Table 5:

Table 5

| | |
|---|---|
| 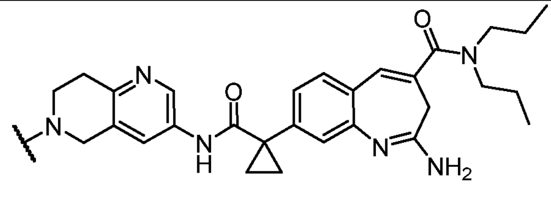 | 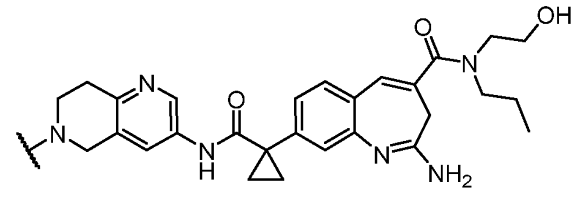 |
| 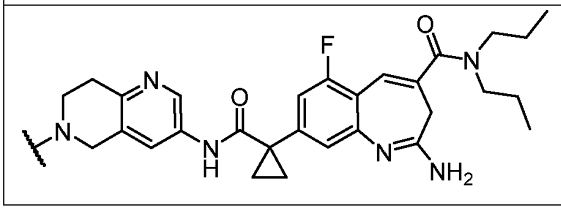 | 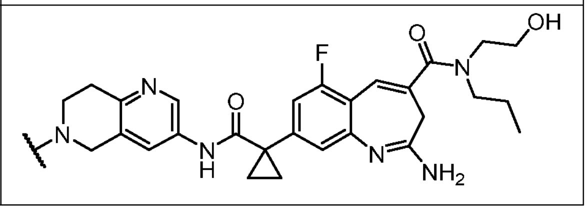 |

| | |
|---|---|
| 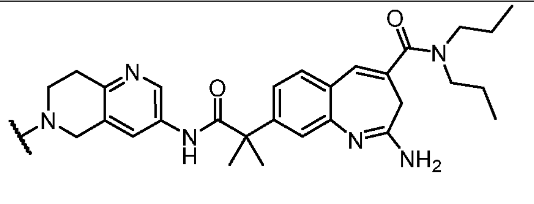 | 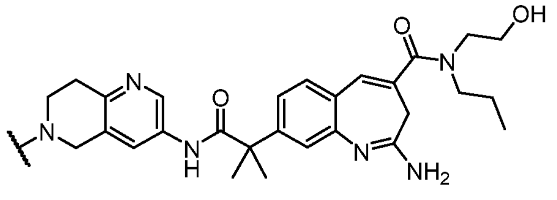 |
| 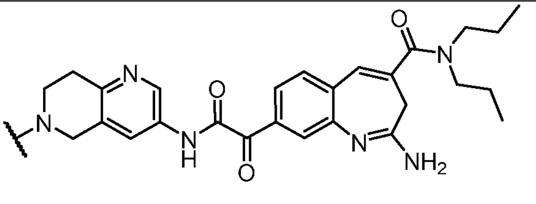 | 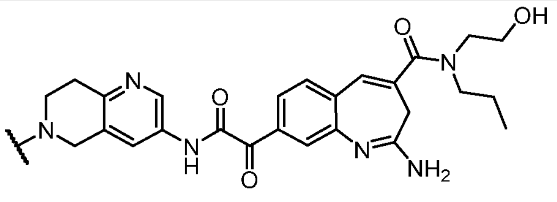 |
| 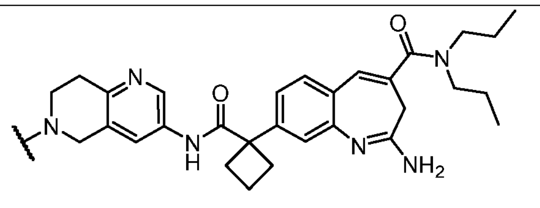 | 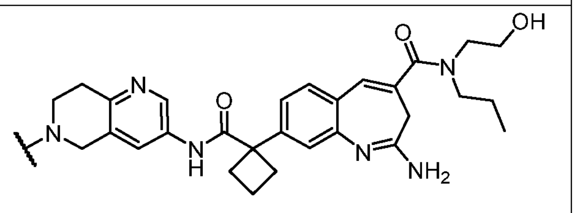 |
| 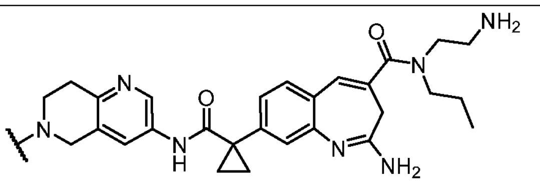 | 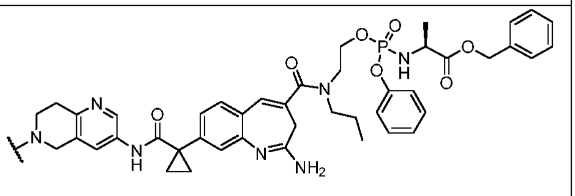 |
| 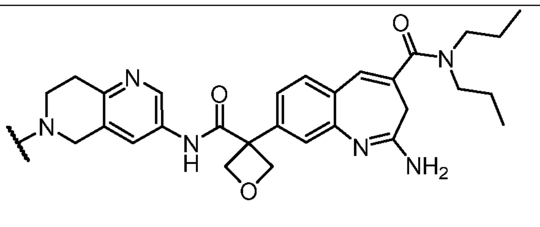 | 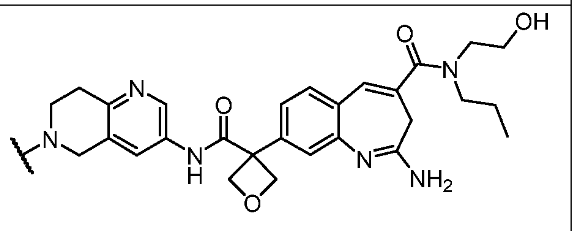 |
| 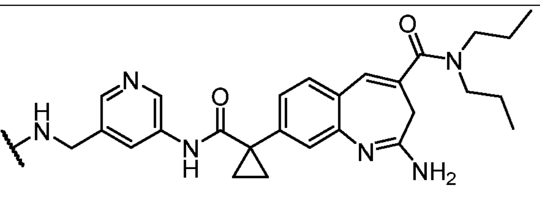 | 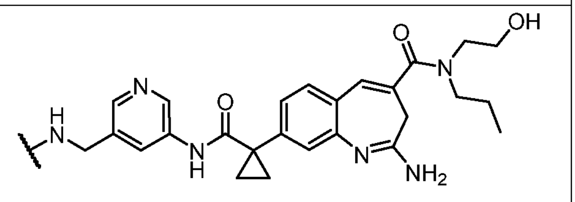 |
| 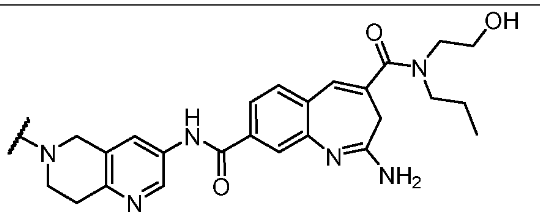 | 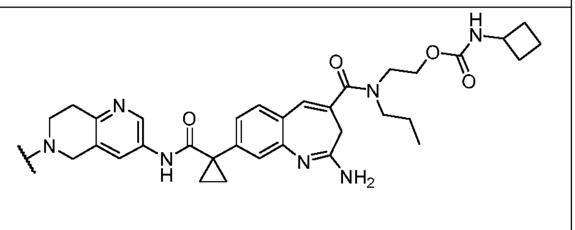 |
| 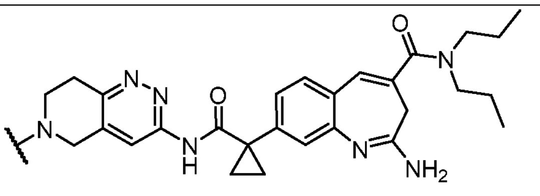 | 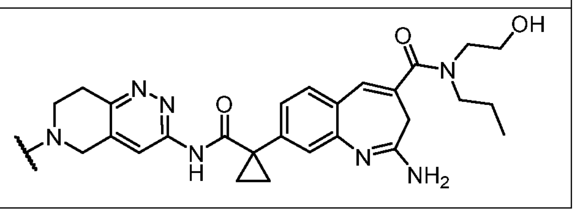 |

| | |
|---|---|
| 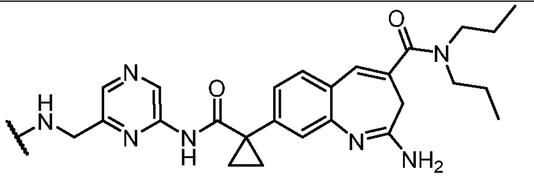 | 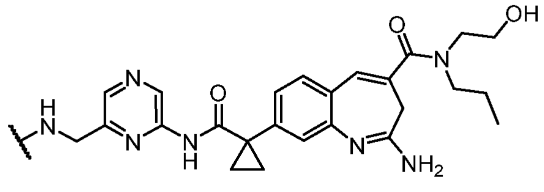 |
| 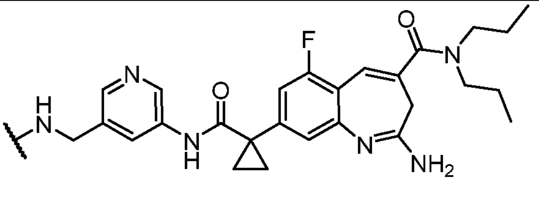 | 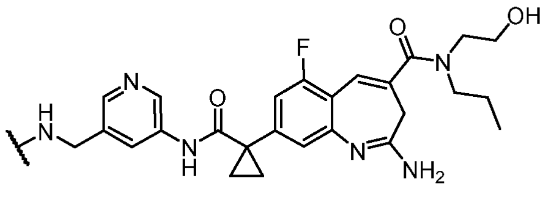 |
| 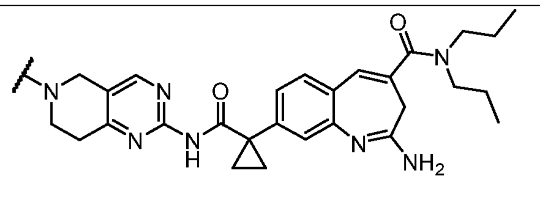 | 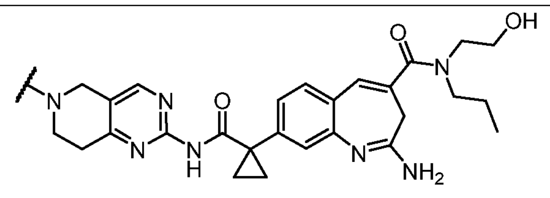 |
| 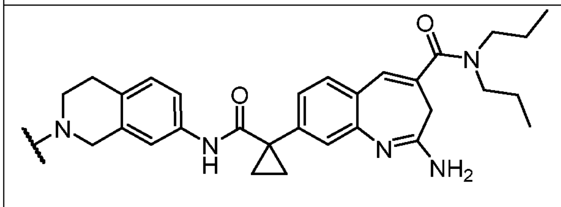 | 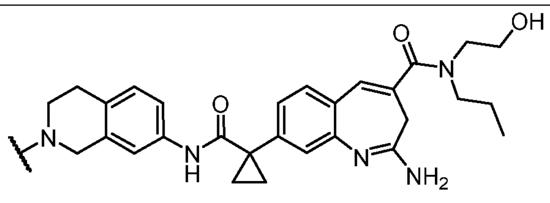 |
| 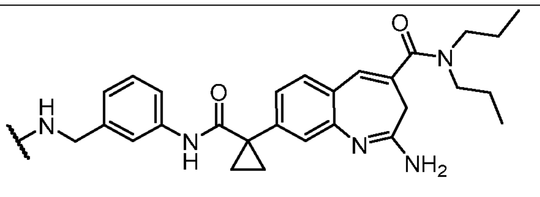 | 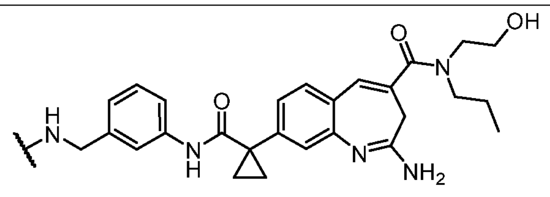 |
| 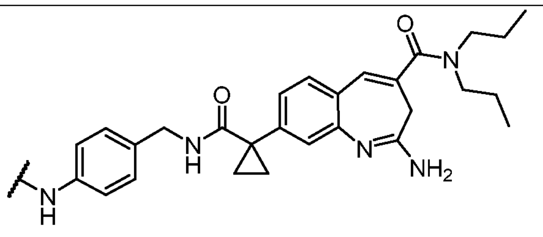 | 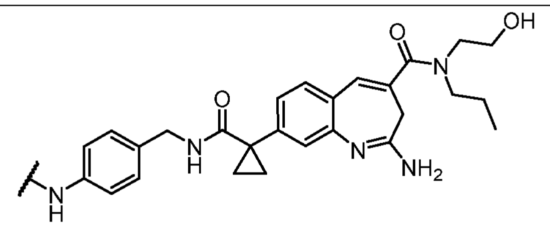 |
| 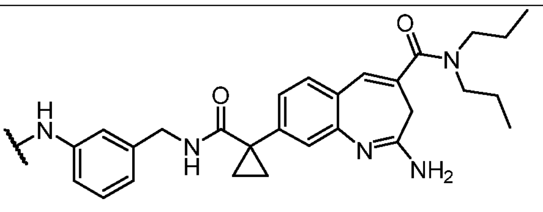 | 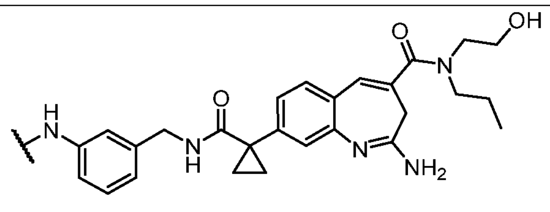 |
| 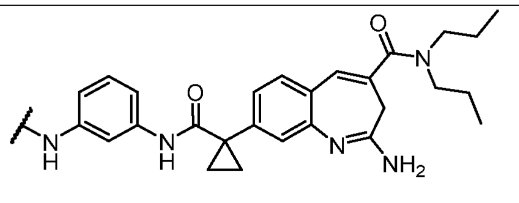 | 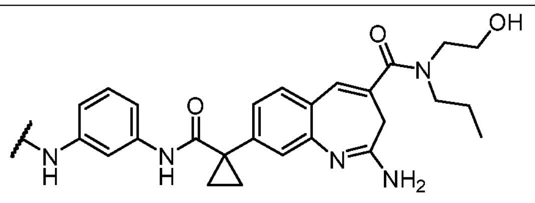 |

| | |
|---|---|
| 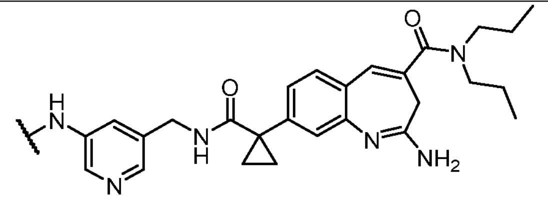 | 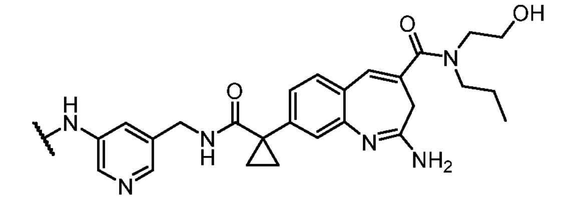 |
| 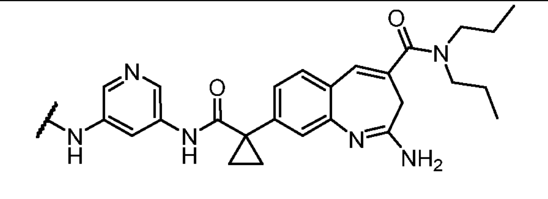 | 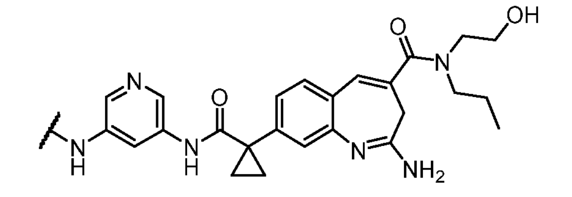 |
| 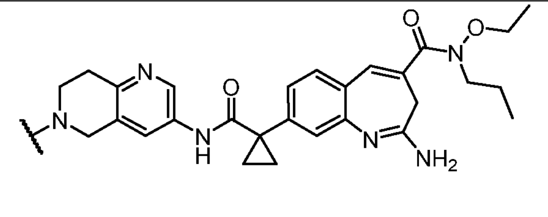 | 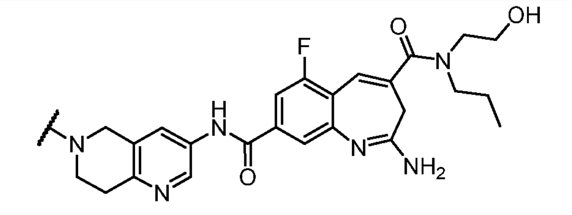 |
| 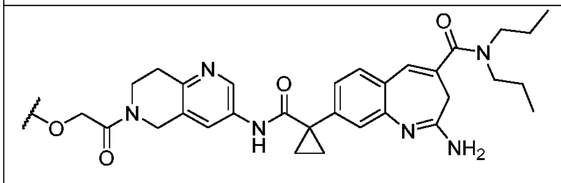 | 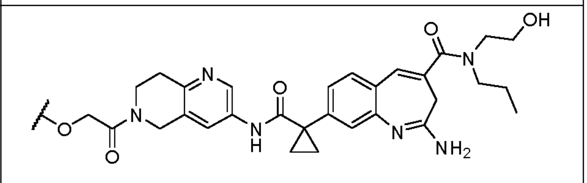 |
| 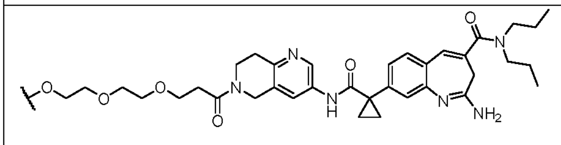 | 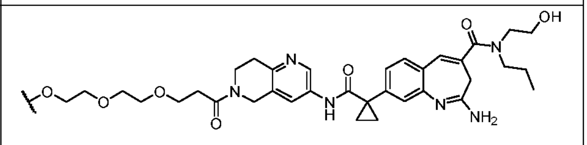 |
| 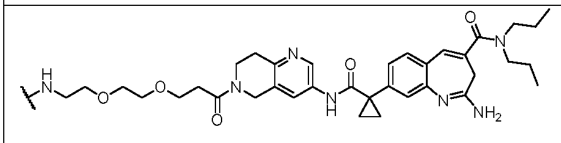 | 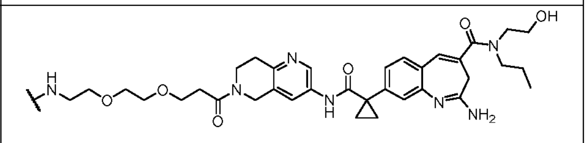 |
| 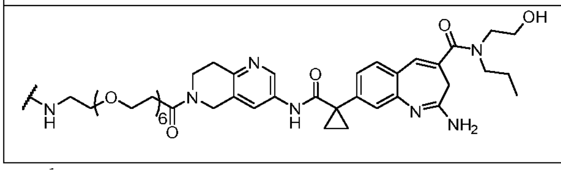 | 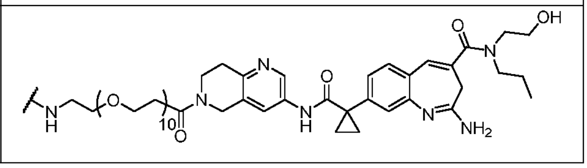 |

; and
(2) LX is

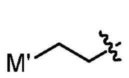 , 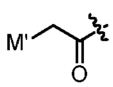 , 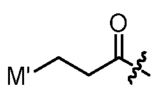 , 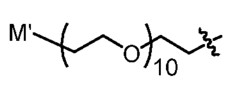 , 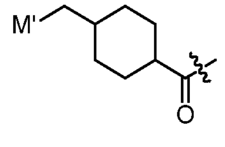 ,

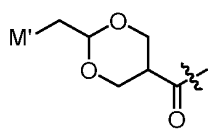 , 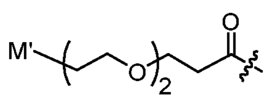 , 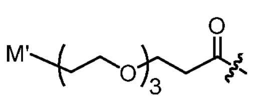 , 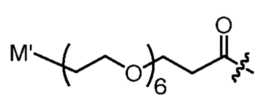 ,

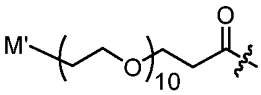,
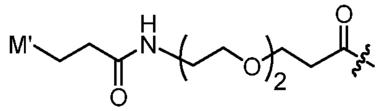,
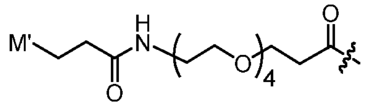,

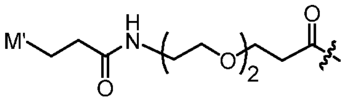,
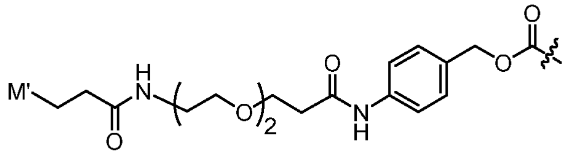,

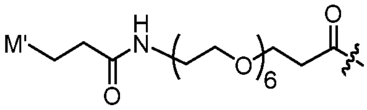,
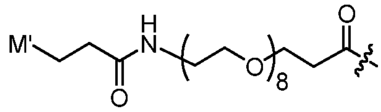,

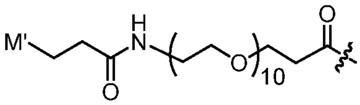,
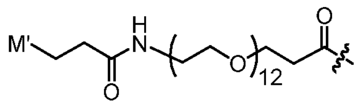,
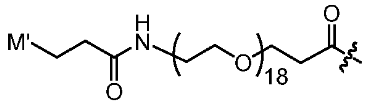,

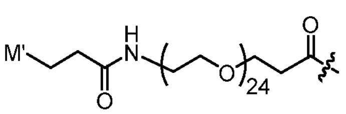,
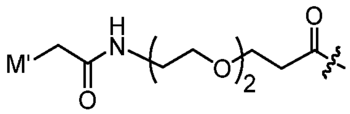,
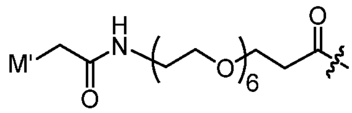,

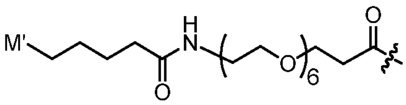,
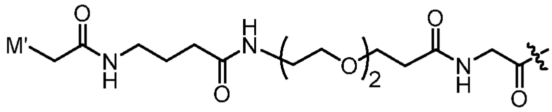,

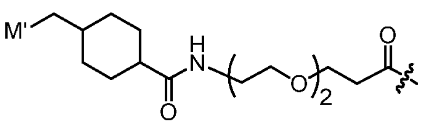,
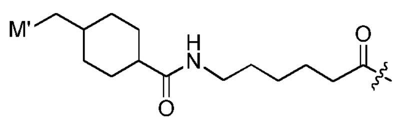,

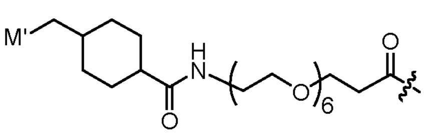,
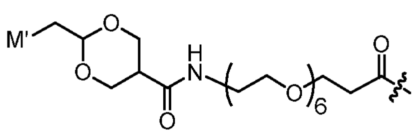,

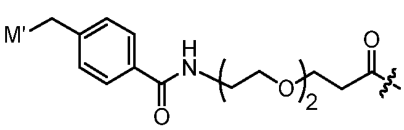,
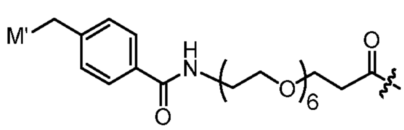,

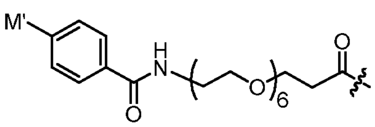,
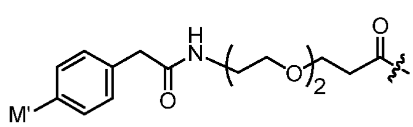,

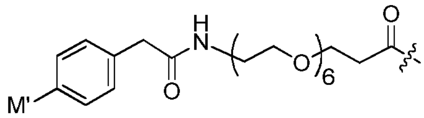,
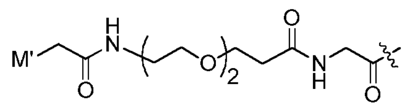,

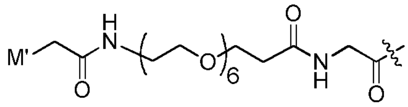,
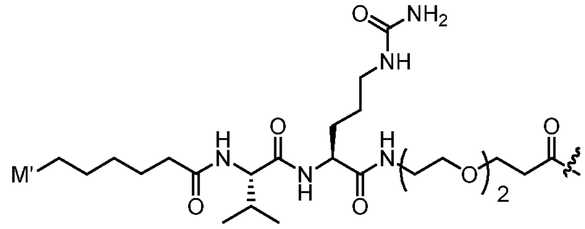,

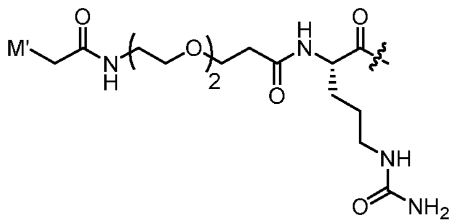,
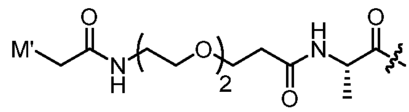,

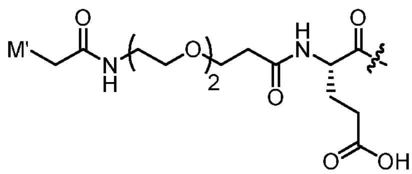
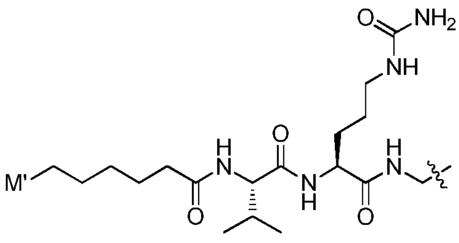,

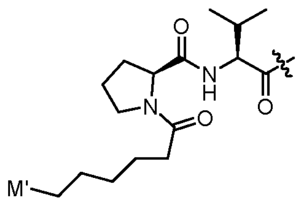,
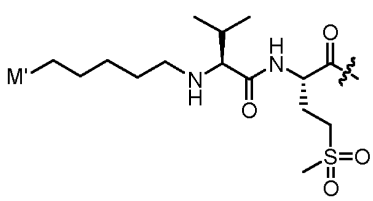,
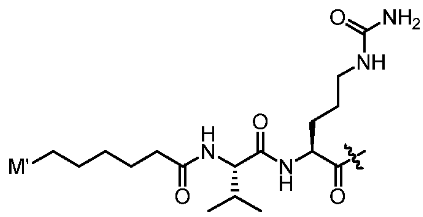,

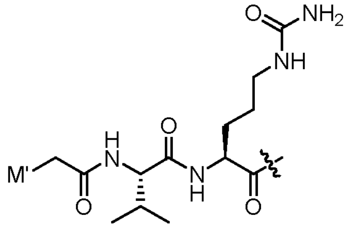,
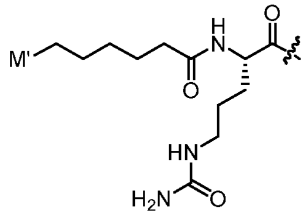,
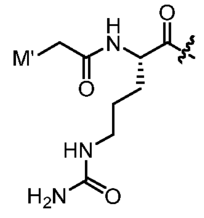,

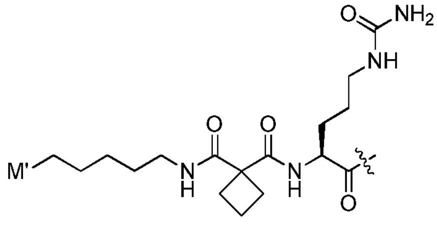,
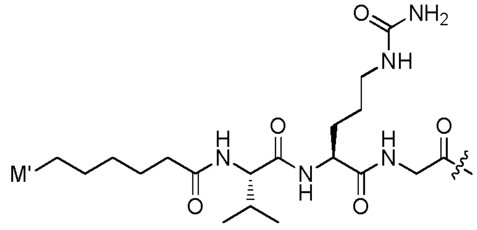

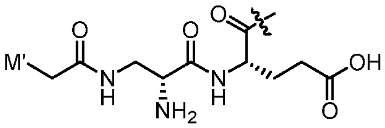

,

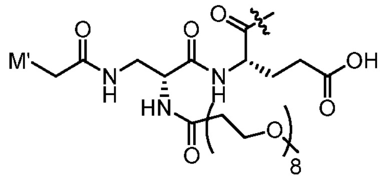

,

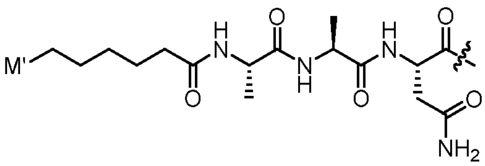

,

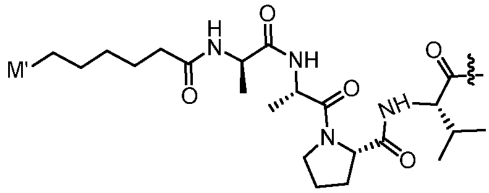

,

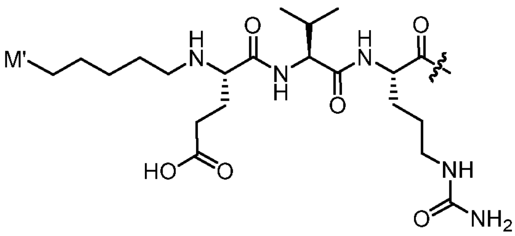

,

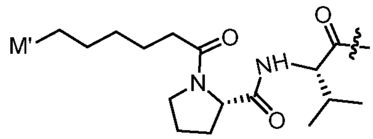

,

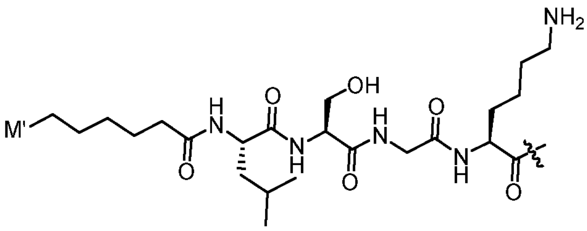

,

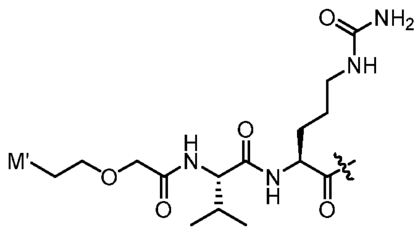

,

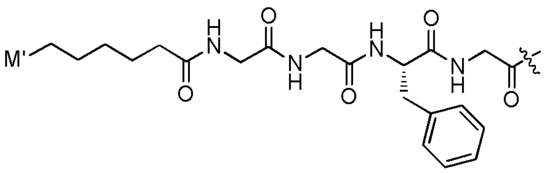

,

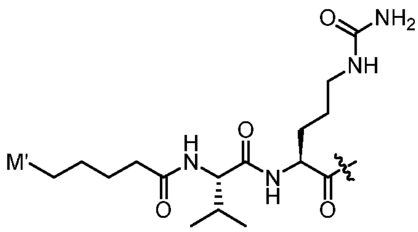

,

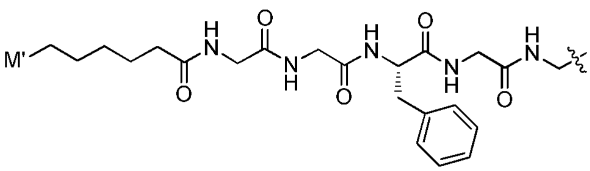

,

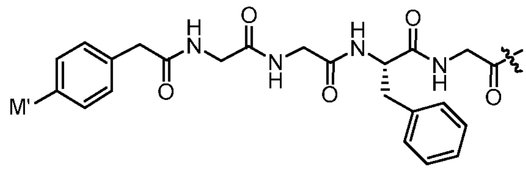

,

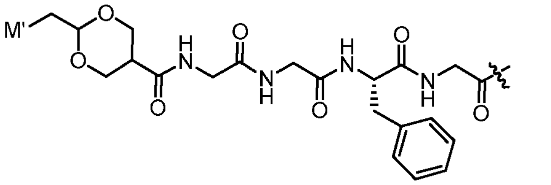

,

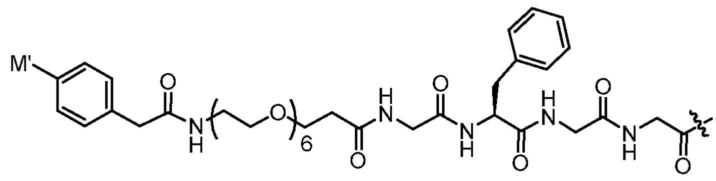

,

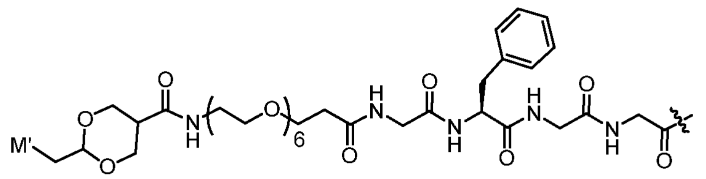

,

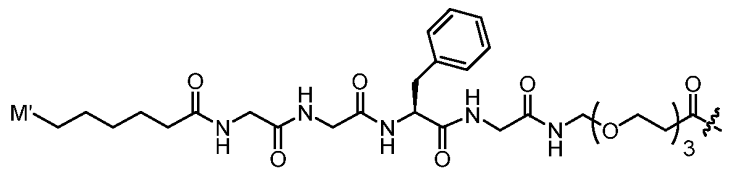

,

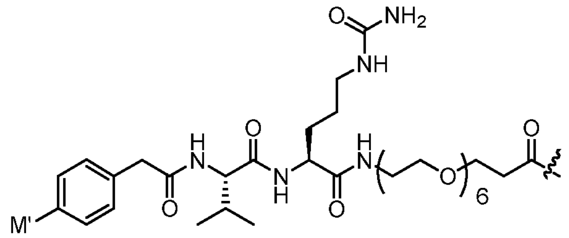

,

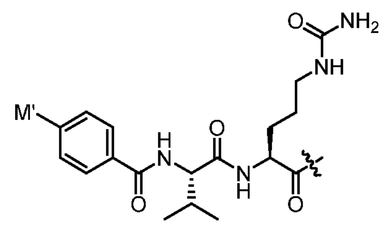

,

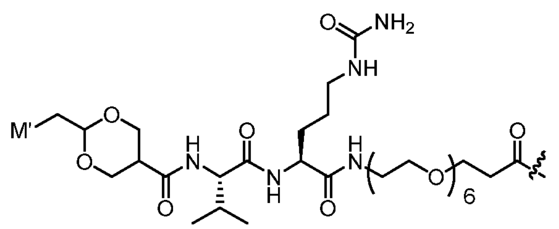

,

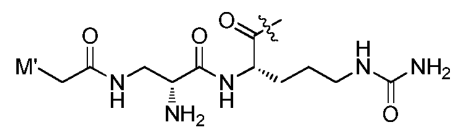

,

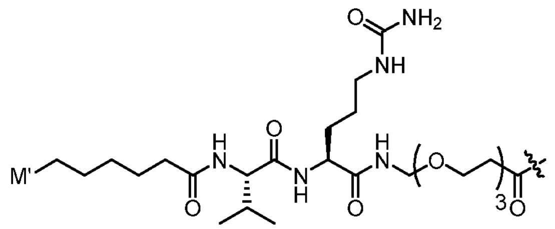

,

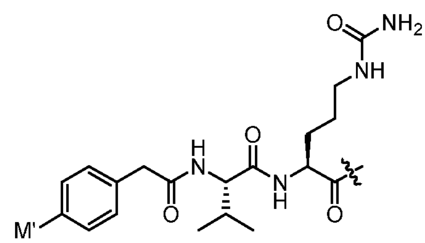

,

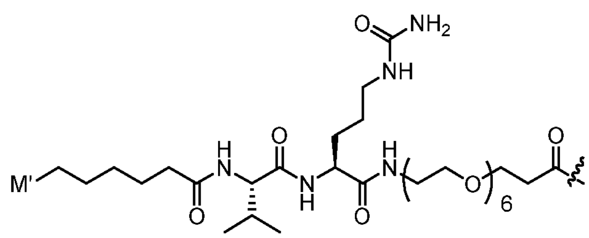

,

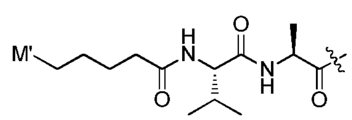

,

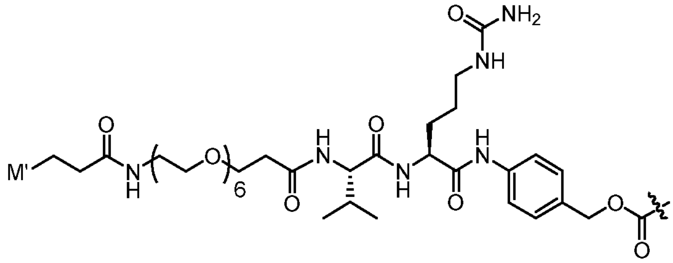

,

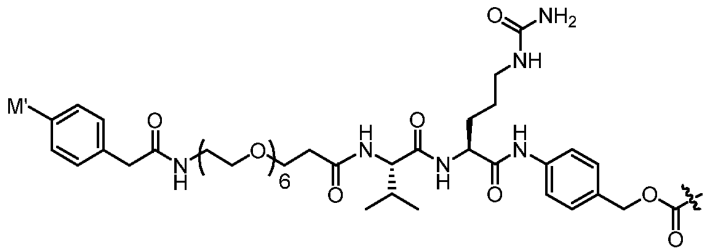

,

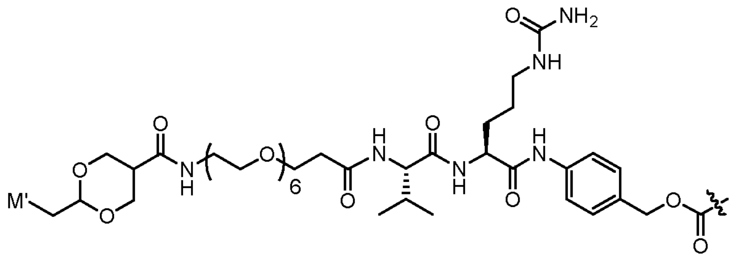

,

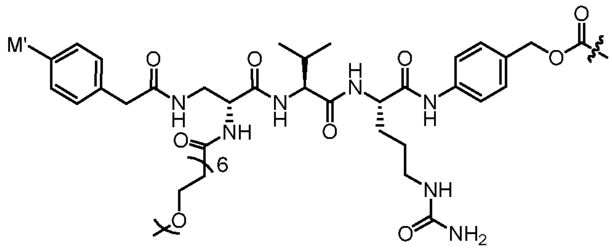

,

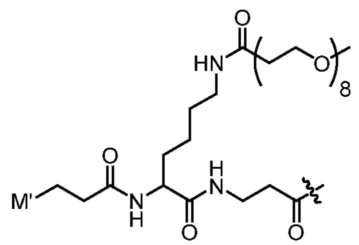

,

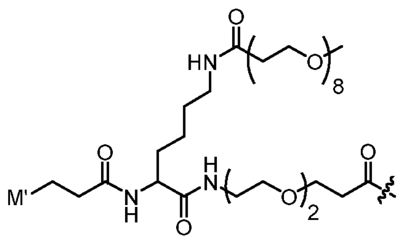

,

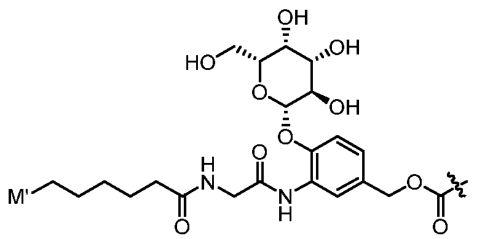

,

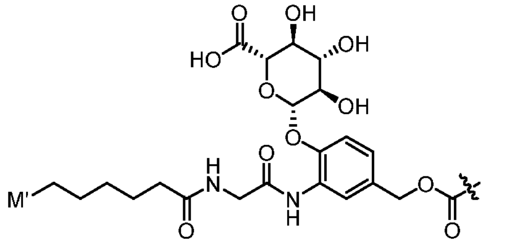

,

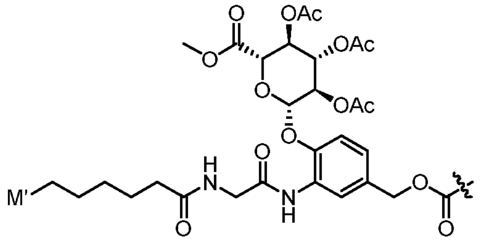

,

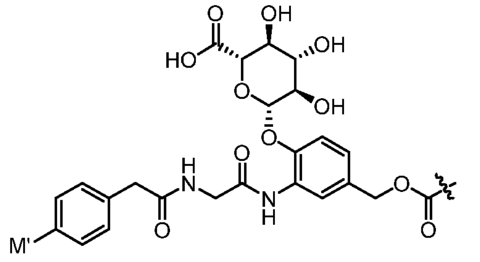,
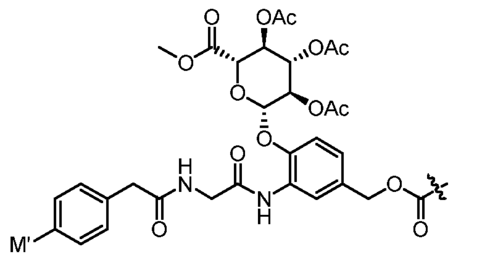,

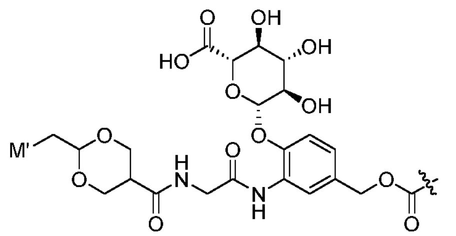,
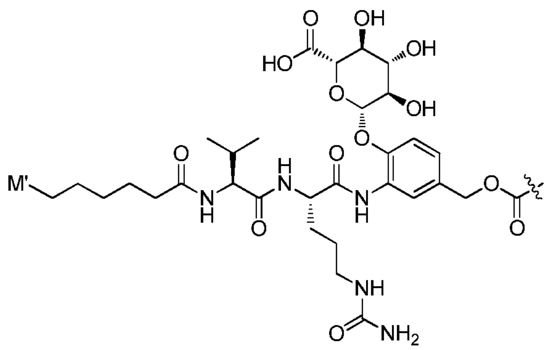,

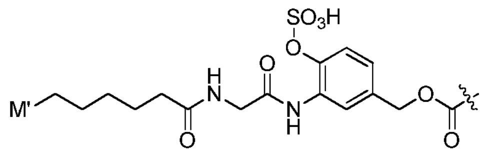,
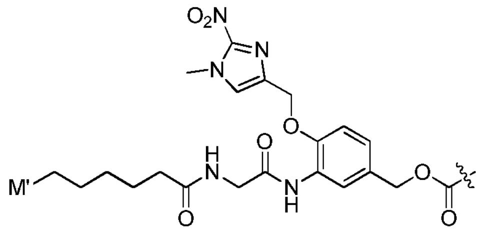,

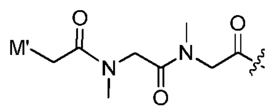,
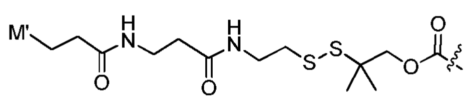,

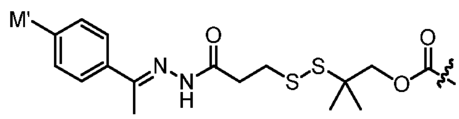,
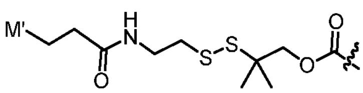,
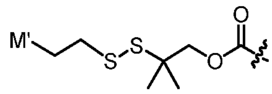,

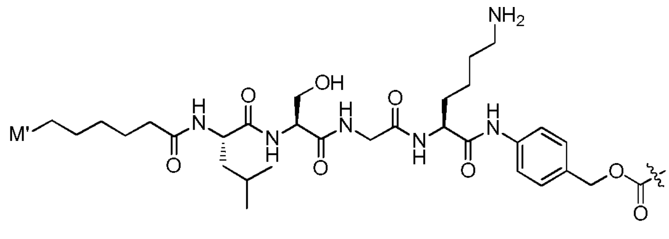,

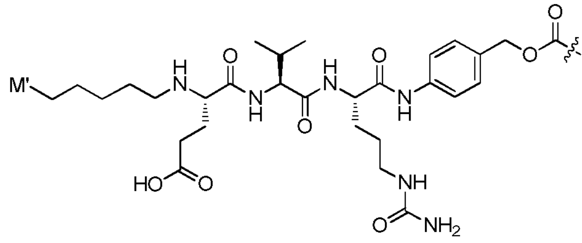
,
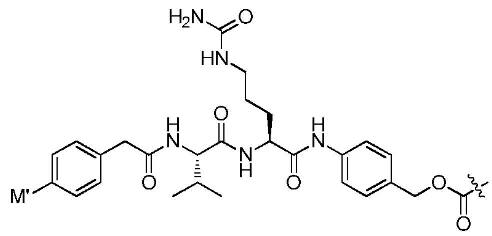
,

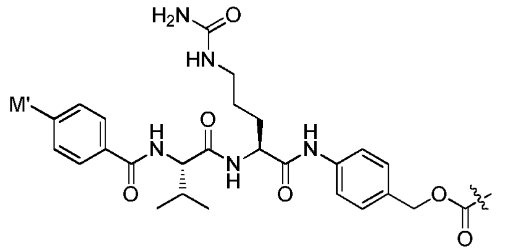
,
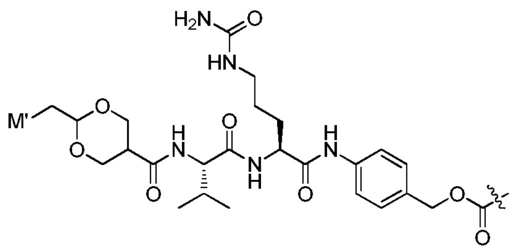
,

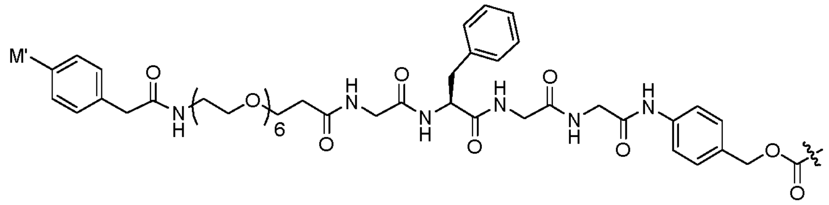
,

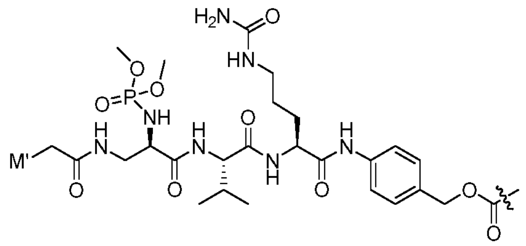
,
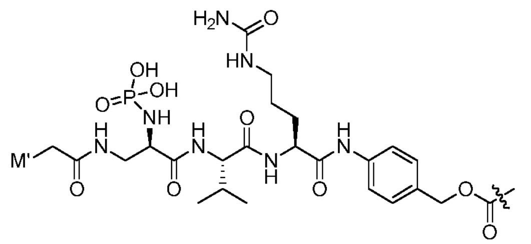
,

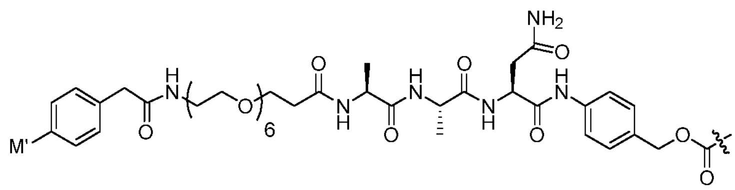
,

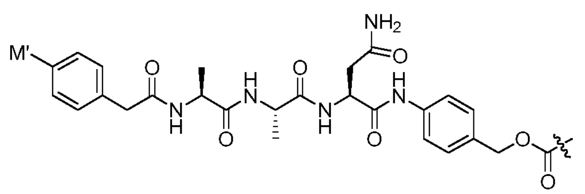
,

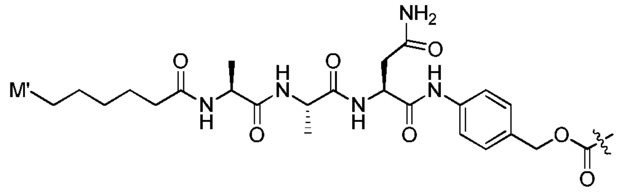

,

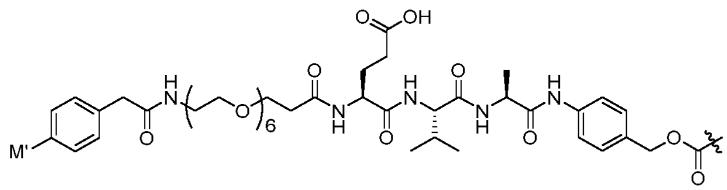

,

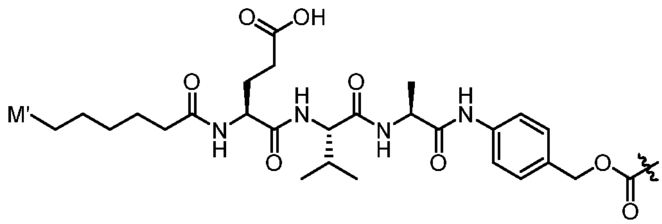

,

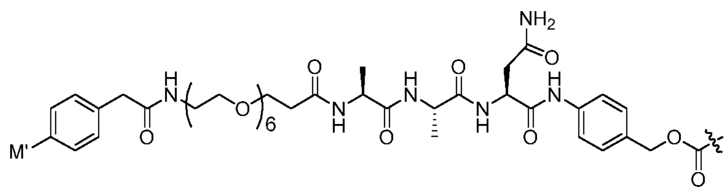

,

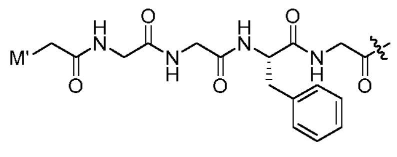

,

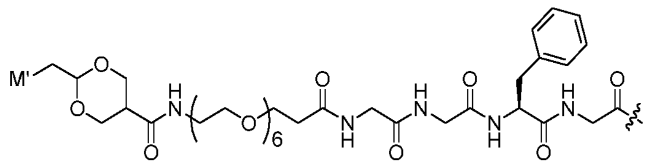

,

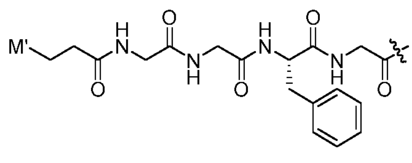

,

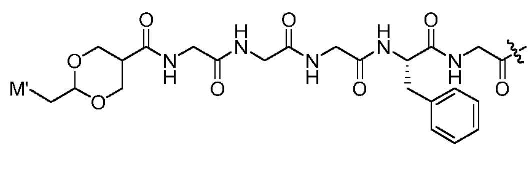

,

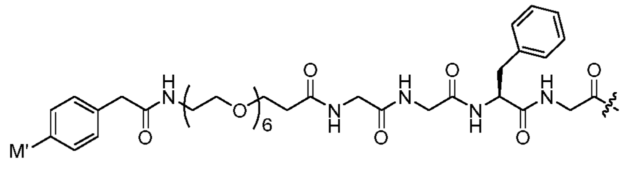

,

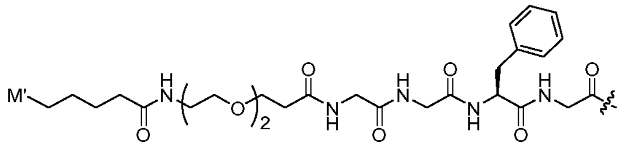

,

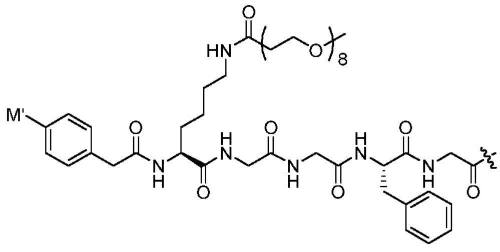

,

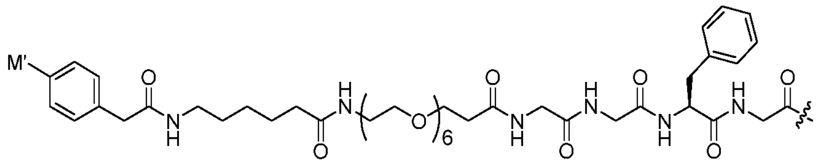

,

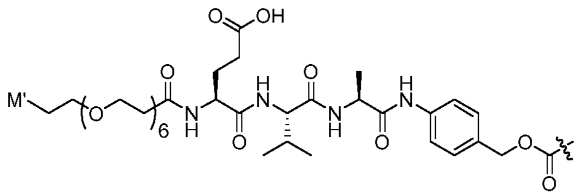

,

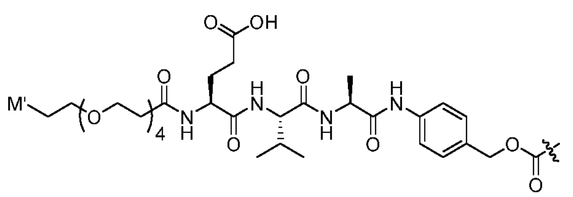

,

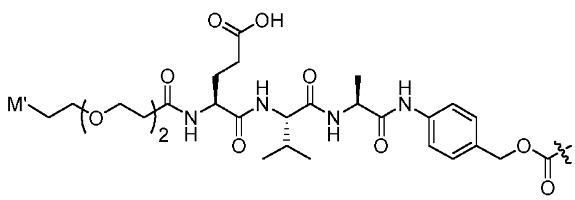

,

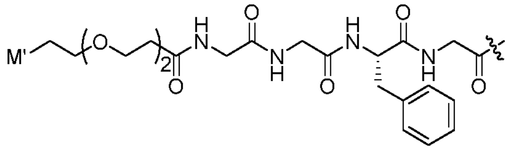

,

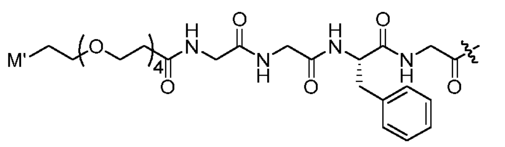

,

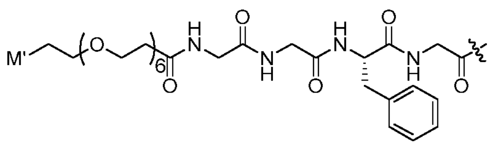

,

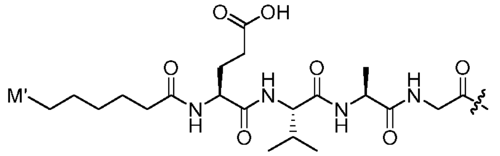

,

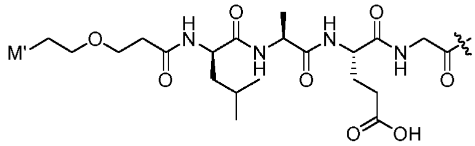

,

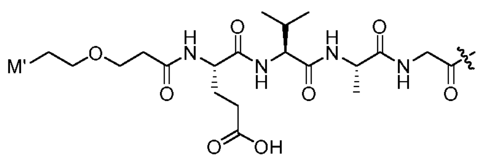,
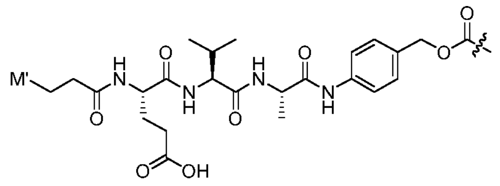,

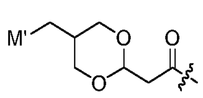,
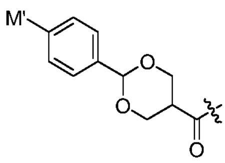,
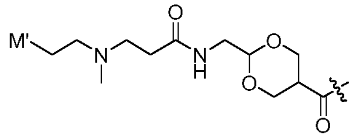,
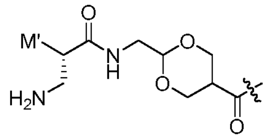,

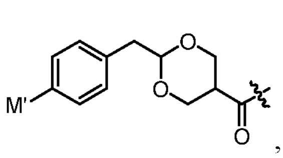,
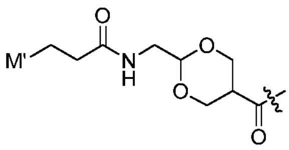
or
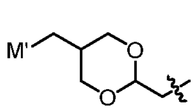.

**17.** The compound of formula II or the pharmaceutically acceptable salt thereof according to claim 12, wherein the compound of formula II is any one of compounds of:

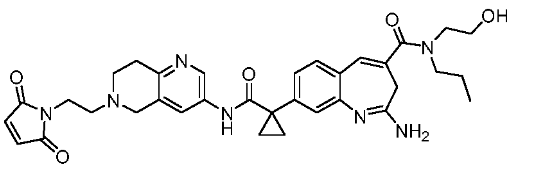,
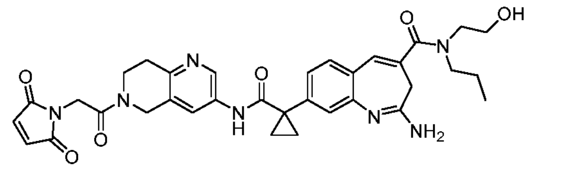,

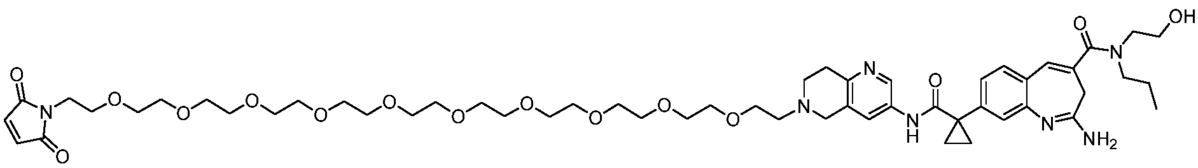

,
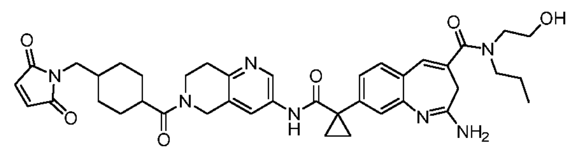,
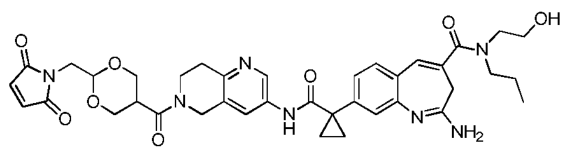,

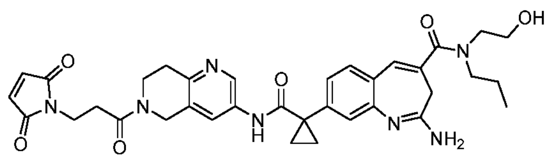,

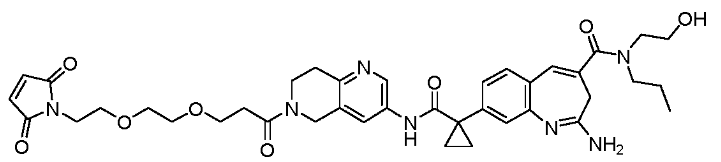,

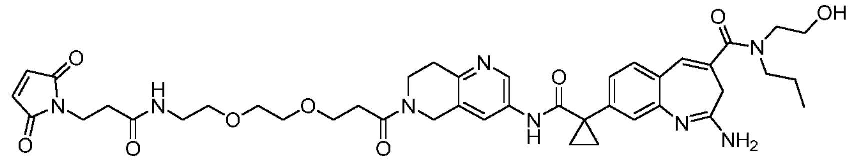

,

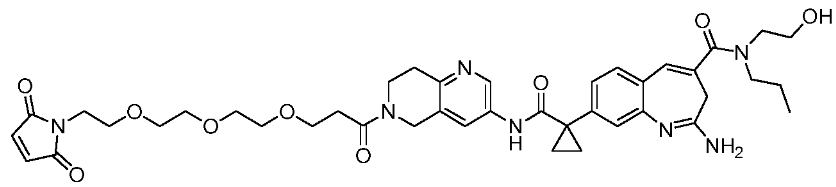

,

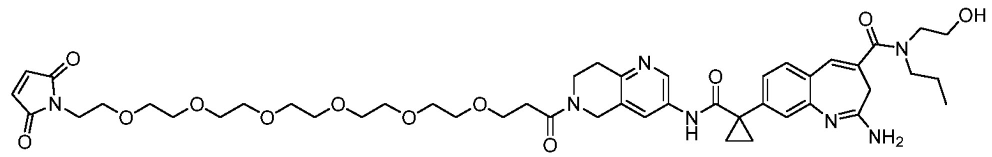

,

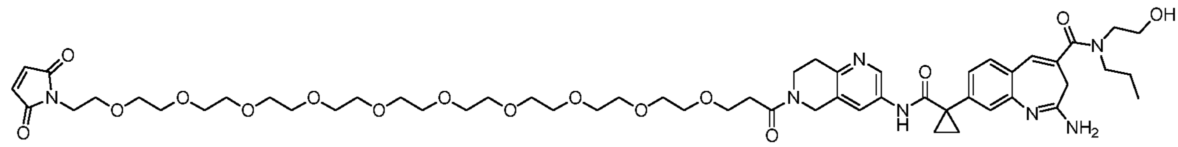

,

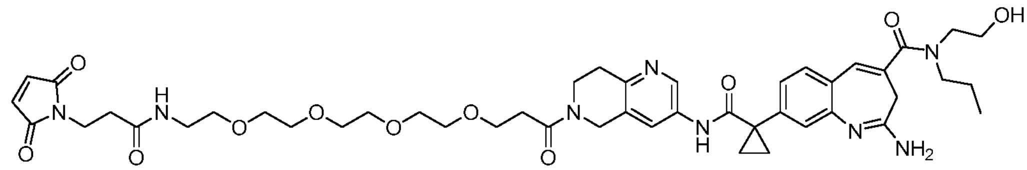

,

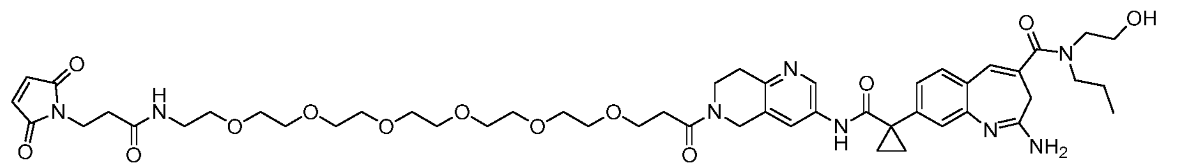

,

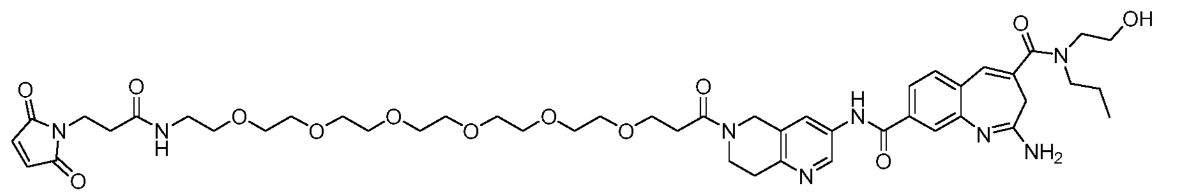

,

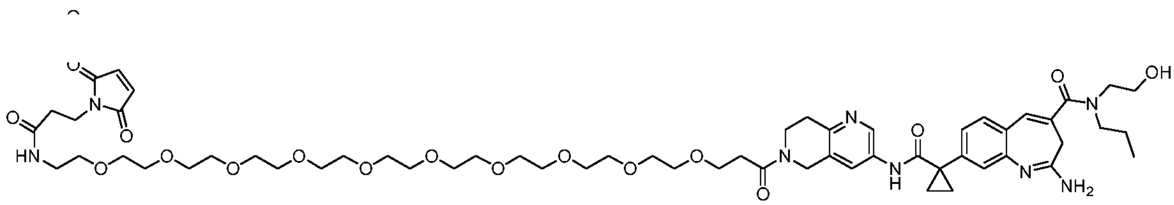

,

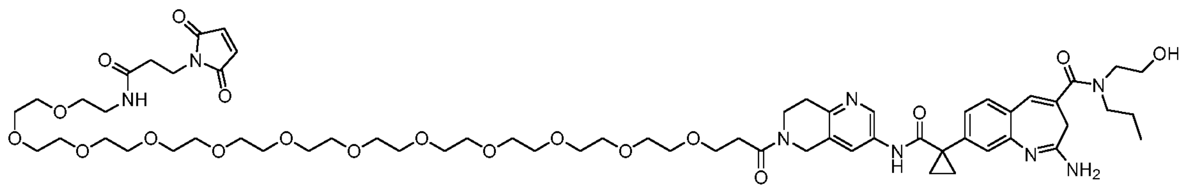

,

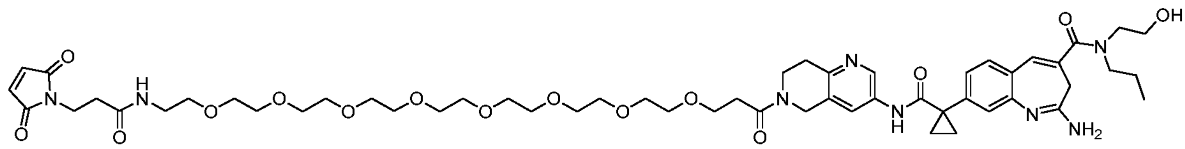

,

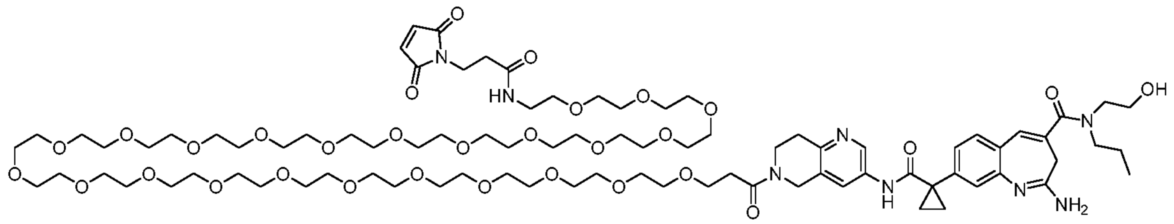

,

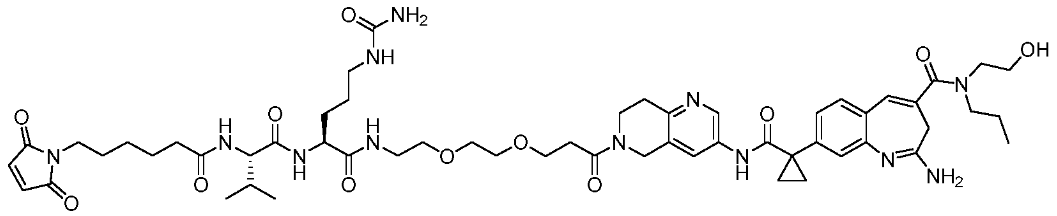

,

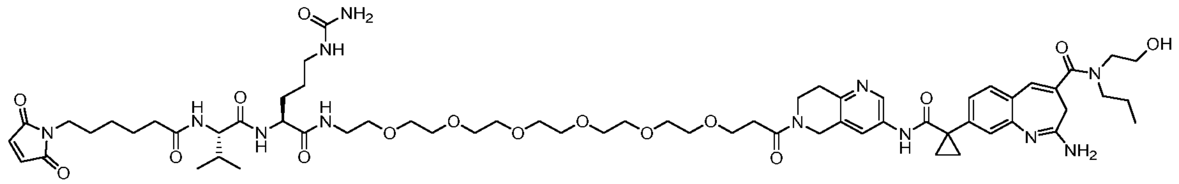

,

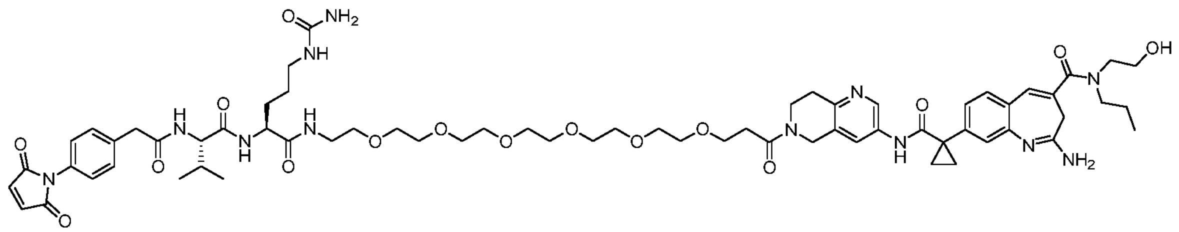

,

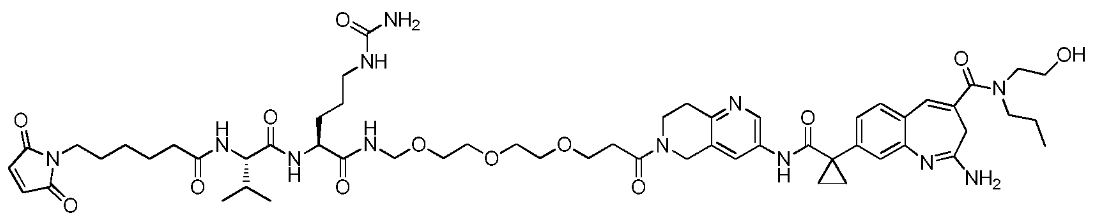

,

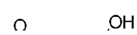

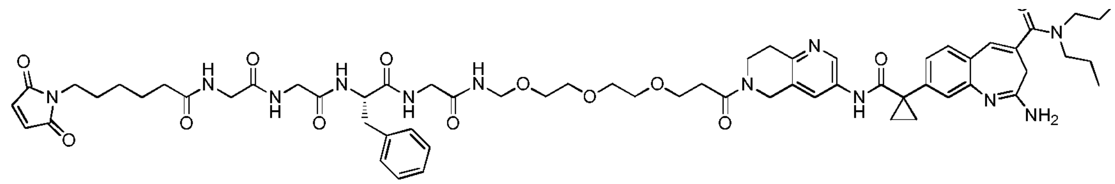

,

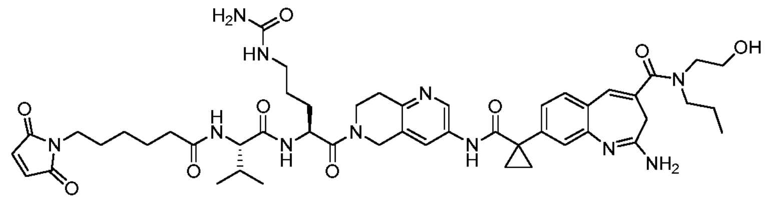

,

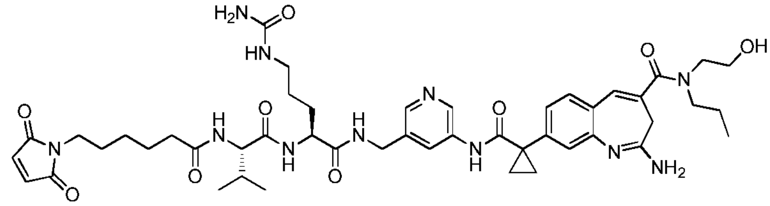

,

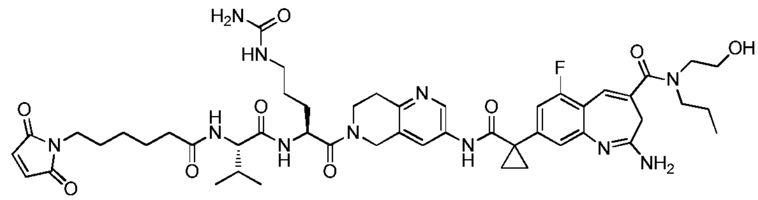

,

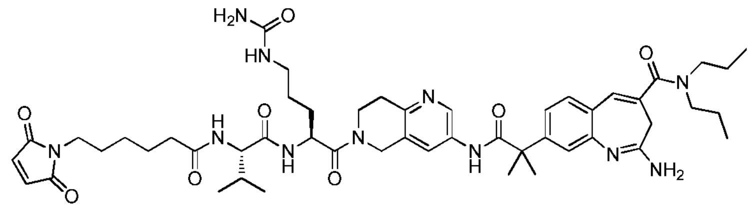

,

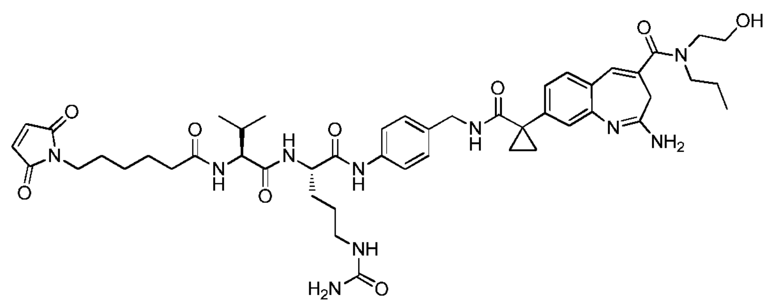

,

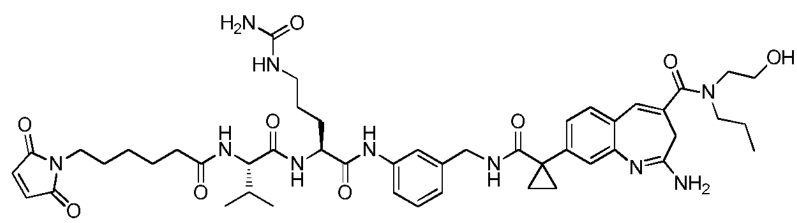

,

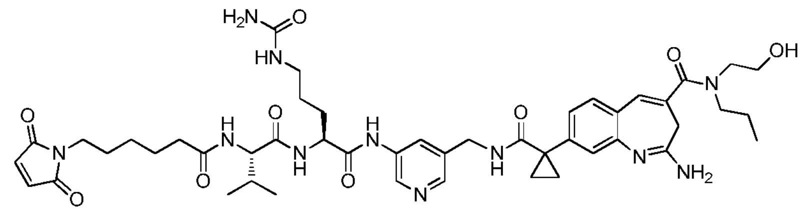

,

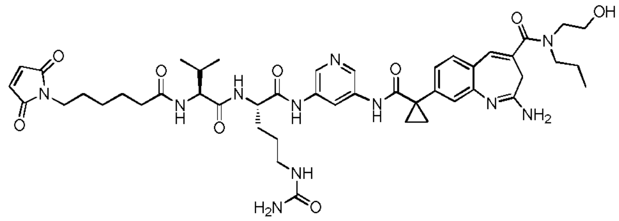

,

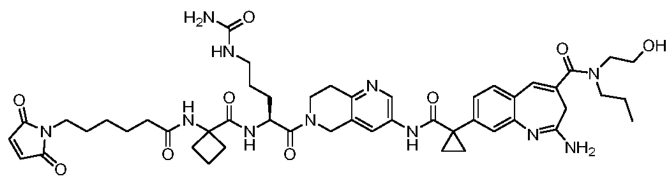

,

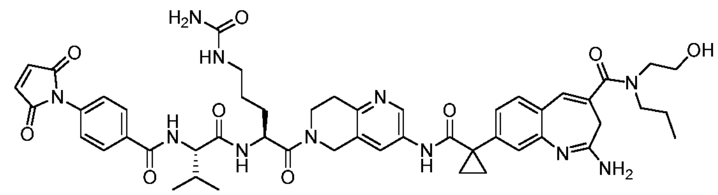

,

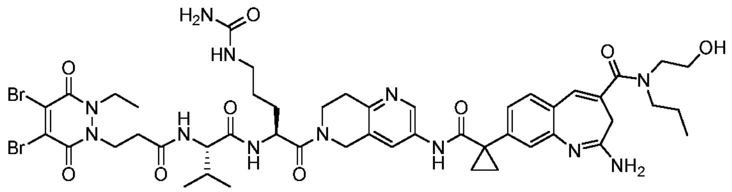

,

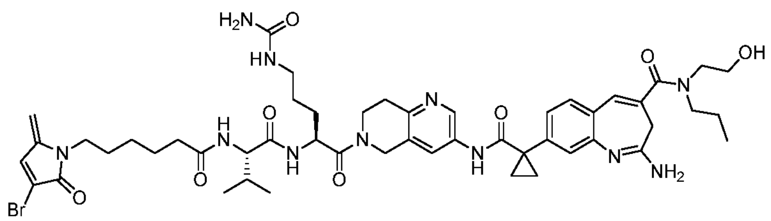

,

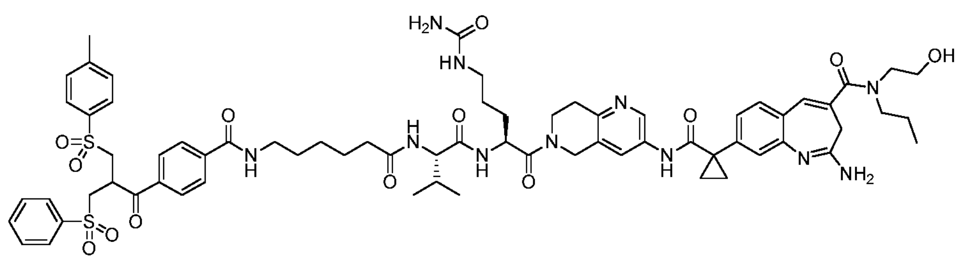

,

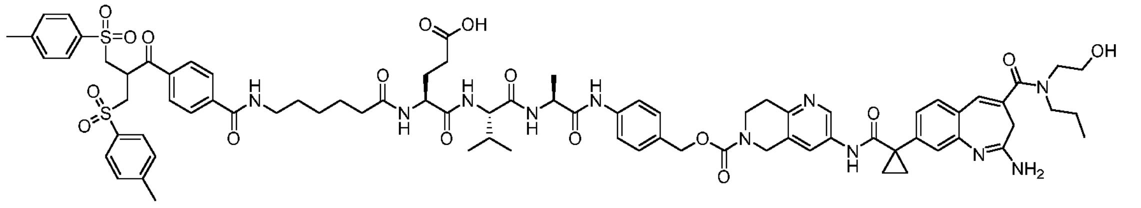

,

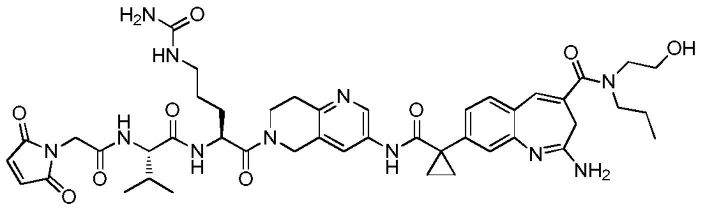

,

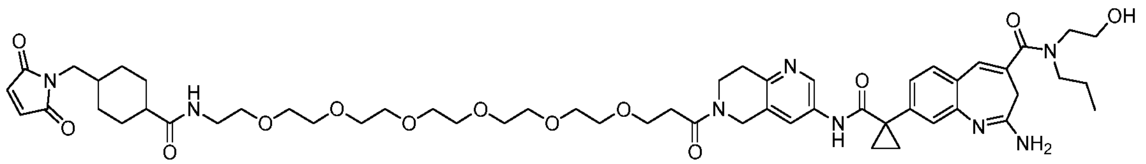

,

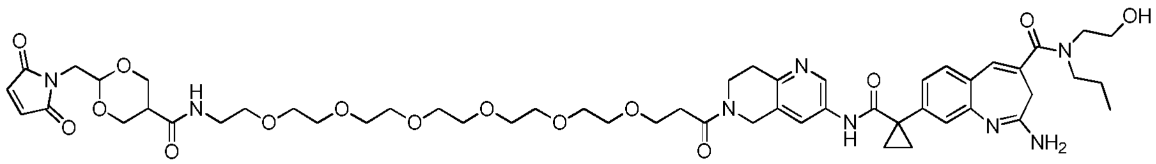

,

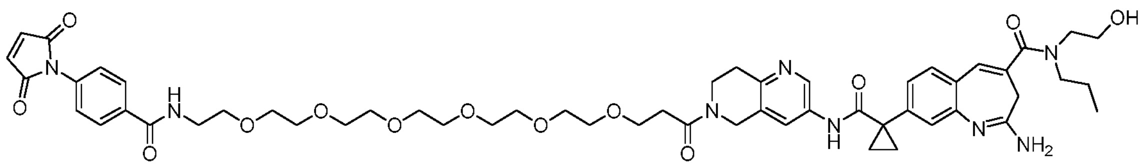

,

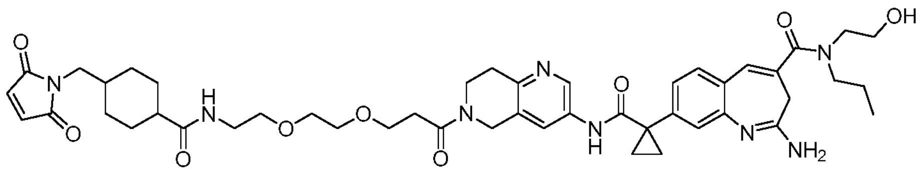

,

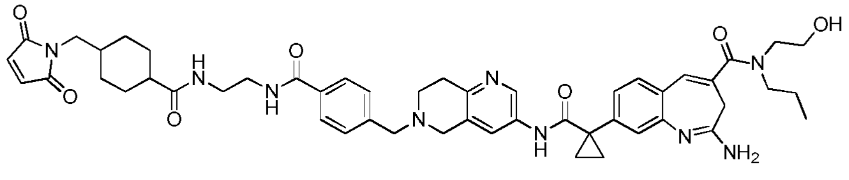

,

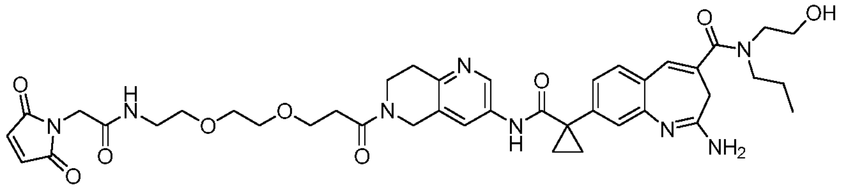

,

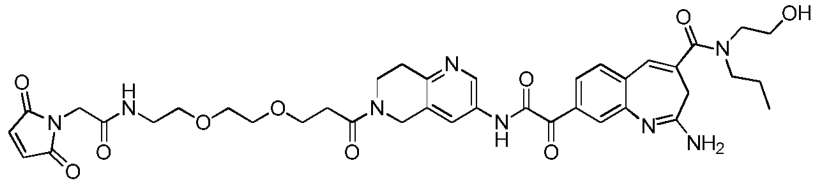

,

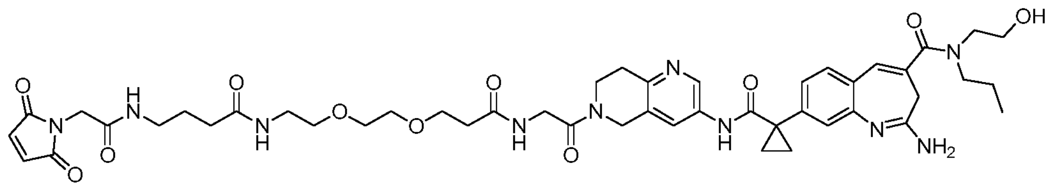

,

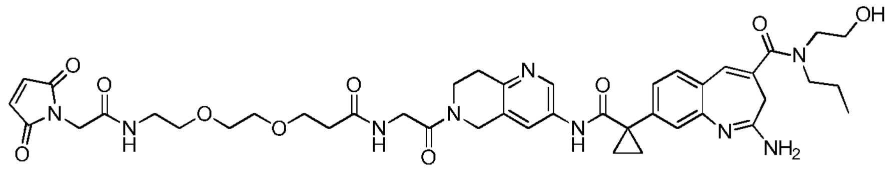

,

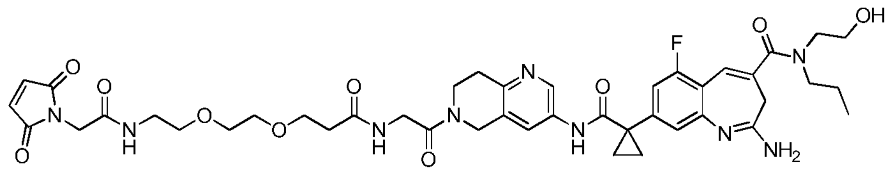

,

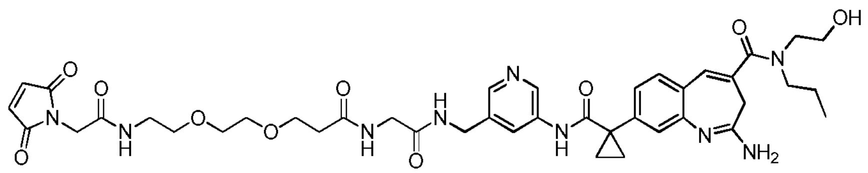

,

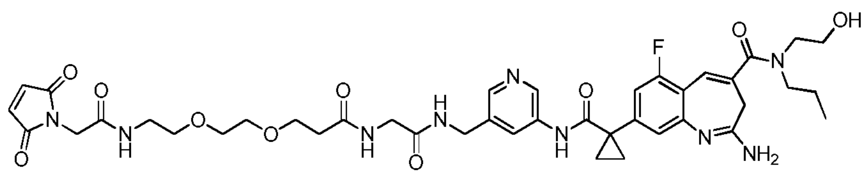

,

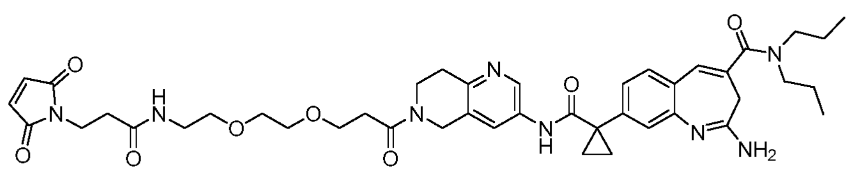

,

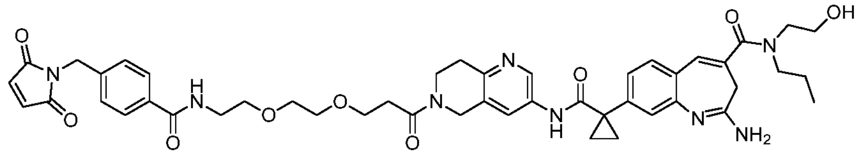

,

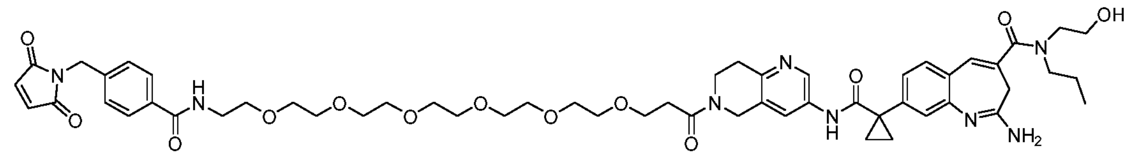

,

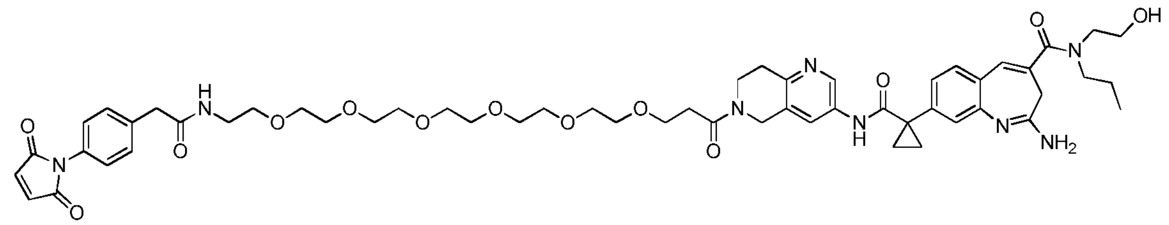

,

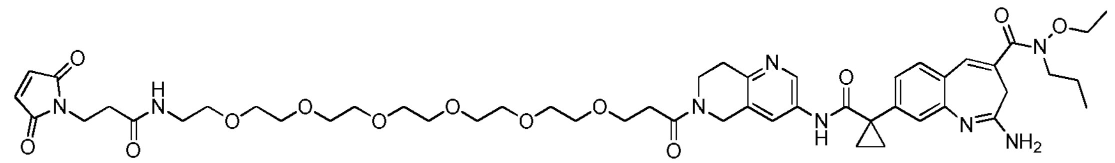

,

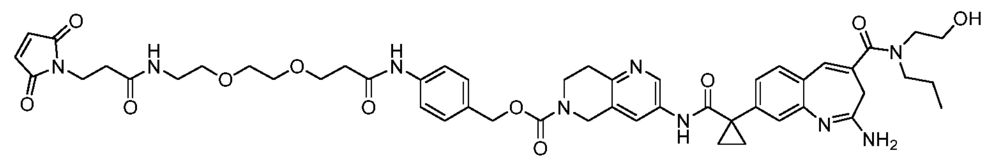

,

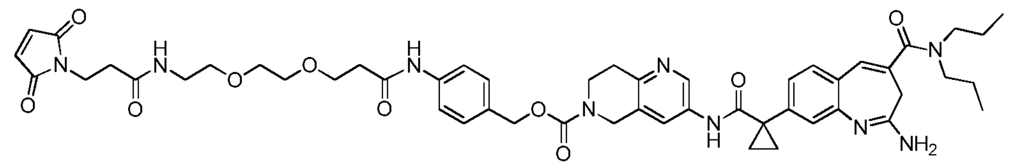

,

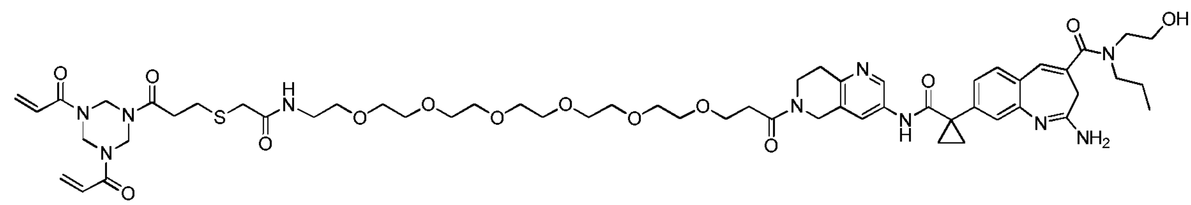

,

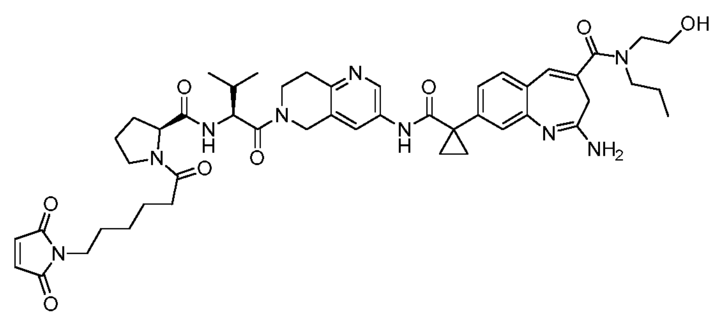

,

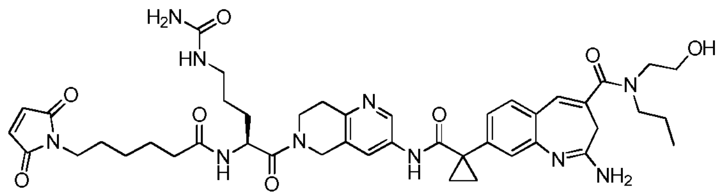

,

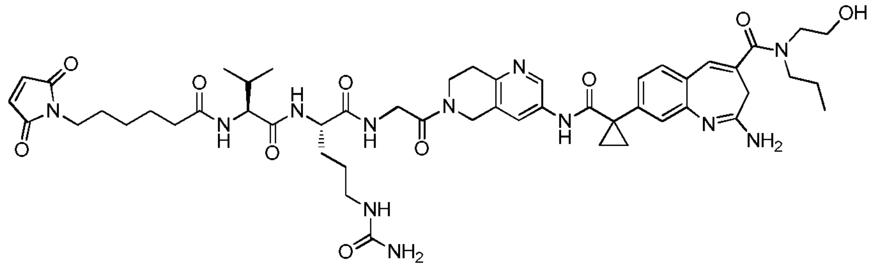

,

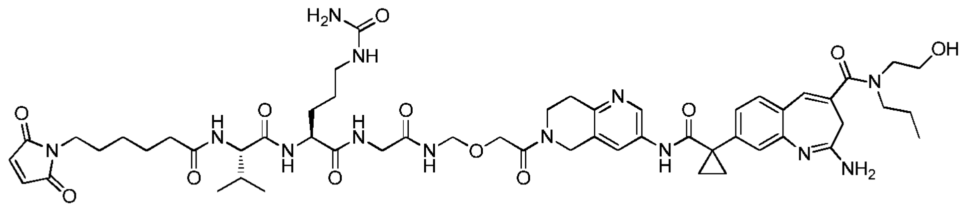

,

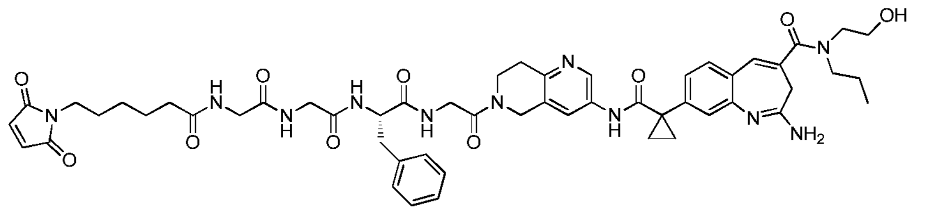

,

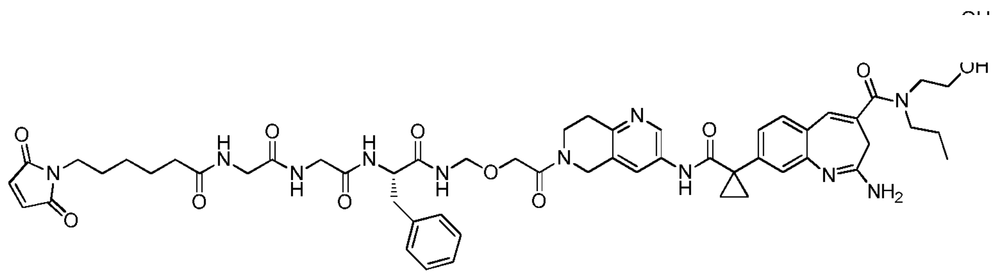

,

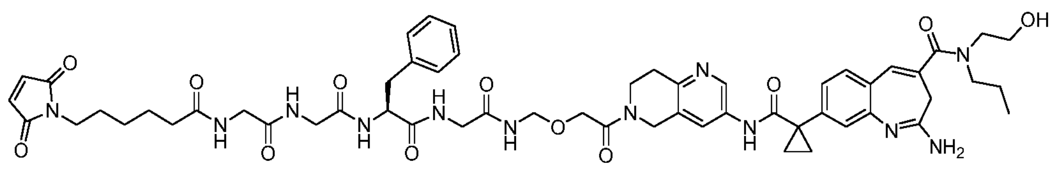

,

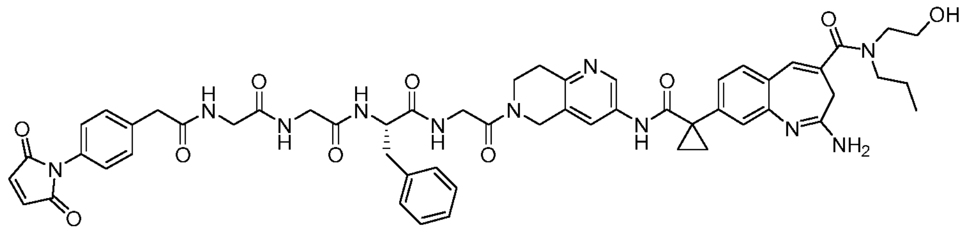

,

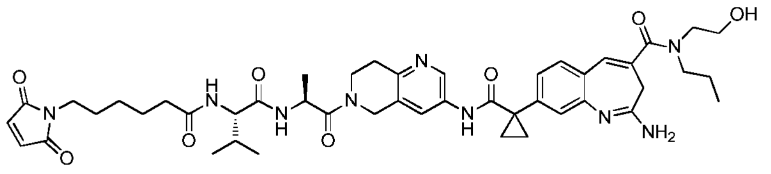

,

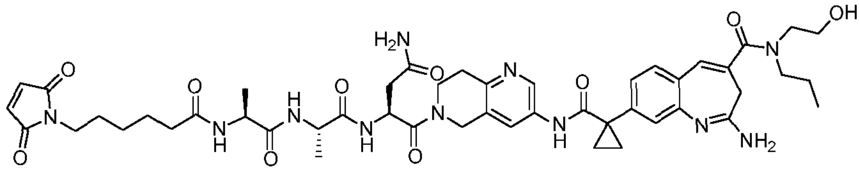

,

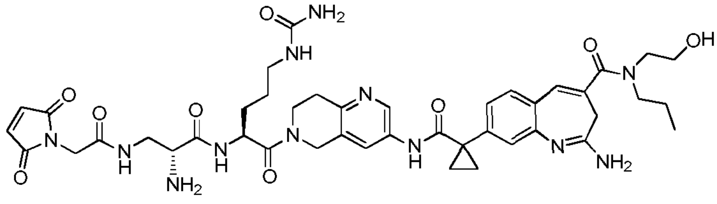

,

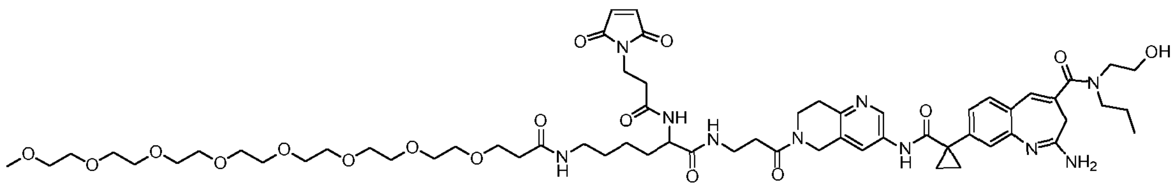

,

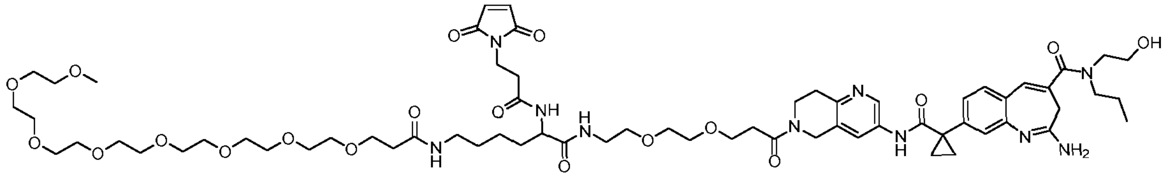

,

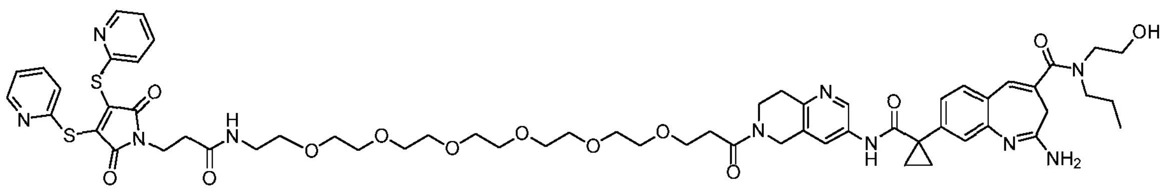

,

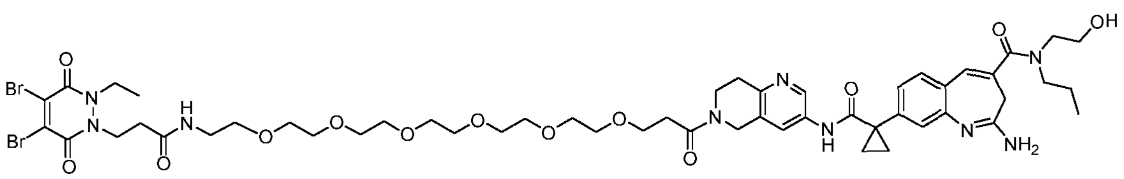

,

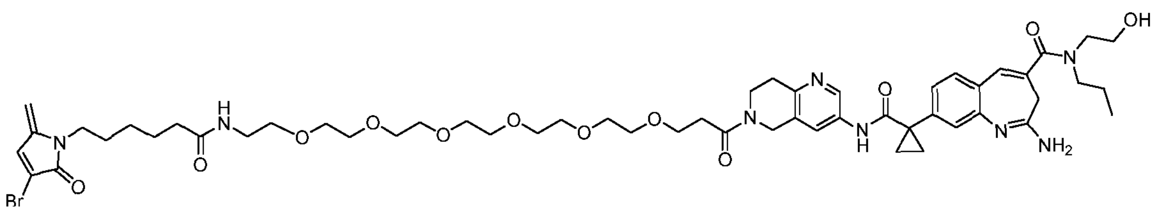

,

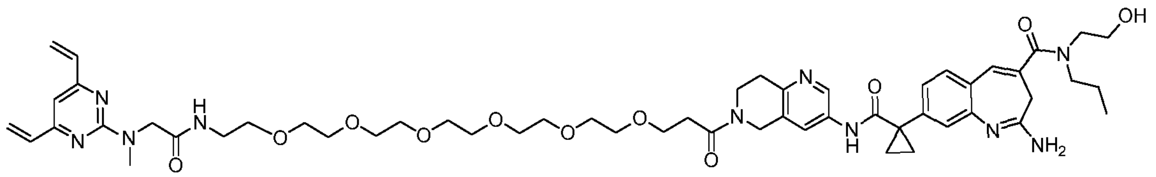

,

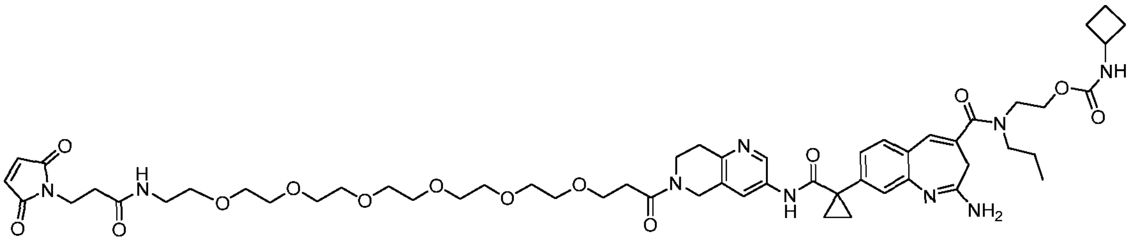

,

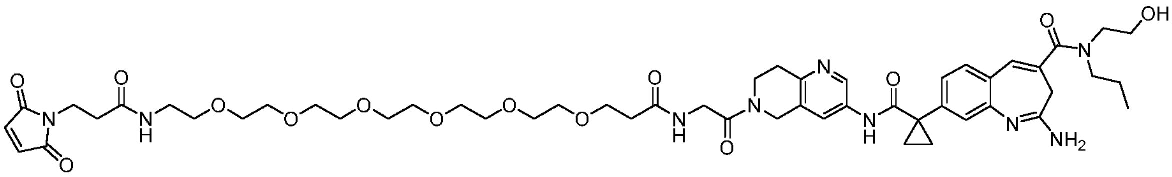

,

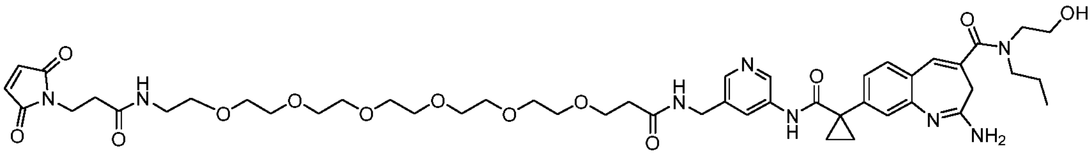

,

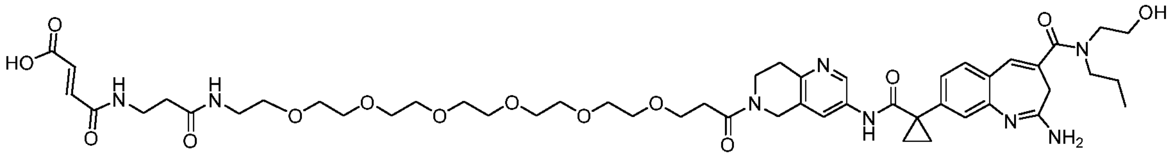

,

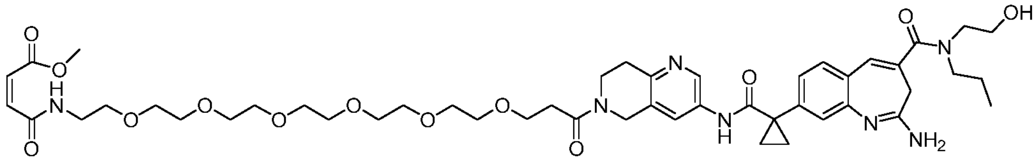

,

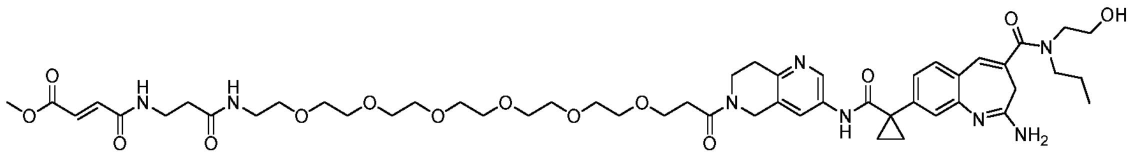

,

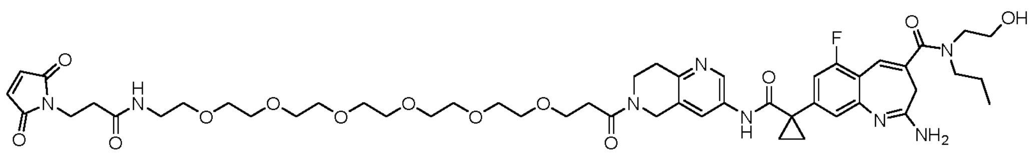

,

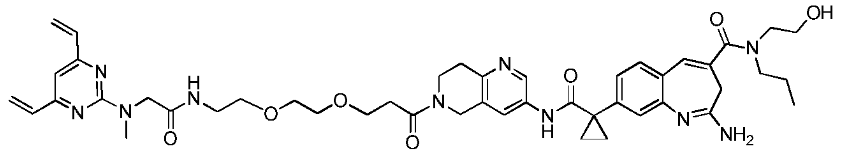

,

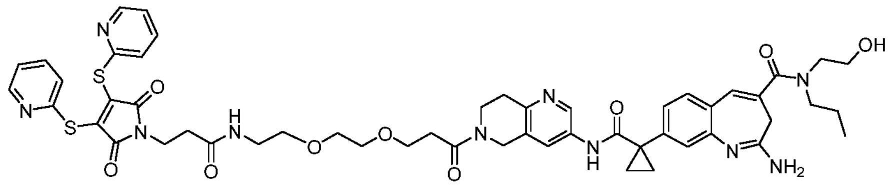

,

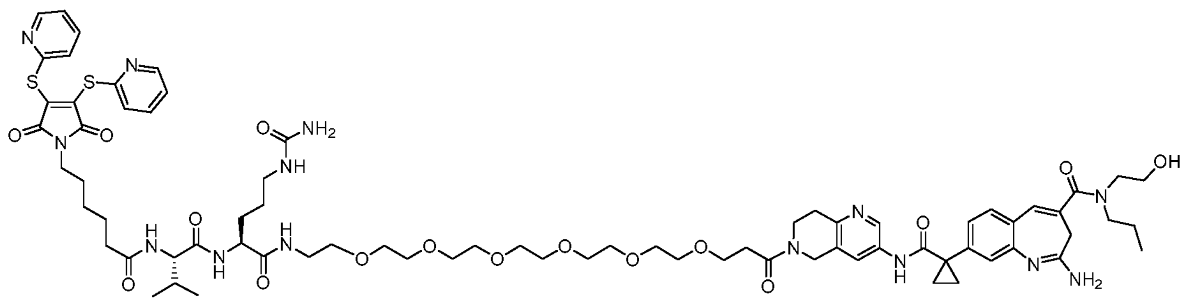

,

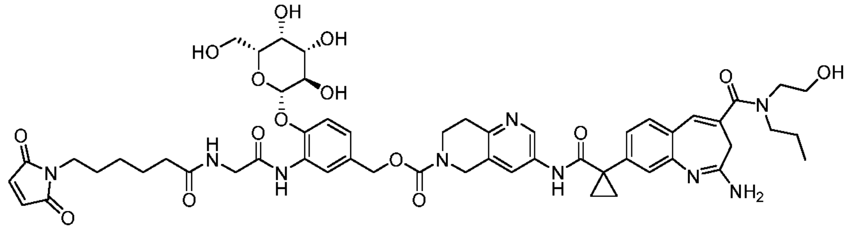

,

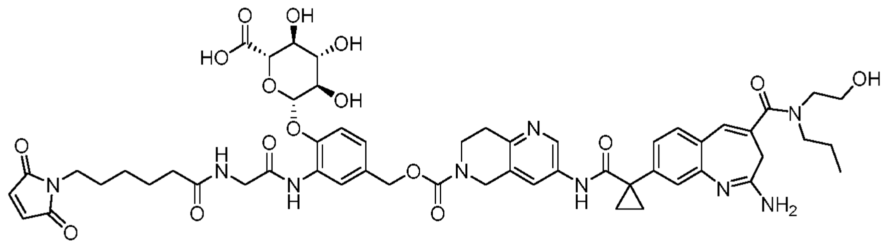

,

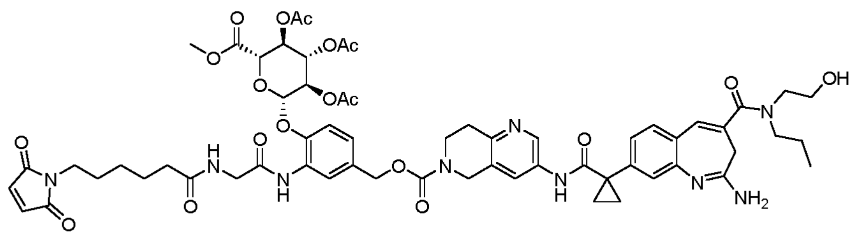

,

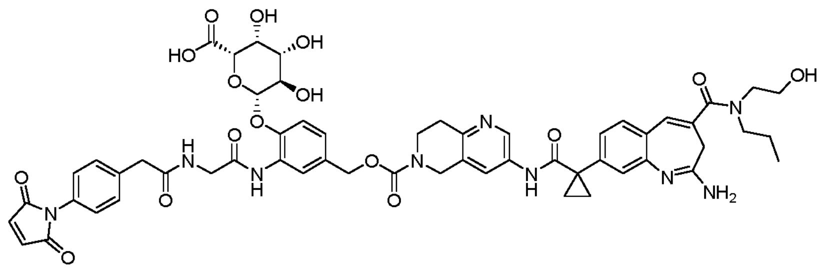

,

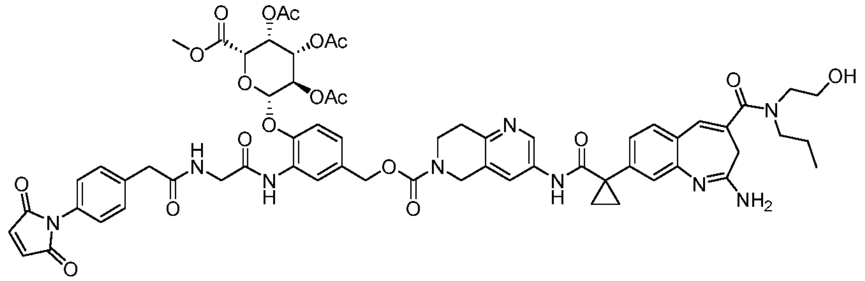

,

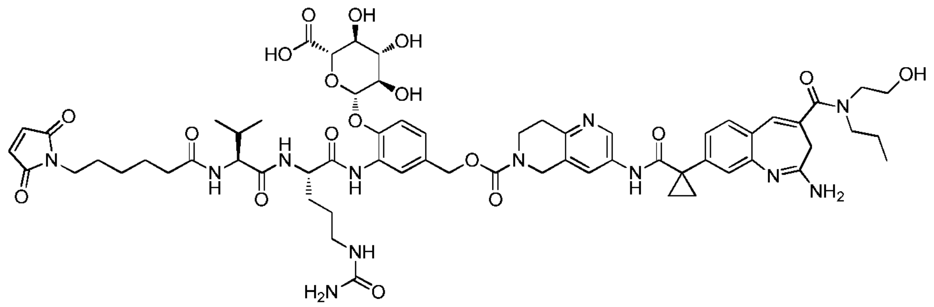

,

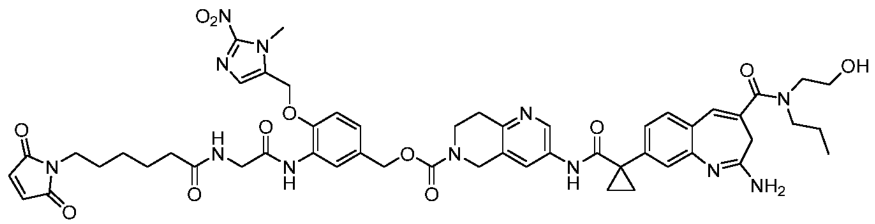

,

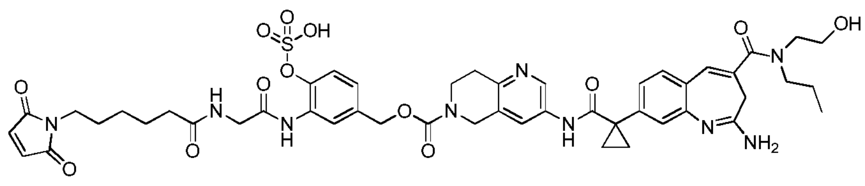

,

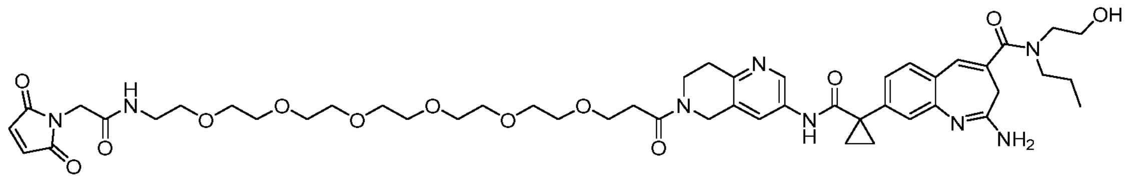

,

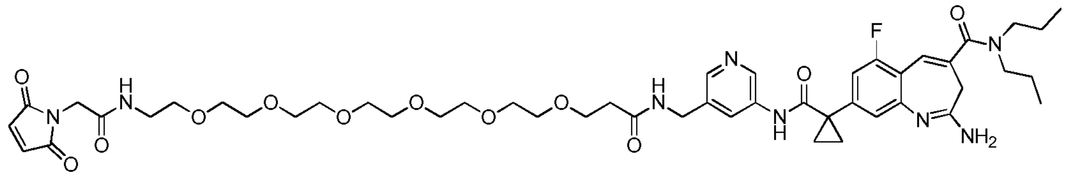

,

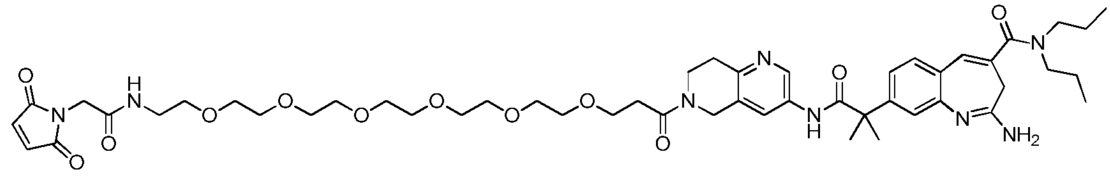

,

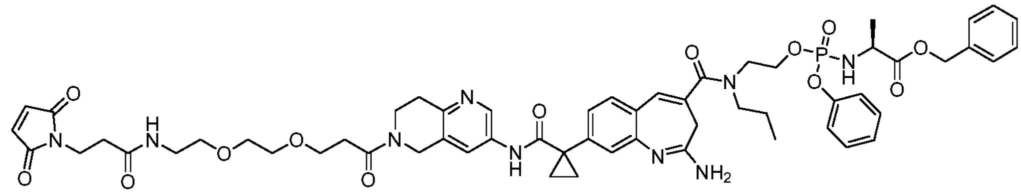

,

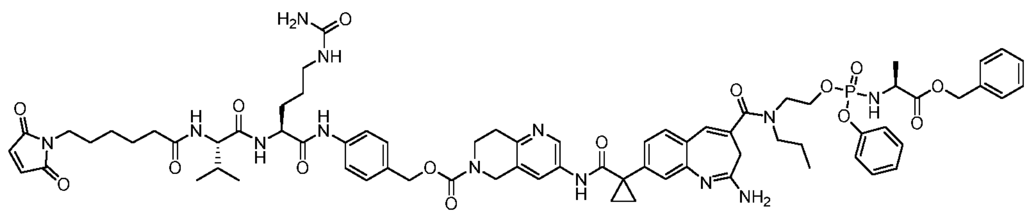

,

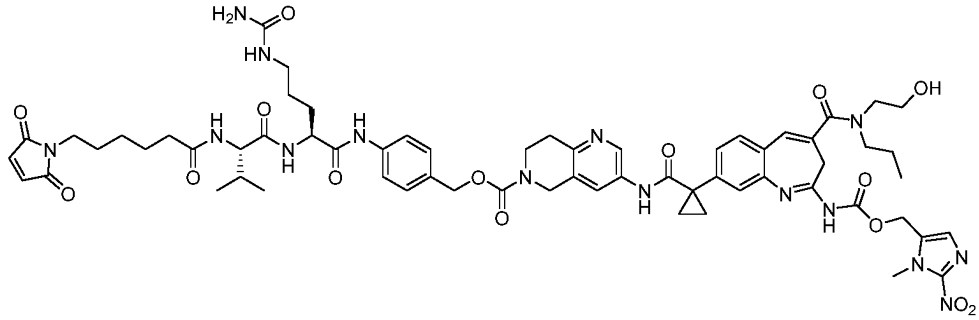

,

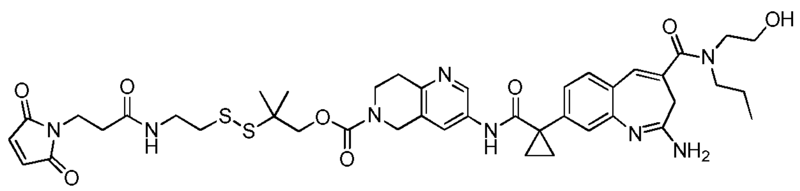

,

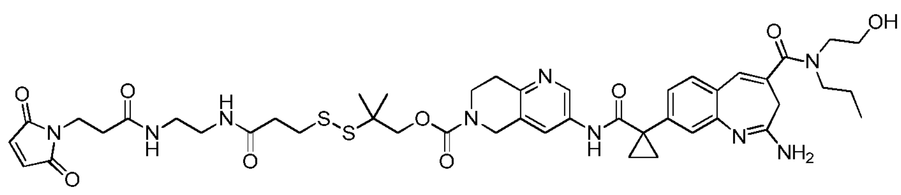

,

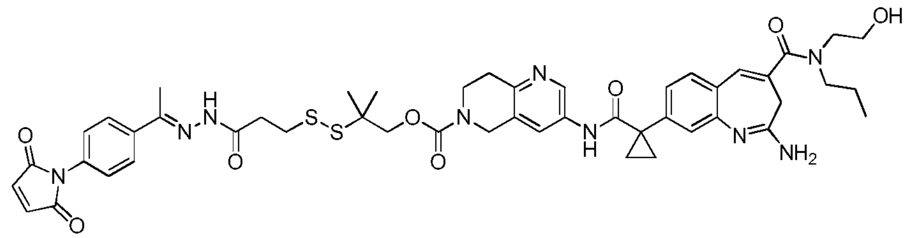

,

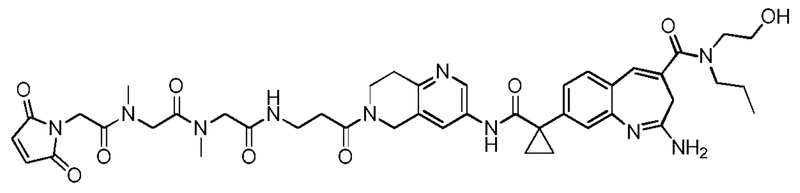

,

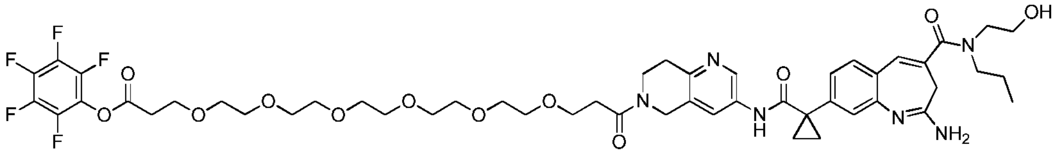

,

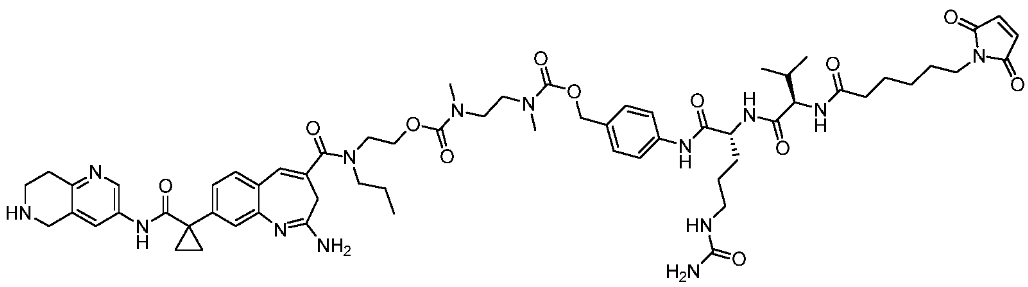

,

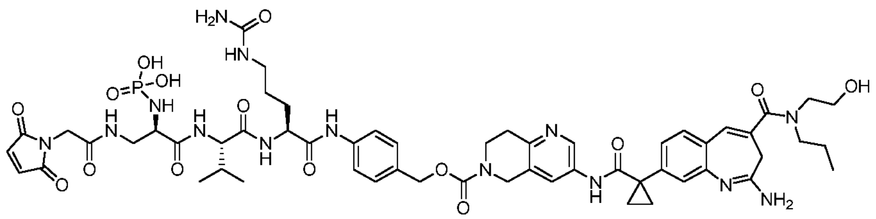

,

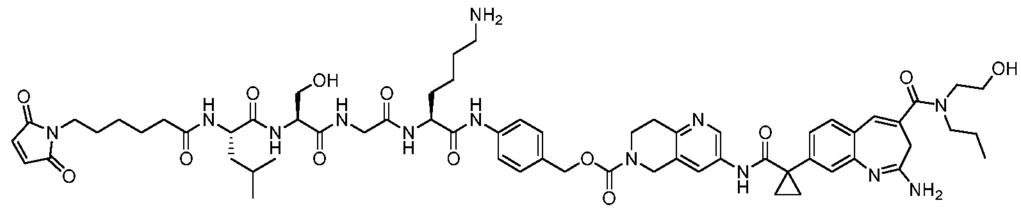

,

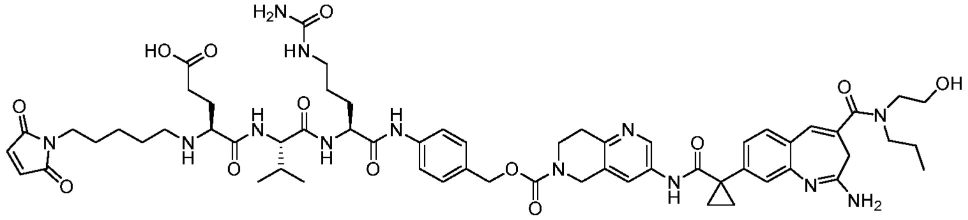

,

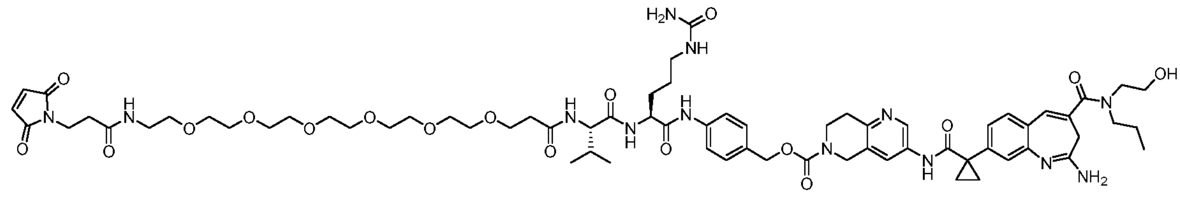

,

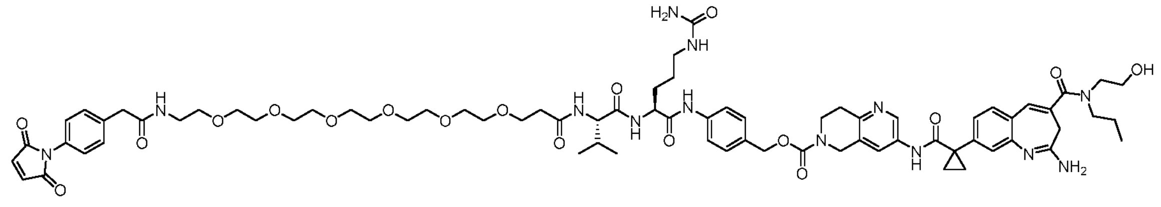

,

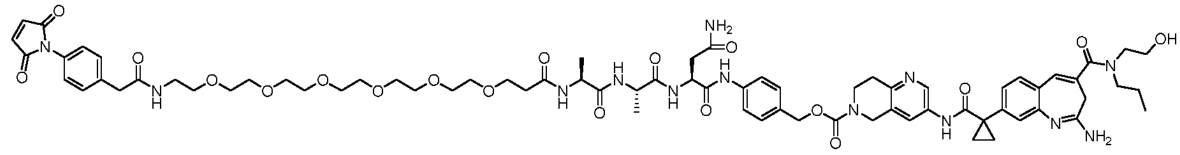

,

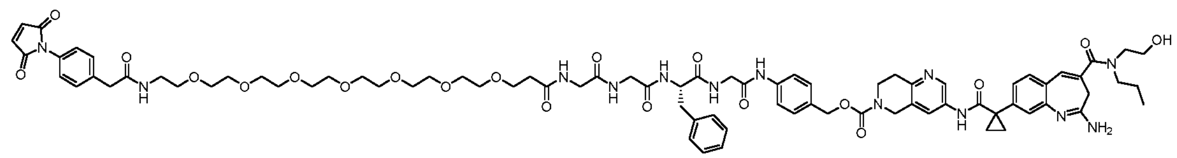

,

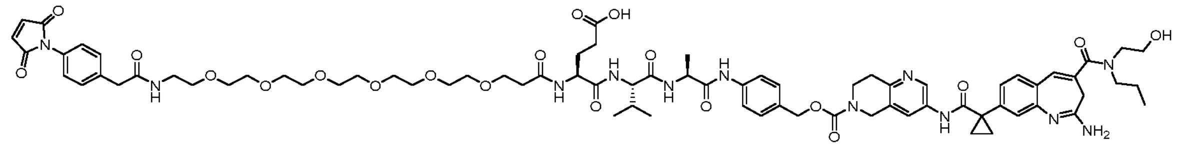

,

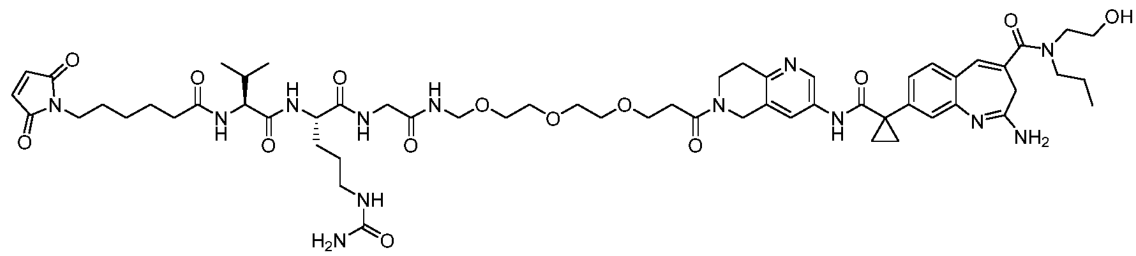

,

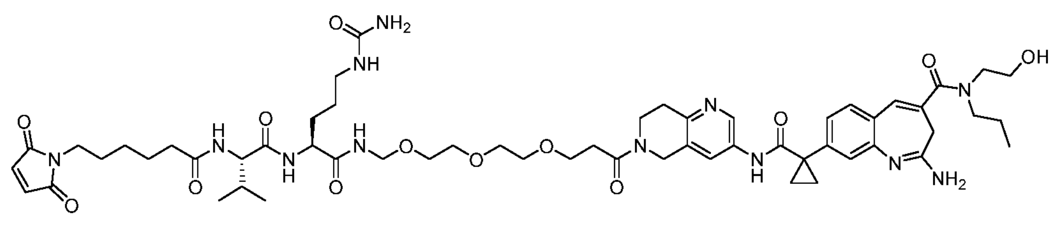

,

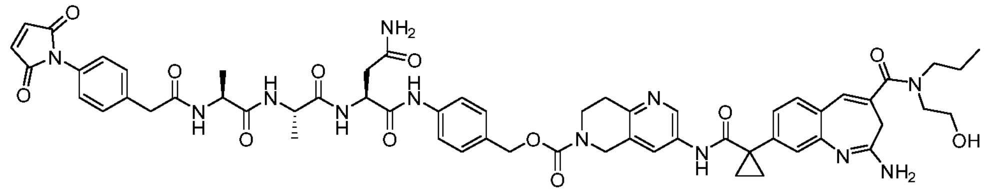

,

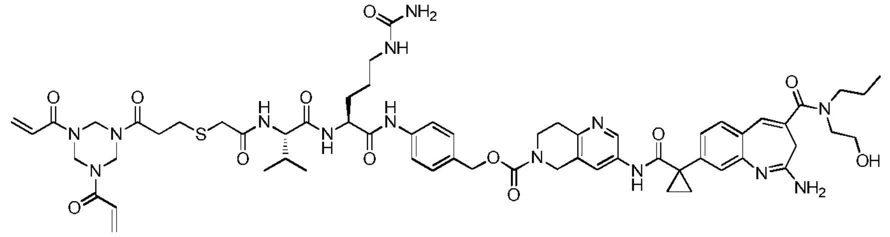

,

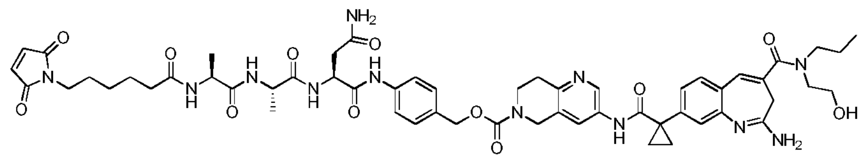

,

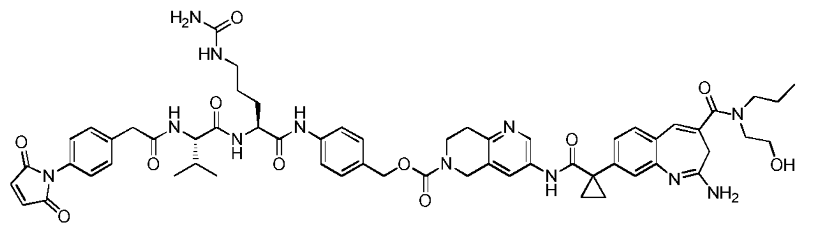

,

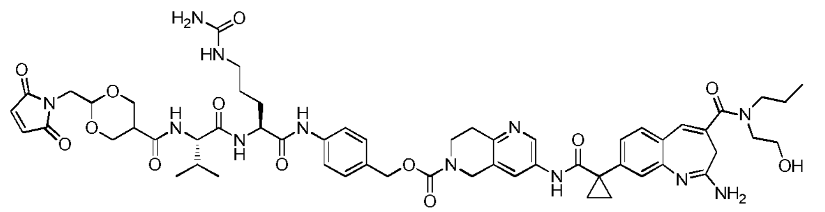

,

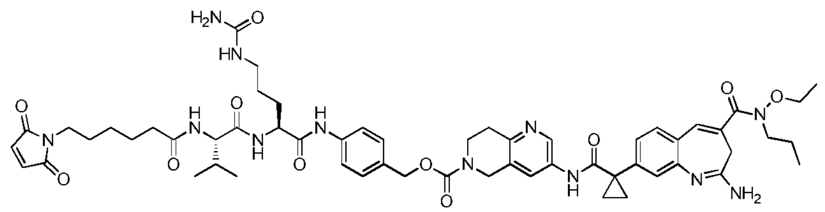

,

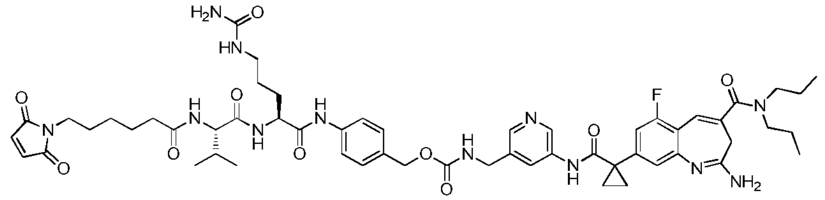

,

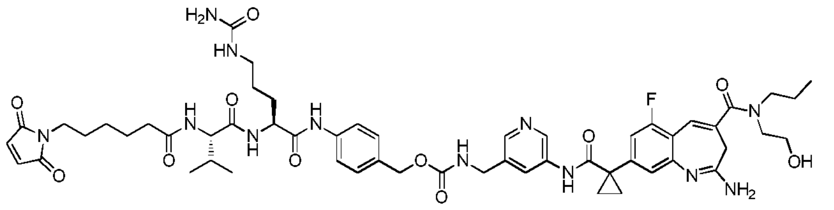

,

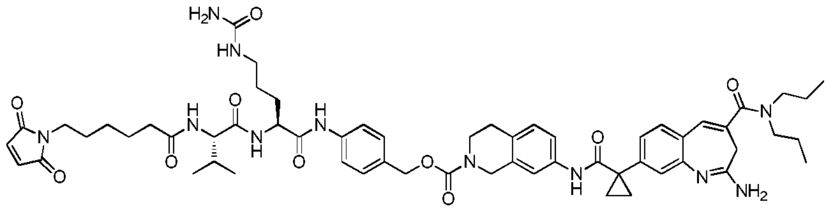

,

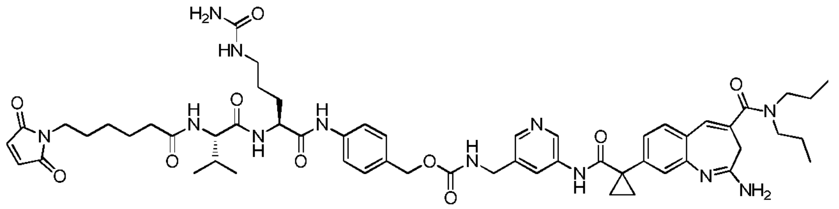

,

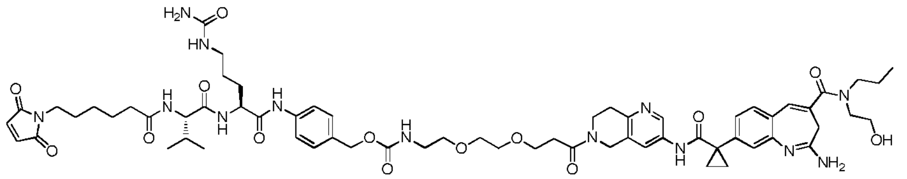

,

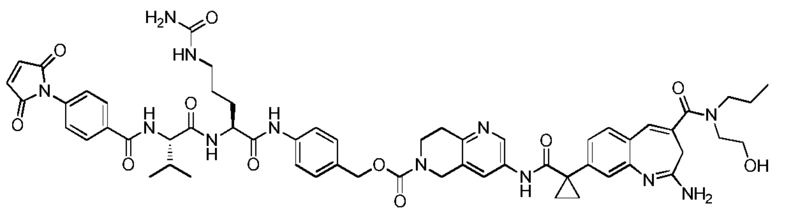

,

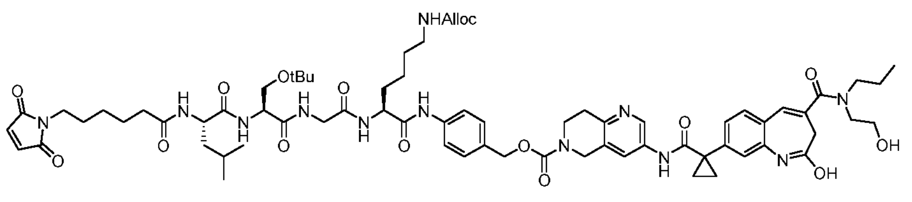

,

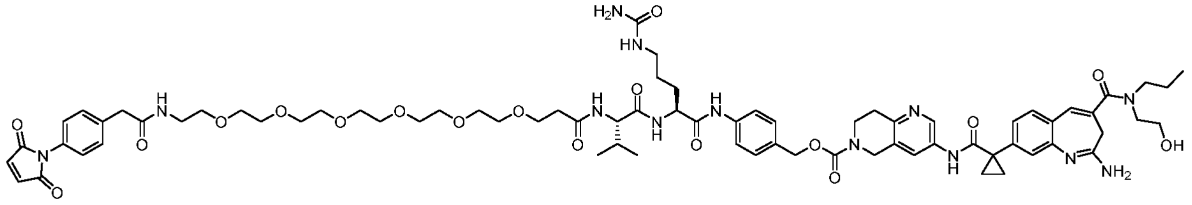

,

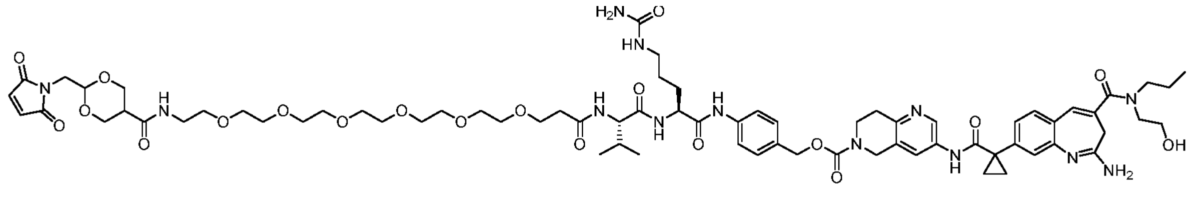

,

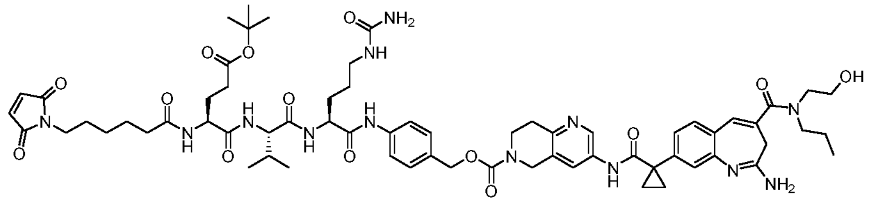

,

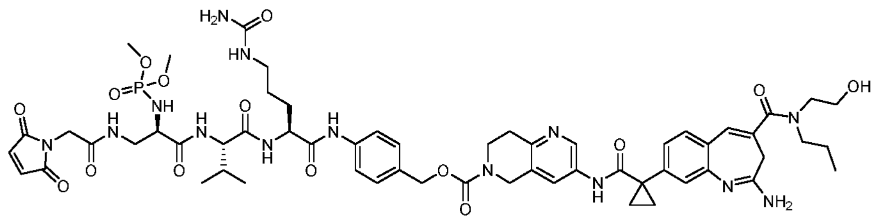

,

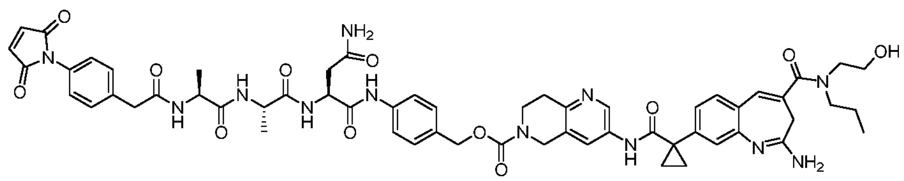

,

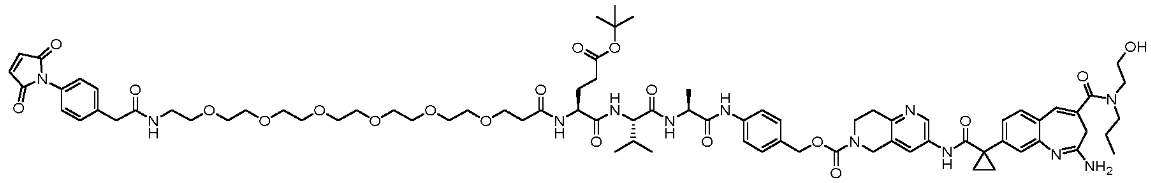

,

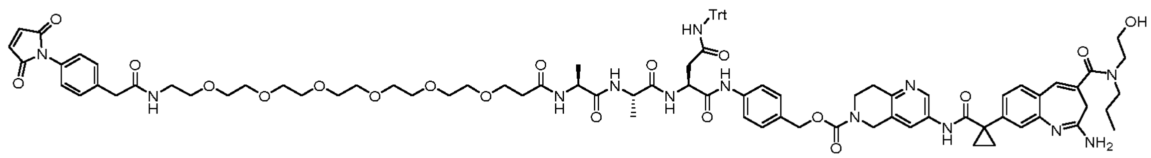

,

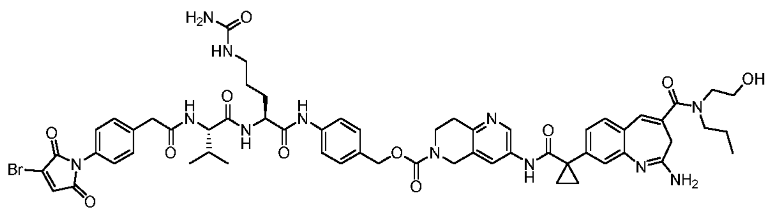

,

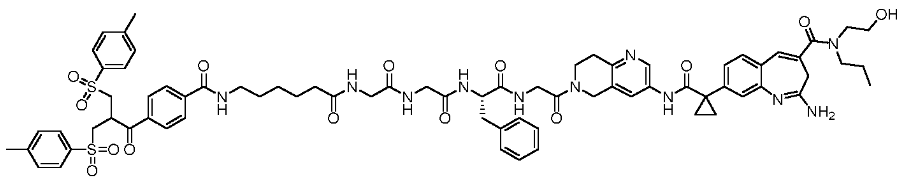

,

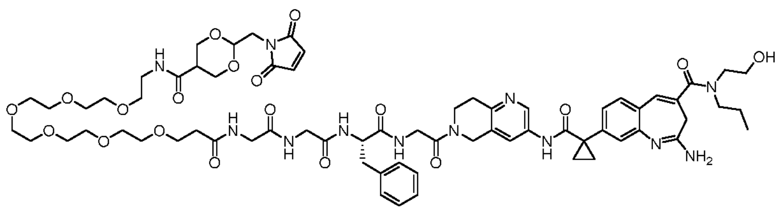

,

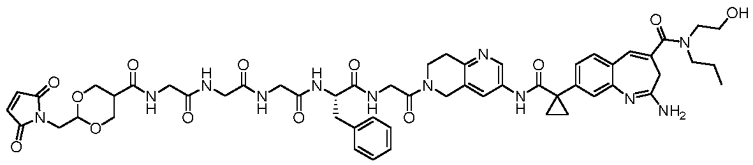

,

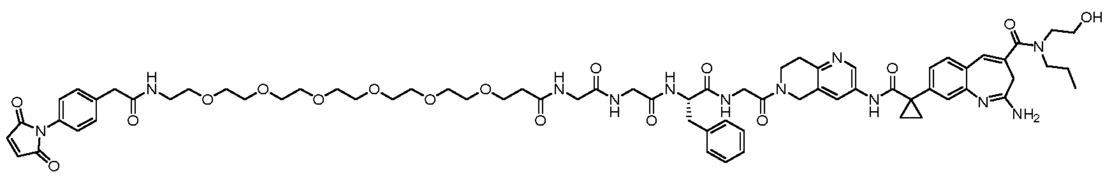

,

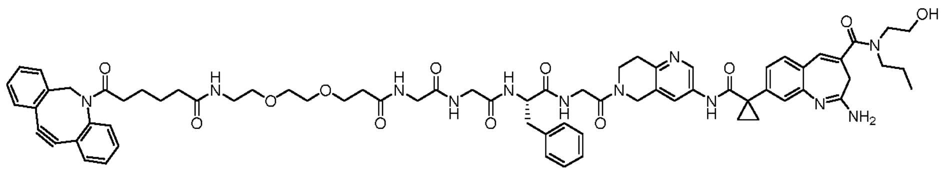

,

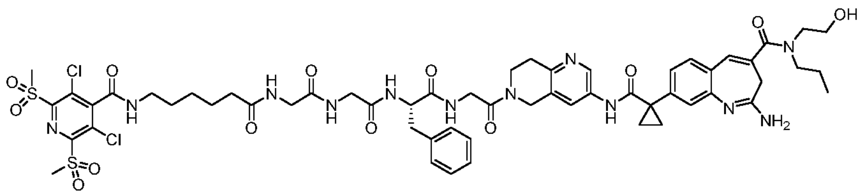

,

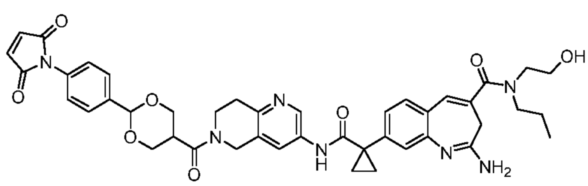

,

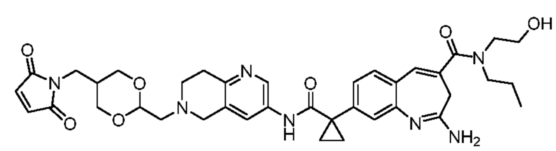

,

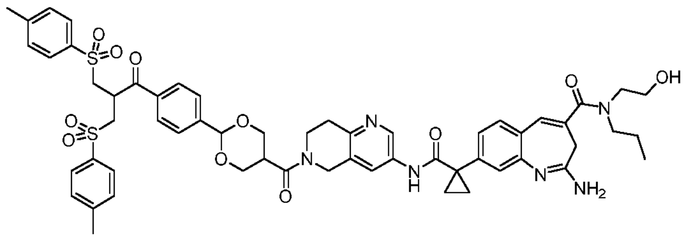

,

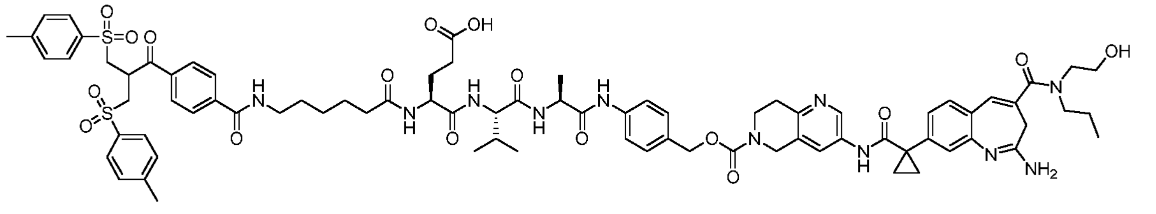

,

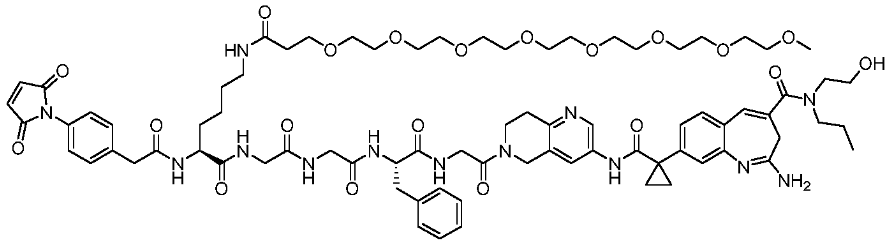

,

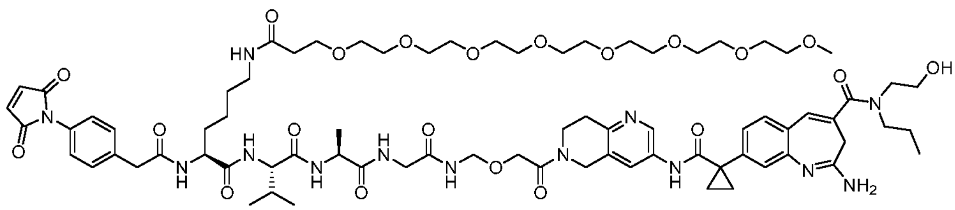

,

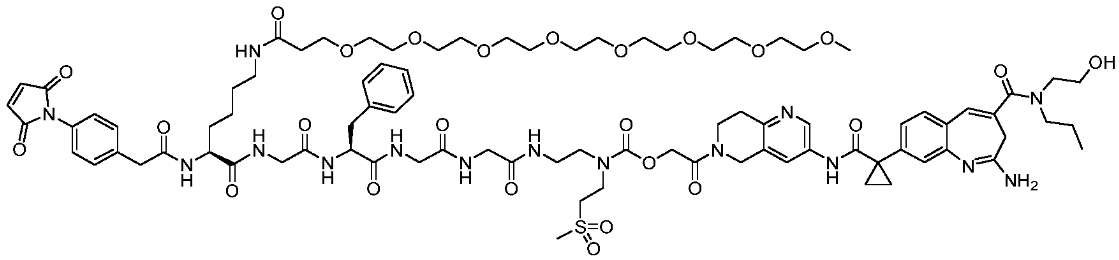

,

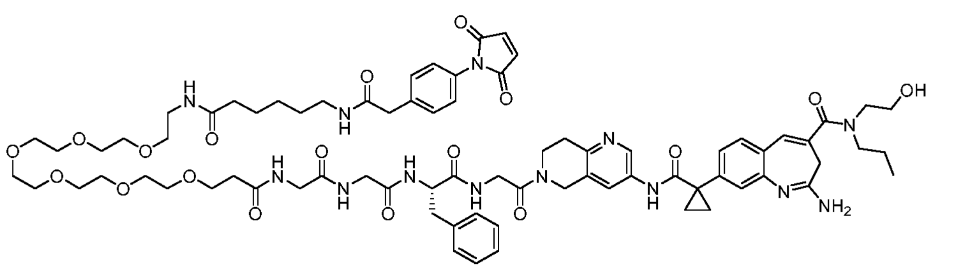

,

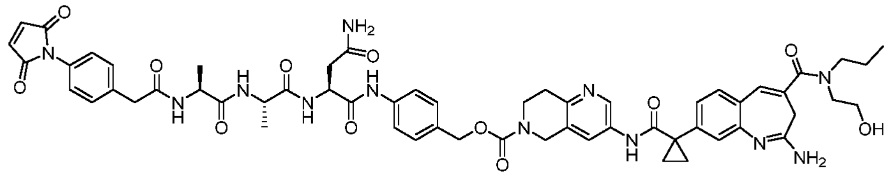

,

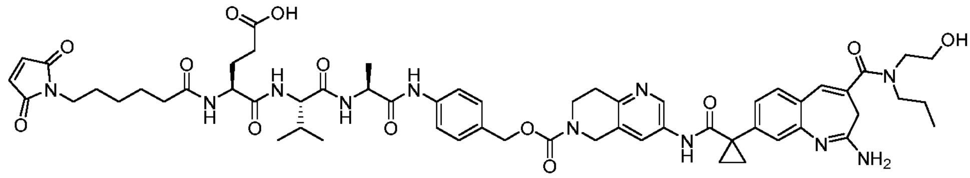

,

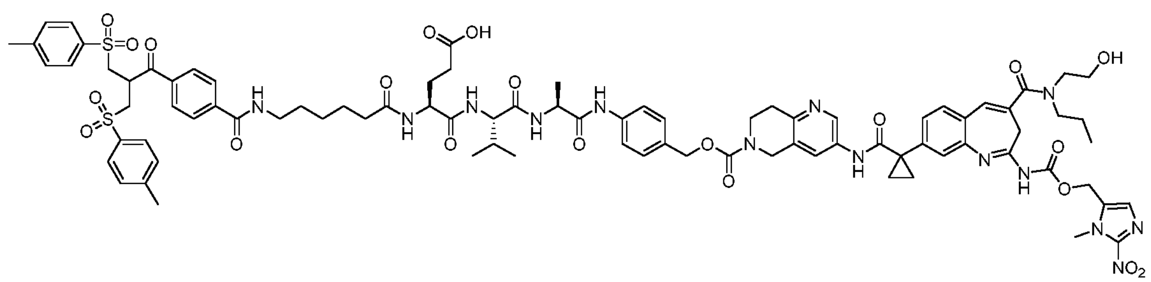

,

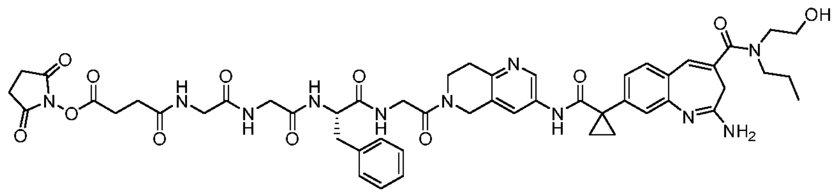

,

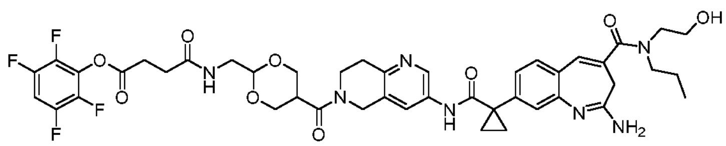

,

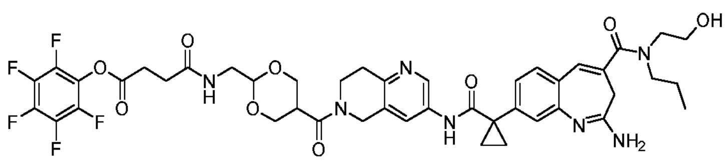

,

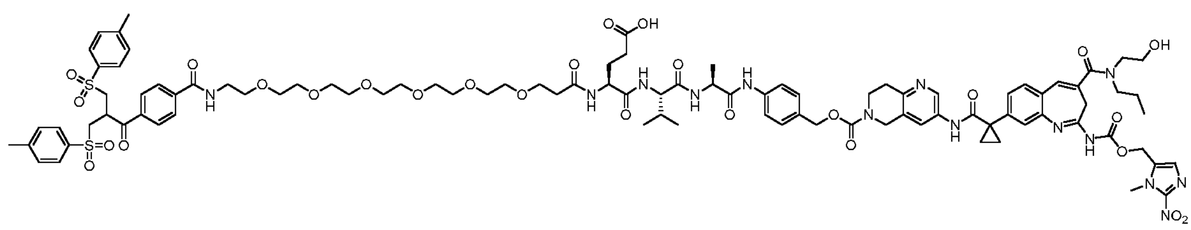

,

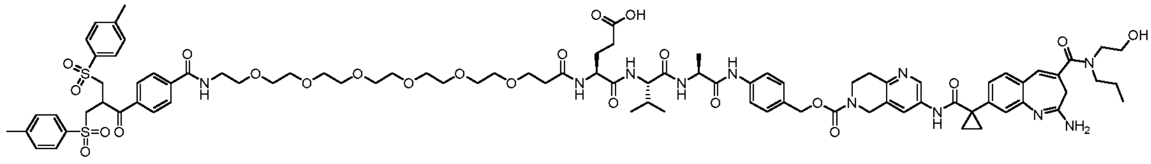

,

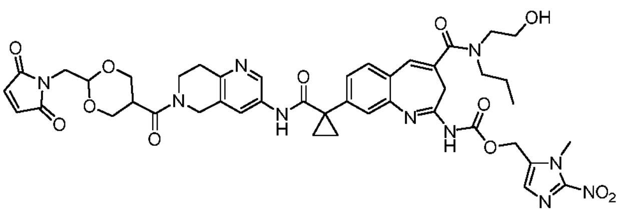

,

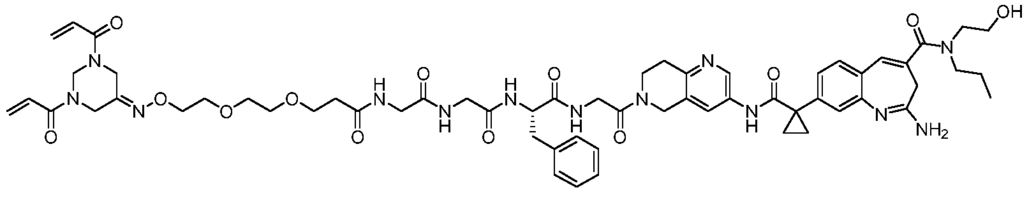

,

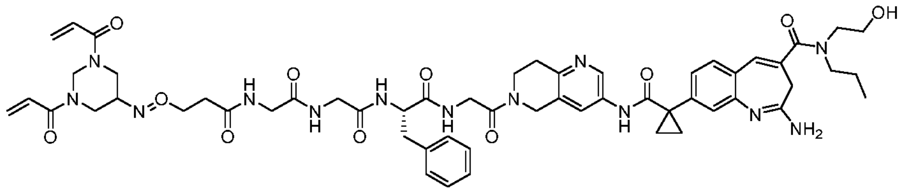

,

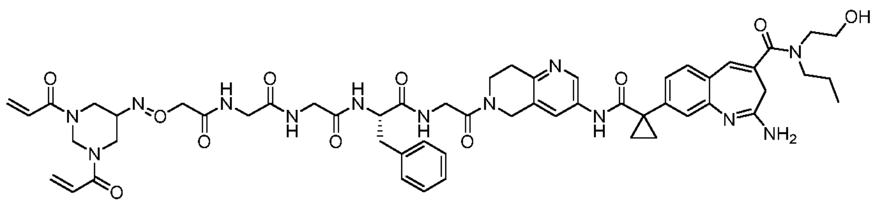

,

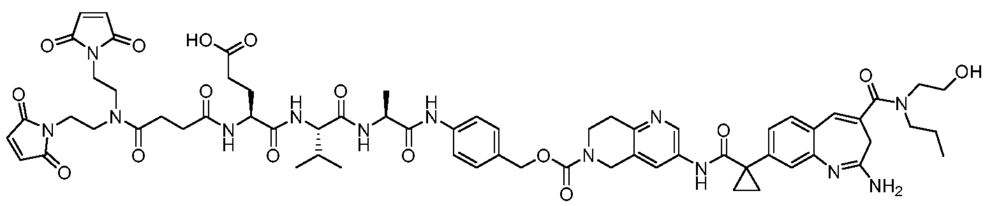

,

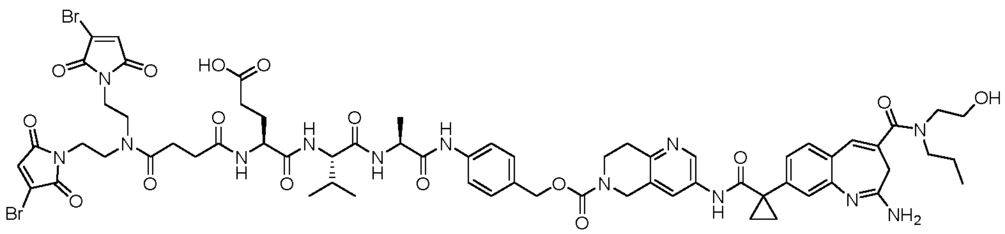

,

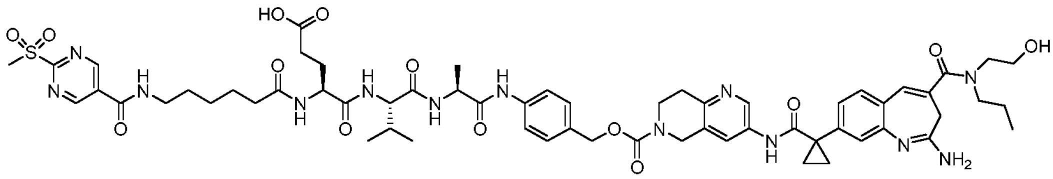

,

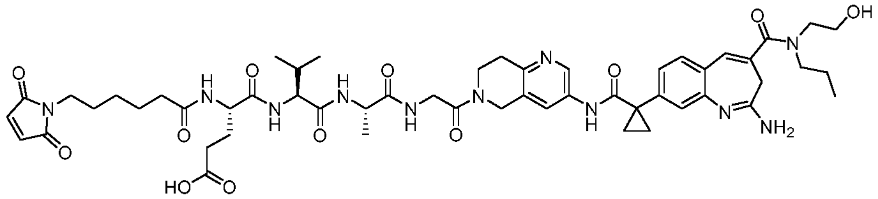

,

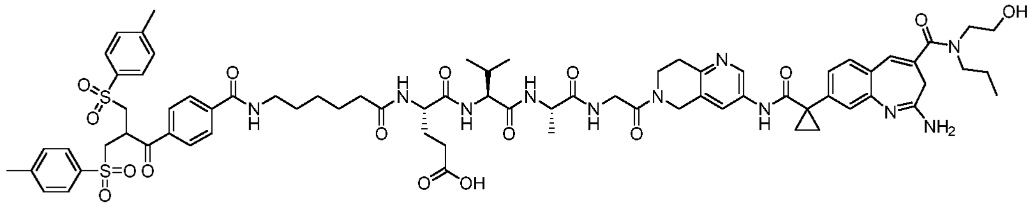

,

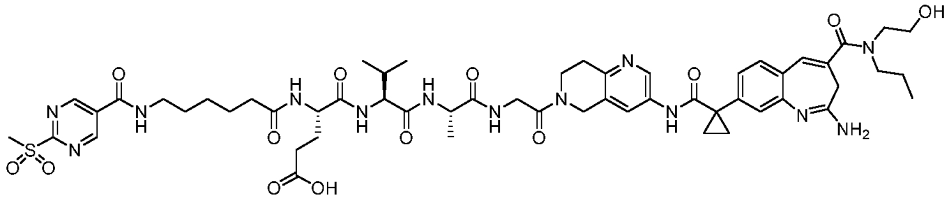

,

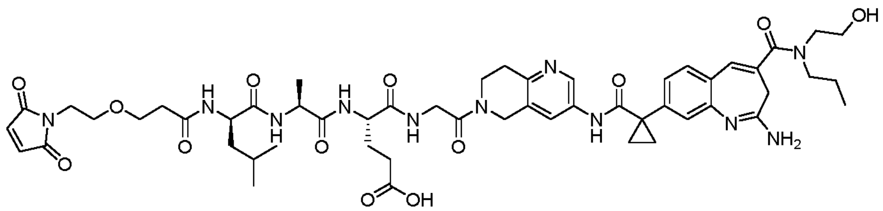

,

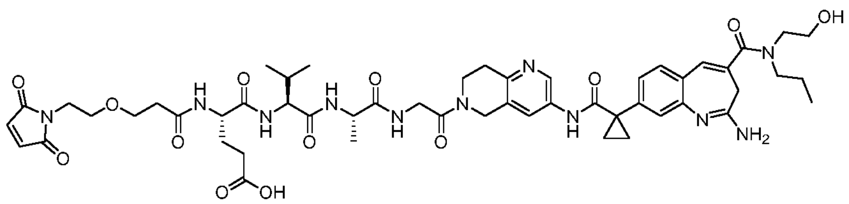

,

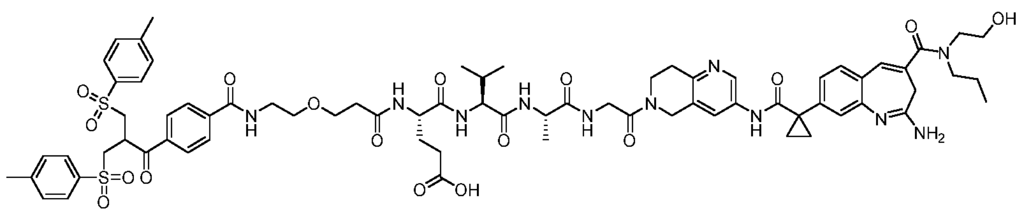

,

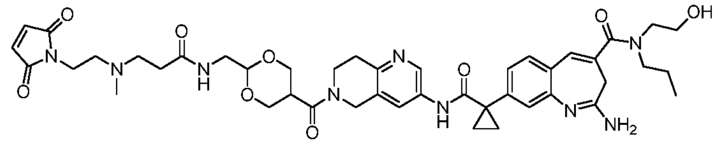

,

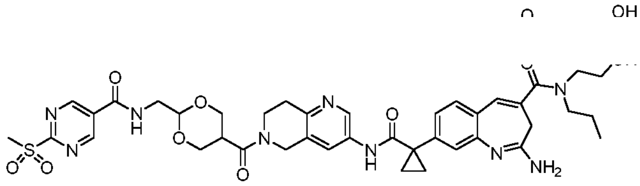

,

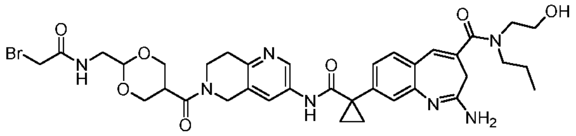

,

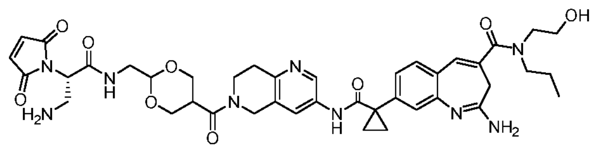

,

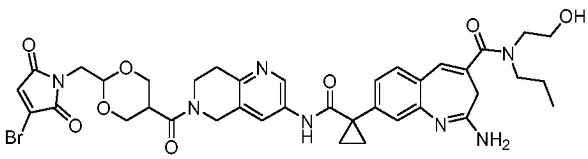

,

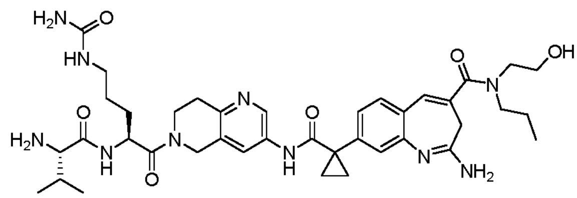

,

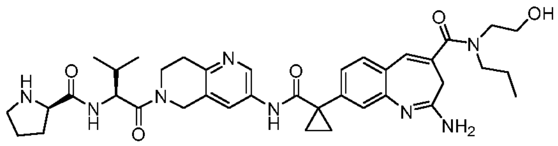

,

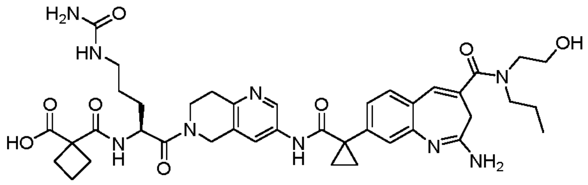

,

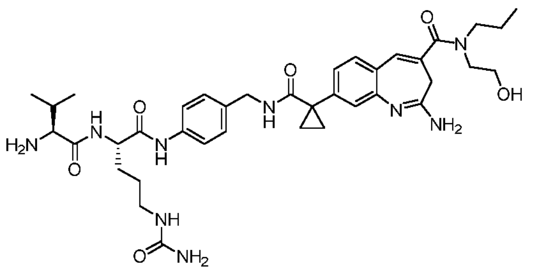

,

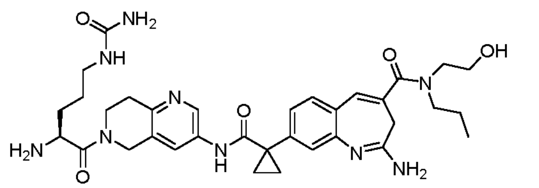

,

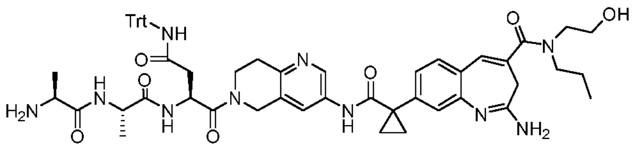

,

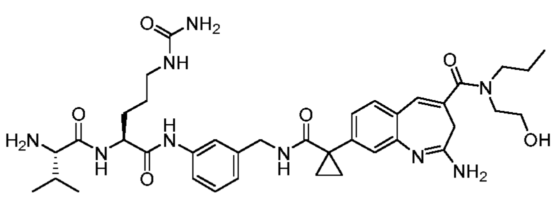

,

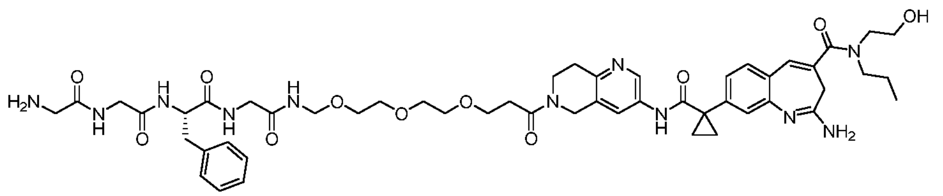

,

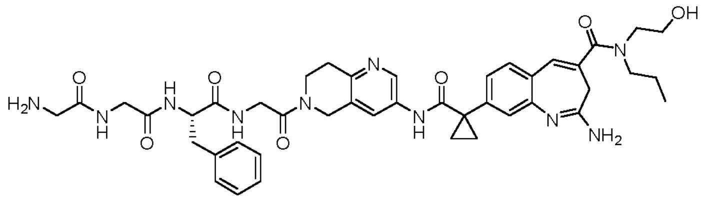

,

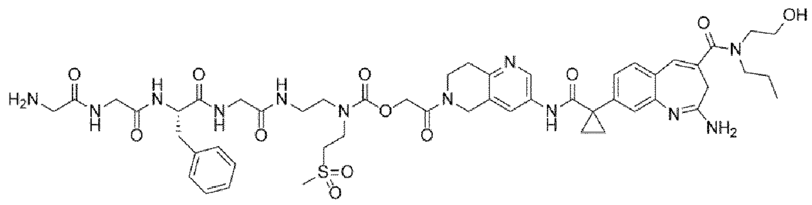

,

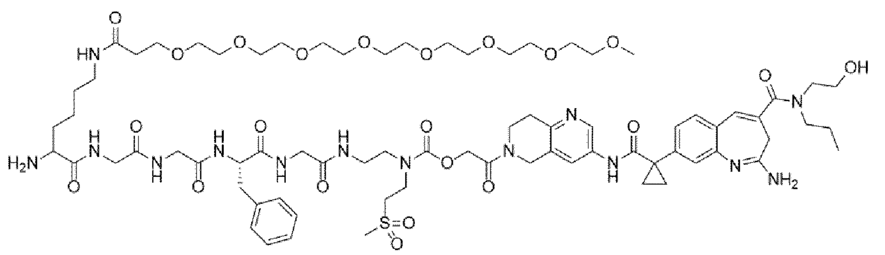

,

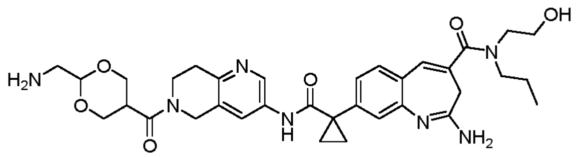

,

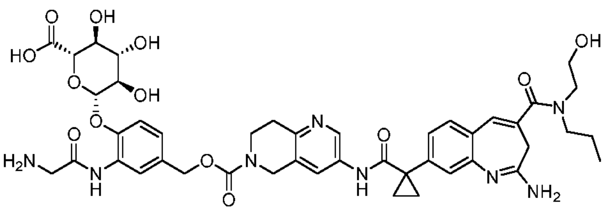

,

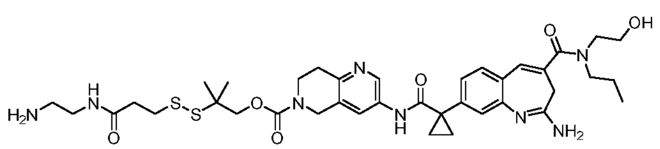

,

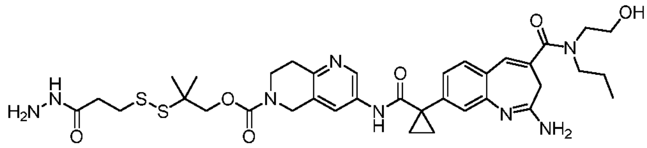

,

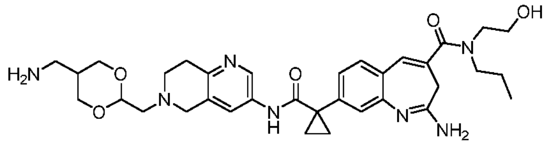

,

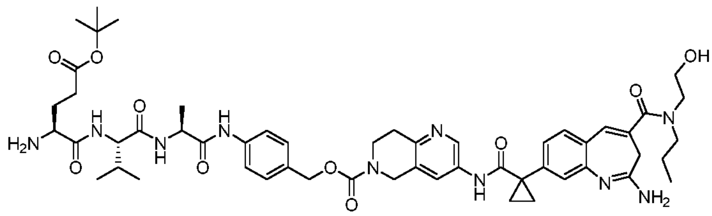

,

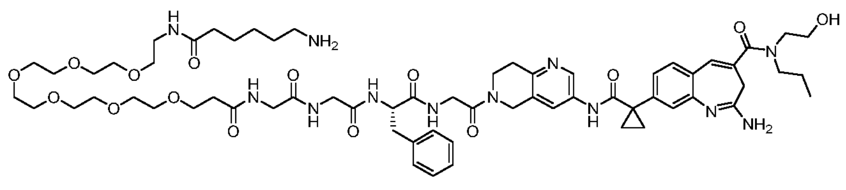

,

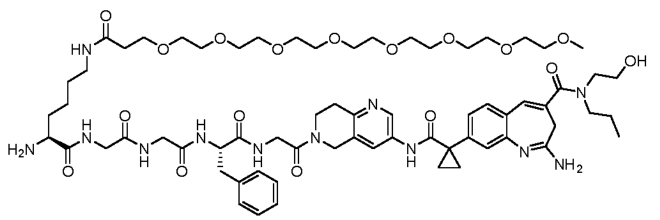

,

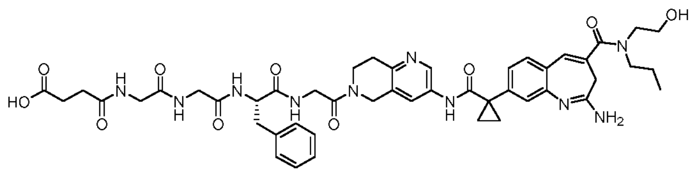

,

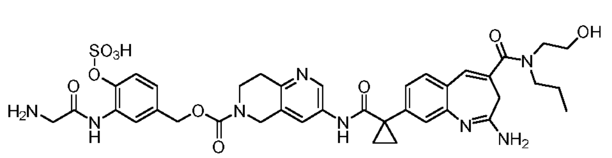

, ,

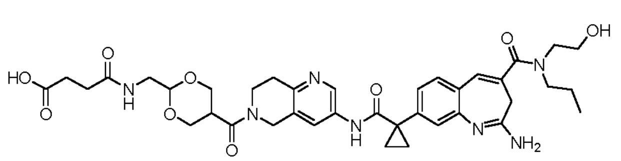

,

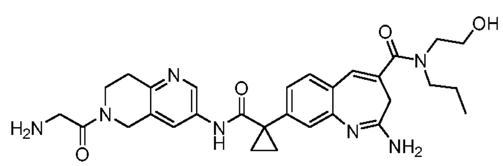

,

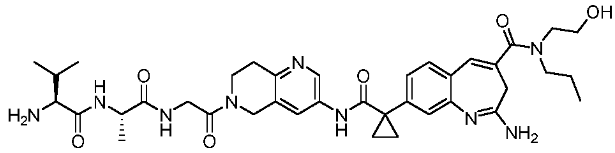

,

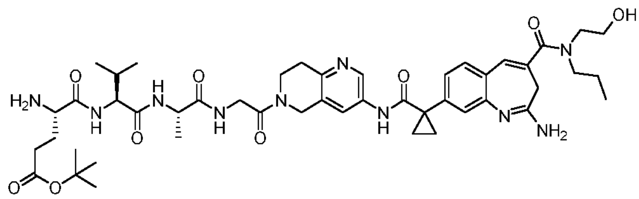

,

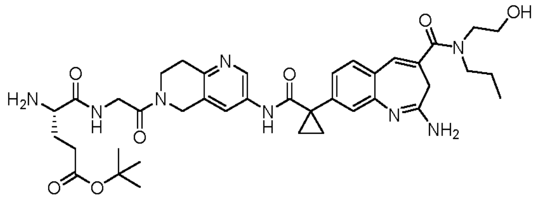

,

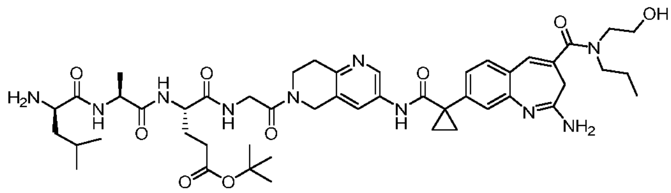

,

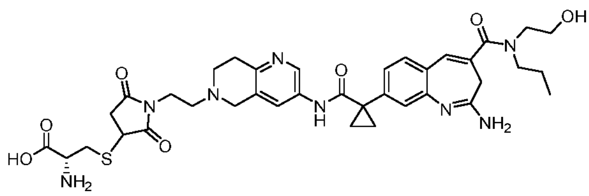

,

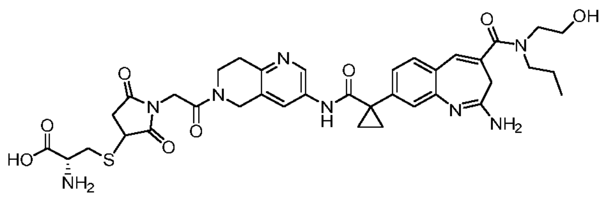

,

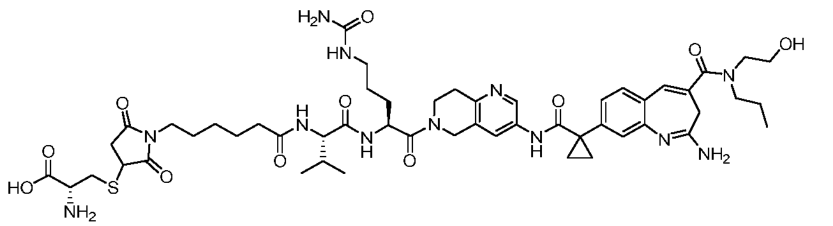

,

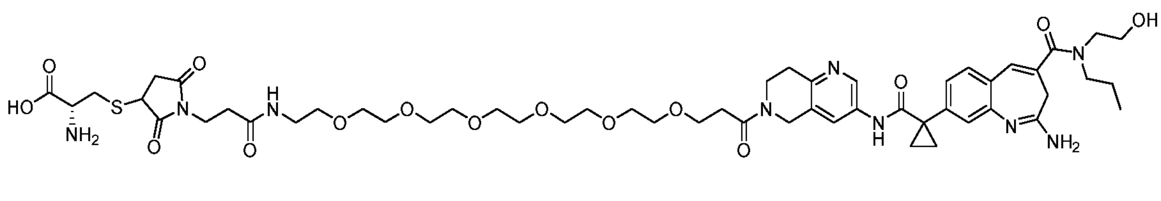

,

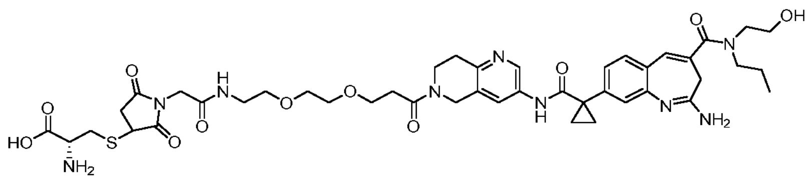

,

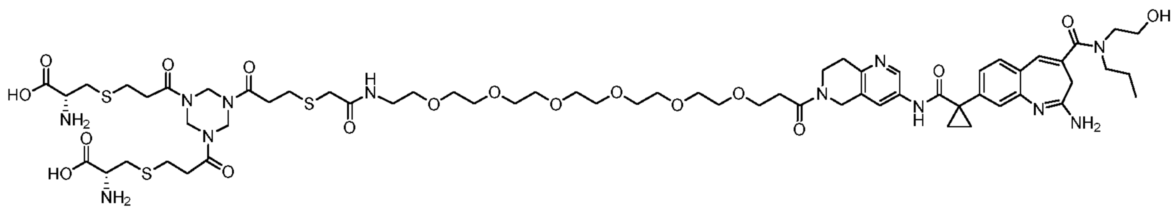

,

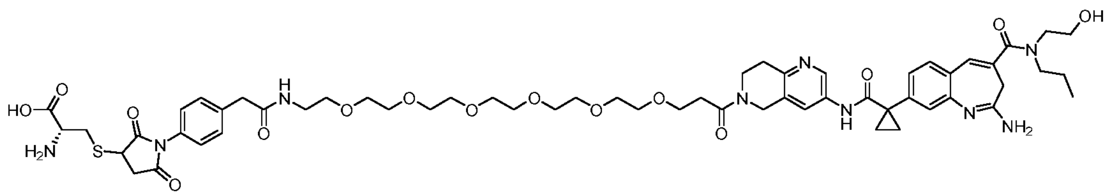

,

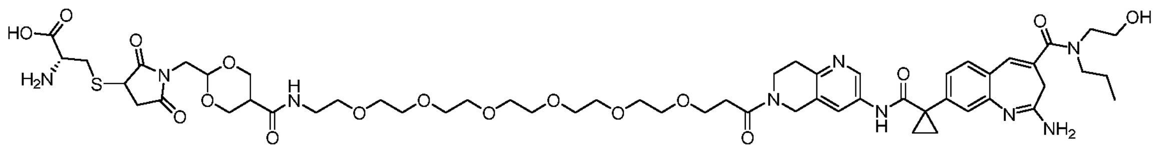

,

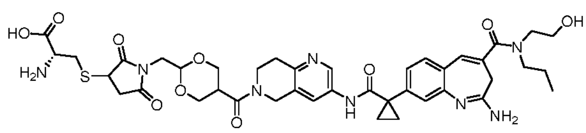

,

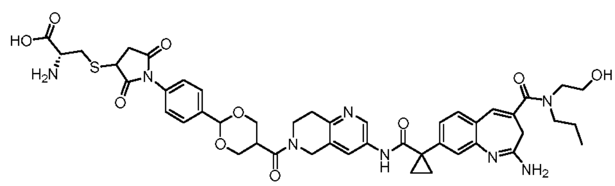

or

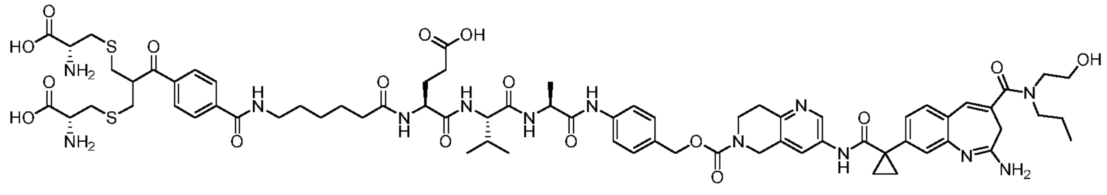

.

**18.** A compound of formula D', a pharmaceutically acceptable salt thereof, a solvate thereof or a solvate of a pharmaceutically acceptable salt thereof,

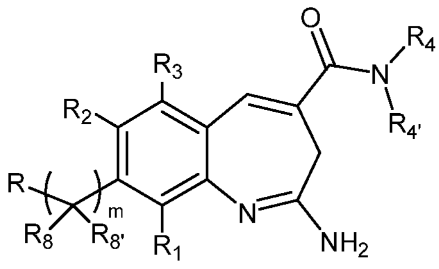

D'

wherein R is -L'-$L_1$-$R_7$; and L' is -C(=O)-, -C(=O)-$NR_9$- or -C(=S)-$NR_9$-;

$R_9$ is independently hydrogen, $C_{1-6}$ alkyl or halo $C_{1-6}$ alkyl;

$L_1$ is independently a linkage bond, $C_{1-6}$ alkylene or $C_{2-6}$ alkenylene;

$R_7$ is independently phenyl, 5-6 membered heteroaryl, 3-8 membered heterocycloalkyl, an 8-12 membered fused ring group, phenyl substituted with 1, 2 or 3 $R_{7-1}$, 5-6 membered heteroaryl substituted with 1, 2 or 3 $R_{7-2}$, 3-8 membered heterocycloalkyl substituted with 1, 2 or 3 $R_{7-3}$ or an 8-12 membered fused ring group substituted with 1, 2 or 3 $R_{7-4}$;

in the $R_7$, in "5-6 membered heteroaryl" in the 5-6 membered heteroaryl and the 5-6 membered heteroaryl substituted with 1, 2 or 3 $R_{7-2}$, the heteroatom is independently selected from one or more of N, O and S, and the number of the heteroatom is independently 1, 2 or 3; and in "3-8 membered heterocycloalkyl" in the 3-8 membered heterocycloalkyl and the 3-8 membered heterocycloalkyl substituted with 1, 2 or 3 $R_{7-3}$, the heteroatom is independently selected from one or more of N, O and S, and the number of the heteroatom is independently 1, 2 or 3;

in the $R_7$, an 8-12 membered fused ring group in the 8-12 membered fused ring group and the 8-12 membered fused ring group substituted with 1, 2 or 3 $R_{7-4}$ is independently a fused ring of ring A and ring B, the ring A is a 5-6 membered heteroaromatic ring or a benzene ring, and the ring B is a 5-6 membered heteroalkene ring; in the 5-6 membered heteroaromatic ring, the heteroatom is selected from one or more of N, O and S, and the number of the heteroatom is 1, 2 or 3; and in the 5-6 membered heteroalkene ring, the heteroatom is selected from one or more of N, O and S, and the number of the heteroatom is 1, 2 or 3;

$R_{7-1}$, $R_{7-2}$, $R_{7-3}$ and $R_{7-4}$ are independently amino, -$L_3$-$NH_2$, -$L_3$-OH, -C(=O)-$L_3$-OH or -C(=O)-$L_3$-$NH_2$, and $L_3$ is independently $C_{1-6}$ alkylene or -$(CH_2CH_2O)_{m'}$-$C_{1-6}$ alkylene-; and m' is 1, 2, 3, 4, 5, 6, 7, 8, 9 or 10; m is 0 or 1;

$R_8$ and $R_{8'}$ are independently hydrogen or $C_{1-6}$ alkyl, or $R_8$ and $R_{8'}$ together with the carbon atom to which they are attached form carbonyl, $C_{3-10}$ cycloalkylene or 3-10 membered heterocycloalkylene; and in the 3-10 membered heterocycloalkylene, the heteroatom is selected from one or more of N, O and S, and the number of the heteroatom is 1, 2 or 3;

$R_1$, $R_2$ and $R_3$ are independently hydrogen, deuterium or halogen;

$R_4$ and $R_{4'}$ are independently $C_{1-6}$ alkyl, $C_{1-6}$ alkoxy or $C_{1-6}$ alkyl substituted with 1, 2 or 3 $R_{4-1}$; and the $R_{4-1}$ is independently hydroxyl, amino, -OC(=O)$NR_aR_b$ or

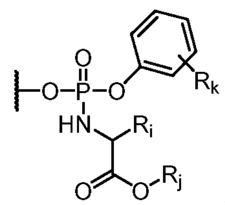

;

$R_a$ and $R_b$ are independently hydrogen, $C_{1-6}$ alkyl or $C_{3-10}$ cycloalkyl;

$R_i$ is hydrogen, $C_{1-6}$ alkyl or benzyl; $R_k$ is hydrogen, halogen or $C_{1-6}$ alkyl; and $R_j$ is $C_{1-6}$ alkyl, $C_{3-10}$ cycloalkyl, 3-10 membered heterocycloalkyl, $C_{6-10}$ aryl, 5-10 membered heteroaryl, $C_{3-10}$ cycloalkyl-$C_{1-6}$ alkyl, 3-10 membered heterocycloalkyl-$C_{1-6}$ alkyl, $C_{6-10}$ aryl-$C_{1-6}$ alkyl or 5-10 membered heteroaryl-$C_{1-6}$ alkyl; and

in the $R_j$, in "3-10 membered heterocycloalkyl" in the 3-10 membered heterocycloalkyl and the 3-10 membered heterocycloalkyl-$C_{1-6}$ alkyl, the heteroatom is independently selected from one or more of N, O and S, and the number of the heteroatom is independently 1, 2 or 3; and in "5-10 membered heteroaryl" in the 5-10 membered heteroaryl and the 5-10 membered heteroaryl-$C_{1-6}$ alkyl, the heteroatom is independently selected from one or more of N, O and S, and the number of the heteroatom is independently 1, 2 or 3;

and the compound of formula D' satisfies 1 or 2 of conditions of:

(1) m is 1; and

(2) at least one of $R_4$ and $R_{4'}$ is $C_{1-6}$ alkoxy or $C_{1-6}$ alkyl substituted with 1, 2 or 3 $R_{4-1}$;

and the compound of formula D' is not a compound shown in Table 3;

Table 3

| | |
|---|---|
| 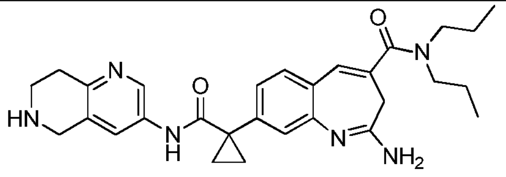 | 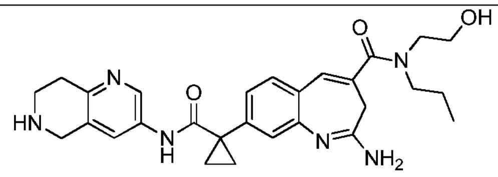 |
| 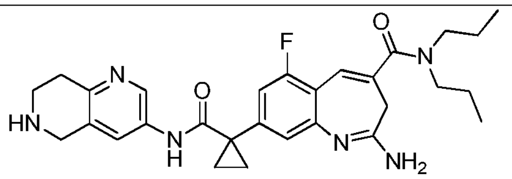 | 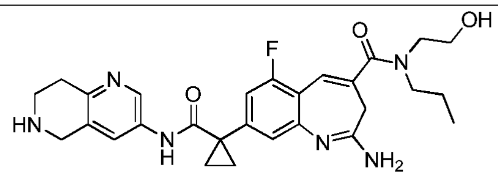 |
| 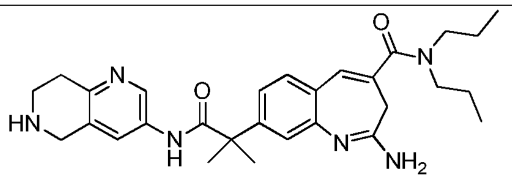 | 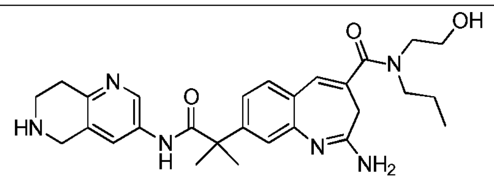 |
| 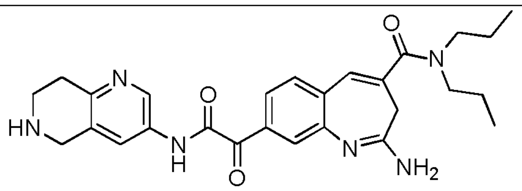 | 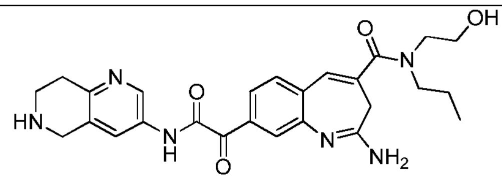 |

| | |
|---|---|
| 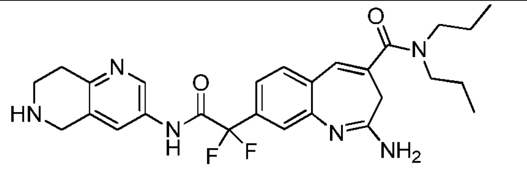 | 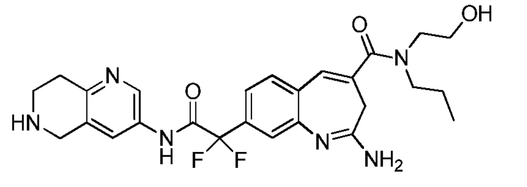 |
| 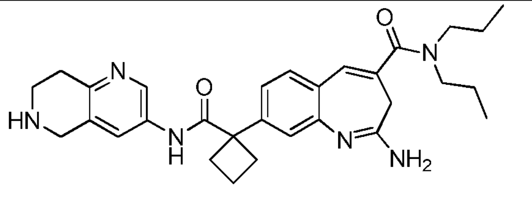 | 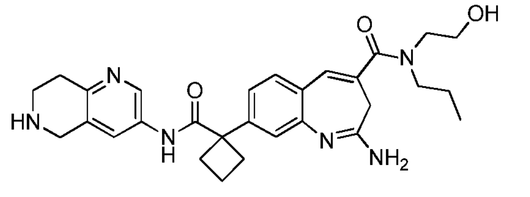 |
| 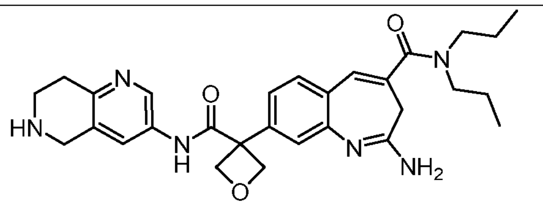 | 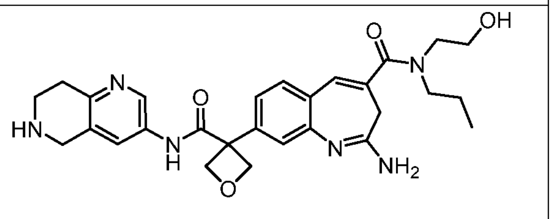 |
| 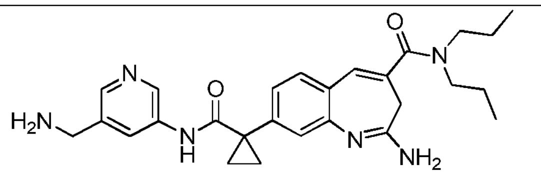 | 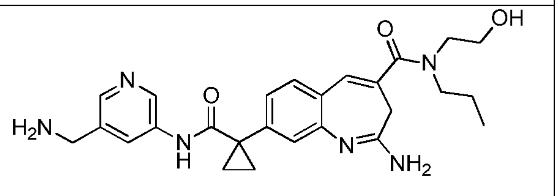 |
| 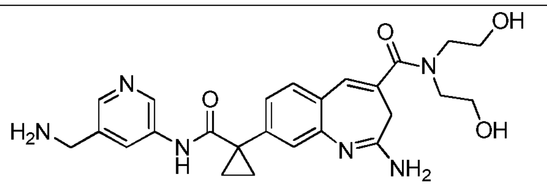 | 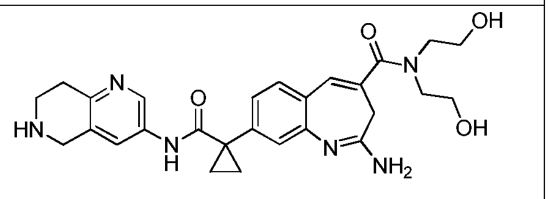 |
| 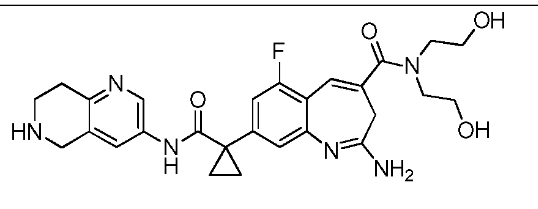 | 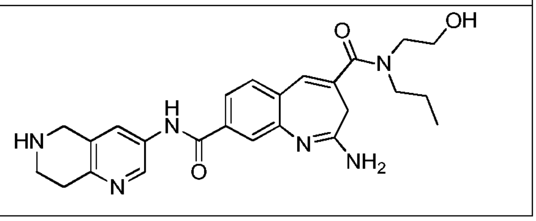 |
| 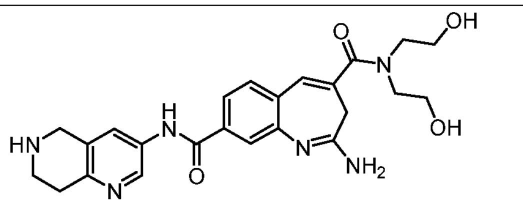 | |

.

**19.** The compound of formula D', the pharmaceutically acceptable salt thereof, the solvate thereof or the solvate of a pharmaceutically acceptable salt thereof according to claim 18, satisfying 1, 2 or 3 of conditions of:

(1) $L_1$ is independently $C_{1-6}$ alkylene or $C_{2-6}$ alkenylene;

(2) $R_7$ is independently phenyl substituted with one amino, 5-6 membered heteroaryl substituted with one amino, 3-8 membered heterocycloalkyl substituted with one amino or an 8-12 membered fused ring group substituted with one amino; and

(3) at least one of $R_4$ and $R_{4'}$ is $C_{1-6}$ alkoxy or $C_{1-6}$ alkyl substituted with 1, 2 or 3 $R_{4-1}$; and the $R_{4-1}$ is independently -OC(=O)$NR_aR_b$ or

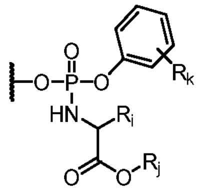
.

**20.** The compound of formula D', the pharmaceutically acceptable salt thereof, the solvate thereof or the solvate of a pharmaceutically acceptable salt thereof according to claim 18, satisfying one or more of conditions of:

(1) L' is -C(=O)-, -C(=O)-$NR_9$- or -C(=S)-$NR_9$-, and a right end thereof is attached to $L_1$;
(2) $R_9$ is hydrogen;
(3) in the $L_1$, the $C_{1-6}$ alkylene is methylene or ethylene;
(4) in the $R_7$, the 8-12 membered fused ring group is a fused ring of ring A and ring B, the ring A is 5-6 membered heteroaryl or phenyl, and the ring B is a 5-6 membered heteroalkenyl ring attached to the $L_1$ through the ring A; in the 5-6 membered heteroaryl, the heteroatom is N, and the number of the heteroatom is 1 or 2; and in the 5-6 membered heteroalkenyl ring, the heteroatom is N, and the number of the heteroatom is 1 or 2;
(5) in the $R_7$, in "5-6 membered heteroaryl" in the 5-6 membered heteroaryl and the 5-6 membered heteroaryl substituted with 1, 2 or 3 $R_{7-2}$, the heteroatom is independently N;
(6) in the $R_7$, in "5-6 membered heteroaryl" in the 5-6 membered heteroaryl and the 5-6 membered heteroaryl substituted with 1, 2 or 3 $R_{7-2}$, the number of the heteroatom is independently 1 or 2;
(7) in the ring A, the heteroatom in the 5-6 membered heteroaryl ring is N;
(8) in the ring A, the number of the heteroatom in the 5-6 membered heteroaryl ring is 1 or 2;
(9) in the ring B, the heteroatom in the 5-6 membered heteroalkenyl ring is N;
(10) one hydrogen atom lost by the compound of formula D' is located on a secondary amine or a primary amine of $R_7$; or one hydrogen atom lost by the compound of formula D' is located on hydroxyl of $R_7$;
(11) m is 1;
(12) in the $R_8$ and $R_{8'}$, the $C_{1-6}$ alkyl is methyl, ethyl, n-propyl or isopropyl;
(13) $R_8$ and $R_{8'}$ together with the carbon atom to which they are attached form $C_{3-10}$ cycloalkylene, and the $C_{3-10}$ cycloalkylene is cyclopropyl or cyclobutyl;
(14) $R_8$ and $R_{8'}$ together with the carbon atom to which they are attached form 3-10 membered heterocycloalkylene, and a heteroatom in the 3-10 membered heterocycloalkylene is N or O;
(15) $R_8$ and $R_{8'}$ together with the carbon atom to which they are attached form 3-10 membered heterocycloalkylene, and the number of the heteroatom in the 3-10 membered heterocycloalkylene is 1 or 2;
(16) in the $R_1$, $R_2$ and $R_3$, the halogen is F, Cl or Br;
(17) in the $R_4$ and $R_4$, the $C_{1-6}$ alkyl is methyl, ethyl, n-propyl or isopropyl;
(18) in the $R_4$ and $R_{4'}$, the $C_{1-6}$ alkoxy is methoxy, ethoxy, n-propoxy or isopropoxy;
(19) in the $R_4$ and $R_4$ , $C_{1-6}$ alkyl in the $C_{1-6}$ alkyl substituted with 1, 2 or 3 $R_{4-1}$ is methyl, ethyl, n-propyl or isopropyl;
(20) in the $R_a$ and $R_b$, the $C_{1-6}$ alkyl is methyl, ethyl, n-propyl or isopropyl;
(21) in the $R_a$ and $R_b$, the $C_{3-10}$ cycloalkyl is cyclopropyl, cyclobutyl or cyclopentyl;
(22) in the $R_i$, the $C_{1-6}$ alkyl is methyl, ethyl, n-propyl or isopropyl;
(23) in the $R_k$, the $C_{1-6}$ alkyl is methyl, ethyl, n-propyl or isopropyl;
(24) in the $R_j$, the $C_{1-6}$ alkyl is methyl, ethyl, n-propyl or isopropyl;
(25) in the $R_j$, in the $C_{6-10}$ aryl-$C_{1-6}$ alkyl, the $C_{6-10}$ aryl is phenyl; and
(26) in the $R_j$, in the $C_{6-10}$ aryl-$C_{1-6}$ alkyl, the $C_{1-6}$ alkyl is methyl, ethyl, n-propyl or isopropyl.

**21.** The compound of formula D', the pharmaceutically acceptable salt thereof, the solvate thereof or the solvate of a pharmaceutically acceptable salt thereof according to claim 20, satisfying one or more of conditions of:

(1) $R_7$ is

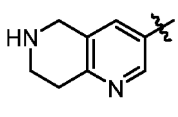
,
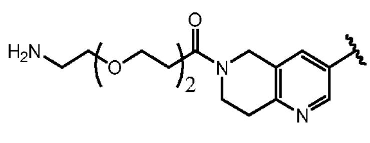
,
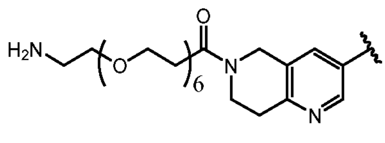
,

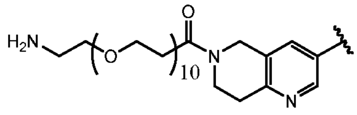, 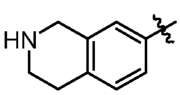, 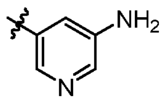, 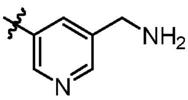, 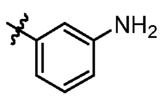,

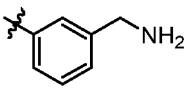, 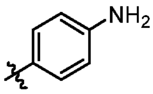, 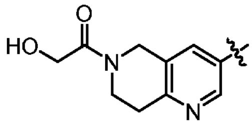 or 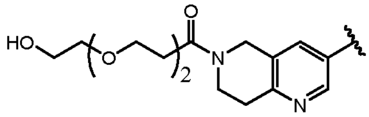;

(2) $R_1$ is H; $R_2$ is H; and $R_3$ is H or F; and

(3) $R_4$ and $R_{4'}$ are independently $-OCH_2CH_3$, $-CH_2CH_2NH_2$, $-CH_2CH_2OH$ or $-CH_2CH_2CH_3$.

**22.** The compound of formula D', the pharmaceutically acceptable salt thereof, the solvate thereof or the solvate of a pharmaceutically acceptable salt thereof according to any one of claims 18 to 21, wherein the compound of formula D' is any one of compounds of:

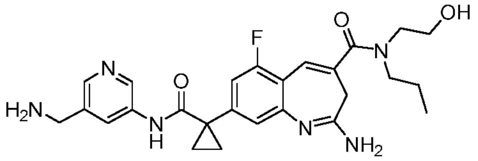,

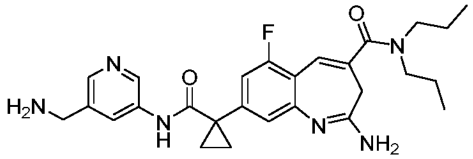,

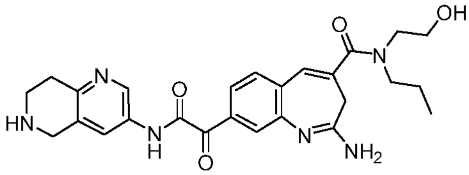,

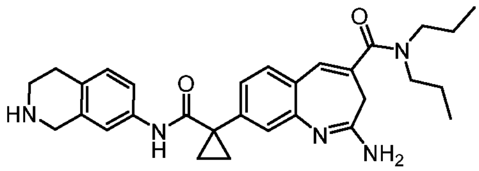,

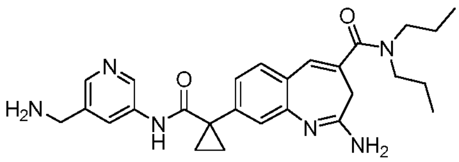,

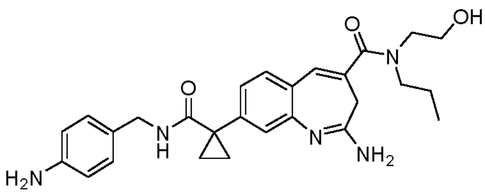,

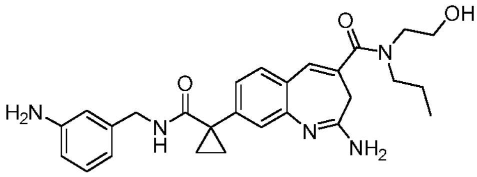,

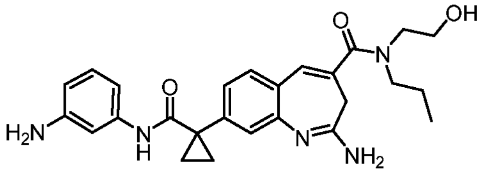,

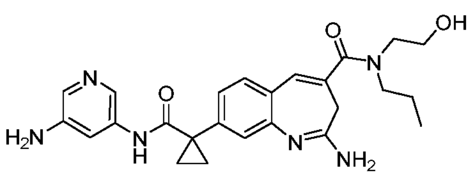,

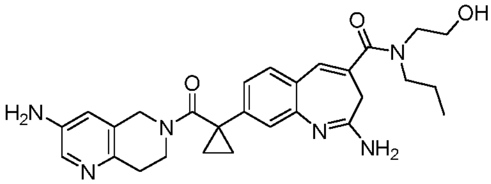,

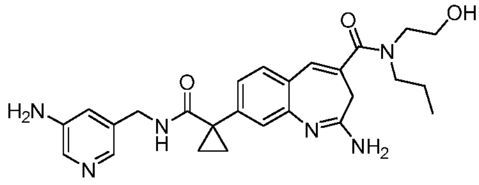

,

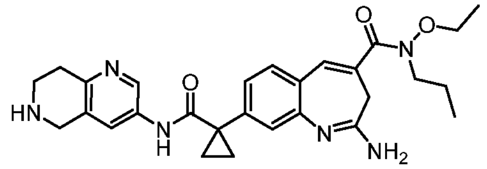

,

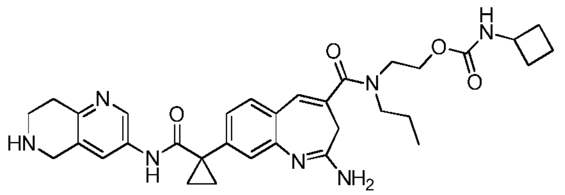

,

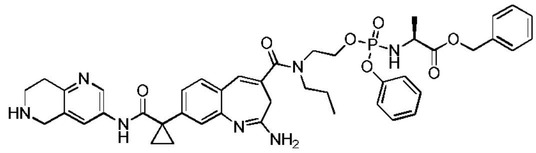

,

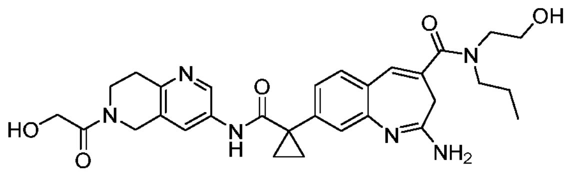

,

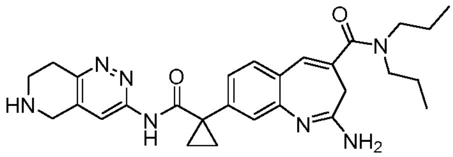

,

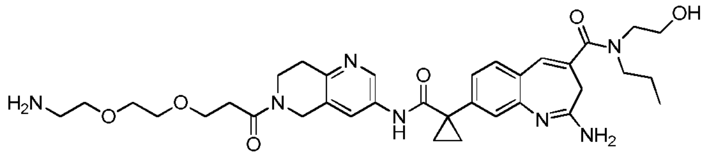

,

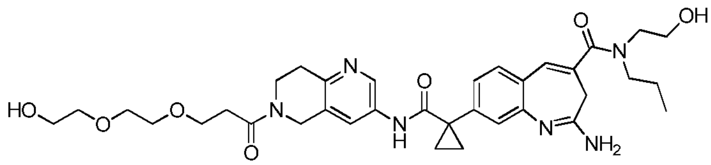

,

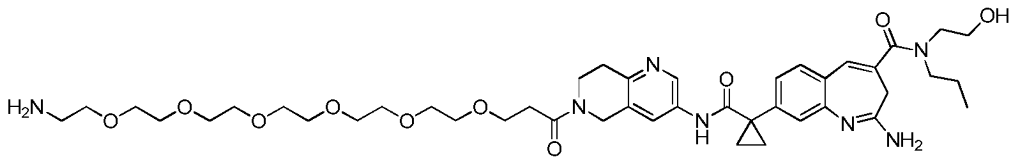

,

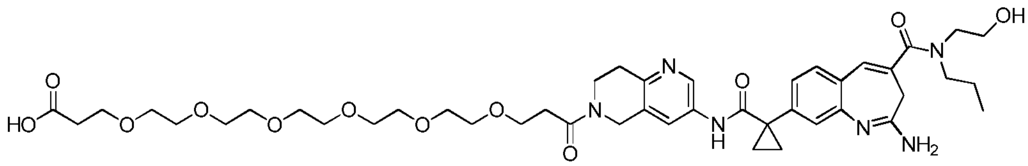

or

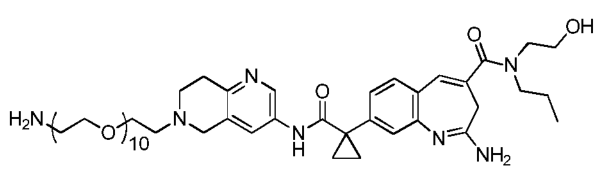

.

**23.** A pharmaceutical composition, comprising a substance K and a pharmaceutically acceptable excipient, wherein the substance K is a substance K-1, a substance K-2 or a substance K-3;

the substance K-1 is the antibody-immunostimulatory conjugate of formula I or the pharmaceutically acceptable salt thereof according to any one of claims 1 to 9;
the substance K-2 is the compound of formula II or the pharmaceutically acceptable salt thereof according to any one of claims 10 to 17; and
the substance K-3 is the compound of formula D', the pharmaceutically acceptable salt thereof, the solvate thereof or the solvate of a pharmaceutically acceptable salt thereof according to any one of claims 18 to 22.

**24.** Use of a substance K or the pharmaceutical composition according to claim 22 in preparation of a medicament,

wherein the medicament is a medicament of a regulatory T cell and other immune cells, a medicament for treating and/or alleviating a tumor or a medicament for treating, alleviating and/or preventing a related disease mediated by TLR8;
wherein the substance K is a substance K-1, a substance K-2 or a substance K-3;
the substance K-1 is the antibody-immunostimulatory conjugate of formula I or the pharmaceutically acceptable salt thereof according to any one of claims 1 to 9;
the substance K-2 is the compound of formula II or the pharmaceutically acceptable salt thereof according to any one of claims 10 to 17; and
the substance K-3 is the compound of formula D', the pharmaceutically acceptable salt thereof, the solvate thereof or the solvate of a pharmaceutically acceptable salt thereof according to any one of claims 18 to 22; and the tumor is preferably a malignant tumor.

**25.** A therapeutic method for treating or preventing a disease, comprising administering the substance K-1 according to claim 24, the substance K-2 according to claim 24 or the substance K-3 according to claim 24 to a subject,

wherein the disease is a disease related to a regulatory T cell and other immune cells, a tumor, or a related disease mediated by TLR8; and
the tumor is preferably a malignant tumor.

**INTERNATIONAL SEARCH REPORT**

International application No.
**PCT/CN2023/122797**

**A. CLASSIFICATION OF SUBJECT MATTER**

C07D223/16(2006.01)i; A61K47/68(2017.01)i; C07K16/28(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K C07K A61P

Documentation searched other than minimum documentation to the extent that such documents are included in the fields searched

Electronic data base consulted during the international search (name of data base and, where practicable, search terms used)

CNABS; CNTXT; ENTXT; DWPI; STNext Registry; STNext Caplus; CNKI: 上海迪诺医药; 张威; 抗体偶联药物; ADC; 苯并氮杂卓; benzazepine; TLR8; 结构检索, structure search

**C. DOCUMENTS CONSIDERED TO BE RELEVANT**

| Category* | Citation of document, with indication, where appropriate, of the relevant passages | Relevant to claim No. |
|---|---|---|
| PX | CN 115448917 A (SHANGHAI DE NOVO PHARMATECH CO., LTD.) 09 December 2022 (2022-12-09) claims 1-28 | 1-25 |
| X | CN 110612104 A (SILVERBACK THERAPEUTICS, INC.) 24 December 2019 (2019-12-24) claims 1-56, 144-184 and 193-207, and table 2 | 1-25 |
| X | CN 114746404 A (BOLT BIOTHERAPEUTICS, INC.) 12 July 2022 (2022-07-12) claims 1-65 | 1-25 |
| X | WO 2020056008 A1 (SILVERBACK THERAPEUTICS, INC.) 19 March 2020 (2020-03-19) claims 1-111 | 1-25 |
| X | WO 2020056194 A1 (SILVERBACK THERAPEUTICS, INC.) 19 March 2020 (2020-03-19) claims 1-218 | 1-25 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 December 2023** | **22 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/122797**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **25**
because they relate to subject matter not required to be searched by this Authority, namely:

Claim 25 relates to a method for treating a disease, and thus does not comply with PCT Rule 39.1(iv). The search is performed on the basis of the corresponding pharmaceutical use of the claim.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/122797**

| Patent document cited in search report | Publication date (day/month/year) | Patent family member(s) | Publication date (day/month/year) |
|---|---|---|---|
| CN 115448917 A | 09 December 2022 | WO 2022242724 A1 | 24 November 2022 |
| | | CA 3218829 A1 | 24 November 2022 |
| CN 110612104 A | 24 December 2019 | EP 3595668 A1 | 22 January 2020 |
| | | EP 3595668 A4 | 01 April 2020 |
| | | EP 3595668 B1 | 21 July 2021 |
| | | ES 2894731 T3 | 15 February 2022 |
| | | IL 269283 A | 28 November 2019 |
| | | IL 269283 B | 01 December 2022 |
| | | IL 269283 B2 | 01 April 2023 |
| | | SG 10202110182 PA | 28 October 2021 |
| | | DK 3595668 T3 | 18 October 2021 |
| | | HUE 056427 T2 | 28 February 2022 |
| | | RU 2019132260 A | 15 April 2021 |
| | | RU 2019132260 A3 | 07 December 2021 |
| | | CA 3055652 A1 | 20 September 2018 |
| | | AU 2018236272 A1 | 17 October 2019 |
| | | AU 2018236272 B2 | 31 March 2022 |
| | | US 2018258048 A1 | 13 September 2018 |
| | | US 10519131 B2 | 31 December 2019 |
| | | SG 10202110181 RA | 28 October 2021 |
| | | JP 2020514419 A | 21 May 2020 |
| | | SG 11201908131 RA | 30 October 2019 |
| | | US 2019169164 A1 | 06 June 2019 |
| | | US 10428045 B2 | 01 October 2019 |
| | | US 2019169165 A1 | 06 June 2019 |
| | | US 10442790 B2 | 15 October 2019 |
| | | SG 10202110184 XA | 28 October 2021 |
| | | BR 112019019116 A2 | 05 May 2020 |
| | | EP 3949969 A2 | 09 February 2022 |
| | | EP 3949969 A3 | 16 February 2022 |
| | | SG 10202110183 VA | 28 October 2021 |
| | | KR 20200031560 A | 24 March 2020 |
| | | PT 3595668 T | 21 October 2021 |
| | | MX 2019010804 A | 23 January 2020 |
| | | US 2019062306 A1 | 28 February 2019 |
| | | US 10239862 B2 | 26 March 2019 |
| | | WO 2018170179 A1 | 20 September 2018 |
| | | US 2020031798 A1 | 30 January 2020 |
| CN 114746404 A | 12 July 2022 | AU 2020359446 A1 | 21 April 2022 |
| | | CA 3152601 A1 | 08 April 2021 |
| | | KR 20220077131 A | 08 June 2022 |
| | | EP 4038053 A1 | 10 August 2022 |
| | | WO 2021067242 A1 | 08 April 2021 |
| | | BR 112022006001 A2 | 12 July 2022 |
| | | JP 2022549510 A | 25 November 2022 |
| | | IL 291760 A | 01 June 2022 |
| | | MX 2022003740 A | 02 May 2022 |
| | | US 2022347310 A1 | 03 November 2022 |
| WO 2020056008 A1 | 19 March 2020 | AU 2019339344 A1 | 18 March 2021 |
| | | SG 11202101980 VA | 30 March 2021 |

Form PCT/ISA/210 (patent family annex) (July 2022)

**INTERNATIONAL SEARCH REPORT**
**Information on patent family members**

International application No.
**PCT/CN2023/122797**

| Patent document cited in search report | Publication date (day/month/year) | Patent family member(s) | Publication date (day/month/year) |
|---|---|---|---|
| | | US 2020113912 A1 | 16 April 2020 |
| | | IL 281210 A | 29 April 2021 |
| | | MX 2021002764 A | 12 May 2021 |
| | | JP 2022500414 A | 04 January 2022 |
| | | KR 20210081332 A | 01 July 2021 |
| | | CA 3111784 A1 | 19 March 2020 |
| | | BR 112021004590 A2 | 25 May 2021 |
| | | EP 3849615 A1 | 21 July 2021 |
| WO 2020056194 A1 | 19 March 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022112114506
- CN 2022114492843
- CN 2023112215516
- US 5821337 A
- US 7560111 B
- WO 2006031653 A1
- US 8044178 B2
- WO 2013068874 A
- CN 103933575 B
- WO 2014197871 A2
- CN 111433188 B
- WO 2016142049 A1
- US 20220024904 A1
- CN 113698414 A
- CN 112341521 A
- CN 104379168 B
- CN 114106088 A
- WO 2023076848 A1
- US 20210308275 A1
- US 10772965 B2
- WO 2005007197 A2
- CN 105474015 B
- US 20120201809 A1
- WO 2018138356 A1
- US 10960082 B2
- CN 100360567 C
- US 10738108 B2
- US 5874540 A
- US 10894090 B2


### Non-patent literature cited in the description


- *Int Immunopharmacol*, 2002, vol. 2, 443-451
- *Proc. Natl. Acad. Sci. U.S.A.*, 15 May 1969, vol. 63 (1), 78-85
- *Journal of Pharmaceutical Sciences.*, 2015, vol. 104 (6), 1866-1884
- **HUDIS CA**. *N Engl J Med.*, 2007, vol. 357 (1), 39-51
- **CHO et al.** *Nature*, 2003, vol. 421, 756-760
- **FRANKLIN et al.** *Cancer Cell*, 2004, vol. 5, 317-328
- **AGUS et al.** *J Clin Oncol.*, 07 February 2005, vol. 23 (11), 2534-43
- **NORDSTROM J. et al.** *Breast Cancer Research*, 2011, vol. 13, R123
- **BERGSTROM D. A. et al.** *Cancer Res.*, 2015, vol. 75, LB-231
- *Mol. Pharmaceutics*, 2015, vol. 12, 3986-3998
- *Org. Biomol. Chem.*, 2014, vol. 12, 7261-7269
- *Bioconjugate Chem.*, 2008, vol. 19, 759-765
- *Bioorganic Med. Chem. Lett.*, 2017, vol. 27, 1154-1158
- *RSC Adv.*, 2017, vol. 7, 9073-9077
- *Org. biomol. Chem.*, 2018, vol. 16, 1359-1366
- *Org. Biomol. Chem.*, 2020, vol. 18, 4739-4743
- *Chem. Sci.*, 2021, vol. 12, 9060-9068
- *Bioconjugate Chem.*, 2007, vol. 18, 61-76
- *Bioconjugate Chem.*, 2014, vol. 25 (2), 202-206
- *Med. Chem. Commun.*, 2018, vol. 9, 827-830
- *Nat. Commun.*, 2016, vol. 7, 13128
- *Nat. Commun.*, 2020, vol. 11 (1015), 1-10
- *RSC Advances*, 2012, vol. 2, 908-914
- **BERGE et al.** Pharmaceutically acceptable salts. *J. Pharm. Sci.*, 1977, vol. 66, 1-19
- *CHEMICAL ABSTRACTS*, 1425485-72-8
- *CHEMICAL ABSTRACTS*, 124242-93-9
- *Molecular Pharmaceutics.*, 2017, vol. 14, 1501-1516